(19) **Europäisches Patentamt / European Patent Office / Office européen des brevets**

(11) **EP 1 923 464 A1**

(12) **EUROPEAN PATENT APPLICATION**

(43) Date of publication:
**21.05.2008 Bulletin 2008/21**

(21) Application number: **08002997.8**

(22) Date of filing: **29.08.2003**

(51) Int Cl.:
*C12N 15/00* (2006.01)     *C07K 14/195* (2006.01)
*C12Q 1/68* (2006.01)     *C12Q 1/02* (2006.01)

(84) Designated Contracting States:
**DE FR GB**

(30) Priority: **30.08.2002   JP 2002319011**

(62) Document number(s) of the earlier application(s) in accordance with Art. 76 EPC:
**03793947.7 / 1 548 105**

(71) Applicant: **Japan Science and Technology Agency Kawaguchi-shi, Saitama 332-0012 (JP)**

(72) Inventors:
• **Imanaka, Tadayuki**
**Suita-shi**
**Osaka 565-0873 (JP)**

• **Atomi, Haruyuki**
**Kyoto-shi**
**Kyoto 606-8102 (DE)**

(74) Representative: **Holliday, Louise Caroline**
**D Young & Co**
**120 Holborn**
**London EC1N 2DY (GB)**

Remarks:
This application was filed on 19-02-2008 as a divisional application to the application mentioned under INID code 62.

(54) **Ligase of a hyperthermostable bacterium and chip using the same**

(57)     It is intended to provide an efficient and sure gene targeting method embodied at an arbitrary position in the genome of an organism and a kit therefor. It is also intended to provide a method for targeted-disruption of an arbitrary gene in the genome of an organism which comprises: 1) the step of providing the whole sequencial data of the genome of the organism; 2) the step of selecting at least one arbitrary region in the sequence; 3) the step of providing a vector containing a sequence homologous with the region selected above and a marker gene; 4) the step of transforming the organism by the vector; and 5) the step of providing the organism under such conditions as allowing homologous recombination. Moreover, the genome of a hyperthermostable bacterium and its array are provided.

**Description**

TECHNICAL FIELD

The present invention relates to genomics. More specifically, the present invention relates to a genome of a hyperthermostable bacterium and a genome chip thereof. The present invention relates to a novel method for targeted disruption.

BACKGROUND ART

Hyperthermostable bacteria survive in high temperature environments, proteins (such as enzymes) produced by the bacteria are generally thermostable, i.e., structurally stable. Further, archaebacteria, to which the hyperthermostable bacteria belong, are living organisms different from conventionally known prokaryotic or eukaryotic organisms. Therefore, it is clear that the hyperthermostable bacteria are evolutionally different from these organisms. Accordingly, even if an enzyme derived from the hyperthermostable bacteria has similar functions to those already known derived from prokaryotic or eukaryotic cells, the enzymes derived from the hyperthermostable bacteria are often structurally and/or enzymatically different from conventional enzymes. For example, chaperonin isolated from the KOD-1 strain (Thermococcus kodakaraensis KOD1, hereinafter also called KOD1 or KOD1 strain; Morikawa,M.et al., Appl.Environ.Microbiol. 60(12), 4559-4566(1994)), a hyperthermostable bacterium, has similar functions to GroEL from Escherichia coli. However, GroEL forms a 14-mer and further complexes with GroES, which forms a 7-mer, in order to achieve its functions, whereas the chaperonin from KOD-1 strain functions alone (Yan, Z. et al., Appl. Environ. Microbiol. 63: 785-789).
Gene disruption using a plasmid is conventionally known as a method for targeted disruption of a gene in thermostable bacteria (Bartolucci S., Third International Congress on Extremophiles Hamburg, Germany, September 3-7, 2000). The method of Bartolucci utilizes a homogeneous or heterogeneous expression system with a recombinant protein using a thermostable bacterium. However, it is unclear as to whether targeted genes are definitely disrupted by this method, and therefore it cannot be said that effecient targeted disruption is achieved.
Accordingly, there is a limitation in gene targeting based on information of some of the genes.
Therefore, it is an object of the invention to provide a method for gene targeting in an efficient and definite manner in an arbitrary site of a genome of a living organism, and a kit therefor.
Further, there is no method as of this date for analysing a genome as a whole in an efficient and/or global manner by the genome of a hyperthermostable bacterium onto a chip. Therefore, it is another object of the invention to develop a technology for analysing such a genome as a whole in an efficient and/or global manner.

SUMMARY OF INVENTION

The above identified problem has been solved by using an entire sequence of a genome of a living organism for targeting a portion of chromosomes thereof. In particular, the present invention demonstrates that the above-mentioned method has been carried out in an efficient and definite manner by sequencing the whole genome of Thermococcus kodakaraensis KOD1 strain, a strain of thermostable bacteria, as an example of genomic sequence.
The present invention also provides for the first time a technology for analyzing an entire genome in an efficient and/or global manner by sequencing the entire genomic sequence of Thermococcus kodakaraensis KOD1 strain, a strain of the thermostable bacteria as an example of the genomic sequence. Therefore, it is now possible to simulate gene expression of the organism per se on a chip.
Accordingly, the present invention provides the following:

1) A method for targeted-disuption of an arbitrary gene in the genome of a living organism comprising the steps of:

A) providing information of the entire sequence of the genome of the living organism;
B) selecting at least one arbitrary region of the sequence;
C) providing a vector comprising a sequence complementary to the selected region and a marker gene;
D) transforming the living organism with the vector; and
E) placing the living organism in a condition allowing homologous recombination.

(2) The method accoding to Item 1 wherein in the step B), the region comprises at least two regions.

(3) The method accoridng to Item 1, wherein the vector further comprises a promoter.

(4) The method according to Item 1 further comprising the step of detecting an expression product of the marker gene.

(5) The method according to Item 1 wherein the marker gene is located in the selected region.

(6) The method according to Item 1, wherein the marker is located outside of the selected region.

(7) The method according to Item 1, wherein the genome is the genome of Thermococcus kodakaraensis KOD1.

(8) The method according to Item 1, wherein the genome has a sequence set forth in SEQ ID NO: 1 or 1087.

(9) The method according to Item 1, wherein the region comprises a sequence encoding at least one sequence selected from the group consisting of SEQ ID NOs: 2-341, 343-722, 724-1086, 1088-1468, 1470-1837 and 1839-2157.

(10) A nucleic acid molecule having a sequence set forth in SEQ ID NO: 1 or 1087.

(11) A nucleic acid molecule comprising at least eight contiguous nucleic acid sequence of a sequence set forth in SEQ ID NO: 1 or 1087.

(12) A nucleic acid molecule comprising a sequence encoding an amino acid sequence encoding at least one sequence selected from the group consisting of SEQ ID NOs: 2-341, 343-722, 724-1086, 1088-1468, 1470-1837 and 1839-2157; or a sequence having 70 % homology thereto.

(13) A nucleic acid molecule wherein when the reading frame of Table 2 is f-1, f-2 or f-3, the nucleic acid molecule has a sequence from the position of nucleic acid number (sense strand, start) of SEQ ID NO: 1 of Table 2, to the position of nucleic acid number (sense strand, stop) or a sequence having at least 70 % homology thereto, or when the reading frame of Table 2 is r-1, r-2 or r-3, the nucleic acid molecule has a a sequence from the position of nucleic acid number (antisense strand, start) of SEQ ID NO: 1087 of Table 2, to the position of nucleic acid number (antisense strand, stop) or a sequence having at least 70 % homology thereto.

(14) A polypeptide comprising at least one amino acid sequence selected from the group consisting of SEQ ID NO: 2-341, 343-722, 724-1086, 1088-1468, 1470-1837 and 1839-2157, or a sequence having at least 70 % homology thereto.

(15) A polypeptide comprising at least three contiguous amino acids of an amino acid sequence selected from the group consisting of SEQ ID NO: 2-341, 343-722, 724-1086, 1088-1468, 1470-1837 and 1839-2157, or a sequence having at least 70 % homology thereto.

(16) A polypeptide comprising at least eight contiguous amino acids of an amino acid sequence selected from the group consisting of SEQ ID NO: 2-341, 343-722, 724-1086, 1088-1468, 1470-1837 and 1839-2157, or a sequence having at least 70 % homology thereto.

(17) A polypeptide comprising at least three contiguous amino acids of an amino acid sequence selected from the group consisting of SEQ ID NO: 2-341, 343-722, 724-1086, 1088-1468, 1470-1837 and 1839-2157, or a sequence having at least 70 % homology thereto, wherein the polypeptide has biological activity.

(18) The polypeptide according to Item 17, wherein the biological activity comprises a function set foth in Table 2.

(19) A method for screening for a heat resistant protein, comprising the steps of:

A) providing the entire sequence of the genome of a thermoresistant living organism;
B) selecting at least one arbitrary region of the sequence;
C) providing a vector comprising a sequence complementary to the selected region and a gene encoding a candidate for the heat resistance protein;
D) transforming the living organism with the vector;
E) placing the thermoresistant living organism in a condition allowing to cause homologous recombination;
F) selecting the thermoresistant living organism in which homologous recombination has occurred; and
G) assaying to identify the thermoresistant protein.

(20) A kit for screening for a thermoresistant protein, comprising:

A) a thermoresistant living organism; and

B) a vector comprising a sequence complementary to the selected region and a gene encoding a candidate for the thermoresistant protein.

(21) The kit according to Item 20, further comprising an assay system for identifying the thermoresistant protein.

(22) The kit according to Item 20, wherein the thermoresistant living organism is hyperthermophilic bacteria.

(23) The kit according to Item 20, wherein the thermoresistant living organism is Thermococcus kodakaraensis KOD1.

(24) A biomolecule chip having at least one nucleic acid molecule having at least eight contiguous or non-contiguous nucleotides of the sequences set forth in SEQ ID NOs: 1 or 1087, or a variant thereof located therein.

(25) The biomolecule chip according to Item 24, wherein the nucleic acid molecule or the variant thereof is located to cover the sequences set forth in SEQ ID NO: 1 or 1087.

(26) The biomolecule chip according to Item 24, wherein the nucleic acid molecule or the variant thereof comprises any open reading frame of the sequences set forth in SEQ ID NO: 1 or 1087.

(27) The biomolecule chip according to Item 24, wherein the nucleic acid molecule or the variant thereof comprises substantially all open reading frames of the sequences set forth in SEQ ID NO: 1 or 1087.

(28) The biomolecule chip according to Item 24, wherein the nucleic acid molecule or the variant thereof comprises a sequence encoding at least one sequence selected from the group consisting of SEQ ID NOs: 2-341, 343-722, 724-1086, 1088-1468, 1470-1837 and 1839-2157.

(29) The biomolecule chip according to Item 24, wherein the nucleic acid molecule or the variant thereof comprises substantially all the sequences encoding sequences selected from the group consisting of SEQ ID NOs: 2-341, 343-722, 724-1086, 1088-1468, 1470-1837 and 1839-2157.

(30) The biomolecule chip according to Item 24, wherein the nucleic acid molecule or the variant thereof comprises at least eight contiguous nucleotide lengths of substantially all the sequences encoding sequences selected from the group consisting of SEQ ID NOs: 2-341, 343-722, 724-1086, 1088-1468, 1470-1837 and 1839-2157.

(31) The biomolecule chip according to Item 24, wherein the nucleic acid molecule or the variant thereof comprises at least fifteen contiguous nucleotide lengths of substantially all the sequences encoding sequences selected from the group consisting of SEQ ID NOs: 2-341, 343-722, 724-1086, 1088-1468, 1470-1837 and 1839-2157.

(32) The biomolecule chip according to Item 24, wherein the nucleic acid molecule or the variant thereof comprises at least thirty contiguous nucleotide lengths of substantially all the sequences encoding sequences selected from the group consisting of SEQ ID NOs: 2-341, 343-722, 724-1086, 1088-1468, 1470-1837 and 1839-2157.

(33) The biomolecule chip according to Item 24, wherein the nucleic acid molecule or the variant thereof, comprises substantially all the sequences encoding sequences selected from the group consisting of SEQ ID NOs: 2-341, 343-722, 724-1086, 1088-1468, 1470-1837 and 1839-2157, or sequences with one or more amino acid substitution, addition and/or deletion thereto.

(34) The biomolecule chip according to Item 24, wherein the nucleic acid molecule or the variant thereof, comprises at least eight contiguous nucleotide lengths of substantially all the sequences encoding sequences selected from the group consisting of SEQ ID NOs: 2-341, 343-722, 724-1086, 1088-1468, 1470-1837 and 1839-2157, or sequences with one or more amino acid substitution, addition and/or deletion thereto.

(35) The biomolecule chip according to Item 24, wherein when the reading frame of Table 2 is f-1, f-2 or f-3, the nucleic acid molecule or the variant thereof, has a sequence from the position of nucleic acid number (sense strand, start) of SEQ ID NO: 1 of Table 2, to the position of nucleic acid number (sense strand, stop) or a sequence having at least 70 % homology thereto, or when the reading frame of Table 2 is r-1, r-2 or r-3, the nucleic acid molecule has a sequence from the position of nucleic acid number (antisense strand, start) of SEQ ID NO: 1087 of Table 2, to the position of nucleic acid number (antisense strand, stop) or a sequence having at least 70 % homology thereto.

(36) The biomoleculeip to Item 24, wherein the substrate is addressable.

(37) A biomolecule chip with a polypeptide or a variant thereof, having at least one amino acid sequence selected from the group consisting of SEQ ID NO: 2-341, 343-722, 724-1086, 1088-1468, 1470-1837 and 1839-2157, or a sequence having at least 70 % homology thereto, located therein.

(38) The biochip according to Item 37, wherein the polypeptide or the variant thereof, has at least three contiguous amino acid lenghs of at least one amino acid sequence selected from the group consisting of SEQ ID NO: 2-341, 343-722, 724-1086, 1088-1468, 1470-1837 and 1839-2157, or a sequence having at least 70 % homology thereto, located therein.

(39) The biochip according to Item 37, wherein the polypeptide or the variant thereof, has at least eight contiguous amino acid lenghs of at least one amino acid sequence selected from the group consisting of SEQ ID NO: 2-341, 343-722, 724-1086, 1088-1468, 1470-1837 and 1839-2157, or a sequence having at least 70 % homology thereto, located therein.

(40) The biochip according to Item 37, wherein the polypeptide or the variant thereof, has at least three contiguous or non-contiguous amino acid lengths of at least an amino acid sequence selected from the group consisting of SEQ ID NO: 2-341, 343-722, 724-1086, 1088-1468, 1470-1837 and 1839-2157, or a sequence having at least 70 % homology thereto, and having a biological function, located therein.

(41) The biomolecule chip according to Item 40, wherein the biological activity comprises a function set forth in Table 2.

(42) The biomolecule chip according to Item 40, wherein the biological activity comprises epitope activity.

(43) A recording medium having stored therein information of a nucleic acid sequence of a nucleic acid molecule having at least eight contiguous or non-contiguous nucleotide sequences of the sequences set forth in SEQ ID NOs: 1 or 1087, or a variant thereof.

(44) The storing medium according to Item 43 wherein the nucleic acid molecule or the variant thereof comprises at least eight contiguous nucleotide lengths of substantially all the sequences selected from the group consisting of SEQ ID NOs: 2-341, 343-722, 724-1086, 1088-1468, 1470-1837 and 1839-2157, or sequences with one or more amino acid substitution, addition and/or deletion thereto.

(45) The storage medium according to Item 43, wherein when the reading frame of Table 2 is f-1, f-2 or f-3, the nucleic acid molecule or the variant thereof has a sequence from the position of nucleic acid number (sense strand, start) of SEQ ID NO: 1 of Table 2, to the position of nucleic acid number (sense strand, stop) or a sequence having at least 70 % homology thereto, or when the reading frame of Table 2 is r-1, r-2 or r-3, the nucleic acid molecule has a sequence from the position of nucleic acid number (antisense strand, start) of SEQ ID NO: 1087 of Table 2, to the position of nucleic acid number (antisense strand, stop) or a sequence having at least 70 % homology thereto.

(46) A storage medium comprising information of a polpeptide or a variant thereof having at least one amino acid sequence selected from the group consisting of SEQ ID NO: 2-341, 343-722, 724-1086, 1088-1468, 1470-1837 and 1839-2157, or a sequence having at least 70 % homology thereto, located therein.

(47) The storage medium according to Item 46, wherein the polypeptide or the variant thereof has at least three contiguous amino acid lengths of at least one amino acid sequence selected from the group consisting of SEQ ID NO: 2-341, 343-722, 724-1086, 1088-1468, 1470-1837 and 1839-2157, or a sequence having at least 70 % homology thereto, located therein.

(48) The storage medium according to Item 46, wherein the polypeptide or the variant thereof ahs at least eight contiguous amino acid lengths of at least one amino acid sequence selected from the group consisting of SEQ ID NO: 2-341, 343-722, 724-1086, 1088-1468, 1470-1837 and 1839-2157, or a sequence having at least 70 % homology thereto, located therein.

(49) The storage medium according to Item 46, wherein the polypeptide or the variant thereof has at least three contiguous or non-contiguous amino acid lengths of at least one amino acid sequence selected from the group consisting of SEQ ID NO: 2-341, 343-722, 724-1086, 1088-1468, 1470-1837 and 1839-2157, or a sequence having at least 70 % homology thereto, and having a biological function, located therein.

(50) The storage medium according to Item 49, wherein the biological activity comprises a function set forth in Table 2.

(51) A biomolecule chip having at least one antibody against a polypeptide or a variant thereof, located on a substrate, the polypeptide or the variant thereof comprises at least one amino acid sequence of sequences selected from the group consisting of SEQ IDNOs: 2-341, 343-722, 724-1086, 1088-1468, 1470-1837 and 1839-2157, or a sequence having at least 70 % homology thereto.

(52) An RNAi molecule having a sequence homologous to a reading frame sequence wherein, when the reading frame of Table 2 is f-1, f-2 or f-3, the reading frame sequence has a sequence from the position of nucleic acid number (sense strand, start) of SEQ ID NO: 1 of Table 2, to the position of nucleic acid number (sense strand, stop) or a sequence having at least 70 % homology thereto, or when the reading frame of Table 2 is r-1, r-2 or r-3, the reading frame sequence has a sequence from the position of nucleic acid number (antisense strand, start) of SEQ ID NO: 1087 of Table 2, to the position of nucleic acid number (antisense strand, stop) or a sequence having at least 70 % homology thereto.

(53) The RNAi molecule according to Item 52, which is an RNA or a variant thereof comprising a double-stranded portion of at least 10 nucleotides in length.

(54) The RNAi molecule according to Item 52, comprising a 3' overhang terminus.

(55) The RNAi molecule according to Item 54, wherein the 3' overhang terminus is a DNA of at least 2 nucleotides in length.

(56) The RNAi molecule according to Item 54, wherein the 3' overhang terminus is a DNA of two to four nucleotides in length.

The prsent biomolecule chip may be DNA chip, protein chip or the like.

Hereinafter the preferable embodiments of the present invention are described. However, it should be appreciated that those skilled in the art can readily and appropriately carry out such embodiments of the invention from the description of the present invention and the well-known technology and common general knowledge of the art, and readily understand the effects and advantages of the present invention therefrom.

BRIEF DESCRIPTION OF THE DRAWINGS

Figure 1 is a schematic diagram of double-cross over disruption.

Figure 2 is a schematic diagram of linear DNA using double cross-over disruption.

Figure 3 is a schematic diagram of single cross-over disruption.

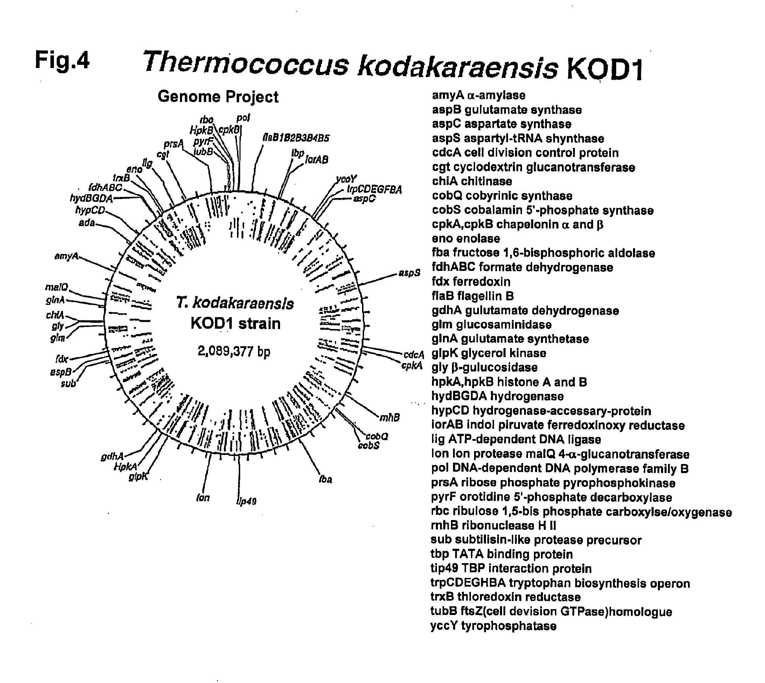

Figure 4 is a diagram showing a genome structure of the present invention.

Figure 5 is another diagram showing a genome structure of the present invention.

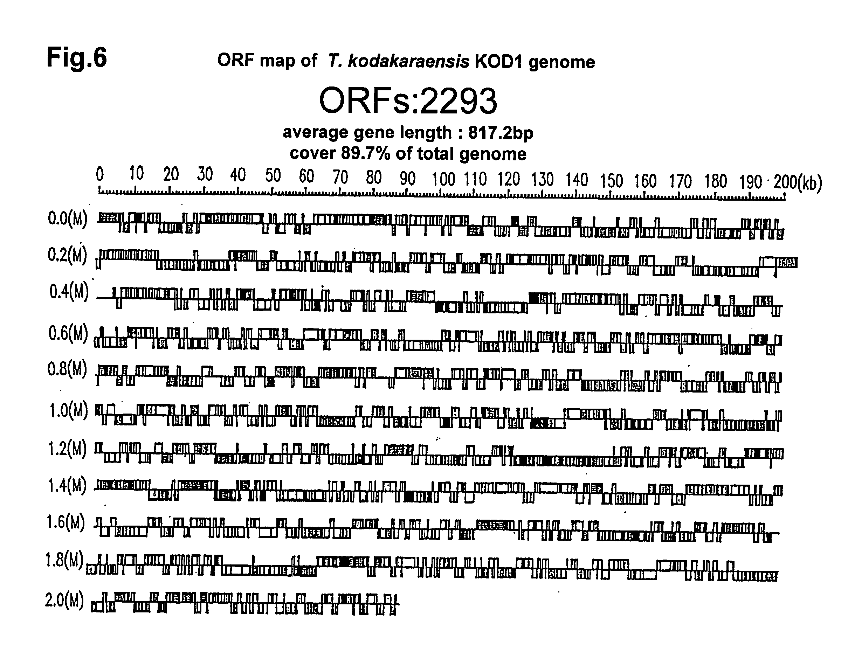

Figure 6 is another diagram showing a genome structure of the present invention.

Figure 7 is an exemplary schematic diagram showing a genomic biomolecule chip.

The description of the sequence listings is set forth in another Table (Table 2).

DETAILED DESCRIPTION OF THE INVENTION

Heterinafter the best modes of the present invention are described. It should be understood throughout the present specification that expression of a singular form includes the concept of their plurality unless otherwise mentioned.

Specifically, articles for a singular form (e.g., "a", "an", "the", etc. in English; "ein", "der", "das", "die", etc. and their inflections in German; "un", "une", "le" "la", etc. in French; "un", "una", "el", "la", etc. in Spanish, and articles, adjectives, etc. in other languages) include the concept of their plurality unless otherwise mentioned. It should be also understood that the terms as used herein have definitions typically used in the art unless otherwise mentioned. Thus, unless otherwise defined, all scientific and technical terms have the same meanings as those generally used by those skilled in the art to which the present invention pertain. If there is contradiction, the present specification (including the definition) precedes.

The embodiments provided hereinafter are provided for better understanding of the present invention, and should be understood that the the scope of the present invention should not be limited to the following description. Accordingly, it is apparent that those skilled in the art can appropriately modify the present invention within the scope thereof upon reading the description of the present specification.

(Definition of Terms)

The definitions of terms used herein are described below.

As used herein the term "organism" is used in the widest sense in the art and refers to a living entity haveing a genome. An organism comprises prokaryotes (for example, E. coli, hyperthermophillic bacteria and the like) and eukaryotes (for example, plants, animals and the like) and the like.

As used herein, the term "genome" refers to a group of genes of a set of chromosomes which is indispensable for supporting living activity of a living organism. In monoploidic organisms such as bacteria, phages, viruses and the like, one DNA or RNA molecule per se is responsible for the genetic information defining these species and is considered the genome. On the other hand, in diploidic organisms such as many eukaryotic organisms, a set of chromosomes (for example, a human has 23 pairs of chromosomes, a mouse has 20 pairs of chromosomes) in a germ cell, and two sets of chromosomes in a somatic cell comprise the genome.

As used herein, the term "gene" refers to an element defining a genetic trait. A gene is typically arranged in a given sequence on a chromosome. A gene which defines the primary structure of a protein is called a structural gene. A gene which regulates the expression of a structural gene is called a regulatory gene. As used herein, the term "gene" may refer to "polynucleotide", "oligonucleotide", "nucleic acid", and "nucleic acid molecule" and/or "protein", "polypeptide", "oligopeptide" and "peptide".

The terms "protein", "polypeptide", "oligopeptide" and "peptide" as used herein have the same meaning and refer to an amino acid polymer having any length. This polymer may be a straight, branched or cyclic chain. An amino acid may be a naturally-occurring or non-naturally-occurring amino acid, or a variant amino acid. The term may include those assembled into a composite or a plurality of polypeptide chains. The term also includes a naturally-occurring or artificially modified amino acid polymer. Such modification includes, for example, disulfide bond formation, glycosylation, lipidation, acetylation, phosphorylation, or any other manipulation or modification (e.g., conjugation with a labeling moiety). This definition encompasses a polypeptide containing at least one amino acid analog (e.g., non-naturally-occurring amino acid, etc.), a peptide-like compound (e.g., peptoid), and other variants known in the art, for example. Gene products comprising a sequence listed in the Sequence Listing usually take a polypeptide form. As used herein, the polypeptide of the present invention has a specific sequence (a sequence set forth in Sequence Listings or a variant thereof). A sequence having a variant may be used for a varitey of purposes, such as diagnostic use, in the present invention.

The terms "polynucleotide", "oligonucleotide", and "nucleic acid" as used herein have the same meaning and refer to a nucleotide polymer having any length. This term also includes an "oligonucleotide derivative" or a "polynucleotide derivative". An "oligonucleotide derivative" or a "polynucleotide derivative" includes a nucleotide derivative, or refers to an oligonucleotide or a polynucleotide having different linkages between nucleotides from typical linkages, which are interchangeably used. Examples of such an oligonucleotide specifically include 2'-O-methyl-ribonucleotide, an oligonucleotide derivative in which a phosphodiester bond in an oligonucleotide is converted to a phosphorothioate bond, an oligonucleotide derivative in which a phosphodiester bond in an oligonucleotide is converted to a N3'-P5' phosphoroamidate bond, an oligonucleotide derivative in which a ribose and a phosphodiester bond in an oligonucleotide are converted to a peptide-nucleic acid bond, an oligonucleotide derivative in which uracil in an oligonucleotide is substituted with C-5 propynyl uracil, an oligonucleotide derivative in which uracil in an oligonucleotide is substituted with C-5 thiazole uracil, an oligonucleotide derivative in which cytosine in an oligonucleotide is substituted with C-5 propynyl cytosine, an oligonucleotide derivative in which cytosine in an oligonucleotide is substituted with phenoxazine-modified cytosine, an oligonucleotide derivative in which ribose in DNA is substituted with 2'-O-propyl ribose, and an oligonucleotide derivative in which ribose in an oligonucleotide is substituted with 2'-methoxyethoxy ribose. Unless otherwise indicated, a particular nucleic acid sequence also implicitly encompasses conservatively-modified variants thereof (e.g. degenerate codon substitutions) and complementary sequences as well as the sequence explicitly indicated. Specifically, degenerate codon substitutions may be produced by generating sequences in which the third position of one or more selected (or all) codons is substituted with mixed-base and/or deoxyinosine residues (Batzer et al., Nucleic Acid Res. 19:5081(1991); Ohtsuka et al., J. Biol. Chem. 260:2605-2608 (1985); Rossolini et al., Mol. Cell. Probes 8:91-98(1994)). The gene of the present invention usually takes this polynucleotide form.

As used herein, the term "nucleic acid molecule" is used interchangeably with "nucleic acid", "oligonucleotide", and "polynucleotide", including cDNA, mRNA, genomic DNA, and the like. As used herein, nucleic acid and nucleic acid molecule may be included by the concept of the term "gene". A nucleic acid molecule encoding the sequence of a given gene includes "splice mutant (variant) ". Similarly, a particular protein encoded by a nucleic acid encompasses any protein encoded by a splice variant of that nucleic acid. "Splice mutants", as the name suggests, are products of alternative splicing of a gene. After transcription, an initial nucleic acid transcript may be spliced such that different (alternative) nucleic acid splice products encode different polypeptides. Mechanisms for the production of splice variants vary, but include alternative splicing of exons. Alternative polypeptides derived from the same nucleic acid by read-through transcription are also encompassed by this definition. Any products of a splicing reaction, including recombinant forms of the splice products, are included in this definition. Such variants are useful for a variety of assays.

As used herein, the term "amino acid" may refer to a naturally-occurring or non-naturally-occurring amino acid as long as the object of the present invention is satisfied.

As used herein, the term "amino acid derivative" or "amino acid analog" refers to an amino acid which is different from a naturally-occurring amino acid and has a function similar to that of the original amino acid. Such amino acid derivatives and amino acid analogs are well known in the art.

The term "naturally-occurring amino acid" refers to an L-isomer of a naturally-occurring amino acid. The naturally-occurring amino acids are glycine, alanine, valine, leucine, isoleucine, serine, methionine, threonine, phenylalanine, tyrosine, tryptophan, cysteine, proline, histidine, aspartic acid, asparagine, glutamic acid, glutamine, y-carboxyglutamic acid, arginine, ornithine, and lysine. Unless otherwise indicated, all amino acids as used herein are L-isomers. An embodiment using a D-isomer of an amino acid falls within the scope of the present invention.

The term "non-naturally-occurring amino acid" refers to an amino acid which is ordinarily not found in nature. Examples of non-naturally-occurring amino acids include D-forms of an amino acid as described above, norleucine, para-nitrophenylalanine, homophenylalanine, para-fluorophenylalanine, 3-amino-2-benzyl propionic acid, D- or L-homoarginine, and D-phenylalanine.

As used herein, the term "amino acid analog" refers to a molecule having a physical property and/or function similar to that of amino acids, but is not an amino acid. Examples of amino acid analogs include, for example, ethionine, canavanine, 2-methylglutamine, and the like. An amino acid mimic refers to a compound which has a structure different from that of the general chemical structure of amino acids but which functions in a manner similar to that of naturally-occurring amino acids.

As used herein, the term "nucleotide" may be either naturally-occurring or non-naturally-occurring. The term "nucleotide derivative" or "nucleotide analog" refers to a nucleotide which is different from naturally-occurring nucleotides and has a function similar to that of the original nucleotide. Such nucleotide derivatives and nucleotide analogs are well known in the art. Examples of such nucleotide derivatives and nucleotide analogs include, but are not limited to, phosphorothioate, phosphoramidate, methylphosphonate, chiral-methylphosphonate, 2-O-methyl ribonucleotide, and peptide-nucleic acid (PNA).

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

As used herein, the term "corresponding" amino acid or nucleic acid refers to an amino acid or nucleotide in a given polypeptide or polynucleotide molecule, which has, or is anticipated to have, a function similar to that of a predetermined amino acid or nucleotide in a polypeptide or polynucleotide as a reference for comparison. Particularly, in the case of enzyme molecules, the term refers to an amino acid which is present at a similar position in an active site and similarly contributes to catalytic activity. For example, in the case of an antisense molecule, a corresponding antisense molecule may be a similar portion in an ortholog corresponding to a particular portion of the antisense molecule.

As used herein, the term "corresponding" gene (e.g., a polypeptide or polynucleotide molecule) refers to a gene in a given species, which has, or is expected to have, a function similar to that of a predetermined gene in a species as a reference for comparison. When there are a plurality of genes having such a function, the term refers to a gene having the same evolutionary origin. Therefore, a gene corresponding to a given gene may be an ortholog of the given gene. Thus, a gene corresponding to each gene can be found in other organisms. Such a corresponding gene can be identified by techniques well known in the art. For example, a corresponding gene in a given organism can be found by searching a sequence database of the organism (e.g., hyperthermophillic bacteria) using the sequence of a reference gene (e.g., gene comprising a sequence set forth in Sequence Listing etc.) as a query sequence.

As used herein, the term "fragment" with respect to a polypeptide or polynucleotide refers to a polypeptide or polynucleotide having a sequence length ranging from 1 to n-1 with respect to the full length of the reference polypeptide or polynucleotide (of length n). The length of the fragment can be appropriately changed depending on the purpose. For example, in the case of polypeptides, the lower limit of the length of the fragment includes 3, 4, 5, 6, 7, 8, 9, 10, 15, 20, 25, 30, 40, 50 or more nucleotides. Lengths represented by integers which are not herein specified (e.g., 11 and the like) may be appropriate as a lower limit. For example, in the case of polynucleotides, the lower limit of the length of the fragment includes 5, 6, 7, 8, 9, 10, 15, 20, 25, 30, 40, 50, 75, 100 or more nucleotides. Lengths represented by integers which are not herein specified (e.g., 11 and the like) may be appropriate as a lower limit. As used herein, the length of polypeptides or polynucleotides can be represented by the number of amino acids or nucleic acids, respectively. However, the above-described numbers are not absolute. The above-described numbers, as the upper or lower limit, are intended to include some greater or smaller numbers (e.g., ±10%), as long as the same function is maintained. For this purpose, "about" may be herein put ahead of the numbers. However, it should be understood that the interpretation of numbers is not affected by the presence or absence of "about" in the present specification.

As used herein, the term "agent specifically interacting with" a biological agent, or "specific agent", such as a polynucleotide, a polypeptide or the like, are used interchangeably and refer to an agent which has an affinity for the biological agent, such as a polynucleotide, a polypeptide or the like, which is representatively higher than or equal to the affinity for other non-related biological agents, such as polynucleotides, polypeptides or the like (particularly, those with identity of less than 30%; in a specific embodiment, less than 99 % identity), and preferably significantly (e.g., statistically significantly) higher. Such affinity may be measured by hybridizatin assay, binding assay and the like. When a biologial agent is a polypeptide, a specific agent to the polypeptide includes a specific antibody, and it should be understood that in a particular embodiment, the specific agents of the present invention may include an agent specific to the specific antibodies. It should be understood that such specific agents to the specific andibodies include the polypeptide of interest *per se.*

As used herein, the "agent" may be any substance or other agent (e.g., energy) as long as the intended purpose can be achieved. Examples of such a substance include, but are not limited to, proteins, polypeptides, oligopeptides, peptides, polynucleotides, oligonucleotides, nucleotides, nucleic acids (e.g., DNA such as cDNA, genomic DNA , or the like, and RNA such as mRNA), polysaccharides, oligosaccharides, lipids, low molecular weight organic molecules (e.g., hormones, ligands, information transfer substances, molecules synthesized by combinatorial chemistry, low molecular weight molecules , and the like (e.g., pharmaceutically acceptable low molecular weight ligands and the like)), and combinations of these molecules. Examples of an agent specific to a polynucleotide include, but are not limited to, a polynucleotide having complementarity to the sequence of the polynucleotide with a predetermined sequence homology (e.g., 70% or more sequence identity), a polypeptide such as a transcriptional agent binding to a promoter region, and the like. Examples of an agent specific to a polypeptide include, but are not limited to, an antibody specifically directed to the polypeptide or derivatives or analogs thereof (e.g., single chain antibody), a specific ligand or receptor when the polypeptide is a receptor or ligand, a substrate when the polypeptide is an enzyme, and the like.

As used herein, the term "low molecular weight organic molecule" refers to an organic molecule having a relatively small molecular weight. Usually, the low molecular weight organic molecule refers to a molecular weight of about 1,000 or less, or may refer to a molecular weight of more than 1,000. Low molecular weight organic molecules can be ordinarily synthesized by methods known in the art or combinations thereof. These low molecular weight organic molecules may be produced by organisms. Examples of the low molecular weight organic molecule include, but are not limited to, hormones, ligands, information transfer substances, molecules synthesized by combinatorial chemistry, pharmaceutically acceptable low molecular weight molecules (e.g., low molecular weight ligands and the like), and the like.

As used herein, the term "antibody" encompasses polyclonal antibodies, monoclonal antibodies, human antibodies, humanized antibodies, polyfunctional antibodies, chimeric antibodies, and anti-idiotype antibodies, and fragments thereof (e.g., F(ab')2 and Fab fragments), and other recombinant conjugates. These antibodies may be fused with an enzyme (e.g., alkaline phosphatase, horseradish peroxidase, α-galactosidase, and the like) via a covalent bond or by recombination.

As used herein, the term "monoclonal antibody" refers to an antibody composition having a group of homologous antibodies. This term is not limited by the production manner thereof. This term encompasses all immunoglobulin molecules and Fab molecules, F(ab')2 fragments, Fv fragments, and other molecules having an immunological binding property of the original monoclonal antibody molecule. Methods for producing polyclonal antibodies and monoclonal antibodies are well known in the art, and will be more sufficiently described below.

Monoclonal antibodies are prepared by using a standard technique well known in the art (e.g., Kohler and Milstein, Nature, 1975, 256: 495) or a modification thereof (e.g., Buck et al., In Vitro, 18, 1982:377). Representatively, a mouse or rat is immunized with a protein bound to a protein carrier, and boosted. Subsequently, the spleen (and optionally several large lymph nodes) is removed and dissociated into single cells. If desired, the spleen cells may be screened (after removal of nonspecifically adherent cells) by applying a cell suspension to a plate or well coated with a protein antigen. B-cells that express membrane-bound immunoglobulin specific for the antigen bind to the plate, and are not rinsed away with the rest of the suspension. Resulting B-cells, or all dissociated spleen cells, are then induced to fuse with myeloma cells to form hybridomas. The hybridomas are used to produce monoclonal antibodies.

As used herein, the term "antigen" refers to any substrate to which an antibody molecule may specifically bind. As used herein, the term "immunogen" refers to an antigen initiating activation of the antigen-specific immune response of a lymphocyte.

As used herein, the term "single chain antibody" refers to a single chain polypeptide formed by linking a heavy chain fragment and the light chain fragment of the Fv region via a peptide crosslinker.

As used herein, the term "composite molecule" refers to a molecule in which a plurality of molecules, such as polypeptides, polynucleotides, lipids, sugars, small molecules, or the like, are linked together. Examples of a composite molecule include, but are not limited to, glycolipids, glycopeptides, and the like. Such composite molecules can be herein used as a DICS1 gene or a product thereof, or an agent of the present invention, as long as they have a similar function to that of the gene or the product thereof, or the agent of the present invention.

As used herein, the term "isolated" biological agent (e.g., nucleic acid, protein, or the like) refers to a biological agent that is substantially separated or purified from other biological agents in cells of a naturally-occurring organism (e.g., in the case of nucleic acids, agents other than nucleic acids and a nucleic acid having nucleic acid sequences other than an intended nucleic acid; and in the case of proteins, agents other than proteins and proteins having an amino acid sequence other than an intended protein). The "isolated" nucleic acids and proteins include nucleic acids and proteins purified by a standard purification method. The isolated nucleic acids and proteins also include chemically synthesized nucleic acids and proteins.

As used herein, the term "purified" biological agent (e.g., nucleic acids, proteins, and the like) refers to one from which at least a part of the naturally accompanying agents are removed. Therefore, ordinarily, the purity of a purified biological agent is higher than that of the biological agent in a normal state (i.e., concentrated).

As used herein, the terms "purified" and "isolated" mean that the same type of biological agent is present preferably at least 75% by weight, more preferably at least 85% by weight, even more preferably at least 95% by weight, and most preferably at least 98% by weight.

As used herein, the term "expression" of a gene, a polynucleotide, a polypeptide, or the like, indicates that the gene or the like is affected by a predetermined action *in vivo* to be changed into another form. Preferably, the term "expression" indicates that genes, polynucleotides, or the like are transcribed and translated into polypeptides. In one embodiment of the present invention, genes may be transcribed into mRNA. More preferably, these polypeptides may have post-translational processing modifications.

Therefore, as used herein, the term "reduction" of "expression" of a gene, a polynucleotide, a polypeptide, or the like indicates that the level of expression is significantly reduced in the presence of or under the action of the agent of the present invention as compared to when the action of the agent is absent. Preferably, the reduction of expression includes a reduction in the amount of expression of a polypeptide. As used herein, the term "increase" of "expression" of a gene, a polynucleotide, a polypeptide, or the like indicates that the level of expression is significantly increased in the presence of the action of the agent of the present invention as compared to when the action of the agent is absent. Preferably, the increase of expression includes an increase in the amount of expression of a polypeptide. As used herein, the term "induction" of "expression" of a gene indicates that the amount of expression of the gene is increased by applying a given agent to a given cell. Therefore, the induction of expression includes allowing a gene to be expressed when expression of the gene is not otherwise observed, and increasing the amount of expression of the gene when expression of the gene is observed.

As used herein, the term "specifically expressed" in relation to a gene indicates that the gene is expressed in a specific site or for a specific period of time, at a level different from (preferably higher than) that in other sites or for other periods of time. The term "specifically expressed" indicates that a gene may be expressed only in a given site (specific site) or may be expressed in other sites. Preferably, the term "specifically expressed" indicates that a gene is expressed only in a given site.

As used herein, the term "biological activity" refers to activity possessed by an agent (e.g., a polynucleotide, a protein, etc.) within an organism, including activities exhibiting various functions (e.g., transcription promoting activity, etc.). For example, when two agents interact with each other (the gene product of the present invention and the receptor therefor), the biological activity thereof includes the binding of the gene product of the present invention and the receptor therefor and a biological change (e.g., apoptosis) caused thereby. In another example, when a certain factor is an enzyme, the biological activity thereof includes its enzyme activity. In still another example, when a certain factor is a ligand, the biological activity thereof includes the binding of the ligand to a receptor corresponding thereto. The above-described biological activity can be measured by techniques well-known in the art. Alternatively, in the present invention, the cases of a modified molecule having similar activity in the living organism may be included in the definition of having biological activity.

As used herein, the term "antisense (activity) " refers to activity which permits specific suppression or reduction of expression of a target gene. The antisense activity is ordinarily achieved by a nucleic acid sequence having a length of at least 8 contiguous nucleotides, which is complementary to the nucleic acid sequence of a target gene (e.g., genes of the present invention, etc.). A molecule having such antisense activity is called an antisense molecule. Such a nucleic acid sequence preferably has a length of at least 9 contiguous nucleotides, more preferably a length of at least 10 contiguous nucleotides, and even more preferably a length of at least 11 contiguous nucleotides, a length of at least 12 contiguous nucleotides, a length of at least 13 contiguous nucleotides, a length of at least 14 contiguous nucleotides, a length of at least 15 contiguous nucleotides, a length of at least 20 contiguous nucleotides, a length of at least 30 contiguous nucleotides, a length of at least 40 contiguous nucleotides, and a length of at least 50 contiguous nucleotides. These nucleic acid sequences include nucleic acid sequences having at least 70% homology thereto, more preferably at least 80%, even more preferably at least 90%, and still even more preferably at least 95%. The antisense activity is preferably complementary to a 5' terminal sequence of the nucleic acid sequence of a target gene. Such an antisense nucleic acid sequence includes the above-described sequences having one or several, or at least one, nucleotide substitutions, additions, and/or deletions.

As used herein, the term "RNAi" is an abbreviation of RNA interference and refers to a phenomenon where an agent for causing RNAi, such as double-stranded RNA (also called dsRNA), is introduced into cells and mRNA homologous thereto is specifically degraded, so that synthesis of gene products is suppressed, and also referes to a technique using the phenomenon. As used herein, RNAi may have the same meaning as that of an agent which causes RNAi.

As used herein, the term "an agent causing RNAi" refers to any agent causing RNAi. As used herein, "an agent causing RNAi for a gene" indicates that the agent causes RNAi relating to the gene and the effect of RNAi is achieved (e.g., suppression of expression of the gene, and the like). Examples of such an agent causing RNAi include, but are not limited to, a sequence having at least about 70% homology to the nucleic acid sequence of a target gene or a sequence hybridizable under stringent conditions, RNA containing a double-stranded portion having a length of at least 10 nucleotides or variants thereof. Herein, this agent may be preferably DNA containing a 3' protruding end, and more preferably the 3' protruding end has a length of 2 or more nucleotides (e.g., 2-4 nucleotides in length).

Though not wishing to be bound by any theory, a mechanism which causes RNAi is considered as follows. When a molecule which causes RNAi, such as dsRNA, is introduced into a cell, an RNase III-like nuclease having a helicase domain (called dicer) cleaves the molecule on about a 20 base pair basis from the 3' terminus in the presence of ATP in the case where the RNA is relatively long (e.g., 40 or more base pairs). As used herein, the term "siRNA" is an abbreviation of short interfering RNA and refers to short double-stranded RNA of 10 or more base pairs which are artificially chemically or biochemically synthesized, synthesized in the organism body, or produced by double-stranded RNA of about 40 or more base pairs being degraded within the body. siRNA typically has a structure having 5'-phosphate and 3'-OH, where the 3' terminus projects by about 2 bases. A specific protein is bound to siRNA to form RISC (RNA-induced-silencing-complex). This complex recognizes and binds to mRNA having the same sequence as that of siRNA and cleaves mRNA at the middle of siRNA due to RNase III-like enzymatic activity. It is preferable that the relationship between the sequence of siRNA and the sequence of mRNA to be cleaved as a target is a 100% match. However, base mutation at a site away from the middle of siRNA does not completely remove the cleavage activity by RNAi, leaving partial activity, while base mutation in the middle of siRNA has a large influence and the mRNA cleavage activity by RNAi is considerably lowered. By utilizing this nature, mRNA having a mutation can be specifically degraded. Specifically, siRNA in which the mutation is provided in the middle thereof is synthesized and is introduced into a cell. Therefore, in the present invention, siRNA per se as well as an agent capable of producing siRNA (e.g., representatively dsRNA of about 40 or more base pairs) can be used as an agent capable of eliciting RNAi.

Also, though not wishing to be bound by any theory, apart from the above-described pathway, the antisense strand of siRNA binds to mRNA and siRNA functions as a primer for RNA-dependent RNA polymerase (RdRP), so that dsRNA is synthesized. This dsRNA is a substrate for a dicer again, leading to production of new siRNA. It is intended that such an action is amplified. Therefore, in the present invention, siRNA *per se* as well as an agent capable of producing siRNA, are useful. In fact, in insects and the like, for example, 35 dsRNA molecules can substantially completely degrade 1000 or more copies of intracellular mRNA, and therefore, it will be understood that siRNA *per se,* as well as an agent capable of producing siRNA, is useful.

In the present invention, double-stranded RNA having a length of about 20 bases (e.g., representatively about 21 to 23 bases) or less than about 20 bases, which is called siRNA, can be used. Expression of siRNA in cells can suppress expression of a pathogenic gene targeted by the siRNA. Therefore, siRNA can be used for treatment of diseases as a prophylaxis, prognosis, and the like.

The siRNA of the present invention may be in any form as long as it can elicit RNAi.

In another embodiment, an agent capable of causing RNAi may have a short hairpin structure having a sticky portion at the 3' terminus (shRNA; short hairpin RNA). As used herein, the term '"shRNA" refers to a molecule of about 20 or more base pairs in which a single-standed RNA partially contains a palindromic base sequence and forms a double-strand structure therein (i.e. , a hairpin structure). shRNA can be artificially synthesized chemically. Alternatively, shRNA can be produced by linking sense and antisense strands of a DNA sequence in reverse directions and synthesizing RNA *in vitro* with T7 RNA polymerase using the DNA as a template. Though not wishing to be bound by any theory, it should be understood that after shRNA is introduced into a cell, the shRNA is degraded in the cell into a length of about 20 bases (e.g., representatively 21, 22, 23 bases), and causes RNAi as with siRNA, leading to the treatment effect of the present invention. It should be understood that such an effect is exhibited in a wide range of organisms, such as insects, plants, animals (including mammals), and the like. Thus, shRNA elicits RNAi as with siRNA and therefore can be used as an effective component of the present invention. shRNA may preferably have a 3' protruding end. The length of the double-stranded portion is not particularly limited, but is preferably about 10 or more nucleotides, and more preferably about 20 or more nucleotides. Here, the 3' protruding end may be preferably DNA, more preferably DNA of at least 2 nucleotides in length, and even more preferably DNA of 2-4 nucleotides in length.

An agent capable of causing RNAi used in the present invention may be artificially synthesized (chemically or biochemically) or naturally occurring. There is substantially no difference therebetween in terms of the effect of the present invention. A chemically synthesized agent is preferably purified by liquid chromatography or the like.

An agent capable of causing RNAi used in the present invention can be produced *in vitro.* In this synthesis system, T7 RNA polymerase and T7 promoter are used to synthesize antisense and sense RNAs from template DNA. These RNAs are annealed and thereafter are introduced into a cell. In this case, RNAi is caused via the above-described mechanism, thereby achieving the effect of the present invention. Here, for example, the introduction of RNA into cell can be carried out by a calcium phosphate method.

Another example of an agent capable of causing RNAi according to the present invention is a single-stranded nucleic acid hybridizable to mRNA or all nucleic acid analogs thereof. Such agents are useful for the method and composition of the present invention.

As used herein, "polynucleotides hybridizing under stringent conditions" refers to conditions commonly used and well known in the art. Such a polynucleotide can be obtained by conducting colony hybridization, plaque hybridization, Southern blot hybridization, or the like using a polynucleotide selected from the polynucleotides of the present invention. Specifically, a filter on which DNA derived from a colony or plaque is immobilized is used to conduct hybridization at 65°C in the presence of 0.7 to 1.0 M NaCl. Thereafter, a 0.1 to 2-fold concentration SSC (saline-sodium citrate) solution (1-fold concentration SSC solution is composed of 150 mM sodium chloride and 15 mM sodium citrate) is used to wash the filter at 65°C. Polynucleotides identified by this method are referred to as "polynucleotides hybridizing under stringent conditions". Hybridization can be conducted in accordance with a method described in, for example, Molecular Cloning 2nd ed., Current Protocols in Molecular Biology, Supplement 1-38, DNA Cloning 1: Core Techniques, A Practical Approach, Second Edition, Oxford University Press (1995), and the like. Here, sequences hybridizing under stringent conditions exclude, preferably, sequences containing only A or T. "Hybridizable polynucleotide" refers to a polynucleotide which can hybridize other polynucleotides under the above-described hybridization conditions. Specifically, the hybridizable polynucleotide includes at least a polynucleotide having a homology of at least 60% to the base sequence of DNA encoding a polypeptide having an amino acid sequence specifically herein disclosed, preferably a polynucleotide having a homology of at least 80%, and more preferably a polynucleotide having a homology of at least 95%.

The term "highly stringent conditions" refers to those conditions that are designed to permit hybridization of DNA strands whose sequences are highly complementary, and to exclude hybridization of significantly mismatched DNAs. Hybridization stringency is principally determined by temperature, ionic strength, and the concentration of denaturing agents such as formamide. Examples of "highly stringent conditions" for hybridization and washing are 0.0015 M sodium chloride, 0.0015 M sodium citrate at 65-68°C or 0.015 M sodium chloride, 0.0015 M sodium citrate, and 50% formamide at 42°C. See Sambrook, Fritsch & Maniatis, Molecular Cloning: A Laboratory Manual (2nd ed. , Cold Spring Harbor Laboratory, N.Y., 1989); Anderson et al., Nucleic Acid Hybridization: A Practical Approach Ch. 4 (IRL Press Limited) (Oxford Express). More stringent conditions (such as higher temperature, lower ionic strength, higher formamide, or other denaturing agents) may be optionally used. Other agents may be included in the hybridization and washing buffers for the purpose of reducing non-specific and/or background hybridization. Examples are 0.1% bovine serum albumin, 0.1% polyvinylpyrrolidone, 0.1% sodium pyrophosphate, 0.1% sodium dodecylsulfate (NaDodSO$_4$ or SDS), Ficoll, Denhardt' s solution, sonicated salmon sperm DNA (or another noncomplementary DNA), and dextran sulfate, although other suitable agents can also be used. The concentration and types of these additives can be changed without substantially affecting the stringency of the hybridization conditions. Hybridization experiments are ordinarily carried out at pH 6.8-7.4; however, at typical ionic strength conditions, the rate of hybridization is nearly independent of pH. See Anderson et al., Nucleic Acid Hybridization: A Practical Approach Ch. 4 (IRL Press Limited, Oxford UK).

Factors affecting the stability of DNA duplex include base composition, length, and degree of base pair mismatch. Hybridization conditions can be adjusted by those skilled in the art in order to accommodate these variables and allow DNAs of different sequence relatedness to form hybrids. The melting temperature of a perfectly matched DNA duplex can be estimated by the following equation:

$$Tm\ (°C) = 81.5 + 16.6\ (\log[Na^+]) + 0.41\ (\%\ G{+}C) - 600/N - 0.72\ (\%\ \text{formamide})$$

where N is the length of the duplex formed, [Na$^+$] is the molar concentration of the sodium ion in the hybridization or washing solution, % G+C is the percentage of (guanine+cytosine) bases in the hybrid. For imperfectly matched hybrids, the melting temperature is reduced by approximately 1°C for each 1% mismatch.

The term "moderately stringent conditions" refers to conditions under which a DNA duplex with a greater degree of base pair mismatching than could occur under "highly stringent conditions" is able to form. Examples of typical "moderately stringent conditions" are 0.015 M sodium chloride, 0.0015 M sodium citrate at 50-65°C or 0.015 M sodium chloride, 0.0015 M sodium citrate, and 20% formamide at 37-50°C. By way of example, "moderately stringent conditions" of 50°C in 0.015 M sodium ion will allow about a 21% mismatch.

It will be appreciated by those skilled in the art that there may be no absolute distinction between "highly stringent conditions" and "moderately stringent conditions". For example, at 0.015 M sodium ion (no formamide), the melting temperature of perfectly matched long DNA is about 71°C. With a wash at 65°C (at the same ionic strength), this would allow for approximately a 6% mismatch. To capture more distantly related sequences, those skilled in the art can simply lower the temperature or raise the ionic strength.

A good estimate of the melting temperature in 1 M NaCl for oligonucleotide probes up to about 20 nucleotides is given by:

$$Tm = (2°C \text{ per A-T base pair}) + (4°C \text{ per G-C base pair}).$$

Note that the sodium ion concentration in 6X salt sodium citrate (SSC) is 1 M. See Suggs et al., Developmental Biology Using Purified Genes 683 (Brown and Fox, eds., 1981).

A naturally-occurring nucleic acid encoding a protein (e.g., Pep5, p75, Rho GDI, MAG, p21, Rho, Rho kinase, or variants or fragments thereof, or the like) may be readily isolated from a cDNA library having PCR primers and hybridization probes containing part of a nucleic acid sequence indicated in the sequence listing. A preferable nucleic acid, or variants or fragments thereof, or the like is hybridizable to the whole or part of a sequence as set forth in SEQ ID NO: 1 or 1087 under low stringent conditions defined by hybridization buffer essentially containing 1% bovine serum alubumin (BSA); 500 mM sodium phosphate ($NaPO_4$); 1mM EDTA; and 7% SDS at 42°C, and wash buffer essentially containing 2xSSC (600 mM NaCl; 60 mM sodium citrate); and 0.1% SDS at 50°C, more preferably under low stringent conditions defined by hybridization buffer essentially containing 1% bovine serum alubumin (BSA); 500 mM sodium phosphate ($NaPO_4$); 15% formamide; 1 mM EDTA; and 7% SDS at 50°C, and wash buffer essentially containing 1xSSC (300 mM NaCl; 30 mM sodium citrate); and 1% SDS at 50°C, and most preferably under low stringent conditions defined by hybridization buffer essentially containing 1% bovine serum alubumin (BSA); 200 mM sodium phosphate ($NaPO_4$); 15% formamide; 1 mM EDTA; and 7% SDS at 50°C, and wash buffer essentially containing 0.5×SSC (150 mM NaCl; 15 mM sodium citrate); and 0.1% SDS at 65°C.

As used herein, the term "probe" refers to a substance for use in searching, which is used in a biological experiment, such as *in vitro* and/or *in vivo* screening or the like, including, but not being limited to, for example, a nucleic acid molecule having a specific base sequence or a peptide containing a specific amino acid sequence.

Examples of a nucleic acid molecule as a usual probe include one having a nucleic acid sequence having a length of at least 8 contiguous nucleotides, which is homologous or complementary to the nucleic acid sequence of a gene of interest. Such a nucleic acid sequence may be preferably a nucleic acid sequence having a length of at least 9 contiguous nucleotides, more preferably a length of at least 10 contiguous nucleotides, and even more preferably a length of at least 11 contiguous nucleotides, a length of 12 contiguous nucleotides, a length of at least 13 contiguous nucleotides, a length of at least 14 contiguous nucleotides, a length of at least 15 contiguous nucleotides, a length of at least 20 contiguous nucleotides, a length of at least 25 contiguous nucleotides, a length of 30 contiguous nucleotides, a length of at least 40 contiguous nucleotides, or a length of at least 50 contiguous nucleotides. A nucleic acid sequence used as a probe includes a nucleic acid sequence having at least 70% homology to the above-described sequence, more preferably at least 80%, and even more preferably at least 90%, or at least 95%.

As used herein, the term "search" indicates that a given nucleic acid base sequence is utilized to find other nucleic acid base sequences having a specific function and/or property electronically or biologically, or other methods. Examples of electronic search include, but are not limited to, BLAST (Altschul et al., J. Mol. Biol. 215:403-410 (1990)), FASTA (Pearson & Lipman, Proc. Natl. Acad. Sci., USA 85:2444-2448 (1988)), Smith and Waterman method (Smith and Waterman, J. Mol. Biol. 147:195-197 (1981)), and Needleman and Wunsch method (Needleman and Wunsch, J. Mol. Biol. 48:443-453 (1970)), and the like. Examples of biological search include, but are not limited to, a macroarray in which genomic DNA is attached to a nylon membrane or the like or a microarray (microassay) in which genomic DNA is attached to a glass plate under stringent hybridization, PCR and in situ hybridization, and the like. It is herein intended that the genes used in the present invention include corresponding genes identified by such an electronic or biological search.

As used herein, the term "primer" refers to a substance required for initiation of a reaction of a macromolecule compound to be synthesized in a macromolecule synthesis enzymatic reaction. In a reaction for synthesizing a nucleic acid molecule, a nucleic acid molecule (e.g., DNA, RNA, or the like) which is complementary to part of a macromolecule compound to be synthesized may be used.

A nucleic acid molecule which is ordinarily used as a primer includes one that has a nucleic acid sequence having a length of at least 8 contiguous nucleotides, which is complementary to the nucleic acid sequence of a gene of interest. Such a nucleic acid sequence preferably has a length of at least 9 contiguous nucleotides, more preferably a length of at least 10 contiguous nucleotides, even more preferably a length of at least 11 contiguous nucleotides, a length of at least 12 contiguous nucleotides, a length of at least 13 contiguous nucleotides, a length of at least 14 contiguous nucleotides, a length of at least 15 contiguous nucleotides, a length of at least 16 contiguous nucleotides, a length of at least 17 contiguous nucleotides, a length of at least 18 contiguous nucleotides, a length of at least 19 contiguous nucleotides, a length of at least 20 contiguous nucleotides, a length of at least 25 contiguous nucleotides, a length of at least 30 contiguous nucleotides, a length of at least 40 contiguous nucleotides, and a length of at least 50 contiguous nucleotides. A nucleic acid sequence used as a primer includes a nucleic acid sequence having at least 70% homology to the above-described sequence, more preferably at least 80%, even more preferably at least 90%, and at least 95%. An appropriate sequence as a primer may vary depending on the property of a sequence to be synthesized (amplified). Those skilled in the art can design an appropriate primer depending on a sequence of interest. Such a primer design is well known in the art and may be performed manually or using a computer program (e. g. , LASERGENE, Primer Select, DNAStar).

As used herein, the term "epitope" refers to a basic structure constituting an antigenic determinant. Therefore, the term "epitope" includes a set of amino acid residues which is involved in recognition by a particular immunoglobulin, or in the context of T cells, those residues necessary for recognition by T cell receptor proteins and/or Major Histocompatibility Complex (MHC) receptors. This term is also used interchangeably with "antigenic determinant" or "antigenic determinant site". In the field of immunology, in vivo or *in vitro,* an epitope is the features of a molecule (e. g. , primary, secondary and tertiary peptide structure, and charge) that form a site recognized by an immunoglobulin, T cell receptor or HLA molecule. An epitope including a peptide comprises 3 or more amino acids in a spatial conformation which is unique to the epitope. Generally, an epitope consists of at least 5 such amino acids, and more ordinarily, consists of at least 6, 7, 8, 9 or 10 such amino acids. The greater the length of an epitope, the more the similarity of the epitope to the original peptide, i.e., longer epitopes are generally preferable. This is not necessarily the case when the conformation is taken into account. Methods of determining the spatial conformation of amino acids are known in the art, and include, for example, X-ray crystallography and 2-dimensional nuclear magnetic resonance spectroscopy. Furthermore, the identification of epitopes in a given protein is readily accomplished using techniques well known in the art. See, also, Geysen et al. , Proc. Natl. Acad. Sci. USA (1984) 81: 3998 (general method of rapidly synthesizing peptides to determine the location of immunogenic epitopes in a given antigen); U. S. Patent No. 4,708,871 (procedures for identifying and chemically synthesizing epitopes of antigens); and Geysen et al., Molecular Immunology (1986) 23: 709 (technique for identifying peptides with high affinity for a given antibody). Antibodies that recognize the same epitope can be identified in a simple immunoassay. Thus, methods for determining epitopes including a peptide are well known in the art. Such an epitope can be determined using a well-known, common technique by those skilled in the art if the primary nucleic acid or amino acid sequence of the epitope is provided.

Therefore, an epitope including a peptide requires a sequence having a length of at least 3 amino acids, preferably at least 4 amino acids, more preferably at least 5 amino acids, at least 6 amino acids, at least 7 amino acids, at least 8 amino acids, at least 9 amino acids, at least 10 amino acids, at least 15 amino acids, at least 20 amino acids, and 25 amino acids. Epitopes may be linear or conformational.

As used herein, "homology" of a gene (e.g., a nucleic acid sequence, an amino acid sequence, or the like) refers to the proportion of identity between two or more gene sequences. As used herein, the identity of a sequence (a nucleic acid sequence, an amino acid sequence, or the like) refers to the proportion of the identical sequence (an individual nucleic acid, amino acid, or the like) between two or more comparable sequences. Therefore, the greater the homology between two given genes, the greater the identity or similarity between their sequences. Whether or not two genes have homology is determined by comparing their sequences directly or by a hybridization method under stringent conditions. When two gene sequences are directly compared with each other, these genes have homology if the DNA sequences of the genes have representatively at least 50% identity, preferably at least 70% identity, more preferably at least 80%, 90%, 95%, 96%, 97%, 98%, or 99% identity with each other.

The similarity, identity and homology of base sequences are herein compared using BLAST (sequence analyzing tool) with the default parameters. The similarity, identity and homology of amino acid sequences are herein compared using BLASTX (sequence analyzing tool) with the default parameters.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

As used herein, the "percentage of (amino acid, nucleotide, or the like) sequence identity, homology or similarity" is determined by comparing two optimally aligned sequences over a window of comparison, wherein the portion of a polynucleotide or polypeptide sequence in the comparison window may comprise additions or deletions (i.e. gaps), as compared to the reference sequences (which does not comprise additions or deletions (if the other sequence includes an addition, a gap may occur)) for optimal alignment of the two sequences. The percentage is calculated by determining the number of positions at which the identical nucleic acid bases or amino acid residues occur in both sequences to yield the number of matched positions, dividing the number of matched positions by the total number of positions in the reference sequence (i.e. the window size) and multiplying the results by 100 to yield the percentage of sequence identity. When used in a search, homology is evaluated by an appropriate technique selected from various sequence comparison algorithms and programs well known in the art. Examples of such algorithms and programs include, but are not limited to, TBLASTN, BLASTP, FASTA, TFASTA and CLUSTALW (Pearson and Lipman, 1988, Proc. Natl. Acad. Sci. USA 85 (8):2444-2448, Altschul et al., 1990, J. Mol. Biol. 215(3):403-410, Thompson et al., 1994, Nucleic Acids Res. 22(2): 4673-4680, Higgins et al., 1996, Methods Enzymol. 266:383-402, Altschul et al., 1990, J. Mol. Biol. 215(3):403-410, Altschul et al., 1993, Nature Genetics 3:266-272). In a particularly preferable embodiment, the homology of a protein or nucleic acid sequence is evaluated using a Basic Local Alignment Search Tool (BLAST) well known in the art (e.g., see Karlin and Altschul, 1990, Proc. Natl. Acad. Sci. USA 87:2267-2268, Altschul et al., 1990, J. Mol. Biol. 215:403-410, Altschul et al., 1993, Nature Genetics 3:266-272, Altschul et al., 1997, Nuc. Acids Res. 25:3389-3402). Particularly, 5 specialized-BLAST programs may be used to perform the following tasks to achieve comparison or search:

(1) comparison of an amino acid query sequence with a protein sequence database using BLASTP and BLAST3;
(2) comparison of a nucleotide query sequence with a nucleotide sequence database using BLASTN;
(3) comparison of a conceptually translated product in which a nucleotide query sequence (both strands) is converted over 6 reading frames with a protein sequence database using BLASTX;
(4) comparison of all protein query sequences converted over 6 reading frames (both strands) with a nucleotide sequence database using TBLASTN; and
(5) comparison of nucleotide query sequences converted over 6 reading frames with a nucleotide sequence database using TBLASTX.

The BLAST program identifies homologous sequences by specifying analogous segments called "high score segment pairs" between amino acid query sequences or nucleic acid query sequences and test sequences obtained from preferably a protein sequence database or a nucleic acid sequence database. A large number of the high score segment pairs are preferably identified (aligned) using a scoring matrix well known in the art. Preferably, the scoring matrix is the BLOSUM62 matrix (Gonnet et al., 1992, Science 256:1443-1445, Henikoff and Henikoff, 1993, Proteins 17:49-61). The PAM or PAM250 matrix may be used, although they are not as preferable as the BLOSUM62 matrix (e.g., see Schwartz and Dayhoff, eds., 1978, Matrices for Detecting Distance Relationships: Atlas of Protein Sequence and Structure, Washington: National Biomedical Research Foundation). The BLAST program evaluates the statistical significance of all identified high score segment pairs and preferably selects segments which satisfy a threshold level of significance independently defined by a user, such as a user set homology. Preferably, the statistical significance of high score segment pairs is evaluated using Karlin's formula (see Karlin and Altschul, 1990, Proc. Natl. Acad. Sci. USA 87:2267-2268).

As used hererin, a sequence is "homologous" refers to that the homology thereof is so high that homologous recombination occurs. Accordingly, those skilled in the art can determine whether a sequence is "homologous" by introducing a DNA capable of completing a variation in a chromosome, and causing *in vivo* gene recombination. There is a method for confirming such a homologous state by determining incorporation of a DNA capable of complementation by a phenotype thereof (for example, if a green fluorescence protein is used, green fluorescence is used). Accordingly, in order that a sequence be homologous, homology between two sequences may be typically at least about 70 %, preferably at least about 80 %, more preferably at least about 90 %, still more preferably at least about 95 %, and most preferably, at least about 99%.

As used herein the term "region" of a sequence, is a portion having a certain length in the sequence. Such a region usually has a function. When used for targeting disruption of the present invention, the "region" of a sequence, is at least about 10 nucleotides in length, preferably at least about 15 nucleotides in length, more preferably at least about 20 nucleotides in length, still more preferably at least about 30 nucleotides in length, yet more preferably at least about 50 nucleotides in length. Preferably, such a region may include a portion responsible for genetic function. In a preferable embodiment, the "region" of a sequence may be one or more genes.

As used herein the term "targeting" refers to to target a certain gene when used in the targeting disruption of a gene.

As used herein the term "biological activity" refers to an activity which an agent (for example, a polypeptide or protein) may have in the living body, and includes those attaining a variety of functions. For example, when an agent is an enzyme, the biological activity thereof includes the enzymatic activity thereof. In another example, when an agent is a ligand, the binding thereof to the receptor therefor is included. In the present invention, each gene product has the biological activities described in Table 2. Alternatively, the polypeptide of the present invention has an epitope activity.

As used herein the term "marker gene" refers to a gene used as a label (or marker) in genetic analysis. Typically, marker genes are those having a clear variant phenotype and are easily detectable rather than having a detailed function. In addition to genes for drug resistance, genes of biochemical property (such as auxotrophic) are often used in microorganism. Genes for morphological properties may also be used. Drug resistance genes include, but are not limited to, for example, kanamycin resistance gene, hygromycin resistance gene, ampicillin resistance gene, chloramphenicol resistance gene, streptomycin resistance gene, and the like.

As used herein the term "vector" refers to one which can transfer a polynucleotide of interest into a cell of interest. Such a vector includes, but is not limited to, for example, one which allows autonomous replication in a host cell such as a prokaryotic cell, yeast cell, animal cell, plant cell, insect cell, animal individual or plant individual or the like, or one which can be incorporated into the chromosome, and comprises a promoter at an appropriate position for trascription of the polynucleotide of the present invention. Preferably, such a vector includes one which can autonomously replicate in Thermococcus kodakarensis KOD1.

As used herein the term "expression vector" refers to a nucleic acid sequence which comprises a structural gene and a promoter regulating the expression thereof, and a number of regulatory elements operably linked in the host cell. Preferably, regulatory elements may comprise a terminator, a selective marker such as a drug resistance gene (for example, kanamycin resistance gene, hygromycin resistance gene and the like), and an enhancer. It is well known in the art that the types of expression vectors used in an organism (for example, plant), and the regulatory elements used may vary depending on the host cell used. In a plant, plant expression vectors used in the present invention may further have a T-DNA region. The T-DNA region enhances the efficiency of introduction of a gene when, in particular, Agrobacterium is used to transform the plant.

As used herein the term "recombinant vector" refers to a vector which can transfer a polynucleotide of interest into a cell of interest. Such a vector includes, but is not limited to, for example, one which allows autonomous replication in a host cell such as a prokaryotic cell, yeast cell, animal cell, plant cell, insect cell, animal individual or plant individual or the like, or one which can be incorporated into the chromosome, and comprises a promoter at an appropriate position for trascription of the polynucleotide of the present invention.

"Recombinant vectors" for prokaryotic cells include pBTrp2, pBTac1, pBTac2 (both available from Roche Molecular Biochemicals), pKK233-2(Pharmacia), pSE280 (Invitrogen), pGEMEX-1 (Promega), pQE-8 (QIAGEN), pKYP10 (Japanese Laid-Open Publication No.: 58-110600), pKYP200 (Agric.Biol.Chem.,48,669(1984)), pLSA1 (Agric.Biol.Chem., 53,277(1989)), pGEL1 (Proc.Natl.Acad.Sci.USA,82,4306 (1985)), pBluescript II SK+(Stratagene), pBluescript II SK(-) (Stratagene), pTrs30 (FERM BP-5407), pTrs32 (FERM BP-5408), pGHA2 (FERM BP-400), pGKA2 (FERM B-6798), pTerm2(Japanese Laid-Open Publication No.: 3-22979, US4686191, US4939094, US5160735), pEG400 (J. Bacteriol., 172, 2392 (1990)), pGEX (Pharmacia), pET systems (Novagen), pSupex, pUB110, pTP5, pC194, pTrxFus (Invitrogen), pMAL-c2 (New England Biolabs), pUC19 (Gene,33,103(1985)), pSTV28 (TaKaRa), pUC118 (TaKaRa), pPA1 (Japanese Laid-Open Publication No.: 63-233798), and the like.

As used herein, the term "promoter" refers to a base sequence which determines the initiation site of transcription of a gene and is a DNA region which directly regulates the frequency of transcription. Transcription is started by RNA polymerase binding to a promoter. A promoter region is usually located within about 2 kbp upstream of the first exon of a putative protein coding region. Therefore, it is possible to estimate a promoter region by predicting a protein coding region in a genomic base sequence using DNA analysis software. A putative promoter region is usually located upstream of a structural gene, but depending on the structural gene, a putative promoter region may be located downstream of a structural gene. Preferably, a putative promoter region is located within about 2 kbp upstream of the translation initiation site of the first exon, but such a putative promoter region is not limited to this and may be located in an intron or downstream of 3' terminus.

As used herein, the term "terminator" refers to a sequence which is located downstream of a protein-encoding region of a gene and which is involved in the termination of transcription when DNA is transcribed into mRNA, and the addition of a poly-A sequence.

When using the present invention, any method for introducing a nucleic acid into a cell may be used as methods for introducing a vector, and includes, for example, transfection, transduction, transformation (calcium chloride method, electroporation method (Japanese Laid-Open Publication 60-251887), particle gun (gene gun) method (Japanese Patent Nos. 2606856, and 2517813)

As used herein, the term "transformant" refers to the whole or a part of an organism, such as a cell, which is produced by transformation. Examples of a transformant include prokaryotic cells, yeast cells, animal cells, plant cells, insect cells and the like. Transformants may be referred to as transformed cells, transformed tissue, transformed hosts, or the like, depending on the subject. As used herein, all of the forms are encompassed, however, a particular form may be specified in a particular context.

As used herein the term "homologous recombination" refers to a recombination in the portion having a homologous base sequence in a pair of double stranded DNA. In a living organism, such homologous recombinations are observed in a form of chromosomal crossover and the like.

As used herein the phrase "conditions under which homologous recombination occurs" refers to conditions under which homologous recombination occurs when an organism having a genome and a nucleic acid molecule having a sequence homologous to at least any one region of the genomic sequence thereof, are present. Such conditions may differ depending on the organism, and are well known for those skilled in the art. Such conditions include, but are not limited to, for example:

Tk-pyrF deleted strain No. 25, No. 27 are cultured in 20ml of ASW-YT liquid medium.
↓
Collect the bacteria from the culture medium (3ml) per one sample (No. 25, No. 27, five samples for each)
↓
Suspend the cells in $0.8 \times ASW + 80mM$ $CaCl_2$ 200μl, and let stand on ice for 30 minutes
↓
3μg pUC118/DS and 3μg pUC118/DD are mixed and let stand on ice for 1 hour (two samples for each. Equivalent volume of TE buffer added sample was used as a control)
↓
heat shock at 85 °C, 45s
↓
let stand on ice for 10 minutes
↓
Preculture in Ura-ASW-AA liquid medium (proliferation occurs based on the incorporated uracil)
↓
Culture on Ura-ASW-AA liquid medium (enriched for PyrF+ strain)
↓
Culture on Ura-ASW-AA solid medium

The present invention is not limited to the above conditions. As used herein the composition of ASW (artificial sea water) is as follows: 1 x Artificial sea water (ASW) (/L) : NaCl 20g ; $MgCl_2 \cdot 6H_2O$ 3g ; $MgSO_4 \cdot 7H_2O$ 6g ; $(NH_4)_2SO_4$ 1g ; $NaHCO_3$ 0.2g ; $CaCl_2 \cdot 2H_2O$ 0.3g ; KCl 0.5g ; NaBr 0.05g ; $SrCl_2 \cdot 6H_2O$ 0.02g ; and $Fe(NH_4)$ citric acid 0.01g.

Homologous recombination may occur when there is at least one homologous region between a genome and a vector, and preferably, when there are two homologous regions between the genome and the vector.

As used herein the term "cross-over" or "crossover", when used for a chromosome, refers to a pair of homologous chromosomes is crossed in this way, resulting in a new combination of nucleic acid sequences.

As used herein the term "single cross over", when used for chromosome, refers to that there is one homologous region causing the cross-over between the nucleic acid molecules , and cross-over occurs only in that particular region, resulting in one nucleic acid sequence thereof that is incorporated in the other sequence.

As used herein the term "double cross-over", when used for chromosome, refers to that there are two homologous regions betweem two nucleic acid molecules for cross-over, and the nucleic acid sequence is replaced with each other between the homologous regions.

As used herein, the term "expression" of a gene, a polynucleotide, a polypeptide, or the like, indicates that the gene or the like is affected by a predetermined action *in vivo* to be changed into another form. Preferably, the term "expression" indicates that genes, polynucleotides, or the like are transcribed and translated into polypeptides. In one embodiment of the present invention, genes may be transcribed into mRNA. More preferably, these polypeptides may have post-translational processing modifications.

As used herein the term "expression product" of a gene, refers to a substance resulting from expression of the gene, and includes mRNA which is a transcription product, a polypeptide which is a translation product, and a polypeptide which is a post-translational product, and the like. Detection of such expression products may be directly or indirectly performed, and may be performed using a well known technology in the art (for example, Southern blotting, Northern blotting and the like). These technologies are described elsewhere herein, as well as in the references cited elsewhere herein.

Polypeptides used in the present invention may be produced by, for example, cultivating primary culture cells producing the peptides or cell lines thereof, followed by separation or purification of the peptides from culture supernatant. Alternatively, genetic manipulation techniques can be used to incorporate a gene encoding a polypeptide of interest into an appropriate expression vector, transform an expression host with the vector, and collect recombinant polypeptides from the culture supernatant of the transformed cells. The above-described host cell may be any host cells conventionally used in genetic manipulation techniques as long as they can express a polypeptide of interest while keeping the physiological activity of the peptide (e.g., *E. coli,* yeast, an animal cell, etc.). Conditions for culturing recombinant host cells may be appropriately selected depending on the type of host cell used. Any host cells which may be used in a recombinant DNA technology may be used as a host cell in the present invention, including bacterial cells, yeast cells, animal cells, plant cells, insect cells, and the like. Preferable host cell is a bacterial cell. Polypeptides derived from the thus-obtained cells may have at least one amino acid substitution, addition, and/or deletion or at least one sugar chain substitution, addition, and/or deletion as long as they have substantially the same function as that of naturally-occurring polypeptides. When an expression product is secreted extracellularly, for example, the supernatant is obtained by centrifuging or filtering a culture, and directly purifying the same or concentrating by precipitation or ultrafiltration for purification. When an expression product is accumulated intracellularly, cells may be disrupted by a cell wall lysis enzyme, change in osmolarity, use of glass beads, homogenizer, or sonication or the like, to obtain cellular extract for purification. Purification may be performed by combining known methods in the art, such as ion sxchange chromatography, gel filtration, affinity chromatography, electrophoresis and the like.

A given amino acid may be substituted with another amino acid in a protein structure, such as a cationic region or a substrate molecule binding site, without a clear reduction or loss of interactive binding ability. A given biological function of a protein is defined by the interactive ability or other property of the protein. Therefore, a particular amino acid substitution may be performed in an amino acid sequence, or at the DNA code sequence level, to produce a protein which maintains the original property after the substitution. Therefore, various modifications of peptides as disclosed herein and DNA encoding such peptides may be performed without clear losses of biological usefulness.

When the above-described modifications are designed, the hydrophobicity indices of amino acids may be taken into consideration. Hydrophobic amino acid indices play an important role in providing a protein with an interactive biological function, which is generally recognized in the art (Kyte, J. and Doolittle, R.F., J. Mol. Biol. 157(1):105-132, 1982). The hydrophobic property of an amino acid contributes to the secondary structure of a protein and then regulates interactions between the protein and other molecules (e.g., enzymes, substrates, receptors, DNA, antibodies, antigens, etc.). Each amino acid is given a hydrophobicity index based on the hydrophobicity and charge properties thereof as follows: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamic acid (-3.5); glutamine (-3.5); aspartic acid (-3.5) ; asparagine (-3.5); lysine (-3.9); and arginine (-4.5).

It is well known that if a given amino acid is substituted with another amino acid having a similar hydrophobicity index, the resultant protein may still have a biological function similar to that of the original protein (e.g., a protein having an equivalent enzymatic activity). For such an amino acid substitution, the hydrophobicity index is preferably within $\pm 2$, more preferably within $\pm 1$, and even more preferably within $\pm 0.5$. It is understood in the art that such an amino acid substitution based on hydrophobicity is efficient.

A hydrophilicity index is also useful for modification of an amino acid sequence of the present invention. As described in US Patent No. 4,554,101, amino acid residues are given the following hydrophilicity indices: arginine (+3.0); lysine (+3.0); aspartic acid (+3.0$\pm$1) ; glutamic acid (+3.0$\pm$1); serine (+0.3); asparagine (+0.2); glutamine (+0.2); glycine (0); threonine (-0.4); proline (-0.5$\pm$1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); and tryptophan (-3.4). It is understood that an amino acid may be substituted with another amino acid which has a similar hydrophilicity index and can still provide a biological equivalent. For such an amino acid substitution, the hydrophilicity index is preferably within $\pm 2$, more preferably $\pm 1$, and even more preferably $\pm 0.5$.

The term "conservative substitution" as used herein refers to amino acid substitution in which a substituted amino acid and a substituting amino acid have similar hydrophilicity indices or/and hydrophobicity indices. For example, the conservative substitution is carried out between amino acids having a hydrophilicity or hydrophobicity index of within $\pm 2$, preferably within $\pm 1$, and more preferably within $\pm 0.5$. Examples of the conservative substitution include, but are not limited to, substitutions within each of the following residue pairs: arginine and lysine; glutamic acid and aspartic acid; serine and threonine; glutamine and asparagine; and valine, leucine, and isoleucine, which are well known to those skilled in the art.

As used herein the term "silent substitution" refers to a substitution in which there are nucleotide sequence substitutions but no amino acid change is encoded by the substituted nucleotides. Such silent substitutions may be performed using genetic code degeneracy. Such degeneracy is well known in the art, and is also described in the references cited herein.

As used herein, the term "variant" refers to a substance, such as a polypeptide, polynucleotide, or the like, which differs partially from the original substance. Examples of such a variant include a substitution variant, an addition variant, a deletion variant, a truncated variant, an allelic variant, and the like. Examples of such a variant include, but are not limited to, a nucleotide or polypeptide having one or several substitutions, additions and/or deletions or a nucleotide or polypeptide having at least one substitution, addition and/or deletion. The term "allele" as used herein refers to a genetic variant located at a locus identical to a corresponding gene, where the two genes are distinguished from each other. Therefore, the term "allelic variant" as used herein refers to a variant which has an allelic relationship with a given gene. Such an allelic variant ordinarily has a sequence the same as or highly similar to that of the corresponding allele, and ordinarily has almost the same biological activity, though it rarely has different biological activity. The term "species homolog" or "homolog" as used herein refers to one that has an amino acid or nucleotide homology with a given gene in a given species (preferably at least 60% homology, more preferably at least 80%, at least 85%, at least 90%, and at least 95% homology). A method for obtaining such a species homolog is clearly understood from the description of the present specification. The term "orthologs" (also called orthologous genes) refers to genes in different species derived from a common ancestry (due to speciation). For example, in the case of the hemoglobin gene family having multigene structure, human and mouse α-hemoglobin genes are orthologs, while the human α-hemoglobin gene and the human β-hemoglobin gene are paralogs (genes arising from gene duplication). Orthologs are useful for estimation of molecular phylogenetic trees. Usually, orthologs in different species may have a function similar to that of the original species. Therefore, orthologs of the present invention may be useful in the present invention.

As used herein, the term "conservative (or conservatively modified) variant" applies to both amino acid and nucleic acid sequences. With respect to particular nucleic acid sequences, conservatively modified variants refer to those nucleic acids which encode identical or essentially identical amino acid sequences. Because of the degeneracy of the genetic code, a large number of functionally identical nucleic acids encode any given protein. For example, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations" which represent one species of conservatively modified variation. Every nucleic acid sequence herein which encodes a polypeptide also describes every possible silent variation of the nucleic acid. Those skilled in the art will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid which encodes a polypeptide is implicit in each described sequence. Preferably, such modification may be performed while avoiding substitution of cysteine which is an amino acid capable of largely affecting the higher-order structure of a polypeptide. Such a conservative modification or silent modification is also within the scope of the present invention.

The above-described nucleic acid can be obtained by a well-known PCR method, i.e., chemical synthesis. This method may be combined with, for example, site-specific mutagenesis, hybridization, or the like.

As used herein, the term "substitution, addition or deletion" for a polypeptide or a polynucleotide refers to the substitution, addition or deletion of an amino acid or its substitute, or a nucleotide or its substitute, with respect to the original polypeptide or polynucleotide, respectively. This is achieved by techniques well known in the art, including a site directed mutagenesis technique and the like. A polypeptide or a polynucleotide may have any number (>0) of substitutions, additions, or deletions. The number can be as large as a variant having such a number of substitutions, additions or deletions which maintains an intended function (e.g., the cancer marker, nervous disorder marker, etc.). For example, such a number may be one or several, and preferably within 20% or 10% of the full length, or no more than 100, no more than 50, no more than 25, or the like.

As used herein, the term "specifically expressed" in the case of genes indicates that a gene is expressed in a specific site or in a specific period of time at a level different from (preferably higher than) that in other sites or periods of time. The term "specifically expressed" includes that a gene may be expressed only in a given site (specific site) or may be expressed in other sites. Preferably, the term "specifically expressed" indicates that a gene is expressed only in a given site.

General molecular biological technologies which may be used in the present invention may be readily performed by those skilled in the art by referring to for example, Ausubel F.A.et al., ed. (1988), Current Protocols in Molecular Biology, Wiley, New York, NY;Sambrook J et al. (1987) Molecular Cloning:A Laboratory Manual, 2nd Ed.,Cold Spring Harbor Laboratory Press,Cold Spring Harbor,NY.

As used herein the term "thermostable" refers to a property having resistance against a temperture which is higher than circumstancial temperature in which a usual organism survives, and includes resistance against temperature higher than 37 °C. More usually, the thermostable refers to resistance against temperature higher than 50 °C. Thermostable, when used for a living organism, may refer to a property thereof in which an organism can survive at lower and higher temperatures. On the other hand, thermostable, when used for a polypeptide, refers to resistance against higher temperature, for example a temperature higher than 37 °C, a temperature higher than 50 °C. Amongst them, the property of having resistance to temperatures higher than 90 °C refers to "hyperthermostable".

As used herein, an organism which can survive at higher temperature is often called "thermophillic bacteria". Thermophillic bacteria usually have survival optimum temperatures of 50-105 °C and do not grow at 30 °C or lower. Amongst them, those having an optimum temperature of 90 °C or higher are called "hyperthermophillic bacteria".

As used herein the term "hyperthermophillic archeabacteria" and "hyperthermostable bacteria" are interchangeably used to refer to a microorganism growing at 90 °C or higher. Preferably, the hyperthermophillic archeabacteria is Thermococcus kodakaraensis KOD1 strain, a thermostable DNA ligase producing, thermostable thiol protease producing bacteria isolated by the present inventors (Morikawa, M. et al., Appl.Environ.Microbiol. 60(12), 4559-4566(1994)). KOD-1 strains were deposited in the International Patent Organism Depositary (Chuo No. 6, Higashi 1-Chome, 1-1, Tsukuba-shi, Ibaraki, 305-8566), and the accession number there of FERM P-15007. KOD-1 strains were originally classified as a Pyrococcus bacteria, as described in the above-mentioned reference. However, when we compared the sequence of 16S rRNA using the registered data in GenBank R91.0 Octber, 1995+Daily Update inputted in DNASIS (Hitachi Software Engineering), it was revealed that KOD-1 strains belongs to the *Thermococcus* genus, rather than the *Pyrococcus* genus, and thus is presently classified as *Thermococcus kodakaraensis* KOD-1.

As used herein, culturing hyperthermophillic archeabateria producing hyperthermostable proteins may be performed under any culture conditions, for example, those described in Appl. Environ. Microbiol. 60(12), 4559-4566 (1994) (ibid). Culture may be either static culture or jar fermentation culture by nitrogen gas, and may be either in a continuous or batch manner.

The chromosomal DNA of a hyperthermophillic archeabacteria may be obtained by solubilizing the cultured bacterial cells with detergent (for example, N-lauryl sarcosin), and fractionating the resultant soluent by cesium chloride ethidium bromide equilibrium density-gradient centrifugation (see, for example, Imanaka et al., J. Bacteriol. 147: 776-786 (1981)). Libraries may be obtained by digesting the resultant chromosomal DNA by a variety of restriction enzymes, followed by ligating the same into a vector (such as a phage or plasmid), which has been digested with the same restriction enzyme or similar restriction enzyme resulting in the same digestion terminus, with an enzyme such as T4 DNA ligase or the like.

Libraries may be screened by selecting a clone comprising a DNA encoding a thermophilic DNA ligase of interest therefrom. Selection may be performed using an oligonucleotide designed based on a partial amino acid sequence of the predetermined hyperthermophillic DNA ligase and a cloned DNA deduced to have homology with the DNA of interest as a probe. Alternatively, selection may be performed by expressing the enzyme of interest. Detection of expression may be performed, for example, when the activity of the enzyme of interest may be readily detected, by detecting the activity of expression product against the substrate added to the plate, or alternatively when an antibody against the enzyme of interest is available, using the reactivity between the expression product and the antibody.

Analysis of the resultant cloned DNA may be perforemd by, for example, isolating a selected DNA, producing a restriction map therefor, and determining the nucleotide sequence, and the like. Technologies such as preparation of a cloned DNA, restriction enzyme processing, subcloning, nucleotide sequencing and the like are well known in the art, and may be performed by referring to "Molecular Cloning: A Laboratory Manual Second Edition, " (Sambrook, Fritsch and Maniatis ed., Cold Spring Harbor Laboratory Press,1989)

Next, the resultant cloned DNA may be expressed by operably inserting the same into an expression vector applicable to a host cell used, transforming a host cell with the expression vector, and culturing the transformed host cell.

(Biomolecule chip)

The genomic information of the present invention may be used for providing a biomolecule chip (for example, DNA chip, protein chip, glycoprotein chip, antibody chip and the like).

The analysis of expression control of the genes of the present invention may be performed by genetic analysis method using a DNA array. The present invention also provides a virtual genome DNA array (also called as "hyperthermophillic genomic array") using the genomic sequence which has first identified in the present invention.

The nucleotides of the present invention may be used in a gene analysis method using a DNA array. A DNA array is widely reviewed (Shujunsha Ed., Saibo-kogaku (Cellular Engineering), Special issue, "DNA-maikuro-arei-to-saisin-PCR-ho [DNA microarray and Up-to-date PCR Method"). Further, plant analysis using a DNA array has been recently used (Schenk PM et al. (2000) Proc. Natl. Acad. Sci. (USA) 97: 11655-11660). Hereinafter, a DNA array and a gene analysis method using the same will be briefly described.

"DNA array" refers to a device in which DNAs are arrayed and immobilized on a plate. DNA arrays are divided into DNA macroarrays, DNA microarrays, and the like according to the size of a plate or the density of DNA placed on the plate, however, the use of these terms are not strict as used herein.

The border between macro and micro is not strictly determined. However, generally, "DNA macroarray" refers to a high density filter in which DNA is spotted on a membrane, while "DNA microarray" refers to a plate of glass, silicon, and the like which carries DNA on a surface thereof. There are a cDNA array, an oligoDNA array, and the like according to the type of DNA placed.

A certain high density oligoDNA array, in which a photolithography technique for production of semiconductor integrated circuits is applied and a plurality of oligoDNAs are simultaneously synthesized on a plate, is particularly called "DNA chip", an adaptation of the term "semiconductor chip". Examples of the DNA chip prepared by this method include GeneChip® (Affymetrix, CA), and the like (Marshall A et al., (1998) Nat. Biotechnol. 16: 27-31 and Ramsay G et al., (1998) Nat. Biotechnol. 16 40-44). Preferably, GeneChip® may be used in gene analysis using a microarray according to the present invention. The DNA chip is defined as described above in a narrow sense, but may refer to all types of DNA arrays or DNA microarrays.

Thus, DNA microarrays are a device in which several thousands to several ten thousands or more of gene DNAs are arrayed on a glass plate in high density. Therefore, it is possible to analyze gene expression profiles or gene polymorphism at a genomic scale by hybridization of cDNA, cRNA or genomic DNA. With this technique, it has been made possible to analyze a signal transfer system and/or a transcription control pathway (Fambrough D et al. (1999), Cell 97, 727-741); the mechanism of tissue repair (Iyer VR et al., (1999), Science 283: 83-87); the action mechanism of medicaments (Marton MJ, (1999), Nat. Med. 4: 1293-1301); fluctuations in gene expression during development and differentiation processes in a wide scale, and the like; identify a gene group whose expression is fluctuated according to pathologic conditions; find a novel gene involved in a signal transfer system or a transcription control; and the like. Further, as to gene polymorphism, it has been made possible to analyze a number of SNP with a single DNA microarray (Cargill M et al., (1999), Nat. Genet. 22:231-238).

The principle of an assay using a DNA microarray will be described. DNA microarrays are prepared by immobilizing a number of different DNA probes in high density on a solid-phase plate, such as a slide glass, whose surface is appropriately processed. Thereafter, labeled nucleic acids (targets) are subjected to hybridization under appropriate hybridization conditions, and a signal from each probe is detected by an automated detector. The resultant data is subjected to massive analysis by a computer. For example, in the case of gene monitoring, target cDNAs integrated with fluorescent labels by reverse transcription from mRNA are allowed to hybridize to oligoDNAs or cDNAs as a probe on a microarray, and are detected with a fluorescence image analyzer. In this case, T7 polymerase may be used to carry out other various signal amplification reactions, such as cRNA synthesis reactions or via enzymatic reactions.

Fodor et al. has developed a technique for synthesizing polymers on a plate using a combination of combinatorial chemistry and photolithography for semiconductor production (Fodor SP et al., (1991) Science 251: 767-773). This is called the synthesized DNA chip. Photolithography allows for extremely minute surface processing, thereby making it possible to produce a DNA microarray having a packing density of as high as 10 $\mu m^2$/DNA sample. In this method, generally, about 25 to about 30 DNAs are synthesized on a glass plate.

Gene expression using a synthesized DNA chip was reported by Lockart et al. (Lockart DJ et al. (1996) Nat. Biotechnol.: 14: 1675-1680). This method overcomes a drawback of the chip of this type in that the specificity is low since the length of synthesized DNA is short. This problem was solved by preparing perfect match (PM) oligonucleotide probes corresponding to from about 10 to about 20 regions and mismatch (MM) oligonucleotide probes having a one base mutation in the middle of the PM probes for the purpose of monitoring the expression of one gene. Here, the MM probes are used as an indicator for the specificity of hybridization. Based on the signal ratio between the PM probe and the MM probe, the level of gene expression may be determined. When the signal ratio between the PM probe and the MM probe is substantially 1:1, the result is called cross hybridization, which is not interpreted as a significant signal.

A so-called attached DNA microarray is prepared by attaching DNAs onto a slide glass, and fluorescence is detected (see also http://cmgm.stanford.edu/pbrown). In this method, no gigantic semiconductor production machine is required, and only a DNA array machine and a detector are used to perform the assay in a laboratory. This method has the advantage that it is possible to select DNAs to be attached. A high density array can be obtained by spotting spots having a diameter of 100 $\mu m$ at intervals of 100 $\mu m$, for example. It is mathematically possible to spot 2500 DNAs per $cm^2$. Therefore, a usual slide glass (the effective area is about 4 $cm^2$) can carry about 10,000 DNAs.

As a labeling method for synthesized DNA arrays, for example, double fluorescence labeling is used. In this method, two different mRNA samples are labeled by different fluorescent dyes respectively. The two samples are subjected to competitive hybridization on the same microarray, and both fluorescences are measured. A difference in gene expression is detected by comparing the fluorescences. Examples of the fluorescent dye include, but are not limited to, Cy5 and Cy3, which are most often used, and the like. The advantage of Cy3 and Cy5 is that the wavelengths of fluorescences do not overlap substantially. Double fluorescence labeling may be used to detect mutations or morphorisms in addition to differences in gene expression.

An array machine may be used for assays using a DNA array. In the array machine, basically, a pin tip or a slide holder is moved in directions along the X, Y and Z axes in combination with a high-performance servo motor under the control of a computer so that DNA samples are transferred from a microtiter plate to the surface of a slide glass. The pin tip is processed into various shapes. For example, a DNA solution is retained in a cloven pen tip like a crow' s bill and spotted onto a plurality of slide glasses. After washing and drying cycles, a DNA sample is then placed on the slide glasses. The above-described steps are repeated. In this case, in order to prevent contamination of the pin tip by a different sample, the pin tip has to be perfectly washed and dried. Examples of such an array machine include SPBIO2000 (Hitachi Software Engineering Co., Ltd.; single strike type), GMS417 Arrayer (Takara Shuzo Co., Ltd.; pin ring type), Gene Tip Stamping (Nippon Laser&Electronics Lab.; fountain pen type), and the like.

There are various DNA immobilizing methods for use in assays using a DNA array. Glass as a material for a plate has a small effective area for immobilization and electrical charge amount as compared to membranes, and therefore is given various coatings such as poly L-lysine coating (Reference 55), silane finishing (Reference 56), or the like. Further, a commercially available precoated slide glass exclusive to DNA microarrays (e.g., polycarboimide glass (Nissin Spinning Co., Ltd.) and the like) may also be used. In the case of oligoDNA, a method of aminating a terminal of the DNA and crosslinking the DNA to silane-finished glass is available.

DNA microarrays may carry mainly cDNA fragments amplified by PCR. When the concentration of cDNA is insufficient, signals cannot be sufficiently detected in some cases. In a case when a sufficient amount of cDNA fragments is not obtained by one PCR operation, PCR is repeated. The resultant overall PCR products may be purified and condensed at one time. A probe cDNA may generally carry a number of random cDNAs, but may carry a group of selected genes (e.g., the gene or promoter groups of the present invention) or candidate genes for gene expression changes obtained by RDA (representational differential analysis) according to the purpose of an experiment. It is preferable to avoid overlapping clones. Clones may be prepared from a stock cDNA library, or cDNA clones may be purchased.

In assays using a DNA array, a fluorescent signal indicating hybridization on the DNA microarray is detected by a fluorescence detector or the like. There are various conventionally available detectors for this purpose. For example, a research group at the Stanford University has developed an original scanner which is a combination of a fluorescence microscope and a movable stage (see http://cmgm.stanford.edu/pbrown). A conventional fluorescence image analyzer for gel, such as FMBIO (Hitachi Software Engineering), Storm (Molecular Dynamics), and the like, can read a DNA microarray if the spots are not arrayed in very high density. Examples of other available detectors include ScanArray 4000 and 5000 (GeneralScanning; scan type (confocal type)), GMS418 Array Scanner (Takara Shuzo; scan type (confocal type)), Gene Tip Scanner (Nippon Laser&Electronics Lab.; scan type (non-confocal type)), Gene Tac 2000 (Genomic Solutions; CCD camera type)), and the like.

The amount of data obtained from DNA microarrays is huge. Software for managing correspondences between clones and spots, analyzing data, and the like is important. Such software attached to each detection system is available (Ermolaeva O et al. (1998) Nat. Genet. 20:19-23). Further, an example of a database format is GATC (genetic analysis technology consortium) proposed by Affymetrix.

The present invention may also be used in gene analysis using a differential display technique.

The differential display technique is a method for detecting or identifying a gene whose expression fluctuates. In this method, cDNA is prepared from each of at least two samples, and amplified by PCR using a set of any primers. Thereafter, a plurality of generated PCR products are separated by gel electrophoresis. After the electrophoresis pattern is produced, expression-fluctuating genes are cloned based on a relative signal strength change between each band.

The term "support" as used herein refers to a material for an array construction of the present invention. Examples of a material for the substrate include any solid material having a property of binding to a biomolecule used in the present invention either by covalent bond or noncovalent bond, or which can be derived in such a manner as to have such a property.

Such a material for the substrate may be any material capable of forming a solid surface, for example, including, but being not limited to, glass, silica, silicon, ceramics, silica dioxide, plastics, metals (including alloys), naturally-occurring and synthetic polymer (e.g., polystyrene, cellulose, chitosan, dextran, and nylon). The substrate may be formed of a plurality of layers made of different materials. For example, an inorganic insulating material, such as glass, silica glass, alumina, sapphire, forsterite, silicon carbide, silicon oxide, silicon nitride, or the like, can be used. Moreover, an organic material, such as polyethylene, ethylene, polypropylene, polyisobutylene, polyethylene terephthalate, unsaturated polyester, fluorine-containing resin, polyvinyl chloride, polyvinylidene chloride, polyvinyl acetate, polyvinyl alcohol, polyvinyl acetal, acrylic resin, polyacrylonitrile, polystyrene, acetal resin, polycarbonate, polyamide, phenol resin, urea resin, epoxy resin, melamine resin, styrene · acrylonitrile copolymer, acrylonitrilebutadienestyrene copolymer, silicone resin, polyphenylene oxide, or polysulfone, can be used. In the present invention, a film used for nucleic acid blotting, such as a nitrocellulose film, a PVDF film, or the like, can also be used. When material constituting the substrate is a solid phase, it is specifically referred to as "solid (phase) substrate" as used herein. As used herein such a substrate may be a form of plate, microwell plate, chip, glass slide, film, bead, metal (surface) and the like. Substrates may or may not be coated.

"Chip" as used herein refers to an ultramicro-integrated circuit having various functions, which constitutes a part of a system. "Biomolecule chip" as used herein refers to a chip comprising a substrate and a biomolecule, in which at least one biomolecule as set forth herein is disposed on the substrate.

The term "address" as used herein refers to a unique position on a substrate which can be distinguished from other unique positions. An address is suitably used to access a biomolecule associated with the address. Any entity present at each address can have an arbitrary shape which allows the entity to be distinguished from entities present at other addresses (e.g., in an optical manner). The shape of an address may be, for example, a circle, an ellipse, a square, or a rectangle, or alternatively an irregular shape.

The size of each address varies depending on, particularly, the size of a substrate, the number of addresses on the specific substrate, the amount of samples to be analyzed and/or an available reagent, the size of a biomolecule, and the magnitude of a resolution required for any method in which the array is used. The size of an address may range from 1-2 nm to several centimeters (e.g., 1-2 mm to several centimeters, etc., 125×80 mm, 10×10 mm, etc.). Any size of an address is possible as long as it matches the array to which it is applied. In such a case, a substrate material is formed into a size and a shape suitable for a specific production process and application of an array. For example, in the case of analysis where a large amount of samples to be measured are available, an array may be more economically constructed on a relatively large substrate (e.g., 1 cm × 1 cm or more). Here, a detection system which does not require much sensitivity and is therefore economical may be further advantageously used. On the other hand, when the amount of an available sample to be analyzed and/or reagent is limited, an array may be designed so that consumption of the sample and reagent is minimized.

The spatial arrangement and forms of addresses are designed in such a manner as to match a specific application in which the microarray is used. Addresses may be densely loaded, widely distributed, or divided into subgroups in a pattern suitable for a specific type of sample to be analyzed. "Array" as used herein refers to a pattern of solid substances fixed on a solid phase surface or a film, or a group of molecules having such a pattern. Typically, an array comprises biomolecules (e.g., DNA, RNA, protein-RNA fusion molecules, proteins, low-weight organic molecules, etc.) conjugated to nucleic acid sequences fixed on a solid phase surface or a film as if the biomolecule captured the nucleic sequence. "Spots" of biomolecules may be arranged on an array. "Spot" as used herein refers to a predetermined set of biomolecules.

Any number of addresses may be arranged on a substrate, typically up to $10^8$ addresses, in other embodiments up to $10^7$ addresses, up to $10^6$ addresses, up to $10^5$ addresses, up to $10^4$ addresses, up to $10^3$ addresses, or up to $10^2$ addresses. Therefore, when one biomolecule is placed on one address, up to $10^8$ biomolecules can be placed on a substrate, and in other embodiment up to $10^7$ biomolecules, up to $10^6$ biomolecules, up to $10^5$ biomolecules, up to $10^4$ biomolecules, up to $10^3$ biomolecules, or up to $10^2$ biomolecules can be placed on a substrate. In these cases, a smaller size of substrate and a smaller size of address are suitable. In particular, the size of an address may be as small as the size of a single biomolecule (i.e., this size may be of the order of 1-2 nm). In some cases, the minimum area of a substrate is determined based on the number of addresses on the substrate.

The term "biomolecule" as used herein refers to a molecule related to an organism. An "organism (or "bio-")" as used herein refers to a biological organic body, including, but being limited to, an animal, a plant, a fungus, a virus, and the like. A biomolecule includes a molecule extracted from an organism, but is not so limited. A biomolecule is any molecule capable of having an influence on an organism. Therefore, a biomolecule also includes a molecule synthesized by combinatorial chemistry, and a low weight molecule capable of being used as a medicament (e.g., a low molecular weight ligand, etc.) as long as they are intended to have an influence on an organism. Examples of such a biomolecule include, but are not limited to, proteins, polypeptides, oligopeptides, peptides, polynucleotides, oligonucleotides, nucleotides, nucleic acids (e.g., including DNA (such as cDNA and genomic DNA) and RNA (such as mRNA)), polysaccharides, oligosaccharides, lipids, low weight molecules (e.g., hormones, ligands, signal transduction substances, low-weight organic molecules, etc.), and complex molecules thereof, and the like. A biomolecule also includes a cell itself, and a part or the whole of a tissue, and the like as long as they can be coupled to a substrate of the present invention. Preferably, a biomolecule includes a nucleic acid or a protein. In a preferable embodiment, a biomolecule is a nucleic acid (e. g. , genomic DNA or cDNA, or DNA synthesized by PCR or the like). In another preferable embodiment, a biomolecule may be a protein. Preferably, one type of biomolecule may be provided for each address on a substrate of the present invention. In another embodiment, a sample containing two or more types of biomolecules may be provided for each address.

As used herein the term "liquid phase" is used to mean as usually used in the art, and usually refers to a state in a solution.

As used herein the term "solid phase" is used to mean as usually used in the art, and usually refers to a state in a solid. As used herein liquid and solid collectively refer to "fluid".

As used herein the term "contact" refers to existing in a sufficient vicinity distance for interaction between two matters (for example, a composition and a cell) to each other.

As used herein the term "interaction" refers, when referring to two matters, to that the two matters exert a force to each other. Such interaction includes, but is not limited to, for example, covalent bonding, hydrpgen bonding, van der Waals forces, ionic interaction, non-ionic interaction, hydrophobic interaction, electrostatic interaction and the like. Preferably, the interaction may be normal interaction caused in a living body such as hydrogen bonding, hydrophobic interaction, and the like.

In one embodiment, the present invention may produce a micoarray for screening for a molecule, by binding a library of biomolecules (for example, organic low-molecular weight moleculre, combinatorial chemistory products) to a substrate, and using the same. Chemical library used in the present invention, may be produced or obtained by any means including ,but is not limited to, for example, by the use of combinatorial chemistry technology, fermentation technology, plant and cell extraction procedures and the like. Production of a combinatorial library is well known in the art. For example, E. R. Felder, Chimia 1994, 48, 512-541; Gallop et al., J. Med. Chem. 1994, 37, 1233-1251; R. A. Houghten, Trends Genet. 1993, 9, 235-239; Houghtenet al., Nature 1991, 354, 84-86; Lam et al., Nature 1991, 354, 82-84; Carell et al., Chem. Biol. 1995, 3, 171-183; Madden et al., Perspectives in Drug Discovery and Design 2, 269-282; Cwirla et al., Biochemistry 1990, 87, 6378-6382; Brenner et al., Proc.Natl. Acad. Sci. USA 1992, 89, 5381-5383; Gordon et al., J. Med. Chem. 1994,37, 1385-1401; Lebl et al., Biopolymers 1995, 37 177-198 ; and references cited therein. These references are incorporated by reference for their entireties

Methods, biomolecule chips and apparatuses of the present invention may be used for, for example, diagnosis, forensic medicine, drug discovery (screening for drugs) and development, molecular biological analysis (for example, nucleotide sequencing based array and gene sequence analysis based on array), analysis of protein properties and functions, pharmacogenomics, proteomics, environmental search, and additional biological and chemical analyses.

The present invention can also be applied to polymorphism analysis, such as RFLP analysis, SNP (snipp, single nucleotide polymorphism) analysis, or the like, analysis of base sequences, and the like. The present invention can also be used for screening of a medicament.

The present invention can be applied to any situation requiring a biomolecule test other than medical applications, such as food testing, quarantine, medicament testing, forensic medicine, agriculture, husbandry, fishery, forestry, and the like.

The present invention can also be used for detection of a gene amplified by PCR, SDA, NASBA, or the like, other than a sample directly collected from an organism. In the present invention, a target gene can be labeled in advance with an electrochemically active substance, a fluorescent substance (e.g., FITC, rhodamine, acridine, Texas Red, fluorecein, etc.), an enzyme (e.g., alkaline phosphatase, peroxidase, glucose oxidase, etc.), a colloid particle (e.g., a hapten, a light-emitting substance, an antibody, an antigen, gold colloid, etc.), a metal, a metal ion, a metal chelate (e.g., trisbipyridine, trisphenanthroline, hexamine, etc.), or the like.

In one embodiment, a nucleic acid component is extracted from these samples in order to test the nucleic acid. The extraction is not limited to a particular method. A liquid-liquid extraction method, such as phenol-chloroform method and the like, or a liquid-solid extraction method using a carrier can be used. Alternatively, a commercially available nucleic acid extraction method such as QIAamp (QIAGEN, Germany) or the like can be used. Next, a sample containing an extracted nucleic acid component is subjected to a hybridization reaction on a biomolecule chip of the present invention. The reaction is conducted in a buffer solution having an ionic strength of 0.01 to 5 and a pH of 5 to 10. To this solution may be added dextran sulfate (hybridization accelerating agent), salmon sperm DNA, bovine thymus DNA, EDTA, a surfactant, or the like. The extracted nucleic acid component is added to the solution, followed by heat denaturation at 90°C or more. Insertion of a biomolecule chip can be carried out immediately after denaturation or after rapid cooling to 0°C. Alternatively, a hybridization reaction can be conducted by dropping a solution on a substrate. The rate of a reaction can be increased by stirring or shaking during the reaction. The temperature of a reaction is in the range of 10°C to 90°C. The time of a reaction is in the range of one minute to about one night. After a hybridization reaction, an electrode is removed and then washed. For washing, a buffer solution having an ionic strength of 0.01 to 5 and a pH of 5 to 10 can be used.

"Label" as used herein refers to an entity which distinguishes an intended molecule or substance from other substances (e.g., a substance, energy, electromagnetic wave, etc.). Examples of such a labeling method include an RI (radioisotope) method, a fluorescence method, a biotin method, a chemiluminescence method, and the like. When both a nucleic acid fragment and its complementary oligonucleotide are labeled by a fluorescence method, they are labeled with fluorescence substances having different maximum wavelengths of fluoresence. The difference in the maximum wavelength of fluorescence is preferably at least 10 nm. Any fluorescence substance which can bind to a base portion of nucleic acid can be used. Preferable fluorescence substances include cyanine dye (e.g., Cy3, Cy5, etc. in Cy Dye™ series), a rhodamine 6G reagent, N-acetoxy-N2-acetylaminofluorene (AAF), AAIF (an iodine derivative of AAF), and the like. Examples of a combination of fluorescence substances having a difference in the maximum wavelength of fluorescence of at least 10 nm, include a combination of Cy5 and a rhodamine 6G reagent, a combination of Cy3 and fluorescein, a combination of a rhodamine 6G reagent and fluorescein, and the like.

"Chip attribute data" as used herein refers to data associated with some information relating to a biomolecule chip of the present invention. Chip attribute data includes information associated with a biomolecule chip, such as a chip ID, substrate data, and biomolecule attribute data. "Chip ID" as used herein refers to a code for identification of each chip. "Substrate data" or "substrate attribute data" as used herein refers to data relating to a substrate used in a biomolecule chip of the present invention. Substrate data may contain information relating to an arrangement or pattern of a biomolecule. "Biomolecule attribute data" refers to information relating to a biomolecule, inclding, for example, the gene sequence of the biomolecule (a nucleotide sequence in the case of nucleic acid, and an amino acid sequence in the case of protein), information relating to a gene sequence (e.g., a relationship between the gene and a specific disease or condition), a function in the case of a low weight molecule or a hormone, library information in the case of a combinatorial library, molecular information relating to affinity for a low weight molecule, and the like. "Personal information data" as used herein refers to data associated with information for identifying an organism or subject to be measured by a method, chip or apparatus of the present invention. When the organism or subject is a human, personal information data includes, but is not limited to, age, sex, health condition, medical history (e.g., drug history), educational background, the company of your insurance, personal genome information, address, name, and the like. When the personal information data is for a domestic animal, the information may include data about the production company of the animal. "Measurement data" as used herein refers to raw data as a result of measurement by a biomolecule substrate, apparatus and system of the present invention and specific processed data derived therefrom. Such raw data may be represented by the intensity of an electric signal. Such processed data may be specific biochemical data, such as a blood sugar level or a gene expression level.

"Recording region" as used herein refers to a region in which data may be recorded. In a recording region, measurement data as well as the above-described chip attribute data can be recorded.

Techniques as used herein are well known techniques commonly used in microfluidics, micromachining, organic chemistry, biochemistry, genetic engineering, molecular biology, genetics, and their related fields within the technical scope of the art, unless otherwise specified. These techniques are sufficiently described in, for example, literature listed below and described elsewhere herein.

Micromachining is described in, for example, Campbell, S. A. (1996). The Science and Engineering of Microelectronic Fabrication, Oxford University Press; Zaut, P. V. (1996). Microarray Fabrication: a Practical Guide to Semiconductor Processing, Semiconductor Services; Madou, M. J. (1997). Fundamentals of Microfabrication, CRC1 5 Press; Rai-Choudhury, P. (1997). Handbook of Microlithography, Micromachining, & Microfabrication: Microlithography; and the like, related portions of which are herein incorporated by reference.

Photolithography is a technique developed by Fodor et al., in which a photoreactive protecting group is utilized (see Science, 251, 767(1991)). A protecting group for a base inhibits a base monomer of the same or different type from binding to that base. Thus, a base terminus to which a protecting group is bound has no new base-binding reaction. A protecting group can be easily removed by irradiation. Initially, amino groups having a protecting group are immobilized throughout a substrate. Thereafter, only spots to which a desired base is to be bound are selectively irradiated by a method similar to a photolithography technique usually used in a semiconductor process, so that another base can be introduced by subsequent binding into only the bases in the irradiated portion. Now, desired bases having the same protecting group at a terminus thereof are bound to such bases. Thereafter, the pattern of a photomask is changed, and other spots are selectively irradiated. Thereafter, bases having a protecting group are similarly bound to the spots. This process is repeated until a desired base sequence is obtained in each spot, thereby preparing a DNA array. Photolithography techniques may be herein used.

An ink jet method (technique) is a technique of projecting considerably small droplets onto a predetermined position on a two-dimensional plane using heat or a piezoelectric effect. This technique is widely used mainly in printers. In production of a DNA array, an ink jet apparatus is used, which has a configuration in which a piezoelectric device is combined with a glass capillary. A voltage is applied to the piezoelectric device which is connected to a liquid chamber, so that the volume of the piezoelectric device is changed and the liquid within the chamber is expelled as a droplet from the capillary connected to the chamber. The size of the expelled droplet is determined by the diameter of the capillary, the volume variation of the piezoelectric device, and the physical property of the liquid. The diameter of the droplet is generally 30 $\mu$m. An ink jet apparatus using such a piezoelectric device can expel droplets at a frequency of about 10 KHz. In a DNA array fabricating apparatus using such an ink jet apparatus, the ink jet apparatus and a DNA array substrate are relatively moved so that droplets can be dropped onto desired spots on the DNA array. DNA array fabricating apparatuses using an ink jet apparatus are roughly divided into two categories. One category includes a DNA array fabricating apparatus using a single ink jet apparatus, and the other includes a DNA array fabricating apparatus using a multi-head ink jet apparatus. The DNA array fabricating apparatus with a single ink jet apparatus has a configuration in which a reagent for removing a protecting group at a terminus of an oligomer is dropped onto desired spots. A protecting group is removed from a spot, to which a desired base is to be introduced, by using the ink jet apparatus so that the spot is activated. Thereafter, the desired base is subjected to a binding reaction throughout a DNA array. In this case, the desired base is bound only to spots having an oligomer whose terminus is activated by the reagent dropped from the ink jet apparatus. Thereafter, the terminus of a newly added base is protected. Thereafter, a spot from which a protecting group is removed is changed and the procedures are repeated until desired nucleotide sequences are obtained. On the other hand, in a DNA array fabricating apparatus using a multi-head ink jet apparatus, an ink jet apparatus is provided for each reagent containing a different base, so that a desired base can be bound directly to each spot. A DNA array fabricating apparatus using a multi-head ink jet apparatus can have a higher throughput than that of a DNA array fabricating apparatus using a single ink jet apparatus. Among methods for fixing a presynthesized oligonucleotide to a substrate is a mechanical microspotting technique in which liquid containing an oligonucleotide, which is attached to the tip of a stainless pin, is mechanically pressed against a substrate so that the oligonucleotide is immobilized on the substrate. The size of a spot obtained by this method is 50 to 300 $\mu$m. After microspotting, subsequent processes, such as immobilization using UV light, are carried out.

DESCRIPTION OF PREFERRED EMBODIMENTS

Hereinafter, preferred embodiments of the present invention will be described. The following embodiments are provided for a better understanding of the present invention and the scope of the present invention should not be limited to the following description. It will be clearly appreciated by those skilled in the art that variations and modifications can be made without departing from the scope of the present invention with reference to the specification.

Next, a novel gene targeted-disruption technique, a feature of the present invention, is described.

In one aspect, the present invention provides a method for targeted-disuption of an arbitrary gene in a genome of a living organism. The subject method comprises the steps of: A) providing information of the entire sequence of the genome of the living organism; B) selecting at least one arbitrary region of the sequence; C) providing a vector comprising a sequence complementary to the selected region and a marker gene; D) transforming the living organism with the vector; and E) placing the living organism in a condition allowing to cause homologous recombination. The method is first attained by clarifying the entire genomic sequence, and is different from the conventional technology in that, for example, a model system using *Sulfolobus solfataricus,* by Bartolucci S., cannot disrupt a desired gene, and can merely utilize the result from accidental disruption. In the present invention, this difference has attained effects which can rapidly disrupt a desired gene in an efficient manner, and allow functional anlaysis.

Preferably, in the step B) of the present invention, the region comprises at least two regions. By having two such regions, targeted-disruption of genes by double cross-over may be available. As demonstrated in the present invention, targeted-disruption of a gene by double cross-over is generally more efficient than targeted-disruption of a gene by single cross-over. Accordingly, it is preferable to have two such regions.

Vectors used in the present invention, are also called disruption vectors, and may further comprise an additional gene regulatory element such as a promoter.

The gene targeting method of the present invention may further comprise the step of detecting an expression product of the marker gene. As used herein, the expression product may be for example an mRNA, a polypeptide, or a post-translationally modified polypeptide.

In one embodiment, the marker gene is located in or outside the selected region.

As used herein, the genome used in the present invention, may be any genome as long as the entire genomic sequence is substantially sequenced. Examples of such a genome include, but are not limited to, for example, archeabacteria such as Aeropyrum pernix, Archaeoglobus fulgidus, Methanobacterium thermoautorophicum, Methanococcus jannaschii, Pyurococcus abyssi, Pyrococcus furiosus, Pyrococcus horikoshii, Sulfolobus solfataricus, Sulfolobus tokodaii, Thermoplasma acidophilum, Thermoplasma volcanium; bacteria such as Aquifex aeolicus, Thermotoga maritima, and the like. In one embodiment, the genome used may be the genome of Thermococcus kodakaraensis KOD1, because the entire genome of Thermococcus kodakaraensis KOD1 has now been sequenced. As used herein, that the entire sequence has been sequenced or substantially sequenced, refers to that sequences are clarified so that for any regional sequence selected, a sufficiently homologous region for causing homologous recombination may be provided. Accordingly, it is preferable that the entire sequence is sequenced without lack of a single base, however, it is permissible to have one, two, or three bases unclarified in a sequences. A plurality of such unclarified sequences may be present as long as for any regional sequence selected, a region sufficiently homologous for causing homologous recombination may be provided.

Preferably, the genome of the present invention has a sequence set forth in SEQ ID NO: 1.

Preferably, in the method of the present invention, the above-mentioned region selected, is an open reading frame of SEQ ID NO; 1, which are selected from the group of sequences of gene Numbers (1) to (2151) in the following Table in the sequence of SEQ ID NO: 1, 342, 723, 1087, 1469 or 1838.

TABLE 1

| gene No. | Nucleic acid No. (sense chain (corresponding to SEQ ID NO: 1, 342, 723), starting nucleotide | Nucleic acid No. (sense chain (corresponding to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1 | 1 | 5016 | 2089377 | 2084362 | 2 |
| 2 | 5134 | 5733 | 2084244 | 2083645 | 3 |
| 3 | 6079 | 6543 | 2083299 | 2082835 | 1468 |
| 4 | 6586 | 7014 | 2082792 | 2082364 | 4 |
| 5 | 7152 | 7391 | 2082226 | 2081987 | 1837 |
| 6 | 7399 | 7614 | 2081979 | 2081764 | 1467 |
| 7 | 7655 | 8755 | 2081723 | 2080623 | 2157 |
| 8 | 8843 | 10093 | 2080535 | 2079285 | 343 |
| 9 | 10095 | 10379 | 2079283 | 2078999 | 724 |
| 10 | 10376 | 10807 | 2079002 | 2078571 | 344 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 11 | 10808 | 11416 | 2078570 | 2077962 | 2156 |
| 12 | 11406 | 11726 | 2077972 | 2077652 | 725 |
| 13 | 11723 | 12286 | 2077655 | 2077092 | 345 |
| 14 | 12338 | 13411 | 2077040 | 2075967 | 346 |
| 15 | 13392 | 13841 | 2075986 | 2075537 | 1836 |
| 16 | 13808 | 14056 | 2075570 | 2075322 | 2155 |
| 17 | 14153 | 14896 | 2075225 | 2074482 | 347 |
| 18 | 15239 | 15964 | 2074139 | 2073414 | 348 |
| 19 | 16151 | 16699 | 2073227 | 2072679 | 349 |
| 20 | 16696 | 17697 | 2072682 | 2071681 | 5 |
| 21 | 17780 | 18793 | 2071598 | 2070585 | 2154 |
| 22 | 18786 | 19280 | 2070592 | 2070098 | 1835 |
| 23 | 19290 | 20183 | 2070088 | 2069195 | 1834 |
| 24 | 20183 | 21187 | 2069195 | 2068191 | 2153 |
| 25 | 21266 | 21919 | 2068112 | 2067459 | 2152 |
| 26 | 21913 | 22569 | 2067465 | 2066809 | 1466 |
| 27 | 22597 | 24195 | 2066781 | 2065183 | 1465 |
| 28 | 23947 | 24834 | 2065431 | 2064544 | 6 |
| 29 | 24813 | 25451 | 2064565 | 2063927 | 726 |
| 30 | 25413 | 25811 | 2063965 | 2063567 | 1833 |
| 31 | 25813 | 27396 | 2063565 | 2061982 | 1464 |
| 32 | 27565 | 28620 | 2061813 | 2060758 | 7 |
| 33 | 28591 | 29334 | 2060787 | 2060044 | 1463 |
| 34 | 29782 | 30681 | 2059596 | 2058697 | 8 |
| 35 | 31102 | 31266 | 2058276 | 2058112 | 9 |
| 36 | 31414 | 32235 | 2057964 | 2057143 | 10 |
| 37 | 32367 | 33251 | 2057011 | 2056127 | 727 |
| 38 | 33291 | 35033 | 2056087 | 2054345 | 728 |
| 39 | 35048 | 35824 | 2054330 | 2053554 | 350 |
| 40 | 35882 | 36541 | 2053496 | 2052837 | 351 |
| 41 | 36553 | 37380 | 2052825 | 2051998 | 11 |
| 42 | 37394 | 37870 | 2051984 | 2051508 | 352 |
| 43 | 37874 | 39298 | 2051504 | 2050080 | 353 |
| 44 | 39760 | 40332 | 2049618 | 2049046 | 12 |

(continued)

| gene No. | Nucleic acid No. (sense chain (corresponding to SEQ ID NO: 1, 342, 723), starting nucleotide | Nucleic acid No. (sense chain (corresponding to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 45 | 40360 | 41070 | 2049018 | 2048308 | 13 |
| 46 | 41072 | 42694 | 2048306 | 2046684 | 354 |
| 47 | 42696 | 44444 | 2046682 | 2044934 | 729 |
| 48 | 44441 | 46435 | 2044937 | 2042943 | 355 |
| 49 | 46470 | 46991 | 2042908 | 2042387 | 730 |
| 50 | 47171 | 47416 | 2042207 | 2041962 | 356 |
| 51 | 47317 | 47799 | 2042061 | 2041579 | 14 |
| 52 | 47937 | 49139 | 2041441 | 2040239 | 1832 |
| 53 | 49153 | 49329 | 2040225 | 2040049 | 1462 |
| 54 | 49393 | 49731 | 2039985 | 2039647 | 15 |
| 55 | 49728 | 50297 | 2039650 | 2039081 | 731 |
| 56 | 50278 | 50559 | 2039100 | 2038819 | 1461 |
| 57 | 50693 | 51412 | 2038685 | 2037966 | 357 |
| 58 | 51483 | 52061 | 2037895 | 2037317 | 1831 |
| 59 | 52063 | 52605 | 2037315 | 2036773 | 1460 |
| 60 | 52602 | 53792 | 2036776 | 2035586 | 1830 |
| 61 | 54169 | 55020 | 2035209 | 2034358 | 16 |
| 62 | 55058 | 55606 | 2034320 | 2033772 | 358 |
| 63 | 55746 | 56018 | 2033632 | 2033360 | 732 |
| 64 | 56132 | 56263 | 2033246 | 2033115 | 359 |
| 65 | 56244 | 56708 | 2033134 | 2032670 | 733 |
| 66 | 56674 | 57267 | 2032704 | 2032111 | 17 |
| 67 | 57264 | 57584 | 2032114 | 2031794 | 1829 |
| 68 | 57599 | 58276 | 2031779 | 2031102 | 2151 |
| 69 | 58855 | 59703 | 2030523 | 2029675 | 18 |
| 70 | 59704 | 59868 | 2029674 | 2029510 | 1459 |
| 71 | 59898 | 61799 | 2029480 | 2027579 | 1828 |
| 72 | 62830 | 63723 | 2026548 | 2025655 | 19 |
| 73 | 64226 | 65992 | 2025152 | 2023386 | 360 |
| 74 | 66045 | 67382 | 2023333 | 2021996 | 734 |
| 75 | 67399 | 68973 | 2021979 | 2020405 | 20 |
| 76 | 69117 | 69374 | 2020261 | 2020004 | 735 |
| 77 | 69583 | 69795 | 2019795 | 2019583 | 21 |
| 78 | 69792 | 70511 | 2019586 | 2018867 | 736 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 79 | 70504 | 71112 | 2018874 | 2018266 | 22 |
| 80 | 71117 | 71245 | 2018261 | 2018133 | 361 |
| 81 | 71679 | 72593 | 2017699 | 2016785 | 737 |
| 82 | 72764 | 73339 | 2016614 | 2016039 | 362 |
| 83 | 73336 | 74643 | 2016042 | 2014735 | 23 |
| 84 | 74603 | 75760 | 2014775 | 2013618 | 363 |
| 85 | 75753 | 76025 | 2013625 | 2013353 | 738 |
| 86 | 76022 | 77458 | 2013356 | 2011920 | 364 |
| 87 | 77735 | 79045 | 2011643 | 2010333 | 365 |
| 88 | 79622 | 79726 | 2009756 | 2009652 | 2150 |
| 89 | 79968 | 80129 | 2009410 | 2009249 | 739 |
| 90 | 80246 | 80428 | 2009132 | 2008950 | 366 |
| 91 | 80432 | 83176 | 2008946 | 2006202 | 367 |
| 92 | 83431 | 83628 | 2005947 | 2005750 | 24 |
| 93 | 83908 | 84267 | 2005470 | 2005111 | 25 |
| 94 | 84264 | 84440 | 2005114 | 2004938 | 740 |
| 95 | 84461 | 85018 | 2004917 | 2004360 | 368 |
| 96 | 84999 | 85340 | 2004379 | 2004038 | 741 |
| 97 | 85421 | 85948 | 2003957 | 2003430 | 369 |
| 98 | 86333 | 87139 | 2003045 | 2002239 | 2149 |
| 99 | 87211 | 87663 | 2002167 | 2001715 | 26 |
| 100 | 87663 | 88265 | 2001715 | 2001113 | 742 |
| 101 | 88266 | 89279 | 2001112 | 2000099 | 743 |
| 102 | 89307 | 90059 | 2000071 | 1999319 | 744 |
| 103 | 90079 | 90267 | 1999299 | 1999111 | 27 |
| 104 | 90276 | 90560 | 1999102 | 1998818 | 745 |
| 105 | 90583 | 91056 | 1998795 | 1998322 | 1458 |
| 106 | 91178 | 91366 | 1998200 | 1998012 | 370 |
| 107 | 91363 | 92979 | 1998015 | 1996399 | 28 |
| 108 | 93072 | 94550 | 1996306 | 1994828 | 746 |
| 109 | 94552 | 95712 | 1994826 | 1993666 | 29 |
| 110 | 96185 | 97636 | 1993193 | 1991742 | 371 |
| 111 | 97620 | 98147 | 1991758 | 1991231 | 747 |
| 112 | 98417 | 99583 | 1990961 | 1989795 | 372 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 113 | 99648 | 100892 | 1989730 | 1988486 | 748 |
| 114 | 100915 | 101205 | 1988463 | 1988173 | 1457 |
| 115 | 101224 | 101733 | 1988154 | 1987645 | 1456 |
| 116 | 101796 | 102347 | 1987582 | 1987031 | 749 |
| 117 | 102393 | 102563 | 1986985 | 1986815 | 750 |
| 118 | 102986 | 103432 | 1986392 | 1985946 | 2148 |
| 119 | 103476 | 104318 | 1985902 | 1985060 | 751 |
| 120 | 104398 | 106101 | 1984980 | 1983277 | 30 |
| 121 | 106210 | 106779 | 1983168 | 1982599 | 31 |
| 122 | 106834 | 107454 | 1982544 | 1981924 | 32 |
| 123 | 107637 | 108455 | 1981741 | 1980923 | 752 |
| 124 | 108482 | 109099 | 1980896 | 1980279 | 2147 |
| 125 | 109092 | 111035 | 1980286 | 1978343 | 1827 |
| 126 | 111643 | 113019 | 1977735 | 1976359 | 1455 |
| 127 | 113205 | 114563 | 1976173 | 1974815 | 753 |
| 128 | 114668 | 115351 | 1974710 | 1974027 | 373 |
| 129 | 115397 | 116401 | 1973981 | 1972977 | 374 |
| 130 | 116482 | 116634 | 1972896 | 1972744 | 1454 |
| 131 | 116676 | 117494 | 1972702 | 1971884 | 1826 |
| 132 | 117475 | 118242 | 1971903 | 1971136 | 1453 |
| 133 | 118178 | 118711 | 1971200 | 1970667 | 2146 |
| 134 | 119061 | 119939 | 1970317 | 1969439 | 1825 |
| 135 | 119973 | 120485 | 1969405 | 1968893 | 754 |
| 136 | 120479 | 120952 | 1968899 | 1968426 | 2145 |
| 137 | 121121 | 121192 | 1968257 | 1968186 | 2144 |
| 138 | 121404 | 121856 | 1967974 | 1967522 | 755 |
| 139 | 122007 | 122438 | 1967371 | 1966940 | 756 |
| 140 | 122431 | 122667 | 1966947 | 1966711 | 33 |
| 141 | 122668 | 123594 | 1966710 | 1965784 | 34 |
| 142 | 123578 | 123868 | 1965800 | 1965510 | 2143 |
| 143 | 123932 | 126157 | 1965446 | 1963221 | 2142 |
| 144 | 126306 | 128561 | 1963072 | 1960817 | 757 |
| 145 | 128631 | 130013 | 1960747 | 1959365 | 1824 |
| 146 | 130150 | 131154 | 1959228 | 1958224 | 1452 |

(continued)

| gene No. | Nucleic acid No. (sense chain (corresponding to SEQ ID NO: 1, 342, 723), starting nucleotide | Nucleic acid No. (sense chain (corresponding to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 147 | 131148 | 133049 | 1958230 | 1956329 | 1823 |
| 148 | 132745 | 133890 | 1956633 | 1955488 | 35 |
| 149 | 133885 | 134547 | 1955493 | 1954831 | 1451 |
| 150 | 134544 | 134834 | 1954834 | 1954544 | 1822 |
| 151 | 134978 | 135754 | 1954400 | 1953624 | 2141 |
| 152 | 137477 | 138172 | 1951901 | 1951206 | 2140 |
| 153 | 138521 | 138676 | 1950857 | 1950702 | 2139 |
| 154 | 139365 | 140972 | 1950013 | 1948406 | 758 |
| 155 | 141078 | 141311 | 1948300 | 1948067 | 759 |
| 156 | 141335 | 141856 | 1948043 | 1947522 | 375 |
| 157 | 141853 | 142707 | 1947525 | 1946671 | 1450 |
| 158 | 142732 | 143793 | 1946646 | 1945585 | 1449 |
| 159 | 143756 | 144931 | 1945622 | 1944447 | 2138 |
| 160 | 144924 | 145235 | 1944454 | 1944143 | 1821 |
| 161 | 145334 | 145951 | 1944044 | 1943427 | 376 |
| 162 | 146007 | 146603 | 1943371 | 1942775 | 1820 |
| 163 | 147207 | 149273 | 1942171 | 1940105 | 1819 |
| 164 | 149293 | 149697 | 1940085 | 1939681 | 1448 |
| 165 | 149699 | 150874 | 1939679 | 1938504 | 2137 |
| 166 | 150876 | 151928 | 1938502 | 1937450 | 1818 |
| 167 | 152076 | 152471 | 1937302 | 1936907 | 760 |
| 168 | 152417 | 152743 | 1936961 | 1936635 | 377 |
| 169 | 152801 | 153490 | 1936577 | 1935888 | 2136 |
| 170 | 153487 | 154752 | 1935891 | 1934626 | 1447 |
| 171 | 154844 | 155881 | 1934534 | 1933497 | 2135 |
| 172 | 156044 | 157309 | 1933334 | 1932069 | 378 |
| 173 | 157368 | 158228 | 1932010 | 1931150 | 761 |
| 174 | 158158 | 159018 | 1931220 | 1930360 | 1446 |
| 175 | 158982 | 159464 | 1930396 | 1929914 | 762 |
| 176 | 159517 | 160083 | 1929861 | 1929295 | 1445 |
| 177 | 160206 | 160256 | 1929172 | 1929122 | 763 |
| 178 | 160526 | 160744 | 1928852 | 1928634 | 2134 |
| 179 | 160787 | 161719 | 1928591 | 1927659 | 2133 |
| 180 | 161795 | 163255 | 1927583 | 1926123 | 2132 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 181 | 163362 | 164405 | 1926016 | 1924973 | 764 |
| 182 | 164398 | 165393 | 1924980 | 1923985 | 1444 |
| 183 | 165390 | 167531 | 1923988 | 1921847 | 1817 |
| 184 | 168881 | 170377 | 1920497 | 1919001 | 2131 |
| 185 | 170457 | 171128 | 1918921 | 1918250 | 1816 |
| 186 | 171130 | 171381 | 1918248 | 1917997 | 1443 |
| 187 | 171383 | 172534 | 1917995 | 1916844 | 2130 |
| 188 | 172527 | 173834 | 1916851 | 1915544 | 1815 |
| 189 | 173896 | 173985 | 1915482 | 1915393 | 1442 |
| 190 | 174404 | 174601 | 1914974 | 1914777 | 379 |
| 191 | 174585 | 175349 | 1914793 | 1914029 | 765 |
| 192 | 175740 | 177038 | 1913638 | 1912340 | 1814 |
| 193 | 177138 | 178151 | 1912240 | 1911227 | 766 |
| 194 | 178184 | 178348 | 1911194 | 1911030 | 380 |
| 195 | 178320 | 179039 | 1911058 | 1910339 | 1813 |
| 196 | 179195 | 180553 | 1910183 | 1908825 | 381 |
| 197 | 180543 | 181031 | 1908835 | 1908347 | 1812 |
| 198 | 181028 | 181288 | 1908350 | 1908090 | 2129 |
| 199 | 181345 | 183324 | 1908033 | 1906054 | 1441 |
| 200 | 183436 | 184935 | 1905942 | 1904443 | 1440 |
| 201 | 185362 | 185955 | 1904016 | 1903423 | 1439 |
| 202 | 185988 | 187004 | 1903390 | 1902374 | 1811 |
| 203 | 187111 | 187953 | 1902267 | 1901425 | 1438 |
| 204 | 188074 | 189315 | 1901304 | 1900063 | 36 |
| 205 | 189865 | 190278 | 1899513 | 1899100 | 37 |
| 206 | 190253 | 190621 | 1899125 | 1898757 | 382 |
| 207 | 190630 | 191799 | 1898748 | 1897579 | 1437 |
| 208 | 191874 | 192509 | 1897504 | 1896869 | 767 |
| 209 | 192535 | 192981 | 1896843 | 1896397 | 38 |
| 210 | 192971 | 193486 | 1896407 | 1895892 | 383 |
| 211 | 193701 | 194033 | 1895677 | 1895345 | 1810 |
| 212 | 194152 | 194358 | 1895226 | 1895020 | 1436 |
| 213 | 195097 | 195405 | 1894281 | 1893973 | 39 |
| 214 | 195742 | 195846 | 1893636 | 1893532 | 1435 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 215 | 195995 | 196111 | 1893383 | 1893267 | 384 |
| 216 | 196138 | 196959 | 1893240 | 1892419 | 1434 |
| 217 | 197032 | 197625 | 1892346 | 1891753 | 1433 |
| 218 | 197747 | 198367 | 1891631 | 1891011 | 385 |
| 219 | 198495 | 199754 | 1890883 | 1889624 | 1809 |
| 220 | 199748 | 200686 | 1889630 | 1888692 | 2128 |
| 221 | 200742 | 201098 | 1888636 | 1888280 | 768 |
| 222 | 201067 | 201738 | 1888311 | 1887640 | 40 |
| 223 | 201692 | 202102 | 1887686 | 1887276 | 386 |
| 224 | 202103 | 202924 | 1887275 | 1886454 | 387 |
| 225 | 202929 | 203372 | 1886449 | 1886006 | 769 |
| 226 | 203585 | 204475 | 1885793 | 1884903 | 388 |
| 227 | 204472 | 205083 | 1884906 | 1884295 | 41 |
| 228 | 205070 | 206200 | 1884308 | 1883178 | 389 |
| 229 | 206280 | 206813 | 1883098 | 1882565 | 770 |
| 230 | 206810 | 207397 | 1882568 | 1881981 | 390 |
| 231 | 207399 | 208100 | 1881979 | 1881278 | 771 |
| 232 | 208082 | 208840 | 1881296 | 1880538 | 391 |
| 233 | 208850 | 209479 | 1880528 | 1879899 | 392 |
| 234 | 209476 | 210486 | 1879902 | 1878892 | 42 |
| 235 | 210470 | 211198 | 1878908 | 1878180 | 393 |
| 236 | 211296 | 211982 | 1878082 | 1877396 | 772 |
| 237 | 211979 | 212956 | 1877399 | 1876422 | 394 |
| 238 | 212938 | 214239 | 1876440 | 1875139 | 43 |
| 239 | 214236 | 214814 | 1875142 | 1874564 | 773 |
| 240 | 214807 | 215433 | 1874571 | 1873945 | 44 |
| 241 | 215426 | 216595 | 1873952 | 1872783 | 395 |
| 242 | 216588 | 217343 | 1872790 | 1872035 | 774 |
| 243 | 217325 | 218095 | 1872053 | 1871283 | 2127 |
| 244 | 218020 | 219114 | 1871358 | 1870264 | 1432 |
| 245 | 219077 | 219253 | 1870301 | 1870125 | 2126 |
| 246 | 219407 | 220474 | 1869971 | 1868904 | 2125 |
| 247 | 220471 | 221718 | 1868907 | 1867660 | 1431 |
| 248 | 221676 | 222236 | 1867702 | 1867142 | 1808 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 249 | 222472 | 222852 | 1866906 | 1866526 | 1430 |
| 250 | 222879 | 223259 | 1866499 | 1866119 | 1807 |
| 251 | 223282 | 223923 | 1866096 | 1865455 | 1429 |
| 252 | 223877 | 225022 | 1865501 | 1864356 | 2124 |
| 253 | 224890 | 225804 | 1864488 | 1863574 | 1428 |
| 254 | 225801 | 226844 | 1863577 | 1862534 | 1806 |
| 255 | 226718 | 227377 | 1862660 | 1862001 | 2123 |
| 256 | 227370 | 227741 | 1862008 | 1861637 | 1805 |
| 257 | 227931 | 228242 | 1861447 | 1861136 | 775 |
| 258 | 228257 | 228718 | 1861121 | 1860660 | 396 |
| 259 | 228710 | 229147 | 1860668 | 1860231 | 2122 |
| 260 | 229347 | 229745 | 1860031 | 1859633 | 1804 |
| 261 | 229732 | 230820 | 1859646 | 1858558 | 1427 |
| 262 | 230826 | 231581 | 1858552 | 1857797 | 1803 |
| 263 | 231591 | 232583 | 1857787 | 1856795 | 1802 |
| 264 | 232580 | 233410 | 1856798 | 1855968 | 2121 |
| 265 | 233428 | 233589 | 1855950 | 1855789 | 1426 |
| 266 | 233684 | 234727 | 1855694 | 1854651 | 2120 |
| 267 | 234715 | 235206 | 1854663 | 1854172 | 1425 |
| 268 | 235203 | 236345 | 1854175 | 1853033 | 1801 |
| 269 | 236342 | 237427 | 1853036 | 1851951 | 2119 |
| 270 | 237653 | 238216 | 1851725 | 1851162 | 2118 |
| 271 | 238509 | 239528 | 1850869 | 1849850 | 776 |
| 272 | 239489 | 239686 | 1849889 | 1849692 | 397 |
| 273 | 239677 | 240426 | 1849701 | 1848952 | 1424 |
| 274 | 240560 | 243028 | 1848818 | 1846350 | 398 |
| 275 | 243977 | 244525 | 1845401 | 1844853 | 399 |
| 276 | 244591 | 245055 | 1844787 | 1844323 | 45 |
| 277 | 245052 | 245747 | 1844326 | 1843631 | 777 |
| 278 | 245738 | 246229 | 1843640 | 1843149 | 2117 |
| 279 | 246239 | 246340 | 1843139 | 1843038 | 2116 |
| 280 | 247226 | 248134 | 1842152 | 1841244 | 2115 |
| 281 | 248197 | 249606 | 1841181 | 1839772 | 1423 |
| 282 | 251161 | 251265 | 1838217 | 1838113 | 46 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 283 | 251394 | 251477 | 1837984 | 1837901 | 778 |
| 284 | 251557 | 251760 | 1837821 | 1837618 | 47 |
| 285 | 254653 | 255162 | 1834725 | 1834216 | 1422 |
| 286 | 255227 | 256987 | 1834151 | 1832391 | 2114 |
| 287 | 257124 | 258452 | 1832254 | 1830926 | 1800 |
| 288 | 258556 | 259233 | 1830822 | 1830145 | 1421 |
| 289 | 260703 | 261923 | 1828675 | 1827455 | 779 |
| 290 | 262176 | 262484 | 1827202 | 1826894 | 1799 |
| 291 | 262544 | 263830 | 1826834 | 1825548 | 2113 |
| 292 | 264065 | 265165 | 1825313 | 1824213 | 2112 |
| 293 | 264895 | 266262 | 1824483 | 1823116 | 1420 |
| 294 | 266696 | 266977 | 1822682 | 1822401 | 2111 |
| 295 | 267002 | 268075 | 1822376 | 1821303 | 2110 |
| 296 | 268109 | 269197 | 1821269 | 1820181 | 2109 |
| 297 | 269297 | 270064 | 1820081 | 1819314 | 400 |
| 298 | 270052 | 270306 | 1819326 | 1819072 | 48 |
| 299 | 270301 | 271278 | 1819077 | 1818100 | 1419 |
| 300 | 271361 | 272119 | 1818017 | 1817259 | 401 |
| 301 | 272121 | 272429 | 1817257 | 1816949 | 780 |
| 302 | 272525 | 274057 | 1816853 | 1815321 | 2108 |
| 303 | 274244 | 274963 | 1815134 | 1814415 | 402 |
| 304 | 275340 | 275564 | 1814038 | 1813814 | 781 |
| 305 | 276688 | 277758 | 1812690 | 1811620 | 49 |
| 306 | 277759 | 278526 | 1811619 | 1810852 | 50 |
| 307 | 278454 | 278981 | 1810924 | 1810397 | 782 |
| 308 | 278969 | 279736 | 1810409 | 1809642 | 403 |
| 309 | 279859 | 280521 | 1809519 | 1808857 | 1418 |
| 310 | 280629 | 281072 | 1808749 | 1808306 | 783 |
| 311 | 281104 | 282072 | 1808274 | 1807306 | 51 |
| 312 | 282069 | 282467 | 1807309 | 1806911 | 784 |
| 313 | 282544 | 283272 | 1806834 | 1806106 | 1417 |
| 314 | 283421 | 284416 | 1805957 | 1804962 | 2107 |
| 315 | 284413 | 285099 | 1804965 | 1804279 | 1416 |
| 316 | 285104 | 285292 | 1804274 | 1804086 | 2106 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 317 | 285716 | 286492 | 1803662 | 1802886 | 2105 |
| 318 | 286543 | 287079 | 1802835 | 1802299 | 52 |
| 319 | 287046 | 287645 | 1802332 | 1801733 | 1798 |
| 320 | 287758 | 288153 | 1801620 | 1801225 | 1415 |
| 321 | 288150 | 288437 | 1801228 | 1800941 | 1797 |
| 322 | 288505 | 289047 | 1800873 | 1800331 | 1414 |
| 323 | 289173 | 289493 | 1800205 | 1799885 | 1796 |
| 324 | 289490 | 289948 | 1799888 | 1799430 | 2104 |
| 325 | 290136 | 291029 | 1799242 | 1798349 | 1795 |
| 326 | 290939 | 291157 | 1798439 | 1798221 | 2103 |
| 327 | 291353 | 292696 | 1798025 | 1796682 | 404 |
| 328 | 292703 | 293509 | 1796675 | 1795869 | 405 |
| 329 | 293510 | 293593 | 1795868 | 1795785 | 2102 |
| 330 | 293627 | 294415 | 1795751 | 1794963 | 406 |
| 331 | 294346 | 294663 | 1795032 | 1794715 | 53 |
| 332 | 294750 | 295001 | 1794628 | 1794377 | 785 |
| 333 | 295115 | 296626 | 1794263 | 1792752 | 407 |
| 334 | 296627 | 297139 | 1792751 | 1792239 | 2101 |
| 335 | 297204 | 297731 | 1792174 | 1791647 | 1794 |
| 336 | 297773 | 298702 | 1791605 | 1790676 | 408 |
| 337 | 298699 | 300825 | 1790679 | 1788553 | 54 |
| 338 | 300795 | 301748 | 1788583 | 1787630 | 786 |
| 339 | 301803 | 303251 | 1787575 | 1786127 | 1793 |
| 340 | 303305 | 303766 | 1786073 | 1785612 | 2100 |
| 341 | 303750 | 304688 | 1785628 | 1784690 | 1792 |
| 342 | 304698 | 305126 | 1784680 | 1784252 | 1791 |
| 343 | 305339 | 306193 | 1784039 | 1783185 | 409 |
| 344 | 306190 | 306858 | 1783188 | 1782520 | 55 |
| 345 | 307473 | 307700 | 1781905 | 1781678 | 787 |
| 346 | 308311 | 308886 | 1781067 | 1780492 | 1413 |
| 347 | 308930 | 309406 | 1780448 | 1779972 | 2099 |
| 348 | 309492 | 310637 | 1779886 | 1778741 | 1790 |
| 349 | 310642 | 311016 | 1778736 | 1778362 | 1412 |
| 350 | 311017 | 311625 | 1778361 | 1777753 | 1411 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 351 | 312108 | 312536 | 1777270 | 1776842 | 1789 |
| 352 | 312637 | 312903 | 1776741 | 1776475 | 56 |
| 353 | 312953 | 313306 | 1776425 | 1776072 | 410 |
| 354 | 313344 | 314120 | 1776034 | 1775258 | 788 |
| 355 | 314205 | 314447 | 1775173 | 1774931 | 789 |
| 356 | 314429 | 315589 | 1774949 | 1773789 | 411 |
| 357 | 315618 | 316058 | 1773760 | 1773320 | 1788 |
| 358 | 316245 | 316973 | 1773133 | 1772405 | 1787 |
| 359 | 317124 | 318272 | 1772254 | 1771106 | 790 |
| 360 | 318265 | 319239 | 1771113 | 1770139 | 1410 |
| 361 | 319807 | 319851 | 1769571 | 1769527 | 1409 |
| 362 | 320239 | 320928 | 1769139 | 1768450 | 57 |
| 363 | 321374 | 321511 | 1768004 | 1767867 | 412 |
| 364 | 321508 | 321696 | 1767870 | 1767682 | 58 |
| 365 | 322012 | 322365 | 1767366 | 1767013 | 59 |
| 366 | 322265 | 324256 | 1767113 | 1765122 | 413 |
| 367 | 324261 | 326399 | 1765117 | 1762979 | 791 |
| 368 | 326552 | 326935 | 1762826 | 1762443 | 414 |
| 369 | 327013 | 327282 | 1762365 | 1762096 | 60 |
| 370 | 327284 | 327514 | 1762094 | 1761864 | 415 |
| 371 | 327518 | 328321 | 1761860 | 1761057 | 416 |
| 372 | 328333 | 328815 | 1761045 | 1760563 | 61 |
| 373 | 328812 | 329288 | 1760566 | 1760090 | 792 |
| 374 | 329290 | 330090 | 1760088 | 1759288 | 62 |
| 375 | 330224 | 331687 | 1759154 | 1757691 | 417 |
| 376 | 331691 | 332452 | 1757687 | 1756926 | 418 |
| 377 | 332449 | 332736 | 1756929 | 1756642 | 63 |
| 378 | 334175 | 334945 | 1755203 | 1754433 | 419 |
| 379 | 335068 | 335664 | 1754310 | 1753714 | 64 |
| 380 | 337045 | 337260 | 1752333 | 1752118 | 65 |
| 381 | 337711 | 338295 | 1751667 | 1751083 | 1408 |
| 382 | 339363 | 339788 | 1750015 | 1749590 | 793 |
| 383 | 340641 | 340727 | 1748737 | 1748651 | 794 |
| 384 | 341558 | 341995 | 1747820 | 1747383 | 420 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 385 | 342397 | 343461 | 1746981 | 1745917 | 66 |
| 386 | 343454 | 343891 | 1745924 | 1745487 | 421 |
| 387 | 343888 | 344076 | 1745490 | 1745302 | 67 |
| 388 | 344090 | 344401 | 1745288 | 1744977 | 422 |
| 389 | 345281 | 345472 | 1744097 | 1743906 | 423 |
| 390 | 345566 | 345622 | 1743812 | 1743756 | 2098 |
| 391 | 345615 | 345740 | 1743763 | 1743638 | 795 |
| 392 | 346174 | 346356 | 1743204 | 1743022 | 68 |
| 393 | 346528 | 346881 | 1742850 | 1742497 | 69 |
| 394 | 346606 | 346668 | 1742772 | 1742710 | 1407 |
| 395 | 347138 | 348463 | 1742240 | 1740915 | 424 |
| 396 | 348567 | 350417 | 1740811 | 1738961 | 1786 |
| 397 | 350537 | 351598 | 1738841 | 1737780 | 425 |
| 398 | 351592 | 352155 | 1737786 | 1737223 | 70 |
| 399 | 352419 | 352985 | 1736959 | 1736393 | 796 |
| 400 | 353923 | 354102 | 1735455 | 1735276 | 71 |
| 401 | 354174 | 355334 | 1735204 | 1734044 | 797 |
| 402 | 355393 | 355872 | 1733985 | 1733506 | 72 |
| 403 | 355856 | 356452 | 1733522 | 1732926 | 2097 |
| 404 | 356449 | 357381 | 1732929 | 1731997 | 1406 |
| 405 | 357378 | 358037 | 1732000 | 1731341 | 1785 |
| 406 | 358034 | 359329 | 1731344 | 1730049 | 2096 |
| 407 | 359407 | 360171 | 1729971 | 1729207 | 73 |
| 408 | 360168 | 361466 | 1729210 | 1727912 | 798 |
| 409 | 361497 | 363407 | 1727881 | 1725971 | 799 |
| 410 | 366699 | 367151 | 1722679 | 1722227 | 1784 |
| 411 | 367290 | 368240 | 1722088 | 1721138 | 1783 |
| 412 | 368237 | 369289 | 1721141 | 1720089 | 2095 |
| 413 | 370634 | 371449 | 1718744 | 1717929 | 426 |
| 414 | 371481 | 372920 | 1717897 | 1716458 | 800 |
| 415 | 374488 | 374550 | 1714890 | 1714828 | 74 |
| 416 | 374583 | 374840 | 1714795 | 1714538 | 801 |
| 417 | 374833 | 375534 | 1714545 | 1713844 | 1405 |
| 418 | 375535 | 376308 | 1713843 | 1713070 | 1404 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 419 | 376000 | 376092 | 1713378 | 1713286 | 75 |
| 420 | 376298 | 376771 | 1713080 | 1712607 | 2094 |
| 421 | 379177 | 380310 | 1710201 | 1709068 | 1403 |
| 422 | 380366 | 381109 | 1709012 | 1708269 | 2093 |
| 423 | 381111 | 382313 | 1708267 | 1707065 | 1782 |
| 424 | 382310 | 382675 | 1707068 | 1706703 | 2092 |
| 425 | 382850 | 383839 | 1706528 | 1705539 | 2091 |
| 426 | 384244 | 384471 | 1705134 | 1704907 | 1402 |
| 427 | 384528 | 385040 | 1704850 | 1704338 | 1781 |
| 428 | 385030 | 386139 | 1704348 | 1703239 | 1401 |
| 429 | 389056 | 390132 | 1700322 | 1699246 | 1400 |
| 430 | 390129 | 391328 | 1699249 | 1698050 | 1780 |
| 431 | 391570 | 392187 | 1697808 | 1697191 | 1399 |
| 432 | 392614 | 393321 | 1696764 | 1696057 | 1398 |
| 433 | 393449 | 394750 | 1695929 | 1694628 | 427 |
| 434 | 394894 | 398109 | 1694484 | 1691269 | 76 |
| 435 | 398178 | 398471 | 1691200 | 1690907 | 1779 |
| 436 | 398502 | 399011 | 1690876 | 1690367 | 802 |
| 437 | 399050 | 404185 | 1690328 | 1685193 | 428 |
| 438 | 404484 | 405290 | 1684894 | 1684088 | 803 |
| 439 | 405419 | 405631 | 1683959 | 1683747 | 2090 |
| 440 | 405628 | 405963 | 1683750 | 1683415 | 1397 |
| 441 | 405960 | 406709 | 1683418 | 1682669 | 1778 |
| 442 | 406835 | 408055 | 1682543 | 1681323 | 429 |
| 443 | 408052 | 408807 | 1681326 | 1680571 | 77 |
| 444 | 408809 | 409462 | 1680569 | 1679916 | 430 |
| 445 | 409459 | 409647 | 1679919 | 1679731 | 78 |
| 446 | 409647 | 410459 | 1679731 | 1678919 | 804 |
| 447 | 410460 | 411080 | 1678918 | 1678298 | 805 |
| 448 | 411176 | 411688 | 1678202 | 1677690 | 431 |
| 449 | 411878 | 413293 | 1677500 | 1676085 | 432 |
| 450 | 413415 | 413915 | 1675963 | 1675463 | 806 |
| 451 | 413926 | 414252 | 1675452 | 1675126 | 79 |
| 452 | 414877 | 415209 | 1674501 | 1674169 | 80 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 453 | 417109 | 417270 | 1672269 | 1672108 | 81 |
| 454 | 417291 | 417929 | 1672087 | 1671449 | 807 |
| 455 | 418636 | 419175 | 1670742 | 1670203 | 82 |
| 456 | 419247 | 420563 | 1670131 | 1668815 | 808 |
| 457 | 420627 | 422132 | 1668751 | 1667246 | 809 |
| 458 | 422333 | 422719 | 1667045 | 1666659 | 433 |
| 459 | 422876 | 424030 | 1666502 | 1665348 | 2089 |
| 460 | 426547 | 426711 | 1662831 | 1662667 | 83 |
| 461 | 426747 | 427742 | 1662631 | 1661636 | 810 |
| 462 | 427799 | 429064 | 1661579 | 1660314 | 434 |
| 463 | 429065 | 430390 | 1660313 | 1658988 | 2088 |
| 464 | 430394 | 430633 | 1658984 | 1658745 | 2087 |
| 465 | 430618 | 430785 | 1658760 | 1658593 | 1396 |
| 466 | 430883 | 432259 | 1658495 | 1657119 | 2086 |
| 467 | 432397 | 432738 | 1656981 | 1656640 | 84 |
| 468 | 432751 | 433449 | 1656627 | 1655929 | 85 |
| 469 | 433446 | 434621 | 1655932 | 1654757 | 1777 |
| 470 | 434530 | 435735 | 1654848 | 1653643 | 86 |
| 471 | 435779 | 436300 | 1653599 | 1653078 | 2085 |
| 472 | 436300 | 436812 | 1653078 | 1652566 | 1395 |
| 473 | 437409 | 438209 | 1651969 | 1651169 | 811 |
| 474 | 438222 | 439658 | 1651156 | 1649720 | 1776 |
| 475 | 439696 | 440403 | 1649682 | 1648975 | 1394 |
| 476 | 440578 | 441444 | 1648800 | 1647934 | 87 |
| 477 | 441511 | 441882 | 1647867 | 1647496 | 88 |
| 478 | 441887 | 442267 | 1647491 | 1647111 | 435 |
| 479 | 442358 | 442873 | 1647020 | 1646505 | 436 |
| 480 | 442922 | 444142 | 1646456 | 1645236 | 437 |
| 481 | 444220 | 444681 | 1645158 | 1644697 | 89 |
| 482 | 444972 | 445310 | 1644406 | 1644068 | 812 |
| 483 | 446197 | 448899 | 1643181 | 1640479 | 1393 |
| 484 | 448945 | 450294 | 1640433 | 1639084 | 1392 |
| 485 | 450481 | 450996 | 1638897 | 1638382 | 90 |
| 486 | 451077 | 451238 | 1638301 | 1638140 | 813 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 487 | 451250 | 451597 | 1638128 | 1637781 | 438 |
| 488 | 452770 | 453123 | 1636608 | 1636255 | 91 |
| 489 | 453183 | 454601 | 1636195 | 1634777 | 814 |
| 490 | 454835 | 455341 | 1634543 | 1634037 | 439 |
| 491 | 455338 | 455502 | 1634040 | 1633876 | 92 |
| 492 | 456330 | 456662 | 1633048 | 1632716 | 815 |
| 493 | 456623 | 456835 | 1632755 | 1632543 | 440 |
| 494 | 456838 | 457587 | 1632540 | 1631791 | 93 |
| 495 | 457618 | 458184 | 1631760 | 1631194 | 94 |
| 496 | 458476 | 459126 | 1630902 | 1630252 | 95 |
| 497 | 459138 | 459680 | 1630240 | 1629698 | 1775 |
| 498 | 459718 | 460674 | 1629660 | 1628704 | 96 |
| 499 | 460667 | 461935 | 1628711 | 1627443 | 2084 |
| 500 | 462618 | 463808 | 1626760 | 1625570 | 1774 |
| 501 | 464266 | 464421 | 1625112 | 1624957 | 1391 |
| 502 | 464460 | 464972 | 1624918 | 1624406 | 1773 |
| 503 | 465336 | 466562 | 1624042 | 1622816 | 816 |
| 504 | 466632 | 466847 | 1622746 | 1622531 | 1772 |
| 505 | 466975 | 467631 | 1622403 | 1621747 | 97 |
| 506 | 467628 | 468806 | 1621750 | 1620572 | 1771 |
| 507 | 471018 | 472637 | 1618360 | 1616741 | 1770 |
| 508 | 472691 | 474145 | 1616687 | 1615233 | 2083 |
| 509 | 474239 | 475240 | 1615139 | 1614138 | 441 |
| 510 | 475250 | 475708 | 1614128 | 1613670 | 442 |
| 511 | 475702 | 477042 | 1613676 | 1612336 | 98 |
| 512 | 477049 | 477657 | 1612329 | 1611721 | 99 |
| 513 | 477738 | 478031 | 1611640 | 1611347 | 817 |
| 514 | 477971 | 479050 | 1611407 | 1610328 | 2082 |
| 515 | 478881 | 479639 | 1610497 | 1609739 | 818 |
| 516 | 479629 | 480162 | 1609749 | 1609216 | 1390 |
| 517 | 480198 | 480755 | 1609180 | 1608623 | 1769 |
| 518 | 480843 | 481127 | 1608535 | 1608251 | 1768 |
| 519 | 481315 | 482679 | 1608063 | 1606699 | 100 |
| 520 | 484981 | 485445 | 1604397 | 1603933 | 101 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 521 | 485442 | 486008 | 1603936 | 1603370 | 1767 |
| 522 | 486065 | 486484 | 1603313 | 1602894 | 443 |
| 523 | 486481 | 488979 | 1602897 | 1600399 | 1389 |
| 524 | 489517 | 490644 | 1599861 | 1598734 | 1388 |
| 525 | 490744 | 491844 | 1598634 | 1597534 | 102 |
| 526 | 491922 | 493376 | 1597456 | 1596002 | 819 |
| 527 | 493561 | 495408 | 1595817 | 1593970 | 103 |
| 528 | 495410 | 496480 | 1593968 | 1592898 | 444 |
| 529 | 497090 | 499186 | 1592288 | 1590192 | 445 |
| 530 | 499596 | 499949 | 1589782 | 1589429 | 1766 |
| 531 | 500938 | 501252 | 1588440 | 1588126 | 1387 |
| 532 | 501249 | 501479 | 1588129 | 1587899 | 1765 |
| 533 | 501658 | 502464 | 1587720 | 1586914 | 1386 |
| 534 | 502547 | 502792 | 1586831 | 1586586 | 2081 |
| 535 | 502785 | 502967 | 1586593 | 1586411 | 1764 |
| 536 | 503187 | 503354 | 1586191 | 1586024 | 820 |
| 537 | 504971 | 505099 | 1584407 | 1584279 | 446 |
| 538 | 506242 | 506664 | 1583136 | 1582714 | 1385 |
| 539 | 507506 | 507592 | 1581872 | 1581786 | 447 |
| 540 | 508803 | 509420 | 1580575 | 1579958 | 1763 |
| 541 | 510163 | 510879 | 1579215 | 1578499 | 1384 |
| 542 | 511923 | 512477 | 1577455 | 1576901 | 1762 |
| 543 | 513104 | 513481 | 1576274 | 1575897 | 448 |
| 544 | 513710 | 514261 | 1575668 | 1575117 | 2080 |
| 545 | 514843 | 515223 | 1574535 | 1574155 | 1383 |
| 546 | 515543 | 515791 | 1573835 | 1573587 | 2079 |
| 547 | 517003 | 517803 | 1572375 | 1571575 | 1382 |
| 548 | 517805 | 518281 | 1571573 | 1571097 | 2078 |
| 549 | 518278 | 518760 | 1571100 | 1570618 | 1381 |
| 550 | 518772 | 519575 | 1570606 | 1569803 | 1761 |
| 551 | 519579 | 519809 | 1569799 | 1569569 | 1760 |
| 552 | 520158 | 520541 | 1569220 | 1568837 | 1759 |
| 553 | 520694 | 522628 | 1568684 | 1566750 | 2077 |
| 554 | 522837 | 524828 | 1566541 | 1564550 | 1758 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 555 | 524728 | 525042 | 1564650 | 1564336 | 1380 |
| 556 | 525397 | 525585 | 1563981 | 1563793 | 1379 |
| 557 | 525884 | 526483 | 1563494 | 1562895 | 2076 |
| 558 | 527199 | 527468 | 1562179 | 1561910 | 821 |
| 559 | 527689 | 528324 | 1561689 | 1561054 | 104 |
| 560 | 528364 | 528969 | 1561014 | 1560409 | 105 |
| 561 | 528984 | 529217 | 1560394 | 1560161 | 822 |
| 562 | 529214 | 529528 | 1560164 | 1559850 | 449 |
| 563 | 529509 | 529739 | 1559869 | 1559639 | 823 |
| 564 | 529736 | 529981 | 1559642 | 1559397 | 450 |
| 565 | 529978 | 530385 | 1559400 | 1558993 | 106 |
| 566 | 530659 | 532146 | 1558719 | 1557232 | 107 |
| 567 | 532123 | 532530 | 1557255 | 1556848 | 1378 |
| 568 | 532615 | 533754 | 1556763 | 1555624 | 108 |
| 569 | 533789 | 534916 | 1555589 | 1554462 | 451 |
| 570 | 534917 | 535363 | 1554461 | 1554015 | 2075 |
| 571 | 535366 | 536694 | 1554012 | 1552684 | 1377 |
| 572 | 536818 | 536871 | 1552560 | 1552507 | 1376 |
| 573 | 536998 | 537846 | 1552380 | 1551532 | 109 |
| 574 | 537847 | 538209 | 1551531 | 1551169 | 110 |
| 575 | 538230 | 539297 | 1551148 | 1550081 | 824 |
| 576 | 539304 | 540950 | 1550074 | 1548428 | 825 |
| 577 | 540986 | 541681 | 1548392 | 1547697 | 452 |
| 578 | 541671 | 542294 | 1547707 | 1547084 | 826 |
| 579 | 542291 | 542914 | 1547087 | 1546464 | 453 |
| 580 | 542904 | 545159 | 1546474 | 1544219 | 827 |
| 581 | 545191 | 545688 | 1544187 | 1543690 | 111 |
| 582 | 545706 | 546455 | 1543672 | 1542923 | 828 |
| 583 | 546468 | 547502 | 1542910 | 1541876 | 829 |
| 584 | 547499 | 547759 | 1541879 | 1541619 | 454 |
| 585 | 547830 | 548183 | 1541548 | 1541195 | 830 |
| 586 | 548218 | 548553 | 1541160 | 1540825 | 112 |
| 587 | 548531 | 549514 | 1540847 | 1539864 | 455 |
| 588 | 549515 | 549850 | 1539863 | 1539528 | 456 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 589 | 550080 | 551150 | 1539298 | 1538228 | 831 |
| 590 | 551249 | 552460 | 1538129 | 1536918 | 457 |
| 591 | 552309 | 553043 | 1537069 | 1536335 | 832 |
| 592 | 553133 | 553699 | 1536245 | 1535679 | 458 |
| 593 | 553745 | 554734 | 1535633 | 1534644 | 2074 |
| 594 | 554855 | 555676 | 1534523 | 1533702 | 459 |
| 595 | 555783 | 556910 | 1533595 | 1532468 | 1757 |
| 596 | 556879 | 558105 | 1532499 | 1531273 | 1375 |
| 597 | 558125 | 558196 | 1531253 | 1531182 | 2073 |
| 598 | 558864 | 559322 | 1530514 | 1530056 | 1756 |
| 599 | 559506 | 560798 | 1529872 | 1528580 | 833 |
| 600 | 560838 | 562364 | 1528540 | 1527014 | 834 |
| 601 | 562361 | 563395 | 1527017 | 1525983 | 460 |
| 602 | 563371 | 564303 | 1526007 | 1525075 | 113 |
| 603 | 564310 | 565311 | 1525068 | 1524067 | 1374 |
| 604 | 565409 | 567541 | 1523969 | 1521837 | 461 |
| 605 | 567556 | 567786 | 1521822 | 1521592 | 1373 |
| 606 | 567865 | 568512 | 1521513 | 1520866 | 1372 |
| 607 | 568711 | 570129 | 1520667 | 1519249 | 114 |
| 608 | 570172 | 570729 | 1519206 | 1518649 | 1371 |
| 609 | 570898 | 570957 | 1518480 | 1518421 | 115 |
| 610 | 571031 | 571738 | 1518347 | 1517640 | 462 |
| 611 | 571735 | 572070 | 1517643 | 1517308 | 1370 |
| 612 | 572149 | 574656 | 1517229 | 1514722 | 1369 |
| 613 | 574653 | 575411 | 1514725 | 1513967 | 1755 |
| 614 | 575490 | 576503 | 1513888 | 1512875 | 1754 |
| 615 | 576540 | 577586 | 1512838 | 1511792 | 1753 |
| 616 | 577750 | 578565 | 1511628 | 1510813 | 116 |
| 617 | 578612 | 579025 | 1510766 | 1510353 | 463 |
| 618 | 579392 | 579454 | 1509986 | 1509924 | 464 |
| 619 | 580461 | 580553 | 1508917 | 1508825 | 1752 |
| 620 | 581070 | 581168 | 1508308 | 1508210 | 1751 |
| 621 | 582573 | 583445 | 1506805 | 1505933 | 1750 |
| 622 | 583582 | 585228 | 1505796 | 1504150 | 1368 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 623 | 585396 | 586382 | 1503982 | 1502996 | 835 |
| 624 | 587383 | 587667 | 1501995 | 1501711 | 1367 |
| 625 | 588220 | 589968 | 1501158 | 1499410 | 1366 |
| 626 | 590029 | 591039 | 1499349 | 1498339 | 1365 |
| 627 | 591078 | 592301 | 1498300 | 1497077 | 1749 |
| 628 | 592190 | 593191 | 1497188 | 1496187 | 465 |
| 629 | 593214 | 593957 | 1496164 | 1495421 | 836 |
| 630 | 593914 | 594495 | 1495464 | 1494883 | 117 |
| 631 | 594739 | 594795 | 1494639 | 1494583 | 1364 |
| 632 | 595329 | 595610 | 1494049 | 1493768 | 837 |
| 633 | 595427 | 597550 | 1493951 | 1491828 | 466 |
| 634 | 597520 | 597798 | 1491858 | 1491580 | 1363 |
| 635 | 598695 | 599399 | 1490683 | 1489979 | 1748 |
| 636 | 599396 | 600097 | 1489982 | 1489281 | 2072 |
| 637 | 600094 | 600945 | 1489284 | 1488433 | 1362 |
| 638 | 600958 | 600999 | 1488420 | 1488379 | 1361 |
| 639 | 601388 | 601828 | 1487990 | 1487550 | 467 |
| 640 | 601912 | 602571 | 1487466 | 1486807 | 1360 |
| 641 | 602643 | 603974 | 1486735 | 1485404 | 1747 |
| 642 | 603976 | 605406 | 1485402 | 1483972 | 1359 |
| 643 | 605506 | 605823 | 1483872 | 1483555 | 118 |
| 644 | 605856 | 606749 | 1483522 | 1482629 | 1746 |
| 645 | 606746 | 607678 | 1482632 | 1481700 | 2071 |
| 646 | 607678 | 608625 | 1481700 | 1480753 | 1358 |
| 647 | 608720 | 609349 | 1480658 | 1480029 | 468 |
| 648 | 609665 | 611200 | 1479713 | 1478178 | 469 |
| 649 | 611281 | 612924 | 1478097 | 1476454 | 119 |
| 650 | 612921 | 613868 | 1476457 | 1475510 | 838 |
| 651 | 613855 | 614616 | 1475523 | 1474762 | 120 |
| 652 | 614613 | 615374 | 1474765 | 1474004 | 839 |
| 653 | 615379 | 616116 | 1473999 | 1473262 | 121 |
| 654 | 616117 | 616626 | 1473261 | 1472752 | 1357 |
| 655 | 616713 | 617375 | 1472665 | 1472003 | 840 |
| 656 | 617430 | 618005 | 1471948 | 1471373 | 1745 |

(continued)

| gene No. | Nucleic acid No. (sense chain (corresponding to SEQ ID NO: 1, 342, 723), starting nucleotide | Nucleic acid No. (sense chain (corresponding to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 657 | 617873 | 619891 | 1471505 | 1469487 | 2070 |
| 658 | 619888 | 620115 | 1469490 | 1469263 | 1356 |
| 659 | 620116 | 620346 | 1469262 | 1469032 | 1355 |
| 660 | 620526 | 621581 | 1468852 | 1467797 | 841 |
| 661 | 621554 | 622366 | 1467824 | 1467012 | 470 |
| 662 | 622338 | 623402 | 1467040 | 1465976 | 842 |
| 663 | 623814 | 624353 | 1465564 | 1465025 | 1.744 |
| 664 | 624301 | 624510 | 1465077 | 1464868 | 1354 |
| 665 | 624735 | 625205 | 1464643 | 1464173 | 1743 |
| 666 | 625223 | 625891 | 1464155 | 1463487 | 471 |
| 667 | 625916 | 626170 | 1463462 | 1463208 | 472 |
| 668 | 626202 | 626936 | 1463176 | 1462442 | 1742 |
| 669 | 626909 | 627853 | 1462469 | 1461525 | 2069 |
| 670 | 627832 | 628989 | 1461546 | 1460389 | 1353 |
| 671 | 629061 | 629687 | 1460317 | 1459691 | 1741 |
| 672 | 629684 | 631024 | 1459694 | 1458354 | 2068 |
| 673 | 631021 | 631839 | 1458357 | 1457539 | 1352 |
| 674 | 631871 | 632350 | 1457507 | 1457028 | 473 |
| 675 | 632430 | 632630 | 1456948 | 1456748 | 843 |
| 676 | 632617 | 633099 | 1456761 | 1456279 | 122 |
| 677 | 633112 | 633933 | 1456266 | 1455445 | 123 |
| 678 | 633964 | 634764 | 1455414 | 1454614 | 124 |
| 679 | 634815 | 635330 | 1454563 | 1454048 | 1740 |
| 680 | 635934 | 636071 | 1453444 | 1453307 | 1739 |
| 681 | 637143 | 637451 | 1452235 | 1451927 | 844 |
| 682 | 637487 | 638062 | 1451891 | 1451316 | 474 |
| 683 | 638134 | 639000 | 1451244 | 1450378 | 1351 |
| 684 | 639553 | 639651 | 1449825 | 1449727 | 125 |
| 685 | 639626 | 640396 | 1449752 | 1448982 | 2067 |
| 686 | 640393 | 641181 | 1448985 | 1448197 | 1350 |
| 687 | 641204 | 641923 | 1448174 | 1447455 | 2066 |
| 688 | 641972 | 642490 | 1447406 | 1446888 | 475 |
| 689 | 642511 | 643098 | 1446867 | 1446280 | 1349 |
| 690 | 643209 | 643670 | 1446169 | 1445708 | 845 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 691 | 644598 | 646496 | 1444780 | 1442882 | 1738 |
| 692 | 647573 | 650017 | 1441805 | 1439361 | 476 |
| 693 | 650078 | 650584 | 1439300 | 1438794 | 477 |
| 694 | 650587 | 651087 | 1438791 | 1438291 | 126 |
| 695 | 651198 | 652340 | 1438180 | 1437038 | 846 |
| 696 | 652343 | 653548 | 1437035 | 1435830 | 2065 |
| 697 | 653784 | 655079 | 1435594 | 1434299 | 847 |
| 698 | 655937 | 657688 | 1433441 | 1431690 | 2064 |
| 699 | 657722 | 658642 | 1431656 | 1430736 | 2063 |
| 700 | 658773 | 659825 | 1430605 | 1429553 | 1737 |
| 701 | 659850 | 660155 | 1429528 | 1429223 | 1736 |
| 702 | 660246 | 664418 | 1429132 | 1424960 | 848 |
| 703 | 664498 | 665586 | 1424880 | 1423792 | 127 |
| 704 | 665627 | 665995 | 1423751 | 1423383 | 478 |
| 705 | 666332 | 666616 | 1423046 | 1422762 | 2062 |
| 706 | 666618 | 667169 | 1422760 | 1422209 | 1735 |
| 707 | 667123 | 667176 | 1422255 | 1422202 | 128 |
| 708 | 667218 | 667724 | 1422160 | 1421654 | 1734 |
| 709 | 667824 | 669488 | 1421554 | 1419890 | 849 |
| 710 | 669735 | 671918 | 1419643 | 1417460 | 850 |
| 711 | 673707 | 673985 | 1415671 | 1415393 | 851 |
| 712 | 674033 | 674911 | 1415345 | 1414467 | 479 |
| 713 | 674957 | 675970 | 1414421 | 1413408 | 480 |
| 714 | 676425 | 677294 | 1412953 | 1412084 | 852 |
| 715 | 677302 | 678150 | 1412076 | 1411228 | 1348 |
| 716 | 678143 | 679063 | 1411235 | 1410315 | 2061 |
| 717 | 679100 | 679813 | 1410278 | 1409565 | 2060 |
| 718 | 679850 | 679924 | 1409528 | 1409454 | 481 |
| 719 | 680156 | 680470 | 1409222 | 1408908 | 482 |
| 720 | 680606 | 681754 | 1408772 | 1407624 | 483 |
| 721 | 682401 | 682496 | 1406977 | 1406882 | 853 |
| 722 | 682446 | 682799 | 1406932 | 1406579 | 1733 |
| 723 | 682717 | 684711 | 1406661 | 1404667 | 129 |
| 724 | 684698 | 685174 | 1404680 | 1404204 | 2059 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 725 | 686253 | 686873 | 1403125 | 1402505 | 1732 |
| 726 | 686863 | 687633 | 1402515 | 1401745 | 1347 |
| 727 | 687638 | 688447 | 1401740 | 1400931 | 2058 |
| 728 | 688516 | 689571 | 1400862 | 1399807 | 130 |
| 729 | 689568 | 690029 | 1399810 | 1399349 | 854 |
| 730 | 690316 | 690513 | 1399062 | 1398865 | 1346 |
| 731 | 690550 | 691353 | 1398828 | 1398025 | 1345 |
| 732 | 691387 | 692820 | 1397991 | 1396558 | 1344 |
| 733 | 692817 | 694928 | 1396561 | 1394450 | 1731 |
| 734 | 694986 | 695405 | 1394392 | 1393973 | 1730 |
| 735 | 695410 | 696654 | 1393968 | 1392724 | 1343 |
| 736 | 696651 | 697808 | 1392727 | 1391570 | 1729 |
| 737 | 697801 | 699510 | 1391577 | 1389868 | 1342 |
| 738 | 699507 | 700274 | 1389871 | 1389104 | 1728 |
| 739 | 700228 | 701004 | 1389150 | 1388374 | 1341 |
| 740 | 701037 | 701399 | 1388341 | 1387979 | 1727 |
| 741 | 701550 | 702359 | 1387828 | 1387019 | 855 |
| 742 | 702356 | 703177 | 1387022 | 1386201 | 484 |
| 743 | 703152 | 703868 | 1386226 | 1385510 | 856 |
| 744 | 703837 | 705249 | 1385541 | 1384129 | 1340 |
| 745 | 705309 | 706460 | 1384069 | 1382918 | 857 |
| 746 | 706455 | 706655 | 1382923 | 1382723 | 1726 |
| 747 | 706739 | 708556 | 1382639 | 1380822 | 485 |
| 748 | 708558 | 711569 | 1380820 | 1377809 | 858 |
| 749 | 711859 | 712440 | 1377519 | 1376938 | 131 |
| 750 | 712445 | 713191 | 1376933 | 1376187 | 2057 |
| 751 | 713142 | 713633 | 1376236 | 1375745 | 859 |
| 752 | 713693 | 714955 | 1375685 | 1374423 | 2056 |
| 753 | 715024 | 715470 | 1374354 | 1373908 | 1339 |
| 754 | 715543 | 716427 | 1373835 | 1372951 | 1338 |
| 755 | 716424 | 718136 | 1372954 | 1371242 | 1725 |
| 756 | 718317 | 719339 | 1371061 | 1370039 | 860 |
| 757 | 719507 | 719788 | 1369871 | 1369590 | 486 |
| 758 | 719790 | 720593 | 1369588 | 1368785 | 1724 |

(continued)

| gene No. | Nucleic acid No. (sense chain (corresponding to SEQ ID NO: 1, 342, 723), starting nucleotide | Nucleic acid No. (sense chain (corresponding to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 759 | 720689 | 721426 | 1368689 | 1367952 | 2055 |
| 760 | 721789 | 722304 | 1367589 | 1367074 | 132 |
| 761 | 722344 | 722481 | 1367034 | 1366897 | 1337 |
| 762 | 722592 | 723116 | 1366786 | 1366262 | 861 |
| 763 | 723142 | 724314 | 1366236 | 1365064 | 1336 |
| 764 | 724419 | 725573 | 1364959 | 1363805 | 1723 |
| 765 | 725704 | 726249 | 1363674 | 1363129 | 133 |
| 766 | 726458 | 726643 | 1362920 | 1362735 | 487 |
| 767 | 728745 | 728798 | 1360633 | 1360580 | 862 |
| 768 | 729082 | 729786 | 1360296 | 1359592 | 1335 |
| 769 | 729844 | 730989 | 1359534 | 1358389 | 134 |
| 770 | 730961 | 731485 | 1358417 | 1357893 | 488 |
| 771 | 731586 | 733985 | 1357792 | 1355393 | 863 |
| 772 | 734016 | 734336 | 1355362 | 1355042 | 864 |
| 773 | 734349 | 734939 | 1355029 | 1354439 | 1722 |
| 774 | 735215 | 735760 | 1354163 | 1353618 | 489 |
| 775 | 735762 | 735941 | 1353616 | 1353437 | 865 |
| 776 | 735965 | 737146 | 1353413 | 1352232 | 2054 |
| 777 | 737210 | 737683 | 1352168 | 1351695 | 490 |
| 778 | 737822 | 739696 | 1351556 | 1349682 | 2053 |
| 779 | 739687 | 740523 | 1349691 | 1348855 | 1334 |
| 780 | 740584 | 741294 | 1348794 | 1348084 | 135 |
| 781 | 741329 | 741541 | 1348049 | 1347837 | 491 |
| 782 | 741920 | 742084 | 1347458 | 1347294 | 492 |
| 783 | 742684 | 743376 | 1346694 | 1346002 | 136 |
| 784 | 743424 | 743609 | 1345954 | 1345769 | 866 |
| 785 | 743587 | 744603 | 1345791 | 1344775 | 1333 |
| 786 | 744560 | 745372 | 1344818 | 1344006 | 493 |
| 787 | 745369 | 746826 | 1344009 | 1342552 | 137 |
| 788 | 746823 | 747761 | 1342555 | 1341617 | 1721 |
| 789 | 747766 | 748353 | 1341612 | 1341025 | 1332 |
| 790 | 748338 | 749033 | 1341040 | 1340345 | 1720 |
| 791 | 749030 | 749443 | 1340348 | 1339935 | 2052 |
| 792 | 749440 | 749877 | 1339938 | 1339501 | 1331 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 793 | 750208 | 750714 | 1339170 | 1338664 | 1330 |
| 794 | 751954 | 752967 | 1337424 | 1336411 | 138 |
| 795 | 753046 | 754110 | 1336332 | 1335268 | 139 |
| 796 | 754166 | 755410 | 1335212 | 1333968 | 2051 |
| 797 | 755496 | 756431 | 1333882 | 1332947 | 867 |
| 798 | 756477 | 756968 | 1332901 | 1332410 | 868 |
| 799 | 756958 | 757629 | 1332420 | 1331749 | 1329 |
| 800 | 757712 | 758458 | 1331666 | 1330920 | 2050 |
| 801 | 758689 | 759645 | 1330689 | 1329733 | 140 |
| 802 | 759762 | 760691 | 1329616 | 1328687 | 869 |
| 803 | 760688 | 761674 | 1328690 | 1327704 | 2049 |
| 804 | 762327 | 763418 | 1327051 | 1325960 | 870 |
| 805 | 763396 | 764058 | 1325982 | 1325320 | 141 |
| 806 | 765200 | 765316 | 1324178 | 1324062 | 2048 |
| 807 | 765637 | 766047 | 1323741 | 1323331 | 142 |
| 808 | 766138 | 766683 | 1323240 | 1322695 | 143 |
| 809 | 766685 | 767974 | 1322693 | 1321404 | 494 |
| 810 | 767976 | 768434 | 1321402 | 1320944 | 871 |
| 811 | 768477 | 769343 | 1320901 | 1320035 | 872 |
| 812 | 769459 | 769962 | 1319919 | 1319416 | 144 |
| 813 | 769950 | 771269 | 1319428 | 1318109 | 873 |
| 814 | 771283 | 771807 | 1318095 | 1317571 | 1328 |
| 815 | 771820 | 773541 | 1317558 | 1315837 | 145 |
| 816 | 773543 | 774817 | 1315835 | 1314561 | 495 |
| 817 | 774838 | 775089 | 1314540 | 1314289 | 146 |
| 818 | 775493 | 776422 | 1313885 | 1312956 | 496 |
| 819 | 776480 | 777643 | 1312898 | 1311735 | 497 |
| 820 | 778176 | 778346 | 1311202 | 1311032 | 874 |
| 821 | 778362 | 779411 | 1311016 | 1309967 | 875 |
| 822 | 779336 | 780247 | 1310042 | 1309131 | 498 |
| 823 | 780438 | 782276 | 1308940 | 1307102 | 876 |
| 824 | 782329 | 783108 | 1307049 | 1306270 | 147 |
| 825 | 783098 | 784927 | 1306280 | 1304451 | 2047 |
| 826 | 785382 | 786104 | 1303996 | 1303274 | 1719 |

(continued)

| gene No. | Nucleic acid No. (sense chain (corresponding to SEQ ID NO: 1, 342, 723), starting nucleotide | Nucleic acid No. (sense chain (corresponding to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 827 | 786218 | 786838 | 1303160 | 1302540 | 2046 |
| 828 | 786930 | 787286 | 1302448 | 1302092 | 1718 |
| 829 | 787283 | 787609 | 1302095 | 1301769 | 2045 |
| 830 | 787749 | 788930 | 1301629 | 1300448 | 1717 |
| 831 | 788975 | 789268 | 1300403 | 1300110 | 499 |
| 832 | 789317 | 789460 | 1300061 | 1299918 | 2044 |
| 833 | 789852 | 790022 | 1299526 | 1299356 | 1716 |
| 834 | 790438 | 791058 | 1298940 | 1298320 | 1327 |
| 835 | 790672 | 790737 | 1298706 | 1298641 | 148 |
| 836 | 791117 | 792469 | 1298261 | 1296909 | 500 |
| 837 | 792505 | 792675 | 1296873 | 1296703 | 149 |
| 838 | 792665 | 793114 | 1296713 | 1296264 | 501 |
| 839 | 793111 | 795000 | 1296267 | 1294378 | 150 |
| 840 | 795038 | 795544 | 1294340 | 1293834 | 502 |
| 841 | 796310 | 797536 | 1293068 | 1291842 | 2043 |
| 842 | 797552 | 798316 | 1291826 | 1291062 | 2042 |
| 843 | 798473 | 799534 | 1290905 | 1289844 | 503 |
| 844 | 799610 | 799858 | 1289768 | 1289520 | 504 |
| 845 | 799848 | 800327 | 1289530 | 1289051 | 877 |
| 846 | 800324 | 800425 | 1289054 | 1288953 | 2041 |
| 847 | 800450 | 800518 | 1288928 | 1288860 | 2040 |
| 848 | 800919 | 802424 | 1288459 | 1286954 | 878 |
| 849 | 802436 | 802672 | 1286942 | 1286706 | 505 |
| 850 | 802669 | 802890 | 1286709 | 1286488 | 151 |
| 851 | 802887 | 803297 | 1286491 | 1286081 | 879 |
| 852 | 803294 | 805027 | 1286084 | 1284351 | 506 |
| 853 | 805220 | 806068 | 1284158 | 1283310 | 507 |
| 854 | 806024 | 807415 | 1283354 | 1281963 | 2039 |
| 855 | 807366 | 808745 | 1282012 | 1280633 | 880 |
| 856 | 808746 | 809576 | 1280632 | 1279802 | 1715 |
| 857 | 810847 | 811266 | 1278531 | 1278112 | 1326 |
| 858 | 811367 | 811606 | 1278011 | 1277772 | 508 |
| 859 | 811608 | 812351 | 1277770 | 1277027 | 881 |
| 860 | 812635 | 813648 | 1276743 | 1275730 | 152 |

(continued)

| gene No. | Nucleic acid No. (sense chain (corresponding to SEQ ID NO: 1, 342, 723), starting nucleotide | Nucleic acid No. (sense chain (corresponding to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 861 | 813652 | 814113 | 1275726 | 1275265 | 153 |
| 862 | 814077 | 816419 | 1275301 | 1272959 | 882 |
| 863 | 816501 | 816650 | 1272877 | 1272728 | 883 |
| 864 | 816754 | 817728 | 1272624 | 1271650 | 154 |
| 865 | 817725 | 818519 | 1271653 | 1270859 | 884 |
| 866 | 818623 | 819468 | 1270755 | 1269910 | 155 |
| 867 | 819475 | 820395 | 1269903 | 1268983 | 156 |
| 868 | 820410 | 821180 | 1268968 | 1268198 | 1714 |
| 869 | 821146 | 822570 | 1268232 | 1266808 | 1325 |
| 870 | 822810 | 823514 | 1266568 | 1265864 | 1713 |
| 871 | 823599 | 824021 | 1265779 | 1265357 | 885 |
| 872 | 824015 | 825196 | 1265363 | 1264182 | 2038 |
| 873 | 825266 | 826294 | 1264112 | 1263084 | 2037 |
| 874 | 826379 | 827413 | 1262999 | 1261965 | 2036 |
| 875 | 827435 | 828904 | 1261943 | 1260474 | 2035 |
| 876 | 828985 | 829728 | 1260393 | 1259650 | 1324 |
| 877 | 829725 | 830471 | 1259653 | 1258907 | 1712 |
| 878 | 830551 | 832368 | 1258827 | 1257010 | 157 |
| 879 | 832337 | 833035 | 1257041 | 1256343 | 509 |
| 880 | 836010 | 837260 | 1253368 | 1252118 | 1711 |
| 881 | 837335 | 837601 | 1252043 | 1251777 | 2034 |
| 882 | 837647 | 839638 | 1251731 | 1249740 | 2033 |
| 883 | 839649 | 839885 | 1249729 | 1249493 | 1710 |
| 884 | 840097 | 840471 | 1249281 | 1248907 | 158 |
| 885 | 840503 | 841321 | 1248875 | 1248057 | 510 |
| 886 | 841293 | 842288 | 1248085 | 1247090 | 886 |
| 887 | 842275 | 842628 | 1247103 | 1246750 | 159 |
| 888 | 842986 | 844059 | 1246392 | 1245319 | 1323 |
| 889 | 844320 | 844517 | 1245058 | 1244861 | 1709 |
| 890 | 844597 | 845652 | 1244781 | 1243726 | 1322 |
| 891 | 845725 | 846387 | 1243653 | 1242991 | 160 |
| 892 | 846422 | 846727 | 1242956 | 1242651 | 511 |
| 893 | 846773 | 847903 | 1242605 | 1241475 | 512 |
| 894 | 847896 | 848990 | 1241482 | 1240388 | 887 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 895 | 848774 | 848884 | 1240604 | 1240494 | 2032 |
| 896 | 848987 | 849100 | 1240391 | 1240278 | 2031 |
| 897 | 849375 | 849638 | 1240003 | 1239740 | 1708 |
| 898 | 849669 | 851036 | 1239709 | 1238342 | 1707 |
| 899 | 851134 | 851325 | 1238244 | 1238053 | 1321 |
| 900 | 851346 | 851582 | 1238032 | 1237796 | 1706 |
| 901 | 851738 | 854035 | 1237640 | 1235343 | 513 |
| 902 | 851818 | 851883 | 1237560 | 1237495 | 1320 |
| 903 | 854126 | 855841 | 1235252 | 1233537 | 514 |
| 904 | 855888 | 856652 | 1233490 | 1232726 | 888 |
| 905 | 856637 | 856798 | 1232741 | 1232580 | 2030 |
| 906 | 857151 | 858227 | 1232227 | 1231151 | 889 |
| 907 | 858728 | 858934 | 1230650 | 1230444 | 515 |
| 908 | 860080 | 860340 | 1229298 | 1229038 | 161 |
| 909 | 860404 | 861084 | 1228974 | 1228294 | 1319 |
| 910 | 861133 | 862545 | 1228245 | 1226833 | 1318 |
| 911 | 862729 | 864021 | 1226649 | 1225357 | 1317 |
| 912 | 864121 | 864819 | 1225257 | 1224559 | 1316 |
| 913 | 865002 | 865454 | 1224376 | 1223924 | 890 |
| 914 | 865387 | 866304 | 1223991 | 1223074 | 162 |
| 915 | 866496 | 868313 | 1222882 | 1221065 | 891 |
| 916 | 868296 | 868430 | 1221082 | 1220948 | 1705 |
| 917 | 868444 | 870222 | 1220934 | 1219156 | 163 |
| 918 | 870263 | 870547 | 1219115 | 1218831 | 516 |
| 919 | 870532 | 870840 | 1218846 | 1218538 | 164 |
| 920 | 870842 | 871846 | 1218536 | 1217532 | 517 |
| 921 | 871836 | 872120 | 1217542 | 1217258 | 892 |
| 922 | 871942 | 872775 | 1217436 | 1216603 | 165 |
| 923 | 872833 | 873117 | 1216545 | 1216261 | 166 |
| 924 | 873524 | 874306 | 1215854 | 1215072 | 518 |
| 925 | 874707 | 874940 | 1214671 | 1214438 | 893 |
| 926 | 875022 | 875840 | 1214356 | 1213538 | 894 |
| 927 | 875837 | 876856 | 1213541 | 1212522 | 2029 |
| 928 | 877020 | 877235 | 1212358 | 1212143 | 895 |

(continued)

| gene No. | Nucleic acid No. (sense chain (corresponding to SEQ ID NO: 1, 342, 723), starting nucleotide | Nucleic acid No. (sense chain (corresponding to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 929 | 877271 | 878197 | 1212107 | 1211181 | 519 |
| 930 | 878209 | 878658 | 1211169 | 1210720 | 1315 |
| 931 | 878718 | 878765 | 1210660 | 1210613 | 896 |
| 932 | 878886 | 879182 | 1210492 | 1210196 | 897 |
| 933 | 879211 | 880500 | 1210167 | 1208878 | 167 |
| 934 | 880506 | 881387 | 1208872 | 1207991 | 898 |
| 935 | 881550 | 881654 | 1207828 | 1207724 | 899 |
| 936 | 882812 | 882925 | 1206566 | 1206453 | 2028 |
| 937 | 885694 | 886539 | 1203684 | 1202839 | 1314 |
| 938 | 886567 | 887178 | 1202811 | 1202200 | 1313 |
| 939 | 887275 | 887487 | 1202103 | 1201891 | 168 |
| 940 | 887717 | 887920 | 1201661 | 1201458 | 520 |
| 941 | 887924 | 890701 | 1201454 | 1198677 | 521 |
| 942 | 891114 | 891398 | 1198264 | 1197980 | 900 |
| 943 | 891434 | 895009 | 1197944 | 1194369 | 522 |
| 944 | 895013 | 895678 | 1194365 | 1193700 | 523 |
| 945 | 895675 | 896097 | 1193703 | 1193281 | 1312 |
| 946 | 896626 | 899040 | 1192752 | 1190338 | 169 |
| 947 | 899156 | 900004 | 1190222 | 1189374 | 2027 |
| 948 | 900134 | 900385 | 1189244 | 1188993 | 524 |
| 949 | 901696 | 902574 | 1187682 | 1186804 | 1311 |
| 950 | 902700 | 903458 | 1186678 | 1185920 | 1704 |
| 951 | 903912 | 904115 | 1185466 | 1185263 | 1703 |
| 952 | 904127 | 904555 | 1185251 | 1184823 | 2026 |
| 953 | 904610 | 905026 | 1184768 | 1184352 | 525 |
| 954 | 905105 | 906898 | 1184273 | 1182480 | 526 |
| 955 | 906982 | 907974 | 1182396 | 1181404 | 170 |
| 956 | 907975 | 908217 | 1181403 | 1181161 | 1310 |
| 957 | 908370 | 909260 | 1181008 | 1180118 | 1702 |
| 958 | 909301 | 910116 | 1180077 | 1179262 | 171 |
| 959 | 910097 | 910516 | 1179281 | 1178862 | 527 |
| 960 | 910513 | 912024 | 1178865 | 1177354 | 172 |
| 961 | 912021 | 912893 | 1177357 | 1176485 | 1701 |
| 962 | 912890 | 914188 | 1176488 | 1175190 | 2025 |

(continued)

| gene No. | Nucleic acid No. (sense chain (corresponding to SEQ ID NO: 1, 342, 723), starting nucleotide | Nucleic acid No. (sense chain (corresponding to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 963 | 914305 | 914493 | 1175073 | 1174885 | 173 |
| 964 | 914711 | 915121 | 1174667 | 1174257 | 528 |
| 965 | 915118 | 916428 | 1174260 | 1172950 | 174 |
| 966 | 916589 | 917257 | 1172789 | 1172121 | 529 |
| 967 | 917348 | 918352 | 1172030 | 1171026 | 530 |
| 968 | 918655 | 918705 | 1170723 | 1170673 | 1309 |
| 969 | 918719 | 919171 | 1170659 | 1170207 | 2024 |
| 970 | 919305 | 923264 | 1170073 | 1166114 | 901 |
| 971 | 924116 | 924814 | 1165262 | 1164564 | 2023 |
| 972 | 925010 | 927244. | 1164368 | 1162134 | 531 |
| 973 | 927249 | 927578 | 1162129 | 1161800 | 1700 |
| 974 | 928257 | 929309 | 1161121 | 1160069 | 1699 |
| 975 | 929424 | 929705 | 1159954 | 1159673 | 1698 |
| 976 | 930480 | 931013 | 1158898 | 1158365 | 1697 |
| 977 | 931103 | 931576 | 1158275 | 1157802 | 532 |
| 978 | 931594 | 932070 | 1157784 | 1157308 | 175 |
| 979 | 932526 | 933086 | 1156852 | 1156292 | 902 |
| 980 | 933128 | 933430 | 1156250 | 1155948 | 533 |
| 981 | 933728 | 933904 | 1155650 | 1155474 | 534 |
| 982 | 933919 | 934392 | 1155459 | 1154986 | 1308 |
| 983 | 934564 | 935379 | 1154814 | 1153999 | 176 |
| 984 | 935513 | 936664 | 1153865 | 1152714 | 2022 |
| 985 | 936666 | 936944 | 1152712 | 1152434 | 1696 |
| 986 | 936987 | 938822 | 1152391 | 1150556 | 1695 |
| 987 | 938954 | 940192 | 1150424 | 1149186 | 535 |
| 988 | 940239 | 940469 | 1149139 | 1148909 | 903 |
| 989 | 940803 | 940937 | 1148575 | 1148441 | 904 |
| 990 | 940934 | 942055 | 1148444 | 1147323 | 536 |
| 991 | 942591 | 942917 | 1146787 | 1146461 | 905 |
| 992 | 942914 | 943306 | 1146464 | 1146072 | 2021 |
| 993 | 943357 | 943545 | 1146021 | 1145833 | 1307 |
| 994 | 943533 | 943778 | 1145845 | 1145600 | 1694 |
| 995 | 943889 | 944536 | 1145489 | 1144842 | 2020 |
| 996 | 944542 | 944994 | 1144836 | 1144384 | 1306 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 997 | 944996 | 945436 | 1144382 | 1143942 | 2019 |
| 998 | 945433 | 945741 | 1143945 | 1143637 | 1305 |
| 999 | 945755 | 946939 | 1143623 | 1142439 | 2018 |
| 1000 | 946932 | 948164 | 1142446 | 1141214 | 1693 |
| 1001 | 948079 | 949662 | 1141299 | 1139716 | 1304 |
| 1002 | 949659 | 953030 | 1139719 | 1136348 | 1692 |
| 1003 | 953048 | 953296 | 1136330 | 1136082 | 2017 |
| 1004 | 953495 | 954190 | 1135883 | 1135188 | 2016 |
| 1005 | 954301 | 955020 | 1135077 | 1134358 | 177 |
| 1006 | 955204 | 956391 | 1134174 | 1132987 | 178 |
| 1007 | 956375 | 956533 | 1133003 | 1132845 | 2015 |
| 1008 | 957270 | 957638 | 1132108 | 1131740 | 906 |
| 1009 | 957640 | 961329 | 1131738 | 1128049 | 1303 |
| 1010 | 961407 | 962324 | 1127971 | 1127054 | 907 |
| 1011 | 962372 | 962575 | 1127006 | 1126803 | 537 |
| 1012 | 962593 | 963804 | 1126785 | 1125574 | 1302 |
| 1013 | 964168 | 964827 | 1125210 | 1124551 | 179 |
| 1014 | 964831 | 965430 | 1124547 | 1123948 | 1301 |
| 1015 | 965603 | 965896 | 1123775 | 1123482 | 538 |
| 1016 | 965901 | 966098 | 1123477 | 1123280 | 908 |
| 1017 | 966166 | 967002 | 1123212 | 1122376 | 180 |
| 1018 | 967002 | 967181 | 1122376 | 1122197 | 909 |
| 1019 | 967184 | 967987 | 1122194 | 1121391 | 539 |
| 1020 | 968134 | 968757 | 1121244 | 1120621 | 181 |
| 1021 | 968754 | 969002 | 1120624 | 1120376 | 910 |
| 1022 | 968995 | 969663 | 1120383 | 1119715 | 182 |
| 1023 | 969660 | 970463 | 1119718 | 1118915 | 911 |
| 1024 | 970555 | 971892 | 1118823 | 1117486 | 183 |
| 1025 | 971952 | 973340 | 1117426 | 1116038 | 1691 |
| 1026 | 973366 | 974772 | 1116012 | 1114606 | 1300 |
| 1027 | 974823 | 976277 | 1114555 | 1113101 | 1690 |
| 1028 | 976234 | 976803 | 1113144 | 1112575 | 1299 |
| 1029 | 976871 | 977053 | 1112507 | 1112325 | 2014 |
| 1030 | 977082 | 977765 | 1112296 | 1111613 | 1689 |

(continued)

| gene No. | Nucleic acid No. (sense chain (corresponding to SEQ ID NO: 1, 342, 723), starting nucleotide | Nucleic acid No. (sense chain (corresponding to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1031 | 977762 | 978706 | 1111616 | 1110672 | 2013 |
| 1032 | 978776 | 979747 | 1110602 | 1109631 | 540 |
| 1033 | 979826 | 981100 | 1109552 | 1108278 | 541 |
| 1034 | 981159 | 981425 | 1108219 | 1107953 | 1688 |
| 1035 | 981762 | 981815 | 1107616 | 1107563 | 1687 |
| 1036 | 982136 | 982483 | 1107242 | 1106895 | 542 |
| 1037 | 982480 | 982953 | 1106898 | 1106425 | 1298 |
| 1038 | 983025 | 983486 | 1106353 | 1105892 | 912 |
| 1039 | 983483 | 983821 | 1105895 | 1105557 | 543 |
| 1040 | 983802 | 984371 | 1105576 | 1105007 | 1686 |
| 1041 | 984359 | 985399 | 1105019 | 1103979 | 2012 |
| 1042 | 985204 | 986352 | 1104174 | 1103026 | 1297 |
| 1043 | 986349 | 986912 | 1103029 | 1102466 | 1685 |
| 1044 | 986851 | 987246 | 1102527 | 1102132 | 1296 |
| 1045 | 987243 | 987566 | 1102135 | 1101812 | 1684 |
| 1046 | 987517 | 988383 | 1101861 | 1100995 | 1295 |
| 1047 | 988383 | 989573 | 1100995 | 1099805 | 1683 |
| 1048 | 989577 | 989894 | 1099801 | 1099484 | 1682 |
| 1049 | 990762 | 991511 | 1098616 | 1097867 | 913 |
| 1050 | 991803 | 991991 | 1097575 | 1097387 | 914 |
| 1051 | 992036 | 993010 | 1097342 | 1096368 | 2011 |
| 1052 | 994241 | 995020 | 1095137 | 1094358 | 544 |
| 1053 | 995047 | 995112 | 1094331 | 1094266 | 184 |
| 1054 | 995380 | 995844 | 1093998 | 1093534 | 185 |
| 1055 | 995878 | 996558 | 1093500 | 1092820 | 1294 |
| 1056 | 997037 | 998464 | 1092341 | 1090914 | 545 |
| 1057 | 998525 | 999265 | 1090853 | 1090113 | 2010 |
| 1058 | 999750 | 1000229 | 1089628 | 1089149 | 915 |
| 1059 | 1000226 | 1001212 | 1089152 | 1088166 | 546 |
| 1060 | 1001217 | 1001987 | 1088161 | 1087391 | 916 |
| 1061 | 1002002 | 1003240 | 1087376 | 1086138 | 2009 |
| 1062 | 1003253 | 1005466 | 1086125 | 1083912 | 547 |
| 1063 | 1005467 | 1006087 | 1083911 | 1083291 | 2008 |
| 1064 | 1006202 | 1007890 | 1083176 | 1081488 | 2007 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1065 | 1007979 | 1010192 | 1081399 | 1079186 | 1681 |
| 1066 | 1010189 | 1010956 | 1079189 | 1078422 | 2006 |
| 1067 | 1011011 | 1011949 | 1078367 | 1077429 | 2005 |
| 1068 | 1012013 | 1012879 | 1077365 | 1076499 | 548 |
| 1069 | 1012961 | 1013278 | 1076417 | 1076100 | 549 |
| 1070 | 1013371 | 1013883 | 1076007 | 1075495 | 186 |
| 1071 | 1013995 | 1014411 | 1075383 | 1074967 | 1293 |
| 1072 | 1014829 | 1017228 | 1074549 | 1072150 | 187 |
| 1073 | 1017331 | 1020711 | 1072047 | 1068667 | 188 |
| 1074 | 1020821 | 1020970 | 1068557 | 1068408 | 2004 |
| 1075 | 1021424 | 1022338 | 1067954 | 1067040 | 550 |
| 1076 | 1022319 | 1023311 | 1067059 | 1066067 | 1680 |
| 1077 | 1023301 | 1023780 | 1066077 | 1065598 | 1292 |
| 1078 | 1023781 | 1024785 | 1065597 | 1064593 | 1291 |
| 1079 | 1024877 | 1025692 | 1064501 | 1063686 | 551 |
| 1080 | 1025682 | 1026086 | 1063696 | 1063292 | 1679 |
| 1081 | 1026083 | 1026376 | 1063295 | 1063002 | 2003 |
| 1082 | 1026357 | 1026986 | 1063021 | 1062392 | 1678 |
| 1083 | 1026983 | 1027579 | 1062395 | 1061799 | 2002 |
| 1084 | 1027657 | 1029558 | 1061721 | 1059820 | 189 |
| 1085 | 1029517 | 1030068 | 1059861 | 1059310 | 1290 |
| 1086 | 1030276 | 1030950 | 1059102 | 1058428 | 1289 |
| 1087 | 1031013 | 1031807 | 1058365 | 1057571 | 1677 |
| 1088 | 1031814 | 1032344 | 1057564 | 1057034 | 1676 |
| 1089 | 1032406 | 1032792 | 1056972 | 1056586 | 190 |
| 1090 | 1032841 | 1034373 | 1056537 | 1055005 | 191 |
| 1091 | 1034458 | 1035498 | 1054920 | 1053880 | 192 |
| 1092 | 1035541 | 1036101 | 1053837 | 1053277 | 193 |
| 1093 | 1036098 | 1036649 | 1053280 | 1052729 | 917 |
| 1094 | 1036636 | 1037469 | 1052742 | 1051909 | 194 |
| 1095 | 1037390 | 1038229 | 1051988 | 1051149 | 2001 |
| 1096 | 1038226 | 1039704 | 1051152 | 1049674 | 1288 |
| 1097 | 1039796 | 1040683 | 1049582 | 1048695 | 552 |
| 1098 | 1041012 | 1041071 | 1048366 | 1048307 | 918 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1099 | 1041624 | 1041935 | 1047754 | 1047443 | 919 |
| 1100 | 1042133 | 1042384 | 1047245 | 1046994 | 553 |
| 1101 | 1042526 | 1043701 | 1046852 | 1045677 | 554 |
| 1102 | 1043676 | 1044812 | 1045702 | 1044566 | 1675 |
| 1103 | 1044809 | 1046068 | 1044569 | 1043310 | 2000 |
| 1104 | 1047016 | 1048092 | 1042362 | 1041286 | 195 |
| 1105 | 1048209 | 1048610 | 1041169 | 1040768 | 1674 |
| 1106 | 1048684 | 1048761 | 1040694 | 1040617 | 1287 |
| 1107 | 1048718 | 1049599 | 1040660 | 1039779 | 555 |
| 1108 | 1049596 | 1051275 | 1039782 | 1038103 | 1286 |
| 1109 | 1051307 | 1051711 | 1038071 | 1037667 | 1999 |
| 1110 | 1051708 | 1051995 | 1037670 | 1037383 | 1285 |
| 1111 | 1052192 | 1052701 | 1037186 | 1036677 | 556 |
| 1112 | 1052753 | 1053022 | 1036625 | 1036356 | 557 |
| 1113 | 1053032 | 1053793 | 1036346 | 1035585 | 558 |
| 1114 | 1053859 | 1055274 | 1035519 | 1034104 | 196 |
| 1115 | 1055358 | 1055663 | 1034020 | 1033715 | 920 |
| 1116 | 1056285 | 1056395 | 1033093 | 1032983 | 921 |
| 1117 | 1056392 | 1057381 | 1032986 | 1031997 | 1998 |
| 1118 | 1057362 | 1057835 | 1032016 | 1031543 | 1673 |
| 1119 | 1057832 | 1058302 | 1031546 | 1031076 | 1997 |
| 1120 | 1058495 | 1059043 | 1030883 | 1030335 | 559 |
| 1121 | 1059047 | 1059307 | 1030331 | 1030071 | 1996 |
| 1122 | 1059399 | 1059863 | 1029979 | 1029515 | 1672 |
| 1123 | 1059921 | 1060517 | 1029457 | 1028861 | 922 |
| 1124 | 1060582 | 1061310 | 1028796 | 1028068 | 197 |
| 1125 | 1061307 | 1061768 | 1028071 | 1027610 | 1671 |
| 1126 | 1061878 | 1063221 | 1027500 | 1026157 | 198 |
| 1127 | 1063298 | 1064599 | 1026080 | 1024779 | 560 |
| 1128 | 1064656 | 1065000 | 1024722 | 1024378 | 1284 |
| 1129 | 1065370 | 1066023 | 1024008 | 1023355 | 1283 |
| 1130 | 1066020 | 1067213 | 1023358 | 1022165 | 1670 |
| 1131 | 1067215 | 1067811 | 1022163 | 1021567 | 1282 |
| 1132 | 1067793 | 1068392 | 1021585 | 1020986 | 1669 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1133 | 1068394 | 1069287 | 1020984 | 1020091 | 1281 |
| 1134 | 1069288 | 1071138 | 1020090 | 1018240 | 1280 |
| 1135 | 1070858 | 1070965 | 1018520 | 1018413 | 561 |
| 1136 | 1071135 | 1072622 | 1018243 | 1016756 | 1668 |
| 1137 | 1072619 | 1072963 | 1016759 | 1016415 | 1995 |
| 1138 | 1072960 | 1073688 | 1016418 | 1015690 | 1279 |
| 1139 | 1073670 | 1073954 | 1015708 | 1015424 | 1667 |
| 1140 | 1073951 | 1074343 | 1015427 | 1015035 | 1994 |
| 1141 | 1074340 | 1074594 | 1015038 | 1014784 | 1278 |
| 1142 | 1074591 | 1075124 | 1014787 | 1014254 | 1666 |
| 1143 | 1075360 | 1075860 | 1014018 | 1013518 | 1277 |
| 1144 | 1076013 | 1077278 | 1013365 | 1012100 | 923 |
| 1145 | 1077432 | 1077986 | 1011946 | 1011392 | 924 |
| 1146 | 1078071 | 1079189 | 1011307 | 1010189 | 1665 |
| 1147 | 1079201 | 1080472 | 1010177 | 1008906 | 1993 |
| 1148 | 1080723 | 1081862 | 1008655 | 1007516 | 925 |
| 1149 | 1082285 | 1084639 | 1007093 | 1004739 | 562 |
| 1150 | 1082363 | 1082779 | 1007015 | 1006599 | 1992 |
| 1151 | 1084640 | 1085716 | 1004738 | 1003662 | 1991 |
| 1152 | 1085820 | 1086698 | 1003558 | 1002680 | 926 |
| 1153 | 1086762 | 1086986 | 1002616 | 1002392 | 927 |
| 1154 | 1087256 | 1088512 | 1002122 | 1000866 | 1990 |
| 1155 | 1088568 | 1088813 | 1000810 | 1000565 | 1664 |
| 1156 | 1088815 | 1089384 | 1000563 | 999994 | 1276 |
| 1157 | 1089160 | 1089210 | 1000218 | 1000168 | 199 |
| 1158 | 1089484 | 1089639 | 999894 | 999739 | 1275 |
| 1159 | 1089909 | 1090604 | 999469 | 998774 | 1663 |
| 1160 | 1091118 | 1091525 | 998260 | 997853 | 1662 |
| 1161 | 1091646 | 1092197 | 997732 | 997181 | 928 |
| 1162 | 1092206 | 1093522 | 997172 | 995856 | 1989 |
| 1163 | 1093556 | 1093957 | 995822 | 995421 | 1988 |
| 1164 | 1093967 | 1095127 | 995411 | 994251 | 1987 |
| 1165 | 1096375 | 1096839 | 993003 | 992539 | 200 |
| 1166 | 1096870 | 1098303 | 992508 | 991075 | 201 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1167 | 1098281 | 1098538 | 991097 | 990840 | 563 |
| 1168 | 1098554 | 1099156 | 990824 | 990222 | 564 |
| 1169 | 1099220 | 1099486 | 990158 | 989892 | 565 |
| 1170 | 1099468 | 1099908 | 989910 | 989470 | 202 |
| 1171 | 1099954 | 1100991 | 989424 | 988387 | 203 |
| 1172 | 1101073 | 1101510 | 988305 | 987868 | 1274 |
| 1173 | 1101868 | 1102326 | 987510 | 987052 | 1273 |
| 1174 | 1102786 | 1103181 | 986592 | 986197 | 1272 |
| 1175 | 1103673 | 1104461 | 985705 | 984917 | 1661 |
| 1176 | 1104585 | 1106492 | 984793 | 982886 | 929 |
| 1177 | 1106686 | 1107264 | 982692 | 982114 | 1271 |
| 1178 | 1107524 | 1108015 | 981854 | 981363 | 1986 |
| 1179 | 1108559 | 1110253 | 980819 | 979125 | 1985 |
| 1180 | 1110347 | 1111819 | 979031 | 977559 | 566 |
| 1181 | 1111862 | 1112080 | 977516 | 977298 | 1984 |
| 1182 | 1112624 | 1113001 | 976754 | 976377 | 1983 |
| 1183 | 1113459 | 1114217 | 975919 | 975161 | 930 |
| 1184 | 1114407 | 1117082 | 974971 | 972296 | 931 |
| 1185 | 1117577 | 1118029 | 971801 | 971349 | 567 |
| 1186 | 1118086 | 1119738 | 971292 | 969640 | 1270 |
| 1187 | 1119840 | 1120178 | 969538 | 969200 | 932 |
| 1188 | 1120172 | 1120504 | 969206 | 968874 | 568 |
| 1189 | 1120505 | 1121407 | 968873 | 967971 | 569 |
| 1190 | 1121408 | 1122520 | 967970 | 966858 | 1982 |
| 1191 | 1122517 | 1123746 | 966861 | 965632 | 1269 |
| 1192 | 1123810 | 1124472 | 965568 | 964906 | 204 |
| 1193 | 1124569 | 1125114 | 964809 | 964264 | 1268 |
| 1194 | 1125170 | 1125637 | 964208 | 963741 | 1981 |
| 1195 | 1125727 | 1126902 | 963651 | 962476 | 205 |
| 1196 | 1128262 | 1128495 | 961116 | 960883 | 1267 |
| 1197 | 1128535 | 1128972 | 960843 | 960406 | 1266 |
| 1198 | 1129034 | 1130476 | 960344 | 958902 | 1980 |
| 1199 | 1130532 | 1131944 | 958846 | 957434 | 1660 |
| 1200 | 1132006 | 1132422 | 957372 | 956956 | 1265 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1201 | 1132432 | 1132659 | 956946 | 956719 | 1264 |
| 1202 | 1132744 | 1135125 | 956634 | 954253 | 1263 |
| 1203 | 1135154 | 1135213 | 954224 | 954165 | 570 |
| 1204 | 1135255 | 1137741 | 954123 | 951637 | 1262 |
| 1205 | 1138634 | 1138867 | 950744 | 950511 | 571 |
| 1206 | 1139159 | 1142494 | 950219 | 946884 | 572 |
| 1207 | 1142537 | 1142836 | 946841 | 946542 | 573 |
| 1208 | 1142873 | 1144054 | 946505 | 945324 | 574 |
| 1209 | 1144054 | 1145121 | 945324 | 944257 | 206 |
| 1210 | 1145177 | 1146514 | 944201 | 942864 | 575 |
| 1211 | 1146553 | 1148040 | 942825 | 941338 | 207 |
| 1212 | 1148086 | 1149231 | 941292 | 940147 | 208 |
| 1213 | 1150093 | 1151094 | 939285 | 938284 | 209 |
| 1214 | 1151091 | 1154534 | 938287 | 934844 | 1659 |
| 1215 | 1155108 | 1155464 | 934270 | 933914 | 933 |
| 1216 | 1155466 | 1155999 | 933912 | 933379 | 1261 |
| 1217 | 1157418 | 1157627 | 931960 | 931751 | 1658 |
| 1218 | 1157624 | 1157836 | 931754 | 931542 | 1979 |
| 1219 | 1157916 | 1158293 | 931462 | 931085 | 1657 |
| 1220 | 1158361 | 1159554 | 931017 | 929824 | 1260 |
| 1221 | 1159686 | 1160306 | 929692 | 929072 | 1656 |
| 1222 | 1161299 | 1161634 | 928079 | 927744 | 1978 |
| 1223 | 1161690 | 1163606 | 927688 | 925772 | 1655 |
| 1224 | 1163703 | 1164656 | 925675 | 924722 | 934 |
| 1225 | 1164663 | 1165082 | 924715 | 924296 | 935 |
| 1226 | 1165121 | 1165714 | 924257 | 923664 | 576 |
| 1227 | 1165724 | 1165948 | 923654 | 923430 | 577 |
| 1228 | 1165959 | 1166231 | 923419 | 923147 | 936 |
| 1229 | 1166259 | 1166948 | 923119 | 922430 | 937 |
| 1230 | 1167001 | 1167234 | 922377 | 922144 | 210 |
| 1231 | 1167503 | 1168657 | 921875 | 920721 | 1977 |
| 1232 | 1168678 | 1169472 | 920700 | 919906 | 1259 |
| 1233 | 1169576 | 1171024 | 919802 | 918354 | 1976 |
| 1234 | 1171021 | 1171905 | 918357 | 917473 | 1258 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1235 | 1172047 | 1172277 | 917331 | 917101 | 211 |
| 1236 | 1172264 | 1173025 | 917114 | 916353 | 1975 |
| 1237 | 1173022 | 1173636 | 916356 | 915742 | 1257 |
| 1238 | 1173687 | 1174022 | 915691 | 915356 | 938 |
| 1239 | 1174023 | 1174274 | 915355 | 915104 | 1654 |
| 1240 | 1174284 | 1174388 | 915094 | 914990 | 1653 |
| 1241 | 1174493 | 1177870 | 914885 | 911508 | 578 |
| 1242 | 1178296 | 1178862 | 911082 | 910516 | 212 |
| 1243 | 1178840 | 1179322 | 910538 | 910056 | 579 |
| 1244 | 1179335 | 1180606 | 910043 | 908772 | 1974 |
| 1245 | 1180603 | 1181361 | 908775 | 908017 | 1256 |
| 1246 | 1181719 | 1181916 | 907659 | 907462 | 1255 |
| 1247 | 1182281 | 1182673 | 907097 | 906705 | 1973 |
| 1248 | 1182899 | 1183855 | 906479 | 905523 | 580 |
| 1249 | 1184435 | 1184731 | 904943 | 904647 | 1972 |
| 1250 | 1184832 | 1185752 | 904546 | 903626 | 1652 |
| 1251 | 1186264 | 1186524 | 903114 | 902854 | 1254 |
| 1252 | 1187372 | 1187653 | 902006 | 901725 | 1971 |
| 1253 | 1188250 | 1188906 | 901128 | 900472 | 1253 |
| 1254 | 1188962 | 1189906 | 900416 | 899472 | 1970 |
| 1255 | 1189940 | 1190062 | 899438 | 899316 | 1969 |
| 1256 | 1191309 | 1191941 | 898069 | 897437 | 1651 |
| 1257 | 1195773 | 1195841 | 893605 | 893537 | 939 |
| 1258 | 1196421 | 1196939 | 892957 | 892439 | 1650 |
| 1259 | 1197121 | 1197330 | 892257 | 892048 | 1252 |
| 1260 | 1197327 | 1197827 | 892051 | 891551 | 1649 |
| 1261 | 1197859 | 1198116 | 891519 | 891262 | 1251 |
| 1262 | 1198129 | 1198395 | 891249 | 890983 | 1250 |
| 1263 | 1198775 | 1198969 | 890603 | 890409 | 581 |
| 1264 | 1199210 | 1199536 | 890168 | 889842 | 1968 |
| 1265 | 1200465 | 1200542 | 888913 | 888836 | 940 |
| 1266 | 1202741 | 1204258 | 886637 | 885120 | 1967 |
| 1267 | 1204260 | 1205624 | 885118 | 883754 | 1648 |
| 1268 | 1205780 | 1207075 | 883598 | 882303 | 1966 |

(continued)

| gene No. | Nucleic acid No. (sense chain (corresponding to SEQ ID NO: 1, 342, 723), starting nucleotide | Nucleic acid No. (sense chain (corresponding to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1269 | 1207362 | 1207793 | 882016 | 881585 | 941 |
| 1270 | 1207790 | 1208482 | 881588 | 880896 | 582 |
| 1271 | 1209464 | 1210141 | 879914 | 879237 | 583 |
| 1272 | 1210174 | 1210893 | 879204 | 878485 | 213 |
| 1273 | 1210890 | 1211111 | 878488 | 878267 | 942 |
| 1274 | 1211128 | 1211787 | 878250 | 877591 | 214 |
| 1275 | 1211850 | 1212755 | 877528 | 876623 | 943 |
| 1276 | 1212760 | 1213104 | 876618 | 876274 | 1249 |
| 1277 | 1213101 | 1214369 | 876277 | 875009 | 1647 |
| 1278 | 1214366 | 1215214 | 875012 | 874164 | 1965 |
| 1279 | 1215250 | 1215861 | 874128 | 873517 | 1248 |
| 1280 | 1217374 | 1217490 | 872004 | 871888 | 215 |
| 1281 | 1219074 | 1219190 | 870304 | 870188 | 944 |
| 1282 | 1219197 | 1220690 | 870181 | 868688 | 1646 |
| 1283 | 1220740 | 1221513 | 868638 | 867865 | 1247 |
| 1284 | 1221503 | 1222201 | 867875 | 867177 | 1964 |
| 1285 | 1222282 | 1223655 | 867096 | 865723 | 216 |
| 1286 | 1223758 | 1225113 | 865620 | 864265 | 217 |
| 1287 | 1225113 | 1225991 | 864265 | 863387 | 945 |
| 1288 | 1226169 | 1226861 | 863209 | 862517 | 946 |
| 1289 | 1227076 | 1227702 | 862302 | 861676 | 1246 |
| 1290 | 1227756 | 1228466 | 861622 | 860912 | 1645 |
| 1291 | 1228622 | 1230493 | 860756 | 858885 | 584 |
| 1292 | 1230580 | 1233081 | 858798 | 856297 | 218 |
| 1293 | 1233236 | 1234546 | 856142 | 854832 | 585 |
| 1294 | 1234563 | 1236284 | 854815 | 853094 | 1644 |
| 1295 | 1236584 | 1237978 | 852794 | 851400 | 1963 |
| 1296 | 1237975 | 1238376 | 851403 | 851002 | 1245 |
| 1297 | 1238433 | 1239707 | 850945 | 849671 | 1643 |
| 1298 | 1239791 | 1239994 | 849587 | 849384 | 1962 |
| 1299 | 1240125 | 1240214 | 849253 | 849164 | 947 |
| 1300 | 1240801 | 1240896 | 848577 | 848482 | 1244 |
| 1301 | 1241592 | 1241921 | 847786 | 847457 | 1642 |
| 1302 | 1241983 | 1243014 | 847395 | 846364 | 1243 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1303 | 1243011 | 1243661 | 846367 | 845717 | 1641 |
| 1304 | 1243692 | 1243778 | 845686 | 845600 | 1640 |
| 1305 | 1243775 | 1244272 | 845603 | 845106 | 1961 |
| 1306 | 1244307 | 1244765 | 845071 | 844613 | 1639 |
| 1307 | 1244788 | 1244973 | 844590 | 844405 | 1242 |
| 1308 | 1245004 | 1246125 | 844374 | 843253 | 1241 |
| 1309 | 1246241 | 1247059 | 843137 | 842319 | 1960 |
| 1310 | 1247369 | 1248709 | 842009 | 840669 | 1959 |
| 1311 | 1248621 | 1249226 | 840757 | 840152 | 948 |
| 1312 | 1250499 | 1251188 | 838879 | 838190 | 1638 |
| 1313 | 1251193 | 1251561 | 838185 | 837817 | 1240 |
| 1314 | 1251632 | 1253578 | 837746 | 835800 | 1958 |
| 1315 | 1253588 | 1253788 | 835790 | 835590 | 1957 |
| 1316 | 1254304 | 1255470 | 835074 | 833908 | 219 |
| 1317 | 1255582 | 1256436 | 833796 | 832942 | 1239 |
| 1318 | 1256379 | 1256846 | 832999 | 832532 | 1637 |
| 1319 | 1257402 | 1258961 | 831976 | 830417 | 949 |
| 1320 | 1258972 | 1259079 | 830406 | 830299 | 220 |
| 1321 | 1259124 | 1259858 | 830254 | 829520 | 950 |
| 1322 | 1259855 | 1260172 | 829523 | 829206 | 1956 |
| 1323 | 1260229 | 1262256 | 829149 | 827122 | 1238 |
| 1324 | 1262388 | 1262651 | 826990 | 826727 | 951 |
| 1325 | 1262709 | 1264661 | 826669 | 824717 | 952 |
| 1326 | 1264658 | 1265074 | 824720 | 824304 | 1955 |
| 1327 | 1265145 | 1265591 | 824233 | 823787 | 953 |
| 1328 | 1265593 | 1266390 | 823785 | 822988 | 221 |
| 1329 | 1266750 | 1267955 | 822628 | 821423 | 954 |
| 1330 | 1268130 | 1269137 | 821248 | 820241 | 1636 |
| 1331 | 1269155 | 1270042 | 820223 | 819336 | 1954 |
| 1332 | 1270062 | 1271162 | 819316 | 818216 | 1635 |
| 1333 | 1271162 | 1272181 | 818216 | 817197 | 1953 |
| 1334 | 1272174 | 1273103 | 817204 | 816275 | 1634 |
| 1335 | 1273100 | 1274158 | 816278 | 815220 | 1952 |
| 1336 | 1274151 | 1275281 | 815227 | 814097 | 1633 |

(continued)

| gene No. | Nucleic acid No. (sense chain (corresponding to SEQ ID NO: 1, 342, 723), starting nucleotide | Nucleic acid No. (sense chain (corresponding to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1337 | 1275461 | 1276135 | 813917 | 813243 | 1951 |
| 1338 | 1276120 | 1276689 | 813258 | 812689 | 1237 |
| 1339 | 1276727 | 1278301 | 812651 | 811077 | 1950 |
| 1340 | 1278636 | 1279535 | 810742 | 809843 | 1632 |
| 1341 | 1279958 | 1280587 | 809420 | 808791 | 1949 |
| 1342 | 1280661 | 1281740 | 808717 | 807638 | 955 |
| 1343 | 1281804 | 1282397 | 807574 | 806981 | 1631 |
| 1344 | 1282384 | 1283034 | 806994 | 806344 | 1236 |
| 1345 | 1283055 | 1284251 | 806323 | 805127 | 1630 |
| 1346 | 1284667 | 1285869 | 804711 | 803509 | 222 |
| 1347 | 1285975 | 1289823 | 803403 | 799555 | 223 |
| 1348 | 1290019 | 1292922 | 799359 | 796456 | 224 |
| 1349 | 1293396 | 1293860 | 795982 | 795518 | 1629 |
| 1350 | 1294892 | 1295722 | 794486 | 793656 | 586 |
| 1351 | 1295748 | 1297115 | 793630 | 792263 | 956 |
| 1352 | 1297116 | 1298444 | 792262 | 790934 | 1628 |
| 1353 | 1298625 | 1298846 | 790753 | 790532 | 957 |
| 1354 | 1299189 | 1300220 | 790189 | 789158 | 1627 |
| 1355 | 1300290 | 1301624 | 789088 | 787754 | 1626 |
| 1356 | 1301759 | 1302934 | 787619 | 786444 | 1948 |
| 1357 | 1302931 | 1303617 | 786447 | 785761 | 1235 |
| 1358 | 1303690 | 1304454 | 785688 | 784924 | 1234 |
| 1359 | 1304451 | 1305239 | 784927 | 784139 | 1625 |
| 1360 | 1305236 | 1306249 | 784142 | 783129 | 1947 |
| 1361 | 1306246 | 1306722 | 783132 | 782656 | 1233 |
| 1362 | 1306665 | 1307039 | 782713 | 782339 | 1624 |
| 1363 | 1307076 | 1307963 | 782302 | 781415 | 1623 |
| 1364 | 1307989 | 1309053 | 781389 | 780325 | 1232 |
| 1365 | 1309106 | 1309948 | 780272 | 779430 | 587 |
| 1366 | 1309950 | 1311020 | 779428 | 778358 | 958 |
| 1367 | 1311965 | 1313317 | 777413 | 776061 | 1946 |
| 1368 | 1313412 | 1314224 | 775966 | 775154 | 1622 |
| 1369 | 1315661 | 1315879 | 773717 | 773499 | 1945 |
| 1370 | 1316041 | 1316151 | 773337 | 773227 | 1231 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1371 | 1316410 | 1317765 | 772968 | 771613 | 225 |
| 1372 | 1317762 | 1318001 | 771616 | 771377 | 959 |
| 1373 | 1317998 | 1318528 | 771380 | 770850 | 588 |
| 1374 | 1318585 | 1319298 | 770793 | 770080 | 226 |
| 1375 | 1319308 | 1319637 | 770070 | 769741 | 227 |
| 1376 | 1319620 | 1320078 | 769758 | 769300 | 1230 |
| 1377 | 1321326 | 1322096 | 768052 | 767282 | 960 |
| 1378 | 1322102 | 1322401 | 767276 | 766977 | 1944 |
| 1379 | 1322840 | 1323004 | 766538 | 766374 | 1943 |
| 1380 | 1323183 | 1323788 | 766195 | 765590 | 1621 |
| 1381 | 1323802 | 1324827 | 765576 | 764551 | 1229 |
| 1382 | 1325139 | 1325336 | 764239 | 764042 | 1620 |
| 1383 | 1325369 | 1325800 | 764009 | 763578 | 1942 |
| 1384 | 1325787 | 1326215 | 763591 | 763163 | 1619 |
| 1385 | 1326222 | 1326593 | 763156 | 762785 | 1618 |
| 1386 | 1326738 | 1327526 | 762640 | 761852 | 1617 |
| 1387 | 1327548 | 1327970 | 761830 | 761408 | 1616 |
| 1388 | 1327967 | 1328509 | 761411 | 760869 | 1941 |
| 1389 | 1328520 | 1329077 | 760858 | 760301 | 1615 |
| 1390 | 1329084 | 1329671 | 760294 | 759707 | 1614 |
| 1391 | 1330058 | 1330213 | 759320 | 759165 | 589 |
| 1392 | 1330540 | 1331565 | 758838 | 757813 | 1228 |
| 1393 | 1331777 | 1332007 | 757601 | 757371 | 1940 |
| 1394 | 1332043 | 1332753 | 757335 | 756625 | 1227 |
| 1395 | 1332861 | 1333112 | 756517 | 756266 | 1613 |
| 1396 | 1333113 | 1333694 | 756265 | 755684 | 1612 |
| 1397 | 1333706 | 1333999 | 755672 | 755379 | 1939 |
| 1398 | 1334020 | 1334550 | 755358 | 754828 | 1226 |
| 1399 | 1334537 | 1335136 | 754841 | 754242 | 1938 |
| 1400 | 1335210 | 1336667 | 754168 | 752711 | 1611 |
| 1401 | 1336699 | 1337145 | 752679 | 752233 | 1225 |
| 1402 | 1337157 | 1337624 | 752221 | 751754 | 1610 |
| 1403 | 1337636 | 1338343 | 751742 | 751035 | 1937 |
| 1404 | 1338340 | 1338954 | 751038 | 750424 | 1224 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1405 | 1338956 | 1339411 | 750422 | 749967 | 1936 |
| 1406 | 1339413 | 1339793 | 749965 | 749585 | 1609 |
| 1407 | 1339810 | 1340373 | 749568 | 749005 | 1223 |
| 1408 | 1340375 | 1340767 | 749003 | 748611 | 1935 |
| 1409 | 1340779 | 1340949 | 748599 | 748429 | 1222 |
| 1410 | 1340951 | 1341502 | 748427 | 747876 | 1934 |
| 1411 | 1341516 | 1342247 | 747862 | 747131 | 1608 |
| 1412 | 1342247 | 1342612 | 747131 | 746766 | 1933 |
| 1413 | 1342624 | 1343049 | 746754 | 746329 | 1221 |
| 1414 | 1343053 | 1343406 | 746325 | 745972 | 1220 |
| 1415 | 1343394 | 1343660 | 745984 | 745718 | 1607 |
| 1416 | 1343657 | 1343953 | 745721 | 745425 | 1932 |
| 1417 | 1343960 | 1344160 | 745418 | 745218 | 1931 |
| 1418 | 1344147 | 1344785 | 745231 | 744593 | 1606 |
| 1419 | 1344782 | 1345252 | 744596 | 744126 | 1930 |
| 1420 | 1345263 | 1345673 | 744115 | 743705 | 1605 |
| 1421 | 1345670 | 1346398 | 743708 | 742980 | 1929 |
| 1422 | 1346403 | 1346663 | 742975 | 742715 | 1604 |
| 1423 | 1346670 | 1347437 | 742708 | 741941 | 1603 |
| 1424 | 1347448 | 1348488 | 741930 | 740890 | 1219 |
| 1425 | 1348490 | 1349344 | 740888 | 740034 | 1928 |
| 1426 | 1349882 | 1351258 | 739496 | 738120 | 1927 |
| 1427 | 1351322 | 1352506 | 738056 | 736872 | 1926 |
| 1428 | 1352613 | 1353269 | 736765 | 736109 | 1602 |
| 1429 | 1354574 | 1355740 | 734804 | 733638 | 590 |
| 1430 | 1355821 | 1356402 | 733557 | 732976 | 1218 |
| 1431 | 1356606 | 1357514 | 732772 | 731864 | 961 |
| 1432 | 1357517 | 1358350 | 731861 | 731028 | 1925 |
| 1433 | 1358441 | 1359433 | 730937 | 729945 | 1924 |
| 1434 | 1361181 | 1362461 | 728197 | 726917 | 962 |
| 1435 | 1362449 | 1362523 | 726929 | 726855 | 591 |
| 1436 | 1363010 | 1363930 | 726368 | 725448 | 1923 |
| 1437 | 1363972 | 1365465 | 725406 | 723913 | 1217 |
| 1438 | 1365589 | 1366155 | 723789 | 723223 | 228 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1439 | 1366195 | 1367346 | 723183 | 722032 | 229 |
| 1440 | 1367357 | 1368481 | 722021 | 720897 | 592 |
| 1441 | 1368582 | 1369193 | 720796 | 720185 | 963 |
| 1442 | 1369248 | 1370567 | 720130 | 718811 | 964 |
| 1443 | 1370627 | 1370989 | 718751 | 718389 | 1922 |
| 1444 | 1371847 | 1372125 | 717531 | 717253 | 230 |
| 1445 | 1372322 | 1373752 | 717056 | 715626 | 593 |
| 1446 | 1373902 | 1376664 | 715476 | 712714 | 231 |
| 1447 | 1376921 | 1378402 | 712457 | 710976 | 594 |
| 1448 | 1378470 | 1379534 | 710908 | 709844 | 1601 |
| 1449 | 1379649 | 1380014 | 709729 | 709364 | 965 |
| 1450 | 1379981 | 1380445 | 709397 | 708933 | 1921 |
| 1451 | 1380532 | 1381284 | 708846 | 708094 | 1216 |
| 1452 | 1381281 | 1382687 | 708097 | 706691 | 1600 |
| 1453 | 1382767 | 1384572 | 706611 | 704806 | 232 |
| 1454 | 1384569 | 1385354 | 704809 | 704024 | 1599 |
| 1455 | 1385351 | 1385914 | 704027 | 703464 | 1920 |
| 1456 | 1386061 | 1387578 | 703317 | 701800 | 1215 |
| 1457 | 1387922 | 1388011 | 701456 | 701367 | 595 |
| 1458 | 1388004 | 1389050 | 701374 | 700328 | 1598 |
| 1459 | 1388485 | 1388589 | 700893 | 700789 | 233 |
| 1460 | 1389047 | 1389982 | 700331 | 699396 | 1919 |
| 1461 | 1390108 | 1390617 | 699270 | 698761 | 234 |
| 1462 | 1390656 | 1391165 | 698722 | 698213 | 966 |
| 1463 | 1391397 | 1391669 | 697981 | 697709 | 967 |
| 1464 | 1393980 | 1394540 | 695398 | 694838 | 968 |
| 1465 | 1396169 | 1396951 | 693209 | 692427 | 596 |
| 1466 | 1396965 | 1397522 | 692413 | 691856 | 969 |
| 1467 | 1397528 | 1397968 | 691850 | 691410 | 1918 |
| 1468 | 1398271 | 1399176 | 691107 | 690202 | 235 |
| 1469 | 1399173 | 1400693 | 690205 | 688685 | 970 |
| 1470 | 1400690 | 1401382 | 688688 | 687996 | 597 |
| 1471 | 1401502 | 1401813 | 687876 | 687565 | 236 |
| 1472 | 1401815 | 1403806 | 687563 | 685572 | 598 |

(continued)

| gene No. | Nucleic acid No. (sense chain (corresponding to SEQ ID NO: 1, 342, 723), starting nucleotide | Nucleic acid No. (sense chain (corresponding to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1473 | 1403824 | 1404309 | 685554 | 685069 | 237 |
| 1474 | 1404349 | 1404960 | 685029 | 684418 | 238 |
| 1475 | 1404957 | 1406060 | 684421 | 683318 | 971 |
| 1476 | 1406057 | 1406365 | 683321 | 683013 | 599 |
| 1477 | 1406372 | 1407382 | 683006 | 681996 | 600 |
| 1478 | 1407475 | 1408257 | 681903 | 681121 | 239 |
| 1479 | 1408254 | 1409654 | 681124 | 679724 | 972 |
| 1480 | 1409674 | 1410327 | 679704 | 679051 | 240 |
| 1481 | 1410413 | 1411189 | 678965 | 678189 | 601 |
| 1482 | 1411199 | 1411954 | 678179 | 677424 | 602 |
| 1483 | 1411938 | 1413167 | 677440 | 676211 | 973 |
| 1484 | 1413235 | 1413960 | 676143 | 675418 | 241 |
| 1485 | 1413935 | 1414642 | 675443 | 674736 | 603 |
| 1486 | 1414943 | 1415797 | 674435 | 673581 | 604 |
| 1487 | 1415800 | 1418658 | 673578 | 670720 | 1214 |
| 1488 | 1418655 | 1420457 | 670723 | 668921 | 1597 |
| 1489 | 1420450 | 1420923 | 668928 | 668455 | 1213 |
| 1490 | 1421049 | 1422080 | 668329 | 667298 | 1596 |
| 1491 | 1422217 | 1422759 | 667161 | 666619 | 242 |
| 1492 | 1422740 | 1423594 | 666638 | 665784 | 1917 |
| 1493 | 1423617 | 1424129 | 665761 | 665249 | 1595 |
| 1494 | 1424266 | 1424787 | 665112 | 664591 | 243 |
| 1495 | 1424787 | 1428260 | 664591 | 661118 | 974 |
| 1496 | 1428306 | 1428734 | 661072 | 660644 | 975 |
| 1497 | 1428842 | 1430410 | 660536 | 658968 | 605 |
| 1498 | 1430421 | 1430807 | 658957 | 658571 | 976 |
| 1499 | 1430801 | 1431283 | 658577 | 658095 | 606 |
| 1500 | 1431290 | 1432483 | 658088 | 656895 | 607 |
| 1501 | 1432547 | 1433398 | 656831 | 655980 | 608 |
| 1502 | 1433432 | 1434445 | 655946 | 654933 | 609 |
| 1503 | 1434874 | 1435398 | 654504 | 653980 | 244 |
| 1504 | 1435395 | 1436108 | 653983 | 653270 | 1594 |
| 1505 | 1436180 | 1436593 | 653198 | 652785 | 1916 |
| 1506 | 1436645 | 1436935 | 652733 | 652443 | 1915 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1507 | 1436958 | 1437776 | 652420 | 651602 | 1593 |
| 1508 | 1437769 | 1438527 | 651609 | 650851 | 1212 |
| 1509 | 1438502 | 1439275 | 650876 | 650103 | 1914 |
| 1510 | 1439272 | 1439982 | 650106 | 649396 | 1211 |
| 1511 | 1439994 | 1440776 | 649384 | 648602 | 1592 |
| 1512 | 1441115 | 1441582 | 648263 | 647796 | 610 |
| 1513 | 1441557 | 1441976 | 647821 | 647402 | 1591 |
| 1514 | 1441888 | 1442184 | 647490 | 647194 | 1210 |
| 1515 | 1442268 | 1442525 | 647110 | 646853 | 977 |
| 1516 | 1442602 | 1444524 | 646776 | 644854 | 245 |
| 1517 | 1444521 | 1444967 | 644857 | 644411 | 1590 |
| 1518 | 1445288 | 1446001 | 644090 | 643377 | 1913 |
| 1519 | 1446421 | 1446744 | 642957 | 642634 | 1209 |
| 1520 | 1447018 | 1447827 | 642360 | 641551 | 246 |
| 1521 | 1447763 | 1448299 | 641615 | 641079 | 1912 |
| 1522 | 1448354 | 1448527 | 641024 | 640851 | 1911 |
| 1523 | 1448733 | 1449227 | 640645 | 640151 | 978 |
| 1524 | 1449764 | 1450072 | 639614 | 639306 | 611 |
| 1525 | 1450076 | 1451272 | 639302 | 638106 | 612 |
| 1526 | 1451362 | 1452348 | 638016 | 637030 | 247 |
| 1527 | 1452345 | 1452566 | 637033 | 636812 | 1589 |
| 1528 | 1452921 | 1453571 | 636457 | 635807 | 1588 |
| 1529 | 1453739 | 1453954 | 635639 | 635424 | 613 |
| 1530 | 1454658 | 1454753 | 634720 | 634625 | 1587 |
| 1531 | 1455780 | 1457495 | 633598 | 631883 | 1586 |
| 1532 | 1458373 | 1458516 | 631005 | 630862 | 1208 |
| 1533 | 1460859 | 1461371 | 628519 | 628007 | 1585 |
| 1534 | 1461343 | 1461726 | 628035 | 627652 | 1207 |
| 1535 | 1462494 | 1463108 | 626884 | 626270 | 1584 |
| 1536 | 1463105 | 1464283 | 626273 | 625095 | 1910 |
| 1537 | 1464255 | 1466492 | 625123 | 622886 | 1583 |
| 1538 | 1466599 | 1467609 | 622779 | 621769 | 1206 |
| 1539 | 1467655 | 1467744 | 621723 | 621634 | 248 |
| 1540 | 1467769 | 1467906 | 621609 | 621472 | 249 |

(continued)

| gene No. | Nucleic acid No. (sense chain (corresponding to SEQ ID NO: 1, 342, 723), starting nucleotide | Nucleic acid No. (sense chain (corresponding to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1541 | 1467891 | 1468676 | 621487 | 620702 | 1582 |
| 1542 | 1468498 | 1469019 | 620880 | 620359 | 1205 |
| 1543 | 1469265 | 1470533 | 620113 | 618845 | 979 |
| 1544 | 1470609 | 1471790 | 618769 | 617588 | 1581 |
| 1545 | 1471812 | 1471937 | 617566 | 617441 | 1580 |
| 1546 | 1471870 | 1472673 | 617508 | 616705 | 250 |
| 1547 | 1474731 | 1474928 | 614647 | 614450 | 1579 |
| 1548 | 1475072 | 1475983 | 614306 | 613395 | 1909 |
| 1549 | 1477107 | 1477574 | 612271 | 611804 | 980 |
| 1550 | 1477584 | 1479029 | 611794 | 610349 | 1578 |
| 1551 | 1479030 | 1479884 | 610348 | 609494 | 1577 |
| 1552 | 1480088 | 1480873 | 609290 | 608505 | 614 |
| 1553 | 1480960 | 1481781 | 608418 | 607597 | 1204 |
| 1554 | 1481753 | 1481869 | 607625 | 607509 | 1908 |
| 1555 | 1482049 | 1482780 | 607329 | 606598 | 1203 |
| 1556 | 1484422 | 1486413 | 604956 | 602965 | 251 |
| 1557 | 1486448 | 1488211 | 602930 | 601167 | 615 |
| 1558 | 1488253 | 1489308 | 601125 | 600070 | 1202 |
| 1559 | 1489417 | 1490157 | 599961 | 599221 | 252 |
| 1560 | 1490211 | 1490753 | 599167 | 598625 | 981 |
| 1561 | 1490896 | 1491087 | 598482 | 598291 | 253 |
| 1562 | 1491222 | 1491395 | 598156 | 597983 | 1576 |
| 1563 | 1491406 | 1491738 | 597972 | 597640 | 1201 |
| 1564 | 1491692 | 1492225 | 597686 | 597153 | 1907 |
| 1565 | 1492222 | 1492431 | 597156 | 596947 | 1200 |
| 1566 | 1492428 | 1493000 | 596950 | 596378 | 1575 |
| 1567 | 1493037 | 1493573 | 596341 | 595805 | 1574 |
| 1568 | 1493631 | 1494593 | 595747 | 594785 | 1573 |
| 1569 | 1494613 | 1495560 | 594765 | 593818 | 1199 |
| 1570 | 1495557 | 1496564 | 593821 | 592814 | 1572 |
| 1571 | 1496677 | 1497216 | 592701 | 592162 | 1198 |
| 1572 | 1497231 | 1497902 | 592147 | 591476 | 1571 |
| 1573 | 1498015 | 1498506 | 591363 | 590872 | 1197 |
| 1574 | 1499893 | 1500954 | 589485 | 588424 | 1196 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1575 | 1500975 | 1501334 | 588403 | 588044 | 982 |
| 1576 | 1501234 | 1501755 | 588144 | 587623 | 254 |
| 1577 | 1501752 | 1502747 | 587626 | 586631 | 983 |
| 1578 | 1502782 | 1504029 | 586596 | 585349 | 255 |
| 1579 | 1503705 | 1503881 | 585673 | 585497 | 1570 |
| 1580 | 1506454 | 1507683 | 582924 | 581695 | 256 |
| 1581 | 1507680 | 1508369 | 581698 | 581009 | 984 |
| 1582 | 1508513 | 1509250 | 580865 | 580128 | 616 |
| 1583 | 1509284 | 1511584 | 580094 | 577794 | 1906 |
| 1584 | 1512986 | 1513759 | 576392 | 575619 | 617 |
| 1585 | 1513756 | 1514835 | 575622 | 574543 | 257 |
| 1586 | 1515877 | 1516842 | 573501 | 572536 | 258 |
| 1587 | 1518510 | 1518569 | 570868 | 570809 | 1569 |
| 1588 | 1519816 | 1521600 | 569562 | 567778 | 259 |
| 1589 | 1519824 | 1519925 | 569554 | 569453 | 1568 |
| 1590 | 1521735 | 1522592 | 567643 | 566786 | 985 |
| 1591 | 1523210 | 1524667 | 566168 | 564711 | 618 |
| 1592 | 1525075 | 1526076 | 564303 | 563302 | 260 |
| 1593 | 1526066 | 1526449 | 563312 | 562929 | 1905 |
| 1594 | 1529489 | 1530295 | 559889 | 559083 | 619 |
| 1595 | 1530296 | 1530733 | 559082 | 558645 | 620 |
| 1596 | 1530894 | 1536164 | 558484 | 553214 | 986 |
| 1597 | 1536298 | 1536771 | 553080 | 552607 | 261 |
| 1598 | 1536811 | 1537365 | 552567 | 552013 | 262 |
| 1599 | 1540326 | 1541702 | 549052 | 547676 | 987 |
| 1600 | 1541901 | 1543691 | 547477 | 545687 | 1567 |
| 1601 | 1543754 | 1544062 | 545624 | 545316 | 621 |
| 1602 | 1544093 | 1544920 | 545285 | 544458 | 622 |
| 1603 | 1544970 | 1545347 | 544408 | 544031 | 988 |
| 1604 | 1545432 | 1545968 | 543946 | 543410 | 1566 |
| 1605 | 1546165 | 1549362 | 543213 | 540016 | 263 |
| 1606 | 1549370 | 1549522 | 540008 | 539856 | 1904 |
| 1607 | 1550195 | 1551454 | 539183 | 537924 | 1903 |
| 1608 | 1551384 | 1551506 | 537994 | 537872 | 989 |

(continued)

| gene No. | Nucleic acid No. (sense chain (corresponding to SEQ ID NO: 1, 342, 723), starting nucleotide | Nucleic acid No. (sense chain (corresponding to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1609 | 1551637 | 1552008 | 537741 | 537370 | 1195 |
| 1610 | 1551975 | 1552217 | 537403 | 537161 | 1565 |
| 1611 | 1552330 | 1553088 | 537048 | 536290 | 264 |
| 1612 | 1553108 | 1555480 | 536270 | 533898 | 1902 |
| 1613 | 1555474 | 1556295 | 533904 | 533083 | 1194 |
| 1614 | 1556455 | 1557438 | 532923 | 531940 | 1193 |
| 1615 | 1557416 | 1558507 | 531962 | 530871 | 1901 |
| 1616 | 1558390 | 1559334 | 530988 | 530044 | 1192 |
| 1617 | 1559337 | 1560350 | 530041 | 529028 | 1564 |
| 1618 | 1560382 | 1561011 | 528996 | 528367 | 1191 |
| 1619 | 1561392 | 1562597 | 527986 | 526781 | 1563 |
| 1620 | 1562832 | 1564286 | 526546 | 525092 | 990 |
| 1621 | 1564489 | 1564938 | 524889 | 524440 | 265 |
| 1622 | 1564960 | 1565772 | 524418 | 523606 | 1190 |
| 1623 | 1565943 | 1569653 | 523435 | 519725 | 991 |
| 1624 | 1569699 | 1571144 | 519679 | 518234 | 1562 |
| 1625 | 1570858 | 1571220 | 518520 | 518158 | 266 |
| 1626 | 1571217 | 1572563 | 518161 | 516815 | 1561 |
| 1627 | 1572612 | 1573637 | 516766 | 515741 | 1560 |
| 1628 | 1573641 | 1573748 | 515737 | 515630 | 1559 |
| 1629 | 1573710 | 1575680 | 515668 | 513698 | 992 |
| 1630 | 1575753 | 1577099 | 513625 | 512279 | 993 |
| 1631 | 1577138 | 1578040 | 512240 | 511338 | 623 |
| 1632 | 1578037 | 1579284 | 511341 | 510094 | 267 |
| 1633 | 1579294 | 1582596 | 510084 | 506782 | 268 |
| 1634 | 1582707 | 1583825 | 506671 | 505553 | 994 |
| 1635 | 1583858 | 1584259 | 505520 | 505119 | 624 |
| 1636 | 1584289 | 1585641 | 505089 | 503737 | 269 |
| 1637 | 1585646 | 1586575 | 503732 | 502803 | 1900 |
| 1638 | 1586361 | 1588547 | 503017 | 500831 | 995 |
| 1639 | 1588597 | 1588962 | 500781 | 500416 | 270 |
| 1640 | 1588919 | 1590214 | 500459 | 499164 | 625 |
| 1641 | 1590298 | 1591578 | 499080 | 497800 | 271 |
| 1642 | 1591902 | 1592372 | 497476 | 497006 | 1558 |

(continued)

| gene No. | Nucleic acid No. (sense chain (corresponding to SEQ ID NO: 1, 342, 723), starting nucleotide | Nucleic acid No. (sense chain (corresponding to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1643 | 1592769 | 1593515 | 496609 | 495863 | 996 |
| 1644 | 1593682 | 1594884 | 495696 | 494494 | 1189 |
| 1645 | 1595017 | 1595325 | 494361 | 494053 | 272 |
| 1646 | 1596465 | 1597058 | 492913 | 492320 | 1557 |
| 1647 | 1597751 | 1598509 | 491627 | 490869 | 1899 |
| 1648 | 1598676 | 1599902 | 490702 | 489476 | 997 |
| 1649 | 1599886 | 1600935 | 489492 | 488443 | 273 |
| 1650 | 1601220 | 1601777 | 488158 | 487601 | 998 |
| 1651 | 1603727 | 1603786 | 485651 | 485592 | 626 |
| 1652 | 1604088 | 1604264 | 485290 | 485114 | 1556 |
| 1653 | 1604708 | 1606048 | 484670 | 483330 | 627 |
| 1654 | 1606039 | 1606902 | 483339 | 482476 | 1188 |
| 1655 | 1606912 | 1607685 | 482466 | 481693 | 1187 |
| 1656 | 1607663 | 1607971 | 481715 | 481407 | 1898 |
| 1657 | 1608213 | 1609220 | 481165 | 480158 | 1555 |
| 1658 | 1609231 | 1610190 | 480147 | 479188 | 1186 |
| 1659 | 1610202 | 1611623 | 479176 | 477755 | 1554 |
| 1660 | 1611635 | 1612684 | 477743 | 476694 | 1897 |
| 1661 | 1612865 | 1615312 | 476513 | 474066 | 1896 |
| 1662 | 1615653 | 1616882 | 473725 | 472496 | 999 |
| 1663 | 1616860 | 1617561 | 472518 | 471817 | 274 |
| 1664 | 1617558 | 1618517 | 471820 | 470861 | 1000 |
| 1665 | 1617756 | 1617815 | 471622 | 471563 | 1553 |
| 1666 | 1618578 | 1619276 | 470800 | 470102 | 1001 |
| 1667 | 1619263 | 1621227 | 470115 | 468151 | 1185 |
| 1668 | 1621305 | 1621934 | 468073 | 467444 | 1552 |
| 1669 | 1622735 | 1622920 | 466643 | 466458 | 628 |
| 1670 | 1622922 | 1624112 | 466456 | 465266 | 1002 |
| 1671 | 1624133 | 1625287 | 465245 | 464091 | 629 |
| 1672 | 1625321 | 1625563 | 464057 | 463815 | 630 |
| 1673 | 1625628 | 1625717 | 463750 | 463661 | 1003 |
| 1674 | 1625816 | 1625929 | 463562 | 463449 | 631 |
| 1675 | 1625919 | 1626824 | 463459 | 462554 | 1551 |
| 1676 | 1627009 | 1627614 | 462369 | 461764 | 1184 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1677 | 1627793 | 1629337 | 461585 | 460041 | 632 |
| 1678 | 1629435 | 1630595 | 459943 | 458783 | 1004 |
| 1679 | 1630596 | 1631720 | 458782 | 457658 | 1005 |
| 1680 | 1630637 | 1630705 | 458741 | 458673 | 1895 |
| 1681 | 1631799 | 1633073 | 457579 | 456305 | 1006 |
| 1682 | 1633129 | 1633257 | 456249 | 456121 | 275 |
| 1683 | 1634125 | 1634739 | 455253 | 454639 | 276 |
| 1684 | 1634253 | 1634369 | 455125 | 455009 | 1550 |
| 1685 | 1634744 | 1635046 | 454634 | 454332 | 633 |
| 1686 | 1635049 | 1636365 | 454329 | 453013 | 1183 |
| 1687 | 1636376 | 1637356 | 453002 | 452022 | 634 |
| 1688 | 1637336 | 1638673 | 452042 | 450705 | 1894 |
| 1689 | 1638670 | 1639755 | 450708 | 449623 | 1182 |
| 1690 | 1639752 | 1640816 | 449626 | 448562 | 1549 |
| 1691 | 1640937 | 1641557 | 448441 | 447821 | 1548 |
| 1692 | 1641581 | 1643545 | 447797 | 445833 | 1893 |
| 1693 | 1643712 | 1644038 | 445666 | 445340 | 1007 |
| 1694 | 1644035 | 1644664 | 445343 | 444714 | 1892 |
| 1695 | 1644711 | 1645832 | 444667 | 443546 | 1008 |
| 1696 | 1645842 | 1646195 | 443536 | 443183 | 1009 |
| 1697 | 1646550 | 1647749 | 442828 | 441629 | 1010 |
| 1698 | 1651192 | 1652691 | 438186 | 436687 | 1181 |
| 1699 | 1652842 | 1653462 | 436536 | 435916 | 277 |
| 1700 | 1653443 | 1654624 | 435935 | 434754 | 635 |
| 1701 | 1654676 | 1655512 | 434702 | 433866 | 636 |
| 1702 | 1655924 | 1656976 | 433454 | 432402 | 1891 |
| 1703 | 1657257 | 1658210 | 432121 | 431168 | 1547 |
| 1704 | 1658633 | 1658857 | 430745 | 430521 | 1890 |
| 1705 | 1659540 | 1660034 | 429838 | 429344 | 1011 |
| 1706 | 1660137 | 1660616 | 429241 | 428762 | 1012 |
| 1707 | 1660605 | 1661033 | 428773 | 428345 | 1546 |
| 1708 | 1661293 | 1661439 | 428085 | 427939 | 278 |
| 1709 | 1661519 | 1662583 | 427859 | 426795 | 1889 |
| 1710 | 1662585 | 1666019 | 426793 | 423359 | 1545 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1711 | 1666185 | 1666505 | 423193 | 422873 | 1544 |
| 1712 | 1667046 | 1668500 | 422332 | 420878 | 1543 |
| 1713 | 1668573 | 1668914 | 420805 | 420464 | 1013 |
| 1714 | 1668871 | 1669944 | 420507 | 419434 | 279 |
| 1715 | 1669941 | 1671896 | 419437 | 417482 | 1542 |
| 1716 | 1671856 | 1672545 | 417522 | 416833 | 1180 |
| 1717 | 1672642 | 1672686 | 416736 | 416692 | 1179 |
| 1718 | 1672713 | 1673096 | 416665 | 416282 | 1541 |
| 1719 | 1673965 | 1674999 | 415413 | 414379 | 1178 |
| 1720 | 1675448 | 1676545 | 413930 | 412833 | 637 |
| 1721 | 1676630 | 1677790 | 412748 | 411588 | 638 |
| 1722 | 1677812 | 1678636 | 411566 | 410742 | 639 |
| 1723 | 1678705 | 1679553 | 410673 | 409825 | 280 |
| 1724 | 1679540 | 1680370 | 409838 | 409008 | 640 |
| 1725 | 1680367 | 1681128 | 409011 | 408250 | 281 |
| 1726 | 1681383 | 1681730 | 407995 | 407648 | 1014 |
| 1727 | 1681740 | 1682333 | 407638 | 407045 | 1015 |
| 1728 | 1682428 | 1682817 | 406950 | 406561 | 282 |
| 1729 | 1682818 | 1683495 | 406560 | 405883 | 1177 |
| 1730 | 1683568 | 1684578 | 405810 | 404800 | 1176 |
| 1731 | 1684439 | 1684564 | 404939 | 404814 | 641 |
| 1732 | 1685535 | 1686689 | 403843 | 402689 | 1540 |
| 1733 | 1686869 | 1687045 | 402509 | 402333 | 642 |
| 1734 | 1687089 | 1687931 | 402289 | 401447 | 1016 |
| 1735 | 1687932 | 1689299 | 401446 | 400079 | 1539 |
| 1736 | 1689399 | 1690175 | 399979 | 399203 | 1017 |
| 1737 | 1691003 | 1692442 | 398375 | 396936 | 1888 |
| 1738 | 1692515 | 1693180 | 396863 | 396198 | 643 |
| 1739 | 1693184 | 1693489 | 396194 | 395889 | 644 |
| 1740 | 1693499 | 1694056 | 395879 | 395322 | 645 |
| 1741 | 1694157 | 1695629 | 395221 | 393749 | 1018 |
| 1742 | 1695642 | 1696265 | 393736 | 393113 | 1538 |
| 1743 | 1696275 | 1697726 | 393103 | 391652 | 1537 |
| 1744 | 1697807 | 1698145 | 391571 | 391233 | 646 |

(continued)

| gene No. | Nucleic acid No. (sense chain (corresponding to SEQ ID NO: 1, 342, 723), starting nucleotide | Nucleic acid No. (sense chain (corresponding to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1745 | 1699092 | 1699178 | 390286 | 390200 | 1019 |
| 1746 | 1699622 | 1700173 | 389756 | 389205 | 1887 |
| 1747 | 1700210 | 1701493 | 389168 | 387885 | 1886 |
| 1748 | 1703531 | 1704163 | 385847 | 385215 | 647 |
| 1749 | 1704224 | 1704970 | 385154 | 384408 | 1885 |
| 1750 | 1704989 | 1705141 | 384389 | 384237 | 1884 |
| 1751 | 1705367 | 1706314 | 384011 | 383064 | 1883 |
| 1752 | 1706139 | 1706984 | 383239 | 382394 | 1020 |
| 1753 | 1706986 | 1707378 | 382392 | 382000 | 283 |
| 1754 | 1707375 | 1708133 | 382003 | 381245 | 1536 |
| 1755 | 1708168 | 1710714 | 381210 | 378664 | 1175 |
| 1756 | 1710855 | 1711487 | 378523 | 377891 | 1535 |
| 1757 | 1712778 | 1714040 | 376600 | 375338 | 1021 |
| 1758 | 1714040 | 1716247 | 375338 | 373131 | 648 |
| 1759 | 1716248 | 1721644 | 373130 | 367734 | 649 |
| 1760 | 1721669 | 1722406 | 367709 | 366972 | 650 |
| 1761 | 1722894 | 1723436 | 366484 | 365942 | 1022 |
| 1762 | 1725222 | 1725860 | 364156 | 363518 | 1023 |
| 1763 | 1725857 | 1726705 | 363521 | 362673 | 1882 |
| 1764 | 1727964 | 1729022 | 361414 | 360356 | 1024 |
| 1765 | 1729029 | 1729787 | 360349 | 359591 | 1025 |
| 1766 | 1729784 | 1730227 | 359594 | 359151 | 651 |
| 1767 | 1730270 | 1731955 | 359108 | 357423 | 652 |
| 1768 | 1731945 | 1732280 | 357433 | 357098 | 1534 |
| 1769 | 1732332 | 1732982 | 357046 | 356396 | 1533 |
| 1770 | 1732998 | 1733120 | 356380 | 356258 | 1532 |
| 1771 | 1733473 | 1734267 | 355905 | 355111 | 284 |
| 1772 | 1734255 | 1735046 | 355123 | 354332 | 1531 |
| 1773 | 1735212 | 1735793 | 354166 | 353585 | 1026 |
| 1774 | 1736419 | 1736520 | 352959 | 352858 | 285 |
| 1775 | 1736456 | 1736896 | 352922 | 352482 | 653 |
| 1776 | 1736893 | 1737423 | 352485 | 351955 | 1174 |
| 1777 | 1737620 | 1738414 | 351758 | 350964 | 1881 |
| 1778 | 1738777 | 1739505 | 350601 | 349873 | 1173 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1779 | 1739502 | 1739852 | 349876 | 349526 | 1530 |
| 1780 | 1739935 | 1740549 | 349443 | 348829 | 1172 |
| 1781 | 1740792 | 1741826 | 348586 | 347552 | 1027 |
| 1782 | 1741926 | 1743704 | 347452 | 345674 | 1028 |
| 1783 | 1743694 | 1743957 | 345684 | 345421 | 1171 |
| 1784 | 1743938 | 1744243 | 345440 | 345135 | 1880 |
| 1785 | 1744245 | 1745591 | 345133 | 343787 | 1529 |
| 1786 | 1745650 | 1746300 | 343728 | 343078 | 286 |
| 1787 | 1746894 | 1747268 | 342484 | 342110 | 1029 |
| 1788 | 1747308 | 1748660 | 342070 | 340718 | 1030 |
| 1789 | 1749755 | 1749931 | 339623 | 339447 | 1879 |
| 1790 | 1749900 | 1749992 | 339478 | 339386 | 1031 |
| 1791 | 1750416 | 1751543 | 338962 | 337835 | 1528 |
| 1792 | 1751717 | 1752793 | 337661 | 336585 | 1878 |
| 1793 | 1752795 | 1753493 | 336583 | 335885 | 1527 |
| 1794 | 1753468 | 1755291 | 335910 | 334087 | 1170 |
| 1795 | 1755444 | 1756100 | 333934 | 333278 | 1526 |
| 1796 | 1756133 | 1756924 | 333245 | 332454 | 1877 |
| 1797 | 1757029 | 1757460 | 332349 | 331918 | 1169 |
| 1798 | 1757494 | 1758735 | 331884 | 330643 | 1168 |
| 1799 | 1758870 | 1758998 | 330508 | 330380 | 1525 |
| 1800 | 1760394 | 1760735 | 328984 | 328643 | 1032 |
| 1801 | 1762166 | 1762558 | 327212 | 326820 | 1876 |
| 1802 | 1762676 | 1762846 | 326702 | 326532 | 654 |
| 1803 | 1762843 | 1763493 | 326535 | 325885 | 1167 |
| 1804 | 1763590 | 1764141 | 325788 | 325237 | 287 |
| 1805 | 1764136 | 1764609 | 325242 | 324769 | 1166 |
| 1806 | 1764704 | 1765804 | 324674 | 323574 | 655 |
| 1807 | 1765840 | 1766682 | 323538 | 322696 | 288 |
| 1808 | 1766679 | 1767068 | 322699 | 322310 | 1033 |
| 1809 | 1767079 | 1767885 | 322299 | 321493 | 1165 |
| 1810 | 1767919 | 1768269 | 321459 | 321109 | 1164 |
| 1811 | 1768271 | 1769350 | 321107 | 320028 | 1875 |
| 1812 | 1769469 | 1770143 | 319909 | 319235 | 1524 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1813 | 1770892 | 1772169 | 318486 | 317209 | 289 |
| 1814 | 1772144 | 1772719 | 317234 | 316659 | 1874 |
| 1815 | 1772653 | 1773303 | 316725 | 316075 | 1163 |
| 1816 | 1773571 | 1774485 | 315807 | 314893 | 1162 |
| 1817 | 1774489 | 1775145 | 314889 | 314233 | 1161 |
| 1818 | 1775139 | 1776068 | 314239 | 313310 | 1523 |
| 1819 | 1776073 | 1776540 | 313305 | 312838 | 1160 |
| 1820 | 1776586 | 1777293 | 312792 | 312085 | 290 |
| 1821 | 1777281 | 1777811 | 312097 | 311567 | 1034 |
| 1822 | 1777799 | 1778830 | 311579 | 310548 | 656 |
| 1823 | 1779069 | 1779554 | 310309 | 309824 | 1035 |
| 1824 | 1779558 | 1779923 | 309820 | 309455 | 1522 |
| 1825 | 1779979 | 1781619 | 309399 | 307759 | 1159 |
| 1826 | 1781597 | 1782928 | 307781 | 306450 | 657 |
| 1827 | 1782866 | 1783828 | 306512 | 305550 | 1873 |
| 1828 | 1784010 | 1784594 | 305368 | 304784 | 1036 |
| 1829 | 1784774 | 1784953 | 304604 | 304425 | 658 |
| 1830 | 1784955 | 1786151 | 304423 | 303227 | 1037 |
| 1831 | 1786148 | 1787092 | 303230 | 302286 | 659 |
| 1832 | 1787147 | 1787473 | 302231 | 301905 | 660 |
| 1833 | 1787485 | 1788669 | 301893 | 300709 | 291 |
| 1834 | 1788671 | 1789675 | 300707 | 299703 | 661 |
| 1835 | 1789714 | 1790697 | 299664 | 298681 | 292 |
| 1836 | 1790705 | 1791568 | 298673 | 297810 | 662 |
| 1837 | 1791624 | 1791959 | 297754 | 297419 | 1038 |
| 1838 | 1791963 | 1792769 | 297415 | 296609 | 1039 |
| 1839 | 1792792 | 1793328 | 296586 | 296050 | 293 |
| 1840 | 1793325 | 1794524 | 296053 | 294854 | 1521 |
| 1841 | 1794521 | 1794823 | 294857 | 294555 | 1872 |
| 1842 | 1794964 | 1796124 | 294414 | 293254 | 294 |
| 1843 | 1796129 | 1797154 | 293249 | 292224 | 1871 |
| 1844 | 1797235 | 1797561 | 292143 | 291817 | 1158 |
| 1845 | 1797561 | 1797665 | 291817 | 291713 | 1520 |
| 1846 | 1797874 | 1798116 | 291504 | 291262 | 1157 |

(continued)

| gene No. | Nucleic acid No. (sense chain (corresponding to SEQ ID NO: 1, 342, 723), starting nucleotide | Nucleic acid No. (sense chain (corresponding to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1847 | 1798158 | 1800545 | 291220 | 288833 | 1519 |
| 1848 | 1800686 | 1801306 | 288692 | 288072 | 1870 |
| 1849 | 1801592 | 1802125 | 287786 | 287253 | 663 |
| 1850 | 1802245 | 1803363 | 287133 | 286015 | 1156 |
| 1851 | 1803363 | 1803602 | 286015 | 285776 | 1518 |
| 1852 | 1803666 | 1804280 | 285712 | 285098 | 1040 |
| 1853 | 1804317 | 1804535 | 285061 | 284843 | 1517 |
| 1854 | 1804571 | 1805047 | 284807 | 284331 | 1869 |
| 1855 | 1805521 | 1805853 | 283857 | 283525 | 1155 |
| 1856 | 1805911 | 1806657 | 283467 | 282721 | 1154 |
| 1857 | 1806654 | 1807073 | 282724 | 282305 | 1516 |
| 1858 | 1807161 | 1808084 | 282217 | 281294 | 1041 |
| 1859 | 1808249 | 1808404 | 281129 | 280974 | 664 |
| 1860 | 1808394 | 1808819 | 280984 | 280559 | 1515 |
| 1861 | 1808985 | 1811618 | 280393 | 277760 | 1042 |
| 1862 | 1811744 | 1812487 | 277634 | 276891 | 665 |
| 1863 | 1812518 | 1813510 | 276860 | 275868 | 1868 |
| 1864 | 1813353 | 1813550 | 276025 | 275828 | 1043 |
| 1865 | 1813638 | 1814054 | 275740 | 275324 | 1514 |
| 1866 | 1814141 | 1814644 | 275237 | 274734 | 1867 |
| 1867 | 1814559 | 1814648 | 274819 | 274730 | 1044 |
| 1868 | 1814829 | 1815962 | 274549 | 273416 | 1045 |
| 1869 | 1815959 | 1817002 | 273419 | 272376 | 666 |
| 1870 | 1816999 | 1817745 | 272379 | 271633 | 295 |
| 1871 | 1817756 | 1818715 | 271622 | 270663 | 667 |
| 1872 | 1819570 | 1819776 | 269808 | 269602 | 1153 |
| 1873 | 1820187 | 1820936 | 269191 | 268442 | 1513 |
| 1874 | 1820961 | 1821659 | 268417 | 267719 | 1512 |
| 1875 | 1821659 | 1821841 | 267719 | 267537 | 1866 |
| 1876 | 1822105 | 1823073 | 267273 | 266305 | 296 |
| 1877 | 1823702 | 1823782 | 265676 | 265596 | 1865 |
| 1878 | 1823857 | 1824675 | 265521 | 264703 | 297 |
| 1879 | 1824662 | 1825624 | 264716 | 263754 | 1864 |
| 1880 | 1825648 | 1826151 | 263730 | 263227 | 298 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1881 | 1826226 | 1826504 | 263152 | 262874 | 1511 |
| 1882 | 1826572 | 1826886 | 262806 | 262492 | 299 |
| 1883 | 1826859 | 1827470 | 262519 | 261908 | 1046 |
| 1884 | 1827563 | 1828408 | 261815 | 260970 | 1863 |
| 1885 | 1828493 | 1829698 | 260885 | 259680 | 668 |
| 1886 | 1829731 | 1830558 | 259647 | 258820 | 300 |
| 1887 | 1830621 | 1831115 | 258757 | 258263 | 1510 |
| 1888 | 1831076 | 1831645 | 258302 | 257733 | 1862 |
| 1889 | 1831699 | 1832772 | 257679 | 256606 | 301 |
| 1890 | 1832777 | 1833709 | 256601 | 255669 | 669 |
| 1891 | 1833706 | 1834158 | 255672 | 255220 | 1152 |
| 1892 | 1834155 | 1834856 | 255223 | 254522 | 1509 |
| 1893 | 1834992 | 1835603 | 254386 | 253775 | 1047 |
| 1894 | 1835581 | 1836201 | 253797 | 253177 | 302 |
| 1895 | 1836239 | 1837111 | 253139 | 252267 | 670 |
| 1896 | 1837108 | 1838508 | 252270 | 250870 | 1151 |
| 1897 | 1838515 | 1839846 | 250863 | 249532 | 1150 |
| 1898 | 1839843 | 1842821 | 249535 | 246557 | 1508 |
| 1899 | 1842996 | 1844864 | 246382 | 244514 | 1507 |
| 1900 | 1844947 | 1845273 | 244431 | 244105 | 303 |
| 1901 | 1845241 | 1845942 | 244137 | 243436 | 1149 |
| 1902 | 1845932 | 1846168 | 243446 | 243210 | 671 |
| 1903 | 1846267 | 1847184 | 243111 | 242194 | 1148 |
| 1904 | 1847191 | 1848111 | 242187 | 241267 | 1147 |
| 1905 | 1848117 | 1849664 | 241261 | 239714 | 1506 |
| 1906 | 1853437 | 1853742 | 235941 | 235636 | 1146 |
| 1907 | 1853826 | 1853894 | 235552 | 235484 | 1048 |
| 1908 | 1853933 | 1854607 | 235445 | 234771 | 1861 |
| 1909 | 1854612 | 1855832 | 234766 | 233546 | 1505 |
| 1910 | 1855928 | 1857586 | 233450 | 231792 | 1860 |
| 1911 | 1857656 | 1858012 | 231722 | 231366 | 672 |
| 1912 | 1858017 | 1859300 | 231361 | 230078 | 1504 |
| 1913 | 1859380 | 1859607 | 229998 | 229771 | 1145 |
| 1914 | 1859695 | 1860141 | 229683 | 229237 | 1144 |

**EP 1 923 464 A1**

(continued)

| gene No. | Nucleic acid No. (sense chain (corresponding to SEQ ID NO: 1, 342, 723), starting nucleotide | Nucleic acid No. (sense chain (corresponding to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1915 | 1860556 | 1860741 | 228822 | 228637 | 1143 |
| 1916 | 1860814 | 1862100 | 228564 | 227278 | 1142 |
| 1917 | 1862097 | 1862900 | 227281 | 226478 | 1503 |
| 1918 | 1862902 | 1863786 | 226476 | 225592 | 1141 |
| 1919 | 1863783 | 1864895 | 225595 | 224483 | 1502 |
| 1920 | 1865656 | 1866711 | 223722 | 222667 | 304 |
| 1921 | 1866693 | 1867223 | 222685 | 222155 | 1049 |
| 1922 | 1867473 | 1868666 | 221905 | 220712 | 1050 |
| 1923 | 1868696 | 1869637 | 220682 | 219741 | 673 |
| 1924 | 1869643 | 1870143 | 219735 | 219235 | 305 |
| 1925 | 1870833 | 1871861 | 218545 | 217517 | 1051 |
| 1926 | 1872015 | 1872557 | 217363 | 216821 | 1052 |
| 1927 | 1872533 | 1872811 | 216845 | 216567 | 674 |
| 1928 | 1872808 | 1873179 | 216570 | 216199 | 306 |
| 1929 | 1873176 | 1873442 | 216202 | 215936 | 1053 |
| 1930 | 1873439 | 1873735 | 215939 | 215643 | 675 |
| 1931 | 1873732 | 1874181 | 215646 | 215197 | 307 |
| 1932 | 1874169 | 1874537 | 215209 | 214841 | 1054 |
| 1933 | 1874534 | 1876078 | 214844 | 213300 | 676 |
| 1934 | 1876071 | 1876427 | 213307 | 212951 | 1055 |
| 1935 | 1876465 | 1876995 | 212913 | 212383 | 308 |
| 1936 | 1876992 | 1877561 | 212386 | 211817 | 1056 |
| 1937 | 1877558 | 1878838 | 211820 | 210540 | 677 |
| 1938 | 1878843 | 1879835 | 210535 | 209543 | 1057 |
| 1939 | 1879832 | 1880263 | 209546 | 209115 | 678 |
| 1940 | 1880264 | 1880797 | 209114 | 208581 | 1859 |
| 1941 | 1880784 | 1881278 | 208594 | 208100 | 1501 |
| 1942 | 1881271 | 1881759 | 208107 | 207619 | 1140 |
| 1943 | 1881790 | 1882272 | 207588 | 207106 | 1139 |
| 1944 | 1882334 | 1883542 | 207044 | 205836 | 679 |
| 1945 | 1883543 | 1884076 | 205835 | 205302 | 680 |
| 1946 | 1884157 | 1885149 | 205221 | 204229 | 309 |
| 1947 | 1885281 | 1886627 | 204097 | 202751 | 1058 |
| 1948 | 1886671 | 1887270 | 202707 | 202108 | 310 |

83

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1949 | 1887267 | 1887560 | 202111 | 201818 | 1500 |
| 1950 | 1887544 | 1888218 | 201834 | 201160 | 1138 |
| 1951 | 1888724 | 1890025 | 200654 | 199353 | 681 |
| 1952 | 1890006 | 1890557 | 199372 | 198821 | 1499 |
| 1953 | 1890634 | 1894026 | 198744 | 195352 | 311 |
| 1954 | 1894318 | 1894365 | 195060 | 195013 | 312 |
| 1955 | 1894442 | 1895158 | 194936 | 194220 | 682 |
| 1956 | 1895222 | 1895692 | 194156 | 193686 | 1858 |
| 1957 | 1895730 | 1896284 | 193648 | 193094 | 1498 |
| 1958 | 1896330 | 1896818 | 193048 | 192560 | 1497 |
| 1959 | 1896886 | 1897806 | 192492 | 191572 | 313 |
| 1960 | 1897803 | 1898744 | 191575 | 190634 | 1496 |
| 1961 | 1898830 | 1899255 | 190548 | 190123 | 1137 |
| 1962 | 1899309 | 1900178 | 190069 | 189200 | 1059 |
| 1963 | 1900171 | 1900881 | 189207 | 188497 | 1136 |
| 1964 | 1901205 | 1901720 | 188173 | 187658 | 1495 |
| 1965 | 1901783 | 1902706 | 187595 | 186672 | 683 |
| 1966 | 1902746 | 1903273 | 186632 | 186105 | 684 |
| 1967 | 1903277 | 1904434 | 186101 | 184944 | 685 |
| 1968 | 1904431 | 1905462 | 184947 | 183916 | 314 |
| 1969 | 1905501 | 1906337 | 183877 | 183041 | 1060 |
| 1970 | 1906334 | 1907098 | 183044 | 182280 | 1857 |
| 1971 | 1907089 | 1908066 | 182289 | 181312 | 1135 |
| 1972 | 1908127 | 1909461 | 181251 | 179917 | 1134 |
| 1973 | 1909517 | 1910014 | 179861 | 179364 | 686 |
| 1974 | 1910023 | 1910727 | 179355 | 178651 | 315 |
| 1975 | 1912010 | 1912546 | 177368 | 176832 | 687 |
| 1976 | 1912651 | 1912902 | 176727 | 176476 | 316 |
| 1977 | 1912921 | 1913589 | 176457 | 175789 | 1133 |
| 1978 | 1913472 | 1914050 | 175906 | 175328 | 1494 |
| 1979 | 1914387 | 1914812 | 174991 | 174566 | 1493 |
| 1980 | 1914882 | 1916204 | 174496 | 173174 | 1492 |
| 1981 | 1916252 | 1916479 | 173126 | 172899 | 688 |
| 1982 | 1916521 | 1917351 | 172857 | 172027 | 317 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 1983 | 1917310 | 1917879 | 172068 | 171499 | 1132 |
| 1984 | 1918215 | 1918709 | 171163 | 170669 | 1061 |
| 1985 | 1918693 | 1920390 | 170685 | 168988 | 1131 |
| 1986 | 1920429 | 1921331 | 168949 | 168047 | 1491 |
| 1987 | 1921407 | 1923065 | 167971 | 166313 | 1490 |
| 1988 | 1923377 | 1923970 | 166001 | 165408 | 1856 |
| 1989 | 1923967 | 1924317 | 165411 | 165061 | 1130 |
| 1990 | 1924478 | 1926250 | 164900 | 163128 | 689 |
| 1991 | 1926252 | 1926566 | 163126 | 162812 | 1062 |
| 1992 | 1926707 | 1929025 | 162671 | 160353 | 690 |
| 1993 | 1929037 | 1930491 | 160341 | 158887 | 1129 |
| 1994 | 1930573 | 1930920 | 158805 | 158458 | 318 |
| 1995 | 1930917 | 1931588 | 158461 | 157790 | 1063 |
| 1996 | 1931535 | 1932002 | 157843 | 157376 | 1489 |
| 1997 | 1932193 | 1932927 | 157185 | 156451 | 319 |
| 1998 | 1932928 | 1933236 | 156450 | 156142 | 1128 |
| 1999 | 1933306 | 1933578 | 156072 | 155800 | 320 |
| 2000 | 1933671 | 1934051 | 155707 | 155327 | 1064 |
| 2001 | 1934029 | 1935735 | 155349 | 153643 | 1127 |
| 2002 | 1935745 | 1936650 | 153633 | 152728 | 1126 |
| 2003 | 1936888 | 1937835 | 152490 | 151543 | 1125 |
| 2004 | 1937965 | 1939305 | 151413 | 150073 | 1124 |
| 2005 | 1941378 | 1941863 | 148000 | 147515 | 1065 |
| 2006 | 1942184 | 1942507 | 147194 | 146871 | 691 |
| 2007 | 1942618 | 1944576 | 146760 | 144802 | 1123 |
| 2008 | 1944729 | 1945865 | 144649 | 143513 | 1488 |
| 2009 | 1945993 | 1946349 | 143385 | 143029 | 1122 |
| 2010 | 1947328 | 1948446 | 142050 | 140932 | 321 |
| 2011 | 1948368 | 1949834 | 141010 | 139544 | 1066 |
| 2012 | 1949788 | 1951875 | 139590 | 137503 | 1121 |
| 2013 | 1951825 | 1953192 | 137553 | 136186 | 322 |
| 2014 | 1953189 | 1954478 | 136189 | 134900 | 1067 |
| 2015 | 1954540 | 1955208 | 134838 | 134170 | 323 |
| 2016 | 1955253 | 1957394 | 134125 | 131984 | 1068 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 2017 | 1957397 | 1958206 | 131981 | 131172 | 1855 |
| 2018 | 1958454 | 1958975 | 130924 | 130403 | 1487 |
| 2019 | 1959384 | 1959980 | 129994 | 129398 | 1486 |
| 2020 | 1959997 | 1960209 | 129381 | 129169 | 1120 |
| 2021 | 1961911 | 1965690 | 127467 | 123688 | 1119 |
| 2022 | 1962226 | 1962360 | 127152 | 127018 | 324 |
| 2023 | 1964567 | 1964629 | 124811 | 124749 | 692 |
| 2024 | 1965873 | 1966658 | 123505 | 122720 | 1069 |
| 2025 | 1966899 | 1969403 | 122479 | 119975 | 1070 |
| 2026 | 1969396 | 1970652 | 119982 | 118726 | 325 |
| 2027 | 1970804 | 1971262 | 118574 | 118116 | 693 |
| 2028 | 1971328 | 1971672 | 118050 | 117706 | 326 |
| 2029 | 1971682 | 1972395 | 117696 | 116983 | 327 |
| 2030 | 1972493 | 1973851 | 116885 | 115527 | 694 |
| 2031 | 1974299 | 1975357 | 115079 | 114021 | 1854 |
| 2032 | 1975695 | 1977017 | 113683 | 112361 | 1071 |
| 2033 | 1976971 | 1977399 | 112407 | 111979 | 1118 |
| 2034 | 1977396 | 1977704 | 111982 | 111674 | 1485 |
| 2035 | 1977819 | 1978400 | 111559 | 110978 | 1484 |
| 2036 | 1978397 | 1978993 | 110981 | 110385 | 1853 |
| 2037 | 1978966 | 1979769 | 110412 | 109609 | 1117 |
| 2038 | 1979866 | 1980489 | 109512 | 108889 | 328 |
| 2039 | 1980484 | 1980942 | 108894 | 108436 | 1116 |
| 2040 | 1980946 | 1981878 | 108432 | 107500 | 1115 |
| 2041 | 1981986 | 1982897 | 107392 | 106481 | 1072 |
| 2042 | 1982894 | 1983307 | 106484 | 106071 | 695 |
| 2043 | 1983573 | 1984325 | 105805 | 105053 | 1483 |
| 2044 | 1984369 | 1985724 | 105009 | 103654 | 1114 |
| 2045 | 1985942 | 1987522 | 103436 | 101856 | 696 |
| 2046 | 1987535 | 1988848 | 101843 | 100530 | 1852 |
| 2047 | 1988883 | 1989671 | 100495 | 99707 | 1482 |
| 2048 | 1989712 | 1990701 | 99666 | 98677 | 1113 |
| 2049 | 1991043 | 1992029 | 98335 | 97349 | 1481 |
| 2050 | 1992178 | 1993323 | 97200 | 96055 | 1112 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 2051 | 1993320 | 1993928 | 96058 | 95450 | 1480 |
| 2052 | 1993956 | 1994684 | 95422 | 94694 | 1479 |
| 2053 | 1994681 | 1995694 | 94697 | 93684 | 1851 |
| 2054 | 1995731 | 1997062 | 93647 | 92316 | 1850 |
| 2055 | 1997062 | 1999713 | 92316 | 89665 | 1111 |
| 2056 | 1999710 | 2001092 | 89668 | 88286 | 1478 |
| 2057 | 2001233 | 2003020 | 88145 | 86358 | 1849 |
| 2058 | 2003136 | 2003711 | 86242 | 85667 | 1073 |
| 2059 | 2003696 | 2004217 | 85682 | 85161 | 697 |
| 2060 | 2004220 | 2004576 | 85158 | 84802 | 1110 |
| 2061 | 2004890 | 2004943 | 84488 | 84435 | 698 |
| 2062 | 2005188 | 2006615 | 84190 | 82763 | 1477 |
| 2063 | 2006536 | 2009136 | 82842 | 80242 | 329 |
| 2064 | 2009133 | 2010641 | 80245 | 78737 | 1074 |
| 2065 | 2010697 | 2012013 | 78681 | 77365 | 330 |
| 2066 | 2012072 | 2012314 | 77306 | 77064 | 699 |
| 2067 | 2012311 | 2012514 | 77067 | 76864 | 1109 |
| 2068 | 2012712 | 2013572 | 76666 | 75806 | 1476 |
| 2069 | 2013609 | 2014661 | 75769 | 74717 | 1475 |
| 2070 | 2014525 | 2015568 | 74853 | 73810 | 1108 |
| 2071 | 2015632 | 2016564 | 73746 | 72814 | 1107 |
| 2072 | 2016684 | 2017421 | 72694 | 71957 | 1075 |
| 2073 | 2017378 | 2018802 | 72000 | 70576 | 331 |
| 2074 | 2019182 | 2019406 | 70196 | 69972 | 1848 |
| 2075 | 2019763 | 2020425 | 69615 | 68953 | 1106 |
| 2076 | 2020435 | 2021076 | 68943 | 68302 | 1105 |
| 2077 | 2021157 | 2021522 | 68221 | 67856 | 1076 |
| 2078 | 2021495 | 2022214 | 67883 | 67164 | 700 |
| 2079 | 2022269 | 2023111 | 67109 | 66267 | 701 |
| 2080 | 2025340 | 2025417 | 64038 | 63961 | 332 |
| 2081 | 2028631 | 2028912 | 60747 | 60466 | 333 |
| 2082 | 2028914 | 2029489 | 60464 | 59889 | 702 |
| 2083 | 2029483 | 2030094 | 59895 | 59284 | 1104 |
| 2084 | 2030142 | 2031023 | 59236 | 58355 | 1474 |

(continued)

| gene No. | Nucleic acid No. (sense chain (corresponding to SEQ ID NO: 1, 342, 723), starting nucleotide | Nucleic acid No. (sense chain (corresponding to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 2085 | 2031138 | 2032727 | 58240 | 56651 | 1077 |
| 2086 | 2032734 | 2033420 | 56644 | 55958 | 1473 |
| 2087 | 2033501 | 2034466 | 55877 | 54912 | 703 |
| 2088 | 2034330 | 2035610 | 55048 | 53768 | 1078 |
| 2089 | 2035637 | 2036254 | 53741 | 53124 | 704 |
| 2090 | 2036331 | 2036594 | 53047 | 52784 | 1079 |
| 2091 | 2036609 | 2037244 | 52769 | 52134 | 705 |
| 2092 | 2037290 | 2038219 | 52088 | 51159 | 706 |
| 2093 | 2038219 | 2039394 | 51159 | 49984 | 334 |
| 2094 | 2039429 | 2040040 | 49949 | 49338 | 707 |
| 2095 | 2039994 | 2040326 | 49384 | 49052 | 1080 |
| 2096 | 2040316 | 2040816 | 49062 | 48562 | 1103 |
| 2097 | 2040797 | 2041732 | 48581 | 47646 | 1847 |
| 2098 | 2043010 | 2044203 | 46368 | 45175 | 1102 |
| 2099 | 2044340 | 2045170 | 45038 | 44208 | 708 |
| 2100 | 2045127 | 2046032 | 44251 | 43346 | 1472 |
| 2101 | 2046077 | 2047399 | 43301 | 41979 | 709 |
| 2102 | 2047406 | 2047780 | 41972 | 41598 | 710 |
| 2103 | 2047777 | 2048313 | 41601 | 41065 | 1101 |
| 2104 | 2048320 | 2049099 | 41058 | 40279 | 1100 |
| 2105 | 2049106 | 2049471 | 40272 | 39907 | 1099 |
| 2106 | 2050697 | 2051614 | 38681 | 37764 | 711 |
| 2107 | 2051664 | 2051900 | 37714 | 37478 | 1081 |
| 2108 | 2051888 | 2052298 | 37490 | 37080 | 712 |
| 2109 | 2052295 | 2053014 | 37083 | 36364 | 335 |
| 2110 | 2053125 | 2053190 | 36253 | 36188 | 1082 |
| 2111 | 2055992 | 2057146 | 33386 | 32232 | 1846 |
| 2112 | 2057204 | 2057467 | 32174 | 31911 | 1845 |
| 2113 | 2057477 | 2058655 | 31901 | 30723 | 1844 |
| 2114 | 2058742 | 2059149 | 30636 | 30229 | 1098 |
| 2115 | 2059310 | 2059501 | 30068 | 29877 | 713 |
| 2116 | 2059560 | 2060801 | 29818 | 28577 | 1083 |
| 2117 | 2060819 | 2061598 | 28559 | 27780 | 714 |
| 2118 | 2061501 | 2061911 | 27877 | 27467 | 1084 |

(continued)

| gene No. | Nucleic acid No. (sense chain (corresponding to SEQ ID NO: 1, 342, 723), starting nucleotide | Nucleic acid No. (sense chain (correspondin g to SEQ ID NO: 1, 342, 723), ending nucleotide | Nucleic acid No. (antisense chain (corresponding to SEQ ID NO: 1087, 1469, 1838), ending nucleotide | Nucleic acid No. (antisense chain (corresponding to SEQ ID NO: 1087, 1469, 1838), starting nucleotide | Corresponding SEQ ID NO. |
|---|---|---|---|---|---|
| 2119 | 2061997 | 2062446 | 27381 | 26932 | 1097 |
| 2120 | 2062448 | 2062966 | 26930 | 26412 | 1843 |
| 2121 | 2062966 | 2063607 | 26412 | 25771 | 1096 |
| 2122 | 2063612 | 2064214 | 25766 | 25164 | 1842 |
| 2123 | 2064280 | 2065428 | 25098 | 23950 | 1095 |
| 2124 | 2065471 | 2066778 | 23907 | 22600 | 1094 |
| 2125 | 2066863 | 2067558 | 22515 | 21820 | 336 |
| 2126 | 2067623 | 2068384 | 21755 | 20994 | 715 |
| 2127 | 2068384 | 2069838 | 20994 | 19540 | 337 |
| 2128 | 2069828 | 2070184 | 19550 | 19194 | 1841 |
| 2129 | 2070189 | 2070728 | 19189 | 18650 | 1471 |
| 2130 | 2070778 | 2071599 | 18600 | 17779 | 1093 |
| 2131 | 2071722 | 2072069 | 17656 | 17309 | 1085 |
| 2132 | 2072066 | 2072986 | 17312 | 16392 | 716 |
| 2133 | 2073002 | 2073490 | 16376 | 15888 | 717 |
| 2134 | 2073534 | 2073737 | 15844 | 15641 | 1470 |
| 2135 | 2074012 | 2075424 | 15366 | 13954 | 338 |
| 2136 | 2075557 | 2076162 | 13821 | 13216 | 339 |
| 2137 | 2076199 | 2076411 | 13179 | 12967 | 1092 |
| 2138 | 2076528 | 2076959 | 12850 | 12419 | 1086 |
| 2139 | 2076986 | 2077663 | 12392 | 11715 | 718 |
| 2140 | 2077703 | 2078152 | 11675 | 11226 | 719 |
| 2141 | 2078164 | 2078964 | 11214 | 10414 | 1091 |
| 2142 | 2079001 | 2080026 | 10377 | 9352 | 1090 |
| 2143 | 2080319 | 2082169 | 9059 | 7209 | 720 |
| 2144 | 2082376 | 2082897 | 7002 | 6481 | 340 |
| 2145 | 2082919 | 2083284 | 6459 | 6094 | 1089 |
| 2146 | 2083288 | 2084007 | 6090 | 5371 | 1088 |
| 2147 | 2084057 | 2085316 | 5321 | 4062 | 1840 |
| 2148 | 2085470 | 2087110 | 3908 | 2268 | 721 |
| 2149 | 2087216 | 2088568 | 2162 | 810 | 1839 |
| 2150 | 2088670 | 2088921 | 708 | 457 | 341 |
| 2151 | 2088905 | 2089378 | 473 | 0 | 722 |

In one embodiment, such a region is selected from the group consisting of genes (1) through (2151).

As used herein , in the above Table, translated amino acid sequences usually start with methionine, and is identified as "amino acid SEQ ID No: Y (SEQ ID NO: 2-341, 343-722, 724-1086, 1088-1468, 1470-1837, and 1839-2157)", however the other reading frames may also be readily translated using known molecular biological techniques. It is also understood that the polypeptide produced by another open reading frame is also encompassed in the scope of the present invention.

The accuracy of the sequence disclosed herein is sufficient and suitable for a variety of applications well known in the art and further described hereinbelow. For example, the sequence of the open reading frame region of SEQ ID NO: 1 is useful for designing a nucleic acid hybridization probe for detection of cDNA contained in the nucleic acid sequence in the open reading frame. These probes also hybridize with a nucleic acid molecule in a biological sample, thereby allowing a variety of forensic and diagnostic methods of the present invention. Similarly, the polypeptide identified by SEQ ID NO: Z may be used for, for example, producing an antibody specifically binding to a protein (including a polypeptide and secreted protein) encoded by an open reading frame identified herein.

Although we have analyzed the sequence of the present invention with special care, DNA sequences produced by sequencing reactions may comprise an error in sequencing. This error may be present as an incorrectly identified nucleotide, or as an insertion or a deletion of a nucleotide, in the DNA sequence produced. Incorrectly inserted or deleted nucleotides cause frame shifts in the deduced amino acid sequence of the reading frame. In such cases, the produced DNA sequences may be identical with more than 99. 9 % identity (for example, 1 base insertion or deletion in an open reading frame over 1000 bases), but the deduced amino acid sequence may differ from the actual amino acid sequence.

Accordingly, in these applications where accuracy is required in nucleotide or amino acid sequence, the present invention also provides the nucleic acid sequence and the amino acid sequence encoded by the genome of Thermococcus kodakaraensis KOD1 of the present invention, which was deposited in the International Patent Organism Depositary (IPOD). Those skilled in the art may determine a more accurate sequence by sequencing the sequence of the deposited Thermococcus kodakaraensis KOD 1 of the present invention. What is also provided in the present invention are allelic variants, orthologs, and/or speicies homologs.

In another aspect, the present invention provides a nucleic acid molecule *per se* having a sequence set forth in SEQ ID NO: 1 or 1087. The nucleic acid molecule per se is useful in the gene targeting disruption method of the present invention.

In another aspect, the present invention provides a nucleic acid molecule comprising at least eight contiguous nucleic acid sequence of the sequence set forth in SEQ ID NO: 1 or 1087.

As used herein, the term "probe" refers to a substance for use in searching, which is a nucleic acid sequence having a variable length. Probes are variable depending on the use thereof. Examples of a nucleic acid molecule as a common probe include one having a nucleic acid sequence of at least about 8 nucleotides in length, preferably at least about 10 nucleotides, preferably at least about 15 nucleotides, preferably at least about 20 nucleotides, preferably at least about 30 nucleotides, preferably at least about 40 nucleotides, preferably at least about 50 nucleotides, preferably at least about 100 nucleotides, or may be at least about 6000 nucleotides. Probes are used for detecting an identical, similar or complementary nucleic acid sequence. Longer probes may be usually available from natural or recombinant sources, are very specific, and hybridize much slower than oligomers. Probes may be single- or double-stranded, and are designed to have specificity in technologies such as PCR, membrane based hybridization or ELIS and the like.

As used herein, the term "primer" refers to a nucleic acid sequence having variable length, and serves for initiation of elongation of a polynucleotide strand in a synthetic reaction of a nucleic acid such as a PCR. Examples of a nucleic acid molecule as a common primer include one having a nucleic acid sequence having a length of at least about 6 nucleotides, at least about 7 nucleotides, at least about 8 nucleotides, preferably at least about 10 nucleotides, preferably at least about 15 nucleotides, at least about 17 nucleotides, preferably at least about 20 nucleotides, preferably at least about 30 nucleotides, preferably at least about 40 nucleotides, preferably at least about 50 nucleotides, preferably at least about 100 nucleotides, or may be at least about 6000 nucleotides.

In one aspect, the present invention provides a polypeptide having an amino acid sequence selected from a group consisting of any Gene ID (1) through (2151) as listed in Table 1 (namely, SEQ ID NOs: 2-341, 343-722, 724-1086, 1088-1468, 1470-1837, and 1839-2157). The polypeptide of the present invention is preferably fused to another protein. These fusion proteins may be used for a variety of applications. For example, fusion of His tag, HA tag, Protein A, IgG domain and maltose binding protein to the polypeptide of the present invention facilitates purification (see also EP A 394,827, Traunecker et al., Nature, 331:84-86(1988)).

In another aspect, the present invention provides a peptide molecule comprising at least one amino acid sequence of an amino acid sequence selected from a group consisting of any Gene ID (1) through (2151) as listed in Table 1 (namely, SEQ ID NOs: 2-341, 343-722, 724-1086, 1088-1468, 1470-1837, and 1839-2157). Such peptide molecules may be used as an epitope. Preferably, such a peptide molecule may comprise at least about a 4 amino acid sequence, at least about a 5 amino acid sequence, at least about a 6 amino acid sequence, at least about a 7 amino acid sequence, at least about a 8 amino acid sequence, at least about a 9 amino acid sequence, at least about a 10 amino acid sequence, at least about a 15 amino acid sequence, at least about a 20 amino acid sequence, at least about a 30 amino acid sequence, at least about a 40 amino acid sequence, at least about a 50 amino acid sequence, or at least about a 100 amino acid sequence. The longer the peptide becomes, the higher the specificity thereof becomes.

As used herein the term "epitope" refers to a portion of a polypeptide having antigenicity or immunogenicity in an animal, preferably a mammal, and most preferably in a human. In a preferable embodiment, the invention comprises a polypeptide comprising an epitope, and a polynucleotide encoding the polypeptide. As used herein the term "immunogenic epitope" is defined as a portion of a protein inducing antibody reaction in an animal, as determined by any method known in the art such as those for producing an antibody described herein below (see for example, Geysen et al., Proc.Natl.Acad.Sci.USA 81:3998-4002(1983)). As used herein the term "antigenic epitope" refers to a portion of a protein capable of binding to an antibody in an immunologically specific manner, as determined by any method well known in the art, such as an immunoassay as described herein. Immunologically specific binding excludes non-immunological binding, but does not necessarily exclude cross-reaction with different antigens. Antigenic epitopes are not necessarily immunogenic.

Fragments working as an epitope may be produced in any method conventionally known in the art (for example, see Houghten, Proc. Natl. Acad. Sci. USA 82:5131-5135(1985); see also, US Patent No. 4,631,211).

As used herein an antigenic epitope may comprise usually at least three amino acids, preferably at least 4 amino acids, at least 5 amino acids, at least 6 amino acids, at least 7 amino acids, more preferably at least 8 amino acids, at least 9 amino acids, at least 10 amino acids, at least 11 amino acids, at least 12 amino acids, at least 13 amino acids, at least 14 amino acids, at least 154 amino acids, at least 20 amino acids, at least 25 amino acids, at least 30 amino acids, at least 40 amino acids, at least 50 amino acids, and most preferably comprises a sequence of between about 15 amino acids and 30 amino acids. Preferable polypeptides comprising an immunogenic epitope or antigenic epitope are at least 10, 15, 20, 25, 30, 35, 40, 45, 50, 55, 60, 65, 70, 75, 80, 85, 90, 95 or 100 amino acid residues in length. Still, non-exclusively preferable antigenic epitopes comprise antigenic epitopes and a portion thereof as disclosed herein. Antigenic epitopes are useful for raising an antibody capable of specifically binding to an epitope (including monoclonal antibodies). Preferable antigenic epitopes comprise any combination of the antigenic epitopes as disclosed herein and 2, 3, 4, 5 or more these antigenic epitopes. Antigenic epitopes may be used as a target molecule in an immunoassay (see, for example, Wilson et al., Cell 37:767-778(1984); Sutcliffe et al., Science 219: 660-666 (1983)).

Similarly, with respect to the use of an immunogenic epitope, for example, an antibody may be induced according to a method well known in the art (see, for example, Sutcliffe et al., (ibid.); Wilson et al., (ibid.); Chow et al., , Proc. Natl. Acad. Sci. USA 82:910-914; and Bittle et al., , J. Gen. Virol. 66: 2347-2354 (1985)). Preferable immunogenic epitopes are those immunogenic epitopes as disclosed herein, and any combination of two, three, four, five or more of these immunogenic epitopes. Polypeptides comprising one or more immunogenic epitopes may be presented for raising antibody response against an animal system (for example, rabbit or mouse) with a carrier protein (for example, albumin), or if the polypeptide is sufficiently long (at least about 25 amino acids), the polypeptide is presented withouth carrier. However, immunogenic epitopes as short as 8-10 amino acids have been shown to be sufficient for raising an antibody capable of binding to (at least) a linear epitope of a modified polypeptide (for example, by Western blotting).

Epitope-containing polypeptides of the present invention may be used for inducing an antibody according to a well known technology in the art. Such a method includes, but is not limited to in vivo immunization, *in vitro* immunization, and phage display method. For example, see Sutcliffe et al. ibid; Wilson et al., ibid; and Bittle et al., J. Gen. Virol., 66: 2347-2354 (1985). When using in vivo immunization, an animal may be immunized using a free peptide. However, anti-peptide antibody titer may be boosted by binding a peptide to a macromolecular carrier (for example, keyhole limpet hemocyanin (KLH) or tetanus toxoid). For example, a peptide comprising a cysteine residue, may be bound to a carrier by the use of a linker such as a maleidobenzoyl-N-hydroxysuccineimideester (MBS). On the other hand, another peptide may be bound to a carrier by the use of more general binder such as glutaraldehyde. An animal such as a rabbit, rat, or mouse may be immunized by peritoneal injection and/or intradermic injection of, for example, an emulsion (containing about 100 μg of a peptide or carrier protein and Freund's adjuvant or any other adjuvant known to stimulate an immunorespose). Some booster injections may be necessary to provide an effective titer of anti-peptide, for example, at about-two week intervals. This titer may be detected by an ELISA assay using a free peptide absorbed onto a solid surface. Titer of such anti-peptide antibodies in the serum derived from an immunized animal may be enhanced by selecting anti-peptide antibodies (for example, by absorption of the peptide on a solid support and elution of the selected antibody according to a well known method in the art).

As can be understood by those skilled in the art, and as discussed hereinabove, the polypeptide of the present invention comprising an immunogenic or antigenic epitope, may be fused to another polypeptide. For example, the polypeptide of the present invention may be fused to a constant domain or a portion thereof (CH1, CH2, CH3 or any combination or fragment thereof), or albumin (including, but not limited to, for example, recombinant albumin (see, for example, US Patent No. 5, 876, 969 (issued March 2, 1999), EP 0 413 622 and US Patent No. 5,766,883 (issued June 16, 1998), which are herein incorporated as reference in their entireties) to result in a chimeric protein. Such a fusion protein may facilitate purification, and enhance half-life *in vivo.* This has been demonstrated for the first two domains of a human CD4-polypeptide, and a chimeric protein consisting of a variety of domains from heavy chain or light chain constant regions of an immunoglobulin of a mammal. For example, see EP 394,827; Traunecker et al., Nature, 331: 84-86 (1988). An enhanced delivery of an antigen into the immune system across the epidermal barrier, has been demonstrated for an antigen (for example, insulin) bound to an IgG or a FcRn binding partner such as Fc fragment (see, PCT publications WO 96/22024 and WO 99/04812). IgG fusion proteins having a dimeric structure due to disulfide bonding of the IgG portions have also been demonstrated to be more effective in binding and neutralizing of another molecule, than a monomer polypeptide or a fragment thereof alone. See Fountoulakis et al., J.Biochem., 270: 3958-3964 (1995). A nucleic acid encoding the epitope may be recombined as a gene of interest as an epitope tag (for example, hemagglutinin "HA" or flag tag) to assist detection and purification of the expressed polypeptide. For example, a system described by Janknecht et al., allows simple purification of a non-modified fusion protein expressed in a human cell line (see Janknecht et al., 1991, Proc. Natl. Acad. Sci. USA 88: 8972-897). In this system, a gene of interest may be subcloned into a vaccinia recombinant plasmid to result in fusion of the open reading frame of the gene with an amino terminal tag consisting of six histidine residues upon translation. This tag functions as a substrate binding domain for the fusion protein. An extract from a cell infected with the recombinant vaccinia virus may be loaded onto a Ni2+ nitriloacetate-agarose column and a histidine tagged protein may be selectively eluted using imidazole containing buffer.

An "isolated" nucleic acid molecule is separated from the other nucleic acid molecules present in the natural source of the subject nucleic acid molecule. Examples of such isolated nucleic acid molecules include, but are not limited to, for example, recombinant DNA molecules contained in a vector, recombinant DNA molecules maintained in a heterologous host cell, nucleic acid molecules partially or substantially purified, and synthetic DNA or RNA molecules. Preferably, "isolated" nucleic acid is free of naturally flanking sequences to the subject nucleic acid in the genomic DNA of the organism from which the subject nucleic acid is derived (i.e., sequences located at 5' and 3' termini of the subject nucleic acid). For example, in a variety of embodiments, isolated novel nucleic acids molecules may include nucleotide sequence of less than about 50 kb, 25 kb, 5 kb, 4 kb, 3 kb, 2 kb, 1 kb, 0.5 kb or 0.1 kb. Further, "isolated" nucleic acid molecules, for example, cDNA molecules, may be substantially free of other cellular materials or culture medium when recombinantly produced, or of chemical precursors or other chemical substances when chemically synthesized.

In one aspect, the present invention provides a nucleic acid molecule comprising a sequence encoding an amino acid sequence having at least one amino acid sequence selected from the group consisting of Gene ID No. 1-2151 of Table 1 (at least one sequence selected from the group consisting of SEQ ID NOs: 2-341, 343-722, 724-1086, 1088-1468, 1470-1837 and 1839-2157); or a sequence having 70 % homology thereto.

In another aspect, the present invention provides a polpeptide, having at least one amino acid sequence selected from the group consisting of Gene ID No. 1-2151 of Table 1 (comprising at least one amino acid sequence selected from the group consisting of SEQ ID NO: 2-341, 343-722, 724-1086, 1088-1468, 1470-1837 and 1839-2157), or a sequence having at least 70 % homology thereto.

In another aspect, the present invention provides an epitope or a variant thereof, having at least one amino acid sequence selected from the group consisting of Gene ID No. 1-2151 of Table 1 (at least one amino acid sequence consisting of SEQ ID NO: 2-341, 343-722, 724-1086, 1088-1468, 1470-1837 and 1839-2157), or a sequence having at least 70 % homology thereto, or a portion thereof.

In another aspect, the present invention provides a method for screening for a thermostable protein. The present method comprises A) providing the entire sequence of the genome of a thermoresistant living organism; B) selecting at least one arbitrary region of the sequence; C) providing a vector comprising a sequence complementary to the selected region and a gene encoding a candidate for the heat resistance protein; D) transforming the living organism with the vector; E) placing the thermoresistant living organism in a condition causing possible homologous recombination; F) selecting the thermoresistant living organism in which homologous recombination has occurred; and G) assaying for identifying the thermoresistant protein. As used herein the entire sequence of the genome may not necessarily be a complete sequence, but preferably is an entire complete sequence. As used herein, as the selected region, two or more regions may be selected. The length of the region may be any length, as long as homologous recombination occurs, and includes, for example, at least about 500 bases, at least about 600 bases, at least about 700 bases, at least about 800 bases, at least about 900 bases, at least about 1000 bases, at least about 2000 bases, and the like. The candidate for the above thermotable proteins may be any protein of the present invention, as long as the expression thereof is expected. Vectors may be any vector, as long as they can express the protein of interest.

Vectors may preferably comprise gene regulation elements such as a promoter. Transformation may be any condition, as long as it is appropriate therefor.

Conditions causing homologous recombination may be any condition, as long as homologous recombination occurs under such conditions. Usually, the following condition may be used:

Tk-pyrF deleted strain No. 25, No. 27 are cultured in 20ml of ASW-YT liquid medium.
↓
Collect the bacteria from the culture medium (3ml) per one sample (No. 25, No. 27, five samples for each)
↓

Suspend the cells in 0.8×ASW+80mM CaCl$_2$ 200μl, and let stand on ice for 30 minutes

↓

3 μg pUC118/DS and 3 μg pUC118/DD are mixed and let stand on ice for 1 hour (two samples for each. Equivalent volume of TE buffer added to the sample was used as a control)

↓

heat shock at 85 °C, 45s

↓

let stand on ice for 10 minutes

↓

Preculture in Ura-ASW-AA liquid medium (proliferation occurs based on the incorporated uracil)

↓

Culture on Ura-ASW-AA liquid medium (enriched for PyrF+ strain)

↓

Culture on Ura-ASW-AA solid medium

The present invention is not limited to the above-condition. As used herein the composition of ASW (artificial sea water) is as follows: 1 x Artificial sea water (ASW) (/L) : NaCl 20g ; MgCl$_2$·6H$_2$O 3g; MgSO$_4$·7H$_2$O 6g; (NH$_4$)$_2$SO$_4$ 1g ; NaHCO$_3$ 0.2g ; CaCl$_2$ · 2H$_2$O 0. 3g ; KCl 0.5g ; NaBr 0.05g ; m SrCl$_2$ · 6H$_2$O 0.02g and Fe (NH$_4$) citric acid 0.01 g.

A method for selecting an organism in which homologous recombination has occurred may be performed by detecting a marker specific for the organism in which homologous recombination has occurred. Accordingly, it is preferable to use a marker which can be expressed in an organism which is expressed upon occurrence of homologous recombination, in the above-mentioned vector.

Identification of a thermostable protein may be performed by determining that the protein of interest is observed to have an activity under the same condition under which the protein usually attains the activity, but changes only the temperature to about 50 °C, preferably to about 60 °C, more preferably to about 70 °C, still more preferably to about 80 °C, most preferably to about 90 °C.

In another aspect, the present invention provides a kit for screening for a thermoresistant protein. The kit comprises A) a thermoresistant living organism; and B) a vector comprising a sequence complementary to the selected region and a gene encoding a candide for the thermoresistant protein.

In a preferable embodiment, the thermostable organism is a hyperthermophillic archaebacteria, and more preferably, Thermococcus kodakaraensis KOD1.

In a preferable embodiment, the kit of the present invention further comprises C) an assay system for identifying the thermoresistant protein. The assay system may vary depending on the activity of the thermostable protein of interest.

(Description of each gene)

Hereinafter, each gene comprised in the genomoic sequence of *Thermococcus kodakaraensis* KOD1 strain as identified in the present invention, is described.

(Overview of the genome of hyperthermophillic bacteria)

Chromosomal DNA of hyperthermophillic bacteria is stable. As double stranded DNA is maintained by hydrogen bonds, it is questionable if it will dissociate into single strands under higher temperature circumstances. KOD 1 strain has two types of basic histone-like proteins, which are stabilized by binding to the DNA, which is negatively charged, to form a nucleosome-like complex to be compacted. In the present invention, polyamines may be used to further enhance stabilization by binding to the same. Acetylated polyamine (acetyl polyamine) is weak in binding ability to the nucleosome-like complex, and thus can more firmly bind to polyamine obtained by the action of deacetylated enzyme. Generally, hyperthermophillic bacetria have much more intracellylar K$^+$ ion than a normal-temperature bacteria, and this should contribute to the stabilization of double-stranded DNA. Actually, when the melting curve of such DNA is observed, this property thereof is clearly demonstrated.

(Universality of thermophillic property)

The present inventors have found universal properties in proteins from hyperthermophillic bacteria through studies of glutumate dehydrogenase (GDH) of KOD-1 strain. That is, it has been demonstrated that proteins from ordinary temperature bacteria generally denature due to heat, whereas recombinant proteins from hyperthermophillic bacteria mature once heat is given. GDH synthesized in the high temperature circumstances in the KOD-1 strain has a hexamer structure and high specific activity. On the other hand, when the GDH gene is expressed in *E. coli* as a host, such GDH has weaker enzymatic activity than a natural form thereof, and is a monomer protein having a different structure. It was demonstrated that when heat treatment at 70 °C for twenty minutes was performed, a recombinant GDH developed similar specific activity and three-dimensional structure of the natural GDH. Once heat treatment is given, the present enzyme behaved similarly to the natural GDH thereof even in the lower temperature range. Such features were acknowledged for not only for GDH, but also all the enzymes anlayzed by the present inventors from hyperthermophillic bacteria. As such, heat is important for maturation of thermostable proteins, and was determined that this is due to irreversible structural change of enzymatic proteins by heat.

(Discovery of enzymes having new structures and functions)

Ribulose 1,5-bisphosphate carboxylase (Rubisco) is present in all the plants, algae, and cyanophyte, and plays an important role in fixing carbon dioxide to an organic material. Rubisco is the most abundant enzyme on earth, and is expected to heavily contribute to the solution of global warming or green house effects, and food problems. To date, archeabacteria, which is close to a primordial living organism, is believed not to possess a Rubisco, however, the present inventors have discovered Rubisco having high carbon dioxide fixation ability in the KOD-1 strain. The present enzyme (Tk-Rubisco) has twenty times greater activity than the conventional Rubisco, and the specificity to the carbon dioxide is extremely high. Tk-Rubisco is novel in terms of structure, and possesses the novel structure of a pentagonal decamaer. Presently, the analysis of physiological role of the present invention and introduction into a plant and the like is performed.

(Analysis of thermostable mechanism of proteins from hyperthermophillic bacteria based on three-dimensional structure)

High thermostablility presented by a protein derived from hyperthermophillic bacteria is not only from the basic field of protein sciences but also from a variety of applied field using the enzymes. The present inventors have clarified a number of three dimensional structures of enzymes derived from the KOD-1 strain, and also clarified a number of thermostable mechanisms. Typical examples thereof include $O^6$-methyl guanine-DNA methyl transferase (Tk-MGMT). Comparing the three dimensional structures of Tk-MGMT and the same derived from E. coli (AdaC), it was demonstrated that Tk-MGMT has a number of intrahelical ionic bond stablizing alpha-helices. Further, there were also a number of intrahelical ionic bonds stablizing the global protein structure. It was shown that AdaC derived from E. coli has less such ionic bonds, and thus the hyperthermophillic bacteria derived enzymes attain high thermostability by a number of ionic bonds and ionic bond networks. This is also true of the above-mentioned GDH, and also demonstrated biochemically. That is, when introducing site-directed mutations disrupting ionic bond networks present inside the GDH, thermostability of the variant enzyme is greatly reduced. On the other hand, a variant enzyme with increased ionic bonds enhanced its thermostability.

(Use of useful enzymes)

Polymerase chain reaction (PCR) method is an essential technology for gene engineering technologies, and the application thereof ranges from medicine, environment fields, to food industries and the like. Presently, improvements presently required for PCR methods, are the shortening of amplification time, prevention of misamplification, and the proliferation of long DNA fragments. In particular, clinical or food tests require rapid and accurate DNA synthesizing DNA polymerases. As a result of our functional analysis of the DNA polymerase (KOD DNA polymerase) from the KOD-1 strain, we found that the present enzyme has improved ability of synthesizing a longer DNA, and the speed of the synthesis of DNA is increased, in comparison of conventional enzymes. In fact, when the DNA polymerase from the KOD-1 strain is used, reaction time for PCR only takes 25 minutes, while the conventional Taq enzyme takes two hours. Further, modified enzyme with 3'->5' exonuclease activity of the KOD DNA polymerase, and the wild type enzyme can be mixed in an appropriate ratio to yield significantly superior reaction efficiency and amplification property. Further, the present inventors further have attained that an antibody to the KOD DNA polymerase is used to suppress mis-amplification which is often seen in the initial period of PCR reactions, and thus could establish an extremely efficient DNA amplification system. The present system is now commercially available from TOYOBO as "KOD-Plus-" in Japan, and available elsewhere thrrough Life Technologies/GIBCO BRL, as "Platinum™ Pfx DNA polymerase" including Europe and America. Recently, the present inventors have further analyzed the KOD DNA polymerase to determine the three dimensional structure thereof. Detailed three dimensional structure could be analyzed with respect to the speed of elongation reaction of the present enzyme, accuracy of the replication capability and the like, in view of what the structure is related to.

The present inventors have identified and analyzed a number of useful thermophillic enzymer other than DNA polymerases. DNA ligases catalyze reaction of binding termini of two DNA fragments, and thus are essential enzymes for genetic engineering. Most conventional enzymes from bacteria and phages are sensitive to heat and unstable. HOwever, the DNA ligase from KOD-1 strain (Tk-Lig) presented high DNA ligase activity from 30-100 °C. Further, substrate specificity in Nick-site of Tk-Lig (base-pairing) was interesting, and it was turned out that it was necessary to form accurate base-pairing against the 3' terminus, while substrate specificity was loose against the 5' terminus. No such DNA ligases having such features are reported to date, and these are expected to be applicable for detection of single nucleotide polymorphisms (SNPs). Sugar-related enzymes identified with respect to biochemical properties include alpha-amylase digesting alpha(1-4)bond as appears in starch and the like, or cyclodextrin glucanotransferase synthesizing cyclodextrine which catalyzes circulation, and 4-alpha-glucanotransferase, catalyzing a transferase reaction. Beta-glucosidase, which digests beta(1-4)bonds, appears in cellulose and chitin, and chitinase were also analyzed in detail. Two chitinase activities are present on the same polypeptide chain in chitinase from the KOD-1 strain, and one is responsable for endochitinase activity, while the other is responsable for exochitinase activity. These catalytic domains attain extremely high chitin degrading activity by synergy.

(Genomic analysis of Thermococcus kodakaraensis KOD-1 strain and Development of gene introduction technology)

Through the present studies, the present inventors have analyzed substantially all the genes relating to the KOD-1 strain, and revealed detailed biochemical properties of a huge variety of proteins. KOD-1 strain is a simple organism, located in the vicinity of the bottom of the evolutionary tree of organisms, and thus is believed to be a good tool for understanding basic mechanisms of life. Further, the KOD-1 strain produces a number of thermostable enzymes with broad applicability or novel enzymes with novel features as described above. Having such as background, the present inventors have proceeded with the entire genomic analysis of the KOD-1 strain. The genome of the KOD-1 strain consists of 2,076,138 base pairs, and is very short, as we have expected (40 % or less of that of *E. coli).* Further, there were about 1, 500 genes. As the KOD-1 strain maintains its life with such low number of genes, it is expected to allow analysis of basic principle of life through the research of the present bacteria.

The most important object of research in the post-genomic era is to analyze the physiological role of unknown genes. Exhaustive gene expression analysis by DNA chips, and exhaustive protein analysis by proteomics are effective analysis methods for these purposes. The present inventors have proceeded using these methods, and recently, have succeeded in constructing a novel system, which is an important new technology for specifically disrupting any gene of interest on the genome of the KOD-1 strain. This technology is used to disrupt a functionally-known gene to allow analysis and clarififcation of the physiological role thereof.

Genes comprised in the genome of KOD1 encompass a variety of species as listed in Table 2 below. Description of such genes are described in biochemistry references well known in the art, such as Sambrook,J.et al.Molecular Cloning:A Laboratory Manual,3rd Ed.Cold Spring Harbor Laboratory Press,Cold Spring Harbor, NY, USA (2001); Ausubel, F. et al.,Short protocols in molecular biology,4th ed.John Wiley&Sons,NJ,USA(1999);Ausubel,F.,et al.,Current Protocols in Molecular Biology, John Wiley&Sons,NJ,USA(1988); Jiro Ota ed., Biochemistry Handbook, Asakura Shoten, (1987); Kazutomo Imabori, Tamio Yamakawa ed., Seikagaku Jiten (Dictionary of BIOCHEMISTRY), Third Edition, Tokyo Kagaku Dojin (1998); Yasudomi NISHIDZUKA ed., Saibokino to Taisha mappu (Cellular Functions and Metabolism map), Tokyo Kagaku Dojin (1997); Lewin Genes VII, Oxford University Press, Oxford, UK (2000) and the like). Further, methods for measuring such function of a protein are described in for example, Sambrook,J.et al.Molecular Cloning:A Laboratory Manual,3rd Ed.Cold Spring Harbor Laboratory Press,Cold Spring Harbor, NY, USA (2001) ; Frank T.,et al., Thermophiles(Archaea:A Laboratory Manual 3),Cold Spring Harbor Laboratory Press,Cold Spring Harbor,NY,USA (1995); KOSOGAKU HANDOBUKKU (Enzyme handbook) edited by Bunj i MARUO, and Nobuo TAMIYA, published by Asakura shoten (1982); Methods in Enzymology series, Academic Press; Kazutomo Imabori, Tamio Yamakawa ed., Seikagaku Jiten (Dictionary of BIOCHEMISTRY), Third Edition, Tokyo Kagaku Dojin (1998); Yasudomi NISHIDZUKA ed., Saibokino to Taisha mappu (Cellular Functions and Metabolism map), Tokyo Kagaku Dojin (1997); Lengeler,J.et al.Biology of the Prokaryotes, Blackwell Science, Oxford, UK(1998); Lewin Genes VII, Oxford University Press, Oxford, UK(2000) and the like.

As such, the functions of genes comprised in the genome of KOD are revealed by the present invention, which are summarized in the following Table. Table 2 describes genes defined by the region (1) as described in Table 2 (hereinafter, Gene ID No. (1) and the like; the amino acid sequence of the gene is a sequence corresponding to the SEQ ID NO: set forth in SEQ ID NO: as described in the table).

TABLE 2 DECRIPTION OF GENES COMPRISED IN THE GENOME OF *Thermococcus kodakaraensis* KOD1

| GENE ID NO: | Nucleic acid No. (sense strand, start) | Nucleic acid No. (sense strand, stop) | Nucleic acid No. (antisense strand, stop) | Nucleic acid No. (antisense strand, start) | corresponding SEQ ID NO: | reading frame | start nucleic acid number having high homolgoy to known genes | stop nucleic acid number having high homolgoy to known genes | amino acid length | gene nomenclature | classification | Description |
|---|---|---|---|---|---|---|---|---|---|---|---|---|
| 1 | 1 | 5016 | 2089377 | 2084362 | 2 | f-1 | 1 | 1216 | 702 | PolB | L | DNA polymerase elongation subunit (family B) (homing endonuclease) |
| 2 | 5134 | 5733 | 2084244 | 2083645 | 3 | f-1 | 5134 | 5707 | 165 | - | R | Predicted metal-dependent hydrolase |
| 3 | 6079 | 6543 | 2083299 | 2082835 | 1468 | r-1 | 6424 | 6541 | 33 | CarB | EF | Carbamoylphosphate synthase large subunit (split gene in MJ) COG0458 CarB |
| 4 | 6586 | 7014 | 2082792 | 2082364 | 4 | f-1 | 6586 | 7012 | 262 | - | R | Predicted CoA-binding protein |
| 5 | 7152 | 7391 | 2082226 | 2081987 | 1837 | r-2 | 7170 | 7338 | 30 | - | R | Predicted ATPase or kinase |
| 6 | 7399 | 7614 | 2081979 | 2081764 | 1467 | r-1 | 7399 | 7549 | 29 | RpoZ | K | DNA-directed RNA polymerase subunit K/omega |

| | | | | | | | | | | | |
|---|---|---|---|---|---|---|---|---|---|---|---|
| 7 | 7655 | 8755 | 2081723 | 2080623 | 2157 | r-3 | 7658 | 8726 | 470 | - | L | Predicted DNA modification methylase |
| 8 | 8843 | 10093 | 2080535 | 2079285 | 343 | f-2 | 9011 | 9572 | 34 | - | G | Predicted N-acetylglucosaminyl transferase |
| 9 | 10095 | 10379 | 2079283 | 2078999 | 724 | f-3 | 10104 | 10299 | 30 | PutA | C | NAD-dependent aldehyde dehydrogenases |
| 10 | 10376 | 10807 | 2079002 | 2078571 | 344 | f-2 | 10385 | 10787 | 161 | - | S | Uncharacterized ACR |
| 11 | 10808 | 11416 | 2078570 | 2077962 | 2156 | r-3 | 10859 | 11414 | 277 | - | R | GTPases |
| 12 | 11406 | 11726 | 2077972 | 2077652 | 725 | f-3 | 11445 | 11646 | 30 | UgpQ | C | Glycerophosphoryl diester phosphodiesterase |
| 13 | 11723 | 12286 | 2077655 | 2077092 | 345 | f-2 | 11759 | 12275 | 150 | - | R | Predicted hydrolases of HD superfamily |
| 14 | 12338 | 13411 | 2077040 | 2075967 | 346 | f-2 | 12404 | 13391 | 550 | ModA | P | ABC-type molybdate transport system |
| 15 | 13392 | 13841 | 2075986 | 2075537 | 1836 | r-2 | 13425 | 13833 | 146 | - | R | Predicted nucleic acid-binding protein |
| 16 | 13808 | 14056 | 2075570 | 2075322 | 2155 | r-3 | 13841 | 14000 | 57 | AbrB | K | Regulators of stationary/sporulation gene expression |
| 17 | 14153 | 14896 | 2075225 | 2074482 | 347 | f-2 | 14159 | 14885 | 379 | CysU | P | ABC-type sulfate/molybdate transport systems |
| 18 | 15239 | 15964 | 2074139 | 2073414 | 348 | f-2 | 15371 | 15962 | 266 | - | R | Predicted ATPases |

| 19 | 16151 | 16699 | 2073227 | 2072679 | 349 | f-2 | 16505 | 16649 | 29 | - | R | Predicted ATPases of PP-loop superfamily |
| 20 | 16696 | 17697 | 2072682 | 2071681 | 5 | f-1 | 16708 | 17686 | 448 | CysA | P | ABC-type sulfate/molybdate transport systems |
| 21 | 17780 | 18793 | 2071598 | 2070585 | 2154 | r-3 | 17879 | 18437 | 40 | HflC | O | Membrane protease subunits |
| 22 | 18786 | 19280 | 2070592 | 2070098 | 1835 | r-2 | 18792 | 19251 | 29 | NqrA | C | Na+-transporting NADH:ubiquinone oxidoreductase alpha subunit |
| 23 | 19290 | 20183 | 2070088 | 2069195 | 1834 | r-2 | 19293 | 19407 | 32 | - | L | Archaea-specific RecJ-like exonuclease |
| 24 | 20183 | 21187 | 2069195 | 2068191 | 2153 | r-3 | 20645 | 20885 | 40 | Pnp | J | Polyribonucleotide nucleotidyltransferase (polynucleotide phosphorylase) |
| 25 | 21266 | 21919 | 2068112 | 2067459 | 2152 | r-3 | 21269 | 21908 | 223 | Gph | R | Predicted phosphatases |
| 26 | 21913 | 22569 | 2067465 | 2066809 | 1466 | r-1 | 21931 | 22552 | 320 | - | S | Uncharacterized ACR |
| 27 | 22597 | 24195 | 2066781 | 2065183 | 1465 | r-1 | 22921 | 24193 | 691 | SAM1 | H | S-adenosylhomocysteine hydrolase |
| 28 | 23947 | 24834 | 2065431 | 2064544 | 6 | f-1 | 23953 | 24808 | 141 | GloB | R | Zn-dependent hydrolases |
| 29 | 24813 | 25451 | 2064565 | 2063927 | 726 | f-3 | 24879 | 25446 | 218 | - | R | Uncharacterized ACR |
| 30 | 25413 | 25811 | 2063965 | 2063567 | 1833 | r-2 | 25476 | 25770 | 159 | RPR2 | J | RNAse P protein subunit RPR2 |

EP 1 923 464 A1

98

| | | | | | | | | | | | |
|---|---|---|---|---|---|---|---|---|---|---|---|
| 31 | 25813 | 27396 | 2063565 | 2061982 | 1464 | r-1 | 25930 | 27364 | 295 | MCM 2 | L | Predicted ATPase involved in replication control |
| 32 | 27565 | 28620 | 2061813 | 2060758 | 7 | f-1 | 27568 | 28012 | 42 | SbcC | L | ATPase involved in DNA repair |
| 33 | 28591 | 29334 | 2060787 | 2060044 | 1463 | r-1 | 28777 | 29116 | 33 | UshA | F | 5'-nucleotidase/2' |
| 34 | 29782 | 30681 | 2059596 | 2058697 | 8 | f-1 | 29791 | 30655 | 227 | - | S | Uncharacterized proteins of WD40-like repeat family |
| 35 | 31102 | 31266 | 2058276 | 2058112 | 9 | f-1 | 31102 | 31264 | 94 | - | S | Uncharacterized ArCR |
| 36 | 31414 | 32235 | 2057964 | 2057143 | 10 | f-1 | 31414 | 32182 | 270 | SmtA | Q R | SAM-dependent methyltransferases COG0500 SmtA |
| 37 | 32367 | 33251 | 2057011 | 2056127 | 727 | f-3 | 32382 | 33087 | 202 | FlaB | N | Archaeal flagellins (flagellin) |
| 38 | 33291 | 35033 | 2056087 | 2054345 | 728 | f-3 | 33309 | 33636 | 125 | FlaB | N | Archaeal flagellins (flagellin) |
| 39 | 35048 | 35824 | 2054330 | 2053554 | 350 | f-2 | 35048 | 35804 | 206 | FlaB | N | Archaeal flagellins (flagellin) |
| 40 | 35882 | 36541 | 2053496 | 2052837 | 351 | f-2 | 35888 | 36533 | 262 | FlaB | N | Archaeal flagellins (flagellin) |
| 41 | 36553 | 37380 | 2052825 | 2051998 | 11 | f-1 | 36553 | 37378 | 290 | FlaB | N | Archaeal flagellins (flagellin) |
| 42 | 37394 | 37870 | 2051984 | 2051508 | 352 | f-2 | 37541 | 37868 | 181 | FlaC | N | Putative archaeal flagellar protein C |
| 43 | 37874 | 39298 | 2051504 | 2050080 | 353 | f-2 | 38870 | 39296 | 258 | FlaD | N | Putative archaeal flagellar protein D/E |
| 44 | 39760 | 40332 | 2049618 | 2049046 | 12 | f-1 | 39862 | 40318 | 194 | FlaG | N | Putative archaeal flagellar protein G |

| 45 | 40360 | 41070 | 2049018 | 2048308 | 13 | f-1 | 40372 | 41068 | 385 | FlaH | N | Predicted ATPases involved in biogenesis of archaeal flagella |
|---|---|---|---|---|---|---|---|---|---|---|---|---|
| 46 | 41072 | 42694 | 2048306 | 2046684 | 354 | f-2 | 41072 | 42692 | 905 | VirB1 1 | N | Predicted ATPases involved in pili and flagella biosynthesis |
| 47 | 42696 | 44444 | 2046682 | 2044934 | 729 | f-3 | 42696 | 44436 | 656 | FlaJ | N | Uncharacterized membrane component of archaeal flagella |
| 48 | 44441 | 46435 | 2044937 | 2042943 | 355 | f-2 | 45869 | 46073 | 36 | - | R | Predicted helicases |
| 49 | 46470 | 46991 | 2042908 | 2042387 | 730 | f-3 | 46497 | 46986 | 294 | Pcm | O | Protein-L-isoaspartate carboxylmethyltransferase |
| 50 | 47171 | 47416 | 2042207 | 2041962 | 356 | f-2 | 47171 | 47321 | 60 | SerB | E | Phosphoserine phosphatase |
| 51 | 47317 | 47799 | 2042061 | 2041579 | 14 | f-1 | 47320 | 47794 | 143 | SerB | E | Phosphoserine phosphatase |
| 52 | 47937 | 49139 | 2041441 | 2040239 | 1832 | r-2 | 47943 | 49128 | 224 | PppA | N | Signal peptidase |
| 53 | 49153 | 49329 | 2040225 | 2040049 | 1462 | r-1 | | | | | | |
| 54 | 49393 | 49731 | 2039985 | 2039647 | 15 | f-1 | 49528 | 49669 | 28 | SPS1 | T | Serine/threonine protein kinases |
| 55 | 49728 | 50297 | 2039650 | 2039081 | 731 | f-3 | 49728 | 50292 | 246 | - | S | Uncharacterized ACR |
| 56 | 50278 | 50559 | 2039100 | 2038819 | 1461 | r-1 | 50290 | 50461 | 29 | - | R | STAS domain protein |
| 57 | 50693 | 51412 | 2038685 | 2037966 | 357 | f-2 | 50705 | 51410 | 276 | - | R | Predicted hydrolases of the HAD superfamily |
| 58 | 51483 | 52061 | 2037895 | 2037317 | 1831 | r-2 | 51492 | 52056 | 219 | PgsA | I | Phosphatidylglycerophosphate synthase |
| 59 | 52063 | 52605 | 2037315 | 2036773 | 1460 | r-1 | 52069 | 52603 | 276 | - | S | Uncharacterized ArCR |

| 60 | 52602 | 53792 | 2036776 | 2035586 | 1830 | r-2 | 53523 | 53715 | 32 | DnaX | L | DNA polymerase III |
|---|---|---|---|---|---|---|---|---|---|---|---|---|
| 61 | 54169 | 55020 | 2035209 | 2034358 | 16 | f-1 | 54250 | 55018 | 407 | - | S | Uncharacterized ACR |
| 62 | 55058 | 55606 | 2034320 | 2033772 | 358 | f-2 | 55322 | 55499 | 44 | - | R | Predicted nucleotidyltransferases |
| 63 | 55746 | 56018 | 2033632 | 2033360 | 732 | f-3 | 55749 | 56010 | 43 | - | S | Uncharacterized ACR |
| 64 | 56132 | 56263 | 2033246 | 2033115 | 359 | f-2 | | | | | | |
| 65 | 56244 | 56708 | 2033134 | 2032670 | 733 | f-3 | 56244 | 56661 | 99 | - | R | Predicted nucleic acid-binding protein |
| 66 | 56674 | 57267 | 2032704 | 2032111 | 17 | f-1 | 56710 | 57265 | 320 | NadR | H | Nicotinamide mononucleotide adenylyltransferase |
| 67 | 57264 | 57584 | 2032114 | 2031794 | 1829 | r-2 | 57408 | 57528 | 28 | AlsT | E | Na+/alanine symporter |
| 68 | 57599 | 58276 | 2031779 | 2031102 | 2151 | r-3 | 57722 | 58157 | 36 | - | R | Predicted helicases |
| 69 | 58855 | 59703 | 2030523 | 2029675 | 18 | f-1 | 58867 | 59701 | 481 | - | R | Predicted methyltransferase |
| 70 | 59704 | 59868 | 2029674 | 2029510 | 1459 | r-1 | 59725 | 59851 | 27 | FabG | Q R | Dehydrogenases with different specificities (related to short-chain alcohol dehydrogenases) COG1028 FabG |
| 71 | 59898 | 61799 | 2029480 | 2027579 | 1828 | r-2 | 59910 | 61719 | 390 | - | C | Aldehyde:ferredoxin oxidoreductase |
| 72 | 62830 | 63723 | 2026548 | 2025655 | 19 | f-1 | 62941 | 63376 | 40 | XerC | L | Integrase |
| 73 | 64226 | 65992 | 2025152 | 2023386 | 360 | f-2 | 64697 | 64985 | 35 | XynB | G | Beta-xylosidase |
| 74 | 66045 | 67382 | 2023333 | 2021996 | 734 | f-3 | 66330 | 66741 | 34 | FliD | N | Flagellar capping protein |

| 75 | 67399 | 68973 | 2021979 | 2020405 | 20 | f-1 | 68080 | 68833 | 173 | AprE | O | Subtilisin-like serine proteases |
|---|---|---|---|---|---|---|---|---|---|---|---|---|
| 76 | 69117 | 69374 | 2020261 | 2020004 | 735 | f-3 | 69240 | 69327 | 32 | - | R | Predicted membrane protein |
| 77 | 69583 | 69795 | 2019795 | 2019583 | 21 | f-1 | | | | | | |
| 78 | 69792 | 70511 | 2019586 | 2018867 | 736 | f-3 | 69903 | 70296 | 36 | FtsW | D | Bacterial cell division membrane protein |
| 79 | 70504 | 71112 | 2018874 | 2018266 | 22 | f-1 | 70885 | 70972 | 32 | - | Q | Phytoene dehydrogenase and related proteins |
| 80 | 71117 | 71245 | 2018261 | 2018133 | 361 | f-2 | 71123 | 71237 | 29 | GcvP | E | Glycine cleavage system protein P (pyridoxal-binding) |
| 81 | 71679 | 72593 | 2017699 | 2016785 | 737 | f-3 | 71922 | 72174 | 38 | IleS | J | Isoleucyl-tRNA synthetase |
| 82 | 72764 | 73339 | 2016614 | 2016039 | 362 | f-2 | 73049 | 73235 | 34 | - | K | Predicted transcriptional regulator |
| 83 | 73336 | 74643 | 2016042 | 2014735 | 23 | f-1 | 74005 | 74110 | 35 | GloB | R | Zn-dependent hydrolases |
| 84 | 74603 | 75760 | 2014775 | 2013618 | 363 | f-2 | | | | | | |
| 85 | 75753 | 76025 | 2013625 | 2013353 | 738 | f-3 | 75786 | 75972 | 28 | FabG | Q R | Dehydrogenases with different specificities (related to short-chain alcohol dehydrogenases) COG1028 FabG |
| 86 | 76022 | 77458 | 2013356 | 2011920 | 364 | f-2 | 76211 | 76475 | 34 | - | S | Uncharacterized BCR |
| 87 | 77735 | 79045 | 2011643 | 2010333 | 365 | f-2 | 77804 | 78005 | 34 | UshA | F | 5'-nucleotidase/2' |
| 88 | 79622 | 79726 | 2009756 | 2009652 | 2150 | r-3 | | | | | | |

EP 1 923 464 A1

| 89 | 79968 | 80129 | 2009410 | 2009249 | 739 | f-3 | 79968 | 80058 | 31 | AbrB | K | Regulators of stationary/sporulation gene expression |
|---|---|---|---|---|---|---|---|---|---|---|---|---|
| 90 | 80246 | 80428 | 2009132 | 2008950 | 366 | f-2 | 80318 | 80402 | 29 | CaiC | IQ | Acyl-CoA synthetases (AMP-forming)/AMP-acid ligases II COG0318 CaiC |
| 91 | 80432 | 83176 | 2008946 | 2006202 | 367 | f-2 | 81101 | 83075 | 233 | MCM 2 | L | Predicted ATPase involved in replication control |
| 92 | 83431 | 83628 | 2005947 | 2005750 | 24 | f-1 | 83440 | 83602 | 33 | GlpC | C | Fe-S  oxidoreductases |
| 93 | 83908 | 84267 | 2005470 | 2005111 | 25 | f-1 | 83947 | 84109 | 28 | - | E | Serine proteases of the peptidase family S9A |
| 94 | 84264 | 84440 | 2005114 | 2004938 | 740 | f-3 | 84303 | 84420 | 26 | DnaJ | O | Molecular chaperones (contain C-terminal Zn finger domain) |
| 95 | 84461 | 85018 | 2004917 | 2004360 | 368 | f-2 | 84530 | 84731 | 29 | | | |
| 96 | 84999 | 85340 | 2004379 | 2004038 | 741 | f-3 | 85002 | 85176 | 28 | - | R | Na+-dependent transporters of the SNF family |
| 97 | 85421 | 85948 | 2003957 | 2003430 | 369 | f-2 | 85448 | 85847 | 100 | XerC | L | Integrase |
| 98 | 86333 | 87139 | 2003045 | 2002239 | 2149 | r-3 | 86345 | 87128 | 428 | DPH5 | J | Diphthamide biosynthesis methyltransferase DPH5 |
| 99 | 87211 | 87663 | 2002167 | 2001715 | 26 | f-1 | 87226 | 87619 | 221 | TroR | K | Mn-dependent transcriptional regulator |

| No. | Description | | Gene | | | | | | | | | |
|---|---|---|---|---|---|---|---|---|---|---|---|---|
| 100 | Na+-driven multidrug efflux pump | Q | NorM | 39 | 88224 | 87912 | f-3 | 742 | 2001113 | 2001715 | 88265 | 87663 |
| 101 | DNA polymerase III alpha subunit | L | PolC | 32 | 88851 | 88395 | f-3 | 743 | 2000099 | 2001112 | 89279 | 88266 |
| 102 | Predicted hydrolases of the HAD superfamily | R | - | 286 | 90003 | 89319 | f-3 | 744 | 1999319 | 2000071 | 90059 | 89307 |
| 103 | Predicted Zn-ribbon RNA-binding protein with a function in translation | J | - | 131 | 90265 | 90088 | f-1 | 27 | 1999111 | 1999299 | 90267 | 90079 |
| 104 | Translation elongation factor EF-1beta | J | EFB1 | 167 | 90558 | 90285 | f-3 | 745 | 1998818 | 1999102 | 90560 | 90276 |
| 105 | Histone acetyltransferase HPA2 and related acetyltransferases COG0454 WecD | K R | WecD | 32 | 90976 | 90811 | r-1 | 1458 | 1998322 | 1998795 | 91056 | 90583 |
| 106 | Chorismate synthase | E | AroC | 28 | 91355 | 91268 | f-2 | 370 | 1998012 | 1998200 | 91366 | 91178 |
| 107 | Na+/proline | EH | PutP | 892 | 92974 | 91363 | f-1 | 28 | 1996399 | 1998015 | 92979 | 91363 |
| 108 | Uncharacterized NAD(FAD)-dependent dehydrogenases | R | HcaD | 717 | 94539 | 93072 | f-3 | 746 | 1994828 | 1996306 | 94550 | 93072 |
| 109 | Glycine/D-amino acid oxidases (deaminating) | E | DadA | 635 | 95710 | 94567 | f-1 | 29 | 1993666 | 1994826 | 95712 | 94552 |

| 110 | 96185 | 97636 | 1993193 | 1991742 | 371 | f-2 | 96185 | 97601 | 702 | HcaD | R | Uncharacterized NAD(FAD)-dependent dehydrogenases |
|---|---|---|---|---|---|---|---|---|---|---|---|---|
| 111 | 97620 | 98147 | 1991758 | 1991231 | 747 | f-3 | 97629 | 98127 | 287 | HycB | C | Fe-S-cluster-containing hydrogenase components 2 |
| 112 | 98417 | 99583 | 1990961 | 1989795 | 372 | f-2 | 98474 | 99581 | 464 | DadA | E | Glycine/D-amino acid oxidases (deaminating) |
| 113 | 99648 | 100892 | 1989730 | 1988486 | 748 | f-3 | 99654 | 100881 | 398 | | | |
| 114 | 100915 | 101205 | 1988463 | 1988173 | 1457 | r-1 | 100975 | 101098 | 30 | - | S | Uncharacterized ACR |
| 115 | 101224 | 101733 | 1988154 | 1987645 | 1456 | r-1 | 101239 | 101695 | 212 | WecD | K R | Histone acetyltransferase HPA2 and related acetyltransferases COG0454 WecD |
| 116 | 101796 | 102347 | 1987582 | 1987031 | 749 | f-3 | 101805 | 102315 | 206 | - | K | Predicted transcription factor |
| 117 | 102393 | 102563 | 1986985 | 1986815 | 750 | f-3 | | | | | | |
| 118 | 102986 | 103432 | 1986392 | 1985946 | 2148 | r-3 | 103016 | 103364 | 182 | - | S | Uncharacterized ArCR |
| 119 | 103476 | 104318 | 1985902 | 1985060 | 751 | f-3 | 103539 | 104313 | 429 | SppA | N O | Periplasmic serine proteases (ClpP class) COG0616 SppA |
| 120 | 104398 | 106101 | 1984980 | 1983277 | 30 | f-1 | 104398 | 106099 | 723 | - | S | Uncharacterized ACR |
| 121 | 106210 | 106779 | 1983168 | 1982599 | 31 | f-1 | 106210 | 106759 | 316 | SPT15 | K | Transcription initiation factor TFIID (TATA-binding protein) |
| 122 | 106834 | 107454 | 1982544 | 1981924 | 32 | f-1 | 106894 | 107104 | 30 | RAD5 5 | T | RecA-superfamily ATPases implicated in signal transduction |

| 123 | 107637 | 108455 | 1981741 | 1980923 | 752 | f-3 | 107640 | 108435 | 354 | AcuC | TQ | Deacetylases |
|---|---|---|---|---|---|---|---|---|---|---|---|---|
| 124 | 108482 | 109099 | 1980896 | 1980279 | 2147 | r-3 | 108491 | 109097 | 374 | PorG | C | Pyruvate:ferredoxin oxidoreductase and related 2-oxoacid:ferredoxin oxidoreductases (Indole-pyruvate ferredoxin oxidoreductase) |
| 125 | 109092 | 111035 | 1980286 | 1978343 | 1827 | r-2 | 109092 | 110067 | 452 | PorA | C | Pyruvate:ferredoxin oxidoreductase and related 2-oxoacid:ferredoxin oxidoreductases (Indole-pyruvate ferredoxin oxidoreductase) |
| 126 | 111643 | 113019 | 1977735 | 1976359 | 1455 | r-1 | 111652 | 113017 | 732 | - | C | Acyl-CoA synthetase (NDP forming) |
| 127 | 113205 | 114563 | 1976173 | 1974815 | 753 | f-3 | 113205 | 114555 | 724 | - | R | Predicted ATPase of the AAA superfamily |
| 128 | 114668 | 115351 | 1974710 | 1974027 | 373 | f-2 | 114677 | 115346 | 390 | - | R | Predicted Zn-dependent hydrolases of the beta-lactamase fold |
| 129 | 115397 | 116401 | 1973981 | 1972977 | 374 | f-2 | 115490 | 116378 | 284 | LytB | M | Putative cell wall-binding domain |
| 130 | 116482 | 116634 | 1972896 | 1972744 | 1454 | r-1 | 116524 | 116596 | 27 | - | R | Predicted nucleic-acid-binding protein containing a Zn-ribbon |

EP 1 923 464 A1

106

| 131 | 116676 | 117494 | 1972702 | 1971884 | 1826 | r-2 | 116700 | 117054 | 34 | RecN | L | ATPases involved in DNA repair |
|-----|--------|--------|---------|---------|------|-----|--------|--------|------|------|----|------------------------------|
| 132 | 117475 | 118242 | 1971903 | 1971136 | 1453 | r-1 | 117556 | 117835 | 34 | - | S | Predicted membrane protein |
| 133 | 118178 | 118711 | 1971200 | 1970667 | 2146 | r-3 | 118235 | 118379 | 30 | PitA | P | Phosphate/sulphate permeases |
| 134 | 119061 | 119939 | 1970317 | 1969439 | 1825 | r-2 | 119100 | 119931 | 416 | SpeE | E | Spermidine synthase |
| 135 | 119973 | 120485 | 1969405 | 1968893 | 754 | f-3 | 120156 | 120420 | 35 | - | R | Hydrolases of the alpha/beta superfamily |
| 136 | 120479 | 120952 | 1968899 | 1968426 | 2145 | r-3 | 120479 | 120947 | 269 | - | S | Uncharacterized ACR |
| 137 | 121121 | 121192 | 1968257 | 1968186 | 2144 | r-3 | | | | | | |
| 138 | 121404 | 121856 | 1967974 | 1967522 | 755 | f-3 | 121443 | 121854 | 245 | GcvH | E | Glycine cleavage system H protein (lipoate-binding) |
| 139 | 122007 | 122438 | 1967371 | 1966940 | 756 | f-3 | 122007 | 122256 | 90 | PspC | KT | Putative stress-responsive transcriptional regulator COG1983 PspC |
| 140 | 122431 | 122667 | 1966947 | 1966711 | 33 | f-1 | | | | | | |
| 141 | 122668 | 123594 | 1966710 | 1965784 | 34 | f-1 | 122680 | 123508 | 313 | CitG | H | Triphosphoribosyl-dephospho-Co A synthetase |
| 142 | 123578 | 123868 | 1965800 | 1965510 | 2143 | r-3 | 123599 | 123710 | 29 | - | L | Archaea-specific RecJ-like exonuclease |
| 143 | 123932 | 126157 | 1965446 | 1963221 | 2142 | r-3 | 123932 | 126146 | 1300 | - | L | Archaea-specific RecJ-like exonuclease |
| 144 | 126306 | 128561 | 1963072 | 1960817 | 757 | f-3 | 126333 | 128553 | 448 | Tar | N | Methyl-accepting chemotaxis |

EP 1 923 464 A1

| | | | | | | | | | | | protein |
|---|---|---|---|---|---|---|---|---|---|---|---|
| 145 | 128631 | 130013 | 1960747 | 1959365 | 1824 | r-2 | 128640 | 130011 | 628 | - | R | Permeases |
| 146 | 130150 | 131154 | 1959228 | 1958224 | 1452 | r-1 | 130150 | 131110 | 392 | MalK | G | ABC-type sugar/spermidine/putrescine/iron/thiamine transport systems |
| 147 | 131148 | 133049 | 1958230 | 1956329 | 1823 | r-2 | 131409 | 133029 | 584 | ThiP | H | ABC-type thiamine transport system |
| 148 | 132745 | 133890 | 1956633 | 1955488 | 35 | f-1 | 132856 | 133831 | 394 | DmpA | EQ | L-aminopeptidase/D-esterase COG3191 DmpA |
| 149 | 133885 | 134547 | 1955493 | 1954831 | 1451 | r-1 | 133900 | 134527 | 182 | CcmA | Q | ABC-type multidrug transport system |
| 150 | 134544 | 134834 | 1954834 | 1954544 | 1822 | r-2 | 134589 | 134763 | 30 | FhaB | M | Putative hemagglutinin/hemolysin |
| 151 | 134978 | 135754 | 1954400 | 1953624 | 2141 | r-3 | 135020 | 135215 | 33 | - | R | Permeases of the major facilitator superfamily |
| 152 | 137477 | 138172 | 1951901 | 1951206 | 2140 | r-3 | 137828 | 138005 | 32 | - | S | Uncharacterized BCR |
| 153 | 138521 | 138676 | 1950857 | 1950702 | 2139 | r-3 | 138590 | 138671 | 28 | Map | J | Methionine aminopeptidase |
| 154 | 139365 | 140972 | 1950013 | 1948406 | 758 | f-3 | 139365 | 140970 | 914 | - | C | Fe-S oxidoreductases family 2 |
| 155 | 141078 | 141311 | 1948300 | 1948067 | 759 | f-3 | 141087 | 141294 | 46 | Lrp | K | Transcriptional regulators |
| 156 | 141335 | 141856 | 1948043 | 1947522 | 375 | f-2 | 141335 | 141797 | 147 | - | K | Predicted transcriptional regulators |

EP 1 923 464 A1

| 157 | 141853 | 142707 | 1947525 | 1946671 | 1450 | r-1 | 141862 | 142702 | 474 | Nfo | L | Endonuclease IV |
|---|---|---|---|---|---|---|---|---|---|---|---|---|
| 158 | 142732 | 143793 | 1946646 | 1945585 | 1449 | r-1 | 142903 | 143602 | 40 | SbcC | L | ATPase involved in DNA repair |
| 159 | 143756 | 144931 | 1945622 | 1944447 | 2138 | r-3 | 143765 | 144896 | 451 | - | S | Predicted membrane protein |
| 160 | 144924 | 145235 | 1944454 | 1944143 | 1821 | r-2 | 144936 | 145224 | 134 | - | S | Uncharacterized ACR |
| 161 | 145334 | 145951 | 1944044 | 1943427 | 376 | f-2 | 145334 | 145949 | 383 | - | S | Uncharacterized ACR |
| 162 | 146007 | 146603 | 1943371 | 1942775 | 1820 | r-2 | 146016 | 146553 | 261 | - | S | Uncharacterized ACR |
| 163 | 147207 | 149273 | 1942171 | 1940105 | 1819 | r-2 | 147309 | 149253 | 934 | - | L | Superfamily I DNA and RNA helicases and helicase subunits |
| 164 | 149293 | 149697 | 1940085 | 1939681 | 1448 | r-1 | 149293 | 149695 | 230 | - | R | Predicted nucleic-acid-binding protein containing a Zn-ribbon |
| 165 | 149699 | 150874 | 1939679 | 1938504 | 2137 | r-3 | 149708 | 150872 | 612 | PaaJ | I | Acetyl-CoA acetyltransferases |
| 166 | 150876 | 151928 | 1938502 | 1937450 | 1818 | r-2 | 150876 | 151926 | 582 | PksG | I | 3-hydroxy-3-methylglutaryl CoA synthase |
| 167 | 152076 | 152471 | 1937302 | 1936907 | 760 | f-3 | 152076 | 152433 | 157 | - | S | Uncharacterized ACR |
| 168 | 152417 | 152743 | 1936961 | 1936635 | 377 | f-2 | 152417 | 152738 | 164 | - | S | Uncharacterized ACR |
| 169 | 152801 | 153490 | 1936577 | 1935888 | 2136 | r-3 | 152810 | 153485 | 416 | NOP1 | J | Fibrillarin-like rRNA methylase |
| 170 | 153487 | 154752 | 1935891 | 1934626 | 1447 | r-1 | 153487 | 154609 | 553 | SIK1 | J | Protein implicated in ribosomal biogenesis |
| 171 | 154844 | 155881 | 1934534 | 1933497 | 2135 | r-3 | 154919 | 155879 | 578 | GCD2 | J | Translation initiation factor eIF-2B delta subunit |

| 172 | 156044 | 157309 | 1933334 | 1932069 | 378 | f-2 | 156056 | 157292 | 602 | ARO8 | KE | Transcriptional regulators containing a DNA-binding HTH domain and an aminotransferase domain (MocR family) and their eukaryotic orthologs COG1167 ARO8 |
|---|---|---|---|---|---|---|---|---|---|---|---|---|
| 173 | 157368 | 158228 | 1932010 | 1931150 | 761 | f-3 | 157452 | 157953 | 129 | - | R | Predicted glutamine amidotransferases |
| 174 | 158158 | 159018 | 1931220 | 1930360 | 1446 | r-1 | 158179 | 159016 | 422 | SplB | L | DNA repair photolyase |
| 175 | 158982 | 159464 | 1930396 | 1929914 | 762 | f-3 | 159054 | 159462 | 216 | - | S | Uncharacterized ACR |
| 176 | 159517 | 160083 | 1929861 | 1929295 | 1445 | r-1 | 159517 | 160081 | 350 | GuaA | F | GMP synthase - Glutamine amidotransferase domain |
| 177 | 160206 | 160256 | 1929172 | 1929122 | 763 | f-3 | | | | | | |
| 178 | 160526 | 160744 | 1928852 | 1928634 | 2134 | r-3 | 160619 | 160733 | 27 | - | C | Acyl-CoA synthetase (NDP forming) |
| 179 | 160787 | 161719 | 1928591 | 1927659 | 2133 | r-3 | 160799 | 161717 | 567 | GuaA | F | GMP synthase - PP-ATPase domain |
| 180 | 161795 | 163255 | 1927583 | 1926123 | 2132 | r-3 | 162410 | 163253 | 495 | GuaB | F | IMP dehydrogenase/GMP reductase |
| 181 | 163362 | 164405 | 1926016 | 1924973 | 764 | f-3 | 163503 | 163761 | 32 | | | |

EP 1 923 464 A1

110

| 182 | 164398 | 165393 | 1924980 | 1923985 | 1444 | r-1 | 164398 | 165388 | 544 | - | R | ATP-utilizing enzymes of ATP-grasp superfamily (probably carboligases) |
|---|---|---|---|---|---|---|---|---|---|---|---|---|
| 183 | 165390 | 167531 | 1923988 | 1921847 | 1817 | r-2 | 165390 | 167505 | 1051 | PurL | F | Phosphoribosylformylglycinamidi ne (FGAM) synthase |
| 184 | 168881 | 170377 | 1920497 | 1919001 | 2131 | r-3 | 169019 | 169826 | 162 | PpsA | G | Phosphoenolpyruvate synthase/pyruvate phosphate dikinase |
| 185 | 170457 | 171128 | 1918921 | 1918250 | 1816 | r-2 | 170457 | 171126 | 385 | PurL | F | Phosphoribosylformylglycinamidi ne (FGAM) synthase |
| 186 | 171130 | 171381 | 1918248 | 1917997 | 1443 | r-1 | 171139 | 171376 | 110 | PurS | F | Phosphoribosylformylglycinamidi ne (FGAM) synthase |
| 187 | 171383 | 172534 | 1917995 | 1916844 | 2130 | r-3 | 171392 | 172532 | 673 | - | R | ATP-utilizing enzymes of ATP-grasp superfamily (probably carboligases) |
| 188 | 172527 | 173834 | 1916851 | 1915544 | 1815 | r-2 | 172539 | 173829 | 602 | PurD | F | Phosphoribosylamine-glycine ligase |
| 189 | 173896 | 173985 | 1915482 | 1915393 | 1442 | r-1 | | | | | | |
| 190 | 174404 | 174601 | 1914974 | 1914777 | 379 | f-2 | 174434 | 174599 | 29 | PolB | L | DNA polymerase elongation subunit (family B) |
| 191 | 174585 | 175349 | 1914793 | 1914029 | 765 | f-3 | 174597 | 174876 | 34 | RAD5 5 | T | RecA-superfamily ATPases implicated in signal transduction |

EP 1 923 464 A1

EP 1 923 464 A1

| | | | | | | | | | | | | |
|---|---|---|---|---|---|---|---|---|---|---|---|---|
| 192 | 175740 | 177038 | 1913638 | 1912340 | 1814 | r-2 | 175749 | 177036 | 781 | PurT | F | Formate-dependent phosphoribosylglycinamide formyltransferase (GAR transformylase) |
| 193 | 177138 | 178151 | 1912240 | 1911227 | 766 | f-3 | 177147 | 178146 | 545 | PurM | F | Phosphoribosylaminoimidazol (AIR) synthetase |
| 194 | 178184 | 178348 | 1911194 | 1911030 | 380 | f-2 | 178217 | 178331 | 28 | PyrF | F | Orotidine-5'-phosphate decarboxylase |
| 195 | 178320 | 179039 | 1911058 | 1910339 | 1813 | r-2 | 178332 | 179028 | 341 | PurC | F | Phosphoribosylaminoimidazolesuccinocarboxamide (SAICAR) synthase |
| 196 | 179195 | 180553 | 1910183 | 1908825 | 381 | f-2 | 179195 | 180551 | 661 | PurF | F | Glutamine phosphoribosylpyrophosphate amidotransferase |
| 197 | 180543 | 181031 | 1908835 | 1908347 | 1812 | r-2 | 180543 | 181002 | 102 | - | R | Predicted nucleic acid-binding protein |
| 198 | 181028 | 181288 | 1908350 | 1908090 | 2129 | r-3 | 181028 | 181277 | 73 | - | S | Uncharacterized ACR |
| 199 | 181345 | 183324 | 1908033 | 1906054 | 1441 | r-1 | 181345 | 183322 | 984 | BisC | C | Anaerobic dehydrogenases |
| 200 | 183436 | 184935 | 1905942 | 1904443 | 1440 | r-1 | 184129 | 184273 | 33 | MalG | G | Sugar permeases |
| 201 | 185362 | 185955 | 1904016 | 1903423 | 1439 | r-1 | 185365 | 185953 | 330 | PDX2 | H | Predicted glutamine amidotransferase involved in pyridoxine biosynthesis |

| No. | | | | | | | | | | Gene | | Description |
|---|---|---|---|---|---|---|---|---|---|---|---|---|
| 202 | 185988 | 187004 | 1903390 | 1902374 | 1811 | r-2 | 185997 | 186966 | 536 | SNZ1 | H | Pyridoxine biosynthesis enzyme |
| 203 | 187111 | 187953 | 1902267 | 1901425 | 1438 | r-1 | 187120 | 187939 | 410 | NadC | H | Nicotinate-nucleotide pyrophosphorylase |
| 204 | 188074 | 189315 | 1901304 | 1900063 | 36 | f-1 | 188083 | 189256 | 188 | GCD1 | MJ | Nucleoside-diphosphate-sugar pyrophosphorylases involved in lipopolysaccharide biosynthesis/translation initiation factor eIF2B subunits COG1208 GCD1 |
| 205 | 189865 | 190278 | 1899513 | 1899100 | 37 | f-1 | 189865 | 190276 | 167 | - | S | Uncharacterized ACR |
| 206 | 190253 | 190621 | 1899125 | 1898757 | 382 | f-2 | 190253 | 190583 | 154 | - | R | Predicted nucleotidyltransferases |
| 207 | 190630 | 191799 | 1898748 | 1897579 | 1437 | r-1 | 190630 | 191785 | 715 | | | |
| 208 | 191874 | 192509 | 1897504 | 1896869 | 767 | f-3 | 191889 | 192489 | 256 | SmtA | Q | SAM-dependent methyltransferases COG0500 SmtA |
| 209 | 192535 | 192981 | 1896843 | 1896397 | 38 | f-1 | 192553 | 192763 | 29 | PilO | N | Fimbrial assembly protein |
| 210 | 192971 | 193486 | 1896407 | 1895892 | 383 | f-2 | 193004 | 193349 | 42 | SbcC | L | ATPase involved in DNA repair |
| 211 | 193701 | 194033 | 1895677 | 1895345 | 1810 | r-2 | 193740 | 194025 | 117 | WecD | K R | Histone acetyltransferase HPA2 and related acetyltransferases COG0454 WecD |
| 212 | 194152 | 194358 | 1895226 | 1895020 | 1436 | r-1 | 194242 | 194350 | 28 | RimL | J | Acetyltransferases |

| 213 | 195097 | 195405 | 1894281 | 1893973 | 39 | f-1 | 195097 | 195313 | 46 | CcmA | Q | ABC-type multidrug transport system |
|---|---|---|---|---|---|---|---|---|---|---|---|---|
| 214 | 195742 | 195846 | 1893636 | 1893532 | 1435 | r-1 | | | | | | |
| 215 | 195995 | 196111 | 1893383 | 1893267 | 384 | f-2 | | | | | | |
| 216 | 196138 | 196959 | 1893240 | 1892419 | 1434 | r-1 | 196138 | 196951 | 291 | WecD | K R | Histone acetyltransferase HPA2 and related acetyltransferases COG0454 WecD |
| 217 | 197032 | 197625 | 1892346 | 1891753 | 1433 | r-1 | 197044 | 197563 | 125 | RimL | J | Acetyltransferases |
| 218 | 197747 | 198367 | 1891631 | 1891011 | 385 | f-2 | 197837 | 198185 | 65 | SmtA | Q R | SAM-dependent methyltransferases COG0500 SmtA |
| 219 | 198495 | 199754 | 1890883 | 1889624 | 1809 | r-2 | 198549 | 198996 | 75 | | | |
| 220 | 199748 | 200686 | 1889630 | 1888692 | 2128 | r-3 | 199901 | 200363 | 33 | RfaG | M | Predicted glycosyltransferases |
| 221 | 200742 | 201098 | 1888636 | 1888280 | 768 | f-3 | 200931 | 201003 | 27 | BtuC | PH | ABC-type cobalamin/Fe3+-siderophores transport systems |
| 222 | 201067 | 201738 | 1888311 | 1887640 | 40 | f-1 | 201067 | 201727 | 360 | - | R | Predicted amidohydrolase |
| 223 | 201692 | 202102 | 1887686 | 1887276 | 386 | f-2 | 201773 | 202100 | 181 | ARC1 | R | EMAP domain |
| 224 | 202103 | 202924 | 1887275 | 1886454 | 387 | f-2 | 202313 | 202922 | 229 | SpeB | E | Arginase/agmatinase/formimiono glutamate hydrolase |
| 225 | 202929 | 203372 | 1886449 | 1886006 | 769 | f-3 | 202944 | 203361 | 187 | CDC1 | T | Predicted protein-tyrosine |

| | | | | | | | | | | 4 | | phosphatase |
|---|---|---|---|---|---|---|---|---|---|---|---|---|
| 226 | 203585 | 204475 | 1885793 | 1884903 | 388 | f-2 | 203633 | 204170 | 82 | HisS | J | Histidyl-tRNA synthetase |
| 227 | 204472 | 205083 | 1884906 | 1884295 | 41 | f-1 | 204484 | 205048 | 155 | HisG | E | ATP phosphoribosyltransferase (histidine biosynthesis) |
| 228 | 205070 | 206200 | 1884308 | 1883178 | 389 | f-2 | 205079 | 206111 | 276 | HisD | E | Histidinol dehydrogenase |
| 229 | 206280 | 206813 | 1883098 | 1882565 | 770 | f-3 | 206280 | 206766 | 117 | HisB | E | Imidazoleglycerol-phosphate dehydratase |
| 230 | 206810 | 207397 | 1882568 | 1881981 | 390 | f-2 | 206810 | 207380 | 182 | HisH | E | Glutamine amidotransferase |
| 231 | 207399 | 208100 | 1881979 | 1881278 | 771 | f-3 | 207405 | 208038 | 162 | HisA | E | Phosphoribosylformimino-5-amin oimidazole carboxamide ribonucleotide (ProFAR) isomerase |
| 232 | 208082 | 208840 | 1881296 | 1880538 | 391 | f-2 | 208082 | 208826 | 310 | HisF | E | Imidazoleglycerol-phosphate synthase |
| 233 | 208850 | 209479 | 1880528 | 1879899 | 392 | f-2 | 208898 | 209171 | 119 | HisI | E | Phosphoribosyl-AMP cyclohydrolase |
| 234 | 209476 | 210486 | 1879902 | 1878892 | 42 | f-1 | 209542 | 210427 | 184 | HisC | E | Histidinol-phosphate aminotransferase/Tyrosine aminotransferase |
| 235 | 210470 | 211198 | 1878908 | 1878180 | 393 | f-2 | 210476 | 210995 | 37 | Gph | R | Predicted phosphatases |

| 236 | Indole-3-glycerol phosphate synthase | E | TrpC | 355 | 211980 | 211296 | f-3 | 772 | 1877396 | 1878082 | 211982 | 211296 |
|---|---|---|---|---|---|---|---|---|---|---|---|---|
| 237 | Anthranilate phosphoribosyltransferase | E | TrpD | 415 | 212951 | 211985 | f-2 | 394 | 1876422 | 1877399 | 212956 | 211979 |
| 238 | Anthranilate/para-aminobenzoate synthases component I COG0147 TrpE | EH | TrpE | 610 | 214228 | 212980 | f-1 | 43 | 1875139 | 1876440 | 214239 | 212938 |
| 239 | Anthranilate/para-aminobenzoate synthases component II COG0512 PabA | EH | PabA | 326 | 214806 | 214236 | f-3 | 773 | 1874564 | 1875142 | 214814 | 214236 |
| 240 | Phosphoribosyl anthranilate isomerase | E | TrpF | 253 | 215428 | 214816 | f-1 | 44 | 1873945 | 1874571 | 215433 | 214807 |
| 241 | Tryptophan synthase beta chain | E | TrpB | 676 | 216587 | 215435 | f-2 | 395 | 1872783 | 1873952 | 216595 | 215426 |
| 242 | Tryptophan synthase alpha chain | E | TrpA | 370 | 217323 | 216588 | f-3 | 774 | 1872035 | 1872790 | 217343 | 216588 |
| 243 | Prephenate dehydrogenase | E' | TyrA | 85 | 217913 | 217328 | r-3 | 2127 | 1871283 | 1872053 | 218095 | 217325 |
| 244 | PLP-dependent aminotransferases | E | AvtA | 191 | 218971 | 218029 | r-1 | 1432 | 1870264 | 1871358 | 219114 | 218020 |
| 245 | Chorismate mutase | E | PheA | 35 | 219221 | 219080 | r-3 | 2126 | 1870125 | 1870301 | 219253 | 219077 |
| 246 | Chorismate synthase | E | AroC | 530 | 220457 | 219407 | r-3 | 2125 | 1868904 | 1869971 | 220474 | 219407 |
| 247 | 5-enolpyruvylshikimate-3-phosphate synthase | E | AroA | 470 | 221710 | 220513 | r-1 | 1431 | 1867660 | 1868907 | 221718 | 220471 |
| 248 | Archaeal shikimate kinase | EH | - | 175 | 222234 | 221742 | r-2 | 1808 | 1867142 | 1867702 | 222236 | 221676 |

| | | | | | | | | | | | COG1685 - |
|---|---|---|---|---|---|---|---|---|---|---|---|
| 249 | 222472 | 222852 | 1866906 | 1866526 | 1430 | r-1 | 222472 | 222850 | 161 | AroE | E | Shikimate 5-dehydrogenase |
| 250 | 222879 | 223259 | 1866499 | 1866119 | 1807 | r-2 | 222879 | 223197 | 142 | AroE | E | Shikimate 5-dehydrogenase |
| 251 | 223282 | 223923 | 1866096 | 1865455 | 1429 | r-1 | 223282 | 223894 | 207 | AroD | E | 3-dehydroquinate dehydratase |
| 252 | 223877 | 225022 | 1865501 | 1864356 | 2124 | r-3 | 223985 | 224876 | 350 | AroB | E | 3-dehydroquinate synthetase |
| 253 | 224890 | 225804 | 1864488 | 1863574 | 1428 | r-1 | 224965 | 225682 | 395 | AroA | E | 3-Deoxy-D-arabino-heptulosonate 7-phosphate (DAHP) synthase |
| 254 | 225801 | 226844 | 1863577 | 1862534 | 1806 | r-2 | 225924 | 226824 | 426 | TktA | G | Transketolase |
| 255 | 226718 | 227377 | 1862660 | 1862001 | 2123 | r-3 | 226742 | 227369 | 278 | TktA | G | Transketolase |
| 256 | 227370 | 227741 | 1862008 | 1861637 | 1805 | r-2 | 227463 | 227547 | 30 | AraC | K | AraC-type DNA-binding domain-containing proteins |
| 257 | 227931 | 228242 | 1861447 | 1861136 | 775 | f-3 | 227985 | 228237 | 71 | ProC | E | Pyrroline-5-carboxylate reductase |
| 258 | 228257 | 228718 | 1861121 | 1860660 | 396 | f-2 | 228257 | 228701 | 136 | ProC | E | Pyrroline-5-carboxylate reductase |
| 259 | 228710 | 229147 | 1860668 | 1860231 | 2122 | r-3 | 228710 | 229079 | 201 | ArgE | E | Acetylornithine deacetylase/Succinyl-diaminopim elate desuccinylase and related deacylases |

EP 1 923 464 A1

| | | | | | | | | | | | |
|---|---|---|---|---|---|---|---|---|---|---|---|
| 260 | 229347 | 229745 | 1860031 | 1859633 | 1804 | r-2 | 229347 | 229716 | 195 | ArgE | E | Acetylornithine deacetylase/Succinyl-diaminopim elate desuccinylase and related deacylases |
| 261 | 229732 | 230820 | 1859646 | 1858558 | 1427 | r-1 | 229732 | 230809 | 523 | ArgD | E | PLP-dependent aminotransferases |
| 262 | 230826 | 231581 | 1858552 | 1857797 | 1803 | r-2 | 230826 | 231579 | 315 | ArgB | E | Acetylglutamate kinase |
| 263 | 231591 | 232583 | 1857787 | 1856795 | 1802 | r-2 | 231591 | 232578 | 564 | ArgC | E | Acetylglutamate semialdehyde dehydrogenase |
| 264 | 232580 | 233410 | 1856798 | 1855968 | 2121 | r-3 | 232589 | 233405 | 437 | RimK | HJ | Glutathione synthase/Ribosomal protein S6 modification enzyme (glutaminyl transferase) COG0189 RimK |
| 265 | 233428 | 233589 | 1855950 | 1855789 | 1426 | r-1 | 233431 | 233512 | 28 | PqiA | S | Uncharacterized paraquat-inducible protein A |
| 266 | 233684 | 234727 | 1855694 | 1854651 | 2120 | r-3 | 233684 | 234692 | 456 | LeuB | E | Isocitrate/isopropylmalate dehydrogenase |
| 267 | 234715 | 235206 | 1854663 | 1854172 | 1425 | r-1 | 234715 | 235201 | 256 | LeuD | E | 3-isopropylmalate dehydratase small subunit |
| 268 | 235203 | 236345 | 1854175 | 1853033 | 1801 | r-2 | 235203 | 236337 | 595 | LeuC | E | 3-isopropylmalate dehydratase large subunit |
| 269 | 236342 | 237427 | 1853036 | 1851951 | 2119 | r-3 | 236342 | 237425 | 536 | LeuA | E | Isopropylmalate/homocitrate/citra malate synthases |

| 270 | 237653 | 238216 | 1851725 | 1851162 | 2118 | r-3 | 237653 | 238214 | 297 | NfnB | C | Nitroreductase |
|---|---|---|---|---|---|---|---|---|---|---|---|---|
| 271 | 238509 | 239528 | 1850869 | 1849850 | 776 | f-3 | 238581 | 239505 | 289 | - | R | Predicted ATPase of the AAA superfamily |
| 272 | 239489 | 239686 | 1849889 | 1849692 | 397 | f-2 | 239495 | 239672 | 76 | - | R | Predicted ATPase of the AAA superfamily |
| 273 | 239677 | 240426 | 1849701 | 1848952 | 1424 | r-1 | 239677 | 240424 | 406 | PhnP | R | Metal-dependent hydrolases of the beta-lactamase superfamily I |
| 274 | 240560 | 243028 | 1848818 | 1846350 | 398 | f-2 | 240662 | 242990 | 424 | PflD | C | Pyruvate-formate lyase |
| 275 | 243977 | 244525 | 1845401 | 1844853 | 399 | f-2 | 244118 | 244322 | 35 | Arp | R | Ankyrin repeat proteins |
| 276 | 244591 | 245055 | 1844787 | 1844323 | 45 | f-1 | 244591 | 245044 | 228 | - | S | Uncharacterized ACR |
| 277 | 245052 | 245747 | 1844326 | 1843631 | 777 | f-3 | 245052 | 245736 | 322 | - | S | Uncharacterized ArCR |
| 278 | 245738 | 246229 | 1843640 | 1843149 | 2117 | r-3 | 245744 | 245888 | 33 | | | |
| 279 | 246239 | 246340 | 1843139 | 1843038 | 2116 | r-3 | 246239 | 246326 | 26 | TehA | P | Tellurite resistance protein and related permeases |
| 280 | 247226 | 248134 | 1842152 | 1841244 | 2115 | r-3 | 247241 | 248132 | 503 | NadA | H | Quinolinate synthase |
| 281 | 248197 | 249606 | 1841181 | 1839772 | 1423 | r-1 | 248275 | 249586 | 598 | NadB | H | Aspartate oxidase |
| 282 | 251161 | 251265 | 1838217 | 1838113 | 46 | f-1 | | | | | | |
| 283 | 251394 | 251477 | 1837984 | 1837901 | 778 | f-3 | | | | | | |
| 284 | 251557 | 251760 | 1837821 | 1837618 | 47 | f-1 | 251602 | 251731 | 32 | GpmA | G | Phosphoglycerate mutase 1 |
| 285 | 254653 | 255162 | 1834725 | 1834216 | 1422 | r-1 | 254653 | 255151 | 248 | KptA | S | Uncharacterized ACR |
| 286 | 255227 | 256987 | 1834151 | 1832391 | 2114 | r-3 | 256304 | 256919 | 57 | ElsH | R | Metal-dependent hydrolase |

| No. | | | | | | | | | | Gene | COG | Function |
|---|---|---|---|---|---|---|---|---|---|---|---|---|
| 287 | 257124 | 258452 | 1832254 | 1830926 | 1800 | r-2 | 258450 | 257133 | 728 | HcaD | R | Uncharacterized NAD(FAD)-dependent dehydrogenases |
| 288 | 258556 | 259233 | 1830822 | 1830145 | 1421 | r-1 | 259231 | 258556 | 310 | PyrH | F | Uridylate kinase |
| 289 | 260703 | 261923 | 1828675 | 1827455 | 779 | f-3 | 261798 | 260703 | 430 | SrmB | LK J | Superfamily II DNA and RNA helicases COG0513 SrmB |
| 290 | 262176 | 262484 | 1827202 | 1826894 | 1799 | r-2 | 262482 | 262176 | 183 | RpsJ | J | Ribosomal protein S10 |
| 291 | 262544 | 263830 | 1826834 | 1825548 | 2113 | r-3 | 263828 | 262544 | 762 | TufB | JE | GTPases - translation elongation factors COG0050 TufB |
| 292 | 264065 | 265165 | 1825313 | 1824213 | 2112 | r-3 | 265157 | 264065 | 634 | FusA | J | Translation elongation and release factors (GTPases) |
| 293 | 264895 | 266262 | 1824483 | 1823116 | 1420 | r-1 | 265954 | 264895 | 642 | FusA | J | Translation elongation and release factors (GTPases) |
| 294 | 266696 | 266977 | 1822682 | 1822401 | 2111 | r-3 | | | | | | |
| 295 | 267002 | 268075 | 1822376 | 1821303 | 2110 | r-3 | 267965 | 267005 | 260 | - | R | HD superfamily phosphohydrolases |
| 296 | 268109 | 269197 | 1821269 | 1820181 | 2109 | r-3 | 269156 | 268109 | 619 | ArgE | E | Acetylornithine deacetylase/Succinyl-diaminopimelate desuccinylase and related deacylases |
| 297 | 269297 | 270064 | 1820081 | 1819314 | 400 | f-2 | 270059 | 269378 | 270 | GloB | R | Zn-dependent hydrolases |
| 298 | 270052 | 270306 | 1819326 | 1819072 | 48 | f-1 | 270304 | 270061 | 147 | - | S | Uncharacterized ArCR |

| 299 | 270301 | 271278 | 1819077 | 1818100 | 1419 | r-1 | 270331 | 270853 | 117 | - | S | Uncharacterized ACR |
|---|---|---|---|---|---|---|---|---|---|---|---|---|
| 300 | 271361 | 272119 | 1818017 | 1817259 | 401 | f-2 | 271361 | 272117 | 317 | TatD | L | Mg-dependent DNase |
| 301 | 272121 | 272429 | 1817257 | 1816949 | 780 | f-3 | 272208 | 272421 | 58 | SmtA | Q R | SAM-dependent methyltransferases COG0500 SmtA |
| 302 | 272525 | 274057 | 1816853 | 1815321 | 2108 | r-3 | 272534 | 274055 | 679 | FolP | H | Dihydropteroate synthase |
| 303 | 274244 | 274963 | 1815134 | 1814415 | 402 | f-2 | 274244 | 274955 | 417 | - | S | Uncharacterized ACR |
| 304 | 275340 | 275564 | 1814038 | 1813814 | 781 | f-3 | 275463 | 275538 | 27 | - | R | Predicted nucleic acid-binding protein |
| 305 | 276688 | 277758 | 1812690 | 1811620 | 49 | f-1 | 277030 | 277165 | 33 | - | C | Aldehyde:ferredoxin oxidoreductase |
| 306 | 277759 | 278526 | 1811619 | 1810852 | 50 | f-1 | 278314 | 278485 | 28 | ThiP | H | ABC-type thiamine transport system |
| 307 | 278454 | 278981 | 1810924 | 1810397 | 782 | f-3 | 278700 | 278793 | 29 | - | K | RNA-binding proteins (RRM domain) |
| 308 | 278969 | 279736 | 1810409 | 1809642 | 403 | f-2 | 279002 | 279638 | 156 | CcmA | Q | ABC-type multidrug transport system |
| 309 | 279859 | 280521 | 1809519 | 1808857 | 1418 | r-1 | 279883 | 280513 | 255 | HIS2 | ER | Histidinol phosphatase and related hydrolases of the PHP family COG1387 HIS2 |
| 310 | 280629 | 281072 | 1808749 | 1808306 | 783 | f-3 | 280638 | 281070 | 251 | Sbm | I | Methylmalonyl-CoA mutase |

| | | | | | | | | | | | | |
|---|---|---|---|---|---|---|---|---|---|---|---|---|
| 311 | Putative periplasmic protein kinase ArgK and related GTPases of G3E family | E | ArgK | 494 | 282061 | 281113 | 51 | f-1 | 1807306 | 1808274 | 282072 | 281104 |
| 312 | Lactoylglutathione lyase and related lyases | E | GloA | 233 | 282462 | 282069 | 784 | f-3 | 1806911 | 1807309 | 282467 | 282069 |
| 313 | Histone acetyltransferase HPA2 and related acetyltransferases COG0454 WecD | K R | WecD | 182 | 283186 | 282544 | 1417 | r-1 | 1806106 | 1806834 | 283272 | 282544 |
| 314 | Allophanate hydrolase subunit 2 | E | DUR1 | 414 | 284405 | 283421 | 2107 | r-3 | 1805957 | 1804962 | 284416 | 283421 |
| 315 | Allophanate hydrolase subunit 1 | E | DUR1 | 318 | 285085 | 284419 | 1416 | r-1 | 1804279 | 1804965 | 285099 | 284413 |
| 316 | Predicted nucleic acid-binding protein | R | VapC | 39 | 285257 | 285107 | 2106 | r-3 | 1804086 | 1804274 | 285292 | 285104 |
| 317 | Uncharacterized proteins | R | - | 455 | 286487 | 285725 | 2105 | r-3 | 1802886 | 1803662 | 286492 | 285716 |
| 318 | Predicted nucleic acid-binding protein | R | - | 214 | 287005 | 286570 | 52 | f-1 | 1802299 | 1802835 | 287079 | 286543 |
| 319 | Predicted nucleotide kinase (related to CMP and AMP kinases) | F | - | 244 | 287643 | 287112 | 1798 | r-2 | 1801733 | 1802332 | 287645 | 287046 |
| 320 | DNA-directed RNA polymerase sigma subunits (sigma70/sigma32) | K | RpoD | 28 | 287881 | 287788 | 1415 | r-1 | 1801225 | 1801620 | 288153 | 287758 |

| No. | | | | | | | | | | | | Description |
|---|---|---|---|---|---|---|---|---|---|---|---|---|
| 321 | 288150 | 288437 | 1801228 | 1800941 | 1797 | r-2 | 288159 | 288423 | 44 | - | S | Uncharacterized ACR |
| 322 | 288505 | 289047 | 1800873 | 1800331 | 1414 | r-1 | 288724 | 288904 | 42 | - | R | Predicted nucleotidyltransferases |
| 323 | 289173 | 289493 | 1800205 | 1799885 | 1796 | r-2 | | | | | | |
| 324 | 289490 | 289948 | 1799888 | 1799430 | 2104 | r-3 | 289502 | 289874 | 33 | - | R | Predicted nucleic acid-binding protein |
| 325 | 290136 | 291029 | 1799242 | 1798349 | 1795 | r-2 | 290193 | 291024 | 363 | AlkA | L | 3-Methyladenine DNA glycosylase |
| 326 | 290939 | 291157 | 1798439 | 1798221 | 2103 | r-3 | 290975 | 291065 | 30 | GlgB | G | 1 |
| 327 | 291353 | 292696 | 1798025 | 1796682 | 404 | f-2 | 291431 | 292670 | 516 | - | N O | Membrane-bound serine protease (ClpP class) COG1030 - 1 |
| 328 | 292703 | 293509 | 1796675 | 1795869 | 405 | f-2 | 292763 | 293507 | 374 | HflC | O | Membrane protease subunits |
| 329 | 293510 | 293593 | 1795868 | 1795785 | 2102 | r-3 | | | | | | |
| 330 | 293627 | 294415 | 1795751 | 1794963 | 406 | f-2 | 293636 | 294413 | 406 | - | D | ATPases involved in chromosome partitioning |
| 331 | 294346 | 294663 | 1795032 | 1794715 | 53 | f-1 | | | | | | |
| 332 | 294750 | 295001 | 1794628 | 1794377 | 785 | f-3 | 294801 | 294969 | 28 | SecA | N | Preprotein translocase subunit SecA (ATPase |
| 333 | 295115 | 296626 | 1794263 | 1792752 | 407 | f-2 | 295115 | 296624 | 782 | DeoA | F | Thymidine phosphorylase |
| 334 | 296627 | 297139 | 1792751 | 1792239 | 2101 | r-3 | 296882 | 297017 | 30 | UvrA | L | Excinuclease ATPase subunit |
| 335 | 297204 | 297731 | 1792174 | 1791647 | 1794 | r-2 | 297270 | 297720 | 278 | MoaC | H | Molybdenum cofactor biosynthesis enzyme |

| 336 | 297773 | 298702 | 1791605 | 1790676 | 408 | f-2 | 297785 | 298694 | 452 | CcmA | Q | ABC-type multidrug transport system |
|---|---|---|---|---|---|---|---|---|---|---|---|---|
| 337 | 298699 | 300825 | 1790679 | 1788553 | 54 | f-1 | 298768 | 300298 | 273 | - | S | Predicted membrane protein |
| 338 | 300795 | 301748 | 1788583 | 1787630 | 786 | f-3 | 300822 | 301671 | 226 | NosY | R | ABC-type transport system involved in multi-copper enzyme maturation |
| 339 | 301803 | 303251 | 1787575 | 1786127 | 1793 | r-2 | 302097 | 303249 | 645 | RtcB | S | Uncharacterized ACR |
| 340 | 303305 | 303766 | 1786073 | 1785612 | 2100 | r-3 | 303374 | 303752 | 140 | - | S | Uncharacterized ACR |
| 341 | 303750 | 304688 | 1785628 | 1784690 | 1792 | r-2 | 303750 | 304662 | 427 | Sun | J | tRNA and rRNA cytosine-C5-methylases |
| 342 | 304698 | 305126 | 1784680 | 1784252 | 1791 | r-2 | 304698 | 305124 | 183 | - | S | Uncharacterized ACR |
| 343 | 305339 | 306193 | 1784039 | 1783185 | 409 | f-2 | 305339 | 306185 | 437 | PanB | H | Ketopantoate hydroxymethyltransferase |
| 344 | 306190 | 306858 | 1783188 | 1782520 | 55 | f-1 | 306193 | 306853 | 272 | WcaA | M | Glycosyltransferases involved in cell wall biogenesis |
| 345 | 307473 | 307700 | 1781905 | 1781678 | 787 | f-3 | 307527 | 307656 | 26 | BaeS | T | Sensory transduction histidine kinases |
| 346 | 308311 | 308886 | 1781067 | 1780492 | 1413 | r-1 | 308311 | 308875 | 240 | ThiI | H | Thiamine biosynthesis ATP pyrophosphatase |
| 347 | 308930 | 309406 | 1780448 | 1779972 | 2099 | r-3 | 308930 | 309377 | 139 | - | S | Predicted membrane protein |
| 348 | 309492 | 310637 | 1779886 | 1778741 | 1790 | r-2 | 309498 | 310497 | 350 | ThiI | H | Thiamine biosynthesis ATP pyrophosphatase |

| | | | | | | | | | | | | |
|---|---|---|---|---|---|---|---|---|---|---|---|---|
| 349 | 310642 | 311016 | 1778736 | 1778362 | r-1 | 1412 | 310708 | 310894 | 31 | ThiP | H | ABC-type thiamine transport system |
| 350 | 311017 | 311625 | 1778361 | 1777753 | r-1 | 1411 | 311035 | 311569 | 62 | NfnB | C | Nitroreductase |
| 351 | 312108 | 312536 | 1777270 | 1776842 | r-2 | 1789 | 312399 | 312528 | 29 | PhoU | P | Phosphate uptake regulator |
| 352 | 312637 | 312903 | 1776741 | 1776475 | f-1 | 56 | | | | | | |
| 353 | 312953 | 313306 | 1776425 | 1776072 | f-2 | 410 | 313193 | 313301 | 32 | - | R | ATPases of the PilT family |
| 354 | 313344 | 314120 | 1776034 | 1775258 | f-3 | 788 | 313407 | 314118 | 356 | - | Q | Maleate cis-trans isomerase |
| 355 | 314205 | 314447 | 1775173 | 1774931 | f-3 | 789 | 314313 | 314436 | 30 | AraC | K | AraC-type DNA-binding domain-containing proteins |
| 356 | 314429 | 315589 | 1774949 | 1773789 | f-2 | 411 | 314453 | 314765 | 39 | GloB | R | Zn-dependent hydrolases |
| 357 | 315618 | 316058 | 1773760 | 1773320 | r-2 | 1788 | 315762 | 315858 | 32 | KatE | P | Catalase |
| 358 | 316245 | 316973 | 1773133 | 1772405 | r-2 | 1787 | 316245 | 316971 | 423 | SpoOJ | K | Predicted transcriptional regulators |
| 359 | 317124 | 318272 | 1772254 | 1771106 | f-3 | 790 | 317136 | 318267 | 480 | - | S | Uncharacterized ACR |
| 360 | 318265 | 319239 | 1771113 | 1770139 | r-1 | 1410 | 318388 | 319225 | 367 | - | S | Uncharacterized ACR |
| 361 | 319807 | 319851 | 1769571 | 1769527 | r-1 | 1409 | | | | | | |
| 362 | 320239 | 320928 | 1769139 | 1768450 | f-1 | 57 | 320308 | 320521 | 38 | XerC | L | Integrase |
| 363 | 321374 | 321511 | 1768004 | 1767867 | f-2 | 412 | | | | | | |
| 364 | 321508 | 321696 | 1767870 | 1767682 | f-1 | 58 | 321517 | 321649 | 28 | - | R | Predicted nucleic acid-binding protein |

| 365 | 322012 | 322365 | 1767366 | 1767013 | 59 | f-1 | 322060 | 322228 | 31 | CysZ | E | Uncharacterized protein involved in cysteine biosynthesis |
|---|---|---|---|---|---|---|---|---|---|---|---|---|
| 366 | 322265 | 324256 | 1767113 | 1765122 | 413 | f-2 | 322982 | 323261 | 36 | - | S | Predicted membrane protein |
| 367 | 324261 | 326399 | 1765117 | 1762979 | 791 | f-3 | 324882 | 325074 | 34 | Arp | R | Ankyrin repeat proteins |
| 368 | 326552 | 326935 | 1762826 | 1762443 | 414 | f-2 | 326639 | 326792 | 31 | AmtB | P | Ammonia permeases |
| 369 | 327013 | 327282 | 1762365 | 1762096 | 60 | f-1 | 327049 | 327217 | 28 | ZntA | P | Cation transport ATPases |
| 370 | 327284 | 327514 | 1762094 | 1761864 | 415 | f-2 | 327386 | 327488 | 27 | DraG | O | ADP-ribosylglycohydrolase |
| 371 | 327518 | 328321 | 1761860 | 1761057 | 416 | f-2 | 328157 | 328313 | 30 | BioD | H | Dethiobiotin synthetase |
| 372 | 328333 | 328815 | 1761045 | 1760563 | 61 | f-1 | 328333 | 328492 | 29 | - | S | Uncharacterized BCR |
| 373 | 328812 | 329288 | 1760566 | 1760090 | 792 | f-3 | 329004 | 329118 | 29 | - | N | Predicted secreted acid phosphatase |
| 374 | 329290 | 330090 | 1760088 | 1759288 | 62 | f-1 | 329380 | 329929 | 44 | Smc | D | Chromosome segregation ATPases |
| 375 | 330224 | 331687 | 1759154 | 1757691 | 417 | f-2 | 330827 | 331406 | 42 | RfaG | M | Predicted glycosyltransferases |
| 376 | 331691 | 332452 | 1757687 | 1756926 | 418 | f-2 | 332153 | 332312 | 32 | GlmU | M | N-acetylglucosamine-1-phosphate uridyltransferase (contains nucleotidyltransferase and I-patch acetyltransferase domains) |
| 377 | 332449 | 332736 | 1756929 | 1756642 | 63 | f-1 | | | | | | |

| | | | | | | | | | | | |
|---|---|---|---|---|---|---|---|---|---|---|---|
| 378 | 334175 | 334945 | 1755203 | 1754433 | 419 | f-2 | 334223 | 334319 | 31 | CirA | P | Outer membrane receptor proteins |
| 379 | 335068 | 335664 | 1754310 | 1753714 | 64 | f-1 | 335158 | 335434 | 35 | - | R | Uncharacterized CBS domain-containing proteins |
| 380 | 337045 | 337260 | 1752333 | 1752118 | 65 | f-1 | 337087 | 337222 | 28 | - | G C | Glycosyl transferases |
| 381 | 337711 | 338295 | 1751667 | 1751083 | 1408 | r-1 | 338050 | 338284 | 37 | - | L | MutS-like ATPases involved in mismatch repair |
| 382 | 339363 | 339788 | 1750015 | 1749590 | 793 | f-3 | 339441 | 339639 | 34 | - | L | Replication factor A large subunit and related ssDNA-binding proteins |
| 383 | 340641 | 340727 | 1748737 | 1748651 | 794 | f-3 | | | | | | |
| 384 | 341558 | 341995 | 1747820 | 1747383 | 420 | f-2 | 341600 | 341747 | 42 | AbrB | K | Regulators of stationary/sporulation gene expression |
| 385 | 342397 | 343461 | 1746981 | 1745917 | 66 | f-1 | 343126 | 343363 | 36 | MarR | K | Transcriptional regulators |
| 386 | 343454 | 343891 | 1745924 | 1745487 | 421 | f-2 | 343538 | 343760 | 32 | - | S | Uncharacterized BCR |
| 387 | 343888 | 344076 | 1745490 | 1745302 | 67 | f-1 | 343912 | 343987 | 29 | PyrG | F | CTP synthase (UTP-ammonia lyase) |
| 388 | 344090 | 344401 | 1745288 | 1744977 | 422 | f-2 | | | | | | |
| 389 | 345281 | 345472 | 1744097 | 1743906 | 423 | f-2 | 345350 | 345464 | 26 | NlpD | M | Membrane proteins related to metalloendopeptidases |
| 390 | 345566 | 345622 | 1743812 | 1743756 | 2098 | r-3 | | | | | | |

EP 1 923 464 A1

| | | | | | | | | | | | |
|---|---|---|---|---|---|---|---|---|---|---|---|
| 391 | 345615 | 345740 | 1743763 | 1743638 | 795 | f-3 | | | | | |
| 392 | 346174 | 346356 | 1743204 | 1743022 | 68 | f-1 | 346183 | 346297 | 28 | NrfG | R | TPR-repeat-containing proteins |
| 393 | 346528 | 346881 | 1742850 | 1742497 | 69 | f-1 | 346651 | 346837 | 28 | - | L | Replication factor A large subunit and related ssDNA-binding proteins |
| 394 | 346606 | 346668 | 1742772 | 1742710 | 1407 | r-1 | | | | | |
| 395 | 347138 | 348463 | 1742240 | 1740915 | 424 | f-2 | 347351 | 348461 | 427 | - | S | Uncharacterized ACR |
| 396 | 348567 | 350417 | 1740811 | 1738961 | 1786 | r-2 | 348567 | 350403 | 1032 | - | E | Serine proteases of the peptidase family S9A |
| 397 | 350537 | 351598 | 1738841 | 1737780 | 425 | f-2 | 350537 | 350981 | 162 | RibD | H | Pyrimidine deaminase |
| 398 | 351592 | 352155 | 1737786 | 1737223 | 70 | f-1 | 351601 | 352150 | 191 | RibC | H | Riboflavin synthase alpha chain |
| 399 | 352419 | 352985 | 1736959 | 1736393 | 796 | f-3 | 352461 | 352647 | 30 | - | R | Predicted membrane-associated |
| 400 | 353923 | 354102 | 1735455 | 1735276 | 71 | f-1 | 354010 | 354097 | 25 | LytR | K | Transcriptional regulator |
| 401 | 354174 | 355334 | 1735204 | 1734044 | 797 | f-3 | 354723 | 355320 | 243 | RibA | H | GTP cyclohydrolase II |
| 402 | 355393 | 355872 | 1733985 | 1733506 | 72 | f-1 | 355414 | 355849 | 170 | RibH | H | Riboflavin synthase beta-chain |
| 403 | 355856 | 356452 | 1733522 | 1732926 | 2097 | r-3 | 355862 | 356387 | 125 | - | S | Uncharacterized ArCR |
| 404 | 356449 | 357381 | 1732929 | 1731997 | 1406 | r-1 | 356455 | 357211 | 170 | - | R | ATP-utilizing enzymes of ATP-grasp superfamily (probably carboligases) |
| 405 | 357378 | 358037 | 1732000 | 1731341 | 1785 | r-2 | 357378 | 357969 | 140 | PurC | F | Phosphoribosylaminoimidazolesu ccinocarboxamide (SAICAR) |

EP 1 923 464 A1

128

| | | | | | | | | | | | |
|---|---|---|---|---|---|---|---|---|---|---|---|
| | | | | | | | | | | | synthase |
| 406 | 358034 | 359329 | 1731344 | 1730049 | 2096 | r-3 | 358043 | 359312 | 651 | ThiC | H | Thiamine biosynthesis protein ThiC |
| 407 | 359407 | 360171 | 1729971 | 1729207 | 73 | f-1 | 359416 | 360163 | 386 | - | R | Flavoproteins |
| 408 | 360168 | 361466 | 1729210 | 1727912 | 798 | f-3 | 360171 | 360888 | 200 | ThiD | H | Hydroxymethylpyrimidine/phosp homethylpyrimidine kinase |
| 409 | 361497 | 363407 | 1727881 | 1725971 | 799 | f-3 | 361506 | 363378 | 1016 | - | R | Uncharacterized ABC-type transporter |
| 410 | 366699 | 367151 | 1722679 | 1722227 | 1784 | r-2 | 366879 | 367050 | 33 | - | R | Predicted metal-dependent membrane protease |
| 411 | 367290 | 368240 | 1722088 | 1721138 | 1783 | r-2 | 367932 | 368190 | 35 | HypF | O | Hydrogenase maturation factor |
| 412 | 368237 | 369289 | 1721141 | 1720089 | 2095 | r-3 | 368243 | 368948 | 301 | SlpA | O | FKBP-type peptidyl-prolyl cis-trans isomerases 2 |
| 413 | 370634 | 371449 | 1718744 | 1717929 | 426 | f-2 | 371216 | 371363 | 30 | CaiA | I | Acyl-CoA dehydrogenases |
| 414 | 371481 | 372920 | 1717897 | 1716458 | 800 | f-3 | 371490 | 372918 | 859 | CysS | J | Cysteinyl-tRNA synthetase |
| 415 | 374488 | 374550 | 1714890 | 1714828 | 74 | f-1 | | | | | | |
| 416 | 374583 | 374840 | 1714795 | 1714538 | 801 | f-3 | 374583 | 374832 | 129 | - | S | Uncharacterized ACR |
| 417 | 374833 | 375534 | 1714545 | 1713844 | 1405 | r-1 | 375247 | 375427 | 32 | - | L | Predicted transposase |
| 418 | 375535 | 376308 | 1713843 | 1713070 | 1404 | r-1 | 375535 | 376294 | 105 | - | S | Uncharacterized ACR |
| 419 | 376000 | 376092 | 1713378 | 1713286 | 75 | f-1 | | | | | | |

| | | | | | | | | | | | |
|---|---|---|---|---|---|---|---|---|---|---|---|
| 420 | 376298 | 376771 | 1713080 | 1712607 | 2094 | r-3 | 376298 | 376769 | 238 | - | K | Predicted transcriptional regulator |
| 421 | 379177 | 380310 | 1710201 | 1709068 | 1403 | r-1 | 379756 | 379984 | 38 | Tar | N | Methyl-accepting chemotaxis protein |
| 422 | 380366 | 381109 | 1709012 | 1708269 | 2093 | r-3 | 380558 | 381047 | 32 | SPS1 | T | Serine/threonine protein kinases |
| 423 | 381111 | 382313 | 1708267 | 1707065 | 1782 | r-2 | 381642 | 382305 | 360 | - | S | Uncharacterized ACR |
| 424 | 382310 | 382675 | 1707068 | 1706703 | 2092 | r-3 | 382454 | 382604 | 29 | HisS | J | Histidyl-tRNA synthetase |
| 425 | 382850 | 383839 | 1706528 | 1705539 | 2091 | r-3 | 382859 | 383837 | 516 | - | S | Uncharacterized ACR |
| 426 | 384244 | 384471 | 1705134 | 1704907 | 1402 | r-1 | 384244 | 384304 | 42 | AbrB | K | Regulators of stationary/sporulation gene expression |
| 427 | 384528 | 385040 | 1704850 | 1704338 | 1781 | r-2 | 384534 | 385035 | 239 | - | L | RecB family exonuclease |
| 428 | 385030 | 386139 | 1704348 | 1703239 | 1401 | r-1 | 385138 | 385843 | 40 | - | R | Predicted ATPase of the AAA superfamily |
| 429 | 389056 | 390132 | 1700322 | 1699246 | 1400 | r-1 | 389056 | 390127 | 503 | - | S | Uncharacterized ACR |
| 430 | 390129 | 391328 | 1699249 | 1698050 | 1780 | r-2 | 390450 | 390630 | 32 | - | S | Uncharacterized proteins of WD40-like repeat family |
| 431 | 391570 | 392187 | 1697808 | 1697191 | 1399 | r-1 | 391570 | 392140 | 247 | - | S | Uncharacterized ACR |
| 432 | 392614 | 393321 | 1696764 | 1696057 | 1398 | r-1 | 392674 | 393319 | 399 | - | C | Acyl-CoA synthetase (NDP forming) |

130

| 433 | 393449 | 394750 | 1695929 | 1694628 | 427 | f-2 | 394415 | 394688 | 30 | WcaG | M G | Nucleoside-diphosphate-sugar epimerases COG0451 WcaG |
|---|---|---|---|---|---|---|---|---|---|---|---|---|
| 434 | 394894 | 398109 | 1694484 | 1691269 | 76 | f-1 | 396901 | 397378 | 42 | Tar | N | Methyl-accepting chemotaxis protein |
| 435 | 398178 | 398471 | 1691200 | 1690907 | 1779 | r-2 | 398202 | 398352 | 27 | Sms | O | Predicted ATP-dependent serine protease |
| 436 | 398502 | 399011 | 1690876 | 1690367 | 802 | f-3 | 398772 | 398904 | 30 | EmrK | Q | Multidrug resistance efflux pump |
| 437 | 399050 | 404185 | 1690328 | 1685193 | 428 | f-2 | 399050 | 401933 | 1348 | - | L | Reverse gyrase |
| 438 | 404484 | 405290 | 1684894 | 1684088 | 803 | f-3 | 404487 | 405282 | 409 | - | K | Predicted transcriptional regulators |
| 439 | 405419 | 405631 | 1683959 | 1683747 | 2090 | r-3 | 405422 | 405554 | 38 | - | K | Predicted transcriptional regulator |
| 440 | 405628 | 405963 | 1683750 | 1683415 | 1397 | r-1 | 405640 | 405955 | 155 | - | R | Uncharacterized Zn-finger containing protein |
| 441 | 405960 | 406709 | 1683418 | 1682669 | 1778 | r-2 | 405975 | 406707 | 256 | SpeB | E | Arginase/agmatinase/formimiono glutamate hydrolase |
| 442 | 406835 | 408055 | 1682543 | 1681323 | 429 | f-2 | 406835 | 407465 | 358 | SgbH | G | 3-hexulose-6-phosphate synthase and related proteins |
| 443 | 408052 | 408807 | 1681326 | 1680571 | 77 | f-1 | 408082 | 408796 | 262 | FtsZ | D | Cell division GTPase |
| 444 | 408809 | 409462 | 1680569 | 1679916 | 430 | f-2 | 408818 | 409448 | 248 | - | R | Predicted hydrolases of the HAD superfamily |

| | | | | | | | | | | | | | |
|---|---|---|---|---|---|---|---|---|---|---|---|---|---|
| 445 | 409459 | 409647 | 1679919 | 1679731 | 78 | f-1 | 409495 | 409645 | 30 | WcaG | M G | Nucleoside-diphosphate-sugar epimerases COG0451 WcaG |
| 446 | 409647 | 410459 | 1679731 | 1678919 | 804 | f-3 | 409902 | 410307 | 33 | - | Q | Polyketide synthase modules and related proteins |
| 447 | 410460 | 411080 | 1678918 | 1678298 | 805 | f-3 | 410499 | 411027 | 205 | - | R | Predicted HD superfamily hydrolase |
| 448 | 411176 | 411688 | 1678202 | 1677690 | 431 | f-2 | 411176 | 411686 | 227 | NusA | K | Transcription terminator |
| 449 | 411878 | 413293 | 1677500 | 1676085 | 432 | f-2 | 412490 | 413045 | 36 | - | K | Predicted transcriptional regulators |
| 450 | 413415 | 413915 | 1675963 | 1675463 | 806 | f-3 | 413523 | 413754 | 39 | GyrA | L | DNA gyrase (topoisomerase II) A subunit |
| 451 | 413926 | 414252 | 1675452 | 1675126 | 79 | f-1 | 413938 | 414175 | 30 | SurA | O | Parvulin-like peptidyl-prolyl isomerase |
| 452 | 414877 | 415209 | 1674501 | 1674169 | 80 | f-1 | 414877 | 415123 | 31 | ArgS | J | Arginyl-tRNA synthetase |
| 453 | 417109 | 417270 | 1672269 | 1672108 | 81 | f-1 | 417115 | 417259 | 27 | PutA | E | Proline dehydrogenase |
| 454 | 417291 | 417929 | 1672087 | 1671449 | 807 | f-3 | 417330 | 417462 | 30 | MetC | E | Cystathionine beta-lyases/cystathionine gamma-synthases |
| 455 | 418636 | 419175 | 1670742 | 1670203 | 82 | f-1 | 418663 | 419017 | 33 | - | S | Uncharacterized proteins of WD40-like repeat family |
| 456 | 419247 | 420563 | 1670131 | 1668815 | 808 | f-3 | 419247 | 420561 | 771 | AsnS | J | Aspartyl/asparaginyl-tRNA |

132

| | | | | | | | | | | | | |
|---|---|---|---|---|---|---|---|---|---|---|---|---|
| | | | | | | | | | | | | synthetases (Aspartyl-tRNA synthetase) |
| 457 | 420627 | 422132 | 1668751 | 1667246 | 809 | f-3 | 421635 | 421917 | 33 | - | R | Uncharacterized membrane protein |
| 458 | 422333 | 422719 | 1667045 | 1666659 | 433 | f-2 | | | | | | |
| 459 | 422876 | 424030 | 1666502 | 1665348 | 2089 | r-3 | 422876 | 424019 | 541 | AbgB | R | Metal-dependent amidase/aminoacylase/carboxype ptidase |
| 460 | 426547 | 426711 | 1662831 | 1662667 | 83 | f-1 | | | | | | |
| 461 | 426747 | 427742 | 1662631 | 1661636 | 810 | f-3 | 426750 | 427734 | 452 | - | R | Predicted methyltransferase |
| 462 | 427799 | 429064 | 1661579 | 1660314 | 434 | f-2 | 427820 | 429011 | 224 | - | R | Uncharacterized ATPases of the AAA superfamily |
| 463 | 429065 | 430390 | 1660313 | 1658988 | 2088 | r-3 | 429065 | 430388 | 624 | TldD | R | Predicted Zn-dependent proteases and their inactivated homologs |
| 464 | 430394 | 430633 | 1658984 | 1658745 | 2087 | r-3 | 430490 | 430592 | 30 | SpoU | J | rRNA methylases |
| 465 | 430618 | 430785 | 1658760 | 1658593 | 1396 | r-1 | 430654 | 430720 | 25 | PncA | Q | Amidases related to nicotinamidase |
| 466 | 430883 | 432259 | 1658495 | 1657119 | 2086 | r-3 | 430883 | 432257 | 780 | TldD | R | Predicted Zn-dependent proteases and their inactivated homologs |
| 467 | 432397 | 432738 | 1656981 | 1656640 | 84 | f-1 | 432397 | 432733 | 176 | - | S | Uncharacterized ACR |
| 468 | 432751 | 433449 | 1656627 | 1655929 | 85 | f-1 | 432760 | 433429 | 319 | RacX | M | Aspartate racemase |

| | | | | | | | | | | | | |
|---|---|---|---|---|---|---|---|---|---|---|---|---|
| 469 | 433446 | 434621 | 1655932 | 1654757 | 1777 | r-2 | 433650 | 434616 | 391 | CorA | P | Mg2+ and Co2+ transporters |
| 470 | 434530 | 435735 | 1654848 | 1653643 | 86 | f-1 | 434542 | 435733 | 681 | - | R | Predicted GTPase |
| 471 | 435779 | 436300 | 1653599 | 1653078 | 2085 | r-3 | 435779 | 436295 | 208 | CyaB | F | Adenylate cyclase |
| 472 | 436300 | 436812 | 1653078 | 1652566 | 1395 | r-1 | 436339 | 436810 | 201 | Lrp | K | Transcriptional regulators |
| 473 | 437409 | 438209 | 1651969 | 1651169 | 811 | f-3 | 437415 | 438207 | 286 | - | S | Uncharacterized ACR |
| 474 | 438222 | 439658 | 1651156 | 1649720 | 1776 | r-2 | 438222 | 439650 | 588 | PykF | G | Pyruvate kinase |
| 475 | 439696 | 440403 | 1649682 | 1648975 | 1394 | r-1 | 439696 | 440368 | 147 | - | R | Predicted Zn-dependent proteases |
| 476 | 440578 | 441444 | 1648800 | 1647934 | 87 | f-1 | 440578 | 441442 | 390 | - | S | Uncharacterized ArCR |
| 477 | 441511 | 441882 | 1647867 | 1647496 | 88 | f-1 | 441511 | 441880 | 136 | CrcB | D | Integral membrane protein possibly involved in chromosome condensation |
| 478 | 441887 | 442267 | 1647491 | 1647111 | 435 | f-2 | 441887 | 442262 | 231 | - | S | Uncharacterized ACR |
| 479 | 442358 | 442873 | 1647020 | 1646505 | 436 | f-2 | 442448 | 442634 | 29 | - | G | 2-Phosphoglycerate kinase |
| 480 | 442922 | 444142 | 1646456 | 1645236 | 437 | f-2 | 442931 | 444140 | 630 | Dfp | H | Phosphopantothenoylcysteine synthetase/decarboxylase |
| 481 | 444220 | 444681 | 1645158 | 1644697 | 89 | f-1 | 444295 | 444607 | 39 | ZntA | P | Cation transport ATPases |
| 482 | 444972 | 445310 | 1644406 | 1644068 | 812 | f-3 | 444972 | 445278 | 69 | - | S | Uncharacterized ACR |
| 483 | 446197 | 448899 | 1643181 | 1640479 | 1393 | r-1 | 446209 | 448864 | 962 | - | R | Distinct helicase family with a unique C-terminal domain including a metal-binding |

| | | | | | | | | | | | |
|---|---|---|---|---|---|---|---|---|---|---|---|
| | | | | | | | | | | | cysteine cluster |
| 484 | 448945 | 450294 | 1640433 | 1639084 | 1392 | r-1 | 449620 | 450244 | 148 | - | R | Predicted hydrolase of the alpha/beta superfamily |
| 485 | 450481 | 450996 | 1638897 | 1638382 | 90 | f-1 | 450481 | 450994 | 274 | - | C | Rubrerythrin |
| 486 | 451077 | 451238 | 1638301 | 1638140 | 813 | f-3 | 451077 | 451236 | 111 | - | C | Rubredoxin |
| 487 | 451250 | 451597 | 1638128 | 1637781 | 438 | f-2 | 451250 | 451595 | 224 | - | C | Desulfoferrodoxin |
| 488 | 452770 | 453123 | 1636608 | 1636255 | 91 | f-1 | 452818 | 452929 | 33 | Mrp | D | ATPases involved in chromosome partitioning |
| 489 | 453183 | 454601 | 1636195 | 1634777 | 814 | f-3 | 453318 | 454590 | 772 | GlyA | E | Glycine hydroxymethyltransferase |
| 490 | 454835 | 455341 | 1634543 | 1634037 | 439 | f-2 | 454952 | 455234 | 33 | - | R | Large extracellular alpha-helical protein |
| 491 | 455338 | 455502 | 1634040 | 1633876 | 92 | f-1 | 455362 | 455437 | 25 | - | G | Cellobiose phosphorylase |
| 492 | 456330 | 456662 | 1633048 | 1632716 | 815 | f-3 | 456330 | 456660 | 174 | RPB9 | K | DNA-directed RNA polymerase subunit M/Transcription elongation factor TFIIS |
| 493 | 456623 | 456835 | 1632755 | 1632543 | 440 | f-2 | 456659 | 456734 | 28 | WecD | K R | Histone acetyltransferase HPA2 and related acetyltransferases COG0454 WecD |

| 494 | 456838 | 457587 | 1632540 | 1631791 | 93 | f-1 | 456838 | 457585 | 358 | DnaN | L | DNA polymerase III beta subunit (Proliferating cell nuclear antigen=PCNA) |
|---|---|---|---|---|---|---|---|---|---|---|---|---|
| 495 | 457618 | 458184 | 1631760 | 1631194 | 94 | f-1 | 457618 | 458128 | 140 | - | S | Uncharacterized ArCR |
| 496 | 458476 | 459126 | 1630902 | 1630252 | 95 | f-1 | 458476 | 459124 | 417 | AhpC | O | Peroxiredoxin |
| 497 | 459138 | 459680 | 1630240 | 1629698 | 1775 | r-2 | 459147 | 459678 | 164 | RimL | J | Acetyltransferases |
| 498 | 459718 | 460674 | 1629660 | 1628704 | 96 | f-1 | 459718 | 460603 | 345 | - | K | Predicted transcriptional regulators |
| 499 | 460667 | 461935 | 1628711 | 1627443 | 2084 | r-3 | 460670 | 461927 | 532 | - | R | HD superfamily phosphohydrolases |
| 500 | 462618 | 463808 | 1626760 | 1625570 | 1774 | r-2 | 462624 | 463764 | 576 | MoeA | H | Molybdopterin biosynthesis enzyme |
| 501 | 464266 | 464421 | 1625112 | 1624957 | 1391 | r-1 | 464320 | 464380 | 26 | RplW | J | Ribosomal protein L23 |
| 502 | 464460 | 464972 | 1624918 | 1624406 | 1773 | r-2 | 464460 | 464970 | 218 | MoaB | H | Molybdopterin biosynthesis enzymes |
| 503 | 465336 | 466562 | 1624042 | 1622816 | 816 | f-3 | 465360 | 466560 | 653 | - | S | Uncharacterized ACR |
| 504 | 466632 | 466847 | 1622746 | 1622531 | 1772 | r-2 | | | | | | |
| 505 | 466975 | 467631 | 1622403 | 1621747 | 97 | f-1 | 466975 | 467581 | 273 | - | R | Predicted phosphoesterases |
| 506 | 467628 | 468806 | 1621750 | 1620572 | 1771 | r-2 | 467637 | 468804 | 686 | AvtA | E | PLP-dependent aminotransferases |
| 507 | 471018 | 472637 | 1618360 | 1616741 | 1770 | r-2 | 471027 | 472629 | 799 | - | O | Predicted carbamoyl transferase |
| 508 | 472691 | 474145 | 1616687 | 1615233 | 2083 | r-3 | 472706 | 474143 | 726 | ProS | J | Prolyl-tRNA synthetase |

136

| 509 | 474239 | 475240 | 1615139 | 1614138 | 441 | f-2 | 474239 | 475193 | 469 | LdhA | C H R | Lactate dehydrogenase and related dehydrogenases COG1052 LdhA |
|---|---|---|---|---|---|---|---|---|---|---|---|---|
| 510 | 475250 | 475708 | 1614128 | 1613670 | 442 | f-2 | 475403 | 475541 | 45 | FrvX | G | Cellulase M and related proteins |
| 511 | 475702 | 477042 | 1613676 | 1612336 | 98 | f-1 | 475768 | 477031 | 662 | - | R | Predicted DNA-binding protein containing a Zn-ribbon domain |
| 512 | 477049 | 477657 | 1612329 | 1611721 | 99 | f-1 | 477061 | 477640 | 249 | - | S | Uncharacterized ACR |
| 513 | 477738 | 478031 | 1611640 | 1611347 | 817 | f-3 | | | | | | |
| 514 | 477971 | 479050 | 1611407 | 1610328 | 2082 | r-3 | 477980 | 479039 | 533 | GCN3 | J | Translation initiation factor eIF-2B alpha subunit |
| 515 | 478881 | 479639 | 1610497 | 1609739 | 818 | f-3 | 479103 | 479622 | 191 | - | R | Predicted ATPases or kinases |
| 516 | 479629 | 480162 | 1609749 | 1609216 | 1390 | r-1 | 479635 | 480148 | 228 | - | R | CBS domains |
| 517 | 480198 | 480755 | 1609180 | 1608623 | 1769 | r-2 | 480219 | 480501 | 52 | ArsR | K | Predicted transcriptional regulators |
| 518 | 480843 | 481127 | 1608535 | 1608251 | 1768 | r-2 | 480852 | 481119 | 129 | Ssh10 b | K | Archaeal DNA-binding protein |
| 519 | 481315 | 482679 | 1608063 | 1606699 | 100 | f-1 | 481315 | 482656 | 775 | PurB | F | Adenylosuccinate lyase |
| 520 | 484981 | 485445 | 1604397 | 1603933 | 101 | f-1 | 485002 | 485437 | 219 | - | H | 6-pyruvoyl-tetrahydropterin synthase |
| 521 | 485442 | 486008 | 1603936 | 1603370 | 1767 | r-2 | 485529 | 485790 | 31 | TrpD | E | Anthranilate phosphoribosyltransferase |

EP 1 923 464 A1

| | | | | | | | | | | | | |
|---|---|---|---|---|---|---|---|---|---|---|---|---|
| 522 | 486065 | 486484 | 1603313 | 1602894 | 443 | f-2 | 486080 | 486473 | 167 | - | R | Predicted DNA-binding proteins with PD1-like DNA-binding motif |
| 523 | 486481 | 488979 | 1602897 | 1600399 | 1389 | r-1 | 486481 | 488977 | 1328 | - | R | Specific archaeal helicases |
| 524 | 489517 | 490644 | 1599861 | 1598734 | 1388 | r-1 | 489604 | 490642 | 651 | TyrS | J | Tyrosyl-tRNA synthetase |
| 525 | 490744 | 491844 | 1598634 | 1597534 | 102 | f-1 | 491755 | 491842 | 38 | OppA | EP | ABC-type dipeptide/oligopeptide/nickel transport systems |
| 526 | 491922 | 493376 | 1597456 | 1596002 | 819 | f-3 | 492033 | 493350 | 412 | TbpA | H | ABC-type iron/thiamine transport systems |
| 527 | 493561 | 495408 | 1595817 | 1593970 | 103 | f-1 | 493843 | 495388 | 396 | ThiP | H | ABC-type thiamine transport system |
| 528 | 495410 | 496480 | 1593968 | 1592898 | 444 | f-2 | 495419 | 496436 | 314 | MalK | G | ABC-type sugar/spermidine/putrescine/iron/thiamine transport systems |
| 529 | 497090 | 499186 | 1592288 | 1590192 | 445 | f-2 | 497276 | 498920 | 114 | Icc | R | Predicted phosphohydrolases |
| 530 | 499596 | 499949 | 1589782 | 1589429 | 1766 | r-2 | 499647 | 499797 | 30 | MipB | G | Transaldolase |
| 531 | 500938 | 501252 | 1588440 | 1588126 | 1387 | r-1 | 500971 | 501085 | 29 | SpoU | J | rRNA methylases |
| 532 | 501249 | 501479 | 1588129 | 1587899 | 1765 | r-2 | 501312 | 501420 | 28 | AceE | C | Pyruvate dehydrogenase |
| 533 | 501658 | 502464 | 1587720 | 1586914 | 1386 | r-1 | 501703 | 502453 | 241 | DnaN | L | DNA polymerase III beta subunit (Proliferating cell nuclear |

138

| | | | | | | | | | | | | antigen=PCNA) |
|---|---|---|---|---|---|---|---|---|---|---|---|---|
| 534 | 502547 | 502792 | 1586586 | 1586831 | 2081 | r-3 | 502661 | 502784 | 30 | XylB | G | Sugar (pentulose and hexulose) kinases |
| 535 | 502785 | 502967 | 1586411 | 1586593 | 1764 | r-2 | 502821 | 502959 | 32 | RpoC | K | DNA-directed RNA polymerase beta' subunit/160 kD subunit (split gene in archaea and Syn) |
| 536 | 503187 | 503354 | 1586024 | 1586191 | 820 | f-3 | 503241 | 503325 | 26 | AcrA | Q | Membrane-fusion protein |
| 537 | 504971 | 505099 | 1584279 | 1584407 | 446 | f-2 | 504971 | 505094 | 26 | MarR | K | Transcriptional regulators |
| 538 | 506242 | 506664 | 1582714 | 1583136 | 1385 | r-1 | 506404 | 506635 | 35 | DeoA | F | Thymidine phosphorylase |
| 539 | 507506 | 507592 | 1581786 | 1581872 | 447 | f-2 | | | | | | |
| 540 | 508803 | 509420 | 1579958 | 1580575 | 1763 | r-2 | 509091 | 509313 | 33 | - | L | Inteins |
| 541 | 510163 | 510879 | 1578499 | 1579215 | 1384 | r-1 | 510235 | 510490 | 34 | - | S | Uncharacterized ACR |
| 542 | 511923 | 512477 | 1576901 | 1577455 | 1762 | r-2 | 512034 | 512298 | 34 | MtlA | C | Phosphotransferase system |
| 543 | 513104 | 513481 | 1575897 | 1576274 | 448 | f-2 | 513269 | 513386 | 30 | ClpA | O | ATPases with chaperone activity |
| 544 | 513710 | 514261 | 1575117 | 1575668 | 2080 | r-3 | 513953 | 514163 | 30 | Hit | FG R | Diadenosine tetraphosphate (Ap4A) hydrolase and other HIT family hydrolases COG0537 Hit |
| 545 | 514843 | 515223 | 1574155 | 1574535 | 1383 | r-1 | 514873 | 515029 | 29 | - | R | Uncharacterized proteins of the AP superfamily |
| 546 | 515543 | 515791 | 1573587 | 1573835 | 2079 | r-3 | 515636 | 515699 | 28 | RplC | J | Ribosomal protein L3 |

| 547 | 517003 | 517803 | 1572375 | 1571575 | 1382 | r-1 | 517276 | 517618 | 44 | Smc | D | Chromosome segregation ATPases |
|---|---|---|---|---|---|---|---|---|---|---|---|---|
| 548 | 517805 | 518281 | 1571573 | 1571097 | 2078 | r-3 | 517997 | 518111 | 29 | - | N | Predicted secreted acid phosphatase |
| 549 | 518278 | 518760 | 1571100 | 1570618 | 1381 | r-1 | 518296 | 518515 | 28 | - | R | Predicted hydrolase of alkaline phosphatase superfamily |
| 550 | 518772 | 519575 | 1570606 | 1569803 | 1761 | r-2 | | | | | | |
| 551 | 519579 | 519809 | 1569799 | 1569569 | 1760 | r-2 | 519735 | 519798 | 26 | Rbn | J | tRNA-processing ribonuclease BN |
| 552 | 520158 | 520541 | 1569220 | 1568837 | 1759 | r-2 | 520245 | 520398 | 31 | AmtB | P | Ammonia permeases |
| 553 | 520694 | 522628 | 1568684 | 1566750 | 2077 | r-3 | 521111 | 521303 | 34 | Arp | R | Ankyrin repeat proteins |
| 554 | 522837 | 524828 | 1566541 | 1564550 | 1758 | r-2 | 523617 | 523854 | 35 | - | S | Predicted membrane protein |
| 555 | 524728 | 525042 | 1564650 | 1564336 | 1380 | r-1 | 524737 | 524905 | 31 | CysZ | E | Uncharacterized protein involved in cysteine biosynthesis |
| 556 | 525397 | 525585 | 1563981 | 1563793 | 1379 | r-1 | 525406 | 525538 | 28 | - | R | Predicted nucleic acid-binding protein |
| 557 | 525884 | 526483 | 1563494 | 1562895 | 2076 | r-3 | 526004 | 526199 | 29 | - | K | Predicted RNA-binding protein homologous to eukaryotic snRNP |
| 558 | 527199 | 527468 | 1562179 | 1561910 | 821 | f-3 | 527208 | 527451 | 153 | RPL43 A | J | Ribosomal protein L37AE/L43A |

EP 1 923 464 A1

| 559 | 527689 | 528324 | 1561689 | 1561054 | 104 | f-1 | 527698 | 528319 | 339 | IMP4 | J | Protein containing the IMP4 domain present in small nuclear ribonucleoproteins; implicated in RNA processing |
|---|---|---|---|---|---|---|---|---|---|---|---|---|
| 560 | 528364 | 528969 | 1561014 | 1560409 | 105 | f-1 | 528364 | 528967 | 266 | MnhE | P | Multisubunit Na+/H+ antiporter |
| 561 | 528984 | 529217 | 1560394 | 1560161 | 822 | f-3 | 528993 | 529212 | 84 | MnhF | P | Multisubunit Na+/H+ antiporter |
| 562 | 529214 | 529528 | 1560164 | 1559850 | 449 | f-2 | 529280 | 529526 | 97 | MnhG | P | Multisubunit Na+/H+ antiporter |
| 563 | 529509 | 529739 | 1559869 | 1559639 | 823 | f-3 | 529509 | 529737 | 61 | MnhB | P | Multisubunit Na+/H+ antiporter |
| 564 | 529736 | 529981 | 1559642 | 1559397 | 450 | f-2 | 529817 | 529979 | 59 | MnhB | P | Multisubunit Na+/H+ antiporter |
| 565 | 529978 | 530385 | 1559400 | 1558993 | 106 | f-1 | 529978 | 530383 | 122 | MnhB | P | Multisubunit Na+/H+ antiporter |
| 566 | 530659 | 532146 | 1558719 | 1557232 | 107 | f-1 | 530749 | 531982 | 315 | HyfB | CP | Formate hydrogenlyase subunit 3/Multisubunit Na+/H+ antiporter |
| 567 | 532123 | 532530 | 1557255 | 1556848 | 1378 | r-1 | 532123 | 532525 | 172 | IlvH | E | Acetolactate synthase |
| 568 | 532615 | 533754 | 1556763 | 1555624 | 108 | f-1 | 532684 | 533521 | 77 | KefB | P | Kef-type K+ transport systems |
| 569 | 533789 | 534916 | 1555589 | 1554462 | 451 | f-2 | 534575 | 534905 | 33 | Smc | D | Chromosome segregation ATPases |
| 570 | 534917 | 535363 | 1554461 | 1554015 | 2075 | r-3 | 534926 | 535361 | 249 | CheW | N | Chemotaxis signal transduction protein |
| 571 | 535366 | 536694 | 1554012 | 1552684 | 1377 | r-1 | 535876 | 536542 | 231 | Tar | N | Methyl-accepting chemotaxis protein |

| | | | | | | | | | | | |
|---|---|---|---|---|---|---|---|---|---|---|---|
| 572 | 536818 | 536871 | 1552560 | 1552507 | 1376 | r-1 | | | | | |
| 573 | 536998 | 537846 | 1552380 | 1551532 | 109 | f-1 | 537025 | 537838 | 375 | CheR | NT | Methylase of chemotaxis methyl-accepting proteins COG1352 CheR |
| 574 | 537847 | 538209 | 1551531 | 1551169 | 110 | f-1 | 537847 | 538207 | 224 | CheY | T | CheY-like receiver domains |
| 575 | 538230 | 539297 | 1551148 | 1550081 | 824 | f-3 | 538230 | 539286 | 509 | CheB | NT | Chemotaxis response regulator CheB |
| 576 | 539304 | 540950 | 1550074 | 1548428 | 825 | f-3 | 539304 | 540906 | 521 | CheA | N | Chemotaxis protein histidine kinase and related kinases |
| 577 | 540986 | 541681 | 1548392 | 1547697 | 452 | f-2 | 540986 | 541628 | 349 | CheA | N | Chemotaxis protein histidine kinase and related kinases |
| 578 | 541671 | 542294 | 1547707 | 1547084 | 826 | f-3 | 541680 | 542289 | 293 | CheC | NT | Chemotaxis protein CheC |
| 579 | 542291 | 542914 | 1547087 | 1546464 | 453 | f-2 | 542291 | 542903 | 303 | CheC | NT | Chemotaxis protein CheC |
| 580 | 542904 | 545159 | 1546474 | 1544219 | 827 | f-3 | 542916 | 545154 | 640 | Tar | N | Methyl-accepting chemotaxis protein |
| 581 | 545191 | 545688 | 1544187 | 1543690 | 111 | f-1 | 545206 | 545686 | 259 | CheD | NT | Chemotaxis protein; stimulates methylation of MCP proteins COG1871 CheD |
| 582 | 545706 | 546455 | 1543672 | 1542923 | 828 | f-3 | 545892 | 546411 | 40 | - | S | Uncharacterized archaeal coiled-coil domain |
| 583 | 546468 | 547502 | 1542910 | 1541876 | 829 | f-3 | 546477 | 547491 | 366 | - | S | Uncharacterized ACR |

| 584 | 547499 | 547759 | 1541879 | 1541619 | 454 | f-2 | 547538 | 547757 | 92 | - | S | Uncharacterized ArCR |
|---|---|---|---|---|---|---|---|---|---|---|---|---|
| 585 | 547830 | 548183 | 1541548 | 1541195 | 830 | f-3 | 547830 | 548181 | 136 | GimC | O | Prefoldin |
| 586 | 548218 | 548553 | 1541160 | 1540825 | 112 | f-1 | 548227 | 548386 | 32 | Tas | C | Predicted oxidoreductases (related to aryl-alcohol dehydrogenases) |
| 587 | 548531 | 549514 | 1540847 | 1539864 | 455 | f-2 | 548531 | 549509 | 423 | - | R | Exopolyphosphatase-related proteins |
| 588 | 549515 | 549850 | 1539863 | 1539528 | 456 | f-2 | 549557 | 549824 | 30 | Cls | I | Phosphatidylserine/phosphatidylg lycerophosphate/cardioli pin synthases and related enzymes |
| 589 | 550080 | 551150 | 1539298 | 1538228 | 831 | f-3 | 550164 | 550494 | 32 | TatA | N | Sec-independent protein secretion pathway components |
| 590 | 551249 | 552460 | 1538129 | 1536918 | 457 | f-2 | 551270 | 552290 | 74 | NrfG | R | TPR-repeat-containing proteins |
| 591 | 552309 | 553043 | 1537069 | 1536335 | 832 | f-3 | 552318 | 553041 | 399 | - | R | Uncharacterized ArCR (contains C-terminal EMAP domain) |
| 592 | 553133 | 553699 | 1536245 | 1535679 | 458 | f-2 | 553214 | 553697 | 265 | - | S | Uncharacterized ACR |
| 593 | 553745 | 554734 | 1535633 | 1534644 | 2074 | r-3 | 553745 | 554720 | 466 | MviM | R | Predicted dehydrogenases and related proteins |
| 594 | 554855 | 555676 | 1534523 | 1533702 | 459 | f-2 | 554867 | 555674 | 401 | - | P | Predicted divalent heavy-metal cations transporter |
| 595 | 555783 | 556910 | 1533595 | 1532468 | 1757 | r-2 | 555882 | 556908 | 419 | FtsY | N | Signal recognition particle GTPase |

| 596 | 556879 | 558105 | 1532499 | 1531273 | 1375 | r-1 | 556879 | 558076 | 334 | - | L | Predicted transposases |
|---|---|---|---|---|---|---|---|---|---|---|---|---|
| 597 | 558125 | 558196 | 1531253 | 1531182 | 2073 | r-3 | | | | | | |
| 598 | 558864 | 559322 | 1530514 | 1530056 | 1756 | r-2 | 558897 | 559002 | 31 | - | L | Superfamily I DNA and RNA helicases and helicase subunits |
| 599 | 559506 | 560798 | 1529872 | 1528580 | 833 | f-3 | 560307 | 560760 | 144 | Med | N | Surface lipoprotein |
| 600 | 560838 | 562364 | 1528540 | 1527014 | 834 | f-3 | 560865 | 562350 | 525 | MglA | G | ABC-type sugar (aldose) transport system |
| 601 | 562361 | 563395 | 1527017 | 1525983 | 460 | f-2 | 562454 | 563390 | 164 | - | R | Uncharacterized ABC-type transport system |
| 602 | 563371 | 564303 | 1526007 | 1525075 | 113 | f-1 | 563407 | 564241 | 201 | - | R | Uncharacterized ABC-type transport system |
| 603 | 564310 | 565311 | 1525068 | 1524067 | 1374 | r-1 | 564310 | 565306 | 276 | ZnuA | P | ABC-type Mn/Zn transport system |
| 604 | 565409 | 567541 | 1523969 | 1521837 | 461 | f-2 | 566648 | 567164 | 34 | AceE | C | Pyruvate dehydrogenase |
| 605 | 567556 | 567786 | 1521822 | 1521592 | 1373 | r-1 | 567565 | 567664 | 28 | - | S | Uncharacterized stress-induced protein |
| 606 | 567865 | 568512 | 1521513 | 1520866 | 1372 | r-1 | 567865 | 568507 | 355 | - | R | Predicted phosphoribosyltransferases |
| 607 | 568711 | 570129 | 1520667 | 1519249 | 114 | f-1 | 568747 | 570121 | 813 | - | C | Acyl-CoA synthetase (NDP forming) |
| 608 | 570172 | 570729 | 1519206 | 1518649 | 1371 | r-1 | 570364 | 570493 | 30 | ChaC | P | Uncharacterized protein involved in cation transport |

| 609 | 570898 | 570957 | 1518480 | 1518421 | 115 | f-1 | | | | | | |
|---|---|---|---|---|---|---|---|---|---|---|---|---|
| 610 | 571031 | 571738 | 1518347 | 1517640 | 462 | f-2 | 571031 | 571736 | 351 | ApaH | T | Diadenosine tetraphosphatase and related serine/threonine protein phosphatases |
| 611 | 571735 | 572070 | 1517643 | 1517308 | 1370 | r-1 | 571735 | 571981 | 42 | - | S | Uncharacterized ACR |
| 612 | 572149 | 574656 | 1517229 | 1514722 | 1369 | r-1 | 572149 | 574636 | 1272 | SpoVK | O | ATPases of the AAA+ class (cell division control protein A) |
| 613 | 574653 | 575411 | 1514725 | 1513967 | 1755 | r-2 | 574734 | 575103 | 32 | - | S | Uncharacterized ACR |
| 614 | 575490 | 576503 | 1513888 | 1512875 | 1754 | r-2 | 575502 | 576498 | 595 | DYS1 | J | Deoxyhypusine synthase |
| 615 | 576540 | 577586 | 1512838 | 1511792 | 1753 | r-2 | 576540 | 577428 | 182 | GltD | ER | NADPH-dependent glutamate synthase beta chain and related oxidoreductases COG0493 GltD |
| 616 | 577750 | 578565 | 1511628 | 1510813 | 116 | f-1 | 577786 | 578563 | 355 | FabG | Q R | Dehydrogenases with different specificities (related to short-chain alcohol dehydrogenases) COG1028 FabG |
| 617 | 578612 | 579025 | 1510766 | 1510353 | 463 | f-2 | 578621 | 578960 | 151 | - | R | Predicted nucleotidyltransferases |
| 618 | 579392 | 579454 | 1509986 | 1509924 | 464 | f-2 | | | | | | |
| 619 | 580461 | 580553 | 1508917 | 1508825 | 1752 | r-2 | | | | | | |
| 620 | 581070 | 581168 | 1508308 | 1508210 | 1751 | r-2 | | | | | | |

| 621 | 582573 | 583445 | 1506805 | 1505933 | 1750 | r-2 | 582573 | 583443 | 326 | HtpX | O | Zn-dependent protease with chaperone function |
|---|---|---|---|---|---|---|---|---|---|---|---|---|
| 622 | 583582 | 585228 | 1505796 | 1504150 | 1368 | r-1 | 583582 | 585172 | 854 | GroL | O | Chaperonin GroEL (HSP60 family) (Chaperonin A) |
| 623 | 585396 | 586382 | 1503982 | 1502996 | 835 | f-3 | 585717 | 586377 | 332 | - | T | Mn2+-dependent serine/threonine protein kinase |
| 624 | 587383 | 587667 | 1501995 | 1501711 | 1367 | r-1 | 587404 | 587620 | 29 | TyrB | E | Aspartate/aromatic aminotransferase |
| 625 | 588220 | 589968 | 1501158 | 1499410 | 1366 | r-1 | 588244 | 589963 | 615 | - | L | MutS-like ATPases involved in mismatch repair |
| 626 | 590029 | 591039 | 1499349 | 1498339 | 1365 | r-1 | 590041 | 591037 | 552 | LdhA | C H R | Lactate dehydrogenase and related dehydrogenases COG1052 LdhA |
| 627 | 591078 | 592301 | 1498300 | 1497077 | 1749 | r-2 | 591276 | 592218 | 147 | SdaC | E | Amino acid permeases |
| 628 | 592190 | 593191 | 1497188 | 1496187 | 465 | f-2 | 592418 | 593168 | 346 | SIR2 | H | NAD-dependent protein deacetylases |
| 629 | 593214 | 593957 | 1496164 | 1495421 | 836 | f-3 | 593229 | 593949 | 332 | - | R | Predicted hydrolases of the HAD superfamily |
| 630 | 593914 | 594495 | 1495464 | 1494883 | 117 | f-1 | 593923 | 594493 | 259 | - | S | Uncharacterized ACR |
| 631 | 594739 | 594795 | 1494639 | 1494583 | 1364 | r-1 | | | | | | |
| 632 | 595329 | 595610 | 1494049 | 1493768 | 837 | f-3 | 595338 | 595602 | 124 | - | S | Uncharacterized membrane protein |

| 633 | 595427 | 597550 | 1493951 | 1491828 | 466 | f-2 | 595616 | 597509 | 1017 | BisC | C | Anaerobic dehydrogenases |
|---|---|---|---|---|---|---|---|---|---|---|---|---|
| 634 | 597520 | 597798 | 1491858 | 1491580 | 1363 | r-1 | 597547 | 597730 | 30 | PlsX | I | Fatty acid/phospholipid biosynthesis enzyme |
| 635 | 598695 | 599399 | 1490683 | 1489979 | 1748 | r-2 | 598704 | 599283 | 38 | NatB | C | ABC-type Na+ efflux pump |
| 636 | 599396 | 600097 | 1489982 | 1489281 | 2072 | r-3 | 599432 | 599996 | 42 | - | R | ABC-type multidrug transport system |
| 637 | 600094 | 600945 | 1489284 | 1488433 | 1362 | r-1 | 600139 | 600934 | 281 | CcmA | Q | ABC-type multidrug transport system |
| 638 | 600958 | 600999 | 1488420 | 1488379 | 1361 | r-1 | | | | | | |
| 639 | 601388 | 601828 | 1487990 | 1487550 | 467 | f-2 | 601388 | 601826 | 188 | - | R | Predicted nucleic acid-binding protein |
| 640 | 601912 | 602571 | 1487466 | 1486807 | 1360 | r-1 | 602386 | 602563 | 65 | - | R | Predicted DNA binding domain |
| 641 | 602643 | 603974 | 1486735 | 1485404 | 1747 | r-2 | 602643 | 603972 | 762 | TldD | R | Predicted Zn-dependent proteases and their inactivated homologs |
| 642 | 603976 | 605406 | 1485402 | 1483972 | 1359 | r-1 | 603985 | 605404 | 756 | TldD | R | Predicted Zn-dependent proteases and their inactivated homologs |
| 643 | 605506 | 605823 | 1483872 | 1483555 | 118 | f-1 | 605530 | 605815 | 174 | MazG | R | Predicted pyrophosphatase |
| 644 | 605856 | 606749 | 1483522 | 1482629 | 1746 | r-2 | 605859 | 606744 | 522 | - | C | MinD superfamily P-loop ATPase containing an inserted ferredoxin domain |

EP 1 923 464 A1

| 645 | 606746 | 607678 | 1482632 | 1481700 | 2071 | r-3 | 606806 | 607664 | 427 | - | C | MinD superfamily P-loop ATPase containing an inserted ferredoxin domain |
|---|---|---|---|---|---|---|---|---|---|---|---|---|
| 646 | 607678 | 608625 | 1481700 | 1480753 | 1358 | r-1 | 607678 | 608620 | 476 | - | C | Fe-S oxidoreductases |
| 647 | 608720 | 609349 | 1480658 | 1480029 | 468 | f-2 | 608720 | 609347 | 295 | SmtA | Q R | SAM-dependent methyltransferases COG0500 SmtA |
| 648 | 609665 | 611200 | 1479713 | 1478178 | 469 | f-2 | 609749 | 611192 | 473 | PutA | C | NAD-dependent aldehyde dehydrogenases |
| 649 | 611281 | 612924 | 1478097 | 1476454 | 119 | f-1 | 612169 | 612835 | 124 | FecB | P | ABC-type Fe3+-siderophores transport systems |
| 650 | 612921 | 613868 | 1476457 | 1475510 | 838 | f-3 | 612963 | 613839 | 185 | BtuC | PH | ABC-type cobalamin/Fe3+-siderophores transport systems |
| 651 | 613855 | 614616 | 1475523 | 1474762 | 120 | f-1 | 613858 | 614590 | 160 | FepC | PH | ABC-type cobalamin/Fe3+-siderophores transport systems |
| 652 | 614613 | 615374 | 1474765 | 1474004 | 839 | f-3 | 614850 | 614994 | 32 | - | R | Putative homoserine kinase type II (protein kinase fold) |
| 653 | 615379 | 616116 | 1473999 | 1473262 | 121 | f-1 | 615379 | 616108 | 323 | - | S | Uncharacterized ACR |
| 654 | 616117 | 616626 | 1473261 | 1472752 | 1357 | r-1 | 616150 | 616618 | 275 | - | S | Uncharacterized ACR |

| 655 | 616713 | 617375 | 1472665 | 1472003 | 840 | f-3 | 616716 | 617373 | 325 | - | R | Metal-dependent hydrolases of the beta-lactamase superfamily II |
|---|---|---|---|---|---|---|---|---|---|---|---|---|
| 656 | 617430 | 618005 | 1471948 | 1471373 | 1745 | r-2 | | | | | | |
| 657 | 617873 | 619891 | 1471505 | 1469487 | 2070 | r-3 | 617873 | 619829 | 739 | FeoB | P | Ferrous ion uptake system protein FeoB (predicted GTPase) |
| 658 | 619888 | 620115 | 1469490 | 1469263 | 1356 | r-1 | 619888 | 620104 | 55 | FeoA | P | Protein |
| 659 | 620116 | 620346 | 1469262 | 1469032 | 1355 | r-1 | 620197 | 620341 | 55 | FeoA | P | Protein |
| 660 | 620526 | 621581 | 1468852 | 1467797 | 841 | f-3 | 620853 | 621561 | 229 | ModA | P | ABC-type molybdate transport system |
| 661 | 621554 | 622366 | 1467824 | 1467012 | 470 | f-2 | 621668 | 622349 | 238 | CysU | P | ABC-type sulfate/molybdate transport systems |
| 662 | 622338 | 623402 | 1467040 | 1465976 | 842 | f-3 | 622377 | 623397 | 335 | CysA | P | ABC-type sulfate/molybdate transport systems |
| 663 | 623814 | 624353 | 1465564 | 1465025 | 1744 | r-2 | 624078 | 624273 | 32 | ARA1 | R | Aldo/keto reductases |
| 664 | 624301 | 624510 | 1465077 | 1464868 | 1354 | r-1 | 624301 | 624502 | 70 | STE14 | O | Putative protein-S-isoprenylcysteine methyltransferase |
| 665 | 624735 | 625205 | 1464643 | 1464173 | 1743 | r-2 | 625065 | 625146 | 28 | GspD | N | General secretory pathway protein D |
| 666 | 625223 | 625891 | 1464155 | 1463487 | 471 | f-2 | 625268 | 625595 | 146 | - | S | Uncharacterized ACR |
| 667 | 625916 | 626170 | 1463462 | 1463208 | 472 | f-2 | | | | | | |

EP 1 923 464 A1

| 668 | 626202 | 626936 | 1463176 | 1462442 | 1742 | r-2 | 626232 | 626790 | 55 | - | R | ABC-type multidrug transport system |
|---|---|---|---|---|---|---|---|---|---|---|---|---|
| 669 | 626909 | 627853 | 1462469 | 1461525 | 2069 | r-3 | 626918 | 627773 | 206 | CcmA | Q | ABC-type multidrug transport system |
| 670 | 627832 | 628989 | 1461546 | 1460389 | 1353 | r-1 | 627964 | 628603 | 44 | - | S | Uncharacterized proteins of WD40-like repeat family |
| 671 | 629061 | 629687 | 1460317 | 1459691 | 1741 | r-2 | 629088 | 629673 | 198 | SmtA | Q R | SAM-dependent methyltransferases COG0500 SmtA |
| 672 | 629684 | 631024 | 1459694 | 1458354 | 2068 | r-3 | 629684 | 631022 | 771 | - | R | Predicted membrane components of an uncharacterized iron-regulated ABC-type transporter SufB |
| 673 | 631021 | 631839 | 1458357 | 1457539 | 1352 | r-1 | 631099 | 631822 | 386 | - | R | Iron-regulated ABC transporter ATPase subunit SufC |
| 674 | 631871 | 632350 | 1457507 | 1457028 | 473 | f-2 | 631886 | 632231 | 196 | - | S | Uncharacterized ACR |
| 675 | 632430 | 632630 | 1456948 | 1456748 | 843 | f-3 | 632430 | 632625 | 46 | - | S | Uncharacterized ArCR |
| 676 | 632617 | 633099 | 1456761 | 1456279 | 122 | f-1 | 632617 | 633070 | 203 | - | R | Predicted nucleic acid-binding protein |
| 677 | 633112 | 633933 | 1456266 | 1455445 | 123 | f-1 | 633121 | 633931 | 381 | - | R | Metal-dependent hydrolases of the beta-lactamase superfamily II |

| 678 | 633964 | 634764 | 1455414 | 1454614 | 124 | f-1 | 633973 | 634762 | 469 | FabG | Q R | Dehydrogenases with different specificities (related to short-chain alcohol dehydrogenases) COG1028 FabG |
|---|---|---|---|---|---|---|---|---|---|---|---|---|
| 679 | 634815 | 635330 | 1454563 | 1454048 | 1740 | r-2 | 634893 | 635016 | 30 | DnaX | L | DNA polymerase III |
| 680 | 635934 | 636071 | 1453444 | 1453307 | 1739 | r-2 | 635982 | 636060 | 27 | - | C | Uncharacterized Fe-S protein |
| 681 | 637143 | 637451 | 1452235 | 1451927 | 844 | f-3 | 637329 | 637425 | 29 | ArtI | E | ABC-type amino acid transport system |
| 682 | 637487 | 638062 | 1451891 | 1451316 | 474 | f-2 | 637520 | 638036 | 145 | - | S | Predicted membrane protein |
| 683 | 638134 | 639000 | 1451244 | 1450378 | 1351 | r-1 | 638206 | 638998 | 409 | - | S | Predicted membrane proteins |
| 684 | 639553 | 639651 | 1449825 | 1449727 | 125 | f-1 | | | | | | |
| 685 | 639626 | 640396 | 1449752 | 1448982 | 2067 | r-3 | 639641 | 640298 | 219 | CbiQ | P | ABC-type cobalt transport system |
| 686 | 640393 | 641181 | 1448985 | 1448197 | 1350 | r-1 | 640393 | 641167 | 299 | CbiO | P | ABC-type cobalt transport system |
| 687 | 641204 | 641923 | 1448174 | 1447455 | 2066 | r-3 | 641438 | 641909 | 84 | BirA | H | Biotin-(acetyl-CoA carboxylase) ligase |
| 688 | 641972 | 642490 | 1447406 | 1446888 | 475 | f-2 | 641981 | 642464 | 146 | BioY | R | Uncharacterized ACR |
| 689 | 642511 | 643098 | 1446867 | 1446280 | 1349 | r-1 | 642511 | 643081 | 162 | MobA | H | Molybdopterin-guanine dinucleotide biosynthesis protein A |
| 690 | 643209 | 643670 | 1446169 | 1445708 | 845 | f-3 | 643221 | 643398 | 31 | HHT1 | L | Histones H3 and H4 |

EP 1 923 464 A1

151

| | | | | | | | | | | | | |
|---|---|---|---|---|---|---|---|---|---|---|---|---|
| 691 | 644598 | 646496 | 1444780 | 1442882 | 1738 | r-2 | 644598 | 646488 | 1164 | DAP2 | E | Dipeptidyl aminopeptidases/acylaminoacyl-peptidases |
| 692 | 647573 | 650017 | 1441805 | 1439361 | 476 | f-2 | 647582 | 650006 | 1260 | - | R | Predicted P-loop ATPase fused to an acetyltransferase |
| 693 | 650078 | 650584 | 1439300 | 1438794 | 477 | f-2 | 650099 | 650570 | 241 | - | S | Uncharacterized ACR |
| 694 | 650587 | 651087 | 1438791 | 1438291 | 126 | f-1 | 650656 | 651073 | 236 | - | S | Uncharacterized ACR |
| 695 | 651198 | 652340 | 1438180 | 1437038 | 846 | f-3 | 651285 | 652236 | 390 | TbpA | H | ABC-type iron/thiamine transport systems |
| 696 | 652343 | 653548 | 1437035 | 1435830 | 2065 | r-3 | 652400 | 653513 | 272 | SsnA | FR | Cytosine deaminase and related metal-dependent hydrolases COG0402 SsnA |
| 697 | 653784 | 655079 | 1435594 | 1434299 | 847 | f-3 | 653784 | 655065 | 724 | AsnS | J | Aspartyl/asparaginyl-tRNA synthetases |
| 698 | 655937 | 657688 | 1433441 | 1431690 | 2064 | r-3 | 655958 | 657119 | 612 | Tgt | J | Queuine/archaeosine tRNA-ribosyltransferase |
| 699 | 657722 | 658642 | 1431656 | 1430736 | 2063 | r-3 | 657722 | 658622 | 210 | PitA | P | Phosphate/sulphate permeases |
| 700 | 658773 | 659825 | 1430605 | 1429553 | 1737 | r-2 | 658797 | 659823 | 362 | - | M | Glycosyltransferases |
| 701 | 659850 | 660155 | 1429528 | 1429223 | 1736 | r-2 | 659850 | 660120 | 59 | - | R | Predicted acetyltransferase |
| 702 | 660246 | 664418 | 1429132 | 1424960 | 848 | f-3 | 662859 | 664401 | 827 | Lhr | R | Lhr-like helicases |

| 703 | 664498 | 665586 | 1424880 | 1423792 | 127 | f-1 | 664582 | 665584 | 608 | GapA | G | Glyceraldehyde-3-phosphate dehydrogenase/erythrose-4-phosphate dehydrogenase |
|---|---|---|---|---|---|---|---|---|---|---|---|---|
| 704 | 665627 | 665995 | 1423751 | 1423383 | 478 | f-2 | 665753 | 665900 | 28 | ThrA | E | Homoserine dehydrogenase |
| 705 | 666332 | 666616 | 1423046 | 1422762 | 2062 | r-3 | 666341 | 666608 | 120 | - | S | Uncharacterized ACR |
| 706 | 666618 | 667169 | 1422760 | 1422209 | 1735 | r-2 | 666663 | 667155 | 258 | - | S | Uncharacterized ACR |
| 707 | 667123 | 667176 | 1422255 | 1422202 | 128 | f-1 | | | | | | |
| 708 | 667218 | 667724 | 1422160 | 1421654 | 1734 | r-2 | 667332 | 667629 | 53 | - | K | Predicted transcriptional regulators |
| 709 | 667824 | 669488 | 1421554 | 1419890 | 849 | f-3 | 667914 | 668805 | 36 | - | R | Predicted drug exporters of the RND superfamily |
| 710 | 669735 | 671918 | 1419643 | 1417460 | 850 | f-3 | 670269 | 671868 | 169 | - | R | Predicted drug exporters of the RND superfamily |
| 711 | 673707 | 673985 | 1415671 | 1415393 | 851 | f-3 | 673707 | 673926 | 32 | - | S | Uncharacterized BCR |
| 712 | 674033 | 674911 | 1415345 | 1414467 | 479 | f-2 | 674039 | 674858 | 79 | - | R | Predicted permeases |
| 713 | 674957 | 675970 | 1414421 | 1413408 | 480 | f-2 | 674957 | 675962 | 570 | FrvX | G | Cellulase M and related proteins |
| 714 | 676425 | 677294 | 1412953 | 1412084 | 852 | f-3 | 676440 | 677232 | 177 | - | R | Predicted ATPase of the AAA superfamily |
| 715 | 677302 | 678150 | 1412076 | 1411228 | 1348 | r-1 | 677314 | 678145 | 374 | XerC | L | Integrase |
| 716 | 678143 | 679063 | 1411235 | 1410315 | 2061 | r-3 | 678329 | 678989 | 45 | - | K | Predicted transcriptional regulators |

| | | | | | | | | | | | | |
|---|---|---|---|---|---|---|---|---|---|---|---|---|
| 717 | 679100 | 679813 | 1410278 | 1409565 | 2060 | r-3 | 679127 | 679811 | 161 | SfsA | G | Sugar fermentation stimulation protein (uncharacterized) |
| 718 | 679850 | 679924 | 1409528 | 1409454 | 481 | f-2 | | | | | | |
| 719 | 680156 | 680470 | 1409222 | 1408908 | 482 | f-2 | 680231 | 680285 | 28 | - | R | Predicted DNA-binding proteins with PD1-like DNA-binding motif |
| 720 | 680606 | 681754 | 1408772 | 1407624 | 483 | f-2 | 680708 | 681752 | 617 | FrvX | G | Cellulase M and related proteins |
| 721 | 682401 | 682496 | 1406977 | 1406882 | 853 | f-3 | | | | | | |
| 722 | 682446 | 682799 | 1406932 | 1406579 | 1733 | r-2 | 682512 | 682641 | 28 | - | S | Uncharacterized ACR |
| 723 | 682717 | 684711 | 1406661 | 1404667 | 129 | f-1 | 682804 | 684694 | 883 | DinG | L | Rad3-related DNA helicases |
| 724 | 684698 | 685174 | 1404680 | 1404204 | 2059 | r-3 | 684719 | 684902 | 33 | - | L | Adenine-specific DNA methylase |
| 725 | 686253 | 686873 | 1403125 | 1402505 | 1732 | r-2 | 686274 | 686841 | 135 | GlpG | R | Uncharacterized membrane protein (homolog of Drosophila rhomboid) |
| 726 | 686863 | 687633 | 1402515 | 1401745 | 1347 | r-1 | 686875 | 687622 | 273 | SuhB | G | Archaeal fructose-1 |
| 727 | 687638 | 688447 | 1401740 | 1400931 | 2058 | r-3 | 687644 | 688424 | 265 | - | S | Predicted membrane proteins |
| 728 | 688516 | 689571 | 1400862 | 1399807 | 130 | f-1 | 688525 | 689569 | 528 | GldA | C | Glycerol dehydrogenase and related enzymes |
| 729 | 689568 | 690029 | 1399810 | 1399349 | 854 | f-3 | 689601 | 690024 | 210 | - | S | Uncharacterized ArCR |
| 730 | 690316 | 690513 | 1399062 | 1398865 | 1346 | r-1 | 690334 | 690502 | 27 | AceF | C | Dihydrolipoamide acyltransferases |

| No. | Start | End | Start2 | End2 | Frame | ORF | Start3 | End3 | Length | Gene | COG | Description |
|---|---|---|---|---|---|---|---|---|---|---|---|---|
| 731 | 690550 | 691353 | 1398828 | 1398025 | r-1 | 1345 | 690550 | 691351 | 381 | - | S | Uncharacterized ACR |
| 732 | 691387 | 692820 | 1397991 | 1396558 | r-1 | 1344 | 691462 | 691798 | 34 | SppA | N | Periplasmic serine proteases (ClpP class) COG0616 SppA |
| 733 | 692817 | 694928 | 1396561 | 1394450 | r-2 | 1731 | 694260 | 694908 | 170 | McrB | L | GTPase subunit of restriction endonuclease |
| 734 | 694986 | 695405 | 1394392 | 1393973 | r-2 | 1730 | 694986 | 695361 | 160 | - | S | Uncharacterized ArCR |
| 735 | 695410 | 696654 | 1393968 | 1392724 | r-1 | 1343 | 695410 | 696643 | 487 | - | S | Uncharacterized ArCR |
| 736 | 696651 | 697808 | 1392727 | 1391570 | r-2 | 1729 | 696663 | 697806 | 699 | - | L | DNA topoisomerase VI |
| 737 | 697801 | 699510 | 1391577 | 1389868 | r-1 | 1342 | 697807 | 699451 | 866 | - | L | DNA topoisomerase VI |
| 738 | 699507 | 700274 | 1389871 | 1389104 | r-2 | 1728 | 699561 | 700224 | 275 | - | R | Predicted RNA-binding protein (contains KH domains) |
| 739 | 700228 | 701004 | 1389150 | 1388374 | r-1 | 1341 | 700237 | 700993 | 413 | RIO1 | T | Predicted serine/threonine protein kinases |
| 740 | 701037 | 701399 | 1388341 | 1387979 | r-2 | 1727 | 701061 | 701394 | 198 | InfA | J | Translation initiation factor IF-1 |
| 741 | 701550 | 702359 | 1387828 | 1387019 | f-3 | 855 | 701577 | 702336 | 277 | ZnuC | P | ABC-type Mn/Zn transport systems |
| 742 | 702356 | 703177 | 1387022 | 1386201 | f-2 | 484 | 702356 | 703175 | 241 | ZnuB | P | ABC-type Mn2+/Zn2+ transport systems |
| 743 | 703152 | 703868 | 1386226 | 1385510 | f-3 | 856 | 703182 | 703782 | 262 | RnhB | L | Ribonuclease HII |
| 744 | 703837 | 705249 | 1385541 | 1384129 | r-1 | 1340 | 704299 | 704578 | 51 | PMT1 | O | Dolichyl-phosphate-mannose--protein O-mannosyl transferase PMT1 |

| 745 | 705309 | 706460 | 1384069 | 1382918 | 857 | f-3 | 705321 | 706449 | 537 | SmtA | Q R | SAM-dependent methyltransferases COG0500 SmtA |
|---|---|---|---|---|---|---|---|---|---|---|---|---|
| 746 | 706455 | 706655 | 1382923 | 1382723 | 1726 | r-2 | 706455 | 706650 | 29 | AroB | E | 3-dehydroquinate synthetase |
| 747 | 706739 | 708556 | 1382639 | 1380822 | 485 | f-2 | 706748 | 708554 | 805 | GlmS | M | Glucosamine 6-phosphate synthetase |
| 748 | 708558 | 711569 | 1380820 | 1377809 | 858 | f-3 | 708582 | 711462 | 590 | - | R | Uncharacterized membrane protein |
| 749 | 711859 | 712440 | 1377519 | 1376938 | 131 | f-1 | 711985 | 712315 | 30 | RpoE | K | DNA-directed RNA polymerase specialized sigma subunits |
| 750 | 712445 | 713191 | 1376933 | 1376187 | 2057 | r-3 | 712517 | 713177 | 349 | Adk | F | Adenylate kinase and related kinases |
| 751 | 713142 | 713633 | 1376236 | 1375745 | 859 | f-3 | 713280 | 713592 | 43 | Smc | D | Chromosome segregation ATPases |
| 752 | 713693 | 714955 | 1375685 | 1374423 | 2056 | r-3 | 713726 | 714947 | 684 | - | C | Uncharacterized flavoproteins |
| 753 | 715024 | 715470 | 1374354 | 1373908 | 1339 | r-1 | 715024 | 715438 | 110 | AhpC | O | Peroxiredoxin |
| 754 | 715543 | 716427 | 1373835 | 1372951 | 1338 | r-1 | 715597 | 716419 | 370 | PorB | C | Pyruvate:ferredoxin oxidoreductase and related 2-oxoacid:ferredoxin oxidoreductases |

| 755 | 716424 | 718136 | 1372954 | 1371242 | 1725 | r-2 | 717030 | 718128 | 453 | PorA | C | Pyruvate:ferredoxin oxidoreductase and related 2-oxoacid:ferredoxin oxidoreductases |
|---|---|---|---|---|---|---|---|---|---|---|---|---|
| 756 | 718317 | 719339 | 1371061 | 1370039 | 860 | f-3 | 718353 | 718866 | 213 | MsrA | O | Peptide methionine sulfoxide reductase |
| 757 | 719507 | 719788 | 1369871 | 1369590 | 486 | f-2 | 719567 | 719732 | 33 | AvtA | E | PLP-dependent aminotransferases |
| 758 | 719790 | 720593 | 1369588 | 1368785 | 1724 | r-2 | 719973 | 720528 | 32 | XynB | G | Beta-xylosidase |
| 759 | 720689 | 721426 | 1368689 | 1367952 | 2055 | r-3 | 720704 | 720962 | 35 | | | |
| 760 | 721789 | 722304 | 1367589 | 1367074 | 132 | f-1 | 721870 | 722299 | 70 | - | S | Uncharacterized ACR |
| 761 | 722344 | 722481 | 1367034 | 1366897 | 1337 | r-1 | 722359 | 722470 | 32 | VacB | K | Exoribonucleases |
| 762 | 722592 | 723116 | 1366786 | 1366262 | 861 | f-3 | 722595 | 723087 | 77 | - | S | Uncharacterized ACR |
| 763 | 723142 | 724314 | 1366236 | 1365064 | 1336 | r-1 | 723160 | 724303 | 528 | | | |
| 764 | 724419 | 725573 | 1364959 | 1363805 | 1723 | r-2 | 724488 | 725553 | 393 | HcaD | R | Uncharacterized NAD(FAD)-dependent dehydrogenases |
| 765 | 725704 | 726249 | 1363674 | 1363129 | 133 | f-1 | 725713 | 726238 | 271 | - | S | Predicted membrane protein |
| 766 | 726458 | 726643 | 1362920 | 1362735 | 487 | f-2 | 726467 | 726614 | 69 | RAD5 5 | T | RecA-superfamily ATPases implicated in signal transduction |
| 767 | 728745 | 728798 | 1360633 | 1360580 | 862 | f-3 | | | | | | |
| 768 | 729082 | 729786 | 1360296 | 1359592 | 1335 | r-1 | 729259 | 729748 | 167 | Lrp | K | Transcriptional regulators |

| 769 | 729844 | 730989 | 1359534 | 1358389 | 134 | f-1 | 729859 | 730951 | 395 | PurK | F | Phosphoribosylaminoimidazole carboxylase (NCAIR synthetase) |
|---|---|---|---|---|---|---|---|---|---|---|---|---|
| 770 | 730961 | 731485 | 1358417 | 1357893 | 488 | f-2 | 730961 | 731462 | 193 | PurE | F | Phosphoribosylcarboxyaminoimid azole (NCAIR) mutase |
| 771 | 731586 | 733985 | 1357792 | 1355393 | 863 | f-3 | 731799 | 733923 | 812 | ZntA | P | Cation transport ATPases |
| 772 | 734016 | 734336 | 1355362 | 1355042 | 864 | f-3 | 734046 | 734259 | 50 | TrxA | O C | Thiol-disulfide isomerase and thioredoxins COG0526 TrxA |
| 773 | 734349 | 734939 | 1355029 | 1354439 | 1722 | r-2 | 734349 | 734931 | 238 | NfnB | C | Nitroreductase |
| 774 | 735215 | 735760 | 1354163 | 1353618 | 489 | f-2 | 735215 | 735749 | 288 | NfnB | C | Nitroreductase |
| 775 | 735762 | 735941 | 1353616 | 1353437 | 865 | f-3 | 735798 | 735924 | 29 | KefB | P | Kef-type K+ transport systems |
| 776 | 735965 | 737146 | 1353413 | 1352232 | 2054 | r-3 | 736043 | 737078 | 368 | ACR3 | P | Arsenite efflux pump ACR3 and related permeases |
| 777 | 737210 | 737683 | 1352168 | 1351695 | 490 | f-2 | 737234 | 737618 | 110 | Wzb | T | Protein-tyrosine-phosphatase |
| 778 | 737822 | 739696 | 1351556 | 1349682 | 2053 | r-3 | 737828 | 739679 | 1055 | - | C | Aldehyde:ferredoxin oxidoreductase |
| 779 | 739687 | 740523 | 1349691 | 1348855 | 1334 | r-1 | 739711 | 740518 | 459 | ARA1 | R | Aldo/keto reductases |
| 780 | 740584 | 741294 | 1348794 | 1348084 | 135 | f-1 | 740716 | 741283 | 283 | - | S | Uncharacterized ACR |
| 781 | 741329 | 741541 | 1348049 | 1347837 | 491 | f-2 | 741419 | 741518 | 27 | - | C | Uncharacterized conserved protein containing a ferredoxin-like domain |

EP 1 923 464 A1

| | | | | | | | | | | | |
|---|---|---|---|---|---|---|---|---|---|---|---|
| 782 | 741920 | 742084 | 1347458 | 1347294 | 492 | f-2 | 741944 | 742076 | 28 | SdrC | T | Predicted secreted protein containing a PDZ domain |
| 783 | 742684 | 743376 | 1346694 | 1346002 | 136 | f-1 | 742684 | 743185 | 259 | - | L | Predicted transposases |
| 784 | 743424 | 743609 | 1345954 | 1345769 | 866 | f-3 | 743481 | 743586 | 28 | VapC | R | Predicted nucleic acid-binding protein |
| 785 | 743587 | 744603 | 1345791 | 1344775 | 1333 | r-1 | 743596 | 744598 | 558 | CobT | H | NaMN:DMB phosphoribosyltransferase |
| 786 | 744560 | 745372 | 1344818 | 1344006 | 493 | f-2 | 744698 | 745208 | 70 | PflA | O | Pyruvate-formate lyase-activating enzyme |
| 787 | 745369 | 746826 | 1344009 | 1342552 | 137 | f-1 | 745381 | 746665 | 377 | CobQ | H | Cobyric acid synthase |
| 788 | 746823 | 747761 | 1342555 | 1341617 | 1721 | r-2 | 746862 | 747171 | 37 | SurA | O | Parvulin-like peptidyl-prolyl isomerase |
| 789 | 747766 | 748353 | 1341612 | 1341025 | 1332 | r-1 | 747778 | 748315 | 251 | - | H | GTP:adenosylcobinamide-phosph ate guanylyltransferase |
| 790 | 748338 | 749033 | 1341040 | 1340345 | 1720 | r-2 | 748338 | 749013 | 272 | CobS | H | Cobalamin-5-phosphate synthase (Cobalamin synthase) |
| 791 | 749030 | 749443 | 1340348 | 1339935 | 2052 | r-3 | 749042 | 749438 | 201 | PgpA | I | Phosphatidylglycerophosphatase A |
| 792 | 749440 | 749877 | 1339938 | 1339501 | 1331 | r-1 | 749548 | 749629 | 28 | - | S | Uncharacterized ACR |
| 793 | 750208 | 750714 | 1339170 | 1338664 | 1330 | r-1 | 750211 | 750661 | 238 | - | R | Predicted ATPases of PP-loop superfamily |

| 794 | 751954 | 752967 | 1337424 | 1336411 | 138 | f-1 | 751999 | 752965 | 486 | HisC | E | Histidinol-phosphate aminotransferase/Tyrosine aminotransferase |
|---|---|---|---|---|---|---|---|---|---|---|---|---|
| 795 | 753046 | 754110 | 1336332 | 1335268 | 139 | f-1 | 753067 | 754081 | 386 | FecB | P | ABC-type Fe3+-siderophores transport systems |
| 796 | 754166 | 755410 | 1335212 | 1333968 | 2051 | r-3 | 754226 | 755408 | 708 | - | G | Predicted phosphoglycerate mutase |
| 797 | 755496 | 756431 | 1333882 | 1332947 | 867 | f-3 | 755586 | 756408 | 195 | ECM2 7 | P | Ca2+/Na+ antiporter |
| 798 | 756477 | 756968 | 1332901 | 1332410 | 868 | f-3 | 756477 | 756957 | 304 | Hit | FG R | Diadenosine tetraphosphate (Ap4A) hydrolase and other HIT family hydrolases COG0537 Hit |
| 799 | 756958 | 757629 | 1332420 | 1331749 | 1329 | r-1 | 756994 | 757156 | 32 | - | R | Predicted amidohydrolase |
| 800 | 757712 | 758458 | 1331666 | 1330920 | 2050 | r-3 | 757733 | 758453 | 417 | THY1 | F | Predicted alternative thymidylate synthase |
| 801 | 758689 | 759645 | 1330689 | 1329733 | 140 | f-1 | 758698 | 759640 | 549 | ArgF | E | Ornithine carbamoyltransferase |
| 802 | 759762 | 760691 | 1329616 | 1328687 | 869 | f-3 | 759762 | 760689 | 549 | Sun | J | tRNA and rRNA cytosine-C5-methylases |
| 803 | 760688 | 761674 | 1328690 | 1327704 | 2049 | r-3 | 760724 | 761135 | 33 | HslU | O | ATP-dependent protease |
| 804 | 762327 | 763418 | 1327051 | 1325960 | 870 | f-3 | 762327 | 763383 | 518 | LYS9 | E | Saccharopine dehydrogenase and related proteins |

| 805 | 763396 | 764058 | 1325982 | 1325320 | 141 | f-1 | 763399 | 764041 | 323 | Mra1 | S | Uncharacterized ACR |
|---|---|---|---|---|---|---|---|---|---|---|---|---|
| 806 | 765200 | 765316 | 1324178 | 1324062 | 2048 | r-3 | | | | | | |
| 807 | 765637 | 766047 | 1323741 | 1323331 | 142 | f-1 | 765637 | 766045 | 238 | Efp | J | Translation elongation factor P/translation initiation factor eIF-5A |
| 808 | 766138 | 766683 | 1323240 | 1322695 | 143 | f-1 | 766195 | 766504 | 34 | - | S | Uncharacterized ACR |
| 809 | 766685 | 767974 | 1322693 | 1321404 | 494 | f-2 | 766703 | 767969 | 542 | ArsB | P | Na+/H+ antiporter NhaD and related arsenite permeases |
| 810 | 767976 | 768434 | 1321402 | 1320944 | 871 | f-3 | 767985 | 768432 | 223 | UspA | T | Universal stress protein UspA and related nucleotide-binding proteins |
| 811 | 768477 | 769343 | 1320901 | 1320035 | 872 | f-3 | 768486 | 769323 | 387 | SpeB | E | Arginase/agmatinase/formimiono glutamate hydrolase |
| 812 | 769459 | 769962 | 1319919 | 1319416 | 144 | f-1 | 769459 | 769954 | 190 | - | R | CBS domains |
| 813 | 769950 | 771269 | 1319428 | 1318109 | 873 | f-3 | 770010 | 771258 | 553 | KefB | P | Kef-type K+ transport systems |
| 814 | 771283 | 771807 | 1318095 | 1317571 | 1328 | r-1 | 771334 | 771469 | 31 | ZntA | P | Cation transport ATPases |
| 815 | 771820 | 773541 | 1317558 | 1315837 | 145 | f-1 | 772069 | 773122 | 177 | EriC | P | Chloride channel protein EriC |
| 816 | 773543 | 774817 | 1315835 | 1314561 | 495 | f-2 | 773552 | 774800 | 647 | - | S | Uncharacterized ACR |
| 817 | 774838 | 775089 | 1314540 | 1314289 | 146 | f-1 | 774847 | 775066 | 52 | AbrB | K | Regulators of stationary/sporulation gene expression |

EP 1 923 464 A1

| 818 | 775493 | 776422 | 1313885 | 1312956 | 496 | f-2 | 775493 | 776399 | 327 | ThiL | H | Thiamine monophosphate kinase |
|---|---|---|---|---|---|---|---|---|---|---|---|---|
| 819 | 776480 | 777643 | 1312898 | 1311735 | 497 | f-2 | 776480 | 777614 | 382 | RfaG | M | Predicted glycosyltransferases |
| 820 | 778176 | 778346 | 1311202 | 1311032 | 874 | f-3 | 778176 | 778329 | 62 | CDA1 | G | Predicted xylanase/chitin deacetylase |
| 821 | 778362 | 779411 | 1311016 | 1309967 | 875 | f-3 | 778362 | 779409 | 622 | PflA | O | Pyruvate-formate lyase-activating enzyme |
| 822 | 779336 | 780247 | 1310042 | 1309131 | 498 | f-2 | 779384 | 779564 | 32 | - | R | Uncharacterized protein |
| 823 | 780438 | 782276 | 1308940 | 1307102 | 876 | f-3 | 782085 | 782205 | 34 | - | L | Archaea-specific RecJ-like exonuclease |
| 824 | 782329 | 783108 | 1307049 | 1306270 | 147 | f-1 | 782773 | 782986 | 29 | Ggt | E | Gamma-glutamyltranspeptidase |
| 825 | 783098 | 784927 | 1306280 | 1304451 | 2047 | r-3 | 783182 | 784919 | 922 | - | C | Uncharacterized Fe-S oxidoreductases |
| 826 | 785382 | 786104 | 1303996 | 1303274 | 1719 | r-2 | 785382 | 786081 | 310 | KsgA | J | Dimethyladenosine transferase (rRNA methylation) |
| 827 | 786218 | 786838 | 1303160 | 1302540 | 2046 | r-3 | 786218 | 786833 | 337 | - | J | Predicted RNA-binding protein |
| 828 | 786930 | 787286 | 1302448 | 1302092 | 1718 | r-2 | 786936 | 787230 | 135 | - | S | Uncharacterized ArCR |
| 829 | 787283 | 787609 | 1302095 | 1301769 | 2045 | r-3 | 787313 | 787604 | 189 | RPL21 A | J | Ribosomal protein L21E |
| 830 | 787749 | 788930 | 1301629 | 1300448 | 1717 | r-2 | 787749 | 788916 | 492 | - | J | Predicted pseudouridylate synthase |
| 831 | 788975 | 789268 | 1300403 | 1300110 | 499 | f-2 | 788975 | 789266 | 138 | - | S | Uncharacterized ArCR |

EP 1 923 464 A1

| 832 | 789317 | 789460 | 1300061 | 1299918 | 2044 | r-3 | 789350 | 789440 | 27 | Rfe | M | UDP-N-acetylmuramyl pentapeptide phosphotransferase/UDP-N-acetylglucosamine-1-phosphate transferase |
|---|---|---|---|---|---|---|---|---|---|---|---|---|
| 833 | 789852 | 790022 | 1299526 | 1299356 | 1716 | r-2 | 789855 | 789993 | 56 | Nfi | L | Deoxyinosine 3'endonuclease (endonuclease V) |
| 834 | 790438 | 791058 | 1298940 | 1298320 | 1327 | r-1 | 790438 | 791038 | 264 | - | L | Translin (RNA-binding protein) |
| 835 | 790672 | 790737 | 1298706 | 1298641 | 148 | f-1 | | | | | | |
| 836 | 791117 | 792469 | 1298261 | 1296909 | 500 | f-2 | 791156 | 792467 | 683 | AnsB | EJ | L-asparaginase/archaeal Glu-tRNAGln amidotransferase subunit D COG0252 AnsB |
| 837 | 792505 | 792675 | 1296873 | 1296703 | 149 | f-1 | 792505 | 792610 | 34 | - | S | Uncharacterized ArCR |
| 838 | 792665 | 793114 | 1296713 | 1296264 | 501 | f-2 | 792665 | 793079 | 77 | - | R | Predicted nucleic acid-binding protein |
| 839 | 793111 | 795000 | 1296267 | 1294378 | 150 | f-1 | 793111 | 794998 | 997 | GatE | J | Archaeal Glu-tRNAGln amidotransferase subunit E (contains GAD domain) |
| 840 | 795038 | 795544 | 1294340 | 1293834 | 502 | f-2 | 795356 | 795491 | 34 | FtsW | D | Bacterial cell division membrane protein |
| 841 | 796310 | 797536 | 1293068 | 1291842 | 2043 | r-3 | 796310 | 797534 | 710 | HMG1 | I | Hydroxymethylglutaryl-CoA |

| | | | | | | | | | | | |
|---|---|---|---|---|---|---|---|---|---|---|---|
| | | | | | | | | | | | reductase |
| 842 | 797552 | 798316 | 1291826 | 1291062 | 2042 | r-3 | 797570 | 798311 | 335 | - | D | ATPases involved in chromosome partitioning |
| 843 | 798473 | 799534 | 1290905 | 1289844 | 503 | f-2 | 798482 | 799517 | 596 | Tdh | ER | Threonine dehydrogenase and related Zn-dependent dehydrogenases COG1063 Tdh |
| 844 | 799610 | 799858 | 1289768 | 1289520 | 504 | f-2 | 799625 | 799838 | 55 | - | S | Uncharacterized ACR |
| 845 | 799848 | 800327 | 1289530 | 1289051 | 877 | f-3 | 799848 | 800325 | 91 | - | R | Predicted nucleic acid-binding protein |
| 846 | 800324 | 800425 | 1289054 | 1288953 | 2041 | r-3 | 800324 | 800402 | 26 | Uup | R | ATPase components of ABC transporters with duplicated ATPase domains |
| 847 | 800450 | 800518 | 1288928 | 1288860 | 2040 | r-3 | | | | | | |
| 848 | 800919 | 802424 | 1288459 | 1286954 | 878 | f-3 | 800919 | 802422 | 753 | PheS | J | Phenylalanyl-tRNA synthetase alpha subunit |
| 849 | 802436 | 802672 | 1286942 | 1286706 | 505 | f-2 | 802478 | 802649 | 32 | - | R | Predicted ATPase of the AAA superfamily |
| 850 | 802669 | 802890 | 1286709 | 1286488 | 151 | f-1 | 802816 | 802876 | 26 | - | R | Predicted RNA-binding protein (contains KH domains) |
| 851 | 802887 | 803297 | 1286491 | 1286081 | 879 | f-3 | 802887 | 803277 | 45 | - | R | Predicted nucleic acid-binding protein |

164

| 852 | 803294 | 805027 | 1286084 | 1284351 | 506 | f-2 | 803303 | 805010 | 933 | PheT | J | Phenylalanyl-tRNA synthetase beta subunit |
|---|---|---|---|---|---|---|---|---|---|---|---|---|
| 853 | 805220 | 806068 | 1284158 | 1283310 | 507 | f-2 | 805265 | 806051 | 266 | TruA | J | Pseudouridylate synthase (tRNA psi55) |
| 854 | 806024 | 807415 | 1283354 | 1281963 | 2039 | r-3 | 806030 | 807359 | 722 | SSL2 | L | DNA or RNA helicases of superfamily II |
| 855 | 807366 | 808745 | 1282012 | 1280633 | 880 | f-3 | 807480 | 808743 | 673 | UbiD | H | 3-polyprenyl-4-hydroxybenzoate decarboxylase and related decarboxylases |
| 856 | 808746 | 809576 | 1280632 | 1279802 | 1715 | r-2 | 808875 | 809043 | 30 | RimI | R | Acetyltransferases |
| 857 | 810847 | 811266 | 1278531 | 1278112 | 1326 | r-1 | 810856 | 811252 | 127 | - | L | Predicted transposase |
| 858 | 811367 | 811606 | 1278011 | 1277772 | 508 | f-2 | 811391 | 811532 | 30 | Hfq | R | Uncharacterized ACR |
| 859 | 811608 | 812351 | 1277770 | 1277027 | 881 | f-3 | 811620 | 812340 | 392 | MobB | H | Molybdopterin-guanine dinucleotide biosynthesis protein |
| 860 | 812635 | 813648 | 1276743 | 1275730 | 152 | f-1 | 812755 | 813613 | 280 | - | R | Predicted periplasmic binding protein |
| 861 | 813652 | 814113 | 1275726 | 1275265 | 153 | f-1 | 813730 | 813889 | 32 | UvrB | L | Helicase subunit of the DNA excision repair complex |
| 862 | 814077 | 816419 | 1275301 | 1272959 | 882 | f-3 | 814140 | 816300 | 432 | - | S | Integral membrane protein |
| 863 | 816501 | 816650 | 1272877 | 1272728 | 883 | f-3 | | | | | | |
| 864 | 816754 | 817728 | 1272624 | 1271650 | 154 | f-1 | 816754 | 817711 | 403 | - | R | Predicted archaeal sugar kinases |

EP 1 923 464 A1

| No. | Description | Category | Gene | | | | Strand | | | | | |
|---|---|---|---|---|---|---|---|---|---|---|---|---|
| 865 | Dehydrogenases with different specificities (related to short-chain alcohol dehydrogenases) COG1028 FabG | Q R | FabG | 33 | 817962 | 817746 | f-3 | 884 | 1270859 | 1271653 | 818519 | 817725 |
| 866 | ABC-type transport system involved in multi-copper enzyme maturation | R | NosY | 49 | 819301 | 818650 | f-1 | 155 | 1269910 | 1270755 | 819468 | 818623 |
| 867 | ABC-type multidrug transport system | Q | CcmA | 317 | 820381 | 819475 | f-1 | 156 | 1268983 | 1269903 | 820395 | 819475 |
| 868 | Acyl-CoA synthetase (NDP forming) | C | - | 412 | 821160 | 820458 | r-2 | 1714 | 1268198 | 1268968 | 821180 | 820410 |
| 869 | Acyl-CoA synthetase (NDP forming) | C | - | 724 | 822553 | 821146 | r-1 | 1325 | 1266808 | 1268232 | 822570 | 821146 |
| 870 | Predicted nucleotidyltransferase | R | - | 395 | 823500 | 822810 | r-2 | 1713 | 1265864 | 1266568 | 823514 | 822810 |
| 871 | Aldo/keto reductases | R | ARA1 | 29 | 823947 | 823815 | f-3 | 885 | 1265357 | 1265779 | 824021 | 823599 |
| 872 | TPR-repeat-containing proteins | R | NrfG | 278 | 825182 | 824069 | r-3 | 2038 | 1264182 | 1265363 | 825196 | 824015 |
| 873 | Putative translation factor (SUA5) | J | SUA5 | 485 | 826289 | 825275 | r-3 | 2037 | 1263084 | 1264112 | 826294 | 825266 |
| 874 | Predicted glycosyltransferases | M | RfaG | 358 | 827411 | 826379 | r-3 | 2036 | 1261965 | 1262999 | 827413 | 826379 |
| 875 | Asparagine synthase (glutamine-hydrolyzing) | E | AsnB | 543 | 828887 | 827453 | r-3 | 2035 | 1260474 | 1261943 | 828904 | 827435 |
| 876 | GTPases | R | - | 355 | 829720 | 828985 | r-1 | 1324 | 1259650 | 1260393 | 829728 | 828985 |

| | | | | | | | | | | | | |
|---|---|---|---|---|---|---|---|---|---|---|---|---|
| 877 | 829725 | 830471 | 1259653 | 1258907 | 1712 | r-2 | 829734 | 830466 | 361 | - | D | ATPases involved in chromosome partitioning |
| 878 | 830551 | 832368 | 1258827 | 1257010 | 157 | f-1 | 830560 | 832363 | 924 | - | R | ATPases of the PilT family |
| 879 | 832337 | 833035 | 1257041 | 1256343 | 509 | f-2 | 832469 | 833018 | 196 | Maf | D | Nucleotide-binding protein implicated in inhibition of septum formation |
| 880 | 836010 | 837260 | 1253368 | 1252118 | 1711 | r-2 | 836019 | 837258 | 744 | GCD1 | MJ | Nucleoside-diphosphate-sugar pyrophosphorylases involved in lipopolysaccharide biosynthesis/translation initiation factor eIF2B subunits COG1208 GCD1 |
| 881 | 837335 | 837601 | 1252043 | 1251777 | 2034 | r-3 | 837341 | 837458 | 35 | MCM 2 | L | Predicted ATPase involved in replication control |
| 882 | 837647 | 839638 | 1251731 | 1249740 | 2033 | r-3 | 837677 | 839612 | 820 | FeoB | P | Ferrous ion uptake system protein FeoB (predicted GTPase) |
| 883 | 839649 | 839885 | 1249729 | 1249493 | 1710 | r-2 | 839664 | 839883 | 83 | FeoA | P | Protein |
| 884 | 840097 | 840471 | 1249281 | 1248907 | 158 | f-1 | 840103 | 840271 | 29 | Rfe | M | UDP-N-acetylmuramyl pentapeptide phosphotransferase/UDP-N-acetylglucosamine-1-phosphate transferase |

| 885 | 840503 | 841321 | 1248875 | 1248057 | 510 | f-2 | 840503 | 841277 | 389 | MesJ | D | Predicted ATPase of the PP-loop superfamily implicated in cell cycle control |
|---|---|---|---|---|---|---|---|---|---|---|---|---|
| 886 | 841293 | 842288 | 1248085 | 1247090 | 886 | f-3 | 841305 | 842244 | 209 | HypE | O | Hydrogenase maturation factor |
| 887 | 842275 | 842628 | 1247103 | 1246750 | 159 | f-1 | 842377 | 842617 | 50 | - | R | Predicted nucleotidyltransferases |
| 888 | 842986 | 844059 | 1246392 | 1245319 | 1323 | r-1 | 843040 | 843955 | 457 | - | R | Predicted RNA-binding proteins |
| 889 | 844320 | 844517 | 1245058 | 1244861 | 1709 | r-2 | | | | | | |
| 890 | 844597 | 845652 | 1244781 | 1243726 | 1322 | r-1 | 844597 | 845650 | 473 | PepP | E | Xaa-Pro aminopeptidase |
| 891 | 845725 | 846387 | 1243653 | 1242991 | 160 | f-1 | 845728 | 846277 | 96 | - | R | Predicted hydrolases of the HAD superfamily |
| 892 | 846422 | 846727 | 1242956 | 1242651 | 511 | f-2 | 846500 | 846725 | 100 | - | J | Ribosomal protein L35AE/L33A |
| 893 | 846773 | 847903 | 1242605 | 1241475 | 512 | f-2 | 846773 | 847895 | 484 | TRM1 | J | N2 |
| 894 | 847896 | 848990 | 1241482 | 1240388 | 887 | f-3 | 847896 | 848988 | 450 | - | S | Uncharacterized membrane proteins |
| 895 | 848774 | 848884 | 1240604 | 1240494 | 2032 | r-3 | 848777 | 848870 | 26 | - | R | Predicted alternative tryptophan synthase beta-subunit (paralog of TrpB) |
| 896 | 848987 | 849100 | 1240391 | 1240278 | 2031 | r-3 | | | | | | |
| 897 | 849375 | 849638 | 1240003 | 1239740 | 1708 | r-2 | 849387 | 849540 | 43 | UvrC | L | Nuclease subunit of the excinuclease complex |

EP 1 923 464 A1

| 898 | 849669 | 851036 | 1239709 | 1238342 | 1707 | r-2 | 849678 | 851004 | 614 | NorM | Q | Na+-driven multidrug efflux pump |
|---|---|---|---|---|---|---|---|---|---|---|---|---|
| 899 | 851134 | 851325 | 1238244 | 1238053 | 1321 | r-1 | 851134 | 851317 | 115 | RPL37A | J | Ribosomal protein L37E |
| 900 | 851346 | 851582 | 1238032 | 1237796 | 1706 | r-2 | 851352 | 851574 | 114 | LSM1 | K | Small nuclear ribonucleoprotein (snRNP) homolog |
| 901 | 851738 | 854035 | 1237640 | 1235343 | 513 | f-2 | 852581 | 854012 | 262 | AmyA | G | Glycosidases |
| 902 | 851818 | 851883 | 1237560 | 1237495 | 1320 | r-1 |  |  |  |  |  |  |
| 903 | 854126 | 855841 | 1235252 | 1233537 | 514 | f-2 | 854129 | 855836 | 978 | GRS1 | J | Glycyl-tRNA synthetase |
| 904 | 855888 | 856652 | 1233490 | 1232726 | 888 | f-3 | 855975 | 856650 | 291 | - | R | Predicted permeases |
| 905 | 856637 | 856798 | 1232741 | 1232580 | 2030 | r-3 | 856637 | 856763 | 27 | PotB | E | ABC-type spermidine/putrescine transport system |
| 906 | 857151 | 858227 | 1232227 | 1231151 | 889 | f-3 | 857238 | 858216 | 375 | - | L | Predicted DNA modification methylase |
| 907 | 858728 | 858934 | 1230650 | 1230444 | 515 | f-2 |  |  |  |  |  |  |
| 908 | 860080 | 860340 | 1229298 | 1229038 | 161 | f-1 | 860128 | 860266 | 29 | MrcA | M | Membrane carboxypeptidase (penicillin-binding protein) |
| 909 | 860404 | 861084 | 1228974 | 1228294 | 1319 | r-1 | 860443 | 861079 | 402 | - | R | Predicted metal-dependent hydrolases related to alanyl-tRNA synthetase HxxxH domain |

| 910 | 861133 | 862545 | 1228245 | 1226833 | 1318 | r-1 | 862474 | 862543 | 40 | OppA | EP | ABC-type dipeptide/oligopeptide/nickel transport systems |
|---|---|---|---|---|---|---|---|---|---|---|---|---|
| 911 | 862729 | 864021 | 1226649 | 1225357 | 1317 | r-1 | 862744 | 864004 | 586 | GltP | C | Na+/H+-dicarboxylate symporters |
| 912 | 864121 | 864819 | 1225257 | 1224559 | 1316 | r-1 | 864133 | 864793 | 199 | BirA | H | Biotin-(acetyl-CoA carboxylase) ligase |
| 913 | 865002 | 865454 | 1224376 | 1223924 | 890 | f-3 | 865107 | 865314 | 30 | - | R | Uncharacterized FAD-dependent dehydrogenases |
| 914 | 865387 | 866304 | 1223991 | 1223074 | 162 | f-1 | 865489 | 866302 | 457 | FbaB | G | DhnA-type fructose-1 |
| 915 | 866496 | 868313 | 1222882 | 1221065 | 891 | f-3 | 866535 | 868305 | 800 | PycA | C | Pyruvate carboxylase |
| 916 | 868296 | 868430 | 1221082 | 1220948 | 1705 | r-2 | 868338 | 868413 | 26 | - | S | Uncharacterized ACR |
| 917 | 868444 | 870222 | 1220934 | 1219156 | 163 | f-1 | 868483 | 870106 | 640 | CstA | T | Carbon starvation protein |
| 918 | 870263 | 870547 | 1219115 | 1218831 | 516 | f-2 | 870374 | 870533 | 30 | OmpR | TK | Response regulators consisting of a CheY-like receiver domain and a HTH DNA-binding domain COG0745 OmpR |
| 919 | 870532 | 870840 | 1218846 | 1218538 | 164 | f-1 | 870586 | 870769 | 29 | OppF | EP | ABC-type dipeptide/oligopeptide/nickel transport system |
| 920 | 870842 | 871846 | 1218536 | 1217532 | 517 | f-2 | 870851 | 871838 | 451 | ArsA | P | Arsenite transporting ATPase |
| 921 | 871836 | 872120 | 1217542 | 1217258 | 892 | f-3 | 871845 | 872079 | 38 | PaaD | R | Putative aromatic ring |

| | | | | | | | | | | | | hydroxylating enzyme |
|---|---|---|---|---|---|---|---|---|---|---|---|---|
| 922 | 871942 | 872775 | 1217436 | 1216603 | 165 | f-1 | 872578 | 872758 | 33 | Smc | D | Chromosome segregation ATPases |
| 923 | 872833 | 873117 | 1216545 | 1216261 | 166 | f-1 | 872863 | 873061 | 50 | AbrB | K | Regulators of stationary/sporulation gene expression |
| 924 | 873524 | 874306 | 1215854 | 1215072 | 518 | f-2 | 873530 | 874292 | 400 | PldB | I | Lysophospholipase |
| 925 | 874707 | 874940 | 1214671 | 1214438 | 893 | f-3 | 874749 | 874902 | 26 | - | D | Intracellular septation protein A |
| 926 | 875022 | 875840 | 1214356 | 1213538 | 894 | f-3 | 875025 | 875277 | 33 | ValS | J | Valyl-tRNA synthetase |
| 927 | 875837 | 876856 | 1213541 | 1212522 | 2029 | r-3 | 875837 | 876854 | 603 | PurA | F | Adenylosuccinate synthase |
| 928 | 877020 | 877235 | 1212358 | 1212143 | 895 | f-3 | 877107 | 877197 | 31 | Hmp | C | Flavodoxin reductases (ferredoxin-NADPH reductases) family 1 |
| 929 | 877271 | 878197 | 1212107 | 1211181 | 519 | f-2 | 877274 | 878180 | 435 | WcaG | M G | Nucleoside-diphosphate-sugar epimerases COG0451 WcaG |
| 930 | 878209 | 878658 | 1211169 | 1210720 | 1315 | r-1 | 878317 | 878650 | 145 | GIM5 | O | Predicted prefoldin |
| 931 | 878718 | 878765 | 1210660 | 1210613 | 896 | f-3 | | | | | | |
| 932 | 878886 | 879182 | 1210492 | 1210196 | 897 | f-3 | | | | | | |
| 933 | 879211 | 880500 | 1210167 | 1208878 | 167 | f-1 | 879229 | 880453 | 249 | | | |
| 934 | 880506 | 881387 | 1208872 | 1207991 | 898 | f-3 | 880518 | 881385 | 365 | EutG | C | Alcohol dehydrogenase IV |

| | | | | | | | | | | | |
|---|---|---|---|---|---|---|---|---|---|---|---|
| 935 | 881550 | 881654 | 1207828 | 1207724 | 899 | f-3 | 881550 | 881646 | 43 | EutG | C | Alcohol dehydrogenase IV |
| 936 | 882812 | 882925 | 1206566 | 1206453 | 2028 | r-3 | | | | | | |
| 937 | 885694 | 886539 | 1203684 | 1202839 | 1314 | r-1 | 885694 | 886495 | 110 | | | |
| 938 | 886567 | 887178 | 1202811 | 1202200 | 1313 | r-1 | 886657 | 887176 | 174 | | | |
| 939 | 887275 | 887487 | 1202103 | 1201891 | 168 | f-1 | 887284 | 887434 | 40 | - | S | Uncharacterized ArCR |
| 940 | 887717 | 887920 | 1201661 | 1201458 | 520 | f-2 | 887720 | 887915 | 54 | - | R | Predicted nucleic acid-binding protein |
| 941 | 887924 | 890701 | 1201454 | 1198677 | 521 | f-2 | 887924 | 890642 | 1093 | Lhr | R | Lhr-like helicases |
| 942 | 891114 | 891398 | 1198264 | 1197980 | 900 | f-3 | 891159 | 891396 | 31 | Nfo | L | Endonuclease IV |
| 943 | 891434 | 895009 | 1197944 | 1194369 | 522 | f-2 | 891443 | 894968 | 1392 | Smc | D | Chromosome segregation ATPases |
| 944 | 895013 | 895678 | 1194365 | 1193700 | 523 | f-2 | 895022 | 895667 | 248 | - | S | Uncharacterized ACR |
| 945 | 895675 | 896097 | 1193703 | 1193281 | 1312 | r-1 | 895888 | 896050 | 30 | AcyP | C | Acylphosphatases |
| 946 | 896626 | 899040 | 1192752 | 1190338 | 169 | f-1 | 896632 | 898126 | 684 | MPH1 | L | ERCC4-like helicases |
| 947 | 899156 | 900004 | 1190222 | 1189374 | 2027 | r-3 | 899165 | 899987 | 342 | DppA | E | Uncharacterized protein associated with dipeptide transport |
| 948 | 900134 | 900385 | 1189244 | 1188993 | 524 | f-2 | 900230 | 900314 | 30 | MglA | G | ABC-type sugar (aldose) transport system |
| 949 | 901696 | 902574 | 1187682 | 1186804 | 1311 | r-1 | 901891 | 901987 | 30 | TenA | K | Putative transcription activator |
| 950 | 902700 | 903458 | 1186678 | 1185920 | 1704 | r-2 | 902703 | 903450 | 387 | - | R | Predicted phosphate-binding enzymes |

| | | | | | | | | | | | | |
|---|---|---|---|---|---|---|---|---|---|---|---|---|
| 951 | 903912 | 904115 | 1185466 | 1185263 | 1703 | r-2 | 903912 | 904077 | 45 | - | S | Uncharacterized ArCR |
| 952 | 904127 | 904555 | 1185251 | 1184823 | 2026 | r-3 | 904127 | 904520 | 173 | - | S | Uncharacterized ACR |
| 953 | 904610 | 905026 | 1184768 | 1184352 | 525 | f-2 | 904871 | 904967 | 28 | TFA1 | K | Transcription initiation factor IIE |
| 954 | 905105 | 906898 | 1184273 | 1182480 | 526 | f-2 | 905105 | 906887 | 998 | - | R | RNase L inhibitor homolog |
| 955 | 906982 | 907974 | 1182396 | 1181404 | 170 | f-1 | 906994 | 907963 | 387 | HypE | O | Hydrogenase maturation factor |
| 956 | 907975 | 908217 | 1181403 | 1181161 | 1310 | r-1 | 907975 | 908215 | 98 | - | S | Uncharacterized ACR |
| 957 | 908370 | 909260 | 1181008 | 1180118 | 1702 | r-2 | 908463 | 909246 | 221 | - | L | Predicted type IV restriction endonuclease |
| 958 | 909301 | 910116 | 1180077 | 1179262 | 171 | f-1 | 909313 | 910093 | 189 | - | R | Predicted glutamine amidotransferase |
| 959 | 910097 | 910516 | 1179281 | 1178862 | 527 | f-2 | 910106 | 910514 | 190 | - | R | CBS domains |
| 960 | 910513 | 912024 | 1178865 | 1177354 | 172 | f-1 | 910531 | 912016 | 744 | Icc | R | Predicted phosphohydrolases |
| 961 | 912021 | 912893 | 1177357 | 1176485 | 1701 | r-2 | 912021 | 912879 | 311 | - | G | 2-Phosphoglycerate kinase |
| 962 | 912890 | 914188 | 1176488 | 1175190 | 2025 | r-3 | 913589 | 913814 | 45 | - | S | Uncharacterized ACR |
| 963 | 914305 | 914493 | 1175073 | 1174885 | 173 | f-1 | 914389 | 914491 | 27 | HHT1 | L | Histones H3 and H4 |
| 964 | 914711 | 915121 | 1174667 | 1174257 | 528 | f-2 | 914711 | 915119 | 153 | ArsR | K | Predicted transcriptional regulators |
| 965 | 915118 | 916428 | 1174260 | 1172950 | 174 | f-1 | 915148 | 915403 | 37 | - | S | Uncharacterized ArCR |
| 966 | 916589 | 917257 | 1172789 | 1172121 | 529 | f-2 | 916604 | 917246 | 142 | - | S | Uncharacterized BCR |
| 967 | 917348 | 918352 | 1172030 | 1171026 | 530 | f-2 | 917357 | 918311 | 400 | | | |
| 968 | 918655 | 918705 | 1170723 | 1170673 | 1309 | r-1 | | | | | | |

| 969 | 918719 | 919171 | 1170659 | 1170207 | 2024 | r-3 | 918779 | 919163 | 149 | - | S | Uncharacterized ACR |
|---|---|---|---|---|---|---|---|---|---|---|---|---|
| 970 | 919305 | 923264 | 1170073 | 1166114 | 901 | f-3 | 920052 | 920499 | 60 | - | L | Micrococcal nuclease (thermonuclease) homologs |
| 971 | 924116 | 924814 | 1165262 | 1164564 | 2023 | r-3 | 924128 | 924773 | 140 | RAD55 | T | RecA-superfamily ATPases implicated in signal transduction |
| 972 | 925010 | 927244 | 1164368 | 1162134 | 531 | f-2 | 925019 | 926708 | 1043 | MetG | J | Methionyl-tRNA synthetase |
| 973 | 927249 | 927578 | 1162129 | 1161800 | 1700 | r-2 | 927339 | 927576 | 92 | - | S | Uncharacterized membrane-associated protein/domain |
| 974 | 928257 | 929309 | 1161121 | 1160069 | 1699 | r-2 | 928353 | 929178 | 45 | SbcC | L | ATPase involved in DNA repair |
| 975 | 929424 | 929705 | 1159954 | 1159673 | 1698 | r-2 | 929538 | 929697 | 33 | Dcp | E | Zn-dependent oligopeptidases |
| 976 | 930480 | 931013 | 1158898 | 1158365 | 1697 | r-2 | 930486 | 930996 | 219 | WecD | K R | Histone acetyltransferase HPA2 and related acetyltransferases COG0454 WecD |
| 977 | 931103 | 931576 | 1158275 | 1157802 | 532 | f-2 | 931145 | 931556 | 147 | Bcp | O | Peroxiredoxin |
| 978 | 931594 | 932070 | 1157784 | 1157308 | 175 | f-1 | 931651 | 932068 | 190 | - | S | Uncharacterized ACR |
| 979 | 932526 | 933086 | 1156852 | 1156292 | 902 | f-3 | 932535 | 933084 | 180 | - | S | Uncharacterized ACR |
| 980 | 933128 | 933430 | 1156250 | 1155948 | 533 | f-2 | 933128 | 933428 | 153 | - | S | Uncharacterized ACR |
| 981 | 933728 | 933904 | 1155650 | 1155474 | 534 | f-2 | 933779 | 933902 | 32 | - | S | Uncharacterized ACR |
| 982 | 933919 | 934392 | 1155459 | 1154986 | 1308 | r-1 | 933925 | 934387 | 75 | - | S | Uncharacterized ACR |

| 983 | 934564 | 935379 | 1154814 | 1153999 | 176 | f-1 | 934612 | 935371 | 180 | MscS | M | Small-conductance mechanosensitive channel |
|---|---|---|---|---|---|---|---|---|---|---|---|---|
| 984 | 935513 | 936664 | 1153865 | 1152714 | 2022 | r-3 | 935549 | 936659 | 541 | - | R | Predicted Fe-S oxidoreductases |
| 985 | 936666 | 936944 | 1152712 | 1152434 | 1696 | r-2 | 936696 | 936942 | 94 | MoaD | H | Molybdopterin converting factor |
| 986 | 936987 | 938822 | 1152391 | 1150556 | 1695 | r-2 | 937005 | 938814 | 977 | - | C | Aldehyde:ferredoxin oxidoreductase |
| 987 | 938954 | 940192 | 1150424 | 1149186 | 535 | f-2 | 938969 | 940178 | 572 | - | S | Uncharacterized ACR |
| 988 | 940239 | 940469 | 1149139 | 1148909 | 903 | f-3 | | | | | | |
| 989 | 940803 | 940937 | 1148575 | 1148441 | 904 | f-3 | | | | | | |
| 990 | 940934 | 942055 | 1148444 | 1147323 | 536 | f-2 | 940943 | 942050 | 604 | - | R | Uncharacterized proteins of the AP superfamily |
| 991 | 942591 | 942917 | 1146787 | 1146461 | 905 | f-3 | 942627 | 942897 | 93 | - | R | Predicted nucleotidyltransferases |
| 992 | 942914 | 943306 | 1146464 | 1146072 | 2021 | r-3 | 943067 | 943286 | 28 | TrmA | J | SAM-dependent methyltransferases related to tRNA (uracil-5-)-methyltransferase |
| 993 | 943357 | 943545 | 1146021 | 1145833 | 1307 | r-1 | 943357 | 943528 | 32 | PyrE | F | Orotate phosphoribosyltransferase |
| 994 | 943533 | 943778 | 1145845 | 1145600 | 1694 | r-2 | 943542 | 943677 | 46 | AbrB | K | Regulators of stationary/sporulation gene expression |

EP 1 923 464 A1

| 995 | 943889 | 944536 | 1145489 | 1144842 | 2020 | r-3 | 943889 | 944534 | 335 | RpsG | J | Ribosomal protein S7 |
|------|--------|--------|---------|---------|------|-----|--------|--------|------|-------|---|----------------------|
| 996 | 944542 | 944994 | 1144836 | 1144384 | 1306 | r-1 | 944542 | 944992 | 263 | RpsL | J | Ribosomal protein S12 |
| 997 | 944996 | 945436 | 1144382 | 1143942 | 2019 | r-3 | 944999 | 945434 | 255 | NusA | K | Transcription terminator |
| 998 | 945433 | 945741 | 1143945 | 1143637 | 1305 | r-1 | 945436 | 945727 | 145 | RPL30 | J | Ribosomal protein L30E |
| 999 | 945755 | 946939 | 1143623 | 1142439 | 2018 | r-3 | 945764 | 946931 | 652 | RpoC | K | DNA-directed RNA polymerase beta' subunit/160 kD subunit (split gene in archaea and Syn) |
| 1000 | 946932 | 948164 | 1142446 | 1141214 | 1693 | r-2 | 947001 | 948162 | 674 | RpoC | K | DNA-directed RNA polymerase beta' subunit/160 kD subunit (split gene in archaea and Syn) |
| 1001 | 948079 | 949662 | 1141299 | 1139716 | 1304 | r-1 | 948088 | 949645 | 961 | RpoC | K | DNA-directed RNA polymerase beta' subunit/160 kD subunit (split gene in archaea and Syn) |
| 1002 | 949659 | 953030 | 1139719 | 1136348 | 1692 | r-2 | 949665 | 953028 | 1967 | RpoB | K | DNA-directed RNA polymerase beta subunit/140 kD subunit (split gene in Mjan) |
| 1003 | 953048 | 953296 | 1136330 | 1136082 | 2017 | r-3 | 953048 | 953294 | 118 | RPB5 | K | DNA-directed RNA polymerase |
| 1004 | 953495 | 954190 | 1135883 | 1135188 | 2016 | r-3 | 953510 | 954185 | 408 | TrxA | O / C | Thiol-disulfide isomerase and thioredoxins COG0526 TrxA |

| 1005 | 954301 | 955020 | 1135077 | 1134358 | 177 | f-1 | 954316 | 955009 | 290 | - | K | Predicted transcriptional regulators |
|---|---|---|---|---|---|---|---|---|---|---|---|---|
| 1006 | 955204 | 956391 | 1134174 | 1132987 | 178 | f-1 | 955213 | 956347 | 629 | FixC | C | Dehydrogenases (flavoproteins) |
| 1007 | 956375 | 956533 | 1133003 | 1132845 | 2015 | r-3 | 956402 | 956498 | 26 | - | S | Uncharacterized BCR |
| 1008 | 957270 | 957638 | 1132108 | 1131740 | 906 | f-3 | 957477 | 957579 | 28 | - | R | Predicted integral membrane protein |
| 1009 | 957640 | 961329 | 1131738 | 1128049 | 1303 | r-1 | 957649 | 958597 | 493 | TopA | L | Topoisomerase IA |
| 1010 | 961407 | 962324 | 1127971 | 1127054 | 907 | f-3 | 961689 | 961947 | 35 | FepC | PH | ABC-type cobalamin/Fe3+-siderophores transport systems |
| 1011 | 962372 | 962575 | 1127006 | 1126803 | 537 | f-2 | 962372 | 962573 | 108 | ThiS | H | Sulfur transfer protein involved in thiamine biosynthesis |
| 1012 | 962593 | 963804 | 1126785 | 1125574 | 1302 | r-1 | 962605 | 963799 | 691 | AvtA | E | PLP-dependent aminotransferases |
| 1013 | 964168 | 964827 | 1125210 | 1124551 | 179 | f-1 | 964495 | 964822 | 139 | - | S | Uncharacterized membrane protein |
| 1014 | 964831 | 965430 | 1124547 | 1123948 | 1301 | r-1 | 965176 | 965329 | 36 | SbcC | L | ATPase involved in DNA repair |
| 1015 | 965603 | 965896 | 1123775 | 1123482 | 538 | f-2 | 965612 | 965894 | 188 | RPL42 A | J | Ribosomal protein L44E |
| 1016 | 965901 | 966098 | 1123477 | 1123280 | 908 | f-3 | 965901 | 966096 | 128 | RPS27 A | J | Ribosomal protein S27E |
| 1017 | 966166 | 967002 | 1123212 | 1122376 | 180 | f-1 | 966175 | 966955 | 461 | SUI2 | J | Translation initiation factor |

EP 1 923 464 A1

| No. | | | | | | | | | | Gene | Cat. | Description |
|---|---|---|---|---|---|---|---|---|---|---|---|---|
|  |  |  |  |  |  |  |  |  |  |  |  | eIF2alpha |
| 1018 | 967002 | 967181 | 1122376 | 1122197 | f-3 | 909 | 967002 | 967176 | 120 | - | J | Predicted Zn-ribbon RNA-binding protein |
| 1019 | 967184 | 967987 | 1122194 | 1121391 | f-2 | 539 | 967184 | 967985 | 394 | - | R | Uncharacterized proteins of the ATP-grasp superfamily |
| 1020 | 968134 | 968757 | 1121244 | 1120621 | f-1 | 181 | 968143 | 968734 | 142 | CbiM | H | Cobalamin biosynthesis protein CbiM |
| 1021 | 968754 | 969002 | 1120624 | 1120376 | f-3 | 910 | 968760 | 968970 | 33 | CbiM | H | Cobalamin biosynthesis protein CbiM |
| 1022 | 968995 | 969663 | 1120383 | 1119715 | f-1 | 182 | 969193 | 969643 | 72 | CbiQ | P | ABC-type cobalt transport system |
| 1023 | 969660 | 970463 | 1119718 | 1118915 | f-3 | 911 | 969660 | 970404 | 233 | CbiO | P | ABC-type cobalt transport system |
| 1024 | 970555 | 971892 | 1118823 | 1117486 | f-1 | 183 | 971431 | 971527 | 33 | AprE | O | Subtilisin-like serine proteases |
| 1025 | 971952 | 973340 | 1117426 | 1116038 | r-2 | 1691 | 971970 | 973332 | 786 | CpsG | G | Phosphomannomutase |
| 1026 | 973366 | 974772 | 1116012 | 1114606 | r-1 | 1300 | 973375 | 974356 | 455 | CpsB | M | Mannose-1-phosphate guanylyltransferase |
| 1027 | 974823 | 976277 | 1114555 | 1113101 | r-2 | 1690 | 975489 | 975720 | 32 | - | K | RNA-binding proteins (RRM domain) |
| 1028 | 976234 | 976803 | 1113144 | 1112575 | r-1 | 1299 | 976240 | 976795 | 340 | - | G R | Thermophilic glucose-6-phosphate isomerase and related metalloenzymes COG2140 - |

| 1029 | 976871 | 977053 | 1112507 | 1112325 | 2014 | r-3 | 976880 | 977042 | 59 | NusA | K | Transcription terminator |
|------|--------|--------|---------|---------|------|-----|--------|--------|------|------|---|---------------------------|
| 1030 | 977082 | 977765 | 1112296 | 1111613 | 1689 | r-2 | 977082 | 977730 | 174 | - | S | Uncharacterized ACR |
| 1031 | 977762 | 978706 | 1111616 | 1110672 | 2013 | r-3 | 977762 | 978671 | 401 | ElaC | R | Metal-dependent hydrolases of the beta-lactamase superfamily III |
| 1032 | 978776 | 979747 | 1110602 | 1109631 | 540 | f-2 | 978791 | 979706 | 234 | NrfG | R | TPR-repeat-containing proteins |
| 1033 | 979826 | 981100 | 1109552 | 1108278 | 541 | f-2 | 979841 | 981095 | 488 | TrmA | J | SAM-dependent methyltransferases related to tRNA (uracil-5-)-methyltransferase |
| 1034 | 981159 | 981425 | 1108219 | 1107953 | 1688 | r-2 | 981168 | 981357 | 28 | DapD | E | Tetrahydrodipicolinate N-succinyltransferase |
| 1035 | 981762 | 981815 | 1107616 | 1107563 | 1687 | r-2 | | | | | | |
| 1036 | 982136 | 982483 | 1107242 | 1106895 | 542 | f-2 | 982136 | 982481 | 168 | - | H | 6-pyruvoyl-tetrahydropterin synthase |
| 1037 | 982480 | 982953 | 1106898 | 1106425 | 1298 | r-1 | 982480 | 982822 | 142 | - | S | Uncharacterized ACR |
| 1038 | 983025 | 983486 | 1106353 | 1105892 | 912 | f-3 | 983058 | 983460 | 115 | GIM5 | O | Predicted prefoldin |
| 1039 | 983483 | 983821 | 1105895 | 1105557 | 543 | f-2 | 983516 | 983723 | 35 | GimC | O | Prefoldin |
| 1040 | 983802 | 984371 | 1105576 | 1105007 | 1686 | r-2 | 983802 | 984354 | 278 | PorG | C | Pyruvate:ferredoxin oxidoreductase and related 2-oxoacid:ferredoxin oxidoreductases |

EP 1 923 464 A1

| 1041 | 984359 | 985399 | 1105019 | 1103979 | 2012 | r-3 | 984554 | 985397 | 537 | PorB | C | Pyruvate:ferredoxin oxidoreductase and related 2-oxoacid:ferredoxin oxidoreductases |
|---|---|---|---|---|---|---|---|---|---|---|---|---|
| 1042 | 985204 | 986352 | 1104174 | 1103026 | 1297 | r-1 | 985204 | 986338 | 639 | PorA | C | Pyruvate:ferredoxin oxidoreductase and related 2-oxoacid:ferredoxin oxidoreductases |
| 1043 | 986349 | 986912 | 1103029 | 1102466 | 1685 | r-2 | 986400 | 986904 | 284 | PorG | C | Pyruvate:ferredoxin oxidoreductase and related 2-oxoacid:ferredoxin oxidoreductases |
| 1044 | 986851 | 987246 | 1102527 | 1102132 | 1296 | r-1 | 986935 | 987235 | 96 | - | S | Uncharacterized ACR |
| 1045 | 987243 | 987566 | 1102135 | 1101812 | 1684 | r-2 | 987297 | 987375 | 32 | - | R | Predicted nucleotidyltransferases |
| 1046 | 987517 | 988383 | 1101861 | 1100995 | 1295 | r-1 | 987517 | 988369 | 501 | PorB | C | Pyruvate:ferredoxin oxidoreductase and related 2-oxoacid:ferredoxin oxidoreductases |
| 1047 | 988383 | 989573 | 1100995 | 1099805 | 1683 | r-2 | 988383 | 989571 | 743 | PorA | C | Pyruvate:ferredoxin oxidoreductase and related 2-oxoacid:ferredoxin oxidoreductases |

| 1048 | 989577 | 989894 | 1099801 | 1099484 | 1682 | r-2 | 989577 | 989877 | 125 | - | C | Ferredoxin 3 |
|---|---|---|---|---|---|---|---|---|---|---|---|---|
| 1049 | 990762 | 991511 | 1098616 | 1097867 | 913 | f-3 | 991125 | 991500 | 33 | CcmA | Q | ABC-type multidrug transport system |
| 1050 | 991803 | 991991 | 1097575 | 1097387 | 914 | f-3 | | | | | | |
| 1051 | 992036 | 993010 | 1097342 | 1096368 | 2011 | r-3 | 992042 | 993002 | 446 | - | R | Predicted Fe-S oxidoreductases |
| 1052 | 994241 | 995020 | 1095137 | 1094358 | 544 | f-2 | 994241 | 994985 | 244 | SurE | R | Survival protein |
| 1053 | 995047 | 995112 | 1094331 | 1094266 | 184 | f-1 | | | | | | |
| 1054 | 995380 | 995844 | 1093998 | 1093534 | 185 | f-1 | 995419 | 995779 | 78 | - | S | Predicted membrane protein |
| 1055 | 995878 | 996558 | 1093500 | 1092820 | 1294 | r-1 | 995881 | 996550 | 278 | SpoV K | O | ATPases of the AAA+ class |
| 1056 | 997037 | 998464 | 1092341 | 1090914 | 545 | f-2 | 997097 | 998456 | 785 | SerS | J | Seryl-tRNA synthetase |
| 1057 | 998525 | 999265 | 1090853 | 1090113 | 2010 | r-3 | 998588 | 999200 | 298 | Nth | L | Predicted EndoIII-related endonuclease |
| 1058 | 999750 | 1000229 | 1089628 | 1089149 | 915 | f-3 | 999843 | 1000212 | 168 | - | K | Predicted transcriptional regulators |
| 1059 | 1000226 | 1001212 | 1089152 | 1088166 | 546 | f-2 | 1000235 | 1001201 | 503 | CcmA | Q | ABC-type multidrug transport system |
| 1060 | 1001217 | 1001987 | 1088161 | 1087391 | 916 | f-3 | 1001217 | 1001982 | 355 | - | R | ABC-type multidrug transport system |
| 1061 | 1002002 | 1003240 | 1087376 | 1086138 | 2009 | r-3 | 1002005 | 1003226 | 590 | Pgk | G | 3-phosphoglycerate kinase |
| 1062 | 1003253 | 1005466 | 1086125 | 1083912 | 547 | f-2 | 1003355 | 1003715 | 40 | Mrr | L | Restriction endonuclease |

EP 1 923 464 A1

| 1063 | 1005467 | 1006087 | 1083911 | 1083291 | 2008 | r-3 | 1005581 | 1005884 | 38 | LeuA | E | Isopropylmalate/homocitrate/citra malate synthases |
|------|---------|---------|---------|---------|------|-----|---------|---------|-----|------|---|---|
| 1064 | 1006202 | 1007890 | 1083176 | 1081488 | 2007 | r-3 | 1006202 | 1007888 | 1040 | Sbm | I | Methylmalonyl-CoA mutase |
| 1065 | 1007979 | 1010192 | 1081399 | 1079186 | 1681 | r-2 | 1008876 | 1009398 | 41 | AlsD | H | Glutamate-1-semialdehyde aminotransferase |
| 1066 | 1010189 | 1010956 | 1079189 | 1078422 | 2006 | r-3 | 1010246 | 1010591 | 94 | NosY | R | ABC-type transport system involved in multi-copper enzyme maturation |
| 1067 | 1011011 | 1011949 | 1078367 | 1077429 | 2005 | r-3 | 1011011 | 1011938 | 464 | CcmA | Q | ABC-type multidrug transport system |
| 1068 | 1012013 | 1012879 | 1077365 | 1076499 | 548 | f-2 | 1012013 | 1012862 | 332 | YSH1 | J | Predicted exonuclease of the beta-lactamase fold involved in RNA processing |
| 1069 | 1012961 | 1013278 | 1076417 | 1076100 | 549 | f-2 | 1013114 | 1013255 | 29 | MdlB | Q | ABC-type multidrug/protein/lipid transport system |
| 1070 | 1013371 | 1013883 | 1076007 | 1075495 | 186 | f-1 | 1013407 | 1013806 | 214 | IbpA | O | Molecular chaperone (small heat shock protein) |
| 1071 | 1013995 | 1014411 | 1075383 | 1074967 | 1293 | r-1 | 1014265 | 1014361 | 30 | FlgD | N | Flagellar hook capping protein |
| 1072 | 1014829 | 1017228 | 1074549 | 1072150 | 187 | f-1 | 1014829 | 1017226 | 1310 | SpoV K | O | ATPases of the AAA+ class |
| 1073 | 1017331 | 1020711 | 1072047 | 1068667 | 188 | f-1 | 1018411 | 1018645 | 56 | - | L | Type II restriction enzyme |

EP 1 923 464 A1

| 1074 | 1020821 | 1020970 | 1068557 | 1068408 | 2004 | r-3 | 1020854 | 1020962 | 26 | - | R | Predicted hydrolase of alkaline phosphatase superfamily |
|---|---|---|---|---|---|---|---|---|---|---|---|---|
| 1075 | 1021424 | 1022338 | 1067954 | 1067040 | 550 | f-2 | 1021535 | 1022261 | 177 | FolP | H | Dihydropteroate synthase |
| 1076 | 1022319 | 1023311 | 1067059 | 1066067 | 1680 | r-2 | 1022328 | 1023294 | 249 | PerM | R | Predicted permease |
| 1077 | 1023301 | 1023780 | 1066077 | 1065598 | 1292 | r-1 | 1023463 | 1023637 | 32 | TldD | R | Predicted Zn-dependent proteases and their inactivated homologs |
| 1078 | 1023781 | 1024785 | 1065597 | 1064593 | 1291 | r-1 | 1023781 | 1024759 | 278 | SppA | N O | Periplasmic serine proteases (ClpP class) COG0616 SppA |
| 1079 | 1024877 | 1025692 | 1064501 | 1063686 | 551 | f-2 | 1024886 | 1025681 | 417 | IolE | G | Sugar phosphate isomerases/epimerases |
| 1080 | 1025682 | 1026086 | 1063696 | 1063292 | 1679 | r-2 | 1025892 | 1026018 | 29 | LeuB | E | Isocitrate/isopropylmalate dehydrogenase |
| 1081 | 1026083 | 1026376 | 1063295 | 1063002 | 2003 | r-3 | 1026122 | 1026374 | 146 | RPB11 | K | DNA-directed RNA polymerase |
| 1082 | 1026357 | 1026986 | 1063021 | 1062392 | 1678 | r-2 | 1026357 | 1026984 | 248 | - | S | Uncharacterized ArCR |
| 1083 | 1026983 | 1027579 | 1062395 | 1061799 | 2002 | r-3 | 1026986 | 1027571 | 280 | - | J | Predicted RNA-binding protein (consists of S1 domain and a Zn-ribbon domain) |
| 1084 | 1027657 | 1029558 | 1061721 | 1059820 | 189 | f-1 | 1027678 | 1029556 | 1040 | ThrS | J | Threonyl-tRNA synthetase |
| 1085 | 1029517 | 1030068 | 1059861 | 1059310 | 1290 | r-1 | 1029589 | 1029943 | 34 | HsdM | L | Type I restriction-modification system methyltransferase subunit |

| 1086 | 1030276 | 1030950 | 1059102 | 1058428 | 1289 | r-1 | 1030711 | 1030900 | 32 | UvrC | L | Nuclease subunit of the excinuclease complex (TBP-interacting protein) |
|---|---|---|---|---|---|---|---|---|---|---|---|---|
| 1087 | 1031013 | 1031807 | 1058365 | 1057571 | 1677 | r-2 | 1031013 | 1031805 | 431 | UppS | I | Undecaprenyl pyrophosphate synthase |
| 1088 | 1031814 | 1032344 | 1057564 | 1057034 | 1676 | r-2 | 1031823 | 1032336 | 291 | PaaY | R | Carbonic anhydrases/acetyltransferases |
| 1089 | 1032406 | 1032792 | 1056972 | 1056586 | 190 | f-1 | 1032412 | 1032781 | 137 | - | L | Holliday junction resolvase - archaeal type |
| 1090 | 1032841 | 1034373 | 1056537 | 1055005 | 191 | f-1 | 1032913 | 1033582 | 45 | - | S | Predicted membrane protein |
| 1091 | 1034458 | 1035498 | 1054920 | 1053880 | 192 | f-1 | 1034458 | 1035493 | 551 | FrvX | G | Cellulase M and related proteins |
| 1092 | 1035541 | 1036101 | 1053837 | 1053277 | 193 | f-1 | 1035547 | 1036087 | 185 | - | R | Predicted Zn-dependent proteases |
| 1093 | 1036098 | 1036649 | 1053280 | 1052729 | 917 | f-3 | 1036104 | 1036623 | 254 | CyaB | F | Adenylate cyclase . |
| 1094 | 1036636 | 1037469 | 1052742 | 1051909 | 194 | f-1 | 1037026 | 1037341 | 48 | NrfG | R | TPR-repeat-containing proteins |
| 1095 | 1037390 | 1038229 | 1051988 | 1051149 | 2001 | r-3 | 1037390 | 1038167 | 275 | CbiO | P | ABC-type cobalt transport system |
| 1096 | 1038226 | 1039704 | 1051152 | 1049674 | 1288 | r-1 | 1038226 | 1039687 | 621 | TrkG | P | Trk-type K+ transport systems |
| 1097 | 1039796 | 1040683 | 1049582 | 1048695 | 552 | f-2 | 1039808 | 1040681 | 417 | Map | J | Methionine aminopeptidase |
| 1098 | 1041012 | 1041071 | 1048366 | 1048307 | 918 | f-3 | | | | | | |
| 1099 | 1041624 | 1041935 | 1047754 | 1047443 | 919 | f-3 | 1041705 | 1041822 | 31 | SurA | O | Parvulin-like peptidyl-prolyl isomerase |

EP 1 923 464 A1

184

| 1100 | 1042133 | 1042384 | 1047245 | 1046994 | 553 | f-2 | 1042145 | 1042382 | 141 | - | R | Predicted nucleic acid-binding protein |
|---|---|---|---|---|---|---|---|---|---|---|---|---|
| 1101 | 1042526 | 1043701 | 1046852 | 1045677 | 554 | f-2 | 1042526 | 1043696 | 659 | - | R | CBS domains |
| 1102 | 1043676 | 1044812 | 1045702 | 1044566 | 1675 | r-2 | 1043805 | 1044027 | 34 | NuoL | CP | NADH:ubiquinone oxidoreductase subunit 5 (chain L)/Multisubunit Na+/H+ antiporter |
| 1103 | 1044809 | 1046068 | 1044569 | 1043310 | 2000 | r-3 | 1044809 | 1046030 | 664 | GCD1 | MJ | Nucleoside-diphosphate-sugar pyrophosphorylases involved in lipopolysaccharide biosynthesis/translation initiation factor eIF2B subunits COG1208 GCD1 |
| 1104 | 1047016 | 1048092 | 1042362 | 1041286 | 195 | f-1 | 1047016 | 1048078 | 543 | - | R | Predicted GTPase |
| 1105 | 1048209 | 1048610 | 1041169 | 1040768 | 1674 | r-2 | 1048218 | 1048596 | 207 | RPS8 A | J | Ribosomal protein S8E |
| 1106 | 1048684 | 1048761 | 1040694 | 1040617 | 1287 | r-1 | | | | | | |
| 1107 | 1048718 | 1049599 | 1040660 | 1039779 | 555 | f-2 | 1049000 | 1049093 | 30 | HypF | O | Hydrogenase maturation factor |
| 1108 | 1049596 | 1051275 | 1039782 | 1038103 | 1286 | r-1 | 1049674 | 1051264 | 897 | PyrG | F | CTP synthase (UTP-ammonia lyase) |
| 1109 | 1051307 | 1051711 | 1038071 | 1037667 | 1999 | r-3 | 1051316 | 1051682 | 168 | - | S | Uncharacterized ArCR |
| 1110 | 1051708 | 1051995 | 1037670 | 1037383 | 1285 | r-1 | 1051720 | 1051993 | 150 | - | S | Uncharacterized ArCR |

EP 1 923 464 A1

| 1111 | 1052192 | 1052701 | 1037186 | 1036677 | 556 | f-2 | 1052495 | 1052684 | 32 | PtsA | G | Phosphoenolpyruvate-protein kinase (PTS system EI component in bacteria) |
|---|---|---|---|---|---|---|---|---|---|---|---|---|
| 1112 | 1052753 | 1053022 | 1036625 | 1036356 | 557 | f-2 | 1052792 | 1053005 | 29 | Tar | N | Methyl-accepting chemotaxis protein |
| 1113 | 1053032 | 1053793 | 1036346 | 1035585 | 558 | f-2 | 1053032 | 1053791 | 411 | NrdG | O | Organic radical activating enzymes |
| 1114 | 1053859 | 1055274 | 1035519 | 1034104 | 196 | f-1 | 1053952 | 1055269 | 727 | TIP49 | L | DNA helicase TIP49 |
| 1115 | 1055358 | 1055663 | 1034020 | 1033715 | 920 | f-3 | 1055370 | 1055445 | 28 | AlsT | E | Na+/alanine symporter |
| 1116 | 1056285 | 1056395 | 1033093 | 1032983 | 921 | f-3 | | | | | | |
| 1117 | 1056392 | 1057381 | 1032986 | 1031997 | 1998 | r-3 | 1056605 | 1056746 | 33 | Rpe | G | Pentose-5-phosphate-3-epimerase |
| 1118 | 1057362 | 1057835 | 1032016 | 1031543 | 1673 | r-2 | 1057494 | 1057680 | 31 | Ffh | N | Signal recognition particle GTPase |
| 1119 | 1057832 | 1058302 | 1031546 | 1031076 | 1997 | r-3 | 1058003 | 1058102 | 28 | - | S | Uncharacterized ACR |
| 1120 | 1058495 | 1059043 | 1030883 | 1030335 | 559 | f-2 | 1058543 | 1059041 | 260 | - | R | Phospholipid-binding protein |
| 1121 | 1059047 | 1059307 | 1030331 | 1030071 | 1996 | r-3 | 1059104 | 1059284 | 30 | RfaG | M | Predicted glycosyltransferases |
| 1122 | 1059399 | 1059863 | 1029979 | 1029515 | 1672 | r-2 | 1059465 | 1059795 | 40 | NrfG | R | TPR-repeat-containing proteins |
| 1123 | 1059921 | 1060517 | 1029457 | 1028861 | 922 | f-3 | 1059933 | 1060434 | 108 | GrxC | O | Glutaredoxin and related proteins |
| 1124 | 1060582 | 1061310 | 1028796 | 1028068 | 197 | f-1 | 1060582 | 1061296 | 247 | CcdA | O | Cytochrome c biogenesis protein |
| 1125 | 1061307 | 1061768 | 1028071 | 1027610 | 1671 | r-2 | 1061322 | 1061766 | 237 | Lrp | K | Transcriptional regulators |

| 1126 | 1061878 | 1063221 | 1027500 | 1026157 | 198 | f-1 | 1061878 | 1063186 | 614 | ArgD | E | PLP-dependent aminotransferases |
|---|---|---|---|---|---|---|---|---|---|---|---|---|
| 1127 | 1063298 | 1064599 | 1026080 | 1024779 | 560 | f-2 | 1063325 | 1064597 | 535 | UraA | F | Xanthine/uracil permeases |
| 1128 | 1064656 | 1065000 | 1024722 | 1024378 | 1284 | r-1 | | | | | | |
| 1129 | 1065370 | 1066023 | 1024008 | 1023355 | 1283 | r-1 | 1065370 | 1065943 | 316 | NuoI | C | Formate hydrogenlyase subunit 6/NADH:ubiquinone oxidoreductase 23 kD subunit (chain I) |
| 1130 | 1066020 | 1067213 | 1023358 | 1022165 | 1670 | r-2 | 1066053 | 1067211 | 652 | NuoD | C | NADH:ubiquinone oxidoreductase 49 kD subunit 7 |
| 1131 | 1067215 | 1067811 | 1022163 | 1021567 | 1282 | r-1 | 1067317 | 1067797 | 180 | NuoC | C | NADH:ubiquinone oxidoreductase 27 kD subunit |
| 1132 | 1067793 | 1068392 | 1021585 | 1020986 | 1669 | r-2 | 1067838 | 1068390 | 335 | NuoB | C | NADH:ubiquinone oxidoreductase 20 kD subunit and related Fe-S oxidoreductases |
| 1133 | 1068394 | 1069287 | 1020984 | 1020091 | 1281 | r-1 | 1068406 | 1069240 | 367 | HyfC | C | Formate hydrogenlyase subunit 4 |
| 1134 | 1069288 | 1071138 | 1020090 | 1018240 | 1280 | r-1 | 1069288 | 1071115 | 678 | HyfB | CP | Formate hydrogenlyase subunit 3/Multisubunit Na+/H+ antiporter |
| 1135 | 1070858 | 1070965 | 1018520 | 1018413 | 561 | f-2 | | | | | | |
| 1136 | 1071135 | 1072622 | 1018243 | 1016756 | 1668 | r-2 | 1071186 | 1072614 | 713 | HyfB | CP | Formate hydrogenlyase subunit 3/Multisubunit Na+/H+ antiporter |
| 1137 | 1072619 | 1072963 | 1016759 | 1016415 | 1995 | r-3 | 1072619 | 1072961 | 194 | MnhC | P | Multisubunit Na+/H+ antiporter |

| 1138 | 1072960 | 1073688 | 1016418 | 1015690 | 1279 | r-1 | 1072963 | 1073686 | 333 | MnhB | P | Multisubunit Na+/H+ antiporter |
|------|---------|---------|---------|---------|------|-----|---------|---------|-----|------|---|-------------------------------|
| 1139 | 1073670 | 1073954 | 1015708 | 1015424 | 1667 | r-2 | 1073745 | 1073919 | 68 | - | P | Predicted subunit of the Multisubunit Na+/H+ antiporter |
| 1140 | 1073951 | 1074343 | 1015427 | 1015035 | 1994 | r-3 | 1073951 | 1074290 | 168 | MnhG | P | Multisubunit Na+/H+ antiporter |
| 1141 | 1074340 | 1074594 | 1015038 | 1014784 | 1278 | r-1 | 1074340 | 1074592 | 133 | MnhF | P | Multisubunit Na+/H+ antiporter |
| 1142 | 1074591 | 1075124 | 1014787 | 1014254 | 1666 | r-2 | 1074591 | 1075119 | 258 | MnhE | P | Multisubunit Na+/H+ antiporter |
| 1143 | 1075360 | 1075860 | 1014018 | 1013518 | 1277 | r-1 | 1075360 | 1075858 | 305 | - | E | Predicted regulator of amino acid metabolism (contains the ACT domain) |
| 1144 | 1076013 | 1077278 | 1013365 | 1012100 | 923 | f-3 | 1076019 | 1077276 | 687 | - | R | Predicted Fe-S oxidoreductase |
| 1145 | 1077432 | 1077986 | 1011946 | 1011392 | 924 | f-3 | 1077708 | 1077936 | 32 | RibF | H | FAD synthase |
| 1146 | 1078071 | 1079189 | 1011307 | 1010189 | 1665 | r-2 | 1078071 | 1079187 | 569 | WecB | M | UDP-N-acetylglucosamine 2-epimerase |
| 1147 | 1079201 | 1080472 | 1010177 | 1008906 | 1993 | r-3 | 1079219 | 1080467 | 577 | WecC | M | UDP-N-acetyl-D-mannosaminuro nate dehydrogenase |
| 1148 | 1080723 | 1081862 | 1008655 | 1007516 | 925 | f-3 | 1080759 | 1081797 | 524 | - | S | Uncharacterized ArCR |
| 1149 | 1082285 | 1084639 | 1007093 | 1004739 | 562 | f-2 | 1082735 | 1084637 | 891 | ArgS | J | Arginyl-tRNA synthetase |
| 1150 | 1082363 | 1082779 | 1007015 | 1006599 | 1992 | r-3 | 1082441 | 1082765 | 123 | LplA | H | Lipoate-protein ligase A |
| 1151 | 1084640 | 1085716 | 1004738 | 1003662 | 1991 | r-3 | 1084640 | 1085696 | 377 | - | R | Predicted ATPase of the AAA superfamily |

188

| 1152 | 1085820 | 1086698 | 1003558 | 1002680 | 926 | f-3 | 1085820 | 1086684 | 375 | DapA | E M | Dihydrodipicolinate synthase/N-acetylneuraminate lyase COG0329 DapA |
|---|---|---|---|---|---|---|---|---|---|---|---|---|
| 1153 | 1086762 | 1086986 | 1002616 | 1002392 | 927 | f-3 | 1086765 | 1086870 | 25 | PhrB | L | Deoxyribodipyrimidine photolyase |
| 1154 | 1087256 | 1088512 | 1002122 | 1000866 | 1990 | r-3 | 1087265 | 1088507 | 746 | eRF1 | J | Peptide chain release factor eRF1 |
| 1155 | 1088568 | 1088813 | 1000810 | 1000565 | 1664 | r-2 | | | | | | |
| 1156 | 1088815 | 1089384 | 1000563 | 999994 | 1276 | r-1 | 1089229 | 1089355 | 32 | - | S | Uncharacterized ArCR |
| 1157 | 1089160 | 1089210 | 1000218 | 1000168 | 199 | f-1 | | | | | | |
| 1158 | 1089484 | 1089639 | 999894 | 999739 | 1275 | r-1 | 1089532 | 1089634 | 26 | Fba | G | Fructose/tagatose bisphosphate aldolase (fructose 1,6-bisphophate aldolase) |
| 1159 | 1089909 | 1090604 | 999469 | 998774 | 1663 | r-2 | 1090068 | 1090266 | 37 | BaeS | T | Sensory transduction histidine kinases |
| 1160 | 1091118 | 1091525 | 998260 | 997853 | 1662 | r-2 | 1091292 | 1091415 | 33 | GloB | R | Zn-dependent hydrolases |
| 1161 | 1091646 | 1092197 | 997732 | 997181 | 928 | f-3 | 1091877 | 1092138 | 37 | - | S | Uncharacterized ACR |
| 1162 | 1092206 | 1093522 | 997172 | 995856 | 1989 | r-3 | 1092212 | 1093496 | 443 | - | M | Predicted membrane-associated Zn-dependent proteases 1 |
| 1163 | 1093556 | 1093957 | 995822 | 995421 | 1988 | r-3 | 1093556 | 1093952 | 189 | - | S | Uncharacterized ACR |
| 1164 | 1093967 | 1095127 | 995411 | 994251 | 1987 | r-3 | 1093967 | 1095125 | 593 | - | S | Uncharacterized ACR |
| 1165 | 1096375 | 1096839 | 993003 | 992539 | 200 | f-1 | 1096384 | 1096816 | 242 | RpsO | J | Ribosomal protein S15P/S13E |

| 1166 | 1096870 | 1098303 | 992508 | 991075 | 201 | f-1 | 1096870 | 1098295 | 681 | RecJ | L | Single-stranded DNA-specific exonuclease |
|------|---------|---------|--------|--------|-----|-----|---------|---------|-----|------|---|------|
| 1167 | 1098281 | 1098538 | 991097 | 990840 | 563 | f-2 | 1098317 | 1098458 | 29 | - | C | Phycocyanin alpha-subunit phycocyanobilin lyase and related proteins |
| 1168 | 1098554 | 1099156 | 990824 | 990222 | 564 | f-2 | 1098614 | 1099148 | 310 | RPS1 A | J | Ribosomal protein S3AE |
| 1169 | 1099220 | 1099486 | 990158 | 989892 | 565 | f-2 | 1099274 | 1099469 | 32 | HtpG | O | Molecular chaperone |
| 1170 | 1099468 | 1099908 | 989910 | 989470 | 202 | f-1 | 1099483 | 1099906 | 165 | - | R | Predicted nucleic acid-binding protein |
| 1171 | 1099954 | 1100991 | 989424 | 988387 | 203 | f-1 | 1099954 | 1100962 | 527 | - | S | Uncharacterized protein sharing a conserved domain with thiamine biosynthesis protein ThiI |
| 1172 | 1101073 | 1101510 | 988305 | 987868 | 1274 | r-1 | 1101076 | 1101448 | 136 | - | S | Predicted membrane protein |
| 1173 | 1101868 | 1102326 | 987510 | 987052 | 1273 | r-1 | 1101886 | 1102324 | 133 | Lrp | K | Transcriptional regulators |
| 1174 | 1102786 | 1103181 | 986592 | 986197 | 1272 | r-1 | 1102795 | 1103179 | 136 | ArsR | K | Predicted transcriptional regulators |
| 1175 | 1103673 | 1104461 | 985705 | 984917 | 1661 | r-2 | 1104120 | 1104330 | 31 | - | P | Putative silver efflux pump |
| 1176 | 1104585 | 1106492 | 984793 | 982886 | 929 | f-3 | 1104651 | 1106463 | 742 | LonB | O | Predicted ATP-dependent protease(Lon protease) |

EP 1 923 464 A1

190

| 1177 | 1106686 | 1107264 | 982692 | 982114 | 1271 | r-1 | 1106686 | 1107262 | 272 | - | K | Predicted transcriptional regulator with C-terminal CBS domains |
|---|---|---|---|---|---|---|---|---|---|---|---|---|
| 1178 | 1107524 | 1108015 | 981854 | 981363 | 1986 | r-3 | 1107524 | 1108007 | 160 | RhaT | GE R | Permeases of the drug/metabolite transporter (DMT) superfamily COG0697 RhaT |
| 1179 | 1108559 | 1110253 | 980819 | 979125 | 1985 | r-3 | 1108979 | 1109507 | 38 | - | S | Uncharacterized archaeal coiled-coil domain |
| 1180 | 1110347 | 1111819 | 979031 | 977559 | 566 | f-2 | 1110839 | 1111814 | 442 | - | R | Exopolyphosphatase-related proteins |
| 1181 | 1111862 | 1112080 | 977516 | 977298 | 1984 | r-3 | 1111871 | 1112075 | 97 | - | S | Uncharacterized ACR |
| 1182 | 1112624 | 1113001 | 976754 | 976377 | 1983 | r-3 | 1112627 | 1112996 | 204 | - | K | Transcriptional regulator of a riboflavin/FAD biosynthetic operon |
| 1183 | 1113459 | 1114217 | 975919 | 975161 | 930 | f-3 | 1113468 | 1114212 | 405 | SmtA | Q R | SAM-dependent methyltransferases COG0500 SmtA |
| 1184 | 1114407 | 1117082 | 974971 | 972296 | 931 | f-3 | 1114416 | 1117071 | 1584 | ValS | J | Valyl-tRNA synthetase |
| 1185 | 1117577 | 1118029 | 971801 | 971349 | 567 | f-2 | 1117577 | 1118027 | 289 | RPS19 A | J | Ribosomal protein S19E (S16A) |
| 1186 | 1118086 | 1119738 | 971292 | 969640 | 1270 | r-1 | 1119022 | 1119301 | 33 | - | R | Predicted metal-dependent RNase |
| 1187 | 1119840 | 1120178 | 969538 | 969200 | 932 | f-3 | 1119840 | 1120176 | 182 | - | R | DNA-binding protein |

| 1188 | 1120172 | 1120504 | 969206 | 968874 | 568 | f-2 | 1120172 | 1120442 | 30 | Lig | L | NAD-dependent DNA ligase (contains BRCT domain type II) |
|---|---|---|---|---|---|---|---|---|---|---|---|---|
| 1189 | 1120505 | 1121407 | 968873 | 967971 | 569 | f-2 | 1120514 | 1121402 | 506 | SUA7 | K | Transcription initiation factor IIB |
| 1190 | 1121408 | 1122520 | 967970 | 966858 | 1982 | r-3 | 1121498 | 1122512 | 451 | Exo | L | 5'-3' exonuclease (including N-terminal domain of PolI) |
| 1191 | 1122517 | 1123746 | 966861 | 965632 | 1269 | r-1 | 1122544 | 1123741 | 591 | MoeA | H | Molybdopterin biosynthesis enzyme |
| 1192 | 1123810 | 1124472 | 965568 | 964906 | 204 | f-1 | 1123828 | 1124440 | 299 | - | J | Predicted subunit of tRNA(5-methylaminomethyl-2-th iouridylate) methyltransferase |
| 1193 | 1124569 | 1125114 | 964809 | 964264 | 1268 | r-1 | 1124614 | 1125112 | 284 | ThiJ | R | Putative intracellular protease/amidase |
| 1194 | 1125170 | 1125637 | 964208 | 963741 | 1981 | r-3 | 1125197 | 1125635 | 194 | Lrp | K | Transcriptional regulators |
| 1195 | 1125727 | 1126902 | 963651 | 962476 | 205 | f-1 | 1125736 | 1126900 | 666 | - | R | Predicted GTPase |
| 1196 | 1128262 | 1128495 | 961116 | 960883 | 1267 | r-1 | 1128271 | 1128466 | 102 | Upp | F | Uracil phosphoribosyltransferase |
| 1197 | 1128535 | 1128972 | 960843 | 960406 | 1266 | r-1 | 1128544 | 1128967 | 233 | Upp | F | Uracil phosphoribosyltransferase |
| 1198 | 1129034 | 1130476 | 960344 | 958902 | 1980 | r-3 | 1129043 | 1130459 | 688 | NorM | Q | Na+-driven multidrug efflux pump |
| 1199 | 1130532 | 1131944 | 958846 | 957434 | 1660 | r-2 | 1130547 | 1131936 | 587 | NorM | Q | Na+-driven multidrug efflux |

| | | | | | | | | | | | | pump |
|------|---------|---------|--------|--------|------|-----|---------|---------|------|------|----|----------------------------------------------|
| 1200 | 1132006 | 1132422 | 957372 | 956956 | 1265 | r-1 | 1132006 | 1132420 | 200 | - | R | Predicted nucleic acid-binding protein |
| 1201 | 1132432 | 1132659 | 956946 | 956719 | 1264 | r-1 | 1132438 | 1132630 | 69 | AbrB | K | Regulators of stationary/sporulation gene expression |
| 1202 | 1132744 | 1135125 | 956634 | 954253 | 1263 | r-1 | 1132753 | 1135042 | 1319 | PpsA | G | Phosphoenolpyruvate synthase/pyruvate phosphate dikinase |
| 1203 | 1135154 | 1135213 | 954224 | 954165 | 570 | f-2 | | | | | | |
| 1204 | 1135255 | 1137741 | 954123 | 951637 | 1262 | r-1 | 1136407 | 1136665 | 50 | - | R | Uncharacterized membrane protein |
| 1205 | 1138634 | 1138867 | 950744 | 950511 | 571 | f-2 | | | | | | |
| 1206 | 1139159 | 1142494 | 950219 | 946884 | 572 | f-2 | 1141529 | 1141982 | 35 | SrmB | LK J | Superfamily II DNA and RNA helicases COG0513 SrmB |
| 1207 | 1142537 | 1142836 | 946841 | 946542 | 573 | f-2 | 1142540 | 1142834 | 165 | - | S | Uncharacterized ACR |
| 1208 | 1142873 | 1144054 | 946505 | 945324 | 574 | f-2 | 1142891 | 1144034 | 531 | NMD3 | J | NMD protein affecting ribosome stability and mRNA decay |
| 1209 | 1144054 | 1145121 | 945324 | 944257 | 206 | f-1 | 1144054 | 1145044 | 228 | - | C | Predicted butyrate kinase |
| 1210 | 1145177 | 1146514 | 944201 | 942864 | 575 | f-2 | 1145180 | 1146512 | 743 | HcaD | R | Uncharacterized NAD(FAD)-dependent dehydrogenases |

| 1211 | 1146553 | 1148040 | 942825 | 941338 | 207 | f-1 | 1146592 | 1148029 | 539 | Kch | P | Kef-type K+ transport systems |
|------|---------|---------|--------|--------|-----|-----|---------|---------|-----|------|---|-------------------------------|
| 1212 | 1148086 | 1149231 | 941292 | 940147 | 208 | f-1 | 1148095 | 1149226 | 549 | - | S | Uncharacterized ACR |
| 1213 | 1150093 | 1151094 | 939285 | 938284 | 209 | f-1 | 1150891 | 1151044 | 33 | AsnB | E | Asparagine synthase (glutamine-hydrolyzing) |
| 1214 | 1151091 | 1154534 | 938287 | 934844 | 1659 | r-2 | 1152798 | 1154532 | 958 | InfB | J | Translation initiation factor 2 (GTPase) |
| 1215 | 1155108 | 1155464 | 934270 | 933914 | 933 | f-3 | 1155324 | 1155450 | 29 | NhaB | P | Na+/H+ antiporter |
| 1216 | 1155466 | 1155999 | 933912 | 933379 | 1261 | r-1 | 1155487 | 1155940 | 256 | Ndk | F | Nucleoside diphosphate kinase |
| 1217 | 1157418 | 1157627 | 931960 | 931751 | 1658 | r-2 | 1157424 | 1157625 | 136 | RPL24 A | J | Ribosomal protein L24E |
| 1218 | 1157624 | 1157836 | 931754 | 931542 | 1979 | r-3 | 1157630 | 1157792 | 77 | RPS28 A | J | Ribosomal protein S28E/S33 |
| 1219 | 1157916 | 1158293 | 931462 | 931085 | 1657 | r-2 | 1157922 | 1158291 | 226 | RPL8 A | J | Ribosomal protein HS6-type (S12/L30/L7a) |
| 1220 | 1158361 | 1159554 | 931017 | 929824 | 1260 | r-1 | 1158373 | 1159537 | 321 | RAD5 5 | T | RecA-superfamily ATPases implicated in signal transduction |
| 1221 | 1159686 | 1160306 | 929692 | 929072 | 1656 | r-2 | 1159695 | 1160295 | 277 | - | S | Uncharacterized archaeal Zn-finger family |
| 1222 | 1161299 | 1161634 | 928079 | 927744 | 1978 | r-3 | 1161314 | 1161596 | 128 | - | R | Uncharacterized ATPases of the AAA superfamily |

194

| | | | | | | | | | | | EH | |
|---|---|---|---|---|---|---|---|---|---|---|---|---|
| 1223 | 1161690 | 1163606 | 927688 | 925772 | 1655 | r-2 | 1162347 | 1163139 | 448 | CysH | O | 3'-phosphoadenosine 5'-phosphosulfate sulfotransferase (PAPS reductase)/FAD synthetase and related enzymes COG0175 CysH |
| 1224 | 1163703 | 1164656 | 925675 | 924722 | 934 | f-3 | 1163775 | 1164561 | 466 | HflC | O | Membrane protease subunits |
| 1225 | 1164663 | 1165082 | 924715 | 924296 | 935 | f-3 | 1164663 | 1165077 | 148 | - | N O | Membrane protein implicated in regulation of membrane protease activity COG1585 - |
| 1226 | 1165121 | 1165714 | 924257 | 923664 | 576 | f-2 | 1165130 | 1165706 | 202 | Tdk | F | Thymidine kinase |
| 1227 | 1165724 | 1165948 | 923654 | 923430 | 577 | f-2 | 1165793 | 1165946 | 81 | RPL39 | J | Ribosomal protein L39E |
| 1228 | 1165959 | 1166231 | 923419 | 923147 | 936 | f-3 | 1165959 | 1166217 | 136 | RPL31A | J | Ribosomal protein L31E |
| 1229 | 1166259 | 1166948 | 923119 | 922430 | 937 | f-3 | 1166259 | 1166943 | 329 | TIF6 | J | Eukaryotic translation initiation factor 6 (EIF6) |
| 1230 | 1167001 | 1167234 | 922377 | 922144 | 210 | f-1 | 1167001 | 1167232 | 91 | RPL20A | J | Ribosomal protein L20A (L18A) |
| 1231 | 1167503 | 1168657 | 921875 | 920721 | 1977 | r-3 | 1167503 | 1168655 | 468 | RfaG | M | Predicted glycosyltransferases |
| 1232 | 1168678 | 1169472 | 920700 | 919906 | 1259 | r-1 | 1168747 | 1169299 | 87 | UbiA | H | 4-hydroxybenzoate polyprenyltransferase |

| 1233 | 1169576 | 1171024 | 919802 | 918354 | 1976 | r-3 | 1169591 | 1170995 | 718 | GltD | ER | NADPH-dependent glutamate synthase beta chain and related oxidoreductases COG0493 GltD |
|------|---------|---------|--------|--------|------|-----|---------|---------|-----|------|------|---|
| 1234 | 1171021 | 1171905 | 918357 | 917473 | 1258 | r-1 | 1171021 | 1171894 | 441 | UbiB | H C | 2-polyprenylphenol hydroxylase and related flavodoxin oxidoreductases~ COG0543 UbiB |
| 1235 | 1172047 | 1172277 | 917331 | 917101 | 211 | f-1 | 1172059 | 1172224 | 35 | PotE | E | Amino acid transporters |
| 1236 | 1172264 | 1173025 | 917114 | 916353 | 1975 | r-3 | 1172264 | 1173023 | 330 | GCD14 | J | Predicted SAM-dependent methyltransferase involved in tRNA-Met maturation |
| 1237 | 1173022 | 1173636 | 916356 | 915742 | 1257 | r-1 | 1173112 | 1173265 | 32 | NemA | C | NADH:flavin oxidoreductases |
| 1238 | 1173687 | 1174022 | 915691 | 915356 | 938 | f-3 | 1173699 | 1173975 | 120 | SEC65 | N | Signal recognition particle 19 kDa protein |
| 1239 | 1174023 | 1174274 | 915355 | 915104 | 1654 | r-2 | 1174041 | 1174227 | 47 | Lrp | K | Transcriptional regulators |
| 1240 | 1174284 | 1174388 | 915094 | 914990 | 1653 | r-2 | | | | | | |
| 1241 | 1174493 | 1177870 | 914885 | 911508 | 578 | f-2 | 1174493 | 1175486 | 467 | - | R | Predicted helicases |
| 1242 | 1178296 | 1178862 | 911082 | 910516 | 212 | f-1 | 1178305 | 1178854 | 198 | CoaE | H | Dephospho-CoA kinase |
| 1243 | 1178840 | 1179322 | 910538 | 910056 | 579 | f-2 | 1178906 | 1179320 | 232 | - | S | Uncharacterized ArCR |
| 1244 | 1179335 | 1180606 | 910043 | 908772 | 1974 | r-3 | 1179335 | 1180583 | 409 | NatB | C | ABC-type Na+ efflux pump |
| 1245 | 1180603 | 1181361 | 908775 | 908017 | 1256 | r-1 | 1180609 | 1181317 | 376 | CcmA | Q | ABC-type multidrug transport |

| | | | | | | | | | | | |
|---|---|---|---|---|---|---|---|---|---|---|---|
| | | | | | | | | | | | system |
| 1246 | 1181719 | 1181916 | 907659 | 907462 | 1255 | r-1 | 1181776 | 1181914 | 82 | - | K | Predicted transcriptional regulators |
| 1247 | 1182281 | 1182673 | 907097 | 906705 | 1973 | r-3 | 1182308 | 1182527 | 32 | CbiM | H | Cobalamin biosynthesis protein CbiM |
| 1248 | 1182899 | 1183855 | 906479 | 905523 | 580 | f-2 | 1183346 | 1183523 | 33 | - | S | Uncharacterized BCR |
| 1249 | 1184435 | 1184731 | 904943 | 904647 | 1972 | r-3 | 1184531 | 1184717 | 29 | InfB | J | Translation initiation factor 2 (GTPase) |
| 1250 | 1184832 | 1185752 | 904546 | 903626 | 1652 | r-2 | 1185366 | 1185510 | 32 | MurG | M | UDP-N-acetylglucosamine:LPS N-acetylglucosamine transferase |
| 1251 | 1186264 | 1186524 | 903114 | 902854 | 1254 | r-1 | | | | | | |
| 1252 | 1187372 | 1187653 | 902006 | 901725 | 1971 | r-3 | | | | | | |
| 1253 | 1188250 | 1188906 | 901128 | 900472 | 1253 | r-1 | 1188304 | 1188649 | 36 | GyrA | L | DNA gyrase (topoisomerase II) A subunit |
| 1254 | 1188962 | 1189906 | 900416 | 899472 | 1970 | r-3 | 1188983 | 1189385 | 35 | GcvP | E | Glycine cleavage system protein P (pyridoxal-binding) |
| 1255 | 1189940 | 1190062 | 899438 | 899316 | 1969 | r-3 | 1190009 | 1190057 | 26 | MalF | G | ABC-type sugar transport systems |
| 1256 | 1191309 | 1191941 | 898069 | 897437 | 1651 | r-2 | 1191474 | 1191585 | 29 | MalK | G | ABC-type sugar/spermidine/putrescine/iron/thiamine transport systems |
| 1257 | 1195773 | 1195841 | 893605 | 893537 | 939 | f-3 | | | | | | |

| 1258 | 1196421 | 1196939 | 892957 | 892439 | 1650 | r-2 | 1196724 | 1196871 | 33 | Gmk | F | Guanylate kinase |
|------|---------|---------|--------|--------|------|-----|---------|---------|-----|------|---|-------------------|
| 1259 | 1197121 | 1197330 | 892257 | 892048 | 1252 | r-1 | 1197211 | 1197322 | 30 | FecB | P | ABC-type Fe3+-siderophores transport systems |
| 1260 | 1197327 | 1197827 | 892051 | 891551 | 1649 | r-2 | 1197588 | 1197801 | 31 | UvrA | L | Excinuclease ATPase subunit |
| 1261 | 1197859 | 1198116 | 891519 | 891262 | 1251 | r-1 | 1197958 | 1198078 | 26 | - | T | SH3 domain protein |
| 1262 | 1198129 | 1198395 | 891249 | 890983 | 1250 | r-1 | 1198141 | 1198300 | 30 | AlkA | L | 3-Methyladenine DNA glycosylase |
| 1263 | 1198775 | 1198969 | 890603 | 890409 | 581 | f-2 | 1198808 | 1198907 | 33 | AbrB | K | Regulators of stationary/sporulation gene expression |
| 1264 | 1199210 | 1199536 | 890168 | 889842 | 1968 | r-3 | 1199303 | 1199522 | 31 | Smc | D | Chromosome segregation ATPases |
| 1265 | 1200465 | 1200542 | 888913 | 888836 | 940 | f-3 | | | | | | |
| 1266 | 1202741 | 1204258 | 886637 | 885120 | 1967 | r-3 | 1202750 | 1204256 | 910 | GcvP | E | Glycine cleavage system protein P (pyridoxal-binding) |
| 1267 | 1204260 | 1205624 | 885118 | 883754 | 1648 | r-2 | 1204269 | 1205598 | 727 | GcvP | E | Glycine cleavage system protein P (pyridoxal-binding) |
| 1268 | 1205780 | 1207075 | 883598 | 882303 | 1966 | r-3 | 1206086 | 1206206 | 32 | FliI | N | Flagellar biosynthesis/type III secretory pathway ATPase |

| 1269 | 1207362 | 1207793 | 882016 | 881585 | 941 | f-3 | 1207452 | 1207662 | 32 | PorG | C | Pyruvate:ferredoxin oxidoreductase and related 2-oxoacid:ferredoxin oxidoreductases |
|---|---|---|---|---|---|---|---|---|---|---|---|---|
| 1270 | 1207790 | 1208482 | 881588 | 880896 | 582 | f-2 | 1207790 | 1208444 | 312 | - | R | Predicted hydrolases of the HAD superfamily |
| 1271 | 1209464 | 1210141 | 879914 | 879237 | 583 | f-2 | 1209512 | 1210130 | 239 | - | R | Predicted ICC-like phosphoesterases |
| 1272 | 1210174 | 1210893 | 879204 | 878485 | 213 | f-1 | 1210189 | 1210885 | 275 | - | S | Uncharacterized membrane protein |
| 1273 | 1210890 | 1211111 | 878488 | 878267 | 942 | f-3 | 1210890 | 1211058 | 33 | Smc | D | Chromosome segregation ATPases |
| 1274 | 1211128 | 1211787 | 878250 | 877591 | 214 | f-1 | 1211251 | 1211392 | 33 | XerC | L | Integrase |
| 1275 | 1211850 | 1212755 | 877528 | 876623 | 943 | f-3 | 1211949 | 1212030 | 33 | FecB | P | ABC-type Fe3+-siderophores transport systems |
| 1276 | 1212760 | 1213104 | 876618 | 876274 | 1249 | r-1 | 1212775 | 1212850 | 36 | NuoG | C | NADH dehydrogenase/NADH:ubiquinon e oxidoreductase 75 kD subunit (chain G) |
| 1277 | 1213101 | 1214369 | 876277 | 875009 | 1647 | r-2 | 1213137 | 1214364 | 572 | HcaD | R | Uncharacterized NAD(FAD)-dependent dehydrogenases |

| 1278 | 1214366 | 1215214 | 875012 | 874164 | 1965 | r-3 | 1214366 | 1215206 | 475 | - | | R | Predicted dehydrogenase |
|---|---|---|---|---|---|---|---|---|---|---|---|---|---|
| 1279 | 1215250 | 1215861 | 874128 | 873517 | 1248 | r-1 | 1215259 | 1215793 | 272 | - | | R | Predicted dehydrogenase |
| 1280 | 1217374 | 1217490 | 872004 | 871888 | 215 | f-1 | 1217374 | 1217461 | 26 | - | | R | CBS domains |
| 1281 | 1219074 | 1219190 | 870304 | 870188 | 944 | f-3 | | | | | | | |
| 1282 | 1219197 | 1220690 | 870181 | 868688 | 1646 | r-2 | 1219197 | 1220676 | 790 | GlpK | | C | Glycerol kinase |
| 1283 | 1220740 | 1221513 | 868638 | 867865 | 1247 | r-1 | 1220767 | 1221511 | 387 | UgpQ | | C | Glycerophosphoryl diester phosphodiesterase |
| 1284 | 1221503 | 1222201 | 867875 | 867177 | 1964 | r-3 | 1221509 | 1222124 | 92 | UgpQ | | C | Glycerophosphoryl diester phosphodiesterase |
| 1285 | 1222282 | 1223655 | 867096 | 865723 | 216 | f-1 | 1222297 | 1223653 | 582 | | | | |
| 1286 | 1223758 | 1225113 | 865620 | 864265 | 217 | f-1 | 1223821 | 1225096 | 605 | | | | |
| 1287 | 1225113 | 1225991 | 864265 | 863387 | 945 | f-3 | 1225179 | 1225965 | 379 | - | | R | Hydrolases of the alpha/beta superfamily |
| 1288 | 1226169 | 1226861 | 863209 | 862517 | 946 | f-3 | 1226217 | 1226835 | 187 | - | | R | Predicted deacetylase |
| 1289 | 1227076 | 1227702 | 862302 | 861676 | 1246 | r-1 | 1227088 | 1227691 | 290 | Tmk | | F | Thymidylate kinase |
| 1290 | 1227756 | 1228466 | 861622 | 860912 | 1645 | r-2 | 1227756 | 1228449 | 365 | CpsG | | G | Phosphomannomutase |
| 1291 | 1228622 | 1230493 | 860756 | 858885 | 584 | f-2 | 1228631 | 1230482 | 1088 | PckA | | C | Phosphoenolpyruvate carboxykinase (GTP) |
| 1292 | 1230580 | 1233081 | 858798 | 856297 | 218 | f-1 | 1230592 | 1233058 | 1177 | GlgP | | G | Glucan phosphorylase |
| 1293 | 1233236 | 1234546 | 856142 | 854832 | 585 | f-2 | 1233818 | 1234340 | 44 | - | | R | Na+-dependent transporters of the SNF family |

| 1294 | 1234563 | 1236284 | 854815 | 853094 | 1644 | r-2 | 1234569 | 1236282 | 931 | GlnS | J | Glutamyl- and glutaminyl-tRNA synthetases |
|---|---|---|---|---|---|---|---|---|---|---|---|---|
| 1295 | 1236584 | 1237978 | 852794 | 851400 | 1963 | r-3 | 1236584 | 1237964 | 630 | DnaG | L | DNA primase (bacterial type) |
| 1296 | 1237975 | 1238376 | 851403 | 851002 | 1245 | r-1 | 1237975 | 1238371 | 177 | - | L | Small primase-like proteins (Toprim domain) |
| 1297 | 1238433 | 1239707 | 850945 | 849671 | 1643 | r-2 | 1238439 | 1239702 | 677 | - | C | Fe-S oxidoreductases family 2 |
| 1298 | 1239791 | 1239994 | 849587 | 849384 | 1962 | r-3 | 1239791 | 1239992 | 92 | HHT1 | L | Histones H3 and H4 (Histon A&B) |
| 1299 | 1240125 | 1240214 | 849253 | 849164 | 947 | f-3 | | | | | | |
| 1300 | 1240801 | 1240896 | 848577 | 848482 | 1244 | r-1 | | | | | | |
| 1301 | 1241592 | 1241921 | 847786 | 847457 | 1642 | r-2 | 1241601 | 1241769 | 98 | RPP1A | J | Ribosomal protein L12E/L44/L45/RPP1/RPP2 |
| 1302 | 1241983 | 1243014 | 847395 | 846364 | 1243 | r-1 | 1241992 | 1243009 | 402 | RplJ | J | Ribosomal protein L10 |
| 1303 | 1243011 | 1243661 | 846367 | 845717 | 1641 | r-2 | 1243011 | 1243656 | 327 | RplA | J | Ribosomal protein L1 |
| 1304 | 1243692 | 1243778 | 845686 | 845600 | 1640 | r-2 | | | | | | |
| 1305 | 1243775 | 1244272 | 845603 | 845106 | 1961 | r-3 | 1243781 | 1244264 | 223 | RplK | J | Ribosomal protein L11 |
| 1306 | 1244307 | 1244765 | 845071 | 844613 | 1639 | r-2 | 1244316 | 1244763 | 257 | NusG | K | Transcription antiterminator |
| 1307 | 1244788 | 1244973 | 844590 | 844405 | 1242 | r-1 | 1244788 | 1244893 | 49 | Sss1 | N | Protein translocase subunit Sss1 |
| 1308 | 1245004 | 1246125 | 844374 | 843253 | 1241 | r-1 | 1245004 | 1246123 | 536 | FtsZ | D | Cell division GTPase |
| 1309 | 1246241 | 1247059 | 843137 | 842319 | 1960 | r-3 | 1246241 | 1247057 | 446 | - | S | Uncharacterized ArCR |
| 1310 | 1247369 | 1248709 | 842009 | 840669 | 1959 | r-3 | 1247567 | 1248584 | 105 | FucI | G | L-fucose isomerase and related |

| | | | | | | | | | | | |
|---|---|---|---|---|---|---|---|---|---|---|---|
| | | | | | | | | | | | proteins |
| 1311 | 1248621 | 1249226 | 840757 | 840152 | 948 | f-3 | 1248630 | 1249179 | 314 | - | S | Uncharacterized ACR |
| 1312 | 1250499 | 1251188 | 838879 | 838190 | 1638 | r-2 | 1250499 | 1251186 | 333 | RpiA | G | Ribose 5-phosphate isomerase |
| 1313 | 1251193 | 1251561 | 838185 | 837817 | 1240 | r-1 | 1251223 | 1251379 | 29 | SUL1 | P | Sulfate permease and related transporters (MFS superfamily) |
| 1314 | 1251632 | 1253578 | 837746 | 835800 | 1958 | r-3 | 1251632 | 1253576 | 1146 | - | R | Predicted metal-dependent RNase |
| 1315 | 1253588 | 1253788 | 835790 | 835590 | 1957 | r-3 | 1253588 | 1253750 | 74 | HslV | O | Proteasome protease subunit |
| 1316 | 1254304 | 1255470 | 835074 | 833908 | 219 | f-1 | 1254304 | 1254742 | 37 | AdkA | F | Archaeal adenylate kinase |
| 1317 | 1255582 | 1256436 | 833796 | 832942 | 1239 | r-1 | 1255594 | 1256431 | 481 | GdhA | E | Glutamate dehydrogenase/leucine dehydrogenase |
| 1318 | 1256379 | 1256846 | 832999 | 832532 | 1637 | r-2 | 1256379 | 1256808 | 256 | GdhA | E | Glutamate dehydrogenase/leucine dehydrogenase |
| 1319 | 1257402 | 1258961 | 831976 | 830417 | 949 | f-3 | 1257411 | 1258956 | 828 | - | R | Na+-dependent transporters of the SNF family |
| 1320 | 1258972 | 1259079 | 830406 | 830299 | 220 | f-1 | 1258972 | 1259038 | 26 | SurA | O | Parvulin-like peptidyl-prolyl isomerase |
| 1321 | 1259124 | 1259858 | 830254 | 829520 | 950 | f-3 | 1259490 | 1259712 | 33 | SgaT | S | Uncharacterized BCR |
| 1322 | 1259855 | 1260172 | 829523 | 829206 | 1956 | r-3 | 1259855 | 1260143 | 100 | GlnK | E | Nitrogen regulatory protein PII |
| 1323 | 1260229 | 1262256 | 829149 | 827122 | 1238 | r-1 | 1260229 | 1261816 | 720 | - | S | Uncharacterized ACR |
| 1324 | 1262388 | 1262651 | 826990 | 826727 | 951 | f-3 | | | | | | |

| 1325 | 1262709 | 1264661 | 826669 | 824717 | 952 | f-3 | 1262709 | 1264623 | 880 | - | K | Predicted RNA-binding protein homologous to eukaryotic snRNP |
|------|---------|---------|--------|--------|-----|-----|---------|---------|-----|---|---|------|
| 1326 | 1264658 | 1265074 | 824720 | 824304 | 1955 | r-3 | 1264658 | 1265072 | 231 | NikR | K | Predicted transcriptional regulators containing the CopG/Arc/MetJ DNA-binding domain and a metal-binding domain |
| 1327 | 1265145 | 1265591 | 824233 | 823787 | 953 | f-3 | 1265307 | 1265409 | 29 | HsdR | L | Restriction enzymes type I helicase subunits and related helicases |
| 1328 | 1265593 | 1266390 | 823785 | 822988 | 221 | f-1 | 1266082 | 1266259 | 31 | UgpB | G | Sugar-binding periplasmic proteins/domains |
| 1329 | 1266750 | 1267955 | 822628 | 821423 | 954 | f-3 | 1266750 | 1267941 | 638 | - | R | Predicted alternative tryptophan synthase beta-subunit (paralog of TrpB) |
| 1330 | 1268130 | 1269137 | 821248 | 820241 | 1636 | r-2 | 1268130 | 1269132 | 523 | Asd | E | Aspartate-semialdehyde dehydrogenase |
| 1331 | 1269155 | 1270042 | 820223 | 819336 | 1954 | r-3 | 1269167 | 1270037 | 312 | ThrB | E | Homoserine kinase |
| 1332 | 1270062 | 1271162 | 819316 | 818216 | 1635 | r-2 | 1270083 | 1271085 | 242 | LysC | E | Aspartokinases |
| 1333 | 1271162 | 1272181 | 818216 | 817197 | 1953 | r-3 | 1271171 | 1272170 | 567 | MetE | E | Methionine synthase II (cobalamin-independent) |

| 1334 | 1272174 | 1273103 | 817204 | 816275 | 1634 | r-2 | 1272174 | 1273068 | 462 | MetE | E | Methionine synthase II (cobalamin-independent) |
|------|----------|----------|--------|--------|------|-----|----------|----------|-----|------|---|--------|
| 1335 | 1273100 | 1274158 | 816278 | 815220 | 1952 | r-3 | 1273109 | 1274144 | 296 | MetF | E | 5 |
| 1336 | 1274151 | 1275281 | 815227 | 814097 | 1633 | r-2 | 1274154 | 1275270 | 484 | MetC | E | Cystathionine beta-lyases/cystathionine gamma-synthases |
| 1337 | 1275461 | 1276135 | 813917 | 813243 | 1951 | r-3 | 1275509 | 1276133 | 239 | - | J | Ribonuclease P subunit Rpp30 |
| 1338 | 1276120 | 1276689 | 813258 | 812689 | 1237 | r-1 | 1276240 | 1276684 | 210 | - | S | Uncharacterized ArCR |
| 1339 | 1276727 | 1278301 | 812651 | 811077 | 1950 | r-3 | 1276892 | 1278245 | 140 | MdlB | Q | ABC-type multidrug/protein/lipid transport system |
| 1340 | 1278636 | 1279535 | 810742 | 809843 | 1632 | r-2 | 1279008 | 1279143 | 32 | LivG | E | ABC-type branched-chain amino acid transport systems |
| 1341 | 1279958 | 1280587 | 809420 | 808791 | 1949 | r-3 | 1279958 | 1280585 | 320 | RPL15A | J | Ribosomal protein L15E |
| 1342 | 1280661 | 1281740 | 808717 | 807638 | 955 | f-3 | 1280670 | 1281729 | 544 | PepP | E | Xaa-Pro aminopeptidase |
| 1343 | 1281804 | 1282397 | 807574 | 806981 | 1631 | r-2 | 1281804 | 1282356 | 295 | - | S | Uncharacterized ACR |
| 1344 | 1282384 | 1283034 | 806994 | 806344 | 1236 | r-1 | 1282417 | 1283032 | 320 | - | S | Uncharacterized ACR |
| 1345 | 1283055 | 1284251 | 806323 | 805127 | 1630 | r-2 | 1283205 | 1284249 | 291 | - | P | Permease |
| 1346 | 1284667 | 1285869 | 804711 | 803509 | 222 | f-1 | 1285024 | 1285702 | 45 | - | S | Uncharacterized archaeal coiled-coil domain |
| 1347 | 1285975 | 1289823 | 803403 | 799555 | 223 | f-1 | 1288144 | 1289155 | 166 | - | L | Type II restriction enzyme |

| 1348 | 1290019 | 1292922 | 799359 | 796456 | 224 | f-1 | 1290019 | 1292920 | 1723 | LeuS | J | Leucyl-tRNA synthetase |
|---|---|---|---|---|---|---|---|---|---|---|---|---|
| 1349 | 1293396 | 1293860 | 795982 | 795518 | 1629 | r-2 | 1293606 | 1293774 | 30 | ErfK | S | Uncharacterized BCR |
| 1350 | 1294892 | 1295722 | 794486 | 793656 | 586 | f-2 | 1295033 | 1295336 | 44 | SmtA | Q R | SAM-dependent methyltransferases COG0500 SmtA |
| 1351 | 1295748 | 1297115 | 793630 | 792263 | 956 | f-3 | 1295760 | 1297065 | 379 | - | R | Predicted ATPase of the AAA superfamily |
| 1352 | 1297116 | 1298444 | 792262 | 790934 | 1628 | r-2 | 1297161 | 1298433 | 640 | ArgE | E | Acetylornithine deacetylase/Succinyl-diaminopimelate desuccinylase and related deacylases |
| 1353 | 1298625 | 1298846 | 790753 | 790532 | 957 | f-3 | 1298646 | 1298805 | 27 | PanC | H | Panthothenate synthetase |
| 1354 | 1299189 | 1300220 | 790189 | 789158 | 1627 | r-2 | 1299189 | 1300218 | 487 | IspA | H | Geranylgeranyl pyrophosphate synthase |
| 1355 | 1300290 | 1301624 | 789088 | 787754 | 1626 | r-2 | 1300290 | 1301619 | 738 | - | R | Predicted hydrolase of the metallo-beta-lactamase superfamily |
| 1356 | 1301759 | 1302934 | 787619 | 786444 | 1948 | r-3 | 1301825 | 1302926 | 586 | LldD | C | L-lactate dehydrogenase (FMN-dependent) and related alpha-hydroxy acid dehydrogenases |
| 1357 | 1302931 | 1303617 | 786447 | 785761 | 1235 | r-1 | 1302940 | 1303612 | 268 | RacX | M | Aspartate racemase |

| 1358 | 1303690 | 1304454 | 785688 | 784924 | 1234 | r-1 | 1303699 | 1304449 | 388 | CinA | R | Predicted nucleotide-utilizing enzyme related to molybdopterin-biosynthesis enzyme MoeA |
|---|---|---|---|---|---|---|---|---|---|---|---|---|
| 1359 | 1304451 | 1305239 | 784927 | 784139 | 1625 | r-2 | 1304451 | 1305222 | 243 | - | R | Predicted archaeal kinases |
| 1360 | 1305236 | 1306249 | 784142 | 783129 | 1947 | r-3 | 1305251 | 1306247 | 484 | ERG1 2 | I | Mevalonate kinase |
| 1361 | 1306246 | 1306722 | 783132 | 782656 | 1233 | r-1 | 1306312 | 1306711 | 150 | - | S | Uncharacterized ACR |
| 1362 | 1306665 | 1307039 | 782713 | 782339 | 1624 | r-2 | 1306704 | 1307028 | 107 | - | R | Predicted nucleotidyltransferases |
| 1363 | 1307076 | 1307963 | 782302 | 781415 | 1623 | r-2 | 1307088 | 1307961 | 485 | - | R | Predicted dioxygenase |
| 1364 | 1307989 | 1309053 | 781389 | 780325 | 1232 | r-1 | 1307989 | 1309027 | 408 | ThrC | E | Threonine synthase |
| 1365 | 1309106 | 1309948 | 780272 | 779430 | 587 | f-2 | 1309133 | 1309940 | 284 | Udp | F | Uridine phosphorylase |
| 1366 | 1309950 | 1311020 | 779428 | 778358 | 958 | f-3 | 1310643 | 1311006 | 36 | - | S | Uncharacterized archaeal coiled-coil domain |
| 1367 | 1311965 | 1313317 | 777413 | 776061 | 1946 | r-3 | 1311974 | 1313285 | 489 | HcaD | R | Uncharacterized NAD(FAD)-dependent dehydrogenases |
| 1368 | 1313412 | 1314224 | 775966 | 775154 | 1622 | r-2 | 1313421 | 1314216 | 415 | Pnp | F | Purine nucleoside phosphorylase |
| 1369 | 1315661 | 1315879 | 773717 | 773499 | 1945 | r-3 | 1315679 | 1315763 | 29 | PrmA | J | Ribosomal protein L11 methylase |
| 1370 | 1316041 | 1316151 | 773337 | 773227 | 1231 | r-1 | | | | | | |
| 1371 | 1316410 | 1317765 | 772968 | 771613 | 225 | f-1 | 1316419 | 1317742 | 693 | Ffh | N | Signal recognition particle |

206

| | | | | | | | | | | | | GTPase |
|---|---|---|---|---|---|---|---|---|---|---|---|---|
| /2 | 1317762 | 1318001 | 771616 | 771377 | 959 | f-3 | 1317765 | 1317993 | 95 | Lrp | K | Transcriptional regulators |
| 1373 | 1317998 | 1318528 | 771380 | 770850 | 588 | f-2 | 1318004 | 1318424 | 189 | - | R | Predicted Fe-S-cluster oxidoreductase |
| 1374 | 1318585 | 1319298 | 770793 | 770080 | 226 | f-1 | 1318585 | 1319296 | 316 | Apt | F | Adenine/guanine phosphoribosyltransferases and related PRPP-binding proteins |
| 1375 | 1319308 | 1319637 | 770070 | 769741 | 227 | f-1 | 1319491 | 1319608 | 29 | SrmB | LK J | Superfamily II DNA and RNA helicases COG0513 SrmB |
| 1376 | 1319620 | 1320078 | 769758 | 769300 | 1230 | r-1 | 1319629 | 1320064 | 179 | Lrp | K | Transcriptional regulators |
| 1377 | 1321326 | 1322096 | 768052 | 767282 | 960 | f-3 | 1321335 | 1322010 | 346 | ApaH | T | Diadenosine tetraphosphatase and related serine/threonine protein phosphatases |
| 1378 | 1322102 | 1322401 | 767276 | 766977 | 1944 | r-3 | 1322102 | 1322399 | 150 | - | S | Uncharacterized ACR |
| 1379 | 1322840 | 1323004 | 766538 | 766374 | 1943 | r-3 | 1322849 | 1323002 | 105 | RPL40 A | J | Ribosomal protein L40E |
| 1380 | 1323183 | 1323788 | 766195 | 765590 | 1621 | r-2 | 1323186 | 1323783 | 368 | RpsB | J | Ribosomal protein S2 |
| 1381 | 1323802 | 1324827 | 765576 | 764551 | 1229 | r-1 | 1323802 | 1324822 | 474 | Eno | G | Enolase |
| 1382 | 1325139 | 1325336 | 764239 | 764042 | 1620 | r-2 | 1325139 | 1325334 | 122 | RPB10 | K | DNA-directed RNA polymerase |
| 1383 | 1325369 | 1325800 | 764009 | 763578 | 1942 | r-3 | 1325393 | 1325798 | 217 | RpsI | J | Ribosomal protein S9 |
| 1384 | 1325787 | 1326215 | 763591 | 763163 | 1619 | r-2 | 1325787 | 1326213 | 254 | RplM | J | Ribosomal protein L13 |

| 1385 | 1326222 | 1326593 | 763156 | 762785 | 1618 | r-2 | 1326231 | 1326591 | 187 | RPL18A | J | Ribosomal protein L18E |
|---|---|---|---|---|---|---|---|---|---|---|---|---|
| 1386 | 1326738 | 1327526 | 762640 | 761852 | 1617 | r-2 | 1326747 | 1327521 | 411 | RpoA | K | DNA-directed RNA polymerase alpha subunit/40 kD subunit |
| 1387 | 1327548 | 1327970 | 761830 | 761408 | 1616 | r-2 | 1327548 | 1327944 | 188 | RpsK | J | Ribosomal protein S11 |
| 1388 | 1327967 | 1328509 | 761411 | 760869 | 1941 | r-3 | 1327967 | 1328507 | 239 | RpsD | J | Ribosomal protein S4 and related proteins |
| 1389 | 1328520 | 1329077 | 760858 | 760301 | 1615 | r-2 | 1328637 | 1329075 | 235 | RpsM | J | Ribosomal protein S13 |
| 1390 | 1329084 | 1329671 | 760294 | 759707 | 1614 | r-2 | 1329084 | 1329669 | 327 | RsmC | J | 16S RNA G1207 methylase RsmC |
| 1391 | 1330058 | 1330213 | 759320 | 759165 | 589 | f-2 | | | | | | |
| 1392 | 1330540 | 1331565 | 758838 | 757813 | 1228 | r-1 | 1330549 | 1331551 | 632 | TruB | J | Pseudouridine synthase |
| 1393 | 1331777 | 1332007 | 757601 | 757371 | 1940 | r-3 | 1331810 | 1331987 | 40 | - | S | Uncharacterized ACR |
| 1394 | 1332043 | 1332753 | 757335 | 756625 | 1227 | r-1 | 1332094 | 1332751 | 201 | FabG | Q R | Dehydrogenases with different specificities (related to short-chain alcohol dehydrogenases) COG1028 FabG |
| 1395 | 1332861 | 1333112 | 756517 | 756266 | 1613 | r-2 | 1332861 | 1333107 | 142 | RPL14A | J | Ribosomal protein L14E |
| 1396 | 1333113 | 1333694 | 756265 | 755684 | 1612 | r-2 | 1333113 | 1333644 | 327 | Cmk | F | Cytidylate kinase 2 |
| 1397 | 1333706 | 1333999 | 755672 | 755379 | 1939 | r-3 | 1333727 | 1333991 | 175 | RPL34A | J | Ribosomal protein L34E |

208

| | | | | | | | | | | | |
|---|---|---|---|---|---|---|---|---|---|---|---|
| 1398 | 1334020 | 1334550 | 755358 | 754828 | 1226 | r-1 | 1334026 | 1334542 | 194 | - | S | Uncharacterized membrane protein |
| 1399 | 1334537 | 1335136 | 754841 | 754242 | 1938 | r-3 | 1334546 | 1335134 | 290 | AdkA | F | Archaeal adenylate kinase |
| 1400 | 1335210 | 1336667 | 754168 | 752711 | 1611 | r-2 | 1335219 | 1336659 | 665 | SecY | N | Preprotein translocase subunit SecY |
| 1401 | 1336699 | 1337145 | 752679 | 752233 | 1225 | r-1 | 1336699 | 1337143 | 155 | RplO | J | Ribosomal protein L15 |
| 1402 | 1337157 | 1337624 | 752221 | 751754 | 1610 | r-2 | 1337157 | 1337622 | 269 | RpmD | J | Ribosomal protein L30/L7E |
| 1403 | 1337636 | 1338343 | 751742 | 751035 | 1937 | r-3 | 1337648 | 1338341 | 426 | RpsE | J | Ribosomal protein S5 |
| 1404 | 1338340 | 1338954 | 751038 | 750424 | 1224 | r-1 | 1338340 | 1338946 | 302 | RplR | J | Ribosomal protein L18 |
| 1405 | 1338956 | 1339411 | 750422 | 749967 | 1936 | r-3 | 1338959 | 1339409 | 213 | RPL19 A | J | Ribosomal protein L19E |
| 1406 | 1339413 | 1339793 | 749965 | 749585 | 1609 | r-2 | 1339473 | 1339791 | 194 | RPL32 | J | Ribosomal protein L32E |
| 1407 | 1339810 | 1340373 | 749568 | 749005 | 1223 | r-1 | 1339810 | 1340371 | 302 | RplF | J | Ribosomal protein L6 |
| 1408 | 1340375 | 1340767 | 749003 | 748611 | 1935 | r-3 | 1340375 | 1340765 | 243 | RpsH | J | Ribosomal protein S8 |
| 1409 | 1340779 | 1340949 | 748599 | 748429 | 1222 | r-1 | 1340779 | 1340947 | 122 | RpsN | J | Ribosomal protein S14 |
| 1410 | 1340951 | 1341502 | 748427 | 747876 | 1934 | r-3 | 1340960 | 1341491 | 307 | RplE | J | Ribosomal protein L5 |
| 1411 | 1341516 | 1342247 | 747862 | 747131 | 1608 | r-2 | 1341516 | 1342245 | 444 | RPS4 A | J | Ribosomal protein S4E |
| 1412 | 1342247 | 1342612 | 747131 | 746766 | 1933 | r-3 | 1342247 | 1342574 | 189 | RplX | J | Ribosomal protein L24 |
| 1413 | 1342624 | 1343049 | 746754 | 746329 | 1221 | r-1 | 1342624 | 1343047 | 203 | RplN | J | Ribosomal protein L14 |
| 1414 | 1343053 | 1343406 | 746325 | 745972 | 1220 | r-1 | 1343062 | 1343389 | 195 | RpsQ | J | Ribosomal protein S17 |

| 1415 | 1343394 | 1343660 | 745984 | 745718 | 1607 | r-2 | 1343394 | 1343655 | 127 | POP4 | J | RNAse P protein subunit P29/POP4 |
|---|---|---|---|---|---|---|---|---|---|---|---|---|
| 1416 | 1343657 | 1343953 | 745721 | 745425 | 1932 | r-3 | 1343657 | 1343951 | 170 | SUI1 | J | Translation initiation factor (SUI1) |
| 1417 | 1343960 | 1344160 | 745418 | 745218 | 1931 | r-3 | 1343984 | 1344158 | 101 | RpmC | J | Ribosomal protein L29 |
| 1418 | 1344147 | 1344785 | 745231 | 744593 | 1606 | r-2 | 1344156 | 1344729 | 316 | RpsC | J | Ribosomal protein S3 |
| 1419 | 1344782 | 1345252 | 744596 | 744126 | 1930 | r-3 | 1344794 | 1345250 | 241 | RplV | J | Ribosomal protein L22 |
| 1420 | 1345263 | 1345673 | 744115 | 743705 | 1605 | r-2 | 1345281 | 1345671 | 203 | RpsS | J | Ribosomal protein S19 |
| 1421 | 1345670 | 1346398 | 743708 | 742980 | 1929 | r-3 | 1345679 | 1346396 | 438 | RplB | J | Ribosomal protein L2 |
| 1422 | 1346403 | 1346663 | 742975 | 742715 | 1604 | r-2 | 1346403 | 1346661 | 153 | RplW | J | Ribosomal protein L23 |
| 1423 | 1346670 | 1347437 | 742708 | 741941 | 1603 | r-2 | 1346670 | 1347435 | 415 | RplD | J | Ribosomal protein L4 |
| 1424 | 1347448 | 1348488 | 741930 | 740890 | 1219 | r-1 | 1347448 | 1348435 | 509 | RplC | J | Ribosomal protein L3 |
| 1425 | 1348490 | 1349344 | 740888 | 740034 | 1928 | r-3 | 1348574 | 1349333 | 394 | - | S | Uncharacterized ACR |
| 1426 | 1349882 | 1351258 | 739496 | 738120 | 1927 | r-3 | 1349882 | 1351238 | 706 | - | R | Predicted ATPase of the AAA superfamily |
| 1427 | 1351322 | 1352506 | 738056 | 736872 | 1926 | r-3 | 1351358 | 1352504 | 501 | - | L | ATP-dependent DNA ligase |
| 1428 | 1352613 | 1353269 | 736765 | 736109 | 1602 | r-2 | 1352721 | 1353255 | 301 | RplP | J | Ribosomal protein L16/L10E |
| 1429 | 1354574 | 1355740 | 734804 | 733638 | 590 | f-2 | 1354601 | 1355738 | 619 | - | E | Serine-pyruvate aminotransferase/archaeal aspartate aminotransferase |
| 1430 | 1355821 | 1356402 | 733557 | 732976 | 1218 | r-1 | 1355821 | 1356397 | 256 | VirB1 1 | N | Predicted ATPases involved in pili and flagella biosynthesis |

| 1431 | 1356606 | 1357514 | 732772 | 731864 | 961 | f-3 | 1356615 | 1357512 | 426 | AsnS | J | Aspartyl/asparaginyl-tRNA synthetases |
|---|---|---|---|---|---|---|---|---|---|---|---|---|
| 1432 | 1357517 | 1358350 | 731861 | 731028 | 1925 | r-3 | 1357520 | 1358333 | 394 | MesJ | D | Predicted ATPase of the PP-loop superfamily implicated in cell cycle control |
| 1433 | 1358441 | 1359433 | 730937 | 729945 | 1924 | r-3 | 1358945 | 1359113 | 36 | LacA | G | Beta-galactosidase |
| 1434 | 1361181 | 1362461 | 728197 | 726917 | 962 | f-3 | 1361181 | 1362417 | 612 | - | M | Glycosyltransferases |
| 1435 | 1362449 | 1362523 | 726929 | 726855 | 591 | f-2 | 1362449 | 1362521 | 43 | - | M | Glycosyltransferases |
| 1436 | 1363010 | 1363930 | 726368 | 725448 | 1923 | r-3 | 1363016 | 1363925 | 512 | MesJ | D | Predicted ATPase of the PP-loop superfamily implicated in cell cycle control |
| 1437 | 1363972 | 1365465 | 725406 | 723913 | 1217 | r-1 | 1364029 | 1365457 | 858 | - | R | Uncharacterized FAD-dependent dehydrogenases |
| 1438 | 1365589 | 1366155 | 723789 | 723223 | 228 | f-1 | 1365643 | 1366150 | 228 | - | R | CBS domains |
| 1439 | 1366195 | 1367346 | 723183 | 722032 | 229 | f-1 | 1366204 | 1367341 | 495 | KefB | P | Kef-type K+ transport systems |
| 1440 | 1367357 | 1368481 | 722021 | 720897 | 592 | f-2 | 1367357 | 1368416 | 353 | KefB | P | Kef-type K+ transport systems |
| 1441 | 1368582 | 1369193 | 720796 | 720185 | 963 | f-3 | 1368636 | 1369188 | 221 | MarC | S | Integral membrane proteins of the MarC family |
| 1442 | 1369248 | 1370567 | 720130 | 718811 | 964 | f-3 | 1369266 | 1370559 | 647 | HisS | J | Histidyl-tRNA synthetase |

211

| 1443 | 1370627 | 1370989 | 718751 | 718389 | 1922 | r-3 | 1370681 | 1370972 | 51 | Cls | I | Phosphatidylserine/phosphatidylg lycerophosphate/cardioli pin synthases and related enzymes |
|------|---------|---------|--------|--------|------|-----|---------|---------|------|------|-----|---|
| 1444 | 1371847 | 1372125 | 717531 | 717253 | 230 | f-1 | 1371853 | 1372030 | 34 | - | S | Uncharacterized archaeal coiled-coil domain |
| 1445 | 1372322 | 1373752 | 717056 | 715626 | 593 | f-2 | 1372358 | 1372637 | 32 | PheS | J | Phenylalanyl-tRNA synthetase alpha subunit |
| 1446 | 1373902 | 1376664 | 715476 | 712714 | 231 | f-1 | 1373911 | 1376659 | 1504 | AlaS | J | Alanyl-tRNA synthetase |
| 1447 | 1376921 | 1378402 | 712457 | 710976 | 594 | f-2 | 1376936 | 1378388 | 653 | PutP | EH R | Na+/proline |
| 1448 | 1378470 | 1379534 | 710908 | 709844 | 1601 | r-2 | 1378470 | 1379532 | 568 | EutG | C | Alcohol dehydrogenase IV |
| 1449 | 1379649 | 1380014 | 709729 | 709364 | 965 | f-3 | 1379802 | 1379913 | 28 | HemB | H | Delta-aminolevulinic acid dehydratase |
| 1450 | 1379981 | 1380445 | 709397 | 708933 | 1921 | r-3 | 1380098 | 1380248 | 33 | FlgH | N | Flagellar basal body L-ring protein |
| 1451 | 1380532 | 1381284 | 708846 | 708094 | 1216 | r-1 | 1380532 | 1381279 | 332 | - | S | Uncharacterized ACR |
| 1452 | 1381281 | 1382687 | 708097 | 706691 | 1600 | r-2 | 1381296 | 1382565 | 209 | - | R | Predicted ATPase of the AAA superfamily |
| 1453 | 1382767 | 1384572 | 706611 | 704806 | 232 | f-1 | 1382809 | 1384570 | 1039 | ELP3 | K | ELP3 component of the RNA polymerase II complex |
| 1454 | 1384569 | 1385354 | 704809 | 704024 | 1599 | r-2 | 1385043 | 1385295 | 44 | - | S | Uncharacterized ACR |

EP 1 923 464 A1

| 1455 | 1385351 | 1385914 | 704027 | 703464 | 1920 | r-3 | 1385360 | 1385834 | 101 | HdeD | S | Uncharacterized BCR |
|---|---|---|---|---|---|---|---|---|---|---|---|---|
| 1456 | 1386061 | 1387578 | 703317 | 701800 | 1215 | r-1 | 1386079 | 1387129 | 150 | - | S | Predicted membrane protein |
| 1457 | 1387922 | 1388011 | 701456 | 701367 | 595 | f-2 | | | | | | |
| 1458 | 1388004 | 1389050 | 701374 | 700328 | 1598 | r-2 | 1388016 | 1388826 | 96 | NosY | R | ABC-type transport system involved in multi-copper enzyme maturation |
| 1459 | 1388485 | 1388589 | 700893 | 700789 | 233 | f-1 | 1388485 | 1388584 | 26 | - | S | Uncharacterized ArCR |
| 1460 | 1389047 | 1389982 | 700331 | 699396 | 1919 | r-3 | 1389059 | 1389962 | 268 | CcmA | Q | ABC-type multidrug transport system |
| 1461 | 1390108 | 1390617 | 699270 | 698761 | 234 | f-1 | 1390108 | 1390498 | 229 | - | R | Predicted Fe-S-cluster oxidoreductase |
| 1462 | 1390656 | 1391165 | 698722 | 698213 | 966 | f-3 | 1390668 | 1391157 | 246 | NIP7 | J | Protein involved in ribosomal biogenesis |
| 1463 | 1391397 | 1391669 | 697981 | 697709 | 967 | f-3 | 1391445 | 1391511 | 28 | GloB | R | Zn-dependent hydrolases |
| 1464 | 1393980 | 1394540 | 695398 | 694838 | 968 | f-3 | 1393980 | 1394523 | 160 | CcmA | Q | ABC-type multidrug transport system |
| 1465 | 1396169 | 1396951 | 693209 | 692427 | 596 | f-2 | 1396205 | 1396946 | 461 | RAD5 5 | T | RecA-superfamily ATPases implicated in signal transduction |
| 1466 | 1396965 | 1397522 | 692413 | 691856 | 969 | f-3 | 1396977 | 1397328 | 206 | - | K | Predicted transcriptional regulators |
| 1467 | 1397528 | 1397968 | 691850 | 691410 | 1918 | r-3 | 1397546 | 1397951 | 245 | SpeD | E | S-adenosylmethionine decarboxylase |

| | | | | | | | | | | | |
|---|---|---|---|---|---|---|---|---|---|---|---|
| 1468 | 1398271 | 1399176 | 691107 | 690202 | 235 | f-1 | 1398328 | 1399144 | 272 | SecF | N | Preprotein translocase subunit SecF |
| 1469 | 1399173 | 1400693 | 690205 | 688685 | 970 | f-3 | 1399188 | 1400673 | 452 | SecD | N | Preprotein translocase subunit SecD |
| 1470 | 1400690 | 1401382 | 688688 | 687996 | 597 | f-2 | 1400693 | 1401374 | 330 | TrkA | P | K+ transport systems |
| 1471 | 1401502 | 1401813 | 687876 | 687565 | 236 | f-1 | 1401502 | 1401802 | 62 | NtpF | C | Archaeal/vacuolar-type H+-ATPase subunit H |
| 1472 | 1401815 | 1403806 | 687563 | 685572 | 598 | f-2 | 1401815 | 1403789 | 681 | NtpI | C | Archaeal/vacuolar-type H+-ATPase subunit I |
| 1473 | 1403824 | 1404309 | 685554 | 685069 | 237 | f-1 | 1403824 | 1404286 | 171 | AtpE | C | F0F1-type ATP synthase c subunit/Archaeal/vacuolar-type H+-ATPase subunit K |
| 1474 | 1404349 | 1404960 | 685029 | 684418 | 238 | f-1 | 1404349 | 1404958 | 186 | NtpE | C | Archaeal/vacuolar-type H+-ATPase subunit E |
| 1475 | 1404957 | 1406060 | 684421 | 683318 | 971 | f-3 | 1404984 | 1406046 | 407 | NtpC | C | Archaeal/vacuolar-type H+-ATPase subunit C |
| 1476 | 1406057 | 1406365 | 683321 | 683013 | 599 | f-2 | 1406057 | 1406360 | 146 | NtpG | C | Archaeal/vacuolar-type H+-ATPase subunit F |
| 1477 | 1406372 | 1407382 | 683006 | 681996 | 600 | f-2 | 1406372 | 1407344 | 399 | NtpA | C | Archaeal/vacuolar-type H+-ATPase subunit A |
| 1478 | 1407475 | 1408257 | 681903 | 681121 | 239 | f-1 | 1407475 | 1408255 | 481 | NtpA | C | Archaeal/vacuolar-type H+-ATPase subunit A |

| 1479 | 1408254 | 1409654 | 681124 | 679724 | 972 | f-3 | 1408257 | 1409646 | 864 | NtpB | C | Archaeal/vacuolar-type H+-ATPase subunit B |
|------|----------|----------|--------|--------|-----|-----|----------|----------|-----|------|---|---|
| 1480 | 1409674 | 1410327 | 679704 | 679051 | 240 | f-1 | 1409683 | 1410316 | 318 | NtpD | C | Archaeal/vacuolar-type H+-ATPase subunit D |
| 1481 | 1410413 | 1411189 | 678965 | 678189 | 601 | f-2 | 1410422 | 1411187 | 442 | - | C | Uncharacterized flavoproteins |
| 1482 | 1411199 | 1411954 | 678179 | 677424 | 602 | f-2 | 1411199 | 1411943 | 322 | Tar | N | Methyl-accepting chemotaxis protein |
| 1483 | 1411938 | 1413167 | 677440 | 676211 | 973 | f-3 | 1411947 | 1413159 | 442 | - | R | Predicted metal-dependent hydrolases related to alanyl-tRNA synthetase HxxxH domain |
| 1484 | 1413235 | 1413960 | 676143 | 675418 | 241 | f-1 | 1413274 | 1413763 | 34 | MetC | E | Cystathionine beta-lyases/cystathionine gamma-synthases |
| 1485 | 1413935 | 1414642 | 675443 | 674736 | 603 | f-2 | 1414058 | 1414295 | 30 | AsnB | E | Asparagine synthase (glutamine-hydrolyzing) |
| 1486 | 1414943 | 1415797 | 674435 | 673581 | 604 | f-2 | 1414952 | 1415792 | 507 | - | R | Predicted metal-dependent hydrolases of the urease superfamily |
| 1487 | 1415800 | 1418658 | 673578 | 670720 | 1214 | r-1 | 1416094 | 1417195 | 315 | GltD | ER | NADPH-dependent glutamate synthase beta chain and related oxidoreductases COG0493 GltD |

| 1488 | 1418655 | 1420457 | 670723 | 668921 | 1597 | r-2 | 1418700 | 1420224 | 632 | NuoF | C | NADH:ubiquinone oxidoreductase |
|---|---|---|---|---|---|---|---|---|---|---|---|---|
| 1489 | 1420450 | 1420923 | 668928 | 668455 | 1213 | r-1 | 1420489 | 1420888 | 150 | NuoE | C | NADH:ubiquinone oxidoreductase 24 kD subunit |
| 1490 | 1421049 | 1422080 | 668329 | 667298 | 1596 | r-2 | 1421058 | 1422069 | 493 | RCL1 | K | RNA phosphate cyclase |
| 1491 | 1422217 | 1422759 | 667161 | 666619 | 242 | f-1 | 1422355 | 1422448 | 30 | SbcC | L | ATPase involved in DNA repair |
| 1492 | 1422740 | 1423594 | 666638 | 665784 | 1917 | r-3 | 1423205 | 1423340 | 35 | LysC | E | Aspartokinases |
| 1493 | 1423617 | 1424129 | 665761 | 665249 | 1595 | r-2 | 1423617 | 1424127 | 253 | - | R | Predicted phosphoesterase |
| 1494 | 1424266 | 1424787 | 665112 | 664591 | 243 | f-1 | 1424407 | 1424518 | 30 | - | R | Uncharacterized CBS domain-containing proteins |
| 1495 | 1424787 | 1428260 | 664591 | 661118 | 974 | f-3 | 1424787 | 1425792 | 442 | MCM 2 | L | Predicted ATPase involved in replication control |
| 1496 | 1428306 | 1428734 | 661072 | 660644 | 975 | f-3 | 1428315 | 1428732 | 250 | GCD7 | J | Translation initiation factor eIF-2 |
| 1497 | 1428842 | 1430410 | 660536 | 658968 | 605 | f-2 | 1429613 | 1430408 | 486 | AccA | I | Acetyl-CoA carboxylase alpha subunit |
| 1498 | 1430421 | 1430807 | 658957 | 658571 | 976 | f-3 | 1430433 | 1430790 | 52 | OadG | C | Na+-transporting methylmalonyl-CoA/oxaloacetate decarboxylase |
| 1499 | 1430801 | 1431283 | 658577 | 658095 | 606 | f-2 | 1430876 | 1431281 | 129 | AccB | I | Biotin carboxyl carrier protein |

EP 1 923 464 A1

| 1500 | 1431290 | 1432483 | 658088 | 656895 | 607 | f-2 | 1431302 | 1432481 | 628 | OadB | C | Na+-transporting methylmalonyl-CoA/oxaloacetate decarboxylase |
|---|---|---|---|---|---|---|---|---|---|---|---|---|
| 1501 | 1432547 | 1433398 | 656831 | 655980 | 608 | f-2 | 1432556 | 1433390 | 422 | - | R | CBS domains |
| 1502 | 1433432 | 1434445 | 655946 | 654933 | 609 | f-2 | 1433447 | 1434437 | 291 | ThrA | E | Homoserine dehydrogenase |
| 1503 | 1434874 | 1435398 | 654504 | 653980 | 244 | f-1 | 1434985 | 1435246 | 33 | - | T | Periplasmic ligand-binding sensor domain |
| 1504 | 1435395 | 1436108 | 653983 | 653270 | 1594 | r-2 | 1435434 | 1436022 | 315 | - | R | C4-type Zn finger |
| 1505 | 1436180 | 1436593 | 653198 | 652785 | 1916 | r-3 | 1436180 | 1436591 | 124 | - | S | Uncharacterized ACR |
| 1506 | 1436645 | 1436935 | 652733 | 652443 | 1915 | r-3 | 1436774 | 1436900 | 31 | - | S | Predicted membrane protein |
| 1507 | 1436958 | 1437776 | 652420 | 651602 | 1593 | r-2 | 1436958 | 1437774 | 418 | - | J | RNase PH-related exoribonuclease |
| 1508 | 1437769 | 1438527 | 651609 | 650851 | 1212 | r-1 | 1437778 | 1438525 | 467 | Rph | J | RNase PH |
| 1509 | 1438502 | 1439275 | 650876 | 650103 | 1914 | r-3 | 1438502 | 1439237 | 411 | RRP4 | J | RNA-binding protein Rrp4 and related proteins (contain S1 domain and KH domain) |
| 1510 | 1439272 | 1439982 | 650106 | 649396 | 1211 | r-1 | 1439272 | 1439980 | 424 | - | S | Uncharacterized ACR |
| 1511 | 1439994 | 1440776 | 649384 | 648602 | 1592 | r-2 | 1439994 | 1440774 | 389 | HslV | O | Proteasome protease subunit |
| 1512 | 1441115 | 1441582 | 648263 | 647796 | 610 | f-2 | 1441115 | 1441553 | 219 | Hit | FG R | Diadenosine tetraphosphate (Ap4A) hydrolase and other HIT family hydrolases COG0537 Hit |

| 1513 | 1441557 | 1441976 | 647821 | 647402 | 1591 | r-2 | 1441659 | 1441965 | 99 | MazG | R | Predicted pyrophosphatase |
|------|---------|---------|--------|--------|------|-----|---------|---------|-----|------|----|----|
| 1514 | 1441888 | 1442184 | 647490 | 647194 | 1210 | r-1 | 1441981 | 1442116 | 30 | SerC | HE | Phosphoserine aminotransferase COG1932 SerC |
| 1515 | 1442268 | 1442525 | 647110 | 646853 | 977 | f-3 | | | | | | |
| 1516 | 1442602 | 1444524 | 646776 | 644854 | 245 | f-1 | 1442671 | 1443574 | 550 | PorA | C | Pyruvate:ferredoxin oxidoreductase and related 2-oxoacid:ferredoxin oxidoreductases |
| 1517 | 1444521 | 1444967 | 644857 | 644411 | 1590 | r-2 | 1444521 | 1444953 | 102 | - | R | Predicted nucleic acid-binding protein |
| 1518 | 1445288 | 1446001 | 644090 | 643377 | 1913 | r-3 | 1445507 | 1445840 | 31 | CcmA | Q | ABC-type multidrug transport system |
| 1519 | 1446421 | 1446744 | 642957 | 642634 | 1209 | r-1 | 1446487 | 1446610 | 28 | - | S | Uncharacterized ACR |
| 1520 | 1447018 | 1447827 | 642360 | 641551 | 246 | f-1 | 1447057 | 1447756 | 221 | PerM | R | Predicted permease |
| 1521 | 1447763 | 1448299 | 641615 | 641079 | 1912 | r-3 | 1447763 | 1448297 | 325 | PncA | Q | Amidases related to nicotinamidase |
| 1522 | 1448354 | 1448527 | 641024 | 640851 | 1911 | r-3 | 1448354 | 1448522 | 79 | - | S | Uncharacterized ACR |
| 1523 | 1448733 | 1449227 | 640645 | 640151 | 978 | f-3 | 1448805 | 1449219 | 164 | PaaI | Q | Uncharacterized protein |
| 1524 | 1449764 | 1450072 | 639614 | 639306 | 611 | f-2 | 1449773 | 1450067 | 143 | - | K | Predicted transcriptional regulators |
| 1525 | 1450076 | 1451272 | 639302 | 638106 | 612 | f-2 | 1450103 | 1451219 | 516 | | | |

| 1526 | 1451362 | 1452348 | 638016 | 637030 | 247 | f-1 | 1451362 | 1452337 | 398 | AnsB | EJ | L-asparaginase/archaeal Glu-tRNAGln amidotransferase subunit D COG0252 AnsB |
|---|---|---|---|---|---|---|---|---|---|---|---|---|
| 1527 | 1452345 | 1452566 | 637033 | 636812 | 1589 | r-2 | | | | | | |
| 1528 | 1452921 | 1453571 | 636457 | 635807 | 1588 | r-2 | 1452930 | 1453569 | 229 | MarC | S | Integral membrane proteins of the MarC family |
| 1529 | 1453739 | 1453954 | 635639 | 635424 | 613 | f-2 | 1453805 | 1453904 | 28 | CheA | N | Chemotaxis protein histidine kinase and related kinases |
| 1530 | 1454658 | 1454753 | 634720 | 634625 | 1587 | r-2 | | | | | | |
| 1531 | 1455780 | 1457495 | 633598 | 631883 | 1586 | r-2 | 1456269 | 1456545 | 33 | LAP4 | E | Aspartyl aminopeptidase |
| 1532 | 1458373 | 1458516 | 631005 | 630862 | 1208 | r-1 | | | | | | |
| 1533 | 1460859 | 1461371 | 628519 | 628007 | 1585 | r-2 | 1461048 | 1461270 | 30 | GlnQ | E | ABC-type polar amino acid transport system |
| 1534 | 1461343 | 1461726 | 628035 | 627652 | 1207 | r-1 | 1461454 | 1461613 | 30 | UvrA | L | Excinuclease ATPase subunit |
| 1535 | 1462494 | 1463108 | 626884 | 626270 | 1584 | r-2 | 1462509 | 1462680 | 28 | VacB | K | Exoribonucleases |
| 1536 | 1463105 | 1464283 | 626273 | 625095 | 1910 | r-3 | 1463141 | 1464236 | 580 | FtsZ | D | Cell division GTPase |
| 1537 | 1464255 | 1466492 | 625123 | 622886 | 1583 | r-2 | 1464516 | 1464702 | 35 | RnhA | L | Ribonuclease HI |
| 1538 | 1466599 | 1467609 | 622779 | 621769 | 1206 | r-1 | 1466614 | 1467604 | 607 | - | C | Fe-S oxidoreductases |
| 1539 | 1467655 | 1467744 | 621723 | 621634 | 248 | f-1 | | | | | | |
| 1540 | 1467769 | 1467906 | 621609 | 621472 | 249 | f-1 | | | | | | |

EP 1 923 464 A1

| | | | | | | | | | | | | |
|---|---|---|---|---|---|---|---|---|---|---|---|---|
| 1541 | 1467891 | 1468676 | 621487 | 620702 | 1582 | r-2 | 1468092 | 1468650 | 200 | HemK | J | Predicted rRNA or tRNA methylase |
| 1542 | 1468498 | 1469019 | 620880 | 620359 | 1205 | r-1 | 1468501 | 1469002 | 255 | - | R | Conserved protein/domain typically associated with flavoprotein oxygenases |
| 1543 | 1469265 | 1470533 | 620113 | 618845 | 979 | f-3 | 1469331 | 1470465 | 343 | AprE | O | Subtilisin-like serine proteases |
| 1544 | 1470609 | 1471790 | 618769 | 617588 | 1581 | r-2 | 1470618 | 1471788 | 664 | PncB | H | Nicotinic acid phosphoribosyltransferase |
| 1545 | 1471812 | 1471937 | 617566 | 617441 | 1580 | r-2 | | | | | | |
| 1546 | 1471870 | 1472673 | 617508 | 616705 | 250 | f-1 | 1471912 | 1472653 | 149 | FabG | Q R | Dehydrogenases with different specificities (related to short-chain alcohol dehydrogenases) COG1028 FabG |
| 1547 | 1474731 | 1474928 | 614647 | 614450 | 1579 | r-2 | 1474809 | 1474893 | 27 | Pcm | O | Protein-L-isoaspartate carboxylmethyltransferase |
| 1548 | 1475072 | 1475983 | 614306 | 613395 | 1909 | r-3 | 1475084 | 1475972 | 427 | - | R | Predicted hydrolase of the metallo-beta-lactamase superfamily |
| 1549 | 1477107 | 1477574 | 612271 | 611804 | 980 | f-3 | 1477110 | 1477398 | 30 | DeoR | K | Transcriptional regulator |

| | | | | | | | | | | GltD | ER | |
|---|---|---|---|---|---|---|---|---|---|---|---|---|
| 1550 | 1477584 | 1479029 | 611794 | 610349 | 1578 | r-2 | 1477599 | 1479027 | 735 | | | NADPH-dependent glutamate synthase beta chain and related oxidoreductases COG0493 GltD |
| 1551 | 1479030 | 1479884 | 610348 | 609494 | 1577 | r-2 | 1479030 | 1479882 | 446 | UbiB | H C | 2-polyprenylphenol hydroxylase and related flavodoxin oxidoreductases~ COG0543 UbiB |
| 1552 | 1480088 | 1480873 | 609290 | 608505 | 614 | f-2 | 1480088 | 1480871 | 429 | - | S | Uncharacterized ArCR |
| 1553 | 1480960 | 1481781 | 608418 | 607597 | 1204 | r-1 | 1480960 | 1481779 | 378 | CysK | E | Cysteine synthase |
| 1554 | 1481753 | 1481869 | 607625 | 607509 | 1908 | r-3 | 1481759 | 1481840 | 31 | CysK | E | Cysteine synthase |
| 1555 | 1482049 | 1482780 | 607329 | 606598 | 1203 | r-1 | 1482049 | 1482757 | 382 | - | K | Predicted transcriptional regulators |
| 1556 | 1484422 | 1486413 | 604956 | 602965 | 251 | f-1 | 1484950 | 1485667 | 224 | AprE | O | Subtilisin-like serine proteases |
| 1557 | 1486448 | 1488211 | 602930 | 601167 | 615 | f-2 | 1487183 | 1487729 | 33 | SqhC | I | Squalene cyclase |
| 1558 | 1488253 | 1489308 | 601125 | 600070 | 1202 | r-1 | 1488253 | 1489306 | 553 | - | R | Predicted methyltransferases |
| 1559 | 1489417 | 1490157 | 599961 | 599221 | 252 | f-1 | 1489417 | 1490146 | 257 | - | R | Uncharacterized ATPases of the PP-loop superfamily |
| 1560 | 1490211 | 1490753 | 599167 | 598625 | 981 | f-3 | 1490298 | 1490748 | 206 | PaaD | R | Putative aromatic ring hydroxylating enzyme |
| 1561 | 1490896 | 1491087 | 598482 | 598291 | 253 | f-1 | 1490896 | 1491073 | 99 | Fer | C | Ferredoxin 1 |
| 1562 | 1491222 | 1491395 | 598156 | 597983 | 1576 | r-2 | 1491249 | 1491393 | 103 | RPS31 | J | Ribosomal protein S27AE |
| 1563 | 1491406 | 1491738 | 597972 | 597640 | 1201 | r-1 | 1491442 | 1491733 | 159 | RPS24 | J | Ribosomal protein S24E |

| | | | | | | | | | | A | | |
|---|---|---|---|---|---|---|---|---|---|---|---|---|
| 1564 | 1491692 | 1492225 | 597686 | 597153 | 1907 | r-3 | 1491692 | 1492217 | 199 | - | S | Uncharacterized ArCR |
| 1565 | 1492222 | 1492431 | 597156 | 596947 | 1200 | r-1 | 1492237 | 1492426 | 99 | - | K | DNA-directed RNA polymerase subunit E" |
| 1566 | 1492428 | 1493000 | 596950 | 596378 | 1575 | r-2 | 1492428 | 1492941 | 261 | RPB7 | K | DNA-directed RNA polymerase subunit E' |
| 1567 | 1493037 | 1493573 | 596341 | 595805 | 1574 | r-2 | 1493037 | 1493571 | 312 | Ppa | C | Inorganic pyrophosphatase |
| 1568 | 1493631 | 1494593 | 595747 | 594785 | 1573 | r-2 | 1494243 | 1494420 | 33 | AcnA | C | Aconitase A |
| 1569 | 1494613 | 1495560 | 594765 | 593818 | 1199 | r-1 | 1494913 | 1495111 | 33 | - | T | GAF domain-containing proteins |
| 1570 | 1495557 | 1496564 | 593821 | 592814 | 1572 | r-2 | 1495563 | 1496529 | 235 | LepB | N | Signal peptidase I |
| 1571 | 1496677 | 1497216 | 592701 | 592162 | 1198 | r-1 | 1496755 | 1496977 | 32 | LeuA | E | Isopropylmalate/homocitrate/citra malate synthases |
| 1572 | 1497231 | 1497902 | 592147 | 591476 | 1571 | r-2 | 1497582 | 1497816 | 33 | - | H | 6-pyruvoyl-tetrahydropterin synthase |
| 1573 | 1498015 | 1498506 | 591363 | 590872 | 1197 | r-1 | 1498126 | 1498402 | 31 | DcuC | C | C4-dicarboxylate transporter |
| 1574 | 1499893 | 1500954 | 589485 | 588424 | 1196 | r-1 | 1500004 | 1500946 | 498 | WcaG | M G | Nucleoside-diphosphate-sugar epimerases COG0451 WcaG |
| 1575 | 1500975 | 1501334 | 588403 | 588044 | 982 | f-3 | 1500978 | 1501332 | 167 | - | R | Predicted nucleotidyltransferases |
| 1576 | 1501234 | 1501755 | 588144 | 587623 | 254 | f-1 | 1501312 | 1501732 | 222 | - | S | Uncharacterized ACR |

EP 1 923 464 A1

| 1577 | 1501752 | 1502747 | 587626 | 586631 | 983 | f-3 | 1501752 | 1502745 | 510 | GCD1 | MJ | Nucleoside-diphosphate-sugar pyrophosphorylases involved in lipopolysaccharide biosynthesis/translation initiation factor eIF2B subunits COG1208 GCD1 |
|---|---|---|---|---|---|---|---|---|---|---|---|---|
| 1578 | 1502782 | 1504029 | 586596 | 585349 | 255 | f-1 | 1502782 | 1503988 | 650 | RfbX | R | Membrane protein involved in the export of O-antigen and teichoic acid |
| 1579 | 1503705 | 1503881 | 585673 | 585497 | 1570 | r-2 | 1503741 | 1503867 | 27 | CysN | P | GTPases - Sulfate adenylate transferase subunit 1 |
| 1580 | 1506454 | 1507683 | 582924 | 581695 | 256 | f-1 | 1506496 | 1507669 | 617 | TagB | M | Putative glycosyl/glycerophosphate transferases involved in teichoic acid biosynthesis TagF/TagB/EpsJ/RodC |
| 1581 | 1507680 | 1508369 | 581698 | 581009 | 984 | f-3 | 1507680 | 1508364 | 371 | IspD | I | 4-diphosphocytidyl-2-methyl-D-e rithritol synthase |
| 1582 | 1508513 | 1509250 | 580865 | 580128 | 616 | f-2 | 1508513 | 1509248 | 404 | WcaA | M | Glycosyltransferases involved in cell wall biogenesis |
| 1583 | 1509284 | 1511584 | 580094 | 577794 | 1906 | r-3 | 1509311 | 1511570 | 800 | - | R | Uncharacterized membrane protein |

| 1584 | 1512986 | 1513759 | 576392 | 575619 | 617 | f-2 | 1513040 | 1513637 | 119 | WcaA | M | Glycosyltransferases involved in cell wall biogenesis |
|---|---|---|---|---|---|---|---|---|---|---|---|---|
| 1585 | 1513756 | 1514835 | 575622 | 574543 | 257 | f-1 | 1513756 | 1514773 | 191 | RfaG | M | Predicted glycosyltransferases |
| 1586 | 1515877 | 1516842 | 573501 | 572536 | 258 | f-1 | 1516165 | 1516792 | 93 | RfaG | M | Predicted glycosyltransferases |
| 1587 | 1518510 | 1518569 | 570868 | 570809 | 1569 | r-2 | | | | | | |
| 1588 | 1519816 | 1521600 | 569562 | 567778 | 259 | f-1 | 1520431 | 1520620 | 32 | LolA | M | Outer membrane lipoprotein-sorting protein |
| 1589 | 1519824 | 1519925 | 569554 | 569453 | 1568 | r-2 | | | | | | |
| 1590 | 1521735 | 1522592 | 567643 | 566786 | 985 | f-3 | 1521990 | 1522401 | 37 | HsdR | L | Restriction enzymes type I helicase subunits and related helicases |
| 1591 | 1523210 | 1524667 | 566168 | 564711 | 618 | f-2 | 1523219 | 1523624 | 31 | - | S | Uncharacterized membrane-associated protein/domain |
| 1592 | 1525075 | 1526076 | 564303 | 563302 | 260 | f-1 | 1525372 | 1525714 | 35 | - | S | Predicted archaeal membrane protein |
| 1593 | 1526066 | 1526449 | 563312 | 562929 | 1905 | r-3 | 1526066 | 1526432 | 84 | RfaG | M | Predicted glycosyltransferases |
| 1594 | 1529489 | 1530295 | 559889 | 559083 | 619 | f-2 | 1529501 | 1530284 | 389 | NagD | G | Predicted sugar phosphatases of the HAD superfamily |
| 1595 | 1530296 | 1530733 | 559082 | 558645 | 620 | f-2 | 1530557 | 1530722 | 33 | - | S | Uncharacterized ACR |
| 1596 | 1530894 | 1536164 | 558484 | 553214 | 986 | f-3 | 1534812 | 1536162 | 744 | NrdA | F | Ribonucleotide reductase alpha |

|  |  |  |  |  |  |  |  |  |  |  | subunit |
|---|---|---|---|---|---|---|---|---|---|---|---|
| 1597 | 1536298 | 1536771 | 553080 | 552607 | 261 | f-1 | 1536307 | 1536769 | 230 | - | R | Predicted phosphoribosyltransferases |
| 1598 | 1536811 | 1537365 | 552567 | 552013 | 262 | f-1 | 1536811 | 1537363 | 268 | LigT | J | 2'-5' RNA ligase |
| 1599 | 1540326 | 1541702 | 549052 | 547676 | 987 | f-3 | 1540326 | 1541697 | 582 | CCA1 | J | tRNA nucleotidyltransferase (CCA-adding enzyme) |
| 1600 | 1541901 | 1543691 | 547477 | 545687 | 1567 | r-2 | 1542636 | 1542834 | 33 | Gmd | M | GDP-D-mannose dehydratase |
| 1601 | 1543754 | 1544062 | 545624 | 545316 | 621 | f-2 | 1543862 | 1544054 | 28 | DPH2 | J | Diphthamide synthase subunit DPH2 |
| 1602 | 1544093 | 1544920 | 545285 | 544458 | 622 | f-2 | 1544096 | 1544915 | 261 | RhaT | GER | Permeases of the drug/metabolite transporter (DMT) superfamily COG0697 RhaT |
| 1603 | 1544970 | 1545347 | 544408 | 544031 | 988 | f-3 | 1545231 | 1545324 | 32 | - | S | Uncharacterized BCR |
| 1604 | 1545432 | 1545968 | 543946 | 543410 | 1566 | r-2 | 1545432 | 1545966 | 183 | - | S | Uncharacterized ACR |
| 1605 | 1546165 | 1549362 | 543213 | 540016 | 263 | f-1 | 1546165 | 1549360 | 1910 | IleS | J | Isoleucyl-tRNA synthetase |
| 1606 | 1549370 | 1549522 | 540008 | 539856 | 1904 | r-3 | 1549385 | 1549490 | 27 | CobQ | H | Cobyric acid synthase |
| 1607 | 1550195 | 1551454 | 539183 | 537924 | 1903 | r-3 | 1550882 | 1551290 | 32 | BaeS | T | Sensory transduction histidine kinases |
| 1608 | 1551384 | 1551506 | 537994 | 537872 | 989 | f-3 |  |  |  |  |  |  |
| 1609 | 1551637 | 1552008 | 537741 | 537370 | 1195 | r-1 | 1551637 | 1552006 | 162 | VapC | R | Predicted nucleic acid-binding protein |

EP 1 923 464 A1

| 1610 | 1551975 | 1552217 | 537403 | 537161 | 1565 | r-2 | 1551975 | 1552212 | 105 | - | S | Uncharacterized ACR |
|---|---|---|---|---|---|---|---|---|---|---|---|---|
| 1611 | 1552330 | 1553088 | 537048 | 536290 | 264 | f-1 | 1552351 | 1552525 | 33 | QcrB | C | Cytochrome b subunit of the bc complex |
| 1612 | 1553108 | 1555480 | 536270 | 533898 | 1902 | r-3 | 1553126 | 1555466 | 1072 | LacA | G | Beta-galactosidase (exo-beta-D-glucosaminidase) |
| 1613 | 1555474 | 1556295 | 533904 | 533083 | 1194 | r-1 | 1555474 | 1556287 | 359 | AgaS | M | Predicted phosphosugar isomerases |
| 1614 | 1556455 | 1557438 | 532923 | 531940 | 1193 | r-1 | 1556482 | 1557424 | 491 | OppF | EP | ABC-type dipeptide/oligopeptide/nickel transport system |
| 1615 | 1557416 | 1558507 | 531962 | 530871 | 1901 | r-3 | 1557539 | 1558493 | 497 | DppD | EP | ABC-type dipeptide/oligopeptide/nickel transport system |
| 1616 | 1558390 | 1559334 | 530988 | 530044 | 1192 | r-1 | 1558408 | 1559320 | 357 | DppC | EP | ABC-type dipeptide/oligopeptide/nickel transport systems |
| 1617 | 1559337 | 1560350 | 530041 | 529028 | 1564 | r-2 | 1559364 | 1560345 | 529 | DppB | EP | ABC-type dipeptide/oligopeptide/nickel transport systems |
| 1618 | 1560382 | 1561011 | 528996 | 528367 | 1191 | r-1 | 1560382 | 1560955 | 219 | OppA | EP | ABC-type dipeptide/oligopeptide/nickel transport systems |

| 1619 | 1561392 | 1562597 | 527986 | 526781 | 1563 | r-2 | 1561392 | 1562439 | 468 | OppA | EP | ABC-type dipeptide/oligopeptide/nickel transport systems |
|---|---|---|---|---|---|---|---|---|---|---|---|---|
| 1620 | 1562832 | 1564286 | 526546 | 525092 | 990 | f-3 | 1562838 | 1564281 | 790 | BglB | G | Beta-glucosidase/6-phospho-beta-glucosidase/beta- galactosidase |
| 1621 | 1564489 | 1564938 | 524889 | 524440 | 265 | f-1 | 1564489 | 1564933 | 158 | VapC | R | Predicted nucleic acid-binding protein |
| 1622 | 1564960 | 1565772 | 524418 | 523606 | 1190 | r-1 | 1564972 | 1565767 | 355 | - | S | deacetylase |
| 1623 | 1565943 | 1569653 | 523435 | 519725 | 991 | f-3 | 1566258 | 1567437 | 330 | ChiA | G | Chitinase |
| 1624 | 1569699 | 1571144 | 519679 | 518234 | 1562 | r-2 | 1570038 | 1571139 | 557 | - | R | Uncharacterized ACR related to pyruvate formate-lyase activating enzyme |
| 1625 | 1570858 | 1571220 | 518520 | 518158 | 266 | f-1 | 1570867 | 1571218 | 169 | POP5 | L | RNase P subunit P14 and its archaeal orthologs |
| 1626 | 1571217 | 1572563 | 518161 | 516815 | 1561 | r-2 | 1571217 | 1572540 | 557 | GlgA | G | Glycogen synthase |
| 1627 | 1572612 | 1573637 | 516766 | 515741 | 1560 | r-2 | 1572624 | 1573587 | 119 | - | K | Predicted transcriptional regulators |
| 1628 | 1573641 | 1573748 | 515737 | 515630 | 1559 | r-2 | | | | | | |
| 1629 | 1573710 | 1575680 | 515668 | 513698 | 992 | f-3 | 1574037 | 1575441 | 267 | AmyA | G | Glycosidases |
| 1630 | 1575753 | 1577099 | 513625 | 512279 | 993 | f-3 | 1575753 | 1577070 | 692 | MalE | G | Maltose-binding periplasmic proteins/domains |

EP 1 923 464 A1

| 1631 | 1577138 | 1578040 | 512240 | 511338 | 623 | f-2 | 1577138 | 1578032 | 480 | MalF | G | ABC-type sugar transport systems |
|---|---|---|---|---|---|---|---|---|---|---|---|---|
| 1632 | 1578037 | 1579284 | 511341 | 510094 | 267 | f-1 | 1578049 | 1579279 | 466 | MalG | G | Sugar permeases |
| 1633 | 1579294 | 1582596 | 510084 | 506782 | 268 | f-1 | 1579300 | 1582387 | 1626 | - | G | Alpha-amylase/alpha-mannosidase |
| 1634 | 1582707 | 1583825 | 506671 | 505553 | 994 | f-3 | 1582707 | 1583823 | 623 | MalK | G | ABC-type sugar/spermidine/putrescine/iron/thiamine transport systems |
| 1635 | 1583858 | 1584259 | 505520 | 505119 | 624 | f-2 | 1583870 | 1584245 | 146 | - | S | Uncharacterized ArCR |
| 1636 | 1584289 | 1585641 | 505089 | 503737 | 269 | f-1 | 1584292 | 1585606 | 321 | CpsG | G | Phosphomannomutase |
| 1637 | 1585646 | 1586575 | 503732 | 502803 | 1900 | r-3 | 1585760 | 1586573 | 431 | PhnP | R | Metal-dependent hydrolases of the beta-lactamase superfamily I |
| 1638 | 1586361 | 1588547 | 503017 | 500831 | 995 | f-3 | 1586673 | 1588470 | 865 | - | S | Uncharacterized membrane protein |
| 1639 | 1588597 | 1588962 | 500781 | 500416 | 270 | f-1 | 1588741 | 1588915 | 31 | LysR | K | Transcriptional regulator |
| 1640 | 1588919 | 1590214 | 500459 | 499164 | 625 | f-2 | 1588952 | 1590212 | 639 | ArgE | E | Acetylornithine deacetylase/Succinyl-diaminopimelate desuccinylase and related deacylases |
| 1641 | 1590298 | 1591578 | 499080 | 497800 | 271 | f-1 | 1590586 | 1590886 | 31 | ThrS | J | Threonyl-tRNA synthetase |
| 1642 | 1591902 | 1592372 | 497476 | 497006 | 1558 | r-2 | 1592157 | 1592334 | 29 | BglC | G | Endoglucanase |

EP 1 923 464 A1

| 1643 | 1592769 | 1593515 | 496609 | 495863 | 996 | f-3 | 1592769 | 1593501 | 411 | SmtA | Q R | SAM-dependent methyltransferases COG0500 SmtA |
|---|---|---|---|---|---|---|---|---|---|---|---|---|
| 1644 | 1593682 | 1594884 | 495696 | 494494 | 1189 | r-1 | 1593694 | 1594882 | 644 | - | R | Predicted SAM-dependent methyltransferases |
| 1645 | 1595017 | 1595325 | 494361 | 494053 | 272 | f-1 | 1595017 | 1595104 | 30 | - | R | Predicted phosphate-binding enzymes |
| 1646 | 1596465 | 1597058 | 492913 | 492320 | 1557 | r-2 | 1596477 | 1596711 | 30 | DltE | R | Short-chain dehydrogenases of various substrate specificities |
| 1647 | 1597751 | 1598509 | 491627 | 490869 | 1899 | r-3 | 1597778 | 1598507 | 387 | RAD5 5 | T | RecA-superfamily ATPases implicated in signal transduction |
| 1648 | 1598676 | 1599902 | 490702 | 489476 | 997 | f-3 | 1598700 | 1599873 | 396 | PRI2 | L | Eukaryotic-type DNA primase |
| 1649 | 1599886 | 1600935 | 489492 | 488443 | 273 | f-1 | 1599904 | 1600903 | 474 | PRI1 | L | Eukaryotic-type DNA primase |
| 1650 | 1601220 | 1601777 | 488158 | 487601 | 998 | f-3 | 1601223 | 1601760 | 67 | RhaT | GE R | Permeases of the drug/metabolite transporter (DMT) superfamily COG0697 RhaT |
| 1651 | 1603727 | 1603786 | 485651 | 485592 | 626 | f-2 | | | | | | |
| 1652 | 1604088 | 1604264 | 485290 | 485114 | 1556 | r-2 | 1604088 | 1604154 | 26 | - | S | Uncharacterized ArCR |
| 1653 | 1604708 | 1606048 | 484670 | 483330 | 627 | f-2 | 1604768 | 1606046 | 714 | GlnA | E | Glutamine synthase |

EP 1 923 464 A1

| | | | | | | | | | | | | |
|---|---|---|---|---|---|---|---|---|---|---|---|---|
| 1654 | 1606039 | 1606902 | 483339 | 482476 | 1188 | r-1 | 1606045 | 1606855 | 363 | RhaT | GE R | Permeases of the drug/metabolite transporter (DMT) superfamily COG0697 RhaT |
| 1655 | 1606912 | 1607685 | 482466 | 481693 | 1187 | r-1 | 1606921 | 1607683 | 375 | NadE | H | NAD synthase |
| 1656 | 1607663 | 1607971 | 481715 | 481407 | 1898 | r-3 | 1607762 | 1607855 | 30 | FUI1 | FH | Cytosine/uracil/thiamine/allantoin permeases COG1953 FUI1 |
| 1657 | 1608213 | 1609220 | 481165 | 480158 | 1555 | r-2 | 1608213 | 1609215 | 592 | OppF | EP | ABC-type dipeptide/oligopeptide/nickel transport system |
| 1658 | 1609231 | 1610190 | 480147 | 479188 | 1186 | r-1 | 1609231 | 1610188 | 581 | DppD | EP | ABC-type dipeptide/oligopeptide/nickel transport system |
| 1659 | 1610202 | 1611623 | 479176 | 477755 | 1554 | r-2 | 1610202 | 1611618 | 657 | DppC | EP | ABC-type dipeptide/oligopeptide/nickel transport systems |
| 1660 | 1611635 | 1612684 | 477743 | 476694 | 1897 | r-3 | 1611635 | 1612673 | 540 | DppB | EP | ABC-type dipeptide/oligopeptide/nickel transport systems |
| 1661 | 1612865 | 1615312 | 476513 | 474066 | 1896 | r-3 | 1613654 | 1614983 | 57 | OppA | EP | ABC-type dipeptide/oligopeptide/nickel transport systems |
| 1662 | 1615653 | 1616882 | 473725 | 472496 | 999 | f-3 | 1615659 | 1616868 | 523 | PyrC | F | Dihydroorotase |

EP 1 923 464 A1

| 1663 | 1616860 | 1617561 | 472518 | 471817 | 274 | f-1 | 1616860 | 1617553 | 338 | UbiB | H C | 2-polyprenylphenol hydroxylase and related flavodoxin oxidoreductases~ COG0543 UbiB |
| 1664 | 1617558 | 1618517 | 471820 | 470861 | 1000 | f-3 | 1617615 | 1618512 | 516 | - | R | Predicted Fe-S oxidoreductases |
| 1665 | 1617756 | 1617815 | 471622 | 471563 | 1553 | r-2 | | | | | | |
| 1666 | 1618578 | 1619276 | 470800 | 470102 | 1001 | f-3 | 1618647 | 1619130 | 33 | DppC | EP | ABC-type dipeptide/oligopeptide/nickel transport systems |
| 1667 | 1619263 | 1621227 | 470115 | 468151 | 1185 | r-1 | 1619266 | 1621183 | 975 | - | G | Alpha-amylase/alpha-mannosidase (4-alpha-glucanotransferase) |
| 1668 | 1621305 | 1621934 | 468073 | 467444 | 1552 | r-2 | 1621305 | 1621890 | 216 | SEC59 | I | Dolichol kinase |
| 1669 | 1622735 | 1622920 | 466643 | 466458 | 628 | f-2 | 1622735 | 1622909 | 33 | - | S | Uncharacterized archaeal membrane protein |
| 1670 | 1622922 | 1624112 | 466456 | 465266 | 1002 | f-3 | 1622940 | 1624086 | 499 | KefB | P | Kef-type K+ transport systems |
| 1671 | 1624133 | 1625287 | 465245 | 464091 | 629 | f-2 | 1624136 | 1625279 | 536 | GadB | E | Glutamate decarboxylase and related PLP-dependent proteins |
| 1672 | 1625321 | 1625563 | 464057 | 463815 | 630 | f-2 | 1625339 | 1625441 | 39 | - | K | Predicted transcriptional regulators containing the CopG/Arc/MetJ DNA-binding domain |
| 1673 | 1625628 | 1625717 | 463750 | 463661 | 1003 | f-3 | 1625631 | 1625709 | 30 | MazF | T | Growth inhibitor |

231

| 1674 | 1625816 | 1625929 | 463562 | 463449 | 631 | f-2 | | | | | | |
|---|---|---|---|---|---|---|---|---|---|---|---|---|
| 1675 | 1625919 | 1626824 | 463459 | 462554 | 1551 | r-2 | 1625964 | 1626810 | 346 | MMT1 | P | Predicted Co/Zn/Cd cation transporters |
| 1676 | 1627009 | 1627614 | 462369 | 461764 | 1184 | r-1 | 1627279 | 1627477 | 32 | RpoB | K | DNA-directed RNA polymerase beta subunit/140 kD subunit (split gene in Mjan |
| 1677 | 1627793 | 1629337 | 461585 | 460041 | 632 | f-2 | 1627817 | 1629101 | 316 | RfbX | R | Membrane protein involved in the export of O-antigen and teichoic acid |
| 1678 | 1629435 | 1630595 | 459943 | 458783 | 1004 | f-3 | 1629435 | 1630491 | 336 | - | M | Predicted membrane-associated Zn-dependent proteases 1 |
| 1679 | 1630596 | 1631720 | 458782 | 457658 | 1005 | f-3 | 1630749 | 1631694 | 526 | MesJ | D | Predicted ATPase of the PP-loop superfamily implicated in cell cycle control |
| 1680 | 1630637 | 1630705 | 458741 | 458673 | 1895 | r-3 | | | | | | |
| 1681 | 1631799 | 1633073 | 457579 | 456305 | 1006 | f-3 | 1631853 | 1633008 | 232 | - | R | Uncharacterized ATPases of the AAA superfamily |
| 1682 | 1633129 | 1633257 | 456249 | 456121 | 275 | f-1 | 1633156 | 1633240 | 30 | IleS | J | Isoleucyl-tRNA synthetase |
| 1683 | 1634125 | 1634739 | 455253 | 454639 | 276 | f-1 | 1634227 | 1634494 | 33 | AraJ | G | Arabinose efflux permease |
| 1684 | 1634253 | 1634369 | 455125 | 455009 | 1550 | r-2 | 1634256 | 1634337 | 27 | SUL1 | P | Sulfate permease and related transporters (MFS superfamily) |

EP 1 923 464 A1

| 1685 | 1634744 | 1635046 | 454634 | 454332 | 633 | f-2 | 1634744 | 1635005 | 108 | MarR | K | Transcriptional regulators |
|---|---|---|---|---|---|---|---|---|---|---|---|---|
| 1686 | 1635049 | 1636365 | 454329 | 453013 | 1183 | r-1 | 1635139 | 1636348 | 703 | BglB | G | Beta-glucosidase/6-phospho-beta-glucosidase/beta- galactosidase |
| 1687 | 1636376 | 1637356 | 453002 | 452022 | 634 | f-2 | 1636376 | 1637351 | 544 | GalT | C | Galactose-1-phosphate uridylyltransferase |
| 1688 | 1637336 | 1638673 | 452042 | 450705 | 1894 | r-3 | 1637342 | 1638653 | 675 | | | |
| 1689 | 1638670 | 1639755 | 450708 | 449623 | 1182 | r-1 | 1638670 | 1639744 | 536 | - | S | Uncharacterized ACR |
| 1690 | 1639752 | 1640816 | 449626 | 448562 | 1549 | r-2 | 1639764 | 1640805 | 404 | GalK | G | Galactokinase |
| 1691 | 1640937 | 1641557 | 448441 | 447821 | 1548 | r-2 | 1641177 | 1641468 | 34 | - | S | Predicted membrane protein |
| 1692 | 1641581 | 1643545 | 447797 | 445833 | 1893 | r-3 | 1641581 | 1643381 | 744 | - | S | Uncharacterized ACR |
| 1693 | 1643712 | 1644038 | 445666 | 445340 | 1007 | f-3 | 1643826 | 1644036 | 33 | ArgS | J | Arginyl-tRNA synthetase |
| 1694 | 1644035 | 1644664 | 445343 | 444714 | 1892 | r-3 | 1644044 | 1644641 | 198 | Pcp | O | Pyrrolidone-carboxylate peptidase (N-terminal pyroglutamyl peptidase) |
| 1695 | 1644711 | 1645832 | 444667 | 443546 | 1008 | f-3 | 1644717 | 1645830 | 464 | FixC | C | Dehydrogenases (flavoproteins) |
| 1696 | 1645842 | 1646195 | 443536 | 443183 | 1009 | f-3 | 1645923 | 1646169 | 33 | BisC | C | Anaerobic dehydrogenases |
| 1697 | 1646550 | 1647749 | 442828 | 441629 | 1010 | f-3 | 1647372 | 1647549 | 32 | UgpB | G | Sugar-binding periplasmic proteins/domains |
| 1698 | 1651192 | 1652691 | 438186 | 436687 | 1181 | r-1 | 1651192 | 1652689 | 865 | - | E | Zn-dependent carboxypeptidases |

| 1699 | 1652842 | 1653462 | 436536 | 435916 | 277 | f-1 | 1652848 | 1653448 | 222 | - | L | Predicted site-specific integrase-resolvase |
| 1700 | 1653443 | 1654624 | 435935 | 434754 | 635 | f-2 | 1653509 | 1654499 | 137 | - | L | Predicted transposases |
| 1701 | 1654676 | 1655512 | 434702 | 433866 | 636 | f-2 | 1654808 | 1655423 | 74 | RbsK | G | Sugar kinases |
| 1702 | 1655924 | 1656976 | 433454 | 432402 | 1891 | r-3 | 1655990 | 1656971 | 407 | - | S | Uncharacterized ACR |
| 1703 | 1657257 | 1658210 | 432121 | 431168 | 1547 | r-2 | 1657269 | 1658208 | 465 | - | R | MoxR-like ATPases |
| 1704 | 1658633 | 1658857 | 430745 | 430521 | 1890 | r-3 | 1658633 | 1658831 | 97 | PppA | N | Signal peptidase |
| 1705 | 1659540 | 1660034 | 429838 | 429344 | 1011 | f-3 | 1659564 | 1659858 | 32 | - | S | Uncharacterized ArCR |
| 1706 | 1660137 | 1660616 | 429241 | 428762 | 1012 | f-3 | 1660143 | 1660560 | 142 | SlpA | O | FKBP-type peptidyl-prolyl cis-trans isomerases 2 |
| 1707 | 1660605 | 1661033 | 428773 | 428345 | 1546 | r-2 | 1660605 | 1661031 | 155 | - | S | Predicted membrane protein |
| 1708 | 1661293 | 1661439 | 428085 | 427939 | 278 | f-1 | | | | | | |
| 1709 | 1661519 | 1662583 | 427859 | 426795 | 1889 | r-3 | 1661531 | 1662581 | 392 | - | S | Predicted membrane protein |
| 1710 | 1662585 | 1666019 | 426793 | 423359 | 1545 | r-2 | 1663962 | 1665537 | 735 | - | L | Inteins |
| 1711 | 1666185 | 1666505 | 423193 | 422873 | 1544 | r-2 | 1666254 | 1666413 | 29 | AcoA | C | Thiamine pyrophosphate-dependent dehydrogenases |
| 1712 | 1667046 | 1668500 | 422332 | 420878 | 1543 | r-2 | 1667046 | 1668477 | 231 | - | S | Uncharacterized ArCR |
| 1713 | 1668573 | 1668914 | 420805 | 420464 | 1013 | f-3 | 1668708 | 1668849 | 30 | - | L | Predicted transposase |
| 1714 | 1668871 | 1669944 | 420507 | 419434 | 279 | f-1 | 1668952 | 1669942 | 506 | - | R | Predicted GTPases |
| 1715 | 1669941 | 1671896 | 419437 | 417482 | 1542 | r-2 | 1670538 | 1670883 | 48 | - | R | ABC-type transport systems |

| ID | | | | | | | | | | | | | Name | R | Description |
|---|---|---|---|---|---|---|---|---|---|---|---|---|---|---|---|
| 1716 | 1671856 | 1672545 | 417522 | 416833 | 1180 | r-1 | 1671859 | 1672504 | 200 | | | | PhnL | R | ABC-type transport systems |
| 1717 | 1672642 | 1672686 | 416736 | 416692 | 1179 | r-1 | | | | | | | | | |
| 1718 | 1672713 | 1673096 | 416665 | 416282 | 1541 | r-2 | 1672713 | 1673079 | 144 | | | | DppC | EP | ABC-type dipeptide/oligopeptide/nickel transport systems |
| 1719 | 1673965 | 1674999 | 415413 | 414379 | 1178 | r-1 | 1673965 | 1674997 | 226 | | | | DppB | EP | ABC-type dipeptide/oligopeptide/nickel transport systems |
| 1720 | 1675448 | 1676545 | 413930 | 412833 | 637 | f-2 | 1675448 | 1676543 | 556 | | | | - | L | Predicted N6-adenine-specific DNA methylases |
| 1721 | 1676630 | 1677790 | 412748 | 411588 | 638 | f-2 | 1676780 | 1677785 | 572 | | | | PstS | P | ABC-type phosphate transport system |
| 1722 | 1677812 | 1678636 | 411566 | 410742 | 639 | f-2 | 1677812 | 1678583 | 259 | | | | IolE | G | Sugar phosphate isomerases/epimerases |
| 1723 | 1678705 | 1679553 | 410673 | 409825 | 280 | f-1 | 1678705 | 1679548 | 414 | | | | PstC | P | ABC-type phosphate transport system |
| 1724 | 1679540 | 1680370 | 409838 | 409008 | 640 | f-2 | 1679555 | 1680299 | 326 | | | | PstA | P | ABC-type phosphate transport system |
| 1725 | 1680367 | 1681128 | 409011 | 408250 | 281 | f-1 | 1680373 | 1681126 | 395 | | | | PstB | P | ABC-type phosphate transport system |
| 1726 | 1681383 | 1681730 | 407995 | 407648 | 1014 | f-3 | 1681476 | 1681683 | 44 | | | | PhoU | P | Phosphate uptake regulator |

| 1727 | 1681740 | 1682333 | 407638 | 407045 | 1015 | f-3 | 1681740 | 1682328 | 251 | PhoU | P | Phosphate uptake regulator |
|------|---------|---------|--------|--------|------|-----|---------|---------|-----|------|---|----|
| 1728 | 1682428 | 1682817 | 406950 | 406561 | 282 | f-1 | 1682536 | 1682704 | 33 | WcaA | M | Glycosyltransferases involved in cell wall biogenesis |
| 1729 | 1682818 | 1683495 | 406560 | 405883 | 1177 | r-1 | 1682821 | 1683493 | 387 | MhpD | Q | 2-keto-4-pentenoate hydratase/2-oxohepta-3-ene-1 |
| 1730 | 1683568 | 1684578 | 405810 | 404800 | 1176 | r-1 | 1683847 | 1684462 | 56 | PepN | E | Aminopeptidase N |
| 1731 | 1684439 | 1684564 | 404939 | 404814 | 641 | f-2 | 1684475 | 1684559 | 26 | Lon | O | ATP-dependent Lon protease |
| 1732 | 1685535 | 1686689 | 403843 | 402689 | 1540 | r-2 | 1685535 | 1686684 | 652 | TrpS | J | Tryptophanyl-tRNA synthetase |
| 1733 | 1686869 | 1687045 | 402509 | 402333 | 642 | f-2 | 1686875 | 1687043 | 62 | - | S | Uncharacterized ArCR |
| 1734 | 1687089 | 1687931 | 402289 | 401447 | 1016 | f-3 | 1687152 | 1687899 | 185 | RhaT | GER | Permeases of the drug/metabolite transporter (DMT) superfamily COG0697 RhaT |
| 1735 | 1687932 | 1689299 | 401446 | 400079 | 1539 | r-2 | 1687932 | 1689249 | 416 | - | R | Predicted ATPase of the AAA superfamily |
| 1736 | 1689399 | 1690175 | 399979 | 399203 | 1017 | f-3 | 1689399 | 1690173 | 345 | PhnP | R | Metal-dependent hydrolases of the beta-lactamase superfamily I |
| 1737 | 1691003 | 1692442 | 398375 | 396936 | 1888 | r-3 | 1691042 | 1692428 | 796 | - | C | Acyl-CoA synthetase (NDP forming) |
| 1738 | 1692515 | 1693180 | 396863 | 396198 | 643 | f-2 | 1692605 | 1693172 | 303 | ArsR | K | Predicted transcriptional regulators |
| 1739 | 1693184 | 1693489 | 396194 | 395889 | 644 | f-2 | 1693184 | 1693484 | 186 | - | S | Uncharacterized ArCR |

| 1740 | 1693499 | 1694056 | 395879 | 395322 | 645 | f-2 | 1693508 | 1694048 | 163 | ArsR | K | Predicted transcriptional regulators |
|---|---|---|---|---|---|---|---|---|---|---|---|---|
| 1741 | 1694157 | 1695629 | 395221 | 393749 | 1018 | f-3 | 1694355 | 1695186 | 159 | AmyA | G | Glycosidases |
| 1742 | 1695642 | 1696265 | 393736 | 393113 | 1538 | r-2 | 1695957 | 1696233 | 33 | PurC | F | Phosphoribosylaminoimidazolesu ccinocarboxamide (SAICAR) synthase |
| 1743 | 1696275 | 1697726 | 393103 | 391652 | 1537 | r-2 | 1696845 | 1697721 | 342 | - | G | Predicted sugar kinase |
| 1744 | 1697807 | 1698145 | 391571 | 391233 | 646 | f-2 | 1697810 | 1697912 | 30 | MelB | G | Na+/melibiose symporter and related transporters |
| 1745 | 1699092 | 1699178 | 390286 | 390200 | 1019 | f-3 | | | | | | |
| 1746 | 1699622 | 1700173 | 389756 | 389205 | 1887 | r-3 | 1699640 | 1700171 | 246 | - | S | Uncharacterized ACR related to the C-terminal domain of histone macroH2A1 |
| 1747 | 1700210 | 1701493 | 389168 | 387885 | 1886 | r-3 | 1700210 | 1701479 | 464 | SsnA | FR | Cytosine deaminase and related metal-dependent hydrolases COG0402 SsnA |
| 1748 | 1703531 | 1704163 | 385847 | 385215 | 647 | f-2 | 1703534 | 1704155 | 92 | - | R | Predicted transglutaminase-like proteases |
| 1749 | 1704224 | 1704970 | 385154 | 384408 | 1885 | r-3 | 1704326 | 1704965 | 243 | GckA | G | Putative glycerate kinase |
| 1750 | 1704989 | 1705141 | 384389 | 384237 | 1884 | r-3 | 1704989 | 1705127 | 25 | - | S | Uncharacterized membrane protein |
| 1751 | 1705367 | 1706314 | 384011 | 383064 | 1883 | r-3 | 1705532 | 1706312 | 441 | Pnp | F | Purine nucleoside phosphorylase |

| 1752 | 1706139 | 1706984 | 383239 | 382394 | 1020 | f-3 | 1706256 | 1706982 | 384 | - | R | Archaeal enzymes of ATP-grasp superfamily |
|------|---------|---------|--------|--------|------|-----|---------|---------|-----|------|----|------|
| 1753 | 1706986 | 1707378 | 382392 | 382000 | 283 | f-1 | 1706995 | 1707373 | 151 | - | S | Uncharacterized ACR |
| 1754 | 1707375 | 1708133 | 382003 | 381245 | 1536 | r-2 | 1707387 | 1708125 | 346 | - | L | Predicted nuclease of the RecB family |
| 1755 | 1708168 | 1710714 | 381210 | 378664 | 1175 | r-1 | 1710097 | 1710712 | 349 | RecA | L | RecA/RadA recombinase |
| 1756 | 1710855 | 1711487 | 378523 | 377891 | 1535 | r-2 | 1710987 | 1711224 | 54 | Kch | P | Kef-type K+ transport systems |
| 1757 | 1712778 | 1714040 | 376600 | 375338 | 1021 | f-3 | 1712805 | 1713984 | 651 | CDC6 | LO | Cdc6-related protein |
| 1758 | 1714040 | 1716247 | 375338 | 373131 | 648 | f-2 | 1714652 | 1716230 | 621 | HYS2 | L | DNA polymerase small subunit |
| 1759 | 1716248 | 1721644 | 373130 | 367734 | 649 | f-2 | 1716272 | 1719128 | 1536 | - | L | Novel archaeal DNA polymerase (contains Zn-fingers) |
| 1760 | 1721669 | 1722406 | 367709 | 366972 | 650 | f-2 | 1721813 | 1722029 | 31 | CpsG | G | Phosphomannomutase |
| 1761 | 1722894 | 1723436 | 366484 | 365942 | 1022 | f-3 | 1723122 | 1723365 | 39 | - | L | Predicted nuclease of the RecB family |
| 1762 | 1725222 | 1725860 | 364156 | 363518 | 1023 | f-3 | 1725222 | 1725828 | 250 | - | S | Uncharacterized ACR |
| 1763 | 1725857 | 1726705 | 363521 | 362673 | 1882 | r-3 | 1725956 | 1726703 | 376 | LplA | H | Lipoate-protein ligase A |
| 1764 | 1727964 | 1729022 | 361414 | 360356 | 1024 | f-3 | 1727964 | 1728660 | 358 | WcaA | M | Glycosyltransferases involved in cell wall biogenesis |
| 1765 | 1729029 | 1729787 | 360349 | 359591 | 1025 | f-3 | 1729104 | 1729779 | 218 | RAD55 | T | RecA-superfamily ATPases implicated in signal transduction |
| 1766 | 1729784 | 1730227 | 359594 | 359151 | 651 | f-2 | 1729898 | 1730222 | 41 | RacX | M | Aspartate racemase |

| 1767 | 1730270 | 1731955 | 359108 | 357423 | 652 | f-2 | 1730270 | 1731941 | 651 | Iap | R | Predicted aminopeptidases |
|---|---|---|---|---|---|---|---|---|---|---|---|---|
| 1768 | 1731945 | 1732280 | 357433 | 357098 | 1534 | r-2 | 1731963 | 1732158 | 40 | - | K | Predicted transcriptional regulators |
| 1769 | 1732332 | 1732982 | 357046 | 356396 | 1533 | r-2 | 1732377 | 1732974 | 216 | - | R | Predicted ICC-like phosphoesterases |
| 1770 | 1732998 | 1733120 | 356380 | 356258 | 1532 | r-2 | | | | | | |
| 1771 | 1733473 | 1734267 | 355905 | 355111 | 284 | f-1 | 1733473 | 1734256 | 398 | - | R | Predicted amidohydrolase |
| 1772 | 1734255 | 1735046 | 355123 | 354332 | 1531 | r-2 | 1734255 | 1735020 | 255 | SmtA | Q R | SAM-dependent methyltransferases COG0500 SmtA |
| 1773 | 1735212 | 1735793 | 354166 | 353585 | 1026 | f-3 | 1735221 | 1735443 | 29 | PstA | P | ABC-type phosphate transport system |
| 1774 | 1736419 | 1736520 | 352959 | 352858 | 285 | f-1 | | | | | | |
| 1775 | 1736456 | 1736896 | 352922 | 352482 | 653 | f-2 | 1736540 | 1736717 | 32 | - | K | Predicted transcriptional regulator |
| 1776 | 1736893 | 1737423 | 352485 | 351955 | 1174 | r-1 | 1737130 | 1737328 | 30 | CcmB | O | ABC-type transport system involved in cytochrome c biogenesis |
| 1777 | 1737620 | 1738414 | 351758 | 350964 | 1881 | r-3 | 1738181 | 1738397 | 33 | FeoB | P | Ferrous ion uptake system protein FeoB (predicted GTPase) |
| 1778 | 1738777 | 1739505 | 350601 | 349873 | 1173 | r-1 | 1738843 | 1738912 | 33 | ChrA | P | Chromate transport protein ChrA |

| 1779 | 1739502 | 1739852 | 349876 | 349526 | 1530 | r-2 | 1739508 | 1739850 | 169 | - | S | Uncharacterized ACR |
|---|---|---|---|---|---|---|---|---|---|---|---|---|
| 1780 | 1739935 | 1740549 | 349443 | 348829 | 1172 | r-1 | 1740337 | 1740451 | 32 | CarA | EF | Carbamoylphosphate synthase small subunit COG0505 CarA |
| 1781 | 1740792 | 1741826 | 348586 | 347552 | 1027 | f-3 | 1740801 | 1741818 | 515 | DPH2 | J | Diphthamide synthase subunit DPH2 |
| 1782 | 1741926 | 1743704 | 347452 | 345674 | 1028 | f-3 | 1742919 | 1743285 | 38 | FlaD | N | Putative archaeal flagellar protein D/E |
| 1783 | 1743694 | 1743957 | 345684 | 345421 | 1171 | r-1 | 1743727 | 1743910 | 31 | RpoE | K | DNA-directed RNA polymerase specialized sigma subunits |
| 1784 | 1743938 | 1744243 | 345440 | 345135 | 1880 | r-3 | 1744073 | 1744232 | 30 | SIR2 | H | NAD-dependent protein deacetylases |
| 1785 | 1744245 | 1745591 | 345133 | 343787 | 1529 | r-2 | 1744263 | 1745559 | 346 | RAD5 5 | T | RecA-superfamily ATPases implicated in signal transduction |
| 1786 | 1745650 | 1746300 | 343728 | 343078 | 286 | f-1 | 1745671 | 1746277 | 250 | - | J | Predicted RNA methylase |
| 1787 | 1746894 | 1747268 | 342484 | 342110 | 1029 | f-3 | 1746915 | 1747134 | 31 | - | L | Superfamily I DNA and RNA helicases and helicase subunits |
| 1788 | 1747308 | 1748660 | 342070 | 340718 | 1030 | f-3 | 1747314 | 1748610 | 504 | Sun | J | tRNA and rRNA cytosine-C5-methylases |
| 1789 | 1749755 | 1749931 | 339623 | 339447 | 1879 | r-3 | 1749755 | 1749899 | 26 | TatC | N | Sec-independent protein secretion pathway component TatC |
| 1790 | 1749900 | 1749992 | 339478 | 339386 | 1031 | f-3 | | | | | | |

| 1791 | 1750416 | 1751543 | 338962 | 337835 | 1528 | r-2 | 1750896 | 1751238 | 32 | CirA | P | Outer membrane receptor proteins |
|---|---|---|---|---|---|---|---|---|---|---|---|---|
| 1792 | 1751717 | 1752793 | 337661 | 336585 | 1878 | r-3 | 1751852 | 1752785 | 449 | MscS | M | Small-conductance mechanosensitive channel |
| 1793 | 1752795 | 1753493 | 336583 | 335885 | 1527 | r-2 | 1752852 | 1753491 | 155 | - | S | Uncharacterized ACR |
| 1794 | 1753468 | 1755291 | 335910 | 334087 | 1170 | r-1 | 1755019 | 1755211 | 37 | Mfd | LK | Transcription-repair coupling factor - superfamily II helicase COG1197 Mfd |
| 1795 | 1755444 | 1756100 | 333934 | 333278 | 1526 | r-2 | 1755450 | 1756041 | 210 | - | S | Uncharacterized ACR |
| 1796 | 1756133 | 1756924 | 333245 | 332454 | 1877 | r-3 | 1756133 | 1756826 | 127 | - | S | Uncharacterized ACR |
| 1797 | 1757029 | 1757460 | 332349 | 331918 | 1169 | r-1 | 1757053 | 1757452 | 175 | - | R | Uncharacterized proteins of PilT N-term./Vapc superfamily |
| 1798 | 1757494 | 1758735 | 331884 | 330643 | 1168 | r-1 | 1757503 | 1758730 | 716 | TufB | JE | GTPases - translation elongation factors COG0050 TufB |
| 1799 | 1758870 | 1758998 | 330508 | 330380 | 1525 | r-2 | | | | | | |
| 1800 | 1760394 | 1760735 | 328984 | 328643 | 1032 | f-3 | 1760619 | 1760721 | 27 | - | L | Adenine-specific DNA methylase |
| 1801 | 1762166 | 1762558 | 327212 | 326820 | 1876 | r-3 | 1762181 | 1762556 | 176 | RPS6 A | J | Ribosomal protein S6E (S10) |
| 1802 | 1762676 | 1762846 | 326702 | 326532 | 654 | f-2 | 1762772 | 1762844 | 27 | Kup | P | K+ transporter |
| 1803 | 1762843 | 1763493 | 326535 | 325885 | 1167 | r-1 | 1763275 | 1763446 | 33 | Smc | D | Chromosome segregation ATPases |
| 1804 | 1763590 | 1764141 | 325788 | 325237 | 287 | f-1 | 1763593 | 1764109 | 251 | - | R | Predicted GTPases |

| 1805 | 1764136 | 1764609 | 325242 | 324769 | 1166 | r-1 | 1764163 | 1764607 | 251 | Lrp | K | Transcriptional regulators |
|---|---|---|---|---|---|---|---|---|---|---|---|---|
| 1806 | 1764704 | 1765804 | 324674 | 323574 | 655 | f-2 | 1764752 | 1765748 | 348 | - | R | Predicted GTPase or GTP-binding protein |
| 1807 | 1765840 | 1766682 | 323538 | 322696 | 288 | f-1 | 1765849 | 1766680 | 343 | UbiA | H | 4-hydroxybenzoate polyprenyltransferase |
| 1808 | 1766679 | 1767068 | 322699 | 322310 | 1033 | f-3 | 1766814 | 1766988 | 29 | Arp | R | Ankyrin repeat proteins |
| 1809 | 1767079 | 1767885 | 322299 | 321493 | 1165 | r-1 | 1767079 | 1767619 | 281 | - | S | Uncharacterized ArCR |
| 1810 | 1767919 | 1768269 | 321459 | 321109 | 1164 | r-1 | 1768081 | 1768183 | 32 | MukB | D | Uncharacterized protein involved in chromosome partitioning |
| 1811 | 1768271 | 1769350 | 321107 | 320028 | 1875 | r-3 | 1768280 | 1769300 | 431 | - | L | Replication factor A large subunit and related ssDNA-binding proteins |
| 1812 | 1769469 | 1770143 | 319909 | 319235 | 1524 | r-2 | 1769559 | 1770099 | 308 | - | K | Predicted transcriptional regulator containing the HTH domain |
| 1813 | 1770892 | 1772169 | 318486 | 317209 | 289 | f-1 | 1770901 | 1772104 | 447 | SfcA | C | Malic enzyme |
| 1814 | 1772144 | 1772719 | 317234 | 316659 | 1874 | r-3 | 1772201 | 1772711 | 199 | FumA | C | Tartrate dehydratase beta subunit/Fumarate hydratase class I |
| 1815 | 1772653 | 1773303 | 316725 | 316075 | 1163 | r-1 | 1772680 | 1773301 | 226 | TtdA | C | Tartrate dehydratase alpha subunit/Fumarate hydratase class I |

| 1816 | 1773571 | 1774485 | 315807 | 314893 | 1162 | r-1 | 1773571 | 1774483 | 523 | SerA | E | Phosphoglycerate dehydrogenase and related dehydrogenases |
|------|---------|---------|--------|--------|------|-----|---------|---------|-----|------|---|---|
| 1817 | 1774489 | 1775145 | 314889 | 314233 | 1161 | r-1 | 1774504 | 1775140 | 266 | - | P | Phosphate transport regulator (distant homolog of PhoU) |
| 1818 | 1775139 | 1776068 | 314239 | 313310 | 1523 | r-2 | 1775139 | 1776039 | 357 | ApbA | H | Ketopantoate reductase |
| 1819 | 1776073 | 1776540 | 313305 | 312838 | 1160 | r-1 | | | | | | |
| 1820 | 1776586 | 1777293 | 312792 | 312085 | 290 | f-1 | 1776589 | 1777270 | 186 | LasT | J | rRNA methylase |
| 1821 | 1777281 | 1777811 | 312097 | 311567 | 1034 | f-3 | 1777287 | 1777806 | 173 | Ada | L | Methylated DNA-protein cysteine methyltransferase (O6 methylguanine DNA methyltransferase) |
| 1822 | 1777799 | 1778830 | 311579 | 310548 | 656 | f-2 | 1777799 | 1778813 | 413 | NrfG | R | TPR-repeat-containing proteins |
| 1823 | 1779069 | 1779554 | 310309 | 309824 | 1035 | f-3 | 1779219 | 1779549 | 131 | EGD2 | K | Transcription factor homologous to NACalpha-BTF3 |
| 1824 | 1779558 | 1779923 | 309820 | 309455 | 1522 | r-2 | 1779657 | 1779912 | 68 | - | S | Uncharacterized ACR |
| 1825 | 1779979 | 1781619 | 309399 | 307759 | 1159 | r-1 | 1780849 | 1781521 | 40 | NtpC | C | Archaeal/vacuolar-type H+-ATPase subunit C |
| 1826 | 1781597 | 1782928 | 307781 | 306450 | 657 | f-2 | 1781600 | 1782872 | 573 | HflX | R | GTPases |
| 1827 | 1782866 | 1783828 | 306512 | 305550 | 1873 | r-3 | 1782914 | 1783826 | 312 | PitA | P | Phosphate/sulphate permeases |

| | | | | | | | | | | | |
|---|---|---|---|---|---|---|---|---|---|---|---|
| 1828 | 1784010 | 1784594 | 305368 | 304784 | 1036 | f-3 | 1784037 | 1784592 | 213 | PorG | C | Pyruvate:ferredoxin oxidoreductase and related 2-oxoacid:ferredoxin oxidoreductases |
| 1829 | 1784774 | 1784953 | 304604 | 304425 | 658 | f-2 | 1784774 | 1784951 | 125 | - | C | Pyruvate:ferredoxin oxidoreductase and related 2-oxoacid:ferredoxin oxidoreductases |
| 1830 | 1784955 | 1786151 | 304423 | 303227 | 1037 | f-3 | 1784964 | 1786149 | 643 | PorA | C | Pyruvate:ferredoxin oxidoreductase and related 2-oxoacid:ferredoxin oxidoreductases |
| 1831 | 1786148 | 1787092 | 303230 | 302286 | 659 | f-2 | 1786157 | 1787090 | 559 | PorB | C | Pyruvate:ferredoxin oxidoreductase and related 2-oxoacid:ferredoxin oxidoreductases |
| 1832 | 1787147 | 1787473 | 302231 | 301905 | 660 | f-2 | 1787156 | 1787471 | 207 | - | C | Pyruvate:ferredoxin oxidoreductase and related 2-oxoacid:ferredoxin oxidoreductases |

| 1833 | 1787485 | 1788669 | 301893 | 300709 | 291 | f-1 | 1787485 | 1788664 | 609 | PorA | C | Pyruvate:ferredoxin oxidoreductase and related 2-oxoacid:ferredoxin oxidoreductases |
|---|---|---|---|---|---|---|---|---|---|---|---|---|
| 1834 | 1788671 | 1789675 | 300707 | 299703 | 661 | f-2 | 1788677 | 1789673 | 537 | PorB | C | Pyruvate:ferredoxin oxidoreductase and related 2-oxoacid:ferredoxin oxidoreductases |
| 1835 | 1789714 | 1790697 | 299664 | 298681 | 292 | f-1 | 1790005 | 1790227 | 33 | MhpC | R | Predicted hydrolases or acyltransferases (alpha/beta hydrolase superfamily) |
| 1836 | 1790705 | 1791568 | 298673 | 297810 | 662 | f-2 | 1791065 | 1791434 | 32 | HemE | H | Uroporphyrinogen-III decarboxylase |
| 1837 | 1791624 | 1791959 | 297754 | 297419 | 1038 | f-3 | 1791801 | 1791948 | 29 | RfaG | M | Predicted glycosyltransferases |
| 1838 | 1791963 | 1792769 | 297415 | 296609 | 1039 | f-3 | 1792029 | 1792191 | 32 | MoaD | H | Molybdopterin converting factor |
| 1839 | 1792792 | 1793328 | 296586 | 296050 | 293 | f-1 | 1792792 | 1793008 | 33 | Qor | CR | NADPH:quinone reductase and related Zn-dependent oxidoreductases COG0604 Qor |
| 1840 | 1793325 | 1794524 | 296053 | 294854 | 1521 | r-2 | 1793325 | 1794519 | 702 | CsdB | E | Selenocysteine lyase |
| 1841 | 1794521 | 1794823 | 294857 | 294555 | 1872 | r-3 | 1794566 | 1794758 | 35 | - | S | Uncharacterized ACR |
| 1842 | 1794964 | 1796124 | 294414 | 293254 | 294 | f-1 | 1794988 | 1796095 | 285 | | | |

EP 1 923 464 A1

| 1843 | 1796129 | 1797154 | 293249 | 292224 | 1871 | r-3 | 1796147 | 1797152 | 553 | HypE | O | Hydrogenase maturation factor |
|---|---|---|---|---|---|---|---|---|---|---|---|---|
| 1844 | 1797235 | 1797561 | 292143 | 291817 | 1158 | r-1 | 1797256 | 1797493 | 70 | - | R | Predicted nucleotidyltransferases |
| 1845 | 1797561 | 1797665 | 291817 | 291713 | 1520 | r-2 | 1797561 | 1797663 | 43 | VapC | R | Predicted nucleic acid-binding protein |
| 1846 | 1797874 | 1798116 | 291504 | 291262 | 1157 | r-1 | | | | | | |
| 1847 | 1798158 | 1800545 | 291220 | 288833 | 1519 | r-2 | 1798227 | 1800540 | 1259 | HypF | O | Hydrogenase maturation factor |
| 1848 | 1800686 | 1801306 | 288692 | 288072 | 1870 | r-3 | 1800704 | 1801031 | 33 | Eno | G | Enolase |
| 1849 | 1801592 | 1802125 | 287786 | 287253 | 663 | f-2 | 1801595 | 1802084 | 187 | Ftn | P | Ferritin-like protein |
| 1850 | 1802245 | 1803363 | 287133 | 286015 | 1156 | r-1 | 1802260 | 1803361 | 605 | HypD | O | Hydrogenase maturation factor |
| 1851 | 1803363 | 1803602 | 286015 | 285776 | 1518 | r-2 | 1803375 | 1803597 | 108 | HypC | O | Hydrogenase maturation factor |
| 1852 | 1803666 | 1804280 | 285712 | 285098 | 1040 | f-3 | 1803675 | 1804212 | 246 | MobA | H | Molybdopterin-guanine dinucleotide biosynthesis protein A |
| 1853 | 1804317 | 1804535 | 285061 | 284843 | 1517 | r-2 | 1804335 | 1804389 | 25 | CelB | G | Phosphotransferase system cellobiose-specific component IIC |
| 1854 | 1804571 | 1805047 | 284807 | 284331 | 1869 | r-3 | 1804607 | 1804994 | 135 | HyaD | C | Ni (Hydrogenase maturation factor) |
| 1855 | 1805521 | 1805853 | 283857 | 283525 | 1155 | r-1 | 1805653 | 1805707 | 28 | SrmB | LK J | Superfamily II DNA and RNA helicases COG0513 SrmB |
| 1856 | 1805911 | 1806657 | 283467 | 282721 | 1154 | r-1 | 1805920 | 1806655 | 359 | Mrp | D | ATPases involved in chromosome partitioning (Hydrogenase |

| | | | | | | | | | | | | maturation factor) |
|---|---|---|---|---|---|---|---|---|---|---|---|---|
| 1857 | 1806654 | 1807073 | 282724 | 282305 | 1516 | r-2 | 1806654 | 1807068 | 204 | HybF | R | Zn finger protein HypA/HybF (possibly regulating hydrogenase expression)(Hydrogenase maturation factor) |
| 1858 | 1807161 | 1808084 | 282217 | 281294 | 1041 | f-3 | 1807203 | 1808076 | 384 | CzcD | P | Co/Zn/Cd efflux system component |
| 1859 | 1808249 | 1808404 | 281129 | 280974 | 664 | f-2 | 1808249 | 1808387 | 80 | - | S | Uncharacterized ACR |
| 1860 | 1808394 | 1808819 | 280984 | 280559 | 1515 | r-2 | 1808403 | 1808814 | 190 | - | C | Ferredoxin 3 |
| 1861 | 1808985 | 1811618 | 280393 | 277760 | 1042 | f-3 | 1810719 | 1811187 | 32 | ArtI | E | ABC-type amino acid transport system |
| 1862 | 1811744 | 1812487 | 277634 | 276891 | 665 | f-2 | 1811753 | 1812473 | 339 | - | R | Predicted permeases |
| 1863 | 1812518 | 1813510 | 276860 | 275868 | 1868 | r-3 | 1812518 | 1813508 | 476 | TehA | P | Tellurite resistance protein and related permeases |
| 1864 | 1813353 | 1813550 | 276025 | 275828 | 1043 | f-3 | 1813368 | 1813533 | 29 | ZntA | P | Cation transport ATPases |
| 1865 | 1813638 | 1814054 | 275740 | 275324 | 1514 | r-2 | 1813665 | 1814004 | 163 | - | S | Uncharacterized ACR |
| 1866 | 1814141 | 1814644 | 275237 | 274734 | 1867 | r-3 | 1814216 | 1814633 | 227 | - | S | Uncharacterized ACR |
| 1867 | 1814559 | 1814648 | 274819 | 274730 | 1044 | f-3 | | | | | | |
| 1868 | 1814829 | 1815962 | 274549 | 273416 | 1045 | f-3 | 1814829 | 1815960 | 486 | FecB | P | ABC-type Fe3+-siderophores transport systems |

| 1869 | 1815959 | 1817002 | 273419 | 272376 | 666 | f-2 | 1815974 | 1816997 | 415 | BtuC | PH | ABC-type cobalamin/Fe3+-siderophores transport systems |
|---|---|---|---|---|---|---|---|---|---|---|---|---|
| 1870 | 1816999 | 1817745 | 272379 | 271633 | 295 | f-1 | 1817017 | 1817737 | 273 | FepC | PH | ABC-type cobalamin/Fe3+-siderophores transport systems |
| 1871 | 1817756 | 1818715 | 271622 | 270663 | 667 | f-2 | 1817828 | 1818653 | 497 | Mrp | D | ATPases involved in chromosome partitioning |
| 1872 | 1819570 | 1819776 | 269808 | 269602 | 1153 | r-1 | 1819570 | 1819675 | 30 | - | S | Uncharacterized BCR |
| 1873 | 1820187 | 1820936 | 269191 | 268442 | 1513 | r-2 | 1820226 | 1820424 | 35 | XerC | L | Integrase |
| 1874 | 1820961 | 1821659 | 268417 | 267719 | 1512 | r-2 | 1821201 | 1821552 | 171 | TFA1 | K | Transcription initiation factor IIE |
| 1875 | 1821659 | 1821841 | 267719 | 267537 | 1866 | r-3 | 1821659 | 1821827 | 32 | DnaG | L | DNA primase (bacterial type) |
| 1876 | 1822105 | 1823073 | 267273 | 266305 | 296 | f-1 | 1822105 | 1823071 | 471 | CcmA | Q | ABC-type multidrug transport system |
| 1877 | 1823702 | 1823782 | 265676 | 265596 | 1865 | r-3 | | | | | | |
| 1878 | 1823857 | 1824675 | 265521 | 264703 | 297 | f-1 | 1823857 | 1824673 | 314 | - | R | ABC-type multidrug transport system |
| 1879 | 1824662 | 1825624 | 264716 | 263754 | 1864 | r-3 | 1824740 | 1825610 | 353 | RbsK | G | Sugar kinases |
| 1880 | 1825648 | 1826151 | 263730 | 263227 | 298 | f-1 | 1825648 | 1826035 | 153 | - | E | Predicted regulator of amino acid metabolism (contains the ACT domain) |
| 1881 | 1826226 | 1826504 | 263152 | 262874 | 1511 | r-2 | 1826229 | 1826502 | 167 | AcyP | C | Acylphosphatases |

| | | | | | | | | | | | |
|---|---|---|---|---|---|---|---|---|---|---|---|
| 1882 | 1826572 | 1826886 | 262806 | 262492 | 299 | f-1 | 1826581 | 1826866 | 147 | CutA | P | Uncharacterized protein involved in tolerance to divalent cations |
| 1883 | 1826859 | 1827470 | 262519 | 261908 | 1046 | f-3 | 1826883 | 1827468 | 267 | - | S | Uncharacterized ACR |
| 1884 | 1827563 | 1828408 | 261815 | 260970 | 1863 | r-3 | 1827782 | 1828406 | 229 | UspA | T | Universal stress protein UspA and related nucleotide-binding proteins |
| 1885 | 1828493 | 1829698 | 260885 | 259680 | 668 | f-2 | 1828493 | 1829693 | 715 | GcvT | E | Glycine cleavage system T protein (aminomethyltransferase) |
| 1886 | 1829731 | 1830558 | 259647 | 258820 | 300 | f-1 | 1829740 | 1830544 | 264 | RhaT | GER | Permeases of the drug/metabolite transporter (DMT) superfamily COG0697 RhaT |
| 1887 | 1830621 | 1831115 | 258757 | 258263 | 1510 | r-2 | 1830621 | 1831113 | 183 | LepB | N | Signal peptidase I |
| 1888 | 1831076 | 1831645 | 258302 | 257733 | 1862 | r-3 | 1831085 | 1831622 | 216 | - | S | Uncharacterized ArCR |
| 1889 | 1831699 | 1832772 | 257679 | 256606 | 301 | f-1 | 1831702 | 1832746 | 182 | NrfG | R | TPR-repeat-containing proteins |
| 1890 | 1832777 | 1833709 | 256601 | 255669 | 669 | f-2 | 1832777 | 1833704 | 455 | - | E | Zn-dependent dipeptidase |
| 1891 | 1833706 | 1834158 | 255672 | 255220 | 1152 | r-1 | 1833727 | 1834135 | 32 | - | K | Predicted transcriptional regulators |
| 1892 | 1834155 | 1834856 | 255223 | 254522 | 1509 | r-2 | 1834173 | 1834839 | 282 | RAD55 | T | RecA-superfamily ATPases implicated in signal transduction |

| | | | | | | | | | | | | |
|---|---|---|---|---|---|---|---|---|---|---|---|---|
| 1893 | 1834992 | 1835603 | 254386 | 253775 | f-3 | 1047 | 1835103 | 1835448 | 32 | EntF | Q | Non-ribosomal peptide synthetase modules and related proteins |
| 1894 | 1835581 | 1836201 | 253797 | 253177 | f-1 | 302 | 1835662 | 1835971 | 31 | MalG | G | Sugar permeases |
| 1895 | 1836239 | 1837111 | 253139 | 252267 | f-2 | 670 | 1836248 | 1837079 | 383 | - | R | Predicted archaeal methyltransferase |
| 1896 | 1837108 | 1838508 | 252270 | 250870 | r-1 | 1151 | 1838029 | 1838308 | 37 | - | S | Uncharacterized ACR |
| 1897 | 1838515 | 1839846 | 250863 | 249532 | r-1 | 1150 | 1838542 | 1839790 | 132 | - | S | Predicted membrane protein |
| 1898 | 1839843 | 1842821 | 249535 | 246557 | r-2 | 1508 | 1840932 | 1841325 | 43 | - | L | Micrococcal nuclease (thermonuclease) homologs |
| 1899 | 1842996 | 1844864 | 246382 | 244514 | r-2 | 1507 | 1842996 | 1844859 | 1005 | DAP2 | E | Dipeptidyl aminopeptidases/acylaminoacyl-p eptidases |
| 1900 | 1844947 | 1845273 | 244431 | 244105 | f-1 | 303 | 1845022 | 1845157 | 33 | RhaT | GE R | Permeases of the drug/metabolite transporter (DMT) superfamily COG0697 RhaT |
| 1901 | 1845241 | 1845942 | 244137 | 243436 | r-1 | 1149 | 1845325 | 1845895 | 161 | - | T | Predicted Ser/Thr protein kinase |
| 1902 | 1845932 | 1846168 | 243446 | 243210 | f-2 | 671 | 1845941 | 1846166 | 142 | Lrp | K | Transcriptional regulators |
| 1903 | 1846267 | 1847184 | 243111 | 242194 | r-1 | 1148 | 1846267 | 1847173 | 317 | CcmA | Q | ABC-type multidrug transport system |

| 1904 | 1847191 | 1848111 | 242187 | 241267 | 1147 | r-1 | 1847221 | 1847701 | 73 | NosY | R | ABC-type transport system involved in multi-copper enzyme maturation |
|---|---|---|---|---|---|---|---|---|---|---|---|---|
| 1905 | 1848117 | 1849664 | 241261 | 239714 | 1506 | r-2 | 1849086 | 1849260 | 38 | NosY | R | ABC-type transport system involved in multi-copper enzyme maturation |
| 1906 | 1853437 | 1853742 | 235941 | 235636 | 1146 | r-1 | 1853590 | 1853701 | 36 | Lon | O | ATP-dependent Lon protease |
| 1907 | 1853826 | 1853894 | 235552 | 235484 | 1048 | f-3 | | | | | | |
| 1908 | 1853933 | 1854607 | 235445 | 234771 | 1861 | r-3 | 1853933 | 1854602 | 294 | - | P | Phosphate transport regulator (distant homolog of PhoU) |
| 1909 | 1854612 | 1855832 | 234766 | 233546 | 1505 | r-2 | 1854621 | 1855830 | 596 | PitA | P | Phosphate/sulphate permeases |
| 1910 | 1855928 | 1857586 | 233450 | 231792 | 1860 | r-3 | 1856972 | 1857395 | 47 | Icc | R | Predicted phosphohydrolases |
| 1911 | 1857656 | 1858012 | 231722 | 231366 | 672 | f-2 | 1857656 | 1857998 | 178 | - | S | Uncharacterized ACR |
| 1912 | 1858017 | 1859300 | 231361 | 230078 | 1504 | r-2 | 1858017 | 1859286 | 652 | MiaB | J | 2-methylthioadenine synthetase |
| 1913 | 1859380 | 1859607 | 229998 | 229771 | 1145 | r-1 | 1859389 | 1859596 | 64 | - | S | Uncharacterized ArCR |
| 1914 | 1859695 | 1860141 | 229683 | 229237 | 1144 | r-1 | 1859701 | 1860133 | 179 | HyaD | C | Ni(Hydrogenase maturation factor) |
| 1915 | 1860556 | 1860741 | 228822 | 228637 | 1143 | r-1 | | | | | | |
| 1916 | 1860814 | 1862100 | 228564 | 227278 | 1142 | r-1 | 1860814 | 1862098 | 674 | - | C | Coenzyme F420-reducing hydrogenase (hydrogenase subunit) |

EP 1 923 464 A1

| 1917 | 1862097 | 1862900 | 227281 | 226478 | 1503 | r-2 | 1862118 | 1862898 | 438 | - | C | Coenzyme F420-reducing hydrogenase (hydrogenase subunit) |
|---|---|---|---|---|---|---|---|---|---|---|---|---|
| 1918 | 1862902 | 1863786 | 226476 | 225592 | 1141 | r-1 | 1862908 | 1863784 | 571 | UbiB | H C | 2-polyprenylphenol hydroxylase and related flavodoxin oxidoreductases~ COG0543 UbiB (hydrogenase subunit) |
| 1919 | 1863783 | 1864895 | 225595 | 224483 | 1502 | r-2 | 1863783 | 1864887 | 705 | NapF | C | Ferredoxin 2 (hydrogenase subunit) |
| 1920 | 1865656 | 1866711 | 223722 | 222667 | 304 | f-1 | 1865683 | 1866691 | 263 | GltD | ER | NADPH-dependent glutamate synthase beta chain and related oxidoreductases COG0493 GltD |
| 1921 | 1866693 | 1867223 | 222685 | 222155 | 1049 | f-3 | 1866717 | 1867119 | 156 | HybA | C | Fe-S-cluster-containing hydrogenase components 1 |
| 1922 | 1867473 | 1868666 | 221905 | 220712 | 1050 | f-3 | 1867578 | 1868649 | 350 | BisC | C | Anaerobic dehydrogenases (formate dehydrogenase) |
| 1923 | 1868696 | 1869637 | 220682 | 219741 | 673 | f-2 | 1868696 | 1869554 | 303 | BisC | C | Anaerobic dehydrogenases (formate dehydrogenase) |
| 1924 | 1869643 | 1870143 | 219735 | 219235 | 305 | f-1 | 1869652 | 1870060 | 172 | HybA | C | Fe-S-cluster-containing hydrogenase components 1 (formate dehydrogenase) |
| 1925 | 1870833 | 1871861 | 218545 | 217517 | 1051 | f-3 | 1871043 | 1871682 | 145 | FocA | P | Formate/nitrite family of |

| | | | | | | | | | | | transporters(formate dehydrogenase) |
|---|---|---|---|---|---|---|---|---|---|---|---|
| 1926 | 1872015 | 1872557 | 217363 | 216821 | 1052 | f-3 | 1872054 | 1872555 | 286 | MnhE | P | Multisubunit Na+/H+ antiporter |
| 1927 | 1872533 | 1872811 | 216845 | 216567 | 674 | f-2 | 1872563 | 1872809 | 128 | MnhF | P | Multisubunit Na+/H+ antiporter |
| 1928 | 1872808 | 1873179 | 216570 | 216199 | 306 | f-1 | 1872817 | 1873159 | 172 | MnhG | P | Multisubunit Na+/H+ antiporter |
| 1929 | 1873176 | 1873442 | 216202 | 215936 | 1053 | f-3 | 1873251 | 1873440 | 35 | - | P | Predicted subunit of the Multisubunit Na+/H+ antiporter |
| 1930 | 1873439 | 1873735 | 215939 | 215643 | 675 | f-2 | 1873439 | 1873733 | 66 | MnhB | P | Multisubunit Na+/H+ antiporter |
| 1931 | 1873732 | 1874181 | 215646 | 215197 | 307 | f-1 | 1873741 | 1874176 | 199 | MnhB | P | Multisubunit Na+/H+ antiporter |
| 1932 | 1874169 | 1874537 | 215209 | 214841 | 1054 | f-3 | 1874178 | 1874535 | 167 | MnhC | P | Multisubunit Na+/H+ antiporter |
| 1933 | 1874534 | 1876078 | 214844 | 213300 | 676 | f-2 | 1874546 | 1876073 | 720 | HyfB | CP | Formate hydrogenlyase subunit 3/Multisubunit Na+/H+ antiporter |
| 1934 | 1876071 | 1876427 | 213307 | 212951 | 1055 | f-3 | 1876080 | 1876188 | 30 | WcaJ | M | Sugar transferases involved in lipopolysaccharide synthesis |
| 1935 | 1876465 | 1876995 | 212913 | 212383 | 308 | f-1 | 1876465 | 1876993 | 309 | - | C | Ni |
| 1936 | 1876992 | 1877561 | 212386 | 211817 | 1056 | f-3 | 1877043 | 1877556 | 248 | HycE | C | Ni |
| 1937 | 1877558 | 1878838 | 211820 | 210540 | 677 | f-2 | 1877567 | 1878836 | 699 | HycE | C | Ni |
| 1938 | 1878843 | 1879835 | 210535 | 209543 | 1057 | f-3 | 1878861 | 1879833 | 389 | HyfC | C | Formate hydrogenlyase subunit 4 |

| 1939 | 1879832 | 1880263 | 209546 | 209115 | 678 | f-2 | 1879847 | 1880195 | 198 | NuoI | C | Formate hydrogenlyase subunit 6/NADH:ubiquinone oxidoreductase 23 kD subunit (chain I) |
|---|---|---|---|---|---|---|---|---|---|---|---|---|
| 1940 | 1880264 | 1880797 | 209114 | 208581 | 1859 | r-3 | 1880270 | 1880729 | 91 | - | S | Uncharacterized ACR |
| 1941 | 1880784 | 1881278 | 208594 | 208100 | 1501 | r-2 | 1880790 | 1881246 | 85 | - | S | Uncharacterized ACR |
| 1942 | 1881271 | 1881759 | 208107 | 207619 | 1140 | r-1 | 1881289 | 1881745 | 103 | - | S | Uncharacterized ACR |
| 1943 | 1881790 | 1882272 | 207588 | 207106 | 1139 | r-1 | 1881790 | 1882261 | 149 | - | S | Uncharacterized protein sharing a conserved domain with thiamine biosynthesis protein ThiI |
| 1944 | 1882334 | 1883542 | 207044 | 205836 | 679 | f-2 | 1882352 | 1883525 | 602 | HolB | L | ATPase involved in DNA replication |
| 1945 | 1883543 | 1884076 | 205835 | 205302 | 680 | f-2 | 1883549 | 1884074 | 176 | - | R | Predicted membrane-bound metal-dependent hydrolases |
| 1946 | 1884157 | 1885149 | 205221 | 204229 | 309 | f-1 | 1884157 | 1885144 | 503 | TrxB | O | Thioredoxin reductase |
| 1947 | 1885281 | 1886627 | 204097 | 202751 | 1058 | f-3 | 1885290 | 1886607 | 544 | ArgD | E | PLP-dependent aminotransferases |
| 1948 | 1886671 | 1887270 | 202707 | 202108 | 310 | f-1 | 1886914 | 1886980 | 30 | NarK | P | Nitrate/nitrite transporter |
| 1949 | 1887267 | 1887560 | 202111 | 201818 | 1500 | r-2 | 1887291 | 1887549 | 33 | - | R | Predicted RNA-binding proteins |
| 1950 | 1887544 | 1888218 | 201834 | 201160 | 1138 | r-1 | 1887553 | 1888216 | 254 | DeoC | F | Deoxyribose-phosphate aldolase |
| 1951 | 1888724 | 1890025 | 200654 | 199353 | 681 | f-2 | 1888727 | 1890020 | 724 | Eno | G | Enolase |

| 1952 | 1890006 | 1890557 | 199372 | 198821 | 1499 | r-2 | 1890105 | 1890522 | 58 | - | K | Predicted transcriptional regulators |
|------|---------|---------|--------|--------|------|-----|---------|---------|-----|-----|---|------|
| 1953 | 1890634 | 1894026 | 198744 | 195352 | 311 | f-1 | 1891621 | 1893961 | 221 | - | R | Predicted drug exporters of the RND superfamily |
| 1954 | 1894318 | 1894365 | 195060 | 195013 | 312 | f-1 | | | | | | |
| 1955 | 1894442 | 1895158 | 194936 | 194220 | 682 | f-2 | 1894442 | 1895156 | 386 | - | S | Uncharacterized ACR |
| 1956 | 1895222 | 1895692 | 194156 | 193686 | 1858 | r-3 | 1895252 | 1895690 | 245 | Lrp | K | Transcriptional regulators |
| 1957 | 1895730 | 1896284 | 193648 | 193094 | 1498 | r-2 | 1895730 | 1896279 | 270 | - | F | Xanthosine triphosphate pyrophosphatase |
| 1958 | 1896330 | 1896818 | 193048 | 192560 | 1497 | r-2 | 1896330 | 1896813 | 298 | - | S | Uncharacterized ACR |
| 1959 | 1896886 | 1897806 | 192492 | 191572 | 313 | f-1 | 1896895 | 1897795 | 332 | Lrp | K | Transcriptional regulators |
| 1960 | 1897803 | 1898744 | 191575 | 190634 | 1496 | r-2 | 1897803 | 1898718 | 293 | - | R | Predicted Fe-S oxidoreductases |
| 1961 | 1898830 | 1899255 | 190548 | 190123 | 1137 | r-1 | 1898833 | 1899241 | 162 | MoaE | H | Molybdopterin converting factor |
| 1962 | 1899309 | 1900178 | 190069 | 189200 | 1059 | f-3 | 1899738 | 1899900 | 33 | Acs | I | Acyl-coenzyme A synthetases/AMP-(fatty) acid ligases |
| 1963 | 1900171 | 1900881 | 189207 | 188497 | 1136 | r-1 | 1900183 | 1900876 | 335 | ThiF | H | Dinucleotide-utilizing enzymes involved in molybdopterin and thiamine biosynthesis family 2 |
| 1964 | 1901205 | 1901720 | 188173 | 187658 | 1495 | r-2 | 1901214 | 1901718 | 248 | CdsA | I | CDP-diglyceride synthetase |

EP 1 923 464 A1

255

| | | | | | | | | | | | | |
|---|---|---|---|---|---|---|---|---|---|---|---|---|
| 1965 | 1901783 | 1902706 | 187595 | 186672 | 683 | f-2 | 1901933 | 1902416 | 32 | BaeS | T | Sensory transduction histidine kinases |
| 1966 | 1902746 | 1903273 | 186632 | 186105 | 684 | f-2 | 1902941 | 1903163 | 32 | - | R | Predicted methyltransferase |
| 1967 | 1903277 | 1904434 | 186101 | 184944 | 685 | f-2 | 1903283 | 1904432 | 596 | Sun | J | tRNA and rRNA cytosine-C5-methylases |
| 1968 | 1904431 | 1905462 | 184947 | 183916 | 314 | f-1 | 1904446 | 1905403 | 212 | - | R | Predicted integral membrane protein |
| 1969 | 1905501 | 1906337 | 183877 | 183041 | 1060 | f-3 | 1905501 | 1906332 | 397 | - | R | Predicted kinase |
| 1970 | 1906334 | 1907098 | 183044 | 182280 | 1857 | r-3 | 1906616 | 1906817 | 32 | AcrR | K | Transcriptional regulator |
| 1971 | 1907089 | 1908066 | 182289 | 181312 | 1135 | r-1 | 1907089 | 1908061 | 538 | QRI7 | O | Metal-dependent proteases with possible chaperone activity |
| 1972 | 1908127 | 1909461 | 181251 | 179917 | 1134 | r-1 | 1908145 | 1909459 | 683 | - | C | Acyl-CoA synthetase (NDP forming) |
| 1973 | 1909517 | 1910014 | 179861 | 179364 | 686 | f-2 | 1909526 | 1909982 | 250 | - | R | Predicted nucleotidyltransferase |
| 1974 | 1910023 | 1910727 | 179355 | 178651 | 315 | f-1 | 1910053 | 1910725 | 372 | TpiA | G | Triosephosphate isomerase |
| 1975 | 1912010 | 1912546 | 177368 | 176832 | 687 | f-2 | 1912019 | 1912544 | 278 | BtuR | H | ATP:corrinoid adenosyltransferase |
| 1976 | 1912651 | 1912902 | 176727 | 176476 | 316 | f-1 | 1912651 | 1912900 | 138 | - | S | Uncharacterized ArCR |
| 1977 | 1912921 | 1913589 | 176457 | 175789 | 1133 | r-1 | 1913035 | 1913575 | 240 | AraD | G | Ribulose-5-phosphate 4-epimerase and related epimerases and aldolases |

| 1978 | 1913472 | 1914050 | 175906 | 175328 | 1494 | r-2 | 1913595 | 1913922 | 33 | RplV | J | Ribosomal protein L22 |
|---|---|---|---|---|---|---|---|---|---|---|---|---|
| 1979 | 1914387 | 1914812 | 174991 | 174566 | 1493 | r-2 | 1914387 | 1914810 | 226 | Lrp | K | Transcriptional regulators |
| 1980 | 1914882 | 1916204 | 174496 | 173174 | 1492 | r-2 | 1914954 | 1916193 | 541 | TrmA | J | SAM-dependent methyltransferases related to tRNA (uracil-5-)-methyltransferase |
| 1981 | 1916252 | 1916479 | 173126 | 172899 | 688 | f-2 | 1916282 | 1916402 | 28 | MarR | K | Transcriptional regulators |
| 1982 | 1916521 | 1917351 | 172857 | 172027 | 317 | f-1 | 1916572 | 1917262 | 240 | - | D | ATPases involved in chromosome partitioning |
| 1983 | 1917310 | 1917879 | 172068 | 171499 | 1132 | r-1 | 1917334 | 1917847 | 221 | PyrE | F | Orotate phosphoribosyltransferase |
| 1984 | 1918215 | 1918709 | 171163 | 170669 | 1061 | f-3 | 1918230 | 1918401 | 32 | - | R | Predicted metal-dependent membrane protease |
| 1985 | 1918693 | 1920390 | 170685 | 168988 | 1131 | r-1 | 1918711 | 1920385 | 880 | CDC9 | L | ATP-dependent DNA ligase |
| 1986 | 1920429 | 1921331 | 168949 | 168047 | 1491 | r-2 | 1920429 | 1921329 | 375 | - | R | Predicted archaeal kinases of the sugar kinase superfamily |
| 1987 | 1921407 | 1923065 | 167971 | 166313 | 1490 | r-2 | 1921407 | 1923051 | 700 | NhaC | C | Na+/H+ antiporter |
| 1988 | 1923377 | 1923970 | 166001 | 165408 | 1856 | r-3 | 1923425 | 1923968 | 301 | - | L | Uracil-DNA glycosylase |
| 1989 | 1923967 | 1924317 | 165411 | 165061 | 1130 | r-1 | 1924060 | 1924255 | 31 | SpoOJ | K | Predicted transcriptional regulators |
| 1990 | 1924478 | 1926250 | 164900 | 163128 | 689 | f-2 | 1924478 | 1926233 | 1040 | - | R | Predicted Fe-S oxidoreductases |

257

| 1991 | 1926252 | 1926566 | 163126 | 162812 | 1062 | f-3 | 1926297 | 1926447 | 28 | LysR | K | Transcriptional regulator |
|---|---|---|---|---|---|---|---|---|---|---|---|---|
| 1992 | 1926707 | 1929025 | 162671 | 160353 | 690 | f-2 | 1926872 | 1929020 | 723 | Tar | N | Methyl-accepting chemotaxis protein |
| 1993 | 1929037 | 1930491 | 160341 | 158887 | 1129 | r-1 | 1930174 | 1930438 | 30 | LysU | J | Lysyl-tRNA synthetase class II |
| 1994 | 1930573 | 1930920 | 158805 | 158458 | 318 | f-1 | 1930582 | 1930909 | 125 | - | R | Putative effector of murein hydrolase LrgA |
| 1995 | 1930917 | 1931588 | 158461 | 157790 | 1063 | f-3 | 1930917 | 1931586 | 258 | LrgB | M | Putative effector of murein hydrolase |
| 1996 | 1931535 | 1932002 | 157843 | 157376 | 1489 | r-2 | 1931541 | 1931976 | 224 | - | S | Uncharacterized ArCR |
| 1997 | 1932193 | 1932927 | 157185 | 156451 | 319 | f-1 | 1932292 | 1932925 | 325 | - | S | Uncharacterized ACR |
| 1998 | 1932928 | 1933236 | 156450 | 156142 | 1128 | r-1 | 1932997 | 1933207 | 32 | PheS | J | Phenylalanyl-tRNA synthetase alpha subunit |
| 1999 | 1933306 | 1933578 | 156072 | 155800 | 320 | f-1 | 1933306 | 1933561 | 93 | - | S | Uncharacterized ACR |
| 2000 | 1933671 | 1934051 | 155707 | 155327 | 1064 | f-3 | 1933671 | 1934034 | 98 | - | R | Predicted nucleic acid-binding protein |
| 2001 | 1934029 | 1935735 | 155349 | 153643 | 1127 | r-1 | 1934029 | 1935685 | 764 | - | J | Queuine tRNA-ribosyltransferases |
| 2002 | 1935745 | 1936650 | 153633 | 152728 | 1126 | r-1 | 1935754 | 1936648 | 433 | - | S | Uncharacterized archaeal coiled-coil domain |
| 2003 | 1936888 | 1937835 | 152490 | 151543 | 1125 | r-1 | 1936891 | 1937824 | 501 | ArcC | E | Carbamate kinase |
| 2004 | 1937965 | 1939305 | 151413 | 150073 | 1124 | r-1 | 1938043 | 1939021 | 52 | HemY | H | Protoporphyrinogen oxidase |

258

| 2005 | 1941378 | 1941863 | 148000 | 147515 | 1065 | f-3 | 1941390 | 1941849 | 78 | CcmA | Q | ABC-type multidrug transport system |
|---|---|---|---|---|---|---|---|---|---|---|---|---|
| 2006 | 1942184 | 1942507 | 147194 | 146871 | 691 | f-2 | 1942184 | 1942454 | 32 | CstA | T | Carbon starvation protein |
| 2007 | 1942618 | 1944576 | 146760 | 144802 | 1123 | r-1 | 1942618 | 1944571 | 1032 | - | C | Aldehyde:ferredoxin oxidoreductase |
| 2008 | 1944729 | 1945865 | 144649 | 143513 | 1488 | r-2 | 1944729 | 1945863 | 697 | - | S | Fructose 1,6-bisphosphatase |
| 2009 | 1945993 | 1946349 | 143385 | 143029 | 1122 | r-1 | 1946074 | 1946263 | 31 | BglX | G | Beta-glucosidase-related glycosidases |
| 2010 | 1947328 | 1948446 | 142050 | 140932 | 321 | f-1 | 1947346 | 1948276 | 98 | ArgE | E | Acetylornithine deacetylase/Succinyl-diaminopim elate desuccinylase and related deacylases |
| 2011 | 1948368 | 1949834 | 141010 | 139544 | 1066 | f-3 | 1949061 | 1949766 | 320 | CysH | EH | 3'-phosphoadenosine 5'-phosphosulfate sulfotransferase (PAPS reductase)/FAD synthetase and related enzymes COG0175 CysH |
| 2012 | 1949788 | 1951875 | 139590 | 137503 | 1121 | r-1 | 1949938 | 1951828 | 691 | - | R | Archaeal serine proteases |
| 2013 | 1951825 | 1953192 | 137553 | 136186 | 322 | f-1 | 1951831 | 1953190 | 555 | TldD | R | Predicted Zn-dependent proteases and their inactivated homologs |

| | | | | | | | | | | | |
|---|---|---|---|---|---|---|---|---|---|---|---|
| 2014 | 1953189 | 1954478 | 136189 | 134900 | f-3 | 1067 | 1953189 | 1954458 | 345 | TldD | R | Predicted Zn-dependent proteases and their inactivated homologs |
| 2015 | 1954540 | 1955208 | 134838 | 134170 | f-1 | 323 | 1954828 | 1955083 | 30 | PPX1 | C | Inorganic pyrophosphatase/exopolyphosphatase |
| 2016 | 1955253 | 1957394 | 134125 | 131984 | f-3 | 1068 | 1955337 | 1957014 | 271 | AmyA | G | Glycosidases (cyclodextrin glucanotransferase) |
| 2017 | 1957397 | 1958206 | 131981 | 131172 | r-3 | 1855 | 1957754 | 1958027 | 31 | AlsD | H | Glutamate-1-semialdehyde aminotransferase |
| 2018 | 1958454 | 1958975 | 130924 | 130403 | r-2 | 1487 | 1958538 | 1958862 | 29 | ELP3 | K | ELP3 component of the RNA polymerase II complex |
| 2019 | 1959384 | 1959980 | 129994 | 129398 | r-2 | 1486 | 1959423 | 1959549 | 29 | GCD1 | MJ | Nucleoside-diphosphate-sugar pyrophosphorylases involved in lipopolysaccharide biosynthesis/translation initiation factor eIF2B subunits COG1208 GCD1 |
| 2020 | 1959997 | 1960209 | 129381 | 129169 | r-1 | 1120 | 1960015 | 1960108 | 26 | Smc | D | Chromosome segregation ATPases |
| 2021 | 1961911 | 1965690 | 127467 | 123688 | r-1 | 1119 | 1963837 | 1964131 | 36 | RluA | J | Pseudouridylate synthases |
| 2022 | 1962226 | 1962360 | 127152 | 127018 | f-1 | 324 | 1962229 | 1962334 | 28 | | | |

260

| | | | | | | | | | | | | |
|---|---|---|---|---|---|---|---|---|---|---|---|---|
| 2023 | 1964567 | 1964629 | 124811 | 124749 | 692 | f-2 | | | | | | |
| 2024 | 1965873 | 1966658 | 123505 | 122720 | 1069 | f-3 | 1965879 | 1966644 | 381 | SgcQ | R | Predicted TIM-barrel enzyme |
| 2025 | 1966899 | 1969403 | 122479 | 119975 | 1070 | f-3 | 1968654 | 1968987 | 35 | RecB | L | ATP-dependent exoDNAse (exonuclease V) beta subunit (contains helicase and exonuclease domains) |
| 2026 | 1969396 | 1970652 | 119982 | 118726 | 325 | f-1 | 1969603 | 1969909 | 35 | AprE | O | Subtilisin-like serine proteases |
| 2027 | 1970804 | 1971262 | 118574 | 118116 | 693 | f-2 | 1970918 | 1971155 | 40 | MazG | R | Predicted pyrophosphatase |
| 2028 | 1971328 | 1971672 | 118050 | 117706 | 326 | f-1 | 1971481 | 1971613 | 37 | IlvE | EH | Branched-chain amino acid aminotransferase/4-amino-4-deoxychorismate lyase COG0115 IlvE |
| 2029 | 1971682 | 1972395 | 117696 | 116983 | 327 | f-1 | 1971904 | 1972216 | 32 | MetG | J | Methionyl-tRNA synthetase |
| 2030 | 1972493 | 1973851 | 116885 | 115527 | 694 | f-2 | 1972502 | 1973849 | 709 | CpsG | G | Phosphomannomutase |
| 2031 | 1974299 | 1975357 | 115079 | 114021 | 1854 | r-3 | 1975178 | 1975346 | 32 | - | S | Uncharacterized BCR |
| 2032 | 1975695 | 1977017 | 113683 | 112361 | 1071 | f-3 | 1975734 | 1976082 | 30 | BacA | S | Uncharacterized ACR |
| 2033 | 1976971 | 1977399 | 112407 | 111979 | 1118 | r-1 | 1977055 | 1977343 | 31 | | | |
| 2034 | 1977396 | 1977704 | 111982 | 111674 | 1485 | r-2 | 1977402 | 1977678 | 118 | ArsR | K | Predicted transcriptional regulators |
| 2035 | 1977819 | 1978400 | 111559 | 110978 | 1484 | r-2 | 1977819 | 1978377 | 218 | - | S | Uncharacterized ACR |
| 2036 | 1978397 | 1978993 | 110981 | 110385 | 1853 | r-3 | 1978406 | 1978982 | 263 | CoaE | H | Dephospho-CoA kinase |

EP 1 923 464 A1

| 2037 | 1978966 | 1979769 | 110412 | 109609 | 1117 | r-1 | 1978966 | 1979275 | 76 | - | R | Uncharacterized ATPases of the PP-loop superfamily |
|---|---|---|---|---|---|---|---|---|---|---|---|---|
| 2038 | 1979866 | 1980489 | 109512 | 108889 | 328 | f-1 | 1979929 | 1980376 | 134 | - | S | Uncharacterized membrane protein |
| 2039 | 1980484 | 1980942 | 108894 | 108436 | 1116 | r-1 | 1980496 | 1980937 | 229 | PyrI | F | Aspartate carbamoyltransferase regulatory subunit |
| 2040 | 1980946 | 1981878 | 108432 | 107500 | 1115 | r-1 | 1980946 | 1981843 | 487 | PyrB | F | Aspartate carbamoyltransferase |
| 2041 | 1981986 | 1982897 | 107392 | 106481 | 1072 | f-3 | 1982367 | 1982880 | 159 | - | S | Uncharacterized ACR |
| 2042 | 1982894 | 1983307 | 106484 | 106071 | 695 | f-2 | 1982894 | 1983305 | 193 | - | S | Uncharacterized ACR |
| 2043 | 1983573 | 1984325 | 105805 | 105053 | 1483 | r-2 | 1983972 | 1984284 | 35 | - | R | Predicted metal-binding domain (associated with helicases in Pyrococcus and Mtub) |
| 2044 | 1984369 | 1985724 | 105009 | 103654 | 1114 | r-1 | 1984369 | 1985722 | 822 | - | S | Uncharacterized ACR |
| 2045 | 1985942 | 1987522 | 103436 | 101856 | 696 | f-2 | 1986548 | 1986680 | 33 | TehA | P | Tellurite resistance protein and related permeases |
| 2046 | 1987535 | 1988848 | 101843 | 100530 | 1852 | r-3 | 1987562 | 1988771 | 205 | - | R | Uncharacterized ATPases of the AAA superfamily |
| 2047 | 1988883 | 1989671 | 100495 | 99707 | 1482 | r-2 | 1988907 | 1989048 | 30 | CpsG | G | Phosphomannomutase |
| 2048 | 1989712 | 1990701 | 99666 | 98677 | 1113 | r-1 | 1990111 | 1990264 | 30 | - | R | ATPase components of various ABC-type transport systems |
| 2049 | 1991043 | 1992029 | 98335 | 97349 | 1481 | r-2 | 1991049 | 1991937 | 223 | ThrC | E | Threonine synthase |
| 2050 | 1992178 | 1993323 | 97200 | 96055 | 1112 | r-1 | 1992334 | 1992553 | 32 | - | F | Deoxyguanosine/deoxyadenosine |

kinase

| | | | | | | | | | | | | |
|---|---|---|---|---|---|---|---|---|---|---|---|---|
| 2051 | 1993320 | 1993928 | 96058 | 95450 | 1480 | r-2 | 1993362 | 1993914 | 320 | HslV | O | Proteasome protease subunit |
| 2052 | 1993956 | 1994684 | 95422 | 94694 | 1479 | r-2 | 1993974 | 1994667 | 297 | FepC | PH | ABC-type cobalamin/Fe3+-siderophores transport systems |
| 2053 | 1994681 | 1995694 | 94697 | 93684 | 1851 | r-3 | 1994681 | 1995686 | 301 | BtuC | PH | ABC-type cobalamin/Fe3+-siderophores transport systems |
| 2054 | 1995731 | 1997062 | 93647 | 92316 | 1850 | r-3 | 1995761 | 1997033 | 280 | - | S | Uncharacterized ArCR |
| 2055 | 1997062 | 1999713 | 92316 | 89665 | 1111 | r-1 | 1997062 | 1998448 | 284 | SbcC | L | ATPase involved in DNA repair |
| 2056 | 1999710 | 2001092 | 89668 | 88286 | 1478 | r-2 | 1999710 | 2000895 | 354 | SbcD | L | DNA repair exonuclease |
| 2057 | 2001233 | 2003020 | 88145 | 86358 | 1849 | r-3 | 2001272 | 2002916 | 595 | - | R | Predicted ATPase |
| 2058 | 2003136 | 2003711 | 86242 | 85667 | 1073 | f-3 | 2003229 | 2003700 | 233 | RimI | R | Acetyltransferases |
| 2059 | 2003696 | 2004217 | 85682 | 85161 | 697 | f-2 | 2003705 | 2004215 | 243 | SEN2 | J | tRNA splicing endonuclease |
| 2060 | 2004220 | 2004576 | 85158 | 84802 | 1110 | r-1 | 2004421 | 2004565 | 31 | AcrR | K | Transcriptional regulator |
| 2061 | 2004890 | 2004943 | 84488 | 84435 | 698 | f-2 | | | | | | |
| 2062 | 2005188 | 2006615 | 84190 | 82763 | 1477 | r-2 | 2005419 | 2006613 | 699 | BioF | H | 7-keto-8-aminopelargonate synthetase and related enzymes |
| 2063 | 2006536 | 2009136 | 82842 | 80242 | 329 | f-1 | 2006722 | 2008342 | 773 | - | L | Inteins |
| 2064 | 2009133 | 2010641 | 80245 | 78737 | 1074 | f-3 | 2009142 | 2010378 | 666 | HolB | L | ATPase involved in DNA |

| | | | | | | | | | | | | |
|---|---|---|---|---|---|---|---|---|---|---|---|---|
| | | | | | | | | | | | | replication |
| 2065 | 2010697 | 2012013 | 78681 | 77365 | 330 | f-1 | 2010787 | 2011984 | 213 | - | R | Uncharacterized ATPases of the AAA superfamily |
| 2066 | 2012072 | 2012314 | 77306 | 77064 | 699 | f-2 | 2012099 | 2012246 | 34 | - | S | Uncharacterized ACR |
| 2067 | 2012311 | 2012514 | 77067 | 76864 | 1109 | r-1 | 2012377 | 2012512 | 66 | - | R | Predicted ATPase of the AAA superfamily |
| 2068 | 2012712 | 2013572 | 76666 | 75806 | 1476 | r-2 | 2012814 | 2013549 | 133 | - | R | Predicted ATPase of the AAA superfamily |
| 2069 | 2013609 | 2014661 | 75769 | 74717 | 1475 | r-2 | 2013648 | 2014656 | 423 | - | R | Predicted methyltransferase |
| 2070 | 2014525 | 2015568 | 74853 | 73810 | 1108 | r-1 | 2014672 | 2014822 | 30 | NosY | R | ABC-type transport system involved in multi-copper enzyme maturation |
| 2071 | 2015632 | 2016564 | 73746 | 72814 | 1107 | r-1 | 2015641 | 2016559 | 429 | MoaA | H | Molybdenum cofactor biosynthesis enzyme |
| 2072 | 2016684 | 2017421 | 72694 | 71957 | 1075 | f-3 | 2016699 | 2017242 | 41 | Smc | D | Chromosome segregation ATPases |
| 2073 | 2017378 | 2018802 | 72000 | 70576 | 331 | f-1 | 2017378 | 2018800 | 704 | CafA | J | Ribonucleases G and E |
| 2074 | 2019182 | 2019406 | 70196 | 69972 | 1848 | r-3 | 2019182 | 2019401 | 108 | AbrB | K | Regulators of stationary/sporulation gene expression |
| 2075 | 2019763 | 2020425 | 69615 | 68953 | 1106 | r-1 | 2019766 | 2020420 | 286 | RecA | L | RecA/RadA recombinase |

| 2076 | 2020435 | 2021076 | 68943 | 68302 | 1105 | r-1 | 2020441 | 2021074 | 272 | - | R | Predicted Zn-dependent hydrolases of the beta-lactamase fold |
|------|---------|---------|-------|-------|------|-----|---------|---------|-----|------|---|----------------------------------------------------------------|
| 2077 | 2021157 | 2021522 | 68221 | 67856 | 1076 | f-3 | 2021199 | 2021334 | 35 | - | R | Predicted GTPases |
| 2078 | 2021495 | 2022214 | 67883 | 67164 | 700 | f-2 | 2021807 | 2022128 | 33 | LrgB | M | Putative effector of murein hydrolase |
| 2079 | 2022269 | 2023111 | 67109 | 66267 | 701 | f-2 | 2022269 | 2023103 | 422 | PrsA | FE | Phosphoribosylpyrophosphate synthetase COG0462 PrsA (ribose phosphate pyrophosphokinase) |
| 2080 | 2025340 | 2025417 | 64038 | 63961 | 332 | f-1 | | | | | | |
| 2081 | 2028631 | 2028912 | 60747 | 60466 | 333 | f-1 | 2028631 | 2028814 | 32 | BaeS | T | Sensory transduction histidine kinases |
| 2082 | 2028914 | 2029489 | 60464 | 59889 | 702 | f-2 | 2028923 | 2029481 | 274 | - | S | Uncharacterized ACR |
| 2083 | 2029483 | 2030094 | 59895 | 59284 | 1104 | r-1 | 2029573 | 2030032 | 47 | SEC59 | I | Dolichol kinase |
| 2084 | 2030142 | 2031023 | 59236 | 58355 | 1474 | r-2 | 2030157 | 2030400 | 35 | FadR | K | Transcriptional regulators |
| 2085 | 2031138 | 2032727 | 58240 | 56651 | 1077 | f-3 | 2031147 | 2032725 | 770 | LysS | J | Lysyl-tRNA synthetase class I |
| 2086 | 2032734 | 2033420 | 56644 | 55958 | 1473 | r-2 | 2032734 | 2033415 | 334 | SmtA | Q R | SAM-dependent methyltransferases COG0500 SmtA |
| 2087 | 2033501 | 2034466 | 55877 | 54912 | 703 | f-2 | 2033519 | 2034458 | 515 | - | R | Predicted archaeal sugar kinases |

| 2088 | 2034330 | 2035610 | 55048 | 53768 | 1078 | f-3 | 2034459 | 2035602 | 596 | - | C | Predicted butyrate kinase |
|---|---|---|---|---|---|---|---|---|---|---|---|---|
| 2089 | 2035637 | 2036254 | 53741 | 53124 | 704 | f-2 | 2035670 | 2036246 | 336 | PorG | C | Pyruvate:ferredoxin oxidoreductase and related 2-oxoacid:ferredoxin oxidoreductases |
| 2090 | 2036331 | 2036594 | 53047 | 52784 | 1079 | f-3 | 2036331 | 2036574 | 124 | PhoU | P | Phosphate uptake regulator |
| 2091 | 2036609 | 2037244 | 52769 | 52134 | 705 | f-2 | 2036609 | 2037239 | 296 | PhoU | P | Phosphate uptake regulator |
| 2092 | 2037290 | 2038219 | 52088 | 51159 | 706 | f-2 | 2037299 | 2038217 | 544 | - | E | Asparaginase |
| 2093 | 2038219 | 2039394 | 51159 | 49984 | 334 | f-1 | 2038231 | 2039368 | 442 | | | |
| 2094 | 2039429 | 2040040 | 49949 | 49338 | 707 | f-2 | 2039429 | 2040026 | 255 | - | R | Biotin synthase-related enzyme |
| 2095 | 2039994 | 2040326 | 49384 | 49052 | 1080 | f-3 | 2040009 | 2040312 | 111 | - | R | Biotin synthase-related enzyme |
| 2096 | 2040316 | 2040816 | 49062 | 48562 | 1103 | r-1 | 2040316 | 2040739 | 45 | NrfG | R | TPR-repeat-containing proteins |
| 2097 | 2040797 | 2041732 | 48581 | 47646 | 1847 | r-3 | 2040797 | 2041718 | 498 | - | T | Predicted serine/threonine protein kinases |
| 2098 | 2043010 | 2044203 | 46368 | 45175 | 1102 | r-1 | 2043010 | 2044201 | 669 | RPT1 | O | ATP-dependent 26S proteasome regulatory subunit |
| 2099 | 2044340 | 2045170 | 45038 | 44208 | 708 | f-2 | 2044421 | 2045141 | 252 | - | S | Uncharacterized ACR |

| | | | | | | | | | | | |
|---|---|---|---|---|---|---|---|---|---|---|---|
| 2100 | 2045127 | 2046032 | 44251 | 43346 | 1472 | r-2 | 2045154 | 2045985 | 298 | Rfe | M | UDP-N-acetylmuramyl pentapeptide phosphotransferase/UDP-N-acetylglucosamine-1-phosphate transferase |
| 2101 | 2046077 | 2047399 | 43301 | 41979 | 709 | f-2 | 2046677 | 2047397 | 303 | WcaA | M | Glycosyltransferases involved in cell wall biogenesis |
| 2102 | 2047406 | 2047780 | 41972 | 41598 | 710 | f-2 | 2047478 | 2047751 | 75 | - | S | Uncharacterized ACR |
| 2103 | 2047777 | 2048313 | 41601 | 41065 | 1101 | r-1 | 2047783 | 2048305 | 325 | ComE B | F | Deoxycytidylate deaminase |
| 2104 | 2048320 | 2049099 | 41058 | 40279 | 1100 | r-1 | 2048482 | 2049088 | 175 | HtpX | O | Zn-dependent protease with chaperone function |
| 2105 | 2049106 | 2049471 | 40272 | 39907 | 1099 | r-1 | 2049106 | 2049469 | 184 | - | K | Predicted transcriptional regulator |
| 2106 | 2050697 | 2051614 | 38681 | 37764 | 711 | f-2 | 2050721 | 2051612 | 493 | PyrD | F | Dihydroorotate dehydrogenase |
| 2107 | 2051664 | 2051900 | 37714 | 37478 | 1081 | f-3 | 2051664 | 2051838 | 85 | AbrB | K | Regulators of stationary/sporulation gene expression |
| 2108 | 2051888 | 2052298 | 37490 | 37080 | 712 | f-2 | 2051894 | 2052257 | 32 | - | R | Uncharacterized proteins of PilT N-term./Vapc superfamily |
| 2109 | 2052295 | 2053014 | 37083 | 36364 | 335 | f-1 | 2052295 | 2053012 | 391 | - | R | Predicted ATPase (PP-loop superfamily) |

| 2110 | 2053125 | 2053190 | 36253 | 36188 | 1082 | f-3 | | | | | | |
|------|---------|---------|-------|-------|------|-----|---------|---------|-----|------|----|---|
| 2111 | 2055992 | 2057146 | 33386 | 32232 | 1846 | r-3 | 2055992 | 2057141 | 554 | | | |
| 2112 | 2057204 | 2057467 | 32174 | 31911 | 1845 | r-3 | 2057216 | 2057441 | 53 | - | S | Predicted membrane protein |
| 2113 | 2057477 | 2058655 | 31901 | 30723 | 1844 | r-3 | 2057486 | 2058653 | 561 | AvtA | E | PLP-dependent aminotransferases |
| 2114 | 2058742 | 2059149 | 30636 | 30229 | 1098 | r-1 | 2058769 | 2059132 | 89 | - | S | Uncharacterized ACR |
| 2115 | 2059310 | 2059501 | 30068 | 29877 | 713 | f-2 | 2059310 | 2059427 | 59 | - | K | Predicted transcriptional regulators containing the CopG/Arc/MetJ DNA-binding domain |
| 2116 | 2059560 | 2060801 | 29818 | 28577 | 1083 | f-3 | 2059560 | 2060775 | 454 | FtsZ | D | Cell division GTPase |
| 2117 | 2060819 | 2061598 | 28559 | 27780 | 714 | f-2 | 2060828 | 2061596 | 420 | Soj | D | ATPases involved in chromosome partitioning |
| 2118 | 2061501 | 2061911 | 27877 | 27467 | 1084 | f-3 | 2061690 | 2061861 | 32 | - | R | WD40 repeat protein |
| 2119 | 2061997 | 2062446 | 27381 | 26932 | 1097 | r-1 | 2062012 | 2062444 | 222 | TagD | MI | Cytidylyltransferase COG0615 TagD |
| 2120 | 2062448 | 2062966 | 26930 | 26412 | 1843 | r-3 | 2062448 | 2062964 | 292 | - | J | PUA domain (predicted RNA-binding domain) |
| 2121 | 2062966 | 2063607 | 26412 | 25771 | 1096 | r-1 | 2062981 | 2063593 | 312 | PyrF | F | Orotidine-5'-phosphate decarboxylase |
| 2122 | 2063612 | 2064214 | 25766 | 25164 | 1842 | r-3 | 2063678 | 2063858 | 35 | DeoR | K | Transcriptional regulator |
| 2123 | 2064280 | 2065428 | 25098 | 23950 | 1095 | r-1 | 2064280 | 2065423 | 586 | INO1 | I | Myo-inositol-1-phosphate |

| | | | | | | | | | | | |
|---|---|---|---|---|---|---|---|---|---|---|---|
| | | | | | | | | | | | synthase |
| 2124 | 2065471 | 2066778 | 23907 | 22600 | 1094 | r-1 | 2065492 | 2066215 | 311 | - | M | Predicted sugar nucleotidyltransferases |
| 2125 | 2066863 | 2067558 | 22515 | 21820 | 336 | f-1 | 2066878 | 2067541 | 320 | - | R | Predicted ATPases of PP-loop superfamily |
| 2126 | 2067623 | 2068384 | 21755 | 20994 | 715 | f-2 | 2067623 | 2068379 | 355 | CcmA | Q | ABC-type multidrug transport system |
| 2127 | 2068384 | 2069838 | 20994 | 19540 | 337 | f-1 | 2068387 | 2069740 | 140 | - | R | Predicted permease |
| 2128 | 2069828 | 2070184 | 19550 | 19194 | 1841 | r-3 | 2069828 | 2070182 | 176 | - | S | Uncharacterized ACR |
| 2129 | 2070189 | 2070728 | 19189 | 18650 | 1471 | r-2 | 2070216 | 2070720 | 238 | - | F | ADP-ribose pyrophosphatase |
| 2130 | 2070778 | 2071599 | 18600 | 17779 | 1093 | r-1 | 2070778 | 2071522 | 124 | RbsK | G | Sugar kinases |
| 2131 | 2071722 | 2072069 | 17656 | 17309 | 1085 | f-3 | 2071722 | 2071995 | 130 | GAR1 | J | RNA-binding protein involved in rRNA processing |
| 2132 | 2072066 | 2072986 | 17312 | 16392 | 716 | f-2 | 2072075 | 2072978 | 343 | SUA7 | K | Transcription initiation factor IIB |
| 2133 | 2073002 | 2073490 | 16376 | 15888 | 717 | f-2 | 2073002 | 2073488 | 145 | - | R | Predicted phosphoesterase |
| 2134 | 2073534 | 2073737 | 15844 | 15641 | 1470 | r-2 | 2073534 | 2073735 | 114 | HHT1 | L | Histones H3 and H4 (Histon A&B) |
| 2135 | 2074012 | 2075424 | 15366 | 13954 | 338 | f-1 | 2074111 | 2075422 | 649 | RbcL | G | Ribulose 1 |
| 2136 | 2075557 | 2076162 | 13821 | 13216 | 339 | f-1 | 2075569 | 2076085 | 224 | - | K | Predicted transcriptional regulators |
| 2137 | 2076199 | 2076411 | 13179 | 12967 | 1092 | r-1 | 2076208 | 2076409 | 113 | RPS17 | J | Ribosomal protein S17E |

| | | | | | | | | | | A | | |
|---|---|---|---|---|---|---|---|---|---|---|---|---|
| 2138 | 2076528 | 2076959 | 12850 | 12419 | 1086 | f-3 | 2076528 | 2076909 | 182 | - | S | Uncharacterized ArCR |
| 2139 | 2076986 | 2077663 | 12392 | 11715 | 718 | f-2 | 2076995 | 2077661 | 351 | - | S | Uncharacterized ACR |
| 2140 | 2077703 | 2078152 | 11675 | 11226 | 719 | f-2 | 2077772 | 2077931 | 31 | CcmC | O | ABC-type transport system involved in cytochrome c biogenesis |
| 2141 | 2078164 | 2078964 | 11214 | 10414 | 1091 | r-1 | 2078167 | 2078932 | 275 | SplB | L | DNA repair photolyase |
| 2142 | 2079001 | 2080026 | 10377 | 9352 | 1090 | r-1 | 2079019 | 2080021 | 335 | NrfG | R | TPR-repeat-containing proteins |
| 2143 | 2080319 | 2082169 | 9059 | 7209 | 720 | f-2 | 2080319 | 2082164 | 1008 | NrdD | F | Oxygen-sensitive ribonucleoside-triphosphate reductase |
| 2144 | 2082376 | 2082897 | 7002 | 6481 | 340 | f-1 | 2082376 | 2082874 | 194 | PflA | O | Pyruvate-formate lyase-activating enzyme |
| 2145 | 2082919 | 2083284 | 6459 | 6094 | 1089 | r-1 | 2082925 | 2083282 | 171 | - | S | Uncharacterized ACR |
| 2146 | 2083288 | 2084007 | 6090 | 5371 | 1088 | r-1 | 2083288 | 2083987 | 359 | Cof | R | Predicted hydrolases of the HAD superfamily |
| 2147 | 2084057 | 2085316 | 5321 | 4062 | 1840 | r-3 | 2084090 | 2085308 | 503 | - | S | Uncharacterized ACR |
| 2148 | 2085470 | 2087110 | 3908 | 2268 | 721 | f-2 | 2085470 | 2087042 | 899 | GroL | O | Chaperonin GroEL (HSP60 family)(Chaperonin B) |
| 2149 | 2087216 | 2088568 | 2162 | 810 | 1839 | r-3 | 2087216 | 2088566 | 753 | Sun | J | tRNA and rRNA cytosine-C5-methylases |

| 2150 | 2088670 | 2088921 | 708 | 457 | 341 | f-1 | 2088691 | 2088823 | 30 | FliA | K | DNA-directed RNA polymerase specialized sigma subunit |
|------|---------|---------|-----|-----|-----|-----|---------|---------|----|------|---|----------------------------------------------------|
| 2151 | 2088905 | 2089378 | 473 | 0 | 722 | f-2 | 2088911 | 2089364 | 73 | - | R | Predicted nucleic acid-binding protein |

In Table 2, f-1 through f-3, as described as reading frames, refers to open reading frames in the sense strand, and r-1 through r-3 refers to open reading frames in the antisense strand. In the classification, J refers to polypeptides relating to translation, ribosome structure or biological development; K refers to polypeptides relating to transcription; L refers to polypeptides relating to DNA replication, recombination or repair; D refers to polypeptides relating to chromosomal fractionation; O refers to polypeptides relating to post-translational events , protein metabolism turnover or chaperone proteins; M refers to polypeptides relating to cellular envelope biological development or outer membranes; N refers to polypeptides relating to cellular movement or secretion; P refers to polypeptides relating to inorganic ion transportation or metabolism; T refers to polypeptides relating to signaling mechanisms; C refers to polypeptides relating to energy production and conversion; G refers to polypeptides relating to carbohydrate transportation and metabolism; E refers to polypeptides relating to amino acid transportation and metabolism; F refers to polypeptides relating to nucleotide transportation and metabolism; H refers to polypeptides relating to coenzyme metabolism; I refers to polypeptides relating to lipid metabolism; Q refers to polypeptides relating to secondary metabolites biosynthesis, transportation or catabolism; R refers to polypeptides predicted to have general function; and S refers to polypeptides with an unknown function. Classification is interim, and two or more classifications may be appropriate, and in such cases, both letters are described therein.

(Biomolecule chip)

In another aspect, the present invention provides a biomolecule chip. The present biomolecule chip comprises a substrate and at least one nucleic acid molecule having at least eight contiguous or non-contiguous nucleotide sequences of the sequence set forth in SEQ ID NOs: 1, or 1087, or a variant thereof located therein.
Accordingly, in one embodiment, the present invention provides a nucleic acid molecule comprising a) a sequence set forth in SEQ ID NO: 1 or 1087, or a complementary sequence or fragment thereof; (b) a polynucleotide encoding a polypeptide consisting of an amino acid sequence selected from the group consisting of SEQ ID NO: 2-341, 343-722, 724-1086, 1088-1468, 1470-1837 and 1839-2157, or a fragment thereof; (c) a polynucleotide encoding a polypeptide having an amino acid sequence selected from the group consisting of SEQ ID NO: 2-341, 343-722, 724-1086, 1088-1468, 1470-1837 and 1839-2157, or a variant thereof having at least one mutation selected from the group consisting of one or more amino acid substitutions, additions, and deletions, wherein the variant polypeptide has biological activity; (d) a polynucleotide capable of hybridizing to a polynucleotide of any of (a) - (c), and encoding a polypeptide having an amino acid sequence having at least 70% identity to any one of the polypeptides of (a) to (c), wherein the polypeptide has biological activity.
In one preferred embodiment, the number of substitutions, additions and deletions described in (c) above may be limited to, for example, preferably 50 or less, 40 or less, 30 or less, 20 or less, 15 or less, 10 or less, 9 or less, 8 or less, 7 or less, 6 or less, 5 or less, 4 or less, 3 or less, or 2 or less. The number of substitutions, additions and deletions is preferably small, but may be large as long as the biological activity is maintained (preferably, the activity is similar to or substantially the same as that as set forth in Table 2, or an abnormal activity thereof (for example, inhibition of normal biological activity).
In other preferable embodiments, the biological activities possessed by the polypeptides of the present invention include, but are not limited to, for example, interactions with specific antibodies against at least one polypeptide selected from the group consisting of SEQ ID NOs: 2-341, 343-722, 724-1086, 1088-1468, 1470-1837 and 1839-2157; a biological activity listed in Table 2, and the like. These may be measured by, for example, immunological assays, labeling assays and the like.
In other preferable embodiments, allelic gene variants as described in (d) above, advantageously have at least 99 % homology to the nucleic acid sequences set forth in SEQ ID NO: 1 or 1087, or a portion thereof (for example, when the reading frame of Table 2 is f-1, f-2 or f-3, the nucleic acid molecule has a sequence from the position of nucleic acid number (sense strand, start) of SEQ ID NO: 1 of Table 2, to the position of nucleic acid number (sense strand, stop), or when the reading frame of Table 2 is r-1, r-2 or r-3, the nucleic acid molecule has a sequence from the position of nucleic acid number (antisense strand, start) of SEQ ID NO: 1087 of Table 2, to the position of nucleic acid number (antisense strand, stop)).
If a gene sequence database for the subject species is available, the above-mentioned species homologs may be identified by searching against the database using a gene sequence of the present invention as a query sequence. Alternatively, a nucleic acid sequence of the present invention, or a portion thereof (for example, when the reading frame of Table 2 is f-1, f-2 or f-3, the nucleic acid molecule has a sequence from the position of nucleic acid number (sense strand, start) of SEQ ID N0: 1 of Table 2, to the position of nucleic acid number (sense strand, stop), or when the reading frame of Table 2 is r-1, r-2 or r-3, the nucleic acid molecule has a sequence from the position of nucleic acid number (antisense strand, start) of SEQ ID NO: 1087 of Table 2, to the position of nucleic acid number (antisense strand, stop)) may be used as a probe or primer to screen a genetic library of the subject species for identification thereof. Such identification methods are well known in the art, and are also described in references cited herein. Species homologs have preferably at least 30 % homology to a nucleic acid sequence set forth in SEQ ID NO: 1 or 1087, or a portion thereof (for example, when the reading frame of Table 2 is f-1, f-2 or f-3, the nucleic acid molecule has a sequence from the position of nucleic acid number (sense strand, start) of SEQ ID NO: 1 of Table 2, to the position of nucleic acid number (sense strand, stop), or when the reading frame of Table 2 is r-1, r-2 or r-3, the nucleic acid molecule has a sequence from the position of nucleic acid number (antisense strand, start) of SEQ ID NO: 1087 of Table 2, to the position of nucleic acid number (antisense strand, stop)). Preferably, the species homologs of the present invention may have at least about 40 % homology, at least about 50 % homology, at least about 60 % homology, at least about 70 % homology, at least about 80 % homology, at least about 90 % homology, at least about 95 % homology, at least about 98 % homology with the above-mentioned standard sequence.

In preferable embodiments, identity against at least one polynucleotide of the above (a)- (e) or the complementary sequence thereto, may be at least about 80 %, more preferably at least 90 %, still more preferably at least about 98 %,most preferably at least about 99 %. Most preferably, the gene sequence of the present invention, has a sequence 100 % identical to a nucleic acid sequence set forth in SEQ ID NO: 1 or 1087, or a portion thereof (for example, when the reading frame of Table 2 is f-1, f-2 or f-3, the nucleic acid molecule has a sequence from the position of nucleic acid number (sense strand, start) of SEQ ID NO: 1 of Table 2, to the position of nucleic acid number (sense strand, stop), or when the reading frame of Table 2 is r-1, r-2 or r-3, the nucleic acid molecule has a a sequence from the position of nucleic acid number (antisense strand, start) of SEQ ID NO: 1087 of Table 2, to the position of nucleic acid number (antisense strand, stop)).

In a preferred embodiment, the nucleic acid molecule of the present invention encoding the gene of the present invention may have a length of at least 8 contiguous nucleotides. The appropriate nucleotide length of the nucleic acid molecule of the present invention may vary depending on the purpose of use of the present invention. More preferably, the nucleic acid molecule of the present invention may have a length of at least 10 contiguous nucleotides, even more preferably at least 15 contiguous nucleotides, still even more preferably at least 20 contiguous nucleotides, and yet still even more preferably at least 30 contiguous or non-contiguous nucleotides. These lower limits of the nucleotide length may be present between the above-specified numbers (e.g., 9, 11, 12, 13, 14, 16, and the like) or above the above-specified numbers (e.g., 21, 22, ... 30, and the like). The upper limit of the length of the polypeptide of the present invention may be greater than or equal to the full length of the sequence as set forth in SEQ ID NO. 1, as long as the polynucleotide can be used for the intended purpose (e.g. antisense, RNAi, marker, primer, probe, capable of interacting with a given agent). Alternatively, when the nucleic acid molecule of the present invention is used as a primer, the nucleic acid molecule typically may have a nucleotide length of at least about 8, preferably a nucleotide length of about 10. When used as a probe, the nucleic acid molecule typically may have a nucleotide length of at least about 15, and preferably a nucleotide length about 17.

In one embodiment, the nucleic acid molecule encoding the gene of the present invention comprises the entire range of the open reading frame of SEQ ID NO: 1. More preferably, the nucleic acid molecule of the present invention consists of at least one sequence set forth in SEQ ID NO: 1 or 1087, or a portion thereof (for example, when the reading frame of Table 2 is f-1, f-2 or f-3, the nucleic acid molecule has a sequence from the position of nucleic acid number (sense strand, start) of SEQ ID NO: 1 of Table 2, to the position of nucleic acid number (sense strand, stop), or when the reading frame of Table 2 is r-1, r-2 or r-3, the nucleic acid molecule has a sequence from the position of nucleic acid number (antisense strand, start) of SEQ ID NO: 1087 of Table 2, to the position of nucleic acid number (antisense strand, stop)).

Accordingly, the biomolecule chip of the present invention preferably uses nucleic acid molecules or variants thereof which encompass the sequence set forth in SEQ ID NO: 1 or 1087. By using nucleic acid molecules of such an encompassing nature, it is possible to analyze functions of the genome in an exhaustive manner. This was first made possible by reading the entire sequence of the genome, and thus has not been attained by prior art technologies, and thus should present significant effects.

In other embodiments, the nucleic acid molecules, or variants thereof of the present invention, to be used in the biomolecule chip, comprise any open reading frame, as set forth in SEQ ID NO: 1 or 1087. As such, the effect by which any open reading frame can be selected on the genome, should be recognized as significant as this has not been possible using prior art technology. In particular, it should be noted that analysis of the entire genome of an organism living in high temperature environments, such as at 90 °C, is possible.

In another embodiment, the nucleic acid molecule or variants thereof, to be used in the biomolecule chip of the present invention, preferably comprise substantially all the open reading frames set forth in SEQ ID NO: 1 or 1087. As used herein the term "substantially all" refers to a number sufficient for global genomic needs. Accordingly, the term "substantially all" is not necessarily all, and depending on the purpose of interest, those skilled in the art may select an appropriate number therefor. Exemplary "substantially all" includes, but is not limited to, for example, at least about 30 %, preferably at least about 40 %, more preferably at least about 50 %, still preferably at least about 80 %, still more preferably at least about 90 %, yet more preferably at least about 95 %, at least about 96 %, at least about 97 %, at least about 98 %, at least about 99 %, and the like, of the total number of entire open reading frames. In other typical examples of the present invention, substantially all may be about 900 genes whose function has already been identified in the present application. The effect by which analysis of substantially all the open reading frame is allowed, is not attainable using prior art technologies.

Accordingly, in another preferable embodiment, the nucleic acid molecule or variants thereof, to be used in the biomolecule chip of the present invention, comprises a sequence encoding at least one sequence selected from the group consisting of SEQ ID NO: 2-341, 343-722, 724-1086, 1088-1468, 1470-1837 and 1839-2157.

In other preferable embodiments, the nucleic acid molecules or variants thereof comprise substantially all sequences encoding sequences selected from the group consisting of SEQ ID NOs: 2-341, 343-722, 724-1086, 1088-1468, 1470-1837 and 1839-2157.

In more preferable embodiments, the nucleic acid molecule or the variant thereof, to be used as the biomolecule of the present invention, comprises at least an eight contiguous nucleotide length of substantially all the sequences encoding sequences selected from the group consisting of SEQ ID NOs: 2-341, 343-722, 724-1086, 1088-1468, 1470-1837 and 1839-2157. As used herein the selection of the sequence may be determined in consideration of a variety of factors as described above.

A nulciec acid molecule at least eight contiguous nucleotides in length may comprise a sequence unique to the hyperthermophillic archeabacteria, and thus is advantageous for such analyses.

In another preferable embodiment, the nucleic acid molecule or the variant thereof to be used as the biomolecule of the present invention, comprises at least a fifteen contiguous nucleotide length of substantially all the sequences encoding sequences selected from the group consisting of SEQ ID NOs: 2-341, 343-722, 724-1086, 1088-1468, 1470-1837 and 1839-2157. A nucleic acid molecule at least fifteen nucleotides in length allows substantially specific identification of sequences unique to the hyperthermophillic archeabacteria, and thus is advantageous for such analyses.

In another more preferable embodiment, the nucleic acid molecule or the variant thereof, to be used in the biomolecule chip of the present invention, comprises at least a thirty contiguous or non-contiguous nucleotide length of substantially all the sequences encoding sequences selected from the group consisting of SEQ ID NOs: 2-341, 343-722, 724-1086, 1088-1468, 1470-1837 and 1839-2157. A nucleic acid molecule at least thirty contiguous or non-contiguous nucleotides in length allows substanitally specific identification of sequences unique to the hyperthermophillic archeabacteria, even when used as a probe, and thus is advantageous for such analyses.

In another more preferable embodiment, the nucleic acid molecule or the variant thereof to be used in the biomolecule chip of the present invention, comprises substantially all the sequences encoding sequences selected from the group consisting of SEQ ID NOs: 2-341, 343-722, 724-1086, 1088-1468, 1470-1837 and 1839-2157, or sequences with one or more amino acid substitution, addition and/or deletion thereto. Such sequences allow exhaustive analyses of nucleic acid molecules encoding polypeptides included or suspected to be included in an archeabacteria, and thus are advantageous for such analyses.

In another more preferable embodiment, the nucleic acid molecule or the variant thereof to be used in the biomolecule chip of the present invention, comprises at least an eight contiguous nucleotide length of substantially all the sequences encoding sequences selected from the group consisting of SEQ ID NOs: 2-341, 343-722, 724-1086, 1088-1468, 1470-1837 and 1839-2157, or sequences with one or more amino acid substitution, addition and/or deletion thereto. Chips containing such sequences may be used for analysis of the behavior of all genes.

In another more preferable embodiment, the nucleic acid molecule or the variant thereof to be used in the biomolecule chip of the- present invention, comprises a molecule where the reading frame of Table 2 is f-1, f-2 or f-3, has a sequence from the position of nucleic acid number (sense strand, start) of SEQ ID NO: 1 of Table 2, to the position of nucleic acid number (sense strand, stop) or a sequence having at least 70 % homology thereto, or when the reading frame of Table 2 is r-1, r-2 or r-3, the nucleic acid molecule has a sequence from the position of nucleic acid number (antisense strand, start) of SEQ ID NO: 1087 of Table 2, to the position of nucleic acid number (antisense strand, stop) or a sequence having at least 70 % homology thereto. Such sequences contain open reading frames actually possessed by hyperthermophillic archeabacteria and thus provide an accurate assay at the genomic level. Thus, the present embodiment may be used for global analysis at such a genomic level.

In another embodiment, the substrate comprising the biomolecule of the present inventin is addressable. Giving addresses facilitates the analyses of all of the nucleic acid molecules. Methods for addressing are well known in the art.

In another aspect, the present invention provides a biomolecule chip with a polypeptide or a variant thereof, having at least an amino acid sequence selected from the group consisting of SEQ ID NO: 2-341, 343-722, 724-1086, 1088-1468, 1470-1837 and 1839-2157, or a sequence having at least 70 % homology thereto, located therein.

Accordingly, in one embodiment, the present invention provides a polypeptide of (a) a polypeptide consisting of an amino acid sequence selected from the group consisting of SEQ ID NO: 2-341, 343-722, 724-1086, 1088-1468, 1470-1837 and 1839-2157, or a fragment thereof; (b) a polypeptide having an amino acid sequence selected from the group consisting of SEQ ID NO: 2-341, 343-722, 724-1086, 1088-1468, 1470-1837 and 1839-2157, or a variant thereof having at least one mutation selected from the group consisting of one or more amino acid substitutions, additions, and deletions, wherein the variant polypeptide has a biological activity; (c) a polypeptide encoded by a sequence or splicing variants or allelic variants thereof, wherein the nucleic acid molecule or the variant thereof, when the reading frame of Table 2 is f-1, f-2 or f-3, has a sequence from the position of nucleic acid number (sense strand, start) of SEQ ID NO: 1 of Table 2, to the position of nucleic acid number (sense strand, stop), or when the reading frame of Table 2 is r-1, r-2 or r-3, the nucleic acid molecule has a sequence from the position of nucleic acid number (antisense strand, start) of SEQ ID NO: 1087 of Table 2, to the position of nucleic acid number (antisense strand, stop); (d) a polypeptide of at least one species homolog of an amino acid sequence selected from the group consisting of SEQ ID NO: 2-341, 343-722, 724-1086, 1088-1468, 1470-1837 and 1839-2157; or (e) a polypeptide having an amino acid sequence having at least 70% identity to any one of the polypeptides of (a) to (c), wherein the polypeptide has biological activity.

In one preferred embodiment, the number of substitutions, additions and deletions described in (b) above may be limited to, for example, preferably 50 or less, 40 or less, 30 or less, 20 or less, 15 or less, 10 or less, 9 or less, 8 or less, 7 or less, 6 or less, 5 or less, 4 or less, 3 or less, or 2 or less. The number of substitutions, additions and deletions is preferably small, but may be large as long as biological activity, is maintained (preferably, the activity is similar to or substantially the same as that of the biological activity of a normal genetic type of a polypeptide having an amino acid sequence selected from the group consisting of SEQ ID NO: 2-341, 343-722, 724-1086, 1088-1468, 1470-1837 and 1839-2157, or an abnormal activity of a polypeptide having an amino acid sequence selected from the group consisting of SEQ ID NO: 2-341, 343-722, 724-1086, 1088-1468, 1470-1837 and 1839-2157).

In another preferred embodiment, the above-described splicing or allelic variants of the polypeptides described in (c) above preferably have at least about 99% homology to a polypeptide having an amino acid sequence selected from the group consisting of SEQ ID NO: 2-341, 343-722, 724-1086, 1088-1468, 1470-1837 and 1839-2157.

In another preferable embodiment, the above-mentioned species homologs preferably have at least about 30 % homology to a polypeptide having an amino acid sequence selected from the group consisting of SEQ ID NO: 2-341, 343-722, 724-1086, 1088-1468, 1470-1837 and 1839-2157. Preferably, the species homologs have homology to the above standard sequence with at least about 40 % homology, at least about 50 % homology, at least about 60 % homology, at least about 70 % homology, at least about 80 % homology, at least about 90 % homology, at least about 95 % homology, at least about 98 % homology.

When a genetic sequence database of the species exists, the above species homologs may be identified by performing a search against the database using a polypeptide having an amino acid sequence selected from the group consisting of SEQ ID NO: 2-341, 343-722, 724-1086, 1088-1468, 1470-1837 and 1839-2157, as a query sequence. Alternatively, the entire amino acid sequence of a polypeptide having an amino acid sequence selected from the group consisting of SEQ ID NO: 2-341, 343-722, 724-1086, 1088-1468, 1470-1837 and 1839-2157, or a portion thereof, may be used as a probe or primer for screening a genetic library of the species. Such methods for identification are well known in the art, and are described in the references cited herein. Species homologs have preferably at least about 30 % homology when the reading frame of Table 2 is f-1, f-2 or f-3, a sequence from the position of nucleic acid number (sense strand, start) of SEQ ID NO: 1 of Table 2, to the position of nucleic acid number (sense strand, stop), or when the reading frame of Table 2 is r-1, r-2 or r-3, a sequence from the position of nucleic acid number (antisense strand, start) of SEQ ID NO: 1087 of Table 2, to the position of nucleic acid number (antisense strand, stop) ; or an amino acid sequence selected from the group consisting of SEQ ID NO: 2-341, 343-722, 724-1086, 1088-1468, 1470-1837 and 1839-2157. Preferably, the species homologs may have homology to the above standard sequence with at least about 40 % homology, at least about 50 % homology, at least about 60 % homology, at least about 70 % homology, at least about 80 % homology, at least about 90 % homology, at least about 95 % homology, at least about 98 % homology.

In another preferable embodiment, the biological activity possessed by the variant polypeptide in (e) above, includes, but is not limited to, for example, interaction with an antibody specific to the polypeptide having an amino acid sequence selected from the group consisting of SEQ ID. NO: 2-341, 343-722, 724-1086, 1088-1468, 1470-1837 and 1839-2157, or a fragment thereof; an enzymatic function as described in Table 2; and the like. Such functions may be measured by enzymatic assays, immunological assays, fluorescence assays and the like.

In preferable embodiments, the above-described homology to any one of the polypeptides described in (a) to (d) above may be at least about 80%, more preferably at least about 90%, even more preferably at least about 98%, and most preferably at least about 99%. Most preferably, the genetic product of the present invention is a sequence consisting of at least one amino acid sequence selected from the group consisting of SEQ ID NO: 2-341, 343-722, 724-1086, 1088-1468, 1470-1837 and 1839-2157.

The polypeptide of the present invention typically has a sequence of at least 3 contiguous amino acids. The amino acid length of the polypeptide of the present invention may be short as long as the peptide is suitable for an intended application, but preferably a longer sequence may be used. Therefore, the amino acid length may be preferably at least 4, more preferably at least 5, at least 6, at least 7, at least 8, at least 9 and at least 10, even more preferably at least 15, and still even more preferably at least 20. These lower limits of the amino acid length may be present between the above-specified numbers (e.g., 11, 12, 13, 14, 16, and the like) or above the above-specified numbers (e.g., 21, 22, ..., 30, and the like). The upper limit of the length of the polypeptide of the present invention may be greater than or equal to the full length of the sequence as set forth in amino acid sequence selected from the group consisting of SEQ ID NO: 2-341, 343-722, 724-1086, 1088-1468, 1470-1837 and 1839-2157 as long as the peptide is capable of interacting with a given agent. As used herein, more preferable forms and constitutions with respect to the sequence to be included, may take any embodiment described herein above for preferable forms and constitutions.

The genetic product of the polypeptide form of the present invention is preferably labeled or may be capable of being labeled. Such a genetic product which is labeled or may be capable of being labeled, may be used to measure the antibody levels against the genetic product, thereby allowing indirect measurement of the level of expression of the genetic product.

In another preferable embodiment, the polypeptide or the variant thereof to be located on to a support of the biomolecule chip of the present invention has a length of at least three contiguous amino acids of an amino acid sequence selected from the group consisting of SEQ ID NO: 2-341, 343-722, 724-1086, 1088-1468, 1470-1837 and 1839-2157, or a sequence having at least 70 % homology thereto. By having a sequence of at least three contiguous three amino acids, it is possible to constitute a specific epitope. As used herein, preferable forms of the sequence to be used, takes any form described herein above.

In preferable embodiments, the polypeptide or the variant thereof to be located on a support of the biomolecule chip of the present invention, has a length of at least eight contiguous amino acids of an amino acid sequence selected from the group consisting of SEQ ID NO: 2-341, 343-722, 724-1086, 1088-1468, 1470-1837 and 1839-2157, or a sequence having at least 70 % homology thereto. By having a sequence of at least eight contiguous amino acids, it is possible to constitute specific epitopes in a more efficient manner. As used herein, preferable forms and constitutions of the sequence to be used, takes any form described herein above.

In preferable embodiments, the polypeptide or the variant thereof to be located on a support of the biomolecule chip of the present invention, has a length of at least three contiguous or non-contiguous amino acids of an amino acid sequence selected from the group consisting of SEQ ID NO: 2-341, 343-722, 724-1086, 1088-1468, 1470-1837 and 1839-2157, or a sequence having at least 70 % homology thereto, and having a biological function. As used herein, the biological activities preferably include a function described in Table 2. In another embodiment, the biological activity includes epitope activity. As used herein, preferable forms and constitutions relating to preferable sequences may have the advantage of any of the forms and constitutions described herein above.

In another aspect, the present invention provides a storage medium having stored therein, information about a nucleic acid sequence of a nucleic acid molecule having a sequence of at least eight contiguous or non-contiguous nucleotides of the sequence set forth in SEQ ID NOs: 1 or 1087, or a variant thereof. As used herein, the information about the nucleic acid sequence includes, in addition to information about the nucleic acid sequence *per se,* information relating to that set forth in a conventional sequence listing. Such additional information includes, but is not limited to, for example, coding region, intron region, specific expression, promoter sequence and activity, biological function, similar sequences, homologs, reference information, and the like.

In a preferable embodiments, the nucleic acid molecule or the variant thereof to be stored in the storage medium of the present invention, comprises a sequence of at least eight contiguous nucleotides of substantially all the sequences encoding sequences selected from the group consisting of SEQ ID NOs: 2-341, 343-722, 724-1086, 1088-1468, 1470-1837 and 1839-2157, or sequences with one or more amino acid substitution, addition and/or deletion thereto. Such information could not be provided by prior art technologies, and thus should be recognized to be an effect attained for the first time by the present invention.

In other embodiments, the reading frame of Table 2 is f-1, f-2 or f-3, the nucleic acid molecule or the variant thereof to be recorded in the storage medium of the present invention, has a sequence from the position of nucleic acid number (sense strand, start) of SEQ ID NO: 1 of Table 2, to the position of nucleic acid number (sense strand, stop) or a sequence having at least 70 % homology thereto, or when the reading frame of Table 2 is r-1, r-2 or r-3, the nucleic acid molecule has a sequence from the position of nucleic acid number (antisense strand, start) of SEQ ID NO: 1087 of Table 2, to the position of nucleic acid number (antisense strand, stop) or a sequence having at least 70 % homology thereto. Such storage medium with information recorded thereon has never been conventionally provided, and thus the storage medium of the present invention has an advantageous effect in allowing analysis of the entire genome. Preferably, the storage medium of the present invention includes information about substantially all the open reading frame sequences. As used herein, preferable forms and constitutions relating such preferable sequences may take advantages of any forms and constitutions described herein above.

In another aspect, the present invention provides a storage medium, comprising information about a polypeptide or a variant thereof having at least an amino acid sequence selected from the group consisting of SEQ ID NO: 2-341, 343-722, 724-1086, 1088-1468, 1470-1837 and 1839-2157, or a sequence having at least 70 % homology thereto, located therein. As used herein, preferable forms and constitutions relating such preferable sequences may take advantage of any forms and constitutions described herein above.

In another embodiment, the polypeptide or the variant thereof to be stored in the storage medium of the present invention with respect to information thereabout, has a sequence of at least three contiguous amino acids of at least an amino acid sequence selected from the group consisting of SEQ ID NO: 2-341, 343-722, 724-1086, 1088-1468, 1470-1837 and 1839-2157, or a sequence having at least 70 % homology thereto. As used herein, the referable forms and constitutions of such preferable sequences may take advantage of any of the forms and constitutions described herein above.

In another embodiment, the polypeptide or the variant thereof to be stored in the storage medium of the present invention with respect to information thereabout, has a sequence of at least eight contiguous amino acids of at least an amino acid sequence selected from the group consisting of SEQ ID NO: 2-341, 343-722, 724-1086, 1088-1468, 1470-1837 and 1839-2157, or a sequence having at least 70 % homology thereto. As used herein, the preferable forms and constitutions of such preferable sequences may take advantages of any of the forms and constitutions described herein above.

In another embodiment, the polypeptide or the variant thereof to be stored in the storage medium of the present invention with respect to information thereabout, has a sequence of at least three contiguous or non-contiguous amino acids of an amino acid sequence selected from the group consisting of SEQ ID NO: 2-341, 343-722, 724-1086, 1088-1468, 1470-1837 and 1839-2157, or a sequence having at least 70 % homology thereto, having biological function. As used herein, preferable forms and constitutions of such preferable sequences may take advantages of any of the forms and constitutions described herein above.

In another embodiment, the biological activity to be included in the storage medium of the present invention with respect to information thereof, comprises a function set forth in Table 2. As used herein, preferable forms and constitutions of such preferable activities may take advantage of any forms and constitutions described herein above.

In another aspect, the present invention provides a biomolecule chip having at least one antibody against a polypeptide or a variant thereof, located on a substrate, the polypeptide or the variant thereof comprises at least one amino acid sequence of sequences selected from the group consisting of SEQ ID NOs: 2-341, 343-722, 724-1086, 1088-1468, 1470-1837 and 1839-2157, or a sequence having at least 70 % homology thereto. As used herein, preferable forms and constitutions of preferable sequences may take advantage of any forms and constitutions described herein above.

In another aspect, the present invention provides an RNAi molecule having a sequence homologous to a reading frame sequence wherein, when the reading frame of Table 2 is f-1, f-2 or f-3, the reading frame sequence has a sequence from the position of nucleic acid number (sense strand, start) of SEQ ID NO: 1 of Table 2, to the position of nucleic acid number (sense strand, stop) or a sequence having at least 70 % homology thereto, or when the reading frame of Table 2 is r-1, r-2 or r-3, the reading frame sequence has a a sequence from the position of nucleic acid number (antisense strand, start) of SEQ ID NO: 1087 of Table 2, to the position of nucleic acid number (antisense strand, stop) or a sequence having at least 70 % homology thereto. As used herein, such an RNAi molecule may take any form described herein above in detail, and those skilled in the art may make and use any appropriate RNAi molecule once the sequence information of the present invention is given.

In preferable embodiments, the RNAi molecule of the present invention is an RNA or a variant thereof comprising double-stranded portion of at least 10 nucleotide length.

In a more preferable embodiment, the RNAi molecule comprises a 3' overhand.

In another preferable embodiment, the above-3' overhang terminus has a DNA molecule of two or more nucleotides in length.

In other preferable embodiments, the 3' overhang has a DNA molecule of 2-4 nucleotides.

Such RNAi molecules may be used for suppressing particular functions of hyperthermophillic archeabacteria. Any RNAi molecules may be used which were not attainable by the prior art, and thus the present invention attains significant effects in this regard.

All patents, patent applications, journal articles and other references mentioned herein are incorporated by reference in their entirety.

The present invention is heretofore described with reference to preferred embodiments to facilitate understanding of the present invention. Hereinafter, the present invention will be described by way of examples. Examples described below are provided for illustrative purposes only. Accordingly, the scope of the present invention is limited only by the appended claims.

EXAMPLES

Hereinafter, the present invention will be described in more detail by way of examples. Thus it should be understood that the present invention is not limited to the examples below.

(EXAMPLE 1: Genomic sequencing)

(Preparation of chromosomal DNA the KOD-1 strain)

The KOD-1 strain was inoculated into 1000 ml of 0.5 X 2216 Marine Broth medium as described in Appl. Environ. Microbiol. 60 (12), 4559-4566 (1994) (2216 Marine Broth : 18.7g/L. PIPES 3.48g/L, CaCl$_2$·H$_2$O 0.725g/L, 0.4 mL 0.2% resazurin, 475mL artificial sea water (NaCl 28.16 g/L, KCl 0.7 g/L, MgCl$_2$-6H$_2$O 5.5 g/L, MgSO$_4$·7H$_2$O 6.9 g/L), distilled water 500 mL, pH7.0) and cultured using 2 liter fermenter. During culture, nitrogen gas was introduced into the fermenter, and was maintained at an internal pressure of 0.1 kg/cm$^2$. Culture was maintained at the temperature of 85 ± 1°C for fourteen hours. Further, the culture was carried out by static culture, and no aeration and agitation was performed with the nitrogen gas in the culture. After culture, the bacteria (about 1,000 ml) were recovered by centrifugation at 10,000 rpm for 10 minutes.

One g of the resulting bacterial pellet was suspended in 10 ml of Solution A (50 mM Tris-HCl, 50mM EDTA, pH 8.0), and centrifuged (8,000 rpm, 5 minutes, 4 °C) to pellet the bacteria and suspended in 3 ml of Solution A containing 15 % sucrose, maintained the temperature at 37 °C for 30 minutes, and added 3 ml of Solution A containing 1 % N-lauryl sarcosine thereto. 5.4 g of cesium chloride and 300 $\mu$l of 10 mg/ml of ethidium bromide were added to the solution, and ultracentrifuged at 55,000 rpm, 16 hours, at 18 °C and chromosomal DNA was fractionated. The resultant chromosomal DNA fractions were subjected to n-butanol extraction to remove ethidium bromide, and dialyzed against TE solution (10 mM Tris-HCl (pH 8.0), 0.1 mM EDTA) to yield chromosomal DNA.

(Screening/sequencing analysis of the chromosomal library)

Determination of the genomic sequence was peformed according to the bottom-down approach, as generally performed in the art. In brief, the outline is as follows: first, isolated DNA was fragmented to clone into a cloning vector such as pUC. Next, cloned fragments were sequenced by shot-gun sequencing. These sequencing reactions were performed at about 15,000 per 1Mbp. The sequences determined for each reaction, were assembled for clarification in a group of sequences called "contig". Thereafter, gaps between the contigs (physical and sequence gaps) were cloned, and the gaps were sequenced to fill the gaps. Thereafter, the analysis of base sequence data was performed to identify open reading frame for performing annotation. The details are as follows:

First, genomic libraries were constructed. As used herein, in order to prevent bias derived from genetic sequences, physical digestions rather than partial digestion using restriction enzymes were performed. In this case, libraries of a plurality of lengths were constructed. Plasmid libaries containing 2-3 kbp fragments, and lambda phage libraries containing about 20 kbp were constructed.

Second, shot gun sequencing of plasmid libraries was performed. A sequencer commercially available from Applied Biosystems was used for sequencing. As used herein, such sequencing was performed so that 400-500 bp base sequences may be obtained for about 150,000 /1Mbp. Similarly, terminal shot gun sequencing of the lambda phage library was performed. As such, theoretically, it was calculated the entire full-length genome was sequenced six times or more.

Third, base sequence data (about 40,000 pieces of data for about 2Mbp genome) was assembled to fill in the gaps. In this instance, terminal sequence data from the lambda phage library consisting of long fragments was determined for relative positions and the direction of each region. What is obtained by this proceedure is usually called a "contig". In the present Example, a number of contigs were obtained. Sequence undetermined regions (gaps) therebetween were filled. When fragments were identified to fill the gap between contigs, such gaps are called sequence gaps, and gaps in which such fragments were not cloned, are called physical gaps. Filling such physical gaps was performed by engineering techniques, such as amplification of LA-PCR and the like, and base sequence determination and the like. As such, substantially all the sequencing data fell within one contig, and the sequencing was thus completed.

Fourth, the sequence data was analyzed. Open reading frames (ORF) were identified and the annotation thereof was performed. In this task, programs such as Hidden Markov model (HMM) and Interpolated Markov model (GLIMMER) and the like were used for identification of ORFs. Thereafter, the search functions of BLAST, BLASTX and FASTA and the like were used to identify the function of each ORF. Thereafter, genetic and biochemical analyses were performed (see, for example, Fraser C.M., Res Microbiol., 151, 79-84 (2000); Fraser C.M.et al., Nature, 406, 799-803 (2000); Nelson et al.,Nat Biotechnol., 18, 1049-1054 (2000); Kawarabayasi Y. et al., DNA Res. , 6, 83-101, 145-222 (1999) and the like).

The nucleic acid sequences determined as above are sequences set forth in SEQ ID NO: 1 (SEQ ID NOs: 1, 342, and 723 are plus (sense) strand, and SEQ ID NOs: 1087, 1469 and 1838 are minus (antisense) strand).

(Functional analysis of each gene)

Next, the amino acid sequence of each gene was compared to those known in the art, as registered in databases such as EMBL, PDB and the like, by using software such as DNASIS, BLAST, and CLUSTAL W. As a result, a variety of polypeptides having high homology with said amino acid sequences were identified, and the function of each gene inferred therefrom (see Table 2).

(EXAMPLE 2: targeting)

(double cross-over disruption)

(Bacterial strains and growth conditions)

*T. kodakaraensis* KOD1 and derivatives thereof were cultured under stringent anaerobic conditions at 85 °C in rich growth medium (ASW-YT) and amino acid-containing synthetic medium (ASW-AA). ASW-YT medium contains 5.0 g/L yeast extract, 5.0 g/L trypton and 0.2 g/L sulfur (pH 6.6) in a diluted artificial sea water to 1.25 fold (ASW x 0.8). The composition of ASW is as follows: NaCl 20g; $MgCl_2 \cdot 6H_2O$ 3g; $MgSO_4 \cdot 7H_2O$ 6g; $(NH_4)_2SO_4$ 1g; $NaHCO_3$ 0.2g; $CaCl_2 \cdot 2H_2O$ 0.3g; KCl 0.5g;NaBr 0.05g; $SrCl_2 \cdot 6H_2O$ 0.02g; and $Fe(NH_4)$ citrate 0.01g. ASW-AA medium is 0.8 x ASW supplemented with 5.0 ml/L modified Wolfe minor mineral (containing in 1L, 0.5g $MnSO_4 \cdot 2H_2O$; 0.1g $CoCl_2$; 0.1g $ZnSO_4$; 0.01g $CuSO_4 \cdot 5H_2O$;0.01g $AlK(SO_4)_2$;0.01g $H_3BO_3$; and 0.01g $NaMoO_4 \cdot 2H20$), 5.0ml/L vitamin mixture (see the following literature), twenty amino acids (containing 250mg cystein·HCl; 75mg alanine; 125mg arginine · HCl;100mg asparagine · $H_2O$; 50mg aspartic acid;50mg glutamine, 200mg glutamic acid;200mg glycine; 100mg histidine · HCl · $H_2O$; 100mg isoleucine;100mg leucine;100mg lysine · HCl;75mg methionine;75mg phenylalanine; 125mg proline; 75mg serine; 100mg threonine; 75mg tryptophane; 100mg tyrosine; and 50mg valine in 1L) and 0.2g/L sulfur element (pH is adjusted to 6.9 with NaOH) (Robb,F.T.,and A.R.Place.1995.Media for Thermophiles, p.167-168. In F.T.Robb and A.R.Place (ed.) Archea: a laboratory manual-Thermophiles.Cold Spring Harbor Press,Cold Spring Harbor,N.Y.). Optionally, 5-FOA (Wako Pure Chemical, Osaka, Japan) and uracil (Kojin, Tokyo, Japan) were added to ASW-AA medium at the concentrations described in Robb. In order to examine tryptophan nutrient requirement, tryptophan-free ASW-AA, ASW-AAW- were used. In order to reduce dissolved oxygen in the medium, 5.0%$Na_2S \cdot 9H_2O$ was added until the color of sodium resazurin salt (1.0mg/L) disappeared. In the case of plate culture, 1.0%(w/v) Gelrite (Wako Pure Chemical) was added, and in lieu of the sulphur element 5.0% $Na_2S \cdot 9H_2O$ solution, 2.0ml/L polysulfide solution (10g$Na_2S \cdot 9H_2O$ and 3.0g sulphur element /15ml) weas used for solidification. The cells were incubated in anaerobic chamber (Tabai Espec, Osaka, Japan), at 85 °C.

DH5-alpha, an E. coli used for general DNA engineering, was routinely cultured on LB medium (Sambrook, J., and D. Russel. 2001. Molecular cloning: a laboratory manual, 3rd edn.Cold Spring Harbor Press, Cold Spring Harbor, N.Y.) which was supplemented with 50μg/ml ????? as necessary.

(Mutation by UV radiation and Isolation of 5-FOA resistant variants)

*T. kodakaraensis* KOD1 was cultured in 2.0 L of ASW-AA liquid medium for 39 hours. Cells within the stationary phase were recovered by centrifugation (6,000 x g, 30 minutes). The following procedures were performed anaerobically in an anaerobic chamber as follows: cells were resuspended in 60 mL of ASW, and a portion of the suspension (10 mL) was placed into a petri dish. The suspension was UV radiated for an appropriate time (0, 30, 60, 90 and 120 seconds) at a distance of 20 cm from 15W sterilization lamp, with agitation. Aliquots (200 μl) were plated on ASW-AA plate medium containing 0.75 % 5-FOA, and uracil nutrition requirement (Pyr-) variants were dominantly screened. In order to support growth of the resultant variants, 10μg/ml uracil was included in the growth media. The cells were incubated at 85 °C for five days. The number of viable cells was deterimined by inoculation onto a ASW-AA plate medium free of 5-FOA at an appropriate dilution ratio, and counting the number of colonies formed.

5-FOA colonies were separated, and cultured in ASW-YT liquid medium. The cells were incubated in ASW-AA liquid medium for two days in order to avoid carry over of uracil, and passaged into ASW-AA liquid medium with or without 5 pg/ml uracil to study the nutritional requirement of the isolates for uracil of isolates.

(Enzymatic Assay)

Cell-free extracts of *T. kodakaraensis* KOD1 and variants thereof were prepared as follows: cells were cultured in ASW-Y liquid medium for twenty hours, and collected by centrifugaion (6,000 x g, 30 minutes), and the cells were resuspended in 50 mM Tris-HC1 (pH 7.5) containing 0.1 % v/v Triton X-100. The samples were vortexed for ten minutes, centrifuged at 3,000 x g for twenty minutes, and the resultant supernatant retained as cell-free extract. Protein concentration was determined using the Bio-Rad Protein Assay System (Bio-Rad, Hercules, CA, USA) using bovine serum albumin as a standard.

Orotidine-5'-monophosphate decarboxylase ( OMPdecase, PyrF) activity was determined by monitoring the reduction in optical density at 285 nm ($OD_{\lambda 285nm}$), derived from the conversion of orotidine-5'-monophosphate (OMP) into uridine-5'-monophosphate (UMP) (Beckwith, J. R., A. B. Pardee, R. Austrian, and F. Jacob. 1962. Coordination of the synthesis of the enzymes in the pyrimidine pathway of E. coli. J. Mol. Biol. 5: 618-634.) . The assay mixture consists of 100 mM Tris-HCl (pH 8.6), 1.5 mM $MgCl_2$, 0.125 mM OMP and enzyme solution in 1ml in total. This mixture was preincubated at 85 °C for 5 minutes in a capped cuvette, and the reaction was initiated by adding an enzyme solution and monitored for 10 minutes at the same temperature.

Orotinate phoshoribosyltransfrase (OPRTase, PyrE) activity was assayed by spectrometrically measuring orotinic acid at 295 nm. When measuring enzyme sample from pyrE$^+$ strain, continuous decarboxylation by intrinsic OMP decase of the reactant product OMP should be taken into account. As OMP decase activity is higher than OPRTase in *T. kodakadaensis,* OPRTase activity may be determined at $\Box\Box_{295}$ of 3,670 $M^{-1}cm^{-1}$. This does not correspond to the conversion from orotinic acid to UMP via OMP. In the case of the pyrF$^-$ strain, we monitored the conversion of the vstarting substrate to OMP by means of $\Box\Box_{295}$ of 2,520 $M^{-1}cm^{-1}$. This reaction was performed in 1 ml mixture comprising Tris-HCl (pH 8.6), 1.5 mM $MgCl_2$, 0.125 mM orotinic acid, cell-free extract, and 1.6 mM 5-phosphoribosylpyrophosphate (PRPP). The same assay mixture free of PRPP was placed in a capped cuvette, and preincubated at 85 °C for 10 minutes, and the reaction was initiated by the addition of PRPP. The decrease in $A_{295}$ was measured at the same temperature for three minutes.

(DNA engineering and sequencing)

General DNA engineering was performed as described in Sambrook and Russel (Sambrook, J., and D.Russel. 2001. Molecular cloning: a laboratory manual, 3rd edn. Cold Spring Harbor Press, Cold Spring Harbor, N.Y.). The genomic DNA of *T. kodakaraensis* was isolated as described above. PCR was performed using KOD -Plus- (TOYOBO, OSAKA , JAPAN) as the DNA polymerase. The sequence of the primers used for PCR are shown below. Optionally, DNA fragments amplified by PCR were phosphorylated by T4 kinase (TOYOBO). Restriction enzymes and modification enzymes were purchased from TaKaRa (Kyoto, Japan) or Toyobo. DNA fragments were collected after agarose gel electrophoresis, and GFX PCR DNA and a Gel Band Purification Kit (Amersham Pharmacia Biotech, Uppsala, Sweden) were used for purification thereof. Plasmid DNA was isolated using Qiagen Plasmid Kits (Qiagen, Hilden, Germany). DNA sequencing was performed using ABI PRISM kit and a Model 3100 capillary sequencer (Applied Biosystems, Foster City, CA, USA).

(Construction of pUDT and pUDT2)

Two disruption vectors pUDT1 (SEQ ID NO: 2158) and pUDT2 (SEQ ID NO 2159) were constructed for respective homologous recombination of single and double cross-over events in *T. kodakaraensis.* They were constructed as follows: a DNA fragment (676bp) containing Tk-pyrF was amplified from *T. kodakaraensis* KOD1 genomic DNA using the following primers TK1-DUR/TK1-DUF:

TK1-DUR/TK1-DUF: 5'-GGG<u>CATATG</u>GAGGAGAGCAGGCTCATTCTGGCG-3' (SEQ ID NO; 2160) / 5' -CTGAGGGGGTGTTTGACTTTCAA-3' (SEQ ID NO: 2161), wherein underlined sequences indicate NdeI sites.

Deduced promoter region (130 bp) was amplified from primers TK2-DPR/TK2-DPF:

TK2-DPR/TK2-DPF: 5'-GGG<u>CTGCAG</u>CCGCAACGCGCATTTTGCTCACCCGAA AA-3' (SEQ ID NO: 2162) /5'-GGG<u>CATATG</u>CATCACCTTTTTAACGGCCCTCTCCAAGAG-3'(SEQID NO: 2163), wherein underlined sequences indicates PstI and NdeI sites, respectively.

Both fragments were subcloned into pUC118 in an appropriate promoter pyrF direction. The resultant plasmid was designated as pUD (3,944). A short fragment (788 bp) of *Tk-trpE* was amplified using the following primers TK3-DTR/TK3-DTF:

TK3-DTR/TK3-DTF : 5'-GGGGCATGCGGTGGCTT CGTTGGCTACGTCTCCTACG-3' ( SEQ ID NO: 2164 ) /5'-GGGCTGCAGTTCGGGGCTCCGGTTAGTGTTCCCGCCG-3' (SEQ ID NO: 2165), wherein underlined sequences indicate SphI and PstI sites. Next, this was ligated with pUD at SphtI and PstI sites to yield pUDT1 (4732 bp).

In order to construct pUDT2, fragments containing *Tk-trpE* and flanking regions (2223 bp) were amplified using the following primers TK4-DT2R/TK4-DT2F:

TK4-DT2R/TK4-DT2F : 5'-GGGGTCGACCGGG TCTGGCGAGGGCAATGAGGGAC-3' (SEQ ID NO: 2166 ) /5'-GGGGAATTCGGTTATAGTGTTCGGAACGACCTTCACTC-3' (SEQ ID NO: 21267), wherein underlined sequences indicate SalI and EcoRI sites, respectively)

This was subcloned into SalI and EcoRI sites of pUC119. The resultant plasmid was designated pUT4 (5,340 bp). pUD was digested with PvuII, and the fragment containing pyrF and the deduced promoter region (1104 bp) was isolated. pUDT2 (6,012 bp) was obtained by inserting the isolated fragment in pUT4, into the blunt ended SacI sites of Tk-trpE.

Linear DNA fragments for homologous recombination in *T. kodakaraensis* were prepared by PCR using pUDT2 as a template, and purified after agarose gel electrophoresis.

(Transformation of *T.kodakaraensis)*

The calcium chloride method for *Methanococcus voltae* PS (Bertani, G., and L.Baresi.1987.Genetic transformation in the methanogen Methanococcus voltae PS. J. Bacteriol.169: 2730-2738.) was modified for transformation of *T. kodakaraensis. T.kodakaraensis* KU25 was cultured for twelve hours in ASW-YT liquid medium, and cells were collected from 3 ml broth during later log phase (17,000 $\times$ g, 5 minutes), and resuspended in 200 $\mu$l transformatinon buffer (in order to avoid precipitation phenomensa between calcium cations and phosphate groups, in 80 mM $CaCl_2$ in 0.8 modified ASW free of $KH_2PO_4$) (1/15 vol.). This was maintained on ice for 30 minutes. Next, 3 $\mu$g DNA was dissolved in TE buffer, and added to the suspension. Further, the cells were incubated on ice for one hour, followed by heat shock at 85 °C for 45 seconds, and further incubated on ice for 10 minutes. As control experiments, an equal volume of TE buffer was added to the cell in lieu of DNA. Processed cells were screened for Pyr+ transformant by passaging two generations in the absence of uracil in 20 ml of ASW-AA liquid medium. Next, the cells were diffused on an ASW-AA plate, free of uracil, and incubated for 5-8 days at 85 °C. Resultant Pyr+ strain was analyzed by Southern hybridization using colony PCR and DIG-DNA labeling and detection kit (Boehringer Mannheim, Mannheim, Germany).

(EXPERIMENTAL PROCEDURES)

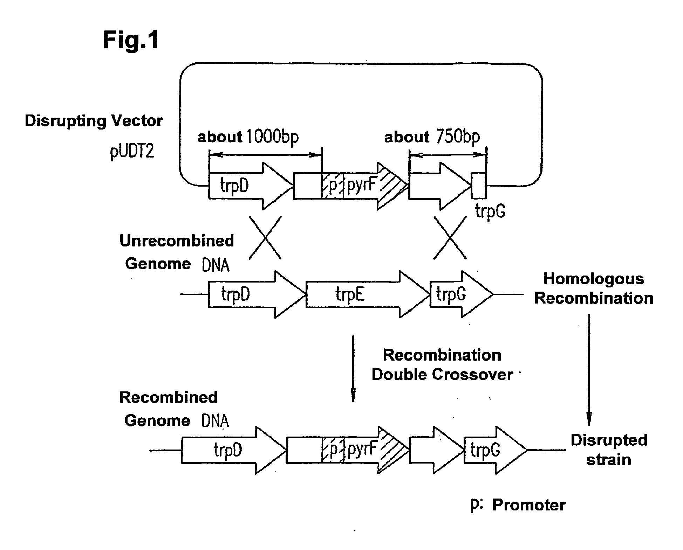

Double targeting disruption was performed using circular DNA molecules for double cross-over gene disruption. The exemplary scheme is shown in Figure 1.

(Preparation of a disruption vector)

(Preparation of KOD-1)

The KOD-1 strain was prepared as described above.

(Transformation and homologous recombination)

As described above, transformed KOD-1 strain was maintained in ASW-AA. In this instance, KOD-1 strain growth is sustained by carried-over uracil.

Next, the KOD-1 strain was inoculated into fresh amino acid liquid medium. PyrF+ is the only strain in which homologous recombination occurred, and therefore grows in fresh amino acid liquid medium, this allowed screening and isolation of strians in which homologous recombination had occurred.

Next, isolated strains were inoculated into ASW-AA. Colonies grown on solid medium were confirmed with colony PCR and Southern blotting analysis. The procedure therefor is described as follows:

Reaction mixture: 2.5 unit KOD polymerase (TOYOBO) 0.5 $\mu$l; 10 x KOD polymerase buffer (TOYOBO) 5.0$\mu$l;25mM $MgCl_2$ 4.0$\mu$l; dNTP mixture 4.0$\mu$l; 20pmol/$\mu$l primer 1 0.5$\mu$l; 20pmol/$\mu$l primer 2 0.5$\mu$l; sterilized water 37.0$\mu$l; cell suspension 0.5$\mu$l.

This reaction mixture was incubated under the following reaction conditions: 96 °C, 2 minutes, 96 °C, 30 seconds, 55 °C, 3 seconds, 72 °C, 30 seconds, 30 cycles; 72 °C 3 minutes.

Colony PCR and Southern blotting analyses were performed to yield the following results:

TABLE 3: Double cross-over gene targeted disruption

| | Control | Transformant1 | Transformant2 |
|---|---|---|---|
| CaCl$_2$ | + | + | + |
| DNA | TE buffer | pUDT2 | pUDT2 |
| Growth in amino acid liquid medium in the presence of carried-over uracil | No growth | Growth | Growth |
| T/C | not available | 12/12 | 5/12 |
| Total T/C | not available | 17/24 | |

T/C refers to the number of clones which were screened by transformant/colony PCR of interest (i.e., PyrF[+] strain).

As shown in the above results, it was demonstrated that targeted double cross-over disruption of genes using circular molecules proceeds at a very high ratio.

(EXAMPLE 3: Examples of double cross-over disruption; cases where linear DNA was used)

Next, examples of double cross-over using linear DNA molecules were shown.

(Production of the disruption vector)

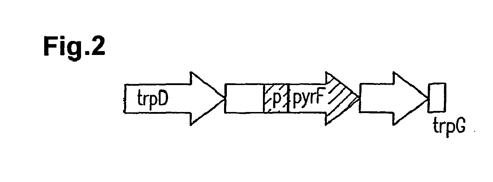

Linear DNA was prepared as shown in Figure 2 as a linear disruption vector. Linear DNA was obtained by amplification using pUDT2 prepared in Example 2 as a template using appropriate primers.

(Preparation of KOD1)

The KOD-1 strain was prepared as described in Example 2.

(Transformation and homologous recombination)

Prepared KOD-1 strain was transformed using the calcium chloride method. The transformed KOD-1 strain was maintained in ASW-AA. In this instance, KOD-1 strain growth is sustained by carried-over uracil.

Next, the KOD-1 strain was inoculated into fresh amino acid liquid medium. PyrF+ strain is the only strain in which homologous recombination occurrs, and therefore grows in fresh amino acid liquid medium, allowing screening and isolation of strains in which homologous recombination has occurred.

Next, isolated strains were inoculated into ASW-AA. Then colonies grown on the solid medium were confirmed by colony PCR and Southern blotting analysis. The procedure therefor is described as follows:

Colony PCR and Southern blotting were performed as described above.
As analyzed above, the following results were obtained.

Table 4: Gene targeted disruption by double cross-over

| | Control | Transformant3 | Transformant4 |
|---|---|---|---|
| CaCl$_2$ | + | + | + |
| DNA | TE buffer | Linear DNA | Linear DNA |
| Growth in | No growth | Growth | Growth |

(continued)

|  | Control | Transformant3 | Transformant4 |
|---|---|---|---|
| amino acid liquid medium in the presence of carried-over uracil |  |  |  |
| T/C | not available | 7/12 | 0/12 |
| Total T/C | not available | 7/24 |  |

T/C refers to the number of clones which were screened by transformant/colony PCR of interest (i.e., PyrF[+] strain).

As shown in the above results, it was demonstrated that targeted double cross-over disruption of genes using linear molecules proceeds at a sufficiently high ratio, although lower than those using circular molecules. It is thought that the reason for lower ratios than that observed using circular molecules include digestion of linear molecules by host nucleases.

Further, in light of the above-mentioned results, when determining a preferable length for linear DNA, if there are at least 500 bases at both termini, targeted disruption progresses at about 5 % or more, and if there are at least respective 1000 bases at both termini, targeted disruption progresses at about 20 % or more. Accordingly, it is understood that targeted disruption using a linear molecule requires at least 500 bases, and preferably at least 1,000 bases of nucleic acid sequences at both termini.

(EXAMPLE 4: Examples of double cross-over disruption: other genes)

A gene other than the above-mentioned genes (for example, a sequence encoding SEQ ID NO: 395 (Tryptophane synthase)) is selected to perform similar experiments based on tryptophane nutritional requirement, and similar targeted disruption was performed.

(EXAMPLE 5: Single cross-over disruption)

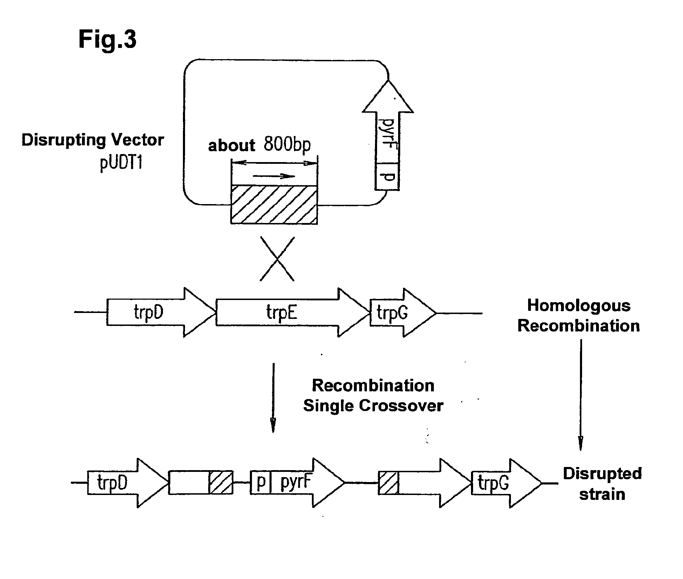

Gene targeted disruption was performed using a circular molecule using a single cross-over dirsuption system. Schematic drawing is shown in Figure 3. pUDT (SEQ ID NO: 2158) was prepared as described above.

(Preparation of KOD1)

The KOD-1 strain was prepared as described in Example 2.

(Transformation and homologous recombination)

Prepared KOD-1 strain was transformed with the calcium chloride method. The Transformed KOD-1 strain was maintained in ASW-AA. In this instance, the KOD-1 strain grows with carried-over uracil.

Next, the KOD-1 strain was inoculated to a fresh amino acid liquid medium. As PyrF+ strain, in which homologous recombination occurred, only grows in fresh amino acid liquid medium, this allows screening and concentration for those in which homologous recombination has occurred.

Next, grown strains were inoculated into ASW-AA. Then colonies grown in the solid medium were confirmed with colony PCR and Southern blotting analysis. The procedure therefor is described as follows:

Colony PCR and Southern blotting were performed as described above.

As analyzed above, the following results were obtained.

TABLE 5: Gene targeted disruption by single cross-over

|  | Control | Transformant5 | Transformant6 |
|---|---|---|---|
| CaCl$_2$ | + | + | + |
| DNA | T E buffer | p U D T 1 | p U D T 1 |
| Growth in amino acid liquid medium in the presence of carried-over uracil | No growth | Growth | Growth |

(continued)

|  | Control | Transformant5 | Transformant6 |
|---|---|---|---|
| T/C | not available | 1/96 | 2/96 |
| total T/C | not available | 3/192 | |

T/C refers to the number of clones which were reviewed by transformant/colony PCR of interest (i.e. , PyrF[+] strain).

As described above, it is understood that gene targeted disruption by single cross-over using a circular molecule progresses at a much lower rate than the gene targeted disruption by double cross-over. A reason why efficiency by single cross-over is lower than that by double cross-over is believed to be the digestion of pUDT1 by restriction enzymes from the host.

As such, the present invention is demonstrated to work in a system using single disruption. Further, when using a linear molecule, the system using single disruption works, although at much lower rate.

(EXAMPLE 6: Examples of single cross-over disruption; other genes)

Genes were disrupted by single cross-over as in Example 4, and it was demonstrated that disruption was permissible, although efficiency thereof was not as good as in Example 5.

(Example 7: expression of DNA ligase gene)

In order to express an ATP dependent DNA ligase in Escherichia coli, the following protocols were used. Fragments of the phage clone comprising the sequence of DNA ligase identified in the present invention (for example, SEQ ID NO: 1131) was used as a template to yield fragments of two types of DNA ligase coding regions, which were inserted into pUC18. The sequences of the inserted fragments were confirmed and the fragments comprising the DNA ligase from the plasmid was inserted into the plasmid pET21a (Novagen) to construct the plasmids. The expression and the activity were confirmed as follows:

Escherichia coli BL21 (DE3) was transformed with the plasmid. The resultant ampicillin resistant transformants were inoculated on to the NZCYM medium (1 % NZ amine, 0.5 % NaCl, 0.5 % yeast extract, 0.1 % casamino acid, 0.2 % MgSO4 ·7H$_2$O(pH7)), cultured at 37 °C until the OD$_{660}$ reached 0.4. Isopropyl-β-D-thiogalactopyranoside (IPTG, 0.1mM) was then added thereto to continue the culture at 37 °C. After culture, cells were collected by centrifugation, broken by sonication, and centrifuged to yield a cell extract, which was disrupted by sonication, and this was again centrifuged to recover soluble fractions. The resultant fraction was processed at 70 °C for ten minutes and the thermostable soluble fraction was centrifuged again to yield a sample. This sample may be further purified using a variety of well known purification methods and a combination thereof.

Enzymatic activities are measured by a method for observing a change of mobility of DNA fragments after the obtained samples were digested with lambda phage DNA Hind III, and the resultant was agarose gel electrophoresed; or a method for reacting the obtained sample to an oligo dT labeled with $^{32}$P and removing unreacted $^{32}$P with alkaline phosphatase, and then measuring radioactivity thereof (see Rossi,R et al,(1997)Nucleic Acids Research,25(11): 2106-2113;Odell,M.et al.,(1996)Virology 221:120-129;Sriskanda,V.et al,(1998)Nucleic Acids Research,26(20): 4618-4625;Takahashi,M. et al.,(1984)The Journal of Biological Chemistry,259(16):10041-10047)).

(Examples 8: expression and confirmation of formic acid dehydrogenase)

Formic acid dehydrogenase is an enzyme catalyzing a reaction oxidizing formic ion into $CO_2$. The reaction thereof is represented by the formula: HCOO-+NAD+⇔$CO_2$+NADH. As used herein, NAD (nicotine amide adenine dinucleotide; reductive type is NADH) is one of the coenzymes relating to the redox reaction.

Formic acid dehydrogenase activity is measured using, for example, NADP+ (340 nm, $\varepsilon$=6.22×10$^3$), methyl viologen (600 nm, $\varepsilon$=1.13×10$^4$), or benzyl viologen (605 nm, $\varepsilon$ =1.47×10$^4$) (Andreesen,J.R.et al., (1974) J. Bacteriol., 120:6-14).

Known formic dehydrogenases include a homodimer consisting only of alpha subunits, a heterodimer and heterotetramer consisting of alpha and beta subunits, and a dodecamer consisting of alpha, beta and gamma subunits.

Formic acid dehydrogenases of the present invention may consist of single or plural subunits. Preferably, the formic acid dehydrogenases consist of two or more subunits.

(Expression of thermostable formic acid dehydrogenase)

In order to express the formic acid dehydrogenases (SEQ ID NO: 305, 673, 1050 and 1051) encoded by an open reading frames obtained by the present invention, in Escherichia coli, the following operations were performed: fragments containing the open reading frames were amplified by PCR technology and inserted in plasmid pET21a(+) (Novagen) to yield an expression plasmid. These plasmids were used to transform Escherichia coli BL1 (DE3) strain.

The resultant ampicillin resistant transformants were inoculated on to the NZCYM medium (1 % NZ amine, 0.5 % NaCl, 0.5 % yeast extract, 0.1 % casamino acid, 0.2 % MgSO4· 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solutions.

The crude enzyme solution was measured for its formic acid dehydrogenase enzymatic activity according to routine method (Andreesen, J.R. et al., (1974) J.Bacterio1.,120: 6-14). Further, the enzyme has an optimum temperature at 90 °C.

(EXAMPLE 9: hyperthermostable beta-glycosidase)

Beta-glycosidases collectively refer to a group of enzymes hydrolyzing a beta-glycoside bond. Beta glycosidases include, for example, beta-glucosidase, beta-galactosidase, beta-mannosidase, beta-fructosidase and the like.

Beta-galactosidase, a type of beta-glycosidase, is an enzyme hydrolyzing beta-D-galactoside to yield D-galactose. Degrading lactose (glucose-beta-D-galactoside) into glucose and galactose using a galactosidase is a method for producing low-lactose milk by processing the lactose in cow milk. For these purposes, in addition to adding the enzyme into milk, the use of a fixed enzyme is also considered. Generally, enzymes used as a fixation enzyme present preferably high activity at the reaction condition used (pH, temperature and the like), and is structurally stable.

As used herein, beta-galactosidase is an enzyme hydrolyzing beta-D-galactoside to produce D-galactose, and is systematically called beta-D-galactoside galactohydrolase. Beta-glycosidase of the present invention may have beta-glucosidase, beta-mannosidase and/or beta-xylosidase activities in addition to beta-galactosidase activity. Beta-glycosidase of the present invention may have transferring activity in addition to hydrolyzing activity of oligosaccharides.

(Expression of beta-glycosidase)

Beta-glycosidase (SEQ ID NO: 1122) was expressed using the same method as described above in the Examples. The resultant ampicillin resistant transformants were inoculated on to the NZCYM medium (1 % NZ amine, 0.5 % NaCl, 0.5 % yeast extract, 0.1 % casamino acid, 0.2 % MgSO4· 7H$_2$O(pH7)) containing amplicillin (50μg/ml), cultured at 37 °C until the OD$_{660}$ reached 0.5. Isopropyl-β-D-thiogalactopyranoside (IPTG, 0.1mM) was then added thereto and the culture was continued at 37 °C. After culture, cells were collected by centrifugation, broken by sonication in 100 mM vicine/KOH (pH8.3)/10mM MgCl$_2$, and centrifuged again to yield a soluble fraction, which was then heated at 85 °C for thirty minutes. Heat-stable soluble fractions were centrifuged and concentrated, and then were subjected to sodium dodecyl sulfate polyacrylamide electrophoresis (SDS-PAGE) to detect a expected band of molecular weight, and the band was seen to increase over time after the induction by IPTG.

The sample was heat treated as above and used for determining the enzymatic chemical properties of beta-glycosidase of the present invention. As for methods of measuring enzymatic activities, see Pisani, F.M. et al., Eur.J.Biochem., 187, 321-328 (1990). Enzymatic acitivity of liberalizing 1 μmol p-nitrophenol per minute was considered 1U.

The optimum pH of beta-glycosidase of the present invention was examined. The reaction was performed in a variety of buffers, including 1.5μg/ml of the enzyme with 2.8 mM pNp beta-glucopyranoside as the substrate at 75 °C. The buffers used were sodium phosphate buffer (pH 6-8), citrate buffer (pH 4-6), borate buffer (pH 8-9), glycine buffer (pH 8.5-10) (data not shown). These results show that the beta-glycosidase has its optimum pH at around pH 6.5.

Optimum temperature for beta-glycosidase of the present invention was also examined. Reactions were performed in sodium phosphate buffer (pH 6.5) including 1.5μg/ml of the enzyme with 2. 8 mM pNp beta-glucopyranoside as the substrate at a variety of temperatures (data not shown). As a result, the beta-glycoidase of the present invention has its optimum temperature at around 100 °C. Further, Arrhenius plotting was performed using this result, and it was demonstrated that the gradient of the line is changed around 75 °C (1/T*10-3=2.87). The results were applied to the formula k=Ae-E/RT (wherein k is reaction rate constant, E is activation energy, R is gas constant, T is absolute temprature, A is frequency factor), it was calculated that E=53.4 kJ/mol in the range of 25-75 °C, and E=17.7 kJ/mol in the range of 75-100 °C.

Thermostability of beta-glycosidase of the present invention was examined. After the above samples were incubated for a variety of times at 90 or 100 °C, enzymatic activity was measured at 80 °C in 50 mM sodium phosphate buffer (pH 6.5), including 1.5 μg/ml of the enzyme and using 2.8 mM pNp-beta-glucopyranoside as a substrate (data not shown). This result indicates that the beta-glycosidase has about 18 hours and 1 hour of thermostability at 90 °C and 100 °C, respectively. Similar experiments were performed at 110 °C, the enzyme was inactivated after about 15 minutes.

Substrate specificity of beta-glycosidase of the present invention was examined. Activities against a variety of substrates at 2.8 mM were measured at 80 °C in 50 mM sodium phosphate buffer (pH 6.5) containing 1.5 pg/ml of enzyme, and it was demonstrated that the beta-glycosidase of the present invention has high beta-glycosidase activity, and further, has beta-mannosidase, beta-glycosidase and beta-xylosidase activities.

Reaction rate constants for these four enzymes were determined by measuring the activity against substrates by incubating each 2 mM of oligosaccharide (beta-lactose, cellobiose, cellotriose, cellotetraose and cellopentaose) with 3.0μg/ml enzyme at the concentration of 0.28 mM to 5.6 mM, in 50 mM sodium phosphate buffer (pH 6.5) containing 1.5 μg/ml at 80 °C for seven hours. Next, the reactant solution was subjected to thin layer chromatography (TLC) (data not shown). Spots of glucoses were observed in lanes other than the beta-lactose lane. Cellotetraose, a tetrasaccharide, was divided into trisaccharide and monosaccharide, and cellopentaose, a pentasaccharide, was divided into tetrasaccharide and monosaccharide, respectively. These results show that the beta-glycosidase of the present invention has an exo-type of hydrolyzing activity.

5 mM solutions of cellobiose, cellotriose, cellotetraose and cellopentaose in 50 mM sodium phosphate buffer (pH 6.5) containing 3 pg/ml of enzyme were incubated at 80 °C for four hours. Cellotetraose was also incubated for 0, 1, 2, 4 and 7 hours in a similar reaction system. Next, the reaction solution was subjected to thin layer chromatography (TLC). Cellobiose, cellotriose, cellotetraose and cellopentaose are disaccharides, trisaccharides, tetrasaccharides and pentasaccharides, respectively, and larger spots than these saccharides were observed after reaction. This result demonstrates that the beta-glycosidase of the present invention has sugar-transferase activity in addition to an exo-type sugar-degrading activity In this reaction condition, glucose and cellobiose were increased over time, and this means that hydrolyzing activity, rather than transferring activity, is increased over time. That is, beta-glycosidase of the present invention can be applied to the synthesis of oligosaccharides having any combination of beta linkage such as oligosaccharide in which cellobiose is linked to mannose, and the like.

(EXAMPLE 10: hyperthermophillic chitinase)

Chitin is a type of mucopolysaccharides, and has a structure of beta-poly-N-acetylglucosamine. Chitinase is an enzyme present as a cell-wall substance of arthropods, molluscs, crustaceans, insects, fungi, bacteria and the like, in an abundant amount, which hydrolyzes a chitin, and is found in the gastric juice of snails, exuvial fluid of an insect, fruit skin, microorganisms and the like. This enzyme produces N-acetylglucosamine by hydrolysis of beta-1,4 linkage of a chitin, and has a systematic name of poly(1,4-beta-(2-acetamide-2-deoxy-D-glucoside)) glucanohydrolase.

Chitinase may be industrially useful for the purpose of decomposing chitin, which is present in an abundant amount in nature, into forms more available to microorganisms and the like. Further, chitinase is also believed to play an important role as a protection mechanism against pathogens in plants, and thus attempts have been made to develop a disease-desistant plant by introducing a gene encoding the subject enzyme.

(Expression of hyperthermophillic chitinase)

As described in the above-mentioned Examples, hyperthermophillic chitinase (SEQ ID NO: 991) was expressed. The resultant ampicillin resistant transformants were inoculated on to the NZCYM medium (1 % NZ amine, 0.5 % NaCl, 0.5 % yeast extract, 0.1 % casamino acid, 0.2 % MgSO4· 7H$_2$O(pH7)), cultured at 37 °C until the OD$_{660}$ reached 0.3. Isopropyl-β-D-thiogalactopyranoside (IPTG, 0.1mM) was then added thereto and the culture was continued at 37 °C. After culture, cells were collected by centrifugation, broken by sonication, and centrifuged to yield a cell extract. The resultant cell extract was heated at 70 °C for ten minutes, and then the obtained thermophillic fraction was centrifuged to yield the supernatant thereof as a sample, which was subjected to sodium dodecyl sulfate polyacrylamide gel electrophoresis (SDS-PAGE), and the expected band was detected at about 130 kDa.

The sample was heat-processsed as above and purified using ammonium sulfate precipitation (40% saturation), anionic exchange column (HiTrapQ), gel filtration column, and anionic exchange column (MonoQ) so that only single band is observed on an SDS-PAGE.

The enzymatic activities were measured in accordance with a method "Chitin, Chitosan Experimental Manual" (Chitin Chitosan Research Ed., Gihodo Publishing) using colloidal chitin. The amount of enzyme required to produce a reduced saccharide corresponding to 1 μmol N-acetylglucosamine per minute was defined as 1 U.

Colloidal chitin as a substrate was prepared as follows: 10 g Chitin (Wako Pure Chemical) was solubilized in 500 ml of 85 % phosphoric acid and agitated for 24 hours at -4 °C. The viscous liquid was added to a ten-fold volume of deionized water while agitating. The precipitate was obtained by centrifugation, and the resultant was repeatedly washed by deionized water until the pH thereof was 5.0 or higher. NaOH was adjusted to pH 7.0, and then washed with deionized water for one more time. This was solubilized in a small volume of water and autoclaved.

The optimum temperature of hyperthermostable chitinase of the present invention was determined by measuring the activities of the above-mentioned purified enzymes in 50 mM sodium phosphate (pH 7.0) for sixty minutes at a variety of temperatures. The reaction was terminated by cooling on ice (data not shown). The hyperthermostable chitinase of the present invention was shown to have an optimum temperature at about 80 °C.

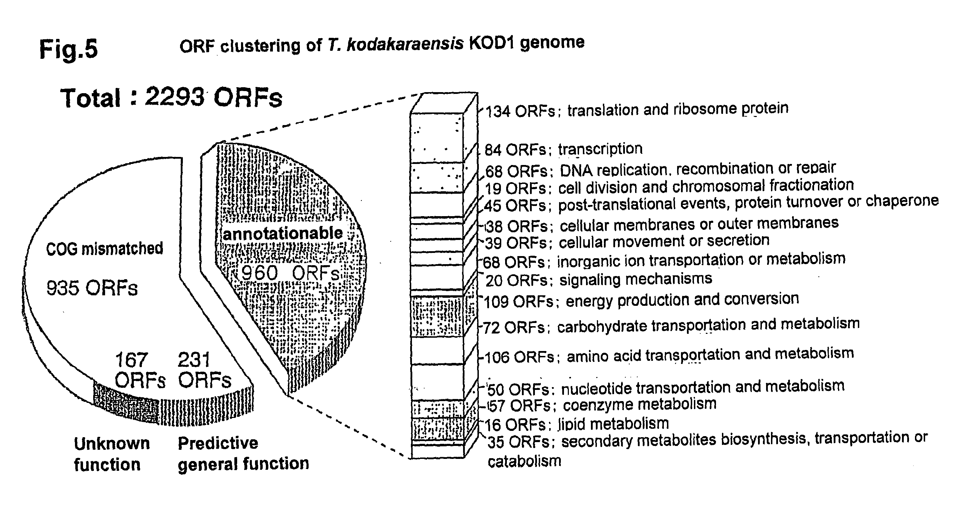

Optimum pH of the hyperthermostable chitinase of the present invention was determined by measuring the activities of the above-mentioned purified enzymes for sixty minutes at a variety of pH levels using the following buffers: 50 mM disodium hydrogen citrate-HCl (pH2.5~4.0); 50mM sodium acetate (pH4.0~5.5); 50mM MES-NaOH (pH5.5~7.0); 50mM Tris-HCl (pH7.0~9.0); 50mM glycine-NaOH (pH9.0~10.0). The reaction was terminated by cooling on ice. The result is shown in Figure 5. The hyperthermostable chitinase of the present invention was demonstrated to have an optimum pH at about 4.0. Further, peaks were observed at about pH 8.0.

The effects of salt on the activity of hyperthermostable chitinase of the present invention was studied by measuring the activities of the above-mentioned purified enzymes in 50 mM sodium phosphate (pH 7.0) with a variety of concentrations of salt (NaCl or KCl) added thereto for 120 minutes at 80 °C. The reaction was terminated by cooling on ice (data not shown). The activity of the hyperthermostable chitinase of the present invention was increased by the addition of the salt, and in particular, the addition of KC1 increased the activity by about two fold.

The hyperthermostable chitinase of the present invention was studied for the effects thereof on oligosaccharide and colloidal chitin. Oligosaccharides used were N - acetyl - D - glucosamine ( G 1), di-N-acetyl-chitobiose (G2), tri-N-acetyl-chitotriose (G3), tetra-N-acetyl-chitotetraose (G4), penta-N-acetyl-chitopentaose (G5) and hexa-N-acetyl-chito-hezaose (G6). Fifty $\mu$l of reaction mixture containing 0.7 mg of each oligosaccharide, 70 mM sodium acetate buffer (pH 6.0), 200 mM KC1, and purified enzyme (for G1-G3, 0.9 $\mu$g, and for G4-G6, 1.8 $\mu$g) was incubated at 80 °C and sampled at 0, 5, 15, 30, 60 or 120 minutes thereafter. As for colloidal chitin, 1 ml total reaction mixture containing 0.16 mg colloidal chitin, 50 mM sodium acetate buffer (pH 5.0), and 0.6 $\mu$g of purified enzyme was incubated at 80 °C, and sampled at 1.5, 3.0 and 4.5 hours thereafter, and centrifuged to concentrate 20 fold. Next, the samples were subjected to TLC as follows: sampled solution was spotted on Kieselgel 60 silica gel plate (Merck), and development solution (n-butanol: methanol:25% ammonia solution:water=5:4:2:1) was used for the development thereof. After development, the plates were dried, and developing reagents (anillin 4 ml, diphenylamine 4 g, acetone 200 mL, 85 % phosphoric acid 30 mL were mixed for preparation) was atomized and this was heated at 180 °C for about five minutes for coloring (data not shown).

From this result, it was demonstrated that the hyperthermophillic chitinase of the present invention has no degrading action against disaccharides or lower, and when chitin was used as a substrate, the enzyme mainly produced chitobiose, a disaccharide, as a main product.

The hyperthermostable chitinase of the present invention was also studied for effects on 4-methyl umbellipherone (4-MU). GlcNAc-4-MU, GlcNAc2-4-MU or GlcNAc3 -4-MU (0.01mM) 10 $\mu$l, 100mM acetate buffer (pH5.0) 990$\mu$l, and the purified enzyme 20$\mu$l (18ng) were incubated at 80 °C. At 0, 5, 15, 30, 45, 60, or 180 minutes, 100 $\mu$l of the reaction solution was sampled, and added to 900 $\mu$l of ice-cold 100 mM glycine-NaOH (pH 11) to terminate the reaction. The samples were measured for their excitation at 350 nm and fluorscence at 440 nm by spectrofluorometer (data not shown). As a result, reation rates against each substrate were determined.

It was reported that reaction rates against disaccharide derivatives and against trisaccharide derivatives were compared and thus the digestion type of the enzymes was either endo-type or exo-type (Robbins, P. W., J. Biol. Chem., 263 (1), 443-447 (1988)). In this case, when the reaction rate against disaccharide derivative is greater than that of the other, the enzyme is expected to be exo-type, whereas when the reaction rate against trisaccharide is greater than that of the other, the enzyme is expected to be endo-type. Based on this description, the hyperthermostable chitinase of the present invention is determined to be endo-type.

Functions possessed by each domain of the hyperthermostable chitinase of the present invention were studied by creating a variety of deletion mutants. Deletion mutants Pk-ChiAΔ1(containing the first Bacillus circulans chitinase homologous region and two cellulose binding domains), Pk-ChiA Δ 2 (containing the fourth Streptomyces erythraeus chitinase homologous region and two cellulose binding domains), Pk-ChiA Δ 3(containing the first Bacillus circulans chitinase homologous region), and Pk-ChiAΔ4 (containing the fourth Streptomyces erythraeus chitinase homologous region), were produced based on the previous reference (Japanese Laid-Open Publication 11-313688).

From the culture of E. coli transformant strains possessing each plasmid, crude enzyme solution was obtained by heat treating at 70 °C for 10 minutes. This crude enzyme solution was spotted on a colloidal chitin plate (0.5 % colloidal chitin, 1.5 % agar) and was incubated to study the activities thereof (data not shown). Deletion mutants having only the first chitinase homologous region showed some activity, and the deletion mutants having the fourth chitinase homologous region only showed little activity. All of the deletion mutants having any chitinase homologous regions and the two cellulose binding domains showed high activities.

Thirty $\mu$ 1 of the crude enzyme solution of deletion mutants Pk -Ch i A Δ 2 and P k -Ch i A Δ 4 was mixed with

30 $\mu$ 1 of 1 % collidal chitin, and incubated at 70 °C for one hour. Next, the reaction solution was centrifuged and the supernatant and a precipitate containing the colloidal chitin was obtained. The precipitate was washed twice with 50 mM sodium phosphate (pH 7.0), and was subjected to SDS-PAGE (data not shown). This result shows that the two cellulose binding domains are necessary for binding to a chitin and for chitinase activity.

(EXAMPLE 11: Hyperthermostable ribulose bisphosphate carboxylate)

Ribulose bisphosphate carboxylase is an enzyme catalyzing photosynthetic reactions and is present in plant chloroplasts and microorganisms having photosynthetic ability. Ribulose bisphosphate carboxylase of higher plants is a macromolecule consisting of eight large subunits and eight small subunits (Type I), and is a major soluble protein in leaves of plants. On the other hand, ribulose bisphosphate carboxylase of microorganisms such as bacteria consists of only small subunits (Type II).

Ribulose bisphosphate carboxylase is used as a marker for plant classification, and for example, as a cell marker for cell fusion. Further, in view of the possible improvement of the global environment, it has been attempted to modify ribulose bisphosphate carboxylase gene to produce a plant with increased fixation ability of $CO_2$ in the air. Breeding of photosynthetic bacteria and device having photosynthetic ability may be intended for development. For such purposes, it is useful to have a gene encoding ribulose bisphosphate carboxylase having increased enzymatic activity and structural stablility.

As used herein, the term "ribulose bisphosphate carboxylase refers to an enzyme adding $CO_2$ to ribulose phosphate to produce two molecules of 3-phosphoglycerinic acid. Further, ribulose bisphosphate carboxylase has an activity of adding $O_2$ to ribulose phosphate to produce 2-phosphoglycolic acid and 3-phosphoglycerinic acid (oxygenase activity).

(Expression of hyperthermostable ribulose bisphosphate carboxylase)

According to the method as described in the Examples above, hyperthermostable ribulose bisphosphate carboxylase (SEQ ID NO: 338) was expressed using PCR method. The resultant ampicillin resistant transformants were inoculated on to the NZCYM medium (1 % NZ amine, 0.5 % NaCl, 0.5 % yeast extract, 0.1 % casamino acid, 0.2 % MgSO4· 7$H_2$O(pH7)) containing amplicillin (50μg/ml), cultured at 37 °C until the $OD_{660}$ reached 0.5. Isopropyl-β-D-thiogalactopyranoside (IPTG, 0.1mM) was then added thereto and the culture was continued at 37 °C. After culture, cells were collected by centrifugation, broken by sonication in 100 mM vicine/KOH (pH8.3)/10mM $MgCl_2$, and centrifuged again to yield a soluble fraction, which was then heated at 85 °C for thirty minutes. Heat-stable soluble fractions were centrifuged and concetrated, and then were subjected to sodium dodecyl sulfate polyacrylamide electrophoresis (SDS-PAGE) to detect an expected band of a particular molecular weight, and the band was increased over time after the induction of IPTG (data not shown).

The samples obtained by centrifugation of the above-mentioned heat-stable soluble fractions were further purified using anion exchange column Resource Q (Amersham Pharmacia Biotech, Uppsala, Sweden), and gel filtration column Superdex 200 HR 10/30 (Amersham Pharmacia Biotech, Uppsala, Sweden), and confirmed that the band was single by SDS-PAGE (data not shown).

Purification was performed using AKTA explorer 10S (Amersham Pharmacia Biotech, Uppsala, Sweden). As for anionic exchange column, separation was performed by using gradient of 0-1.0 M NaCl, against buffer of 100 mM vicine/KOH (pH8.3)/10 mM $MgCl_2$. As for gel filtration, 50 mM sodium phosphate/0.15 M NaCl buffer was used.

Analysis using gel filtration suggests that the expressed enzyme forms an octamer consisting of only large subunits.

The carboxylase activity of samples as purified above were measured by using D-ribulose 1,5-bisphosphate (RuBP)(Sigma) as substrate, in accordance with a method described in Uemura, K.et al., Plant Cell Physiol., 37(3), 325-331 (1996).

First, optimal pH of the hyperthermostable ribulose bisphosphate carboxylase of the present invention was studied. Reactions were performed using a buffer containing citrate buffer (pH5.6), sodium phosphate buffer(pH6.3), vicine buffer (pH7.3, 7.8, 8.0 or 8.3), or glycine buffer (pH9.1 or 10.1), 10 mM $MgCl_2$, and 30 mM RuBP as substrate at a variety of temperatures. One unit of activity was characterized as fixing 1 μmol CO2 per mg per minute. The results were expressed as a ratio against activity at pH 8.3. These results demonstrate that the hyperthermostable ribulose bisphosphate carboxylase has an optimum pH at about 8.3.

The hyperthermostable ribulose bisphophate carboxylase of the present invention was investigated for its optimum temperature. Reactions were performed in buffer containing 1 0 0 mM vicine-KOH (pH8. 3) and 10 mM $MgCl_2$, using 3 0 mM R u B P as substrate at a variety of temperatures (data not shown). It was demonstrated that the hyperthemostable ribulose bisphosphate carboxylase of the present invention has an optimum temperature of about 90 °C.

The thermostablity of hyperthermostable ribulose bisphosphate carboxylase of the present invention was studied. The purified enzyme was measured for its remnant activities after incubation for a variety of time periods at 80 °C and 100 °C (data not shown). It was demonstrated that the thermostable ribulose bisphosphate carboxylase of the present invention has a half life of about 15 hours at 80 °C.

The carboxylase activity and oxygensase activity of the hyperthermostable ribulose phosphate carboxylase of the present invention was measured at 50-90°C. Further, τ value, which is carboxy activity/oxigenase activity, was calculated (see Ezaki et al., J. Biol. Chem. (J Biol Chem.1999 Feb 19;274(8):5078-82)).

From the increase in carbon dioxide in the air, environmental problems such as green house effects have occurred. As a solution thereto, ribulose phosphate carboxylase catalyzing carbon dioxide fixation is noted. The ratio of oxygen versus carbon dioxide in the air is about 20:0.03, and oxygen is much more abundant than carbon dioxide. Accordingly, for the purpose of the above, a high specificity against carboxylase reaction, that is greater τ value, is required. The enzymes from KOD-1 strain have higher τ values than those of conventional type II enzymes (about 30-200X) or those of type I enzymes (about 10X), and thus are expected to be useful for the application of more efficient carbon dioxide fixation.

(Example 12: fructose 1,6-bisphophate aldolase)

In order to express the fructose 1,6-bisphophate aldolase (SEQ ID NO: 1275) encoded by an open reading frame obtained by the present invention in Escherichia coli, the following operations were performed: fragments containing the open reading frames was amplified by PCR technology and inserted to plasmid pET21a(+) (Novagen) to yield an expression plasmid. This plasmid was used to transform the Escherichia coli BL1 (DE3) strain.

The resultant ampicillin resistant transformants were inoculated on to the NZCYM medium (1 % NZ amine, 0.5 % NaCl, 0.5 % yeast extract, 0.1% casamino acid, 0.2% MgSO4· 7H₂O(pH7)), cultured at 37 °C until the OD₆₆₀ reached 0.4. Isopropyl-β-D-thiogalactopyranoside (IPTG, 0.1mM) was then added thereto and culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.

The crude enzyme solution was measured according to KOSOGAKU HANDOBUKKU (Enzyme handbook) edited by Bunji MARUO, and Nobuo TAMIYA, published by Asakura shoten (1982), to confirm that the crude enzyme solutions have the fructose 1,6-bisphophate aldolase activity of interest. Further, the enzyme has an optimum temperature of 90 °C.

(Example 13: glycerol kinase)

In order to express the glycerol kinase (SEQ ID NO: 1646) encoded by an open reading frame obtained by the present invention, in Escherichia coli, the following operations were performed: fragments containing the open reading frames was amplified by PCR technology and inserted into plasmid pET21a(+) (Novagen) to yield expression plasmids. This plasmid was used to transform the Escherichia coli BL1 (DE3) strain.

The resultant ampicillin resistant transformants were inoculated on to the NZCYM medium (1 % NZ amine, 0.5 % NaCl, 0.5 % yeast extract, 0.1 % casamino acid, 0.2 % MgSO4·7H₂O(pH7)), cultured at 37 °C until the OD₆₆₀ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as crude enzyme solutions.

The crude enzyme solution was measured according to KOSOGAKU HANDOBUKKU (Enzyme handbook) edited by Bunj i MARUO, and Nobuo TAMIYA, published by Asakura shoten (1982), to confirm that the crude enzyme solutions have the enzymatic activity of interest. Further, the enzyme has an optimum temperature at 90 °C.

(Example 14: glutamate dehydrogenase)

In order to express the glutamate dehydeogenases (SEQ ID NO: 1239 and 1637) encoded by an open reading frame obtained by the present invention, in Escherichia coli, the following operations were performed: fragments containing the open reading frames was amplified by PCR technology and inserted to plasmid pET21a (+) (Novagen) to yield expression plasmids. These plasmids were used to transform the Escherichia coli BL1 (DE3) strain.

The resultant ampicillin resistant transformants were inoculated on to the NZCYM medium (1 % NZ amine, 0.5 % NaCl, 0.5 % yeast extract, 0.1 % casamino acid, 0.2 % MgSO₄·7H₂O(pH7)), cultured at 37 °C until the OD₆₆₀ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which were used as crude enzyme solutions.

These crude enzyme solutions were measured according to KOSOGAKU HANDOBUKKU (Enzyme handbook) edited by Bunji MARUO, and Nobuo TAMIYA, published by Asakura shoten (1982), to confirm that the crude enzyme solutions have the enzymatic activity of interest. Further, these enzymes have an optimum temperature at 90 °C.

(Example 15: pyruvate kinase)

In order to express the pyruvate kinase (SEQ ID NO: 1776) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % MgSO4·7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.

This crude enzyme solution was measured according to KOSOGAKU HANDOBUKKU (Enzyme handbook) edited by Bunji MARUO, and Nobuo TAMIYA, published by Asakura shoten (1982), to confirm that the crude enzyme solution has the enzymatic activity of interest. Further, this enzyme has an optimum temperature at 90 °C.

(Example 16: enolase)

In order to express the enolase (SEQ ID NO:681) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % MgSO4· 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.

This crude enzyme solution was measured according to KOSOGAKU HANDOBUKKU (Enzyme handbook) edited by Bunj i MARUO, and Nobuo TAMIYA, published by Asakura shoten (1982), to confirm that the crude enzyme solution has the enzymatic activity of interest. Further, this enzyme has an optimum temperature at 90 °C.

(Example 17: fructose 1,6-bisphophatase)

In order to express the fructose 1,6-bisphophatase (SEQ ID NO:1488) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % MgS04·7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.

This crude enzyme solution was measured according to KOSOGAKU HANDOBUKKU (Enzyme handbook) edited by Bunji MARUO, and Nobuo TAMIYA, published by Asakura shoten (1982), to confirm that the crude enzyme solution has the enzymatic activity of interest. Further, this enzyme has an optimum temperature at 90 °C.

(Example 18: hydrogenase)

In order to express the hydrogenase (each subunits correspond to SEQ ID NO:1141, 1142, 1502, and 1503) encoded by an open reading frames obtained by the present invention, in Escherichia coli, the following operations were performed: fragments containing the open reading frames were amplified by PCR technology and inserted into plasmid pET21a(+) (Novagen) to yield expression plasmids. These plasmids were used to transform the Escherichia coli BL1 (DE3) strains.

The resultant ampicillin resistant transformants were inoculated on to the NZCYM medium (1 % NZ amine, 0.5 % NaCl, 0.5 % yeast extract, 0.1 % casamino acid, 0.2 % MgSO4· 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extracts were heated at 80 °C for fifteen minutes, and then centrifuged to yield the supernatants thereof, which were used as crude enzyme solutions.

The crude enzyme solutions were measured according to KOSOGAKU HANDOBUKKU (Enzyme handbook) edited by Bunj i MARUO, and Nobuo TAMIYA, published by Asakura shoten (1982), to confirm that the crude enzyme solution has the enzymatic activity of interest. Further, this enzyme has an optimum temperature at 90 °C.

(Example 19: β-glycosidase)

In order to express the β-glycosidase (SEQ ID NO: 990) encoded by an open reading frame obtained by the present invention, in Escherichia coli, the following operations were performed: a fragment containing the open reading frame was amplified by PCR technology and inserted into plasmid pET21a (+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % MgSO$_4$· 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.

This crude enzyme solution was measured according to KOSOGAKU HANDOBUKKU (Enzyme handbook) edited by Bunji MARUO, and Nobuo TAMIYA, published by Asakura shoten (1982), to confirm that the crude enzyme solution has the enzymatic activity of interest. Further, this enzyme has an optimum temperature at 90 °C.

(Example 20: α-amylase)

In order to express the α-amylase (SEQ ID NO:268) encoded by an open reading frame obtained by the present invention, in Escherichia coli, the following operations were performed: a fragment containing the open reading frame was amplified by PCR technology and inserted into plasmid pET21a (+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % MgSO4· 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.

This crude enzyme solution was measured according to KOSOGAKU HANDOBUKKU (Enzyme handbook) edited by Bunj i MARUO, and Nobuo TAMIYA, published by Asakura shoten (1982), to confirm that the crude enzyme solution has the enzymatic activity of interest. Further, this enzyme has an optimum temperature at 90 °C.

(Example 21: deacetylase)

In order to express the deacetylase (SEQ ID NO:1190) encoded by an open reading frame obtained by the present invention, in Escherichia coli, the following operations were performed: a fragment containing the open reading frame was amplified by PCR technology and inserted into plasmid pET21a (+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % MgSO4· 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.

This crude enzyme solution was measured according to KOSOGAKU HANDOBUKKU (Enzyme handbook) edited by Bunj i MARUO, and Nobuo TAMIYA, published by Asakura shoten (1982), to confirm that the crude enzyme solution has the enzymatic activity of interest. Further, this enzyme has an optimum temperature at 90 °C.

(Example 22: cyclodextrin glucanotransfrase)

In order to express the cyclodextrin glucanotransfrase(SEQ ID NO:1068) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.
The resultant ampicillin resistant transformant was inoculated on to the NZCYM medium (1 % NZ amine, 0.5 % NaCl, 0.5 % yeast extract, 0.1 % casamino acid, 0.2 % MgSO4· 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.
This crude enzyme solution was measured according to KOSOGAKU HANDOBUKKU (Enzyme handbook) edited by Bunji MARUO, and Nobuo TAMIYA, published by Asakura shoten (1982), to confirm that the crude enzyme solution has the enzymatic activity of interest. Further, this enzyme has an optimum temperature at 90 °C.

(Example 23: 4-α-D-glucanotransferase)

In order to express the 4-α-D-glucanotransferase (SEQ ID NO:1185) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.
The resultant ampicillin resistant transformant was inoculated on to the NZCYM medium (1 % NZ amine, 0.5 % NaCl, 0.5 % yeast extract, 0.1 % casamino acid, 0.2 % MgSO4. 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.
This crude enzyme solution was measured according to KOSOGAKU HANDOBUKKU (Enzyme handbook) edited by Bunj i MARUO, and Nobuo TAMIYA, published by Asakura shoten (1982), to confirm that the crude enzyme solution has the enzymatic activity of interest. Further, this enzyme has an optimum temperature at 90 °C.

(Example 24: DNA polymerases)

In order to express the DNA polymerases (SEQ ID NO:2, 93, 379, 648, 649, 743, 1386, 1740 and 1830) encoded by open reading frames obtained by the present invention, in Escherichia coli, the following operations were performed: fragments containing the open reading frames were amplified by PCR technology and inserted into plasmid pET21a(+) (Novagen) to yield expression plasmids. These plasmids were used to transform the Escherichia coli BL1 (DE3) strains.
The resultant ampicillin resistant transformants were inoculated on to the NZCYM medium (1 % NZ amine, 0.5 % NaCl, 0.5 % yeast extract, 0.1 % casamino acid, 0.2 % MgSO4·7H$_2$O(pH7)), cultured at 37 °C until the OD$_{660}$ reached 0.4. Isopropyl-β-D-thiogalactopyranoside (IPTG, 0.1mM) was then added thereto and culture was continued at 37 °C for four hours. After culture, cells were collected by centrifugation, broken by sonication, and centrifuged to yield a cell extract. The resultant cell extracts were heated at 80 °C for fifteen minutes, and then centrifuged to yield the supernatants thereof, which were used as crude enzyme solutions.
These crude enzyme solutions were measured according to KOSOGAKU HANDOBUKKU (Enzyme handbook) edited by Bunji MARUO, and Nobuo TAMIYA, published by Asakura shoten (1982), to confirm that the crude enzyme solution has the enzymatic activity of interest for the respective sequences. Further, this enzyme has an optimum temperature at 90 °C for the respective sequences.

(Example 25: homing endonuclease)

In order to express the homing endonuclease (SEQ ID NO:2) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.
The resultant ampicillin resistant transformant was inoculated on to the NZCYM medium (1 % NZ amine, 0.5 % NaCl, 0.5 % yeast extract, 0.1 % casamino acid, 0.2 % MgSO4· 7H$_2$O(pH7)), cultured at 37 °C until the OD$_{660}$ reached 0.4. Isopropyl-β-D-thiogalactopyranoside (IPTG, 0.1mM) was then added thereto and culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.

This crude enzyme solution was measured by a modified method of endonuclease assay according KOSOGAKU HANDOBUKKU (Enzyme handbook) edited by Bunji MARUO, and Nobuo TAMIYA, published by Asakura shoten (1982), to confirm that the crude enzyme solution has the enzymatic activity of interest. Further, this enzyme has an optimum temperature at 90 °C.

(Example 26: histones)

In order to express the histones (SEQ ID NO: 173, 1470 and 1963 and the like) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % MgSO4. 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude protein solution.

This crude protein solution was measured by a method using histone kinase as described in KOSOGAKU HANDOBUKKU (Enzyme handbook) edited by Bunji MARUO, and Nobuo TAMIYA, published by Asakura shoten (1982), to confirm that the crude protein solutions hasve an activity as a substrate for the activity of interest. Further, this protein was stable at 90 °C.

(Example 27: histones A&B)

In order to express the histones A and B (SEQ ID NO: 1470 and 1962) encoded by open reading frames obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % MgSO4· 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatants thereof, which were used as crude protein solutions.

These crude protein solutions were measured by a method using histone kinase as described in KOSOGAKU HANDOBUKKU (Enzyme handbook) edited by Bunji MARUO, and Nobuo TAMIYA, published by Asakura shoten (1982), to confirm that the crude protein solutions have an activity as a substrate for the activity of interest. Further, these proteins were stable at 90 °C.

(Example 28: Rec protein)

In order to express the Rec protein (SEQ ID NO:1106) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % MgSO4· 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude protein solution.

This crude protein solution was measured according to Methods in Enzymology 262 (1995) to confirm that the crude protein solution has an activity of the Rec protein. Further, this protein was stable at 90 °C.

(Example 29: O[6]-methylguanine DNA methyl transferase)

In order to express the O[6]-methylguanine DNA methyl transferase (SEQ ID NO: 1034) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % $MgSO_4 \cdot 7H_2O$(pH7)), cultured at 37 °C until the $OD_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.

This crude enzyme solution was measured according to Methods in Enzymology 262 (1995) to confirm that the crude enzyme solution has the enzymatic activity of interest. Further, this enzyme has an optimum temperature at 90 °C.

(Example 30: PCNA)

In order to express the PCNA (Proliferating Cell Nuclear Antigen) (SEQ ID NO:93) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % $MgSO4 \cdot 7H_2O$(pH7)), cultured at 37 °C until the $OD_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude protein solution.

This crude protein solution was measured according to Methods in Enzymology 262 (1995) to confirm that the crude protein solution has the activity of the PCNA protein. Further, this protein was stable at 90 °C.

(Example 31: indole pyruvate ferredoxin oxydoreductases)

In order to express the indole pyruvate ferredoxin oxydoreductases (SEQ ID NOs: ) encoded by open reading frames obtained by the present invention, in Escherichia coli, the following operations were performed: fragments containing the open reading frames were amplified by PCR technology and inserted into plasmid pET21a(+) (Novagen) to yield expression plasmids. These plasmids were used to transform Escherichia coli BL1 (DE3) strains.

The resultant ampicillin resistant transformants were inoculated on to the NZCYM medium (1 % NZ amine, 0.5 % NaCl, 0.5 % yeast extract, 0.1% casamino acid, 0.2 % $MgSO4 \cdot 7H_2O$(pH7)), cultured at 37 °C until the $OD_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extracts were heated at 80 °C for fifteen minutes, and then centrifuged to yield the supernatants thereof, which were used as crude enzyme solutions.

These crude enzyme solutions were measured according to KOSOGAKU HANDOBUKKU (Enzyme handbook), edited by Bunji MARUO and Nobuo TAMIYA, published by Asakura shoten (1982), to confirm that the crude enzyme solutions have the enzymatic activity of interest for the respective sequences. Further, these enzymes have an optimum temperature at 90 °C for the respective sequences.

(Example 32: glutamine synthase)

In order to express the glutamine synthase (SEQ ID NO:627) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % $MgSO4 \cdot 7H_2O$(pH7)) cultured at 37 °C until the $OD_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.

This crude enzyme solution was measured according to KOSOGAKU HANDOBUKKU (Enzyme handbook) edited by Bunj i MARUO, and Nobuo TAMIYA, published by Asakura shoten (1982), to confirm that the crude enzyme solution has the enzymatic activity of interest. Further, this enzyme has an optimum temperature at 90 °C.

(Example 33: anthranilate phosphoribosyl transferases)

In order to express the anthranilate phosphoribosyl transferases (SEQ ID NO:394 and 1767) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % MgS04. 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatants thereof, which were used as crude enzyme solutions.

The crude enzyme solutions were measured according to KOSOGAKU HANDOBUKKU (Enzyme handbook) edited by Bunji MARUO, and Nobuo TAMIYA, published by Asakura shoten (1982), to confirm that the crude enzyme solutions have the enzymatic activity of interest. Further, this enzyme has an optimum temperature at 90 °C.

(Example 34: cobyric acid synthase)

In order to express the cobyric acid synthases (SEQ ID NO: 137 and 1904) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % MgSO4· 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatants thereof, which were used as crude enzyme solutions.

The crude enzyme solutions were measured according to Methods in Enzymology, Acadmic Press, to confirm that the crude enzyme solutions have the enzymatic activity of interest. Further, this enzyme has an optimum temperature of 90 °C.

(Example 35: phosphoribosyl anthranilate isomerase)

In order to express the phosphoribosyl anthranilate isomerase (SEQ ID NO:44) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % MgSO4·7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.

This crude enzyme solution was measured according to KOSOGAKU HANDOBUKKU (Enzyme handbook) edited by Bunji MARUO, and Nobuo TAMIYA, published by Asakura shoten (1982), to confirm that the crude enzyme solution has the enzymatic activity of interest. Further, this enzyme has an optimum temperature of 90 °C.

(Example 36: cobalamin synthase)

In order to express the cobalamin synthase (SEQ ID NO:181, 910, 1720 and 1973) encoded by open reading frames obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformants were inoculated on to the NZCYM medium (1 % NZ amine, 0.5 % NaCl, 0.5 % yeast extract, 0.1% casamino acid, 0.2 % MgSO4·7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatants thereof, which were used as crude enzyme solutions.

The crude enzyme solutions were measured according to Methods in Enzymology, Acadmic Press, to confirm that the crude enzymes solutions have the enzymatic activity of interest. Further, these enzymes have an optimum temperature of 90 °C.

(Example 37: indole-3-glycerole-phophate synthase)

In order to express the indole-3-glycerole-phophate synthase (SEQ ID NO: 772) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % MgSO4· 7H$_2$O (pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.

This crude enzyme solution was measured according to KOSOGAKU HANDOBUKKU (Enzyme handbook) edited by Bunj i MARUO, and Nobuo TAMIYA, published by Asakura shoten (1982), to confirm that the crude enzyme solution has the enzymatic activity of interest. Further, this enzyme has an optimum temperature of 90 °C.

(Example 38: tryptophane synthase)

In order to express the tryptophane synthase (SEQ ID NO:395, 774, 954 and 2032) encoded by open reading frames obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformants were inoculated on to the NZCYM medium (1 % NZ amine, 0.5 % NaCl, 0.5 % yeast extract, 0.1 % casamino acid, 0.2 % MgSO4·7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatants thereof, which were used as crude enzyme solutions.

The crude enzyme solutions were measured according to KOSOGAKU HANDOBUKKU (Enzyme handbook) edited by Bunj i MARUO, and Nobuo TAMIYA, published by Asakura shoten (1982), to confirm that the crude enzyme solutions have the enzymatic activity of interest. Further, these enzymes have an optimum temperature at 90 °C.

(Example 39: ribose phosphate pyrophosphokinase)

In order to express the ribose phosphate pyrophosphokinase (SEQ ID NO: 701) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % MgSO4· 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.

This crude enzyme solution was measured according to KOSOGAKU HANDOBUKKU (Enzyme handbook) edited by Bunj i MARUO, and Nobuo TAMIYA, published by Asakura shoten (1982), to confirm that the crude enzyme solution has the enzymatic activity of interest. Further, this enzyme has an optimum temperature at 90 °C.

(Example 40: glutamate synthase)

In order to express the glutamate synthase (SEQ ID NO: 1578) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % MgSO4· 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.

This crude enzyme solution was measured according to KOSOGAKU HANDOBUKKU (Enzyme handbook) edited by Bunji MARUO, and Nobuo TAMIYA, published by Asakura shoten (1982), to confirm that the crude enzyme solution has the enzymatic activity of interest. Further, this enzyme has an optimum temperature at 90 °C.

(Example 41: Orotidine-5'-phosphate decarboxylase)

In order to express the orotidine-5'-phosphate decarboxylase (SEQ ID NO: 1096) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % MgSO4· 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.

This crude enzyme solution was measured according to KOSOGAKU HANDOBUKKU (Enzyme handbook) edited by Bunji MARUO, and Nobuo TAMIYA, published by Asakura shoten (1982), to confirm that the crude enzyme solution has the enzymatic activity of interest. Further, this enzyme has an optimum temperature at 90 °C.

(Example 42: anthranilate synthase)

In order to express the anthranilate synthase (SEQ ID NO:43 and 773) encoded by open reading frames obtained by the present invention, in Escherichia coli, the following operations were performed: a fragment containing the open reading frame was amplified by PCR technology and inserted into plasmid pET21a(+) (Novagen) to yield an expression plasmid. These plasmids were used to transform the Escherichia coli BL1 (DE3) strain.

The resultant ampicillin resistant transformants were inoculated on to the NZCYM medium (1 % NZ amine, 0.5 % NaCl, 0.5 % yeast extract, 0.1 % casamino acid, 0.2 % MgSO$_4$· 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatants thereof, which were used as crude enzyme solutions.

The crude enzyme solutions were measured according to KOSOGAKU HANDOBUKKU (Enzyme handbook) edited by Bunji MARUO, and Nobuo TAMIYA, published by Asakura shoten (1982), to confirm that the crude enzyme solutions have the enzymatic activity of interest. Further, these enzymes have an optimum temperature at 90 °C.

(Example 43: aspartyl-tRNA synthase)

In order to express the aspartyl-tRNA synthase (SEQ ID NO: 808) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % casamino acid, 0.2 % MgSO$_4$· 7H$_2$O(pH7)), cultured at 37 °C until the OD$_{660}$ reached

0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.

This crude enzyme solution was measured according to KOSOGAKU HANDOBUKKU (Enzyme handbook) edited by Bunj i MARUO, and Nobuo TAMIYA, published by Asakura shoten (1982), to confirm that the crude enzyme solution has the enzymatic activity of interest. Further, this enzyme has an optimum temperature at 90 °C.

(Example 44: phenylalanyl-tRNA-synthase)

In order to express the phenylalanyl-tRNA-synthase (SEQ ID NO:506 and 878) encoded by open reading frames obtained by the present invention, in Escherichia coli, the following operations were performed: a fragment containing the open reading frame was amplified by PCR technology and inserted into plasmid pET21a(+) (Novagen) to yield an expression plasmid. These plasmids were used to transform the Escherichia coli BL1 (DE3) strain.

The resultant ampicillin resistant transformants were inoculated on to the NZCYM medium (1 % NZ amine, 0.5 % NaCl, 0.5 % yeast extract, 0.1 % casamino acid, 0.2 % $MgSO_4 \cdot 7H_2O$(pH7)), cultured at 37 °C until the $OD_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatants thereof, which were used as crude enzyme solutions.

The crude enzyme solutions were measured according to KOSOGAKU HANDOBUKKU (Enzyme handbook) edited by Bunji MARUO, and Nobuo TAMIYA, published by Asakura shoten (1982), to confirm that the crude enzyme solutions have the enzymatic activity of interest. Further, these enzyme has an optimum temperature at 90 °C.

(Example 45: chaperonins)

In order to express the chaperonin A (SEQ ID NO: 1368) and the chaperonin B (SEQ ID NO: 721) encoded by open reading frames obtained by the present invention, in Escherichia coli, the following operations were performed: a fragment containing the open reading frames were amplified by PCR technology and inserted into plasmid pET21a(+) (Novagen) to yield an expression plasmid. These plasmids were used to transform the Escherichia coli BL1 (DE3) strain.

The resultant ampicillin resistant transformants were inoculated on to the NZCYM medium (1 % NZ amine, 0.5 % NaCl, 0.5 % yeast extract, 0.1 % casamino acid, 0.2 % $MgSO4 \cdot 7H_2O$(pH7)), cultured at 37 °C until the $OD_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatants thereof, which were used as crude protein solutions.

These crude protein solutions were measured by a method described in Frydman, J. et al. (1994 ) Nature 370, 111., to confirm that the crude protein solutions have activity as a substrate for the enzyme of interest. Further, these proteins were stable at 90 °C.

(Example 46: TATA binding protein)

In order to express the TATA binding protein (SEQ ID NO: 31) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % $MgSO4 \cdot 7H_2O$(pH7)), cultured at 37 °C until the $OD_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude protein solution.

This crude protein solution was measured according to Methods in Enzymology, Academic Press, to confirm that the crude protein solution has the activity of the protein. Further, this protein was stable at 90 °C.

(Example 47: TBP-interacting protein)

In order to express the TBP-interacting protein (SEQ ID NO: 1289) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % $MgSO_4 \cdot 7H_2O(pH7)$), cultured at 37 °C until the $OD_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude protein solution.

This crude protein solution was measured according to Methods in Enzymology, Academic Press, to confirm that the crude protein solution has the activity of the protein. Further, this protein was stable at 90 °C.

(Example 48: RNase HII)

In order to express the RNase HII (SEQ ID NO:856) encoded by an open reading frame obtained by the present invention, in Escherichia coli, the following operations were performed: a fragment containing the open reading frame was amplified by PCR technology and inserted into plasmid pET21a (+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % $MgSO4 \cdot 7H_2O(pH7)$), cultured at 37 °C until the $OD_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.

This crude enzyme solution was measured according to KOSOGAKU HANDOBUKKU (Enzyme handbook) edited by Bunji MARUO, and Nobuo TAMIYA, published by Asakura shoten (1982), to confirm that the crude enzyme solution has the enzymatic activity of interest. Further, this enzyme has an optimum temperature at 90 °C.

(Example 49: hydrogenase maturation factor)

In order to express the hydrogenase maturation factors (SEQ ID NO: 1144, 1154, 1156, 1516, 1518, 1519, 1869 and 1871) encoded by open reading frames obtained by the present invention, in Escherichia coli, the following operations were performed: a fragment containing the open reading frames were amplified by PCR technology and inserted into plasmid pET21a (+) (Novagen) to yield an expression plasmid. These plasmids were used to transform the Escherichia coli BL1 (DE3) strain.

The resultant ampicillin resistant transformants were inoculated on to the NZCYM medium (1 % NZ amine, 0.5 % NaCl, 0.5 % yeast extract, 0.1 % casamino acid, 0.2 % $MgSO4. 7H_2O(pH7)$), cultured at 37 °C until the $OD_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatants thereof, which were used as crude protein solutions.

This crude protein solutions were measured according to KOSOGAKU HANDOBUKKU (Enzyme handbook) edited by Bunji MARUO, and Nobuo TAMIYA, published by Asakura shoten (1982), to confirm that the crude protein solutions have activity as substrates for the enzyme of interest. Further, these proteins were stable at 90 °C.

(Example 50: Lon protease)

In order to express the Lon protease (SEQ ID NO: 929) encoded by an open reading frame obtained by the present invention, in Escherichia coli, the following operations were performed: a fragment containing the open reading frame was amplified by PCR technology and inserted into plasmid pET21a (+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % $MgSO4 \cdot 7H_2O(pH7)$), cultured at 37 °C until the $OD_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.

This crude enzyme solution was measured according to KOSOGAKU HANDOBUKKU (Enzyme handbook) edited by Bunj i MARUO, and Nobuo TAMIYA, published by Asakura shoten (1982), to confirm that the crude enzyme solution has the enzymatic activity of interest. Further, this enzyme has an optimum temperature at 90 °C.

(Example 51: thiol protease)

In order to express the thiol protease encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % MgSO4· 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.

This crude enzyme solution was measured according to KOSOGAKU HANDOBUKKU (Enzyme handbook) edited by Bunj i MARUO, and Nobuo TAMIYA, published by Asakura shoten (1982), to confirm that the crude enzyme solution has the enzymatic activity of interest. Further, this enzyme has an optimum temperature at 90 °C.

(Example 52: fragellins)

In order to express the fragellins (SEQ ID NO: 11, 350, 351, 727, and 728) encoded by open reading frames obtained by the present invention, in Escherichia coli, the following operations were performed: a fragment containing the open reading frames were amplified by PCR technology and inserted into plasmid pET21a(+) (Novagen) to yield an expression plasmid. These plasmids were used to transform the Escherichia coli BL1 (DE3) strain.

The resultant ampicillin resistant transformants were inoculated on to the NZCYM medium (1 % NZ amine, 0.5 % NaCl, 0.5 % yeast extract, 0.1% casamino acid, 0.2 % MgSO4· 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatants thereof, which were used as crude protein solutions.

This crude protein solutions were measured according to Aldridge P, Hughes KT.,Curr Opin Microbiol. 2002 Apr; 5(2):160-5 and the references cited therein, to confirm that the crude protein solutions have activity as a substrate for the protein of interest. Further, these proteins were stable at 90 °C.

(Example 53: subtilin-like protease)

In order to express the subtilin-like protease (SEQ ID NO: 979) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % MgSO4· 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.

This crude enzyme solution was measured according to KOSOGAKU HANDOBUKKU (Enzyme handbook) edited by Bunj i MARUO, and Nobuo TAMIYA, published by Asakura shoten (1982), to confirm that the crude enzyme solution has the enzymatic activity of interest. Further, this enzyme has an optimum temperature at 90 °C.

(Example 54: cell division control protein A)

In order to express the cell division control protein A (SEQ ID NO: 1369) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % MgSO4· 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude protein solution.

This crude protein solution was measured for cell division controlling activity, to confirm that the crude protein solution has the activity of the protein of interest. Further, this protein was stable at 90 °C.

(Example 55: endonucleases)

In order to express the endonucleases (SEQ ID NOs: 547, 697, 900, 1450, 1702, 1716, 1731, and 2010) encoded by open reading frames obtained by the present invention, in Escherichia coli, the following operations were performed: fragments containing the open reading frames were amplified by PCR technology and inserted into plasmid pET21a(+) (Novagen) to yield expression plasmids. These plasmids were used to transform the Escherichia coli BL1 (DE3) strains.

The resultant ampicillin resistant transformants were inoculated on to the NZCYM medium (1 % NZ amine, 0.5 % NaCl, 0.5 % yeast extract, 0.1 % casamino acid, 0.2 % MgSO$_4$· 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extracts were heated at 80 °C for fifteen minutes, and then centrifuged to yield the supernatants thereof, which were used as crude enzyme solutions.

These crude enzyme solutions were measured according to KOSOGAKU HANDOBUKKU (Enzyme handbook) edited by Bunji MARUO, and Nobuo TAMIYA, published by Asakura shoten (1982), to confirm that the crude enzyme solutions have the enzymatic activity of interest for the respective sequences. Further, these enzymes have an optimum temperature at 90 °C for the respective sequences.

(Example 56: ferredoxin)

In order to express the ferredoxin (SEQ ID NO:253) encoded by an open reading frame obtained by the present invention, in Escherichia coli, the following operations were performed: a fragment containing the open reading frame was amplified by PCR technology and inserted into plasmid pET21a (+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % MgSO4· 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude protein solution.

This crude protein solution was measured according to KOSOGAKU HANDOBUKKU (Enzyme handbook) edited by Bunji MARUO, and Nobuo TAMIYA, published by Asakura shoten (1982), to confirm that the crude protein solution has the activity of the protein of interest. Further, this protein was stable at 90 °C.

(Example 57: exo-β-D-glucosaminidase)

In order to express the exo-β-D-glucosaminidase (SEQ ID NO:1902) encoded by an open reading frame obtained by the present invention, in Escherichia coli, the following operations were performed: a fragment containing the open reading frame was amplified by PCR technology and inserted into plasmid pET21a(+) (Novagen) to yield an expression plasmid. This plasmid was used to transform the Escherichia coli BL1 (DE3) strain.

The resultant ampicillin resistant transformant was inoculated on to the NZCYM medium (1 % NZ amine, 0.5 % NaCl, 0.5 % yeast extract, 0.1 % casamino acid, 0.2 % MgSO4· 7H$_2$O(pH7)), cultured at 37 °C until the OD$_{660}$ reached 0.4. Isopropyl-β-D-thiogalactopyranoside (IPTG, 0.1mM) was then added thereto and the culture was continued at 37 °C for four hours. After culture, cells were collected by centrifugation, broken by sonication, and centrifuged to yield a cell extract. The resultant cell extract was heated at 80 °C for fifteen minutes, and then centrifuged to yield the supernatant thereof, which was used as a crude enzyme solution.

This crude enzyme solution was measured according to KOSOGAKU HANDOBUKKU (Enzyme handbook) edited by Bunj i MARUO, and Nobuo TAMIYA, published by Asakura shoten (1982), to confirm that the crude enzyme solution has the enzymatic activity of interest. Further, this enzyme has an optimum temperature at 90 °C.

(Example 58: confirmation of other deduced functions)

In order to express the gene products encoded by open reading frames obtained by the present invention, in Escherichia coli, the following operations are performed: fragments containing the open reading frames are amplified by PCR technology and inserted into plasmid pET21a(+) (Novagen) to yield expression plasmids. These plasmids are used to transform the Escherichia coli BL1 (DE3) strains.

The resultant ampicillin resistant transformants are inoculated on to the NZCYM medium (1 % NZ amine, 0.5 % NaCl, 0.5 % yeast extract, 0.1 % casamino acid, 0.2 % MgSO4·7H$_2$O(pH7)), cultured at 37 °C until the OD$_{660}$ reached 0.4. Isopropyl-β-D-thiogalactopyranoside (IPTG, 0.1mM) is then added thereto and the culture is continued at 37 °C for four hours. After culture, cells are collected by centrifugation, broken by sonication, and centrifuged to yield a cell extract. The resultant cell extracts are heated at 80 °C for fifteen minutes, and then centrifuged to yield the supernatants thereof, which are used as crude enzyme solutions.

These crude enzyme solutions are measured according to KOSOGAKU HANDOBUKKU (Enzyme handbook) edited by Bunji MARUO, and Nobuo TAMIYA, published by Asakura shoten (1982), to confirm that the crude enzyme solution has the activity of interest for the respective sequences. Further, this enzyme has an optimum temperature or is stable at 90 °C for the respective sequences.

(EXAMPLE 59: biomolecule chip - DNA chip)

Next, an exemplary prepration of a biomolecule chip is demonstrated. In this Example, methods for DNAs having different sequences being aligned and immobilized thereon are described.

Aggregates of DNA fragments having specific sequences of the present invention are immobilized in a DNA spot form on a substrate. As a substrate, glass is usually used but plastic may also be used. Formats for DNA chips may be rectangular or circular. Each DNA dot comprises a DNA encoding a different gene of the present invention, and is immobilized onto the substrate. The size of the DNA dot is 100-200 $\mu$m in diameter in case of microarrays, and in the case of a DNA chip, about 10-30 $\mu$m.

Next, methods for forming each DNA spot are described. For example, a DNA solution of interest is located onto a DNA substrate using pin methods, inkjet format and the like.

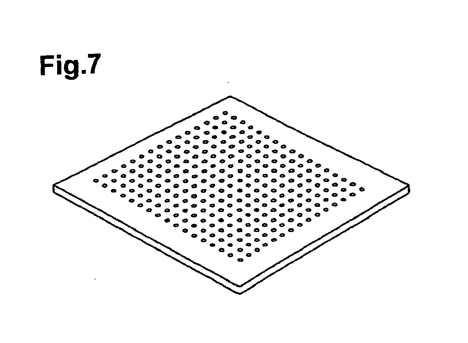

Exemplary preparation of such DNA chips prepared thereby is shown in Figure 7.

(Example 60: Biomolecule chip - Protein Chip)

Next, an exemplary preparation of biomolecule chips is demonstrated. In this Example, methods for aligning proteins having different sequences on a substrate and immobilized thereto, are described.

Aggregates of the protein fragments of specific sequences of the present invention are immobilized on a substrate in a form of a dot. Glass is usually used as a substrate, but plastic may also be used. Formats may be rectangular, as with a DNA chip, or circular. Each protein dot comprises a protein from a different gene of the present invention and is immobilized onto the substrate. The size of the protein dot is 100-200 $\mu$m in diameter in case of microarrays, and in the case of DNA chip, about 10-30 $\mu$m.

Next, methods for forming each protein spot are described. For example, the protein solution of interest is located onto a protein substrate using pin methods, inkjet format and the like.

Exemplary preparation of such protein chips prepared thereby is shown in Figure 7. Outlooks thereof are similar to that of DNA chip.

Although certain preferred embodiments have been described herein, it is not intended that such embodiments be construed as limitations on the scope of the invention except as set forth in the appended claims. Various other modifications and equivalents will be apparent to and can be readily made by those skilled in the art, after reading the description herein, without departing from the scope and spirit of this invention. All patents, published patent applications and publications cited herein are incorporated by reference as if set forth fully herein.

(Effects of the invention)

The present invention provides a method and kit for gene targeting in an efficient and accurate manner at any position in the genome of an organism. Further, information of the entire genomic sequence of Thermococcus kodakaraensis KOD1, and the gene information contained therein are also provided.

INDUSTRIAL APPLICABILITY

The present invention provides a variety of hyperthermostable gene products, and thus is useful in providing a method and kit for gene targeting in an efficient and accurate manner at any position in the genome of an organism. Such a variety of hyperthermostable gene products are applicable to global analysis of a hyperthermostable organism in genomic analysis and the like.

**Claims**

1. A nucleic acid molecule comprising a sequence encoding an amino acid sequence SEQ ID NO: 1131; or a sequence having 70% homology thereto.

2. A polypeptide comprising an amino acid sequence encoded by the nucleic acid sequence SEQ ID NO: 1131, or a sequence having at least 70% homology thereto.

3. A polypeptide comprising at least three contiguous amino acids of an amino acid sequence SEQ ID NO: 1131, or a sequence having at lease 70% homology thereto.

4. A polypeptide comprising at lease eight contiguous amino acids of an amino acid sequence encoded by the nucleic acid sequence SEQ ID NO: 1131, or a sequence having at lease 70% homology thereto.

5. A polypeptide comprising at least three contiguous amino acids of an amino acid sequence encoded by the nucleic acid sequence SEQ ID NO: 1131, or a sequence having at lease 70% homology thereto, wherein the polypeptide has biological activity.

6. The polypeptide according to claim 5, wherein the biological activity comprises an ATP-dependent DNA ligase.

7. The biomolecule chip having a nucleic acid sequence or the variant thereof comprising SEQ ID NO: 1131.

8. A biomolecule chip with a polypeptide or a variant thereof, having amino acid sequence encoded by the nucleic acid sequence of SEQ ID NO: 1131, or a sequence having at lease 70% homology thereto, located therein.

9. A biomolecule chip having at least one antibody against a polypeptide or a variant thereof, located on a substrate, the polypeptide or the variant thereof comprises an amino acid sequence encoded by the nucleic acid sequence of SEQ ID NO: 1131, or a sequence having at least 70% homology thereto.

# Fig.1

**Disrupting Vector**
pUDT2

about 1000bp      about 750bp

trpD    p pyrF    trpG

**Unrecombined
Genome DNA**

trpD    trpE    trpG

**Homologous
Recombination**

**Recombination
Double Crossover**

**Recombined
Genome DNA**

trpD    p pyrF    trpG

**Disrupted
strain**

P: **Promoter**

**Fig.2**

# Fig.3

# Fig.4 *Thermococcus kodakaraensis* KOD1

## Genome Project

*T. kodakaraensis*
**KOD1 strain**
2,089,377 bp

Labels around circle: tbo, pol, HpkB, cpkB, prsA, pyrF, cgt, lubB, flaB1B2B3B4B5, tbp, lorAB, eno, lig, trxB, fdhABC, hydBGDA, hypCD, ada, ycoY, trpCDEGFBA, aspC, amyA, aspS, malQ, glnA, chiA, gly, glm, cdcA, cpkA, fdx, aspB, sub, mhB, cobQ, cobS, gdhA, HpkA, glpK, lon, tip49, fba

amyA α-amylase
aspB gulutamate synthase
aspC aspartate synthase
aspS aspartyl-tRNA shynthase
cdcA cell division control protein
cgt cyclodextrin glucanotransferase
chiA chitinase
cobQ cobyrinic synthase
cobS cobalamin 5'-phosphate synthase
cpkA,cpkB chapelonin α and β
eno enolase
fba fructose 1,6-bisphosphoric aldolase
fdhABC formate dehydrogenase
fdx ferredoxin
flaB flagellin B
gdhA gulutamate dehydrogenase
glm glucosaminidase
glnA gulutamate synthetase
glpK glycerol kinase
gly β-gulucosidase
hpkA,hpkB histone A and B
hydBGDA hydrogenase
hypCD hydrogenase-accessary-protein
lorAB indol piruvate ferredoxinoxy reductase
lig ATP-dependent DNA ligase
lon lon protease malQ 4-α-glucanotransferase
pol DNA-dependent DNA polymerase family B
prsA ribose phosphate pyrophosphokinase
pyrF orotidine 5'-phosphate decarboxylase
rbc ribulose 1,5-bis phosphate carboxylse/oxygenase
rnhB ribonuclease H II
sub subtilisin-like protease precursor
tbp TATA binding protein
tip49 TBP interaction protein
trpCDEGHBA tryptophan biosynthesis operon
trxB thioredoxin reductase
tubB ftsZ(cell devision GTPase)homologue
yccY tyrophosphatase

EP 1 923 464 A1

**Fig.5** ORF clustering of *T. kodakaraensis* KOD1 genome

**Total : 2293 ORFs**

COG mismatched
935 ORFs

167 ORFs
231 ORFs

annotationable
960 ORFs

Unknown function    Predictive general function

134 ORFs; translation and ribosome protein
84 ORFs; transcription
68 ORFs; DNA replication, recombination or repair
19 ORFs; cell division and chromosomal fractionation
45 ORFs; post-translational events, protein turnover or chaperone
38 ORFs; cellular membranes or outer membranes
39 ORFs; cellular movement or secretion
68 ORFs; inorganic ion transportation or metabolism
20 ORFs; signaling mechanisms
109 ORFs; energy production and conversion
72 ORFs; carbohydrate transportation and metabolism
106 ORFs; amino acid transportation and metabolism
50 ORFs; nucleotide transportation and metabolism
57 ORFs; coenzyme metabolism
16 ORFs; lipid metabolism
35 ORFs; secondary metabolites biosynthesis, transportation or catabolism

# Fig.6

ORF map of *T. kodakaraensis* KOD1 genome

## ORFs:2293

**average gene length : 817.2bp**
**cover 89.7% of total genome**

EP 1 923 464 A1

Fig.7

**European Patent Office**

**EUROPEAN SEARCH REPORT**

Application Number

EP 08 00 2997

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NAKATANI M ET AL: "A DNA LIGASE FROM A HYPERTHERMOPHILIC ARCHAEON WITH UNIQUE COFACTOR SPECIFICITY" JOURNAL OF BACTERIOLOGY, WASHINGTON, DC, US, vol. 182, no. 22, 2000, pages 6424-6433, XP002974649 ISSN: 0021-9193 * The sequence of the ligase is 100% identical to present SEQ. ID. No. 1131 over 562 amino acids * * figure 2 * * page 6429, last two sentences of the paragraph bridging the left and right columns * | 1-9 | INV. C12N15/00 C07K14/195 C12Q1/68 C12Q1/02 |
| X | NAKATANI MASARU ET AL: "Substrate recognition and fidelity of strand joining by an archaeal DNA ligase" EUROPEAN JOURNAL OF BIOCHEMISTRY, BERLIN, DE, vol. 269, no. 2, January 2002 (2002-01), pages 650-656, XP002377933 ISSN: 0014-2956 * figure 5 * | 1-9 | TECHNICAL FIELDS SEARCHED (IPC) C07K C12Q |
| X | IMANAKA TADAYUKI ET AL: "Catalyzing "hot" reactions: enzymes from hyperthermophilic Archaea." CHEMICAL RECORD (NEW YORK, N.Y.) 2002, vol. 2, no. 3, May 2002 (2002-05), pages 149-163, XP002475086 ISSN: 1527-8999 * page 157 * * figures 7,8 * | 1-9 | |

-/--

The present search report has been drawn up for all claims

| Place of search | Date of completion of the search | Examiner |
|---|---|---|
| Munich | 4 April 2008 | Mauhin, Viviane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EUROPEAN SEARCH REPORT

**European Patent Office**

**Application Number**

EP 08 00 2997

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | FUJIWARA S ET AL: "The world of archaea: genome analysis, evolution and thermostable enzymes" GENE: AN INTERNATIONAL JOURNAL ON GENES AND GENOMES, ELSEVIER, AMSTERDAM, NL, vol. 179, no. 1, 7 November 1996 (1996-11-07), pages 165-170, XP004071979 ISSN: 0378-1119 ----- | | |

**TECHNICAL FIELDS SEARCHED (IPC)**

The present search report has been drawn up for all claims

| Place of search | Date of completion of the search | Examiner |
|---|---|---|
| Munich | 4 April 2008 | Mauhin, Viviane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4708871 A
- JP 58110600 A
- JP 3022979 A
- US 4686191 A
- US 4939094 A
- US 5160735 A
- JP 63233798 A
- JP 60251887 A
- JP 2606856 B
- JP 2517813 B

- US 4554101 A
- EP 394827 A
- US 4631211 A
- US 5876969 A
- EP 0413622 A
- US 5766883 A
- WO 9622024 A
- WO 9904812 A
- JP 11313688 A

**Non-patent literature cited in the description**

- **MORIKAWA, M. et al.** *Appl. Environ. Microbiol.,* 1994, vol. 60 (12), 4559-4566
- **YAN, Z. et al.** *Appl. Environ. Microbiol.,* vol. 63, 785-789
- **BARTOLUCCI S.** *Third International Congress on Extremophiles Hamburg,* 03 September 2000
- **BATZER et al.** *Nucleic Acid Res.,* 1991, vol. 19, 5081
- **OHTSUKA et al.** *J. Biol. Chem.,* 1985, vol. 260, 2605-2608
- **ROSSOLINI et al.** *Mol. Cell. Probes,* 1994, vol. 8, 91-98
- **KOHLER ; MILSTEIN.** *Nature,* 1975, vol. 256, 495
- **BUCK et al.** *In Vitro,* 1982, vol. 18, 377
- Current Protocols in Molecular Biology. DNA Cloning 1: Core Techniques, A Practical Approach. Oxford University Press, 1995, 1-38
- **SAMBROOK ; FRITSCH ; MANIATIS.** Molecular Cloning: A Laboratory Manual. Cold Spring Harbor Laboratory, 1989
- **ANDERSON et al.** Nucleic Acid Hybridization: A Practical Approach. IRL Press Limited
- **SUGGS et al.** *Developmental Biology Using Purified Genes,* 1981, vol. 683
- **ALTSCHUL et al.** *J. Mol. Biol.,* 1990, vol. 215, 403-410
- **PEARSON ; LIPMAN.** *Proc. Natl. Acad. Sci.,* 1988, vol. 85, 2444-2448
- **SMITH ; WATERMAN.** *J. Mol. Biol.,* 1981, vol. 147, 195-197
- **NEEDLEMAN ; WUNSCH.** *J. Mol. Biol.,* 1970, vol. 48, 443-453
- **GEYSEN et al.** *Proc. Natl. Acad. Sci. USA,* 1984, vol. 81, 3998

- **GEYSEN et al.** *Molecular Immunology,* 1986, vol. 23, 709
- **PEARSON ; LIPMAN.** *Proc. Natl. Acad. Sci. USA,* 1988, vol. 85 (8), 2444-2448
- **ALTSCHUL et al.** *J. Mol. Biol.,* 1990, vol. 215 (3), 403-410
- **THOMPSON et al.** *Nucleic Acids Res.,* 1994, vol. 22 (2), 4673-4680
- **HIGGINS et al.** *Methods Enzymol.,* 1996, vol. 266, 383-402
- **ALTSCHUL et al.** *Nature Genetics,* 1993, vol. 3, 266-272
- **KARLIN ; ALTSCHUL.** *Proc. Natl. Acad. Sci. USA,* 1990, vol. 87, 2267-2268
- **ALTSCHUL et al.** *Nuc. Acids Res.,* 1997, vol. 25, 3389-3402
- **GONNET et al.** *Science,* 1992, vol. 256, 1443-1445
- **HENIKOFF ; HENIKOFF.** *Proteins,* 1993, vol. 17, 49-61
- Matrices for Detecting Distance Relationships: Atlas of Protein Sequence and Structure. National Biomedical Research Foundation, 1978
- *Agric. Biol. Chem,* 1984, vol. 48, 669
- *Agric. Biol. Chem.,* 1989, vol. 53, 277
- *Proc. Natl. Acad. Sci. USA,* 1985, vol. 82, 4306
- *J. Bacteriol.,* 1990, vol. 172, 2392
- *Gene,* 1985, vol. 33, 103
- **KYTE, J. ; DOOLITTLE, R.F.** *J. Mol. Biol.,* 1982, vol. 157 (1), 105-132
- Current Protocols in Molecular Biology. Wiley, 1988
- **SAMBROOK J et al.** Molecular Cloning: A Laboratory Manual. Cold Spring Harbor Laboratory Press, 1987

- **MORIKAWA, M. et al.** *Appl.Environ.Microbiol.,* 1994, vol. 60 (12), 4559-4566
- *Appl. Environ. Microbiol.,* 1994, vol. 60 (12), 4559-4566
- **IMANAKA et al.** *J. Bacteriol,* 1981, vol. 147, 776-786
- Molecular Cloning: A Laboratory Manual. Cold Spring Harbor Laboratory Press, 1989
- DNA-maikuro-arei-to-saisin-PCR-ho [DNA microarray and Up-to-date PCR Method. Saibo-kogaku (Cellular Engineering
- **SCHENK PM et al.** *Proc. Natl. Acad. Sci.,* 2000, vol. 97, 11655-11660
- **MARSHALL A et al.** *Nat. Biotechnol.,* 1998, vol. 16, 27-31
- **RAMSAY G et al.** *Nat. Biotechnol.,* 1998, vol. 16, 40-44
- **FAMBROUGH D et al.** *Cell,* 1999, vol. 97, 727-741
- **IYER VR et al.** *Science,* 1999, vol. 283, 83-87
- **MARTON MJ.** *Nat. Med.,* 1999, vol. 4, 1293-1301
- **CARGILL M et al.** *Nat. Genet.,* 1999, vol. 22, 231-238
- **FODOR SP et al.** *Science,* 1991, vol. 251, 767-773
- **LOCKART DJ et al.** *Nat. Biotechnol.,* 1996, vol. 14, 1675-1680
- **ERMOLAEVA O et al.** *Nat. Genet.,* 1998, vol. 20, 19-23
- **E. R. FELDER.** *Chimia,* 1994, vol. 48, 512-541
- **GALLOP et al.** *J. Med. Chem.,* 1994, vol. 37, 1233-1251
- **R. A. HOUGHTEN.** *Trends Genet.,* 1993, vol. 9, 235-239
- **HOUGHTENET.** *Nature,* 1991, vol. 354, 84-86
- **LAM et al.** *Nature,* 1991, vol. 354, 82-84
- **CARELL et al.** *Chem. Biol.,* 1995, vol. 3, 171-183
- **MADDEN et al.** *Perspectives in Drug Discovery and Design,* vol. 2, 269-282
- **CWIRLA et al.** *Biochemistry,* 1990, vol. 87, 6378-6382
- **BRENNER et al.** *Proc.Natl. Acad. Sci. USA,* 1992, vol. 89, 5381-5383
- **GORDON et al.** *J. Med. Chem.,* 1994, vol. 37, 1385-1401
- **LEBL et al.** *Biopolymers,* 1995, vol. 37, 177-198
- **CAMPBELL, S. A.** The Science and Engineering of Microelectronic Fabrication. Oxford University Press, 1996
- **ZAUT, P. V.** Microarray Fabrication: a Practical Guide to Semiconductor Processing, Semiconductor Services. 1996
- **MADOU, M. J.** Fundamentals of Microfabrication. CRC1 5 Press, 1997
- **RAI-CHOUDHURY, P.** Handbook of Microlithography, Micromachining, & Microfabrication: Microlithography. 1997
- *Science,* 1991, vol. 251, 767
- **TRAUNECKER et al.** *Nature,* 1988, vol. 331, 84-86
- **GEYSEN et al.** *Proc.Natl.Acad.Sci.USA,* 1983, vol. 81, 3998-4002
- **HOUGHTEN.** *Proc. Natl. Acad. Sci. USA,* 1985, vol. 82, 5131-5135
- **WILSON et al.** *Cell,* 1984, vol. 37, 767-778
- **SUTCLIFFE et al.** *Science,* 1983, vol. 219, 660-666
- **CHOW et al.** *Proc. Natl. Acad. Sci. USA,* vol. 82, 910-914
- **BITTLE et al.** *J. Gen. Virol.,* 1985, vol. 66, 2347-2354
- **FOUNTOULAKIS et al.** *J.Biochem.,* 1995, vol. 270, 3958-3964
- **JANKNECHT et al.** *Proc. Natl. Acad. Sci. USA,* 1991, vol. 88, 8972-897
- **SAMBROOK,J. et al.** Molecular Cloning:A Laboratory Manual. Cold Spring Harbor Laboratory Press, 2001
- **AUSUBEL, F. et al.** Short protocols in molecular biology. John Wiley&Sons, 1999
- **AUSUBEL,F.** Current Protocols in Molecular Biology. John Wiley&Sons, 1988
- Biochemistry Handbook. Asakura Shoten, 1987
- Dictionary of BIOCHEMISTRY. Tokyo Kagaku Dojin, 1998
- Saibokino to Taisha mappu (Cellular Functions and Metabolism map. 1997
- Lewin Genes VII. Oxford University Press, 2000
- **FRANK T.** *Thermophiles(Archaea:A Laboratory Manual 3,* 1995
- *KOSOGAKU HANDOBUKKU (Enzyme handbook,* 1982
- *Methods in Enzymology*
- Seikagaku Jiten (Dictionary of BIOCHEMISTRY. 1998
- Saibokino to Taisha mappu (Cellular Functions and Metabolism map. Tokyo Kagaku Dojin, 1997
- **LENGELER,J.** Biology of the Prokaryotes. Blackwell Science, 1998
- **LEWIN.** Genes. Oxford University Press, 2000, vol. VII
- **FRASER C.M.** *Res Microbiol.,* 2000, vol. 151, 79-84
- **FRASER C.M. et al.** *Nature,* 2000, vol. 406, 799-803
- **NELSON et al.** *Nat Biotechnol.,* 2000, vol. 18, 1049-1054
- **KAWARABAYASI Y. et al.** *DNA Res.,* 1999, vol. 6, 83-101145-222

- Media for Thermophiles. **ROBB,F.T. ; A.R.PLACE.** Archea: a laboratory manual-Thermophiles. Cold Spring Harbor Press, 1995, 167-168
- **SAMBROOK, J. ; D. RUSSEL.** Molecular cloning: a laboratory manual. Cold Spring Harbor Press, 2001
- **BECKWITH, J. R ; A. B. PARDEE ; R. AUSTRIAN ; F. JACOB.** Coordination of the synthesis of the enzymes in the pyrimidine pathway of E. coli. *J. Mol. Biol.,* 1962, vol. 5, 618-634
- **SAMBROOK, J. ; D.RUSSEL.** Molecular cloning: a laboratory manual. Cold Spring Harbor Press, 2001
- **BERTANI, G ; L.BARESI.** Genetic transformation in the methanogen Methanococcus voltae PS. *J. Bacteriol,* 1987, vol. 169, 2730-2738
- **ROSSI,R et al.** *Nucleic Acids Research,* 1997, vol. 25 (11), 2106-2113
- **ODELL,M.** *Virology,* 1996, vol. 221, 120-129
- **SRISKANDA,V. et al.** *Nucleic Acids Research,* 1998, vol. 26 (20), 4618-4625
- **TAKAHASHI,M et al.** *The Journal of Biological Chemistry,* 1984, vol. 259 (16), 10041-10047
- **ANDREESEN,J.R. et al.** *J. Bacteriol,* 1974, vol. 120, 6-14
- **ANDREESEN, J.R et al.** *J.Bacterio1,* 1974, vol. 120, 6-14
- **PISANI, F.M. et al.** *Eur.J.Biochem.,* 1990, vol. 187, 321-328
- Chitin, Chitosan Experimental Manual. Gihodo Publishing
- **ROBBINS, P. W.** *J. Biol. Chem.,* 1988, vol. 263 (1), 443-447
- **UEMURA, K.** *Plant Cell Physiol.,* 1996, vol. 37 (3), 325-331
- **EZAKI et al.** *J. Biol. Chem.,* 19 February 1999, vol. 274 (8), 5078-82
- KOSOGAKU HANDOBUKKU (Enzyme handbook. Asakura shoten, 1982
- *Methods in Enzymology,* 1995, vol. 262
- Methods in Enzymology. Acadmic Press
- **FRYDMAN, J. et al.** *Nature,* 1994, vol. 370, 111
- Methods in Enzymology. Academic Press
- **ALDRIDGE P ; HUGHES KT.** *Curr Opin Microbiol.,* April 2002, vol. 5 (2), 160-5